# EUROPEAN PATENT APPLICATION

(11) **EP 1 552 850 A2**
(43) Date of publication of application: **13.07.2005**
(21) Application number: 05008493.8
(22) Date of filing: 22.12.1999
(51) Int. Cl.: A61K 45/06, A61P 3/10

(54) **A combination of FBPase inhibitors and insulin sensitizers for the treatment of diabetes**

(30) Priority: 24.12.1998 US 114718 P
(62) Divisional of application: 99964313.3
(71) Applicant: Metabasis Therapeutics, Inc., San Diego, CA 92121 (US)
(72) Inventor: Erion, Mark D., Del Mar, CA 92014 (US); Vanpoelje, Paul, La Jolla, CA 92037 (US)
(74) Representative: Sexton, Jane Helen

(57) **Abstract**

Use of an insulin sensitizer agent and an FBPase inhibitor or a prodrug or salt thereof in the manufacture of a medicament for preventing diabetes in an animal at risk of developing diabetes.

## Description

### Related Applications

This application is a continuation-in-part of Provisional Application Serial No. 60/114,718, filed December 23, 1998 and is hereby incorporated by reference in its entirety.

### Background of the Invention

A combination therapy of an insulin sensitizer and an FBPase inhibitor is disclosed for the treatment of diabetes, and other diseases where the control of blood glucose levels or an improvement in insulin sensitivity, reduction in insulin levels or an enhancement of insulin secretion is beneficial. Compositions used in the therapy are also disclosed.

### Background of the Invention

Diabetes mellitus (or diabetes) is one of the most prevalent diseases in the world today. Diabetes patients have been divided into two classes, namely type I or insulin-dependent diabetes mellitus and type II or non-insulin dependent diabetes mellitus (NIDDM). NIDDM accounts for approximately 90% of all diabetics and is estimated to affect 12-14 million adults in the U. S. alone (6.6% of the population). NIDDM is characterized by both fasting hyperglycemia and exaggerated postprandial increases in plasma glucose levels. NIDDM is associated with a variety of long-term complications, including microvascular diseases such as retinopathy, nephropathy and neuropathy, and macrovascular diseases such as coronary heart disease. Numerous studies in animal models demonstrate a causal relationship between long term hyperglycemia and complications. Results from the Diabetes Control and Complications Trial (DCCT) and the Stockholm Prospective Study demonstrate this relationship for the first time in man by showing that insulin-dependent diabetics with tighter glycemic control are at substantially lower risk for the development and progression of these complications. Tighter control is also expected to benefit NIDDM patients.

Current therapies used to treat NIDDM patients entail both controlling lifestyle risk factors and pharmaceutical intervention. First-line therapy for NIDDM is typically a tightly-controlled regimen of diet and exercise since an overwhelming number of NIDDM patients are overweight or obese (67%) and since weight loss can improve insulin secretion, insulin sensitivity and lead to normoglycemia. Normalization of blood glucose occurs in less than 30% of these patients due to poor compliance and poor response. Patients with hyperglycemia not controlled by diet alone are subsequently treated with oral hypoglycemics or insulin. Until recently, the sulfonylureas were the only class of oral hypoglycemic agents available for NIDDM. Treatment with sulfonylureas leads to effective blood glucose lowering in only 70% of patients and only 40% after 10 years of therapy. Patients that fail to respond to diet and sulfonylureas are subsequently treated with daily insulin injections to gain adequate glycemic control.

Although the sulfonylureas represent a major therapy for NIDDM patients, four factors limit their overall success. First, as mentioned above, a large segment of the NIDDM population do not respond adequately to sulfonylurea therapy (*i.e.* primary failures) or become resistant (*i.e.* secondary failures). This is particularly true in NIDDM patients with advanced NIDDM since these patients have severely impaired insulin secretion. Second, sulfonylurea therapy is associated with an increased risk of severe hypoglycemic episodes. Third, chronic hyperinsulinemia has been associated with increased cardiovascular disease although this relationship is considered controversial and unproven. Last, sulfonylureas are associated with weight gain, which leads to worsening of peripheral insulin sensitivity and thereby can accelerate the progression of the disease.

Results from the U.K. Diabetes Prospective Study also showed that patients undergoing maximal therapy of a sulfonylurea, metformin, or a combination of the two, were unable to maintain normal fasting glycemia over the six year period of the study. U.K. Prospective Diabetes Study 16. Diabetes, 44:1249-158 (1995). These results further illustrate the great need for alternative therapies.

Another drug therapy recently developed for NIDDM patients acts on the underlying mechanisms of insulin resistance and thereby lower glucose by enhancing insulin action at both peripheral and hepatic sites. Saltiel & Olefsky Diabetes 45: 1661-1669 (1996). Accordingly, these agents are reported to increase insulin-dependent glucose disposal and to inhibit HGO. These agents are commonly referred to as "insulin sensitizers".

One class of insulin sensitizers are compounds containing a thiazolidinedione. These compounds are reported to enhance insulin action without directly stimulating insulin secretion. Thiazolidinediones markedly decrease glucose levels in a variety of obese, insulin-resistant diabetic animal models including the KK-mouse, ob/ob mouse, Zucker Diabetic Fatty rat and db/db mouse. Similar effects are found in non-genetic diabetic animal models, including the fructose fed rat and high fat fed rat. Animal models characterized by severe hypoinsulinemia, e.g. the STZ rat, fail to respond to these agents unless treated with insulin. Thiazolidinediones are also reported to restore the ability of insulin to suppress HGO.

Although the molecular target of insulin sensitizers and more specifically thiazolidinedione analogs is unknown, several studies suggest that peroxisome proliferator-activated receptors (PPAR γs) may be the target and therefore that ligands to these receptors will be useful antihyperglycemic agents. Lehmann et al. J. Biol. Chem. 270: 12953-12956 (1995). PPAR γs are members of the steroid/thyroid hormone receptor superfamily of transcription factors. At least three PPAR γs exist, namely the α, β and γ receptors and thiazolidinediones have been identified as ligands that activate the β and γ receptors. Binding occurs at a concentration achieved in vivo and some data suggests that there is a correlation between PPAR γ binding affinity and in vivo activity. Wilson et al. J. Med. Chem. 39: 665-668 (1996).

PPAR γs exist as a heterodimer with the retinoic acid X receptor (RXR). A co-repressor protein has been postulated to maintain the receptor in an inactive state similar to other nuclear receptors. Binding of molecules to the complex, i.e. PPAR γ ligands and/or RXR ligands may lead to dissociation of the co-repressor protein and activation of the receptor, which in turn is postulated to interact with specific DNA sequences, PPRE's, and to activate or repress gene transcription. Accordingly, RXR ligands are thought to enhance insulin sensitivity and therefore be useful as antidiabetics either alone or in combination with PPAR γ agonists such as a thiazolidinedione. Heyman et al., WO 97/10819. In db/db mice, the combination of an RXR ligand and a PPAR γ agonist reduces glucose levels more than either component alone.

Other classes of insulin sensitizers (i.e. non-thiazolidinediones) have been identified. For example, the insulin sensitizers SB 236636 and SB 219994 are 3-aryl-2-alkoxy propanoic acids. These compounds are reported to bind to human PPAR γ with high affinity. SB 236636 is equipotent with thiazolidinedione BRL 49653 in stimulation of glucose transport in differentiated 3T3-L1 adipocytes and in glucose lowering activity in ob/ob mice. Young et al. Diabetes (1997). Relative to other thiazolidinediones, SB 236636 was shown to bind with higher affinity to crude extracts of Sf9 cells transfected with full length hPPAR γ and rat adipocytes. This higher binding affinity correlated well with in vivo potency.

Some data suggests that chronic activation of PKC isoenzymes is involved in the generation of muscle insulin resistance and that insulin sensitizers may decrease the translocation of PKC isoenzymes from the cytosolic to particulate fractions in red skeletal muscle and therefore PKC activation. Schmitz-Peiffer et al. Am. J. Physiol. 273: E915-E921 (1997)

Angiotensin II antagonists and angiotensin converting enzyme inhibitors may be useful in enhancing insulin sensitivity based on potential interactions between angiotensin II and insulin signaling systems. Torlone et al.Diabetes Care 16: 1347-1355 (1993); Howard G. et al., Circulation 93: 1809-1817 (1996); Folli et al. J. Clin. Invest. 100: 2158-2169 (1997); Tamura et al., W09737688 A2.

Thus, there are several mechanisms by which agents may act as insulin sensitizers.

Gluconeogenesis from pyruvate is a highly regulated biosynthetic pathway requiring eleven enzymes. Seven enzymes catalyze reversible reactions and are common to both gluconeogenesis and glycolysis. Four enzymes catalyze reactions unique to gluconeogenesis, namely pyruvate carboxylase, phosphoenolpyruvate carboxykinase, fructose-1,6-bisphosphatase and glucose-6-phosphatase. Overall flux through the pathway is controlled by the specific activities of these enzymes, the enzymes that catalyzed the corresponding steps in the glycolytic direction, and by substrate availability. Dietary factors (glucose, fat) and hormones (insulin, glucagon, glucocorticoids, epinephrine) coordinatively regulate enzyme activities in the gluconeogenesis and glycolysis pathways through gene expression and post-translational mechanisms.

Synthetic inhibitors of fructose-1,6-bisphosphatase (hereinafter "FBPase") have been reported. McNiel reported that fructose-2,6-bisphosphate analogs inhibit FBPase by binding to the substrate site. J. Am. Chem. Soc., 106:7851-7853 (1984); U.S. Patent No. 4,968,790 (1984). These compounds, however, were relatively weak and did not inhibit glucose production in hepatocytes presumably due to poor cell penetration.

Gruber reported that some nucleosides can lower blood glucose in the whole animal through inhibition of FBPase. These compounds exert their activity by first undergoing phosphorylation to the corresponding monophosphate. EP 0 427 799 B1.

Gruber et al. U.S. Patent No. 5,658,889 described the use of inhibitors of the AMP site of FBPase to treat diabetes. WO 98/39344, WO 98/39343, and WO 98/39342 describe the use of FBPase inhibitors to treat diabetes.

### Summary of the Invention

The instant invention is a combination therapy and a composition for the treatment for diabetes and diseases responding to improved glycemic control or to improve peripheral insulin sensitivity. The therapy requires administration of a insulin sensitizer agent, e.g. PPAR γ agonist, RXR ligand, or another agent known to enhance insulin action and an FBPase inhibitor either together or at a different time such that improved glycemic control is achieved. In another aspect of the invention, the combined therapy results in decreases in hepatic glucose output beyond that observed for glucose lowering doses of the insulin sensitizer agent. Furthermore, the combined therapy can result in improvements in insulin resistance and/or insulin secretion beyond that observed for either agent alone. Yet another aspect of the invention is that a combination therapy achieves similar benefits as observed with one or the other therapies alone but at significantly lower doses.

The present invention relates to methods and compositions for treating an animal having NIDDM or a condition associated with insulin resistance by administering to the animal a composition containing a pharmaceutically effective amount of an agent that enhances insulin sensitivity and a pharmaceutically effective amount of an FBPase inhibitor. The compositions of this invention are adapted to cure, improve or prevent one or more symptoms of NIDDM. A preferred drug combination will have high potency and low toxicity.

Another object of the invention relates to methods and compositions for treating insulin-requiring NIDDM patients. The combination therapy decreases the insulin requirement and associated safety risks.

Another object of the invention relates to methods and compositions for treating diseases or conditions characterized by insulin resistance, including obesity, hypertension, impaired glucose tolerance, and polycystic ovarian syndrome. Individuals with syndrome X, renal disease, or pancreatitis are also effectively treated with the combination therapy.

Another object of the invention is the use of insulin sensitizer to attenuate certain potentially adverse effects that could be associated with FBPase inhibitor therapy such as lactate and triglyceride elevation.

Another object of the invention is the use of FBPase inhibitors to attenuate certain potentially adverse effects that could be associated with insulin sensitizers such as weight gain.

Another aspect of the invention is to use FBPase inhibitors in combination with insulin sensitizer therapies that include administration of agents that enhance endogenous or exogenous insulin levels, such as sulfonylureas, insulin, or insulin mimetics.

### Definitions

In accordance with the present invention and as used herein, the following terms are defined with the following meanings, unless explicitly stated otherwise.

X and X³ group nomenclature as used herein in formulae I and X describes the group attached to the phosphonate and ends with the group attached to the heteroaromatic ring. For example, when X is alkylamino, the following structure is intended:

(heteroaromatic ring)-NR-alk-P(O)(OR¹)₂

Likewise, A, B, C, D, E, A", B", C", D", E", A², L², E², and J² groups and other substituents of the heteroaromatic ring are described in such a way that the term ends with the group attached to the heteroaromatic ring. Generally, substituents are named such that the term ends with the group at the point of attachment.

The term "aryl" refers to aromatic groups which have 5-14 ring atoms and at least one ring having a conjugated pi electron system and includes carbocyclic aryl, heterocyclic aryl and biaryl groups, all of which may be optionally substituted. Suitable aryl groups include phenyl and furan-2,5-diyl.

Carbocyclic aryl groups are groups wherein the ring atoms on the aromatic ring are carbon atoms. Carbocyclic aryl groups include monocyclic carbocyclic aryl groups and polycyclic or fused compounds such as optionally substituted naphthyl groups.

Heterocyclic aryl or heteroaryl groups are groups having from 1 to 4 heteroatoms as ring atoms in the aromatic ring and the remainder of the ring atoms being carbon atoms. Suitable heteroatoms include oxygen, sulfur, nitrogen, and selendum. Suitable heteroaryl groups include furanyl, thienyl, pyridyl, pyrrolyl, N-lower alkyl pyrrolyl, pyridyl-N-oxide, pyrimidyl, pyrazinyl, imidazolyl, and the like, all optionally substituted.

The term "annulation" or "annulated" refers to the formation of an additional cyclic moiety onto an existing aryl or heteroaryl group. The newly formed ring may be carbocyclic or heterocyclic, saturated or unsaturated, and contains 2-9 new atoms of which 0-3 may be heteroatoms taken from the group of N, O, and S. The annulation may incorporate atoms from the X group as part of the newly formed ring. For example, the phrase "together L² and E² form an annulated cyclic group," includes

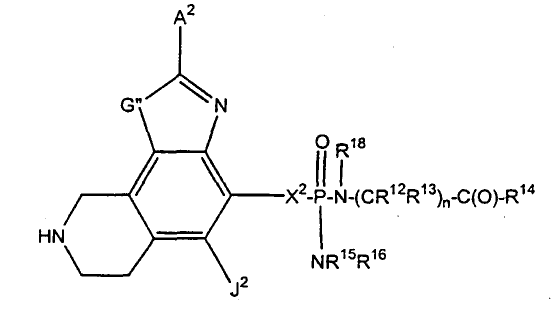

The term "biaryl" represents aryl groups containing more than one aromatic ring including both fused ring systems and aryl groups substituted with other aryl groups. Such groups may be optionally substituted. Suitable biaryl groups include naphthyl and biphenyl.

The term "alicyclic" means compounds which combine the properties of aliphatic and cyclic compounds. Such cyclic compounds include but are not limited to, aromatic, cycloalkyl and bridged cycloalkyl compounds. The cyclic compound includes heterocycles. Cyclohexenylethyl and cyclohexylethyl are suitable alicyclic groups. Such groups may be optionally substituted.

The term "optionally substituted" or "substituted" includes groups substituted by one to four substituents, independently selected from lower alkyl, lower aryl, lower aralkyl, lower alicyclic, hydroxy, lower alkoxy, lower aryloxy, perhaloalkoxy, aralkoxy, heteroaryl, heteroaryloxy, heteroarylalkyl, heteroaralkoxy, azido, amino, guanidino, amidino, halo, lower alkylthio, oxo, acylalkyl, carboxy esters, carboxyl, -carboxamido, nitro, acyloxy, aminoalkyl, alkylaminoaryl, alkylaryl, alkylaminoalkyl, alkoxyaryl, arylamino, aralkylamino, phosphono, sulfonyl, -carboxamidoalkylaryl, -carboxamidoaryl, hydroxyalkyl, haloalkyl, alkylaminoalkylcarboxy-, aminocarboxamidoalkyl-, cyano, lower alkoxyalkyl, lower perhaloalkyl, and arylalkyloxyalkyl. "Substituted aryl" and "substituted heteroaryl" preferably refers to aryl and heteroaryl groups substituted with 1-3 substituents. Preferably these substituents are selected from the group consisting of lower alkyl, lower alkoxy, lower perhaloalkyl, halo, hydroxy, and amino. "Substituted" when , describing an R⁵ group does not include annulation.

The term "aralkyl" refers to an alkyl group substituted with an aryl group. Suitable aralkyl groups include benzyl, picolyl, and the like, and may be optionally substituted. The term "-aralkyl-" refers to a divalent group -aryl-alkylene-. "Heteroarylalkyl" refers to an alkylene group substituted with a heteroaryl group.

The term "-alkylaryl-" refers to the group -alk-aryl- where "alk" is an alkylene group. "Lower -alkylaryl-" refers to such groups where alkylene is lower alkylene.

The term "lower" referred to herein in connection with organic radicals or compounds respectively defines such as with up to and including 10, preferably up to and including 6, and advantageously one to four carbon atoms. Such groups may be straight chain, branched, or cyclic.

The terms "arylamino" (a), and "aralkylamino" (b), respectively, refer to the group -NRR' wherein respectively, (a) R is aryl and R' is hydrogen, alkyl, aralkyl or aryl, and (b) R is aralkyl and R' is hydrogen or aralkyl, aryl, alkyl.

The term "acyl" refers to -C(O)R where R is alkyl and aryl.

The term "carboxy esters" refers to -C(O)OR where R is alkyl, aryl, aralkyl, and alicyclic, all optionally substituted.

The term "carboxyl" refers to -C(O)OH.

The term "oxo" refers to =O in an alkyl group.

The term "amino" refers to -NRR' where R and R' are independently selected from hydrogen, alkyl, aryl, aralkyl and alicyclic, all except H are optionally substituted; and R and R¹ can form a cyclic ring system.

The term "carbonylamino" and "-carbonylamino-" refers to RCONR- and -CONR-, respectively, where each R is independently hydrogen or alkyl.

The term "halogen" or "halo" refers to -F, -Cl, -Br and -I.

The term "-oxyalkylamino-" refers to -O-alk-NR-, where "alk" is an alkylene group and R is H or alkyl.

The term "-alkylaminoalkylcarboxy-" refers to the group -alk-NR-alk-C(O)-O-where "alk" is an alkylene group, and R is a H or lower alkyl.

The term "-alkylaminocarbonyl-" refers to the group -alk-NR-C(O)- where "alk" is an alkylene group, and R is a H or lower alkyl.

The term "-oxyalkyl-" refers to the group -O-alk- where "alk" is an alkylene group.

The term "-alkylcarboxyalkyl-" refers to the group -alk-C(O)-O-alk- where each alk is independently an alkylene group.

The term "alkyl" refers to saturated aliphatic groups including straight-chain, branched chain and cyclic groups. Alkyl groups may be optionally substituted. Suitable alkyl groups include methyl, isopropyl, and cyclopropyl.

The term "cyclic alkyl" or "cycloalkyl" refers to alkyl groups that are cyclic. Suitable cyclic groups include norbornyl and cyclopropyl. Such groups may be substituted.

The term "heterocyclic" and "heterocyclic alkyl" refer to cyclic groups of 3 to 10 atoms, more preferably 3 to 6 atoms, containing at least one heteroatom, preferably 1 to 3 heteroaroms. Suitable heteroatoms include oxygen, sulfur, and nitrogen. Heterocyclic groups may be attached through a nitrogen or through a carbon atom in the ring. Suitable heterocyclic groups include pyrrolidinyl, morpholino, morpholinoethyl, and pyridyl.

The term "phosphono" refers to -PO₃R₂, where R is selected from the group consisting of -H, alkyl, aryl, aralkyl, and alicyclic.

The term "sulphonyl" or "sulfonyl" refers to -SO₃R, where R is H, alkyl, aryl, aralkyl, and alicyclic.

The term "alkenyl" refers to unsaturated groups which contain at least one carbon-carbon double bond and includes straight-chain, branched-chain and cyclic groups. Alkenyl groups may be optionally substituted. Suitable alkenyl groups include allyl. "1-alkenyl" refers to alkenyl groups where the double bond is between the first and second carbon atom. If the 1-alkenyl group is attached to another group, *e*.*g*. it is a W substituent attached to the cyclic phosph(oramid)ate, it is attached at the first carbon.

The term "alkynyl" refers to unsaturated groups which contain at least one carbon-carbon triple bond and includes straight-chain, branched-chain and cyclic groups. Alkynyl groups may be optionally substituted. Suitable alkynyl groups include ethynyl. "1-alkynyl" refers to alkynyl groups where the triple bond is between the first and second carbon atom. If the 1-alkynyl group is attached to another group, *e.g.* it is a W substituent attached to the cyclic phosph(oramid)ate, it is attached at the first carbon.

The term "alkylene" refers to a divalent straight chain, branched chain or cyclic saturated aliphatic group.

The term "-cycloalkylene-COOR³" refers to a divalent cyclic alkyl group or heterocyclic group containing 4 to 6 atoms in the ring, with 0-1 heteroatoms selected from O, N, and S. The cyclic alkyl or heterocyclic group is substituted with -COOR³.

The term "acyloxy" refers to the ester group -O-C(O)R, where R is H, alkyl, alkenyl, alkynyl, aryl, aralkyl, or alicyclic.

The term "aminoalkyl-" refers to the group NR₂-alk- wherein "alk" is an alkylene group and R is selected from H, alkyl, aryl, aralkyl, and alicyclic.

The term "-alkyl(hydroxy)-" refers to an -OH off the alkyl chain. When this term is an X group, the -OH is at the position α to the phosphorus atom.

The term "alkylaminoalkyl-" refers to the group alkyl-NR-alk- wherein each "alk" is an independently selected alkylene, and R is H or lower alkyl. "Lower alkylaminoalkyl-" refers to groups where each alkylene group is lower alkylene.

The term "arylaminoalkyl-" refers to the group aryl-NR-alk- wherein "alk" is an alkylene group and R is H, alkyl, aryl, aralkyl, and alicyclic. In "lower arylaminoalkyl-", the alkylene group is lower alkylene.

The term "alkylaminoaryl-" refers to the group alkyl-NR-aryl- wherein "aryl" is a divalent group and R is H, alkyl, aralkyl, and alicyclic. In "lower alkylaminoaryl-", the alkylene group is lower alkyl.

The term "alkyloxyaryl-" refers to an aryl group substituted with an alkyloxy group. In "lower alkyloxyaryl-", the alkyl group is lower alkyl.

The term "aryloxyalkyl-" refers to an alkyl group substituted with an aryloxy group.

The term "aralkyloxyalkyl-" refers to the group aryl-alk-O-alk- wherein "alk" is an alkylene group. "Lower aralkyloxyalkyl-" refers to such groups where the alkylene groups are lower alkylene.

The term "-alkoxy-" or "-alkyloxy-" refers to the group -alk-O- wherein "alk" is an alkylene group. The term "alkoxy-" refers to the group alkyl-O-.

The term "-alkoxyalkyl-" or "-alkyloxyalkyl-" refer to the group -alk-O-alk-wherein each "alk" is an independently selected alkylene group. In "lower -alkoxyalkyl-", each alkylene is lower alkylene.

The terms "alkylthio-" and "-alkylthio-" refer to the groups alkyl-S-, and -alk-S-, respectively, wherein "alk" is alkylene group.

The term "-alkylthioalkyl-" refers to the group -alk-S-alk- wherein each "alk" is an independently selected alkylene group. In "lower -alkylthioalkyl-" each alkylene is lower alkylene.

The term "alkoxycarbonyloxy-" refers to alkyl-O-C(O)-O-.

The term "aryloxycarbonyloxy-" refers to aryl-O-C(O)-O-.

The term "alkylthiocarbonyloxy-" refers to alkyl-S-C(O)-O-.

The term "-alkoxycarbonylamino-" refers to -alk-O-C(O)-NR¹-,where "alk" is alkylene and R¹ includes -H, alkyl, aryl, alicyclic, and aralkyl.

The term "-alkylaminocarbonylamino-" refers to -alk-NR¹-C(O)-NR¹-, where "alk" is alkylene and R¹ is independently selected from H, alkyl, aryl, aralkyl, and alicyclic.

The terms "amido" or "carboxamido" refer to NR₂-C(O)- and RC(O)-NR¹-, where R and R¹ include H, alkyl, aryl, aralkyl, and alicyclic. The term does not include urea, - NR-C(O)-NR-.

The terms "carboxamidoalkylaryl" and "carboxamidoaryl" refers to an aryl-alk-NR¹-C(O)-, and an -NR¹-C(O)-alk-, respectively, where "ar" is aryl, and "alk" is alkylene, R¹ and R include H, alkyl, aryl, aralkyl, and aliyclic.

The term "-alkylcarboxamido-" or "-alkylcarbonylamino-" refers to the group -alk-C(O)N(R)- wherein "alk" is an alkylene group and R is H or lower alkyl.

The term "-alkylaminocarbonyl-" refers to the group -alk-NR-C(O)- wherein "alk" is an alkylene group and R is H or lower alkyl.

The term "aminocarboxamidoalkyl-" refers to the group NR₂-C(O)-N(R)-alk-wherein R is an alkyl group or H and "alk" is an alkylene group. "Lower aminocarboxamidoalkyl-" refers to such groups wherein "alk" is lower alkylene.

The term "thiocarbonate" refers to -O-C(S)-O- either in a chain or in a cyclic group.

The term "hydroxyalkyl" refers to an alkyl group substituted with one -OH.

The term "haloalkyl" refers to an alkyl group substituted with one halo, selected from the group I, Cl, Br, F.

The term "cyano" refers to -C≡N.

The term "nitro" refers to -NO₂.

The term "acylalkyl" refers to an alkyl-C(O)-alk-, where "alk" is alkylene.

The term "heteroarylalkyl" refers to an alkyl group substituted with a heteroaryl group.

The term "-1,1-dihaloalkyl-" refers to an X group where the 1 position and therefore halogens are α to the phosphorus atom.

The term "perhalo" refers to groups wherein every C-H bond has been replaced with a C-halo bond on an aliphatic or aryl group. Suitable perhaloalkyl groups include -CF₃ and -CFCl₂.

The term "guanidino" refers to both -NR-C(NR)-NR₂ as well as -N=C(NR₂)₂ where each R group is independently selected from the group of -H, alkyl, alkenyl, alkynyl, aryl, and alicyclic, all except -H are optionally substituted.

The term "amidino" refers to -C(NR)-NR₂ where each R group is independently selected from the group of -H, alkyl, alkenyl, alkynyl, aryl, and alicyclic, all except -H are optionally substituted.

The term "2-thiazolyl-" or "2-oxazolyl-" or "2-selenozoly" refers to the corresponding base and its attachment of the X group at the 2-position of the heterocycle.

The term "pharmaceutically acceptable salt" includes salts of compounds of formula I and its prodrugs derived from the combination of a compound of this invention and an organic or inorganic acid or base. Suitable acids include HCl.

The term "prodrug" as used herein refers to any compound that when administered to a biological system generates the "drug" substance (a biologically active compound) as a result of spontaneous chemical reaction(s), enzyme catalyzed chemical reaction(s), and/or metabolic chemical reaction(s). Standard prodrugs are formed using groups attached to functionality, *e*.*g*. HO-, HS-, HOOC-, R₂N-, associated with the FBPase inhibitor, that cleave *in vivo.* Standard prodrugs include but are not limited to carboxylate esters where the group is alkyl, aryl, aralkyl, acyloxyalkyl, alkoxycarbonyloxyalkyl as well as esters of hydroxyl, thiol and amines where the group attached is an acyl group, an alkoxycarbonyl, aminocarbonyl, phosphate or sulfate. The groups illustrated are exemplary, not exhaustive, and one skilled in the art could prepare other known varieties of prodrugs. Such prodrugs of the compounds of formulae I and X, fall within the scope of the present invention. Prodrugs must undergo some form of a chemical transformation to produce the compound that is biologically active or is a precursor of the biologically active compound. In some cases, the prodrug is biologically active usually less than the drug itself, and serves to improve efficacy or safety through improved oral bioavailability, pharmacodynamic half-life, etc.
The term "prodrug ester" as employed herein includes, but is not limited to, the following groups and combinations of these groups:
[1] Acyloxyalkyl esters which are well described in the literature (Farquhar et al., J. Pharm. Sci. 72, 324-325 (1983)) and are represented by formula A 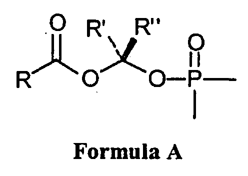 wherein R, R', and R" are independently H, alkyl, aryl, alkylaryl, and alicyclic; (see WO 90/08155; WO 90/10636).
[2] Other acyloxyalkyl esters are possible in which an alicyclic ring is formed such as shown in formula B. These esters have been shown to generate phosphorus-containing nucleotides inside cells through a postulated sequence of reactions beginning with deesterification and followed by a series of elimination reactions (e.g. Freed et al., Biochem. Pharm. 38: 3193-3198 (1989)). 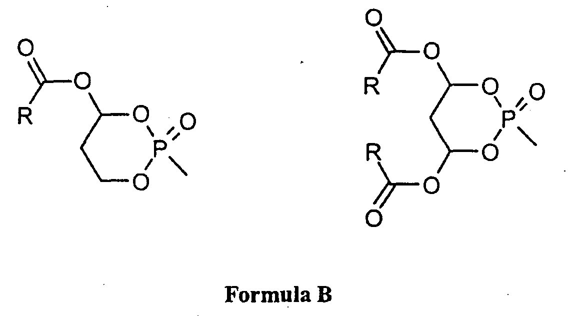 wherein R is -H, alkyl, aryl, alkylaryl, alkoxy, aryloxy, alkylthio, arylthio, alkylamino, arylamino, cycloalkyl, or alicyclic.
[3] Another class of these double esters known as alkyloxycarbonyloxymethyl esters, as shown in formula A, where R is alkoxy, aryloxy, alkylthio, arylthio, alkylamino, and arylamino; R', and R" are independently H, alkyl, aryl, alkylaryl, and alicyclic, have been studied in the area of β-lactam antibiotics (Tatsuo Nishimura et al. *J. Antibiotics,* **1987**, *40(1)*, 81-90; for a review see Ferres, H., *Drugs of Today*, **1983**, *19*, 499.). More recently Cathy, M. S., et al. (Abstract from AAPS Western Regional Meeting, April, **1997)** showed that these alkyloxycarbonyloxymethyl ester prodrugs on (9-[(R)-2-phosphonomethoxy)propyl]adenine (PMPA) are bioavailable up to 30% in dogs.
[4] Aryl esters have also been used as phosphonate prodrugs (*e.g.* Erion, DeLambert et al., J. Med. Chem. 37: 498, 1994; Serafinowska et al., J. Med. Chem. 38: 1372, 1995). Phenyl as well as mono and poly-substituted phenyl proesters have generated the parent phosphonic acid in studies conducted in animals and in man (Formula C). Another approach has been described where Y is a carboxylic ester ortho to the phosphate. Khamnei and Torrence, J. Med. Chem.; 39:4109-4115 (1996). 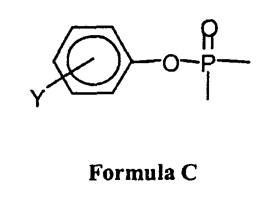 wherein Y is H, alkyl, aryl, alkylaryl, alkoxy, acyloxy, halogen, amino, alkoxycarbonyl, hydroxy, cyano, and alicyclic.
[5] Benzyl esters have also been reported to generate the parent phosphonic acid. In some cases, using substituents at the para-position can accelerate the hydrolysis. Benzyl analogs with 4-acyloxy or 4-alkyloxy group [Formula D, X = H, OR or O(CO)R or O(CO)OR] can generate the 4-hydroxy compound more readily through the action of enzymes, *e.g.* oxidases, esterases, etc. Examples of this class of prodrugs are described in Mitchell et al., J. Chem. Soc. Perkin Trans. I 2345 (1992); Brook, et al. WO 91/19721. 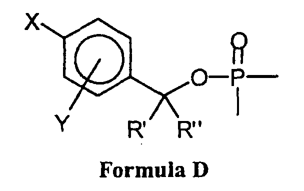 wherein X and Y are independently H, alkyl, aryl, alkylaryl, alkoxy, acyloxy, hydroxy, cyano, nitro, perhaloalkyl, halo, or alkyloxycarbonyl; and
   R' and R" are independently H, alkyl, aryl, alkylaryl, halogen, and alicyclic.
[6] Thio-containing phosphonate proesters have been described that are useful in the delivery of FBPase inhibitors to hepatocytes. These proesters contain a protected thioethyl moiety as shown in formula E. One or more of the oxygens of the phosphonate can be esterified. Since the mechanism that results in de-esterification requires the generation of a free thiolate, a variety of thiol protecting groups are possible. For example, the disulfide is reduced by a reductase-mediated process (Puech et al., Antiviral Res., 22: 155-174 (1993)). Thioesters will also generate free thiolates after esterase-mediated hydrolysis. Benzaria, et al., J. Med. Chem., 39:4958 (1996). Cyclic analogs are also possible and were shown to liberate phosphonate in isolated rat hepatocytes. The cyclic disulfide shown below has not been previously described and is novel. 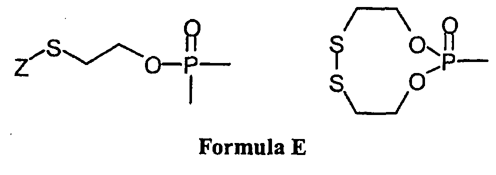 wherein Z is alkylcarbonyl, alkoxycarbonyl, arylcarbonyl, aryloxycarbonyl, or alkylthio.
   Other examples of suitable prodrugs include proester classes exemplified by Biller and Magnin (U.S. Patent No. 5,157,027); Serafinowska et al. (J. Med. Chem. 38, 1372 (1995)); Starrett et al. (J. Med. Chem. 37, 1857 (1994)); Martin et al. J. Pharm. Sci. 76, 180 (1987); Alexander et al., Collect. Czech. Chem. Commun, 59, 1853 (1994)); and EPO patent application 0 632 048 A1. Some of the structural classes described are optionally substituted, including fused lactones attached at the omega position (formulae E-1 and E-2) and optionally substituted 2-oxo-1,3.-dioxolenes attached through a methylene to the phosphorus oxygen (formula E-3) such as: 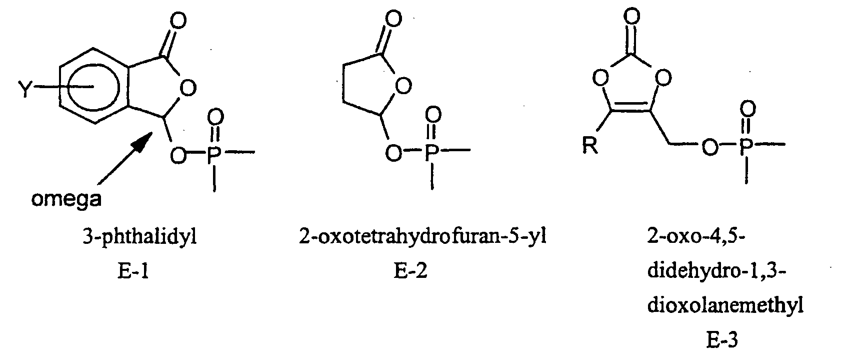 wherein R is -H, alkyl, cycloalkyl, or alicyclic; and
   wherein Y is -H, alkyl, aryl, alkylaryl, cyano, alkoxy, acyloxy, halogen, amino, alicyclic, and alkoxycarbonyl.
   The prodrugs of Formula E-3 are an example of "optionally substituted alicyclic
   where the cyclic moiety contains a carbonate or thiocarbonate."
[7] Propyl phosphonate proesters can also be used to deliver FBPase inhibitors into hepatocytes. These proesters may contain a hydroxyl and hydroxyl group derivatives at the 3-position of the propyl group as shown in formula F. The R and X groups can form a cyclic ring system as shown in formula F. One or more of the oxygens of the phosphonate can be esterified. 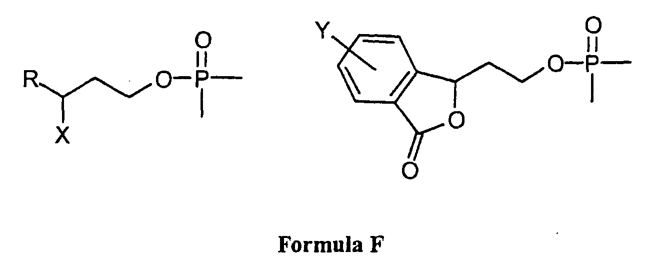 wherein R is alkyl, aryl, heteroaryl;
   X is hydrogen, alkylcarbonyloxy, alkyloxycarbonyloxy; and
   Y is alkyl, aryl, heteroaryl, alkoxy, alkylamino, alkylthio, halogen, hydrogen, hydroxy, acyloxy, amino.
[8] Phosphoramidate derivatives have been explored as phosphate prodrugs (e.g. McGuigan et al., *J. Med. Chem*., **1999**, *42*: 393 and references cited therein) and phosphonate prodrugs (Bischofberger, *et al*., U.S. 5,798,340 and references cited therein) as shown in Formulae G and H.

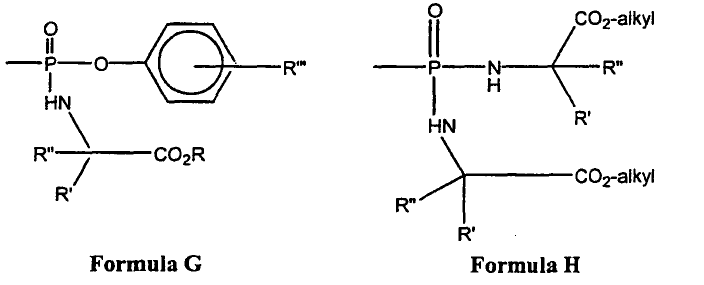

Cyclic phosphoramidates have also been studied as phosphonate prodrugs because of their speculated higher stability compared to non-cyclic phosphoramidates (e.g. Starrett et al., *J. Med. Chem*., **1994**, *37*: 1857).

Another type of nucleotide prodrug was reported as the combination of S-acyl-2-thioethyl ester and phosphoramidate (Egron et al., *Nucleosides & Nucleotides*, **1999**, *18*, 981) as shown in Formula J.

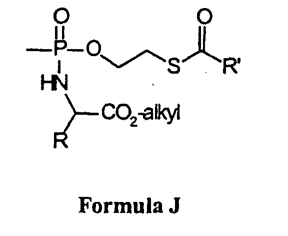

Other prodrugs are possible based on literature reports such as substituted ethyls for example, bis(trichloroethyl)esters as disclosed by McGuigan, et al. Bioorg Med. Chem. Lett., 3:1207-1210 (1993), and the phenyl and benzyl combined nucleotide esters reported by Meier, C. et al. Bioorg. Med. Chem. Lett., 7:99-104 (1997).

The structure 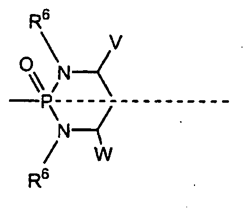 has a plane of symmetry running through the phosphorus-oxygen double bond when R⁶=R⁶, V=W, W'=H, and V and W are either both pointing up or both pointing down. The same is true of structures where each -NR⁶ is replaced with -O-.

The term "cyclic 1',3'-propane ester", "cyclic 1,3-propane ester", "cyclic 1',3'-propanyl ester", and "cyclic 1,3-propanyl ester" refers to the following:

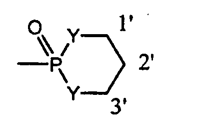

The phrase "together V and Z are connected via an additional 3-5 atoms to form a cyclic group containing 5-7 atoms, optionally containing 1 heteroatom, substituted with hydroxy, acyloxy, alkoxycarbonyloxy, or aryloxycarbonyloxy attached to a carbon atom that is three atoms from both Y groups attached to the phosphorus" includes the following:

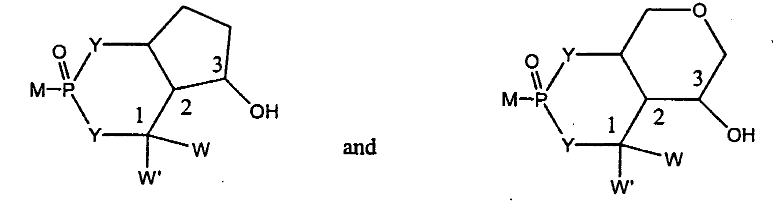

The structure shown above (left) has an additional 3 carbon atoms that forms a five member cyclic group. Such cyclic groups must possess the listed substitution to be oxidized.

The phrase "together V and Z are connected via an additional 3-5 atoms to form a cyclic group, optionally containing one heteroatom, that is fused to an aryl group attached at the beta and gamma position to the Y attached to the phosphorus" includes the following:

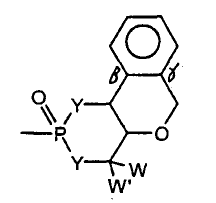

The phrase "together V and W are connected via an additional 3 carbon atoms to form an optionally substituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said additional carbon atoms that is three atoms from a Y attached to the phosphorus" includes the following:

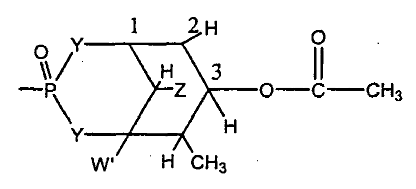

The structure above has an acyloxy substituent that is three carbon atoms from a Y, and an optional substituent, -CH₃, on the new 6-membered ring. There has to be at least one hydrogen at each of the following positions: the carbon attached to Z; both carbons alpha to the carbon labelled "3"; and the carbon attached to "OC(O)CH₃" above.

The phrase "together W and W' are connected via an additional 2-5 atoms to form a cyclic group, optionally containing 0-2 heteroatoms, and V must be aryl, substituted aryl, heteroaryl, or substituted heteroaryl" includes the following:

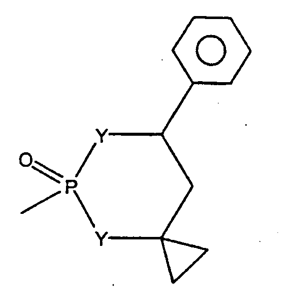

The structure above has V=aryl, and a spiro-fused cyclopropyl group for W and W'.

The term "cyclic phosph(oramid)ate" refers to 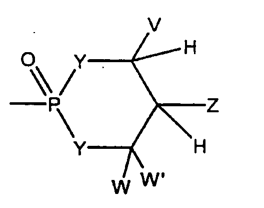 where Y is independently -O- or -NR⁶-. The carbon attached to V must have a C-H bond. The carbon attached to Z must also have a C-H bond.

The term "enhancing" refers to increasing or improving a specific property.

The term "enhanced oral bioavailability" refers to an increase of at least 50% of the absorption of the dose of the parent drug or prodrug(not of this invention) from the gastrointestinal tract. More preferably it is at least 100%. Measurement of oral bioavailability usually refers to measurements of the prodrug, drug, or drug metabolite in blood, tissues, or urine following oral administration compared to measurements following systemic administration.

The term "parent drug" refers to any compound which delivers the same biologically active compound. The parent drug form is M-X-P(O)(OH)₂ and standard prodrugs, such as esters.

The term "drug metabolite" refers to any compound produced *in vivo* or *in vitro* from the parent drug, which can include the biologically active drug.

The term "pharmacodynamic half-life" refers to the time after administration of the drug or prodrug to observe a diminution of one half of the measured pharmacological response. Pharmacodynamic half-life is enhanced when the half-life is increased by preferably at least 50%.

The term "pharmacokinetic half-life" refers to the time after administration of the drug or prodrug to observe a dimunition of one half of the drug concentration in plasma or tissues.

The term "therapeutic index" refers to the ratio of the dose of a drug or prodrug that produces a therapeutically beneficial response relative to the dose that produces an undesired response such as death, an elevation of markers that are indicative of toxicity, and/or pharmacological side effects.

The term "biologically active drug or agent" refers to the chemical entity that produces a biological effect. Thus, active drugs or agents include compounds which as M-X-P(O)(OH)₂ are biologically active.

The term "therapeutically effective amount" refers to an amount that has any beneficial effect in treating a disease or condition.

FBPase inhibitors used in the invention are compounds that inhibit human FBPase activity (Example A), inhibit glucose production from hepatocytes (Examples C and D), lower glucose levels in fasted animals (Examples E-F), and decrease blood glucose levels in diabetic animal models (Examples N-T).

Insulin sensitizers used in this invention are compounds that alter the body's response to endogenous or exogeneous insulin or insulin-like molecules. This reponse can include an improvement in whole-body glucose disposal, a reduction in hepatic glucose output, an increase in insulin-mediated glycogenesis, and other manifestations of improved peripheral insulin resistance. In some instances, the insulin sensitizers used in this invention may also lower circulating triglycerides and/or free fatty acids, may increase HDL cholesterol levels, may reduce hyperinsulinemia, or may improve the pancreatic insulin secretory response. Examples of insulin sensitizers include compounds that activate or are agonists of the PPARγ receptor, are ligands of RXR that activate transcriptional activity of the RXR:PPAR γ heterodimer, or are compounds that achieve enhanced insulin sensitivity through modulation of enzyme activities in cell signalling pathways associated with insulin receptor activation. Enzymes in the latter pathways include protein kinase C, tyrosine phosphatase, PI-3-kinase, MAP kinase, and others. The insulin sensitizers used in this invention have affinity for PPARγ1, PPARγ2, and/or other isoforms of the PPARγ family, and contain either a thiazolidinedione ring structure, a modified thiazolidinedione ring structure, or have a structure that is unrelated to the thiazolidinediones (eg. the 3-aryl-2-alkoxy propanoic acids). The insulin sensitizers also include compounds with affinity for RXRα, RXRβ, RXRγ and/or other RXR receptor isoforms, and are either retinoids such as 9-cis-retinoic acid and its analogs, rexinoids such as (tetramethyltetrahydronaphthyl)carbonylbenzoic acid analogs, or are of other structural classes. Insulin sensitizers used in this invention typically exhibit activity in assays known to be useful for characterizing compounds that act as insulin sensitizers. The assays include but are not limited to: (a) PPARγ binding assays; (b) RXR or RXR-PPARγ activation assays (eg. co-transfection or cis-trans assays); (c) insulin signaling assays such as those measuring receptor or signaling protein phosphorylation/expression (d) adipocyte binding assays; (e) glucose uptake assays in adipocytes or L6 myocytes; (f) adipocyte differentiation assays using triglyceride accumulation, glucose oxidation, or fat/carbohydrate metabolism gene expression as indeces; (g) insulin secretion assays in beta cell islets or the perfused pancreas; (h) pancreatic islet histology assays; (i) in vivo glucose disposal assays; (j) whole body insulin sensitivity assays using the in vivo hyperinsulinemic-glucose clamp technique; (k) hepatic glucose output assays utilizing labeling or NMR techniques; and (l) antihyperglycemic and/or triglyceride/free fatty acid lowering activity in animal models of diabetes such as the KK, ob/ob, or db/db mouse or the ZDF rat.

### Detailed Description Of The Invention

The instant invention is a combination therapy and a composition for the treatment for diabetes and diseases responding to increased glycemic control or to decreased insulin levels. The combination therapy consists of administering one or more FBPase inhibitors and one or more agents known to enhance insulin action, i.e. an insulin sensitizer agent. Known insulin sensitizers include thiazolidinediones, PPAR γ agonists, RXR ligands and inhibitors of the RAX system or angiotensin II action. The therapy is useful for treating diseases characterized by hyperglycemia, impaired glucose tolerance or insulin resistance. Such diseases include diabetes, obesity, hypertension, impaired glucose tolerance and polycystic ovarian syndrome, pancreatitis and renal disease.

In some cases, the combined therapy provides a method for improved glycemic control. The combined therapy will provide improved therapy for one or more of these conditions relative to either agent alone. The combined therapy provides a method for improved glycemic control in NIDDM subjects beyond that achievable by either agent alone. The combined therapy can result in decreases in hepatic glucose output beyond that observed for glucose-lowering doses of the insulin sensitizer agent. Furthermore, the combined therapy can result in improvements in insulin resistance and/or insulin secretion beyond that observed for FBPase inhibitors. In other cases, combining an insulin sensitizer with an FBPase inhibitor or visa versa has an insignificant effect on glycemia but instead results in an improved therapy by minimizing potential adverse pharmacologies sometimes associated with FBPase and insulin sensitizer therapies. For example, FBPase therapy may be associated with elevations of lactate, tryglicerides, free fatty acids or potential side-effects resulting from the renal clearance of the inhibitor. Insulin sensitizer therapy is known to be associated with weight gain, elevation of liver enzymes, and reductions in heamatocrit. In still another aspect of the invention is that the combination therapy achieves similar benefits as observed with one or the other therapies alone but at significantly lower doses. The lower doses improve or eliminate side effects and/or toxicologies associated with the individual drug treatment. The combined therapy entails administration to the host the agents either separately or simultaneously.

Most preferred would be the administration of both agents simultaneously in either the same capsule or as separate pills. Another preferred embodiment is the administration of both agents during meal time (just prior to feeding or just after feeding). Another preferred embodiment is the administration of the insulin sensitizer during meal time and the FBPase inhibitor during times of fasting such as at bed time.

The compounds of the invention may be administered for therapy by any suitable route including, oral, rectal, nasal, topical, vaginal, parenteral (including subcutaneous, intramuscular, intravenous and intradermal) and transdermal. The preferred route is oral.

The present invention relates to methods and compositions for treating a host having NIDDM or a condition associated with insulin resistance by administering to the host a composition containing a pharmaceutically effective amount of an agent that enhances insulin sensitivity and a pharmaceutically effective amount of an FBPase inhibitor. The compositions of this invention are adapted to cure, improve or prevent one or more symptoms of NIDDM. A preferred drug combination will have high potency and low toxicity as can be determined by standard pharmaceutical procedures in cell cultures or experimental animal models, e.g. by determining the LD50 and the ED50.

Preferred FBPase inhibitors encompassed by the instant invention are compounds that inhibit enzyme activity as determined by conducting *in vitro* inhibition studies (Examples A - D). In some cases, *in vivo* metabolic activation of a compound may be required to generate the FBPase inhibitor. This class of compounds may be inactive in the enzyme inhibition screen (Example A), may or may not be active in hepatocytes (Examples C and D), but is active *in vivo* as evidenced by glucose lowering in the normal, fasted rat (Examples E and F) and/or in animal models of diabetes (Examples N-T).

Although the present invention is not limited to the following structures, the FBPase inhibitors generally are of the following formulae:

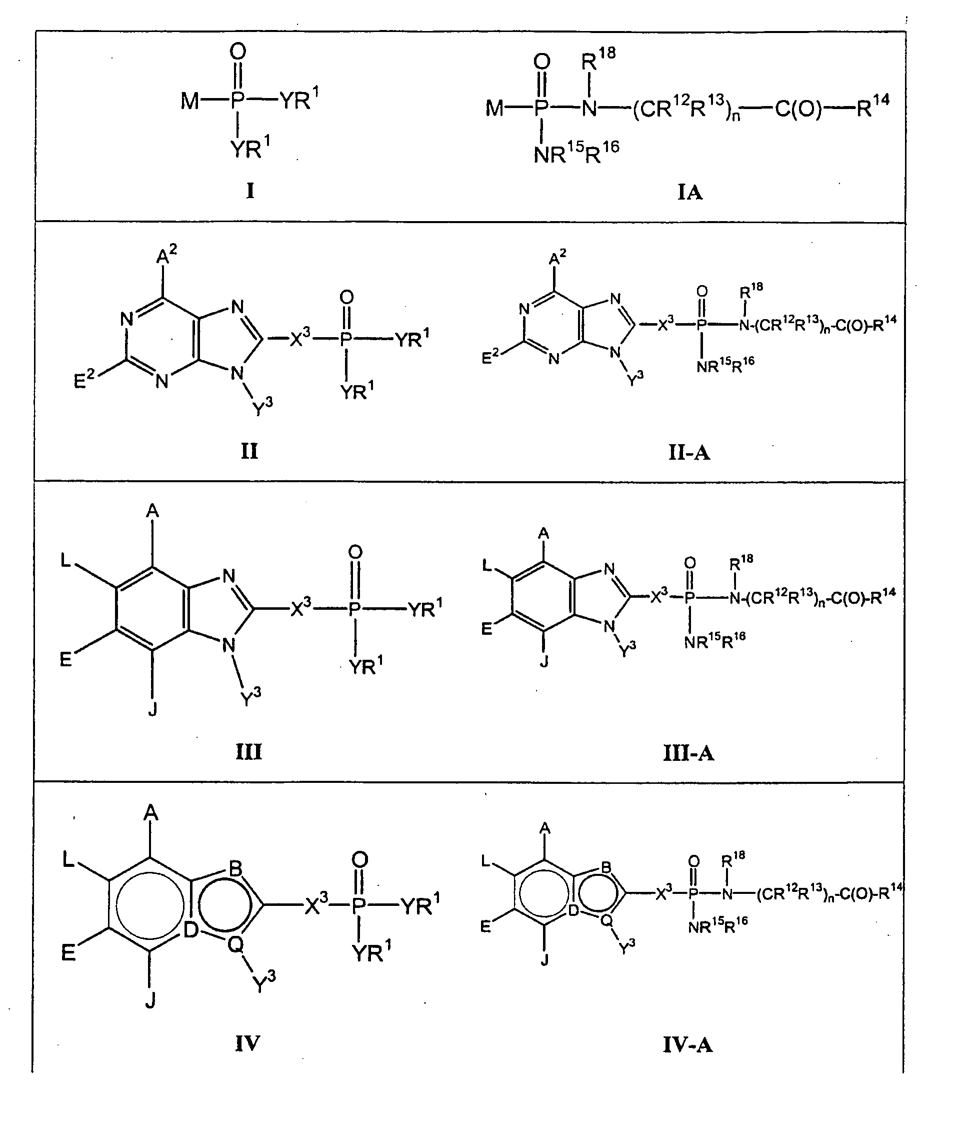

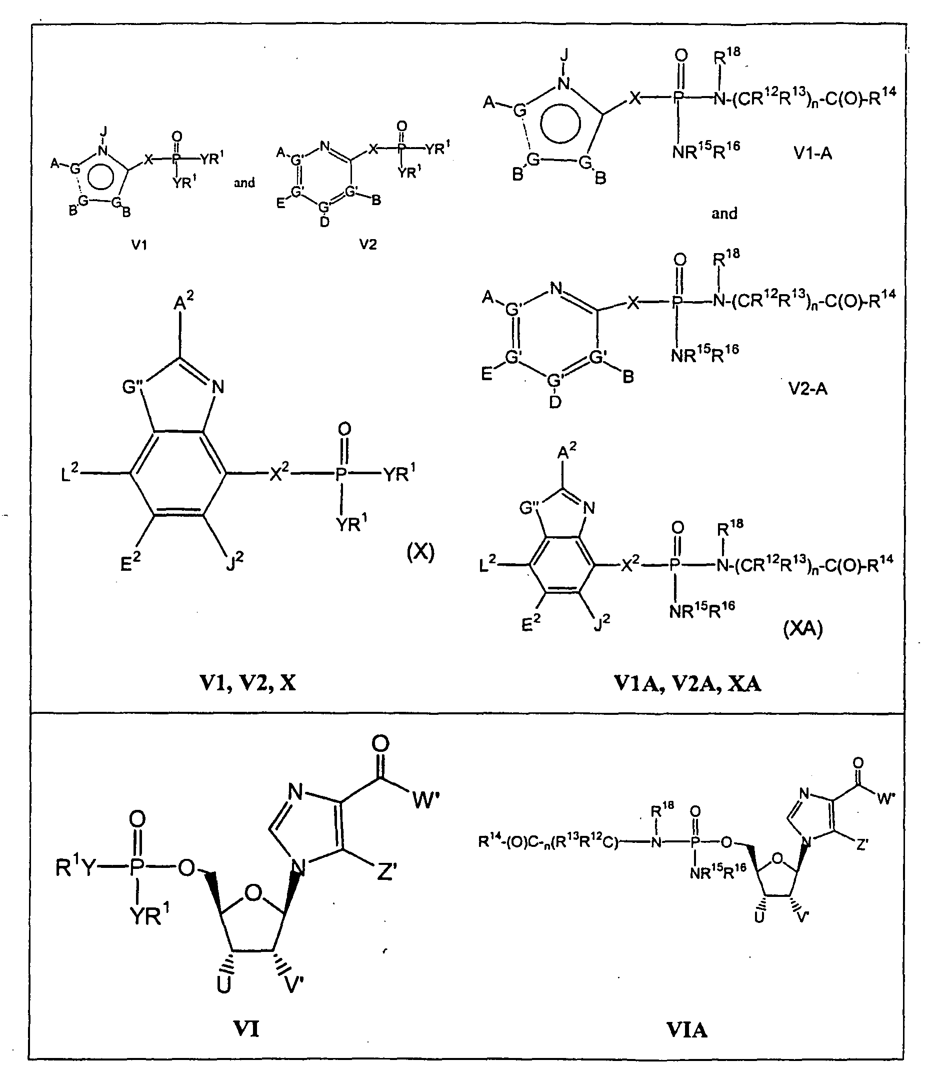

### Preferred Compounds

Suitable alkyl groups include groups having from 1 to about 20 carbon atoms. Suitable aryl groups include groups having from 1 to about 20 carbon atoms. Suitable aralkyl groups include groups having from 2 to about 21 carbon atoms. Suitable acyloxy groups include groups having from 1 to about 20 carbon atoms. Suitable alkylene groups include groups having from 1 to about 20 carbon atoms. Suitable alicyclic groups include groups having 3 to about 20 carbon atoms. Suitable heteroaryl groups include groups having from 1 to about 20 carbon atoms and from 1 to 4 heteroatoms, preferably independently selected from nitrogen, oxygen, phosphorous, and sulfur. Suitable heteroalicyclic groups include groups having from 2 to about twenty carbon atoms and from 1 to 5 heteroatoms, preferably independently selected from nitrogen, oxygen, phosphorous, and sulfur.

Preferred are the following compounds of formula IA 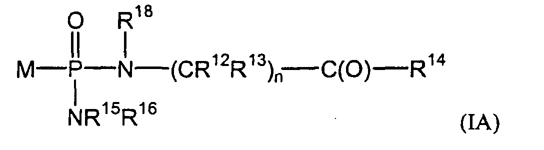 wherein *in vivo* or *in vitro* compounds of formula IA are converted to M-PO₃²⁻ which is an inhibitor of fructose -1, 6-bisphosphatase and
n is an integer from 1 to 3;
R¹⁸ is independently selected from the group consisting ofH, lower alkyl, aryl, aralkyl, or together with R¹² is connected via 1-4 carbon atoms to form a cyclic group;
each R¹² and R¹³ is independently selected from the group consisting of H, lower alkyl, lower aryl, lower aralkyl, all optionally substituted, or R¹² and R¹³ together are connected via 2-6 carbon atoms to form a cyclic group;
each R¹⁴ is independently selected from the group consisting of-OR¹⁷, -N(R¹⁷)₂, -NHR¹⁷, and -SR¹⁷;
R¹⁵ is selected from the group consisting of -H, lower alkyl, lower aryl, lower arakyl, or together with R¹⁶ is connected via 2-6 atoms, optionally including 1 heteroatom selected from the group consisting of O, N, and S;
R¹⁶ is selected from the group consisting of -(CR¹²R¹³)ₙ-C(O)-R¹⁴, lower alkyl, lower aryl, lower aralkyl, or together with R¹⁵ is connected via 2-6 atoms, optionally including 1 heteroatom selected from the group consisting of O, N, and S;
each R¹⁷ is independently selected from the group consisting of lower alkyl, lower aryl, and lower aralkyl, or together R¹⁷ and R¹⁷ on N is connected via 2-6 atoms, optionally including 1 heteroatom selected from the group consisting of O, N, and S;
and pharmaceutically acceptable salts thereof.

More preferred are FBPase inhibitors where M-PO₃²⁻ has an IC₅₀ on isolated human FBPase enzyme of less than or equal to 5µM. Especially preferred are such compounds that bind to the AMP site of FBPase.

In one aspect, preferred are compounds of formula IA or formula I wherein M is R⁵-X-
wherein
R⁵ is selected from the group consisting of: 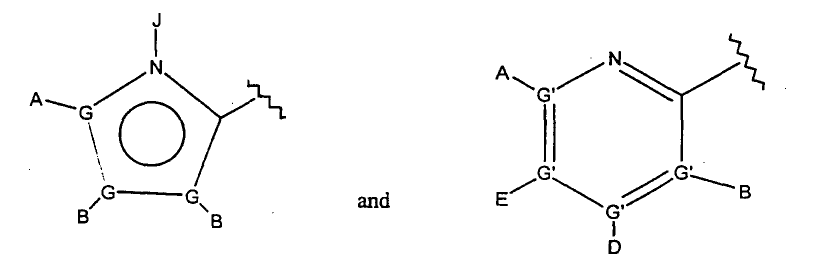 wherein:
each G is independently selected from the group consisting of C, N, O, S, and Se, and wherein only one G may be O, S, or Se, and at most one G is N;
each G' is independently selected from the group consisting of C and N and wherein no more than two G' groups are N;
A is selected from the group consisting of -H, -NR⁴₂, -CONR⁴₂, -CO₂R³, halo, -S(O)R³, -SO₂R³, alkyl, alkenyl, alkynyl, perhaloalkyl, haloalkyl, aryl, -CH₂OH, -CH₂NR⁴₂, -CH₂CN, -CN, -C(S)NH₂, -OR³, -SR³, -N₃, -NHC(S)NR⁴₂, -NHAc, and null;
each B and D are independently selected from the group consisting of -H, alkyl, alkenyl, alkynyl, aryl, alicyclic, aralkyl, alkoxyalkyl, -C(O)R¹¹, -C(O)SR³, -SO₂R¹¹, -S(O)R³, -CN, -NR⁹₂, -OR³, -SR³, perhaloalkyl, halo, -NO₂, and null, all except -H, -CN, perhaloalkyl, -NO₂, and halo are optionally substituted;
E is selected from the group consisting of -H, alkyl, alkenyl, alkynyl, aryl, alicyclic, alkoxyalkyl, -C(O)OR³, -CONR⁴₂, -CN, -NR⁹₂, -NO₂, -OR³, -SR³, perhaloalkyl, halo, and null, all except -H, -CN, perhaloalkyl, and halo are optionally substituted;
J is selected from the group consisting of -H and null;
X is an optionally substituted linking group that links R⁵ to the phosphorus atom via 2-4 atoms, including 0-1 heteroatoms selected from N, O, and S, except that if X is urea or carbamate there is 2 heteroatoms, measured by the shortest path between R⁵ and the phosphorus atom, and wherein the atom attached to the phosphorus is a carbon atom, and wherein X is selected from the group consisting of -alkyl(hydroxy)-, -alkynyl-, -heteroaryl-, -carbonylalkyl-, -1,1-dihaloalkyl-, -alkoxyalkyl-, -alkyloxy-, -alkylthioalkyl-, -alkylthio-, -alkylaminocarbonyl-, -alkylcarbonylamino-, -alkoxycarbonyl-, -carbonyloxyalkyl-, -alkoxycarbonylamino-, and -alkylaminocarbonylamino-, all optionally substituted; with the proviso that X is not substituted with -COOR², -SO₃H, or -PO₃R²₂;
R² is selected from the group consisting of R³ and -H;
R³ is selected from the group consisting of alkyl, aryl, alicyclic, and aralkyl;
each R⁴ is independently selected from the group consisting of -H, and alkyl, or together R⁴ and R⁴ form a cyclic alkyl group;
each R⁹ is independently selected from the group consisting of -H, alkyl, aralkyl, and alicyclic, or together R⁹ and R⁹ form a cyclic alkyl group;
R¹¹ is selected from the group consisting of alkyl, aryl, -NR²₂, and -OR²;
and with the proviso that:
   1) when G' is N, then the respective A, B, D, or E is null;
   2) at least one of A and B, or A, B, D, and E is not selected from the group consisting of -H or null;
   3) when R⁵ is a six-membered ring, then X is not any 2 atom linker, an optionally substituted -alkyloxy-, or an optionally substituted -alkylthio-;
   4) when G is N, then the respective A or B is not halogen or a group directly bonded to G via a heteroatom;
   5) when X is not a -heteroaryl- group, then R⁵ is not substituted with two or more aryl groups;
   and pharmaceutically acceptable prodrugs and salts thereof.

More preferred are such compounds wherein when R⁵ is 2-thiazolyl, 2-oxazolyl, or 2-selenazolyl, and X is -alkoxyalkyl-, -alkylthioalkyl-, -alkyloxy-, or -alkylthio-, then A is not -CONH₂ and B is not -H. Also more preferred are such compounds wherein when R⁵ is 2-thiazolyl, 2-oxazolyi, or 2-selenazolyl, then X is not -alkyloxyalkyl-, -alkylthioalkyl-, -alkyloxy-, or -alkylthio-.

Preferably, compounds of formula IA have an IC₅₀ of ≤ 50 µ M on glucose production in isolated rat hepatocytes.

More preferred R⁵ groups include pyrrolyl, imidazolyl, oxazolyl, thiazolyl, isothiazolyl, 1,2,4-thiadiazolyl, pyrazolyl, isoxazolyl, 1,2,3-oxadiazolyl, 1,2,4-oxadiazolyl, 1,2,5-oxadiazolyl, 1,3,4-oxadiazolyl, 1,2,4-thiadiazolyl, 1,3,4-thiadiazolyl, pyridinyl, pyrimidinyl, pyrazinyl, pyridazinyl, 1,3,5-triazinyl, 1,2,4-triazinyl, and 1,3-selenazolyl, all of which contain at least one substituent. Preferably, R⁵ is not 2-thiazolyl, or 2-oxazolyl.

In one aspect, preferred R⁵ groups are substituted with the following groups:
A is selected from the group consisting of -H, -NR⁴₂, -CONR⁴₂, -CO₂R³, halo, C1-C6 alkyl, C2-C6 alkenyl, C2-C6 alkynyl, C1-C6 perhaloalkyl, C1-C6 haloalkyl, aryl, -CH₂OH, -CH₂NR⁴₂, -CH₂CN, -CN, -C(S)NH₂, -OR⁴, -SR⁴, -N₃, -NHC(S)NR⁴₂, -NHAc, and null;
each B and D are independently selected from the group consisting of-H, alkyl, alkenyl, alkynyl, aryl, alicyclic, aralkyl, alkoxyalkyl, -C(O)R¹¹, -C(O)SR³, -SO₂R¹¹, -S(O)R³, -CN, -NR²₂, -OR³, -SR³, perhaloalkyl, halo, and null, all except -H, -CN, perhaloalkyl, and halo are optionally substituted;
E is selected from the group consisting of -H, C1-C6 alkyl, C2-C6 alkenyl, C2-C6 alkynyl, aryl, C4-C6 alicyclic, alkoxyalkyl, -C(O)OR³, -CONR⁴₂, -CN, -NR⁹₂, -OR³, -SR³, C1-C6 perhaloalkyl, halo, and null, all except -H, -CN, perhaloalkyl, and halo are optionally substituted; and
each R⁴ is independently selected from the group consisting of -H, and C1-C2 alkyl.

In another aspect, preferred are compounds wherein R⁵ is:

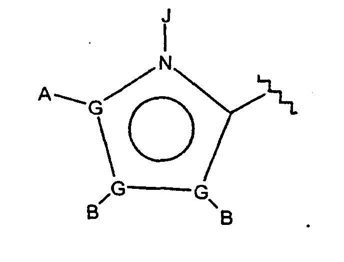

In another aspect, preferred are compounds wherein R⁵ is:

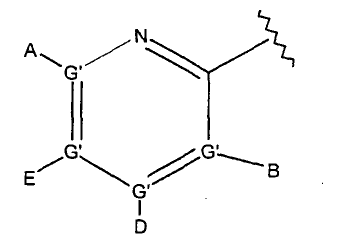

More preferred are compounds wherein R⁵ is selected from the group consisting of: 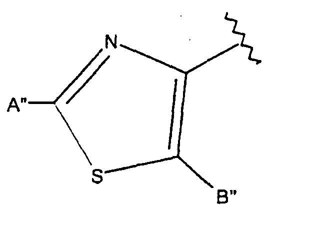 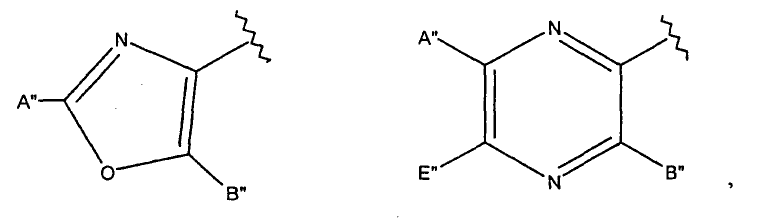 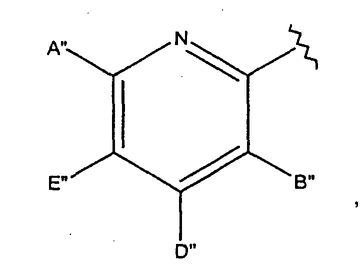 and 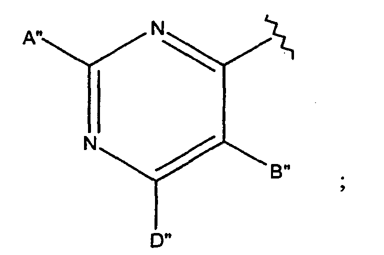 wherein
A" is selected from the group consisting of -H, -NR⁴₂, -CONR⁴₂, -CO₂R³, halo, C1-C6 alkyl, C2-C6 alkenyl, C2-C6 alkynyl, C1-C6 perhaloalkyl, C1-C6 haloalkyl, aryl, -CH₂OH, -CH₂NR⁴₂, -CH₂CN, -CN, -C(S)NH₂, -OR³, -SR³, -N₃, -NHC(S)NR⁴₂, and -NHAc;
B" and D" are independently selected from the group consisting of -H, alkyl, alkenyl, alkynyl, aryl, alicyclic, aralkyl, alkoxyalkyl, -C(O)R¹¹, -C(O)SR³, -SO₂R¹¹, -S(O)R³, -CN, -NR⁹₂, -OR³, -SR³, perhaloalkyl, and halo, all except -H, -CN, perhaloalkyl, and halo are optionally substituted;
E" is selected from the group consisting of -H, C1-C6 alkyl, C2-C6 alkenyl, C2-C6 alkynyl, C4-C6 alicyclic, alkoxyalkyl, -C(O)OR³, -CONR⁴₂, -CN, -NR⁹₂, -OR³, -SR³, C1-C6 perhaloalkyl, and halo, all except H, -CN, perhaloalkyl, and halo are optionally substituted; and
each R⁴ is independently selected from the group consisting of -H and C1-C2 alkyl.

Especially preferred are those compounds of formula V-1A and formula V-2A
wherein
A" is selected from the group consisting of -NH₂, -CONH₂, halo, -CH₃, -CF₃, - CH₂-halo, -CN, -OCH₃, -SCH₃, and -H;
B" is selected from the group consisting of -H, -C(O)R¹¹, -C(O)SR³, alkyl, aryl, alicyclic, halo, -CN, -SR³, OR³ and -NR⁹₂;
D" is selected from the group consisting of -H, -C(O)R¹¹, -C(O)SR³, -NR⁹₂, alkyl, aryl, alicyclic, halo, and -SR³;
E" is selected from the group consisting of -H, C1-C6 alkyl, lower alicyclic, halo, -CN, -C(O)OR³, and -SR³.
X is selected from the group consisting of -heteroaryl-, -alkoxycarbonyl-, and -alkylaminocarbonyl-, all optionally substituted;
R¹⁸ and R¹⁵ are selected from the group consisting of H, and methyl;
R² is selected from the group consisting of R³ and -H;
R³ is selected from the group consisting of alkyl, aryl, alicyclic, and aralkyl;
each R¹² and R¹³ is independently selected from the group consisting of -H, methyl, i-propyl, i-butyl, and benzyl, or together R¹² and R¹³ are connected via 2-5 carbon atoms to form a cycloalkyl group;
n is 1;
R¹⁴ is -OR¹⁷;
R¹⁶ is -(CR¹²R¹³)ₙ-C(O)-R¹⁴; and
R¹⁷ is selected from the group consisting of methyl, ethyl, propyl, phenyl, and benzyl. Most preferred are such compounds with 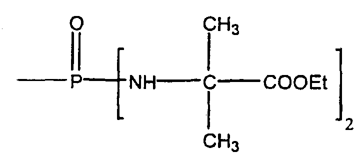 and 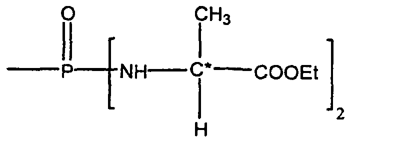
wherein C* has S stereochemistry.

Also particularly preferred are such compounds wherein R⁵ is selected from the group consisting of:

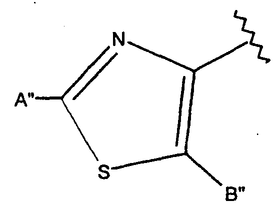

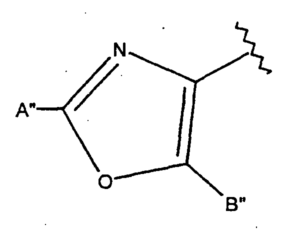

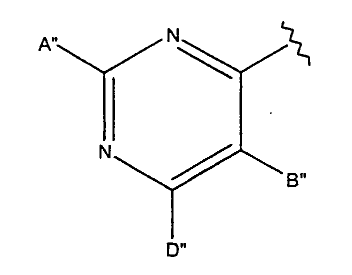

Also particularly preferred are such compounds wherein R⁵ is selected from the group consisting of:

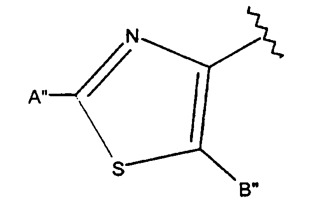

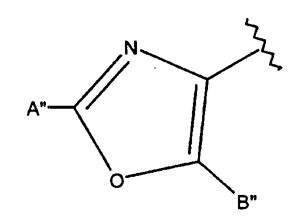

Also particularly preferred are such compounds wherein R⁵ is selected from the group consisting of: 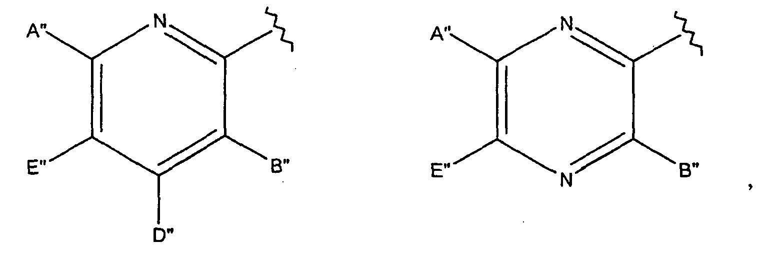 and

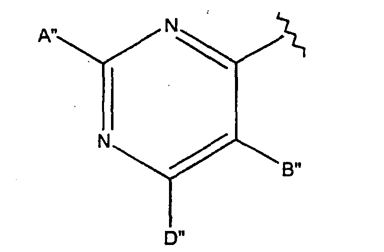

In one especially preferred aspect, R⁵ is 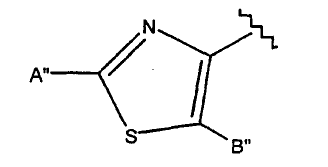 X is selected from the group consisting of methylenoxycarbonyl, and furan-2,5-diyl, and pharmaceutically acceptable salts and prodrugs thereof. More preferred are such compounds wherein A" is -NH2, X is furan-2,5-diyl, and B" is -S(CH₂)₂CH₃; wherein A" is -NH₂, X is furan-2,5-diyl, and B" is -CH₂-CH(CH₃)₂; wherein A" is -NH₂, X is furan-2,5-diyl, and B" is -COOEt; wherein A" is -NH₂, X is furan-2,5-diyl, and B" is -SMe; or wherein A" is -NH₂, X is methyleneoxycarbonyl, and B" is -CH(CH₃)₂.

Most preferred are such thiazoles where A" is -NH₂, X is furan-2,5-diyl, B" is - S(CH₂)₂CH₃ and wherein 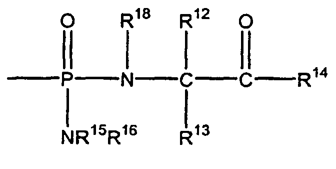 is 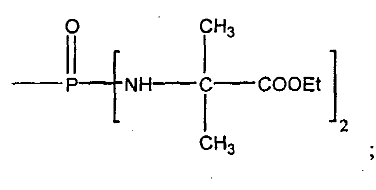 or
wherein 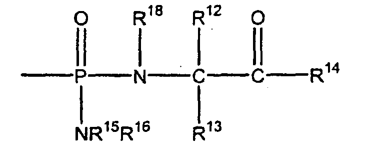 is 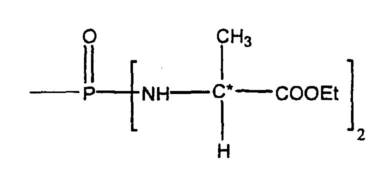 wherein C* has S stereochemistry.
Also most preferred are such thiazoles where A" is -NH₂, X is furan-2,5-diyl, B" is -CH₂-CH(CH₃)₂, and wherein 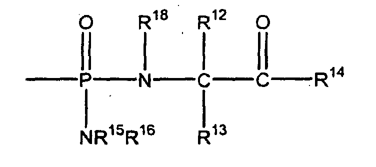 is 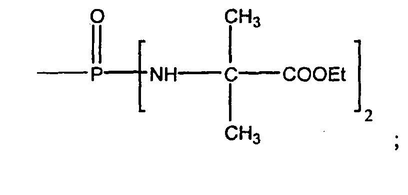 or 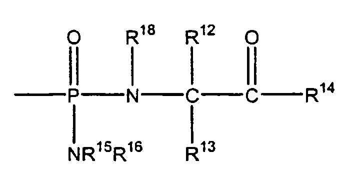 is 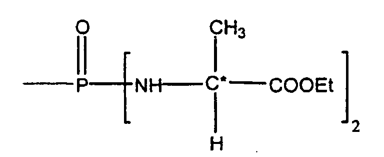 wherein C* has S stereochemistry.

In another preferred aspect, R⁵ is

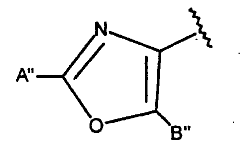

X is selected from the group consisting of furan-2,5-diyl, and methyleneoxycarbonyl, A" is -NH₂, and pharmaceutically acceptable salts and prodrugs thereof. More preferred are such compounds wherein X is furan -2,5-diyl, and B" is -SCH₂CH₂CH₃.

In another preferred aspect, R⁵ is

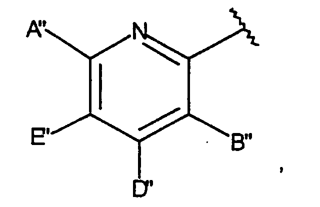

A" is -NH₂, E" and D" are -H, B" is selected from the group consisting of cyclopropyl, and n-propyl, X is selected from the group consisting of methyleneoxycarbonyl, and furan-2,5-diyl, and pharmaceutically acceptable salts and prodrugs thereof.

In another preferred aspect, R⁵ is

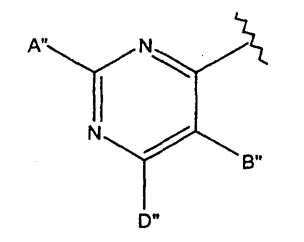

A" is -NH₂, D" is -H, B" is selected from the group consisting of n-propyl, and cyclopropyl, X is selected from the group consisting of furan-2,5-diyl, and methyleneoxycarbonyl, and pharmaceutically acceptable salts and prodrugs thereof.

Preferred groups include -heteroaryl-, -alkylcarbonylamino-, -alkylaminocarbonyl-, and -alkoxycarbonyl-. More preferred is -heteroaryl-, and - alkoxycarbonyl-.

The compounds of formula 1A are preferred.

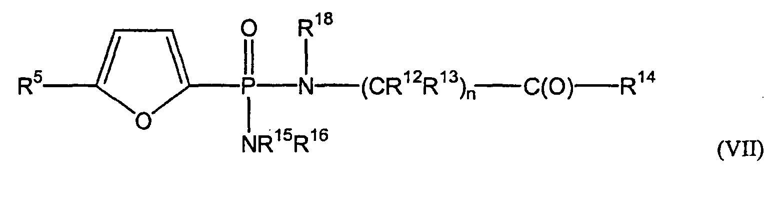

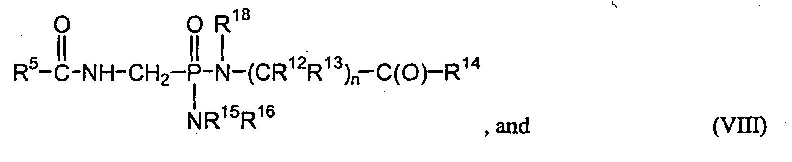

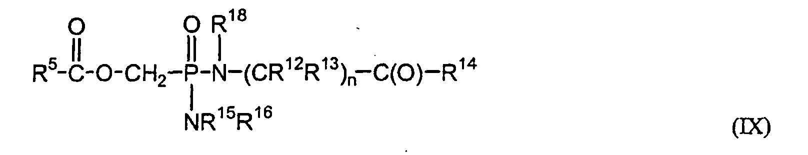

More preferred are compounds of formulae VII or IX:

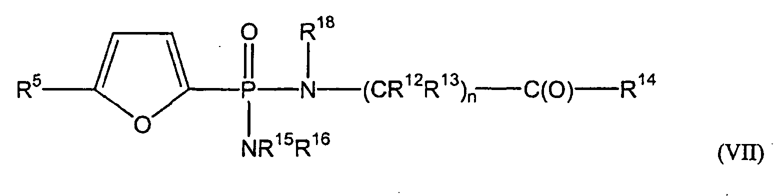

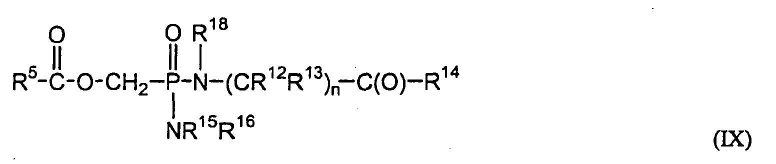

Preferred A" groups include -NH₂, -CONH₂, halo, -CH₃, -CF₃, -CH₂-halo, -CN, -OCH₃, -SCH₃, and -H.

More preferred A" groups include -NH₂, -Cl, -Br, and -CH₃. Preferred B" groups include -H, -C(O)R¹¹, -C(O)SR³, alkyl, aryl, alicyclic, halo, -CN, -SR³, -NR⁹₂, and -OR³. More preferred is -H, -C(O)OR³, -C(O)SR³, C1-C6 alkyl, alicyclic, halo, heteroaryl, and -SR³.

Preferred D" groups include -H, -C(O)R¹¹, -C(O)SR³, alkyl, aryl, alicyclic, halo, - NR⁹₂, and -SR³. More preferred is -H, -C(O)OR³, lower alkyl, alicyclic, and halo.

Preferred E" groups include -H, C1-C6 alkyl, lower alicyclic, halogen, -CN, - C(O)OR³, -SR³, and -CONR⁴₂. More preferred is -H, -Br, and -Cl.

Preferred R¹⁸ groups include -H, methyl, and ethyl. More preferred is -H and methyl. Especially preferred is -H.

Preferred compounds include those wherein each R¹² and R¹³ is independently selected from the group consisting of -H, methyl, ethyl, n-propyl, i-propyl, n-butyl, i-butyl, -CH₂CH₂-SCH₃, phenyl, and benzyl, or together R¹² and R¹³ are connected via 2-5 carbon atoms to form a cycloalkyl group. More preferred is each R¹² and R¹³ is independently selected from the group consisting of -H, methyl, i-propyl, i-butyl, and benzyl, or together R¹² and R¹³ are connected via 2-5 carbon atoms to form a cycloalkyl group. Also more preferred are such compounds wherein each R¹² and R¹³ is independently selected from the group consisting of-H, methyl, i-propyl, and benzyl, or together R¹² and R¹³ are connected via 4 carbon atoms to form a cyclopentyl group. Especially preferred are those compounds wherein R¹² and R¹³ are both -H, both methyl, or R¹² is H and R¹³ is selected from the group consisting of methyl, i-propyl, and benzyl. Most preferred are such compounds wherein n is 1, and R¹² is -H, then the carbon attached to R¹² and R¹³ has S stereochemistry.

Preferably, n is an integer of from 1-2. More preferred is when n is 1.

Preferred compounds include those wherein each R¹⁴ is independently selected from the group consisting of -OR¹⁷, and -SR¹⁷; and R¹⁷ is selected from the group consisting of optionally substituted methyl, ethyl, propyl, t-butyl, and benzyl. More preferred are such compounds wherein each R¹⁴ is independently selected from the group consisting of -OR¹⁷; and R¹⁷ is selected from the group consisting of methyl, ethyl, propyl, and benzyl. Most preferred are such compounds wherein R¹⁷ is selected from the group consisting of ethyl, and benzyl.

Preferred are compounds wherein R¹⁵ is not H. More preferred are compounds wherein R¹⁵ and R¹⁶ are independently selected from the group consisting of lower alkyl, and lower aralkyl, or together R¹⁵ and R¹⁶ are connected via 2-6 atoms, optionally including 1 heteroatom selected from the group consisting of O, N, and S. Also more preferred are compounds wherein R¹⁵ and R¹⁶ are independently selected from the group consisting of C1-C6 alkyl, or together R¹⁵ and R¹⁶ are connected via 2-6 atoms, optionally including 1 heteroatom selected from the group consisting of O, N, and S. In one aspect, particularly preferred are compounds wherein -NR¹⁵R¹⁶ is a cyclic amine. Especially preferred are such compounds wherein -NR¹⁵R¹⁶ is selected from the group consisting of morpholinyl and pyrrolidinyl.

Preferred are compounds where R¹⁶ is -(CR¹²R¹³)ₙ-C(O)-R¹⁴. Particularly preferred are such compounds that are of the formula:

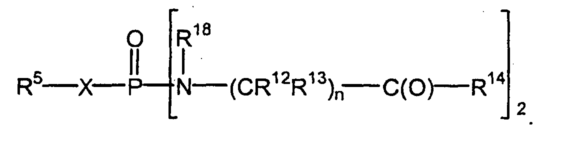

More preferred are such compounds wherein n is 1. Especially preferred are such compounds wherein when R¹² and R¹³ are not the same, then H₂N-CR¹²R¹³-C(O)-R¹⁴ is an ester, or thioester of a naturally occurring amino acid; and R¹⁴ is selected from the group consisting of -OR¹⁷ and -SR¹⁷.

More preferred are compounds where n is1 and wherein
R¹⁸ is selected from the group consisting of-H, methyl, and ethyl;
R¹² and R¹³ are independently selected from the group consisting of-H, methyl, i-propyl, i-butyl, and benzyl, or together are connected via 2-5 carbon atoms to form a cycloalkyl group;
R¹⁴ is OR¹⁷;
R¹⁷ is selected from the group consisting of methyl, ethyl, propyl, t-butyl, and benzyl; and
R¹⁵ and R¹⁶ are independently selected from the group consisting of lower alkyl, and lower aralkyl, or together R¹⁵ and R¹⁶ are connected via 2-6 atoms, optionally including 1 heteroatom selected from the group consisting of O, and N.

In one aspect, preferred are compounds of Formula IA wherein M is 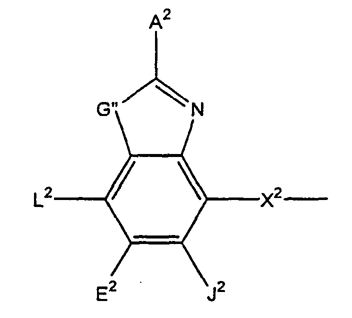 wherein:
G" is selected from the group consisting of -O- and -S-;
A², L², E², and J² are selected from the group consisting of -NR⁴₂, -NO₂, -H, -OR², -SR², -C(O)NR⁴₂, halo, -COR¹¹, -SO₂R³, guanidinyl, amidinyl, aryl, aralkyl, alkyoxyalkyl, -SCN, -NHSO₂R⁹, -SO₂NR⁴₂, -CN, -S(O)R³, perhaloacyl, perhaloalkyl, perhaloalkoxy, C1-C5 alkyl, C2-C5 alkenyl, C2-C5 alkynyl, and lower alicyclic, or together L² and E² or E² and J² form an annulated cyclic group;
X² is selected from the group consisting of -CR²₂-, -CF₂-, - OCR²₂-, -SCR²₂-,
-O-C(O)-, -S-C(O)-, -O-C(S)-; and -NR¹⁹CR²₂-, and wherein in the atom attached to the phosphorus is a carbon atom; with the proviso that X² is not substituted with -COOR², - SO₃H, or -PO₃R²₂;
R² is selected from the group consisting of R³ and -H;
R³ is selected from the group consisting of alkyl, aryl, alicyclic, and aralkyl;
each R⁴ is independently selected from the group consisting of -H, and alkyl, or together R⁴ and R⁴ form a cyclic alkyl group;
each R⁹ is independently selected from the group consisting of -H, alkyl, aralkyl, and alicyclic, or together R⁹ and R⁹ form a cyclic alkyl group;
R¹¹ is selected from the group consisting of alkyl, aryl, -NR²₂, and -OR²;
R¹⁹ is selected from the group consisting of lower alkyl, -H, and -COR²; and
pharmaceutically acceptable prodrugs and salts thereof. More preferred are compounds wherein G" is -S-. Most preferred are compounds wherein A², L², E², and J² are independently selected from the group consisting of -H, -NR⁴₂, -S-C≡N, halogen, -OR³, hydroxy, -alkyl(OH), aryl, alkyloxycarbonyl, -SR³, lower perhaloalkyl, and C1-C5 alkyl, or together L² and E² form an annulated cyclic group. More preferably A², L², E² and J² are independently selected from the group consisting of -H, -NR⁴₂, -S-C≡N, halogen, lower alkoxy, hydroxy, lower alkyl(hydroxy), lower aryl, and C1-C5 alkyl, or together L² and E² form an annulated cyclic group.

Most preferred A² groups include -NH₂, -H, halo, and C1-C5 alkyl.

Most preferred L² and E² groups are those independently selected from the group consisting of -H, -S-C≡N, lower alkoxy, C1-C5 alkyl, lower alkyl(hydroxy), lower aryl, and halogen or together L² and E² form an annulated cyclic group containing an additional 4 carbon atoms.

Most preferred J² groups include -H, and C1-C5 alkyl.

Preferred X² groups include -CF₂-, -CH₂-, -OC(O)- -OCH₂-, -SCH₂-, -NHCH₂-, and -N(C(O)CH₃)-CH₂-. More preferred are -OCH₂-, -SCH₂-, and -N(C(O)CH₃)-CH₂-. Most preferred is -OCH₂-.
One preferred aspect include compound wherein A² is selected from the group consisting of -H, -NH₂, -CH₃, -Cl, and -Br;
L² is -H, lower alkyl, halogen, lower alkyloxy, hydroxy, -alkenylene-OH, or together with E² forms a cyclic group including aryl, cyclic alkyl, heteroaryls, heterocyclic alkyl;
E² is selected from the groups consisting of H, lower alkyl, halogen, SCN, lower alkyloxycarbonyl, lower alkyloxy, or together with L² forms a cyclic group including aryl, cyclic alkyl, heteroaryl, or heterocyclic alkyl;
J² is selected from the groups consisting ofH, halogen, and lower alkyl;
G" is -S-;
X² is -OCH₂-;
and pharmaceutically acceptable salts and prodrugs thereof. More preferred are such compounds wherein
R¹⁸ is selected from the group consisting of-H, methyl, and ethyl;
R¹² and R¹³ are independently selected from the group consisting of-H, methyl, i-propyl, i-butyl, and benzyl, or together are connected via 2-5 carbon atoms to form a cycloalkyl group;
R¹⁴ is -OR¹⁷;
R¹⁷ is selected from the group consisting of methyl, ethyl, propyl, t-butyl, and benzyl; and
R¹⁵ and R¹⁶ are independently selected from the group consisting of lower alkyl, and lower aralkyl, or together R¹⁵ and R¹⁶ are connected via 2-6 atoms, optionally including 1 heteroatom selected from the group consisting of O, and N.

Also more preferred are such compounds where A² is NH₂, L² is selected from the group consisting of-Et and -Cl, E² is selected from the group consisting of -SCN, -Et, and -Br, and J² is -H. Particularly preferred are such compounds wherein 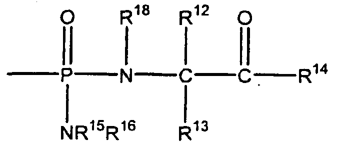 is selected from the group consisting of 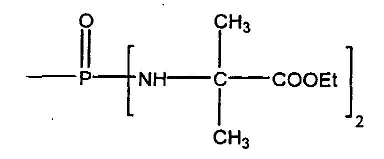 and 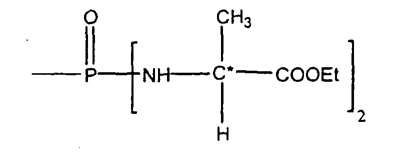 wherein C* has S stereochemistry.

Preferred R¹⁸ groups include -H, methyl, and ethyl. More preferred is -H and methyl. Especially preferred is -H.

Preferred compounds include those wherein each R¹² and R¹³ is independently selected from the group consisting of-H, methyl, ethyl, n-propyl, i-propyl, n-butyl, i-butyl, -CH₂CH₂-SCH₃, phenyl, and benzyl, or together R¹² and R¹³ are connected via 2-5 carbon atoms to form a cycloalkyl group. More preferred is each R¹² and R¹³ is independently selected from the group consisting of-H, methyl, i-propyl, i-butyl, and benzyl, or together R¹² and R¹³ are connected via 2-5 carbon atoms to form a cycloalkyl group. Also more preferred are such compounds wherein each R¹² and R¹³ is independently selected from the group consisting of -H, methyl, i-propyl, and benzyl, or together R¹² and R¹³ are connected via 4 carbon atoms to form a cyclopentyl group. Especially preferred are those compounds wherein R¹² and R¹³ are both -H, both methyl, or R¹² is H and R¹³ is selected from the group consisting of methyl, i-propyl, and benzyl. Most preferred are such compounds wherein n is 1, and R¹² is -H, then the carbon attached to R¹² and R¹³ has S stereochemistry.

Preferably, n is an integer of from 1-2. More preferred is when n is 1.

Preferred compounds include those wherein each R¹⁴ is independently selected from the group consisting of -OR¹⁷, and -SR¹⁷; and R¹⁷ is selected from the group consisting of optionally substituted methyl, ethyl, propyl, t-butyl, and benzyl. More preferred are such compounds wherein each R¹⁴ is independently selected from the group consisting of -OR¹⁷; and R¹⁷ is selected from the group consisting of methyl, ethyl, propyl, and benzyl. Most preferred are such compounds wherein R¹⁷ is selected from the group consisting of ethyl, and benzyl.

Preferred are compounds wherein R¹⁵ is not H. More preferred are compounds wherein R¹⁵ and R¹⁶ are independently selected from the group consisting of lower alkyl, and lower aralkyl, or together R¹⁵ and R¹⁶ are connected via 2-6 atoms, optionally including 1 heteroatom selected from the group consisting of O, N, and S. Also more preferred are compounds wherein R¹⁵ and R¹⁶ are independently selected from the group consisting of C1-C6 alkyl, or together R¹⁵ and R¹⁶ are connected via 2-6 atoms, optionally including 1 heteroatom selected from the group consisting of O, N, and S. In one aspect, particularly preferred are compounds wherein -NR¹⁵R¹⁶ is a cyclic amine. Especially preferred are such compounds wherein -NR¹⁵R¹⁶ is selected from the group consisting of morpholinyl and pyrrolidinyl.

Preferred are compounds R¹⁶ is -(CR¹²R¹³)ₙ-C(O)-R¹⁴.

More preferred are compounds where n is 1, and wherein
R¹⁸ is selected from the group consisting of-H, methyl, and ethyl;
R¹² and R¹³ are independently selected from the group consisting of-H, methyl, i-propyl, i-butyl, and benzyl, or together are connected via 2-5 carbon atoms to form a cycloalkyl group;
R¹⁴ is -OR¹⁷;
R¹⁷ is selected from the group consisting of methyl, ethyl, propyl, t-butyl, and benzyl; and
R¹⁵ and R¹⁶ are independently selected from the group consisting of lower alkyl, and lower aralkyl, or together R¹⁵ and R¹⁶ are connected via 2-6 atoms, optionally including 1 heteroatom selected from the group consisting of O, and N. Particularly preferred are such compounds that are of the formula:

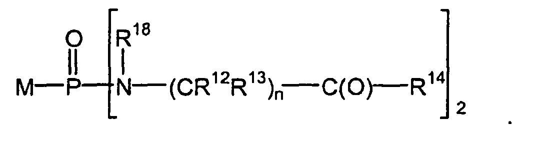

More preferred are such compounds wherein n is 1. Especially preferred are such compounds wherein when R¹² and R¹³ are not the same, then H₂N-CR¹²R¹³-C(O)-R¹⁴ is an ester, or thioester of a naturally occurring amino acid; and R¹⁴ is selected from the group consisting of -OR¹⁷ and -SR¹⁷.

In one aspect, preferred are compounds of formula IA or formula I wherein M is 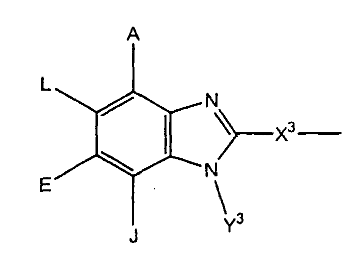 wherein:
A, E, and L are selected from the group consisting of -NR⁸₂, -NO₂, -H, -OR⁷, -SR⁷, -C(O)NR⁴₂, halo, -COR¹¹, -SO₂R³, guanidine, amidine, -NHSO₂R⁵, -SO₂NR⁴₂, -CN, sulfoxide, perhaloacyl, perhaloalkyl, perhaloalkoxy, C1-C5 alkyl, C2-C5 alkenyl, C2-C5 alkynyl, and lower alicyclic, or together A and L form a cyclic group, or together L and E form a cyclic group, or together E and J form a cyclic group including aryl, cyclic alkyl, and heterocyclic;
J is selected from the group consisting of -NR⁸₂, -NO₂, -H, -OR⁷, -SR⁷, -C(O)NR⁴₂, halo, -C(O)R¹¹, -CN, sulfonyl, sulfoxide, perhaloalkyl, hydroxyalkyl, perhaloalkoxy, alkyl, haloalkyl, aminoalkyl, alkenyl, alkynyl, alicyclic, aryl, and aralkyl, or together with Y forms a cyclic group including aryl, cyclic alkyl and heterocyclic alkyl;
X³ is selected from the group consisting of -alkyl(hydroxy)-, -alkyl-, -alkynyl-, -aryl-, -carbonylalkyl-, -1,1-dihaloalkyl-, -alkoxyalkyl-, -alkyloxy-, -alkylthioalkyl-, -alkylthio-, -alkylaminocarbonyl-, -alkylcarbonylamino-, -alicyclic-, -aralkyl-, -alkylaryl-, -alkoxycarbonyl-, -carbonyloxyalkyl-, -alkoxycarbonylamino-, and -alkylaminocarbonylamino-, all optionally substituted; with the proviso that X³ is not substituted with -COOR², -SO₃H, or -PO₃R²₂;
Y³ is selected from the group consisting of -H, alkyl, alkenyl, alkynyl, aryl, alicyclic, aralkyl, aryloxyalkyl, alkoxyalkyl, -C(O)R³, -S(O)₂R³, -C(O)-R¹¹, -CONHR³, -NR²₂, and -OR³, all except H are optionally substituted;
R² is selected from the group consisting of R³ and -H;
R³ is selected from the group consisting of alkyl, aryl, alicyclic, and aralkyl;
each R⁴ is independently selected from the group consisting of -H, and alkyl, or together R⁴ and R⁴ form a cyclic alkyl group;
R⁵ is selected from the group consisting of lower alkyl, lower aryl, lower aralkyl, and lower alicyclic;
R⁷ is independently selected from the group consisting of-H, lower alkyl, lower alicyclic, lower aralkyl, lower aryl, and -C(O)R¹⁰;
R⁸ is independently selected from the group consisting of -H, lower alkyl, lower aralkyl, lower aryl, lower alicyclic, -C(O)R¹⁰, or together they form a bidendate alkyl;
each R⁹ is independently selected from the group consisting of -H, alkyl, aralkyl, and alicyclic, or together R⁹ and R⁹ form a cyclic alkyl group;
R¹⁰ is selected from the group consisting of -H, lower alkyl, -NH₂, lower aryl, and lower perhaloalkyl;
R¹¹ is selected from the group consisting of alkyl, aryl, -NR²₂, and -OR²; and pharmaceutically acceptable prodrugs and salts thereof; with the provisos that:
   a) when X³ is alkyl or alkene, then A is -N(R⁸₂);
   b) X³ is not alkylamine and alkylaminoalkyl substituted with phosphonic esters and acids; and
   c) A, L, E, J, and Y³ together may only form 0-2 cyclic groups.

More preferred are such compounds wherein X³ is not -alkoxyalkyl-, -alkyloxy-, -alkythioalkyl-, and -alkylthio-. Particularly preferred are such compounds with the additional proviso that when X³ is aryl or alkylaryl, said aryl or alkylaryl group is not linked 1,4 through a six-membered aromatic ring.

Especially preferred benzimidazole compounds include those wherein A, L, and E are independently selected from the group consisting of -H, -NR⁸₂, -NO₂, hydroxy, halogen, -OR⁷, alkylaminocarbonyl, -SR⁷, lower perhaloalkyl, and C1-C5 alkyl, or together E and J together form a cyclic group; and wherein J is selected from the group consisting of -H, halogen, lower alkyl, lower hydroxyalkyl, -NR⁸₂, lower R⁸₂N-alkyl, lower haloalkyl, lower perhaloalkyl, lower alkenyl, lower alkynyl, lower aryl, heterocyclic, and alicyclic; and wherein Y is selected from the group consisting of alicyclic and lower alkyl; wherein X³ is selected from the group consisting of -heteroaryl-, -alkylcarbonylamino-, -alkylaminocarbonyl-, and -alkoxycarbonyl-. More preferred are such compounds wherein
R¹⁸ is selected from the group consisting of -H, methyl, and ethyl;
R¹² and R¹³ are independently selected from the group consisting of -H, methyl, i-propyl, i-butyl, and benzyl, or together are connected via 2-5 carbon atoms to form a cycloalkyl group;
R¹⁴ is -OR¹⁷;
R¹⁷ is selected from the group consisting of methyl, ethyl, propyl, t-butyl, and benzyl; and
R¹⁵ and R¹⁶ are independently selected from the group consisting of lower alkyl, and lower aralkyl, or together R¹⁵ and R¹⁶ are connected via 2-6 atoms, optionally including 1 heteroatom selected from the group consisting of O, and N. Most preferred are such compounds wherein A is selected from the group consisting of -H, -NH₂, -F, and -CH₃;
L is selected from the group consisting of -H, -F, -OCH₃, Cl and -CH₃;
E is selected from the group consisting of -H, and -Cl;
J is selected from the group consisting of -H, halo, C1-C5 hydroxyalkyl, C1-C5 haloalkyl, C1-C5 R⁸₂ N-alkyl, C1-C5 alicyclic, and C1-C5 alkyl;
X³ is selected from the group consisting of -CH₂OCH₂-, -methyleneoxycarbonyl-, and -furan-2,5-diyl-; and
Y is lower alkyl.

Also more preferred are such benzimidazoles where A is -NH₂, L is -F, E is -H, J is ethyl, Y is isobutyl, and X³ is -furan-2,5-diyl-; or
where A is -NH₂, L is -F, E is -H, J is N,N-dimethylaminopropyl, Y is isobutyl, and X³ is -furan-2,5-diyl-.

Particularly preferred are those compounds wherein 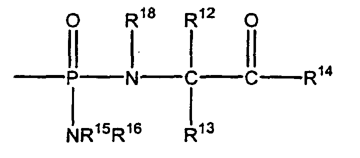 is selected from the group consisting of 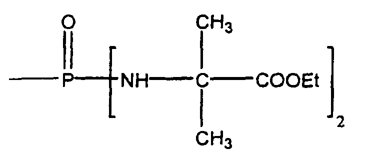 and 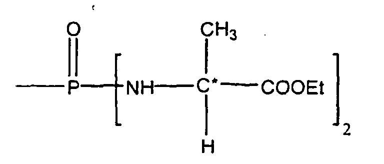 wherein C* has S stereochemistry.
Preferably, oral bioavailability is at least 5%. More preferably, oral bioavailability is at least 10%.

The prodrugs of formula IA may have two isomeric forms around the phosphorus. Preferred is when the phosphorus is not chiral. Also preferred is when there is no chiral center in the amino groups attached to the phosphorus. Also preferred is when n is 1 and R¹² is -H, then the carbon attached to R¹² and R¹³ has S stereochemistry.

In another aspect preferred are compounds of formula I or I-A where M is 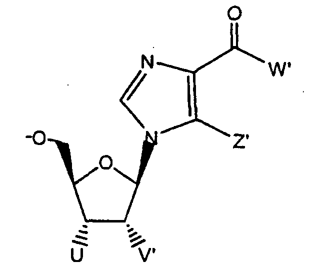 wherein
Z' is selected from the group consisting of alkyl or halogen,
U and V' are independently selected from the group consisting of hydrogen, hydroxy, acyloxy or when taken together form a lower cyclic ring containing at least one oxygen;
W' is selected from the group consisting of amino and lower alkyl amino;
and pharmaceutically acceptable salts thereof.

In another aspect, preferred are compounds of formula II 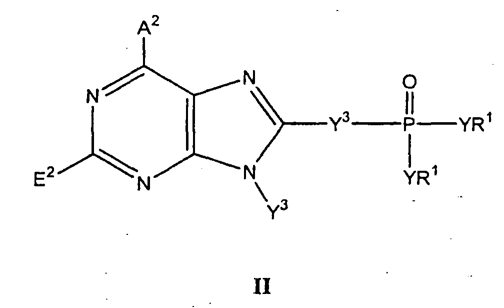 wherein
A² is selected from the group consisting of -NR⁸₂, NHSO₂R³, -OR⁵, -SR⁵, halogen, lower alkyl, -CON(R⁴)₂, guanidine, amidine, -H, and perhaloalkyl;
E² is selected from the group consisting of -H, halogen, lower alkylthio, lower perhaloalkyl, lower alkyl, lower alkenyl, lower alkynyl, lower alkoxy, -CN, and -NR⁷₂;
X³ is selected from the group consisting of -alkyl(hydroxy)-, -alkyl-, -alkynyl-, -aryl-, -carbonylalkyl-, -1,1-dihaloalkyl-, -alkoxyalkyl-, -alkyloxy-, -alkylthioalkyl-, -alkylthio-, -alkylaminocarbonyl-, -alkylcarbonylamino-, -alicyclic-, -aralkyl-, -alkylaryl-, -alkoxycarbonyl-, -carbonyloxyalkyl-, -alkoxycarbonylamino-, and -alkylaminocarbonylamino-, all optionally substituted; with the proviso that X³ is not substituted with -COOR², -SO₃H, or -PO₃R²₂;
Y³ is selected from the group consisting of -H, alkyl, alkenyl, alkynyl, aryl, alicyclic, aralkyl, aryloxyalkyl, alkoxyalkyl, -C(O)R³, -S(O)₂R³, -C(O)-R¹¹, -CONHR³, -NR²₂, and -OR³, all except H are optionally substituted;
Y is independently selected from the group consisting of -O-, and -NR⁶-;
when Y is -O-, then R¹ attached to -O- is independently selected from the group consisting of -H, alkyl, optionally substituted aryl, optionally substituted alicyclic where the cyclic moiety contains a carbonate or thiocarbonate, optionally substituted -alkylaryl, -C(R²)₂OC(O)NR²₂, -NR²-C(O)-R³, -C(R²)₂- OC(O)R³, -C(R²)₂-O-C(O)OR³, -C(R²)₂OC(O)SR³, -alkyl-S-C(O)R³, -alkyl-S-S-alkylhydroxy, and -alkyl-S-S-S-alkylhydroxy,
when Y is -NR⁶-, then R¹ attached to -NR⁶- is independently selected from the group consisting of -H, -[C(R²)₂]_{q}-COOR³, -C(R⁴)₂COOR³, -[C(R²)₂]_{q}C(O)SR, and -cycloalkylene-COOR³;
or when either Y is independently selected from -O- and -NR⁶-, then together R¹ and R' are -alkyl-S-S-alkyl- to form a cyclic group, or together R¹ and R¹ are 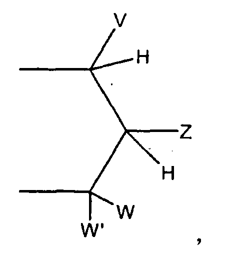
wherein
V, W, and W' are independently selected from the group consisting of -H, alkyl, aralkyl, alicyclic, aryl, substituted aryl, heteroaryl, substituted heteroaryl, 1-alkenyl, and 1-alkynyl; or
together V and Z are connected via an additional 3-5 atoms to form a cyclic group containing 5-7 atoms, optionally 1 heteroatom, substituted with hydroxy, acyloxy, alkoxycarbonyloxy, or aryloxycarbonyloxy attached to a carbon atom that is three atoms from both Y groups attached to the phosphorus; or
together V and Z are connected via an additional 3-5 atoms to form a cyclic group, optionally containing 1 heteroatom, that is fused to an aryl group at the beta and gamma position to the Y attached to the phosphorus;
together V and W are connected via an additional 3 carbon atoms to form an optionally substituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said carbon atoms that is three atoms from a Y attached to the phosphorus;
together Z and W are connected via an additional 3-5 atoms to form a cyclic group, optionally containing one heteroatom, and V must be aryl, substituted aryl, heteroaryl, or substituted heteroaryl;
together W and W' are connected via an additional 2-5 atoms to form a cyclic group, optionally containing 0-2 heteroatoms, and V must be aryl, substituted aryl, heteroaryl, or substituted heteroaryl;
Z is selected from the group consisting of -CHR²OH , -CHR²OC(O)R³, -CHR²OC(S)R³, -CHR²OC(S)OR³, -CHR²OC(O)SR³, -CHR²OCO₂R³, -OR² , -SR², -CHR²N₃, -CH₂aryl, -CH(aryl)OH, -CH(CH=CR²₂)OH, -CH(C≡CR²)OH, -R², -NR²₂, -OCOR³, -OCO₂R³, -SCOR³, -SCO₂R³, -NHCOR², -NHCO₂R³, -CH₂NHaryl, -(CH₂)ₚ-OR², and -(CH₂)ₚ-SR²;
p is an integer 2 or 3;
q is an integer 1 or 2;
with the provisos that:
   a) V, Z, W, W' are not all -H; and
   b) when Z is -R², then at least one of V, W, and W' is not -H, alkyl, aralkyl, or alicyclic;
R² is selected from the group consisting of R³ and -H;
R³ is selected from the group consisting of alkyl, aryl, alicyclic, and aralkyl;
each R⁴ is independently selected from the group consisting of -H, and alkyl, or together R⁴ and R⁴ form a cyclic alkyl group;
R⁶ is selected from the group consisting of -H, lower alkyl, acyloxyalkyl, alkoxycarbonyloxyalkyl, and lower acyl;
R⁵ is selected from the group consisting of lower alkyl, lower aryl, lower aralkyl, and lower alicyclic;
R⁹ is independently selected from the group consisting of -H, loweralkyl, lower alicyclic, lower aralkyl, lower aryl, and -(CO)R¹⁰;
R⁸ is independently selected from the group consisting of -H, lower alkyl, lower aralkyl, lower aryl, lower alicyclic, -C(O)R¹⁰, or together they form a bidendate alkyl;
R⁹ is selected from the group consisting of alkyl, aralkyl, and alicyclic;
R¹⁰ is selected from the group consisting of -H, lower alkyl, -NH₂, lower aryl, and lower perhaloalkyl; and
R¹¹ is selected from the group consisting of alkyl, aryl, -NR₂², and -OR²; and pharmaceutically acceptable prodrugs and salts thereof.

Preferred A² groups for formula II include -NR⁸₂, lower alkyl, lower perhaloalkyl, lower alkoxy, and halogen. Particularly preferred are -NR⁸₂, and halogen. Especially preferred is -NR⁸₂. Most preferred is -NH₂.

Preferred E² groups for formula II include -H, halogen, lower perhaloalkyl, -CN, lower alkyl, lower alkoxy, and lower alkylthio. Particularly preferred E² groups include - H, -SMe, -Et, and -Cl. Especially preferred is -H and -SCH₃.

Preferred X³ groups for formula II include -alkyl-, -alkynyl-, -alkoxyalkyl-, -alkylthio-, -aryl-, -1,1-dihaloalkyl-, -carbonylalkyl-, -heteroaryl-, -alkylcarbonylamino-, and -alkylaminocarbonyl. Particularly preferred is -alkyl- substituted with 1 to 3 substituents selected from halogen, and -OH. Particularly preferred are - alkylaminocarbonyl-, -alkoxyalkyl-, and -heteroaryl-. Preferred -alkoxyalkyl- groups include -methoxymethyl-. Preferred -heteroaryl- groups include -furan-2,5-diyl-, optionally substituted.

Preferred Y³ groups for formula II include aralkyl, alicyclic, alkyl, and aryl, all optionally substituted. Particularly preferred is lower alkyl. Particularly preferred Y³ groups include (2-naphthyl)methyl, cyclohexylethyl, phenylethyl, nonyl, cyclohexylpropyl, ethyl, cyclopropylmethyl, cyclobutylmethylphenyl, (2-methyl)propyl, neopentyl, cyclopropyl, cyclopentyl, (1-imidozolyl)propyl, 2-ethoxybenzyl, 1-hydroxy-2,2-dimethylpropyl, 1-chloro-2,2-dimethylpropyl, 2,2-dimethylbutyl , 2-(spiro-3 ,3 - dimethylcyclohex-4-enyl)propyl, and 1-methylneopentyl. Especially preferred is neopentyl and isobutyl.

Preferred R⁴ and R⁷ groups are -H, and lower alkyl. Particularly preferred are -H, and methyl.

In another preferred aspect, A² is -NR⁸₂ or halogen, E² is -H, halogen, -CN, lower alkyl, lower perhaloalkyl, lower alkoxy, or lower alkylthio, X³ is -alkyl-, -alkoxyalkyl-, -alkynyl-, -1,1-dihaloalkyl-, -carbonylakyl-, -alkyl(OH)-, -alkylcarbonylamino-, -alkylaminocarbonyl-, -alkylthio-, -aryl-, or -heteroaryl-, and R⁴ and R⁷ is -H or lower alkyl. Particularly preferred are such compounds where Y³ is aralkyl, aryl, alicyclic, or alkyl.

In another preferred aspect, A² is -NR⁸₂, E² is -H, Cl-, or methylthio, and X³ is optionally substituted -furan-2,5-diyl-, or -alkoxyalkyl-. Particularly preferred are such compounds where A² is -NH₂, X³ is -furan-2,5-diyl-, or -methoxymethyl-, and Y³ is lower alkyl. Most preferred are such compounds where E² is H, X³ is -furan-2,5-diyl-, and Y³ is neopentyl; those where E² is -SCH₃, X³ is -furan-2,5-diyl-, and Y³ is isobutyl; and those where E² is -H, X³ is -furan-2,5-diyl-, and Y³ is 1-(3-chloro-2,2-dimethyl)-propyl. Especially preferred are such compounds where R¹ is -CH₂O-C(O)-C(CH₃)₃.

In another aspect, preferred are compounds of formula III 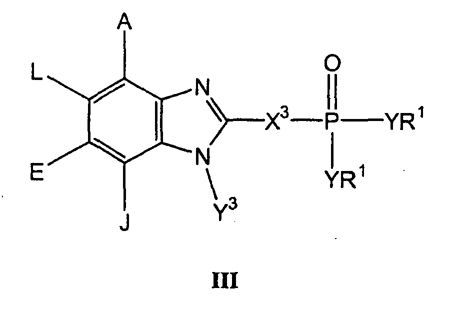 wherein:
A, E, and L are selected from the group consisting of -NR⁸₂, -NO₂, -H, -OR⁷, -SR⁷, -C(O)NR⁴₂, halo, -COR¹¹, -SO₂R³, guanidine, amidine, -NHSO₂R⁵, -SO₂NR⁴₂, -CN, sulfoxide, perhaloacyl, perhaloalkyl, perhaloalkoxy, C1-C5 alkyl, C2-C5 alkenyl, C2-C5 alkynyl, and lower alicyclic, or together A and L form a cyclic group, or together L and E form a cyclic group, or together E and J form a cyclic group including aryl, cyclic alkyl, and heterocyclic;
J is selected from the group consisting of -NR⁸₂, -NO₂, -H, -OR⁷, -SR⁷, -C(O)NR⁴₂, halo, -C(O)R¹¹, -CN, sulfonyl, sulfoxide, perhaloalkyl, hydroxyalkyl, perhaloalkoxy, alkyl, haloalkyl, aminoalkyl, alkenyl, alkynyl, alicyclic, aryl, and aralkyl, or together with Y forms a cyclic group including aryl, cyclic alkyl and heterocyclic alkyl;
X³ is selected from the group consisting of -alkyl(hydroxy)-, -alkyl-, -alkynyl-, -aryl-, -carbonylalkyl-, -1,1-dihaloalkyl-, -alkoxyalkyl-, -alkyloxy-, -alkylthioalkyl-, -alkylthio-, -alkylaminocarbonyl-, -alkylcarbonylamino-, -alicyclic-, -aralkyl-, -alkylaryl-, -alkoxycarbonyl-, -carbonyloxyalkyl-, -alkoxycarbonylamino-, and -alkylaminocarbonylamino-, all optionally substituted; with the proviso that X³ is not substituted with -COOR², -SO₃H, or -PO₃R²₂;
Y³ is selected from the group consisting of -H, alkyl, alkenyl, alkynyl, aryl, alicyclic, aralkyl, aryloxyalkyl, alkoxyalkyl, -C(O)R³, -S(O)₂R³, -C(O)-R¹¹, -CONHR³, -NR²₂, and -OR³, all except H are optionally substituted;
Y is independently selected from the group consisting of -O-, and -NR⁶-;
when Y is -O-, then R¹ attached to -O- is independently selected from the group consisting of -H, alkyl, optionally substituted aryl, optionally substituted alicyclic where the cyclic moiety contains a carbonate or thiocarbonate, optionally substituted -alkylaryl, -C(R²)₂OC(O)NR²₂, -NR²-C(O)-R³, -C(R²)₂- OC(O)R³, -C(R²)₂-O-C(O)OR³, -C(R²)₂OC(O)SR³, -alkyl-S-C(O)R³, -alkyl-S-S-alkylhydroxy, and -alkyl-S-S-S-alkylhydroxy,
when Y is -NR⁶-, then R¹ attached to -NR⁶- is independently selected from the group consisting of -H, -[C(R²)₂]_{q}-COOR³, -C(R⁴)₂COOR³, -[C(R²)₂]_{q}-C(O)SR, and
-cycloalkylene-COOR³;
or when either Y is independently selected from -O- and -NR⁶-, then together R¹ and R¹ are -alkyl-S-S-alkyl- to form a cyclic group, or together R¹ and R¹ are 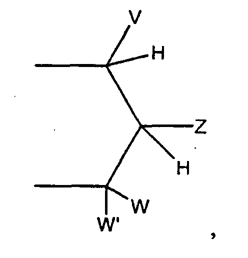 wherein
V, W, and W' are independently selected from the group consisting of -H, alkyl, aralkyl, alicyclic, aryl, substituted aryl, heteroaryl, substituted heteroaryl, 1-alkenyl, and 1-alkynyl; or
together V and Z are connected via an additional 3-5 atoms to form a cyclic group containing 5-7 atoms, optionally 1 heteroatom, substituted with hydroxy, acyloxy, alkoxycarbonyloxy, or aryloxycarbonyloxy attached to a carbon atom that is three atoms from both Y groups attached to the phosphorus; or
together V and Z are connected via an additional 3-5 atoms to form a cyclic group, optionally containing 1 heteroatom, that is fused to an aryl group at.the beta and gamma position to the Y attached to the phosphorus;
together V and W are connected via an additional 3 carbon atoms to form an optionally substituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said carbon atoms that is three atoms from a Y attached to the phosphorus;
together Z and W are connected via an additional 3-5 atoms to form a cyclic group, optionally containing one heteroatom, and V must be aryl, substituted aryl, heteroaryl, or substituted heteroaryl;
together W and W' are connected via an additional 2-5 atoms to form a cyclic group, optionally containing 0-2 heteroatoms, and V must be aryl, substituted aryl, heteroaryl, or substituted heteroaryl;
Z is selected from the group consisting of -CHR²OH, -CHR²OC(O)R³, -CHR²OC(S)R³, -CHR²OC(S)OR³, -CHR²OC(O)SR³, -CHR²OCO₂R³, -OR², -SR², -CHR²N₃, -CH₂aryl, -CH(aryl)OH, -CH(CH=CR²₂)OH, -CH(C≡CR²)OH, -R², -NR²₂, -OCOR³, -OCO₂R³, -SCOR³, -SCO₂R³, -NHCOR², -NHCO₂R³, -CH₂NHaryl, -(CH₂)ₚ-OR², and -(CH₂)ₚ-SR²;
p is an integer 2 or 3;
q is an integer 1 or 2;
with the provisos that:
   a) V, Z, W, W' are not all -H; and
   b) when Z is -R², then at least one of V, W, and W' is not -H, alkyl, aralkyl, or alicyclic;
R² is selected from the group consisting of R³ and -H;
R³ is selected from the group consisting of alkyl, aryl, alicyclic, and aralkyl;
each R⁴ is independently selected from the group consisting of -H, and alkyl, or together R⁴ and R⁴ form a cyclic alkyl group;
R⁶ is selected from the group consisting of -H, lower alkyl, acyloxyalkyl, alkoxycarbonyloxyalkyl, and lower acyl;
R⁵ is selected from the group consisting of lower alkyl, lower aryl, lower aralkyl, and lower alicyclic;
R⁷ is independently selected from the group consisting of -H, lower alkyl, lower alicyclic, lower aralkyl, lower aryl, and -C(O)R¹⁰;
R⁸ is independently selected from the group consisting of -H, lower alkyl, lower aralkyl, lower aryl, lower alicyclic, -C(O)R¹⁰, or together they form a bidendate alkyl;
R⁹ is selected from the group consisting of alkyl, aralkyl, and alicyclic;
R¹⁰ is selected from the group consisting of -H, lower alkyl, -NH₂, lower aryl, and lower perhaloalkyl;
R¹¹ is selected from the group consisting of alkyl, aryl, -NR₂², and -OR², and pharmaceutically acceptable prodrugs and salts thereof.

Preferred A, L, and E groups for formula III include -H, -NR⁸₂, -NO₂, hydroxy, alkylaminocarbonyl, halogen, -OR⁷, -SR⁷, lower perhaloalkyl, and C1-C5 alkyl, or together E and J form a cyclic group. Such a cyclic group may be aromatic, cyclic alkyl, or heterocyclic alkyl, and may be optionally substituted. Suitable aromatic groups include thiazole. Particularly preferred A, L and E groups are -NR⁸₂, -H, hydroxy, halogen, lower alkoxy, lower perhaloalkyl, and lower alkyl.

Preferred A groups for formula III include, -NR⁸₂, -H, halogen, lower perhaloalkyl, and lower alkyl.

Preferred L and E groups for formula III include -H, lower alkoxy, lower alkyl, and halogen.

Preferred J groups for formula III include -H, halogen, lower alkyl, lower hydroxylalkyl, -NR⁸₂, lower R⁸₂N-alkyl, lower haloalkyl, lower perhaloalkyl, lower alkenyl, lower alkynyl, lower aryl, heterocyclic, and alicyclic, or together with Y forms a cyclic group. Such a cyclic group may be aromatic, cyclic alkyl, or heterocyclic, and may be optionally substituted. Particularly preferred J groups include -H, halogen, and lower alkyl, lower hydroxyalkyl, -NR⁸₂, lower R⁸₂N-alkyl, lower haloalkyl, lower alkenyl, alicyclic, and aryl. Especially preferred are alicyclic and lower alkyl.

Preferred X³ groups for formula III include -alkyl-, -alkynyl-, -aryl-, -alkoxyalkyl-, -alkylthio-, -alkylaminocarbonyl-, -alkylcarbonylamino-, -1,1-dihaloalkyl-, -carbonylalkyl-, and -alkyl(OH)-. Particularly preferred is -heteroaryl-, -alkylaminocarbonyl-, -1,1-dihaloalkyl-, and -alkoxyalkyl-. Also particularly preferred are -heteroaryl-, -alkylaminocarbonyl-, and -alkoxyalkyl-. Especially preferred are - methylaminocarbonyl-, -methoxymethyl-, and -furan-2,5-diyl-.

In another preferred aspect, when X³ is aryl or alkylaryl, these groups are not linked 1,4 through a 6-membered aromatic ring.

Preferred Y³ groups for formula III include -H, alkyl, aralkyl, aryl, and alicyclic, all except -H may be optionally substituted. Particularly preferred are lower alkyl, and alicyclic.

Preferred R⁴ and R⁷ groups include -H, and lower alkyl.

In one preferred aspect of compounds of formula III, A, L, and E are independently -H, lower alkyl, hydroxy, halogen, lower alkoxy, lower perhaloalkyl, and -NR⁸₂; X³ is -aryl-, -alkoxyalkyl-, -alkyl-, -alkylthio-, -1,1-dihaloalkyl-, -carbonylalkyl-, -alkyl(hydroxy)-, -alkylaminocarbonyl-, and -alkylcarbonylamino-; and each R⁴ and R⁷ is independently -H, and lower alkyl. Particularly preferred are such compounds where A, L, and E are independently -H, lower alkyl, halogen, and -NR⁸₂; J is -H, halogen, haloalkyl, hydroxyalkyl, R⁸₂N-alkyl, lower alkyl, lower aryl, heterocyclic, and alicyclic, or together with Y³ forms a cyclic group; and X³ is -heteroaryl-, -alkylaminocarbonyl-, -1,1-dihaloalkyl-, and -alkoxyalkyl-. Especially preferred are such compounds where A is -H, -NH₂, -F, and -CH₃, L is -H, -F, -OCH₃, -Cl, and -CH₃, E is -H and -Cl, J is -H, halo, C1-C5 hydroxyalkyl, C1-C5 haloalkyl, C1-C5 R⁸₂N-alkyl, C1-C5 alicyclic, and C1-C5 alkyl, X³ is -CH₂OCH₂-, and -furan-2,5-diyl-, and Y³ is lower alkyl. Most preferred are the following such compounds and their salts, and prodrug and their salts:
1) A is -NH₂, L is -F, E is -H, J is -H, Y³ is isobutyl, and X³ is -furan-2,5-diyl-;
2) A, L, and J are -H, E is -Cl, Y³ is isobutyl, and X³ is -furan-2,5-diyl-;
3) A is -NH₂, L is -F, E and J are -H, Y³ is cyclopropylmethyl, and X³ is -furan-2,5-diyl-;
4) A is -NH₂, L is -F, E is -H, J is ethyl, Y³ is isobutyl, and X³ is -furan-2,5-diyl-;
5) A is -CH₃, L is -Cl, E and J are -H, Y³ is isobutyl, and X³ is -furan-2,5-diyl-;
6) A is -NH₂, L is -F, E is -H, J is -Cl, Y³ is isobutyl, and X³ is -furan-2,5-diyl-;
7) A is -NH₂, L is -F, E is -H, J is -Br, Y³ is isobutyl, and X³ is -CH₂OCH₂-; and
8) A, L, E, and J are -CH₃, Y³ is cyclopropylmethyl, and X³ is -furan-2,5-diyl-.

Also especially preferred are compounds where A is -NH₂, L is -F, E is -H, J is bromopropyl, bromobutyl, chlorobutyl, cyclopropyl, hydroxypropyl, or N,N-dimethylaminoptopyl, and X³ is -furan-2,5-diyl-. The preferred prodrug is where R¹ is pivaloyloxymethyl or its HCl salt.

In another aspect, preferred are compounds of formula IV 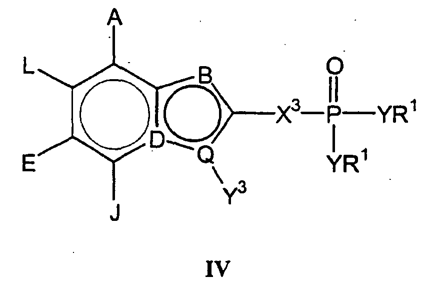 wherein:
B is selected from the group consisting of -NH-, -N= and -CH=;
D is selected from the group consisting of 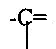 and 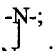
Q is selected from the group consisting of -C= and -N'- with the proviso that when B is -NH- then Q is -C= and D is 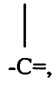 when B is -CH= then Q is -N- and D is 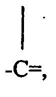 when B is -N=, then D is 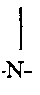 and Q is -C=;
A, E, and L are selected from the group consisting of -NR⁸₂, -NO₂, -H, -OR⁷, -SR⁷, -C(O)NR⁴₂, halo, -COR¹¹, -SO₂R³, guanidino, amidino, -NHSO₂R⁵, -SO₂NR⁴₂, -CN, sulfoxide, perhaloacyl, perhaloalkyl, perhaloalkoxy, C1-C5 alkyl, C2-C5 alkenyl, C2-C5 alkynyl, and lower alicyclic, or together A and L form a cyclic group, or together L and E form a cyclic group, or together E and J form a cyclic group including aryl, cyclic alkyl, and heterocyclic;
J is selected from the group consisting of -NR⁸₂, -NO₂, -H, -OR⁷, -SR⁷, -C(O)NR⁴₂, halo, -C(O)R¹¹, -CN, sulfonyl, sulfoxide, perhaloalkyl, hydroxyalkyl, perhaloalkoxy, alkyl, haloalkyl, aminoalkyl, alkenyl, alkynyl, alicyclic, aryl, and aralkyl, or together with Y forms a cyclic group including aryl, cyclic alkyl and heterocyclic alkyl;
X³ is selected from the group consisting of -alkyl(hydroxy)-, -alkyl-, -alkynyl-, -aryl-, -carbonylalkyl-, -1,1-dihaloalkyl-, -alkoxyalkyl-, -alkyloxy-, -alkylthioalkyl-, -alkylthio-, -alkylaminocarbonyl-, -alkylcarbonylamino-, -alicyclic-, -aralkyl-, -alkylaryl-, -alkoxycarbonyl-, -carbonyloxyalkyl-, -alkoxycarbonylamino-, and -alkylaminocarbonylamino-, all optionally substituted; with the proviso that X³ is not substituted with -COOR², -SO₃H, or -PO₃R²₂;
Y³ is selected from the group consisting of -H, alkyl, alkenyl, alkynyl, aryl, alicyclic, aralkyl, aryloxyalkyl, alkoxyalkyl, -C(O)R³, -S(O)₂R³, -C(O)-R¹¹, -CONHR³, -NR²₂, and -OR³, all except H are optionally substituted;
Y is independently selected from the group consisting of -O-, and -NR⁶-;
when Y is -O-, then R¹ attached to -O- is independently selected from the group consisting of -H, alkyl, optionally substituted aryl, optionally substituted alicyclic where the cyclic moiety contains a carbonate or thiocarbonate, optionally substituted -alkylaryl, -C(R²)₂OC(O)NR²₂, -NR²-C(O)-R³, -C(R²)₂- OC(O)R³, -C(R²)₂-O-C(O)OR³, -C(R²)₂OC(O)SR³, -alkyl-S-C(O)R³, -alkyl-S-S-alkylhydroxy, and -alkyl-S-S-S-alkylhydroxy,
when Y is -NR⁶-, then R¹ attached to -NR⁶- is independently selected from the group consisting of -H, -[C(R²)₂]_{q}-COOR³, -C(R⁴)₂COOR³, -[C(R²)₂]_{q}-C(O)SR, and -cycloalkylene-COOR³;
or when either Y is independently selected from -O- and -NR⁶-, then together R¹ and R¹ are -alkyl-S-S-alkyl- to form a cyclic group, or together R¹ and R¹ are 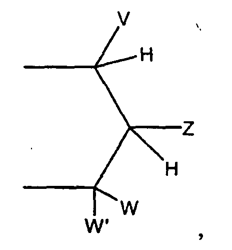
wherein
V, W, and W' are independently selected from the group consisting of -H, alkyl, aralkyl, alicyclic, aryl, substituted aryl, heteroaryl, substituted heteroaryl, 1-alkenyl, and 1-alkynyl; or
together V and Z are connected via an additional 3-5 atoms to form a cyclic group containing 5-7 atoms, optionally 1 heteroatom, substituted with hydroxy, acyloxy, alkoxycarbonyloxy, or aryloxycarbonyloxy attached to a carbon atom that is three atoms from both Y groups attached to the phosphorus; or
together V and Z are connected via an additional 3-5 atoms to form a cyclic group, optionally containing 1 heteroatom, that is fused to an aryl group at the beta and gamma position to the Y attached to the phosphorus;
together V and W are connected via an additional 3 carbon atoms to form an optionally substituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said carbon atoms that is three atoms from a Y attached to the phosphorus;
together Z and W are connected via an additional 3-5 atoms to form a cyclic group, optionally containing one heteroatom, and V must be aryl, substituted aryl, heteroaryl, or substituted heteroaryl;
together W and W' are connected via an additional 2-5 atoms to form a cyclic group, optionally containing 0-2 heteroatoms, and V must be aryl, substituted aryl, heteroaryl, or substituted heteroaryl;
Z is selected from the group consisting of -CHR²OH, -CHR²OC(O)R³, -CHR²OC(S)R³, -CHR²OC(S)OR³, -CHR²OC(O)SR³, -CHR²OCO₂R³, -OR², -SR², -CHR²N₃, -CH₂aryl, -CH(aryl)OH, -CH(CH=CR²₂)OH, -CH(C≡CR²)OH, -R², -NR²₂, -OCOR³, -OCO₂R³, -SCOR³, -SCO₂R³, -NHCOR², -NHCO₂R³, -CH₂NHaryl, -(CH₂)ₚ-OR², and -(CH₂)ₚ-SR²;
p is an integer 2 or 3;
q is an integer 1 or 2;
with the provisos that:
   a) V, Z, W, W' are not all -H; and
   b) when Z is -R², then at least one of V, W, and W' is not -H, alkyl, aralkyl, or alicyclic;
R² is selected from the group consisting of R³ and -H;
R³ is selected from the group consisting of alkyl, aryl, alicyclic, and aralkyl;
each R⁴ is independently selected from the group consisting of-H, and alkyl, or together R⁴ and R⁴ form a cyclic alkyl group;
R⁶ is selected from the group consisting of -H, lower alkyl, acyloxyalkyl, alkoxycarbonyloxyalkyl, and lower acyl;
R⁵ is selected from the group consisting of lower alkyl, lower aryl, lower aralkyl, and lower alicyclic;
R⁷ is independently selected from the group consisting of -H, lower alkyl, lower alicyclic, lower aralkyl, lower aryl, and -C(O)R¹⁰;
R⁸ is independently selected from the group consisting of -H, lower alkyl, lower aralkyl, lower aryl, lower alicyclic, -C(O)R¹⁰, or together they form a bidentate alkyl;
R⁹ is selected from the group consisting of alkyl, aralkyl, and alicyclic;
R¹⁰ is selected from the group consisting of-H, lower alkyl, -NH₂, lower aryl, and lower perhaloalkyl;
R¹¹ is selected from the group consisting of alkyl, aryl, -NR₂² and -OR²; and pharmaceutically acceptable prodrugs and salts thereof.

Preferred A, L, and E groups in formula IV include -H, -NR⁸₂, -NO₂, hydroxy, halogen, -OR⁷, alkylaminocarbonyl, -SR⁷, lower perhaloalkyl, and C1-C5 alkyl, or together E and J form a cyclic group. Such a cyclic group may be aromatic or cyclic alkyl, and may be optionally substituted. Suitable aromatic groups include thiazole. Particularly preferred A, L and E groups are -NR⁸₂, -H, hydroxy, halogen, lower alkoxy, lower perhaloalkyl, and lower alkyl.

Preferred A groups in formula IV include -NR⁸₂, lower alkyl, -H, halogen, and lower perhaloalkyl.

Preferred L and E groups in formula IV include -H, lower alkoxy, lower alkyl, and halogen.

Preferred J groups in formula IV include -H, halogen, lower alkyl, lower hydroxyalkyl, -NR⁸₂, lower R⁸₂N-alkyl, lower haloalkyl, lower perhaloalkyl, lower alkenyl, lower alkynyl, lower aryl, heterocyclic, and alicyclic or together with Y³ forms a cyclic group. Such a cyclic group may be aromatic or cyclic alkyl, and may be optionally substituted. Particularly preferred J groups -H, halogen, lower alkyl, lower hydroxyalkyl, -NR⁸₂, lower R⁸₂N-alkyl, lower haloalkyl, lower alkenyl, alicyclic, and aryl.

Preferred X³ groups in formula IV include -alkyl-, -alkynyl-, -alkoxyalkyl-, -alkylthio-, -aryl-, -alkylaminocarbonyl-, -alkylcarbonylamino-, -1,1-dihaloalkyl-, -carbonylalkyl-, and -alkyl(OH)-. Particularly preferred is -1,1-dihaloalkyl-, -alkylaminocarbonyl-, -alkoxyalkyl-, and -heteroaryl-. Such compounds that are especially preferred are -heteroaryl-, -alkylaminocarbonyl-, and -alkoxyalkyl-. Most preferred is -methylaminocarbonyl-, -methoxymethyl-, and -furan-2,5-diyl.

In one preferred aspect, X³ is not -(C2-C3 alkyl)aminocarbonyl-.

Preferred Y³ groups for formula IV include -H, alkyl, aryl, aralkyl, and alicyclic, all except -H may be optionally substituted. Particularly preferred Y³ groups include lower alkyl, and alicyclic.

Preferred R⁴ and R⁷ groups include -H, and lower alkyl.

In one preferred aspect of formula IV, B is NH, D is 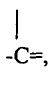 and Q is -C=. In another preferred aspect, B is -N=, D is 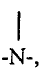 and Q is -C=.

In another preferred aspect of formula IV, A, L, and E are independently -NR⁸₂, lower alkyl, lower perhaloalkyl, lower alkoxy, halogen, -OH, or -H, X³ is -aryl-, -alkoxyalkyl-, -alkyl-, -alkylthio-, -1,1-dihaloalkyl-, -carbonylalkyl-, -alkyl(hydroxy)-, -alkylaminocarbonyl-, and -alkylcarbonylamino-, and each R⁴ and R⁷ is independently -H, or lower alkyl. Particularly preferred are such compounds where A, L, and E are independently -H, lower alkyl, halogen, and -NR⁸₂; J is -H, halogen, haloalkyl, hydroxyalkyl, -R⁸₂N-alkyl, lower alkyl, lower aryl, heterocyclic, and alicyclic, or together with Y³ forms a cyclic group; and X³ is -heteroaryl-, -alkylaminocarbonyl-, -1,1-dihaloalkyl-, and -alkoxyalkyl-. Especially preferred are such compounds where A is -H, -NH₂, -F, or -CH₃, L is -H, -F, -OCH₃, or -CH₃, E is -H, or -Cl, J is -H, halo, C1-C5 hydroxyalkyl, C1-C5 haloalkyl, C1-C5 R⁸₂N-alkyl, C1-C5 alicyclic or C1-C5 alkyl, X³ is -CH₂OCH₂-, or -furan-2,5-diyl-; and Y³ is lower alkyl. Preferred are such compounds where
B is NH, D is 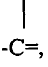 and Q is -C= or where B is -N=, D is 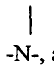 and Q is -C=.

Most preferred are compounds where:
1) A is -NH₂, L is -F, E is -H, J is -H, Y³ is isobutyl, and X³ is -furan-2,5-diyl-;
2) A is -NH₂, L is -F, E is -H, J is -Cl, Y³ is isobutyl, and X³ is -furan-2,5-diyl-.
3) A is -H, L is -H, E is -Cl, J is -H, B is -NH, D is 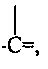 Q is -C=, and Y³ is isobutyl; and
4) A is -CH₃, L is -H, E is -H, J is -H, B is -N=, D is 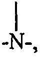 Q is -C=, and Y³ is isobutyl.

Particularly preferred are such compounds where R¹ is -CH₂OC(O)-C(CH₃)₃.

Another especially preferred aspect are such compounds where A, L, and E are -H, lower alkyl, halogen, or -NR⁸₂, J is -H, halogen, lower alkyl, lower aryl, heterocyclic, or alicyclic, or together with Y³ forms a cyclic group, and X³ is -heteroaryl-, -alkylaminocarbonyl-, or -alkoxyalkyl-.

In another aspect, preferred are compounds of formula V wherein M is R⁵-X-wherein
R⁵ is selected from the group consisting of: 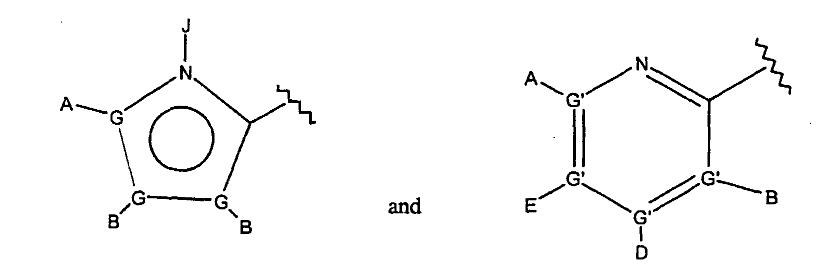 wherein:
each G is independently selected from the group consisting of C, N, O, S, and Se, and wherein only one G may be O, S, or Se, and at most one G is N;
each G' is independently selected from the group consisting of C and N and wherein no more than two G' groups are N;
A is selected from the group consisting of -H, -NR⁴₂, -CONR⁴₂, -CO₂R³, halo, -S(O)R³, -SO₂R³, allcyl, alkenyl, alkynyl, perhaloalkyl, haloalkyl, aryl, -CH₂OH, -CH₂NR⁴₂, -CH₂CN, -CN, -C(S)NH₂, -OR³, -SR³, -N₃, -NHC(S)NR⁴₂, -NHAc, and null;
each B and D are independently selected from the group consisting of -H, alkyl, alkenyl, alkynyl, aryl, alicyclic, aralkyl, alkoxyalkyl, -C(O)R¹¹, -C(O)SR³, -SO₂R¹¹, -S(O)R³, -CN, -NR⁹₂, -OR³, -SR³, perhaloalkyl, halo, -NO₂, and null, all except -H, -CN, perhaloalkyl, -NO₂, and halo are optionally substituted;
E is selected from the group consisting of -H, alkyl, alkenyl, alkynyl, aryl, alicyclic, alkoxyalkyl, -C(O)OR³, -CONR⁴₂, -CN, -NR⁹₂, -NO₂, -OR³, -SR³, perhaloalkyl, halo, and null, all except -H, -CN, perhaloalkyl, and halo are optionally substituted;
J is selected from the group consisting of -H and null;
X is an optionally substituted linking group that links R⁵ to the phosphorus atom via 2-4 atoms, including 0-1 heteroatoms selected from N, O, and S, except that if X is urea or carbamate there is 2 heteroatoms, measured by the shortest path between R⁵ and the phosphorus atom, and wherein the atom attached to the phosphorus is a carbon atom, and wherein X is selected from the group consisting of -alkyl(hydroxy)-, -alkynyl-, -heteroaryl-, -carbonylalkyl-, -1,1-dihaloalkyl-, -alkoxyalkyl-, -alkyloxy-, -alkylthioalkyl-, -alkylthio-, -alkylaminocarbonyl-, -alkylcarbonylamino-, -alkoxycarbonyl-, -carbonyloxyalkyl-, -alkoxycarbonylamino-, and -alkylaminocarbonylamino-, all optionally substituted; with the proviso that X is not substituted with -COOR², -SO₃H, or -PO₃R²₂;
R² is selected from the group consisting of R³ and -H;
R³ is selected from the group consisting of alkyl, aryl, alicyclic, and aralkyl;
each R⁴ is independently selected from the group consisting of -H, and alkyl, or together R⁴ and R⁴ form a cyclic alkyl group;
each R⁹ is independently selected from the group consisting of -H, alkyl, aralkyl, and alicyclic, or together R⁹ and R⁹ form a cyclic alkyl group;
R¹¹ is selected from the group consisting of alkyl, aryl, -NR²₂, and -OR²;
and with the proviso that:
   1) when G' is N, then the respective A, B, D, or E is null;
   2) at least one of A and B, or A, B, D, and E is not selected from the group consisting of -H or null;
   3) when R⁵ is a six-membered ring, then X is not any 2 atom linker, an optionally substituted -alkyloxy-, or an optionally substituted -alkylthio-;
   4) when G is N, then the respective A or B is not halogen or a group directly bonded to G via a heteroatom;
   5) when X is not a -heteroaryl- group, then R⁵ is not substituted with two or more aryl groups;
Y is independently selected from the group consisting of -O-, and -NR⁶-;
when Y is -O-, then R¹ attached to -O- is independently selected from the group consisting of -H, alkyl, optionally substituted aryl, optionally substituted alicyclic where the cyclic moiety contains a carbonate or thiocarbonate, optionally substituted -alkylaryl, -C(R²)₂OC(O)NR²₂, -NR²-C(O)-R³, -C(R²)₂- OC(O)R³, -C(R²)₂-O-C(O)OR³, -C(R²)₂OC(O)SR³, -alkyl-S-C(O)R³, -alkyl-S-S-alkylhydroxy, and -alkyl-S-S-S-alkylhydroxy,
when Y is -NR⁶-, then R¹ attached to -NR⁶- is independently selected from the group consisting of -H, -[C(R²)₂]_{q}-COOR³, -C(R⁴)₂COOR³, -[C(R²)₂]_{q}-C(O)SR, and -cycloalkylene-COOR³;
or when either Y is independently selected from -O- and -NR⁶-, then together R¹ and R¹ are -alkyl-S-S-alkyl- to form a cyclic group, or together R¹ and R¹ are 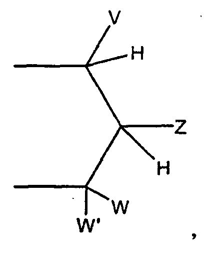
wherein
V, W, and W' are independently selected from the group consisting of -H, alkyl, aralkyl, alicyclic, aryl, substituted aryl, heteroaryl, substituted heteroaryl, 1-alkenyl, and 1-alkynyl; or
together V and Z are connected via an additional 3-5 atoms to form a cyclic group containing 5-7 atoms, optionally 1 heteroatom, substituted with hydroxy, acyloxy, alkoxycarbonyloxy, or aryloxycarbonyloxy attached to a carbon atom that is three atoms from both Y groups attached to the phosphorus; or
together V and Z are connected via an additional 3-5 atoms to form a cyclic group, optionally containing 1 heteroatom, that is fused to an aryl group at the beta and gamma position to the Y attached to the phosphorus;
together V and W are connected via an additional 3 carbon atoms to form an optionally substituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said carbon atoms that is three atoms from a Y attached to the phosphorus;
together Z and W are connected via an additional 3-5 atoms to form a cyclic group, optionally containing one heteroatom, and V must be aryl, substituted aryl, heteroaryl, or substituted heteroaryl;
together W and W' are connected via an additional 2-5 atoms to form a cyclic group, optionally containing 0-2 heteroatoms, and V must be aryl, substituted aryl, heteroaryl, or substituted heteroaryl;
Z is selected from the group consisting of -CHR²OH , -CHR²OC(O)R³, -CHR²OC(S)R³, -CHR²OC(S)OR³, -CHR²OC(O)SR³, -CHR²OCO₂R³, -OR², -SR², -CHR²N₃, -CH₂aryl, -CH(aryl)OH, -CH(CH=CR²₂)OH, -CH(C≡CR²)OH, -R², -NR²₂, -OCOR³, -OCO₂R³, -SCOR³, -SCO₂R³, -NHCOR², -NHCO₂R³, -CH₂NHaryl, -(CH₂)ₚ-OR², and -(CH₂)ₚ-SR²;
p is an integer 2 or 3;
q is an integer 1 or 2;
with the provisos that:
   a) V, Z, W, W' are not all -H; and
   b) when Z is -R², then at least one of V, W, and W' is not -H, alkyl, aralkyl, or alicyclic;
R² is selected from the group consisting of R³ and -H;
R³ is selected from the group consisting of alkyl, aryl, alicyclic, and aralkyl;
each R⁴ is independently selected from the group consisting of -H, and alkyl, or together R⁴ and R⁴ form a cyclic alkyl group;
R⁶ is selected from the group consisting of -H, lower alkyl, acyloxyalkyl, alkoxycarbonyloxyalkyl, and lower acyl;

In one preferred aspect of formula V-1 and formula V-2 compounds,
A" is selected from the group consisting of -NH₂, -CONH₂, halo, -CH₃, -CF₃, - CH₂-halo, -CN, -OCH₃, -SCH₃, and -H;
B" is selected from the group consisting of -H, -C(O)R¹¹, -C(O)SR³, alkyl, aryl, alicyclic, halo, -CN, -SR³, OR³ and -NR⁹₂;
D" is selected from the group consisting of -H, -C(O)R¹¹, -C(O)SR³, -NR⁹₂, alkyl, aryl, alicyclic, halo, and -SR³;
E" is selected from the group consisting of-H, C1-C6 alkyl, lower alicyclic, halo, -CN, -C(O)OR³, and -SR³.
X is selected from the group consisting of -alkyl(hydroxy)-, -alkyl-, -alkynyl-, -aryl-, -carbonylalkyl-, -1,1-dihaloalkyl-, -alkoxyalkyl-, -alkyloxy-, -alkylthioalkyl-, -alkylthio-, -alkylaminocarbonyl-, -alkylcarbonylamino-, -alicyclic-, -aralkyl-, -alkylaryl-, -alkoxycarbonyl-, -carbonyloxyalkyl-, -alkoxycarbonylamino-, and -alkylaminocarbonylamino-, all optionally substituted;
when both Y groups are -O-, then R¹ is independently selected from the group consisting of optionally substituted aryl, optionally substituted benzyl, -C(R²)₂OC(O)R³,-C(R²)₂OC(O)OR³, and
   -H; or
when one Y is -O-, then R¹ attached to -O- is optionally substituted aryl; and the other Y is -NR⁶-, then R¹ attached to -NR⁶- is selected from the group consisting of -C(R⁴)₂COOR³, and -C(R²)₂COOR³; or
when Y is -O- or -NR⁶-, then together R¹ and R¹ are 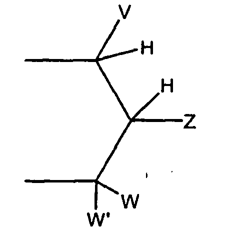
wherein
V, W, and W' are independently selected from the group consisting of -H, alkyl, aralkyl, alicyclic, aryl, substituted aryl, heteroaryl, substituted heteroaryl, 1-alkenyl, and 1-alkynyl, or
together V and W are connected via an additional 3 carbon atoms to form an optionally substituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said carbon atoms that is three atoms from a Y attached to the phosphorus;
together Z and W are connected via an additional 3-5 atoms to form a cyclic group, optionally containing one heteroatom, and V must be aryl, substituted aryl, heteroaryl, or substituted heteroaryl;
together W and W' are connected via an additional 2-5 atoms to form a cyclic group, optionally containing 0-2 heteroatoms, and V must be aryl, substituted aryl, heteroaryl, or substituted heteroaryl;
Z is selected from the group consisting of -CHR²OH, -CHR²OC(O)R³, -CHR²OC(S)R³, -CHR²OC(S)OR³, -CHR²OC(O)SR³, -CHR²OCO₂R³, -OR², -SR², -R², -NHCOR², -NHCO₂R³, -(CH₂)ₚ-OR², and -(CH₂)ₚ-SR²;
p is an integer 2 or 3;
with the provisos that:
   a) V, Z, W, W' are not all -H;
   b) when Z is -R², then at least one of V, W, and W' is not -H, alkyl, aralkyl, or alicyclic; and
   c) both Y groups are not -NR⁶-;
R² is selected from the group consisting of R³ and -H;
R³ is selected from the group consisting of alkyl, aryl, alicyclic, and aralkyl;
R⁶ is selected from the group consisting of -H, and lower alkyl.

In one particularly preferred aspect of formula I where M is R⁵-X- and R⁵ is

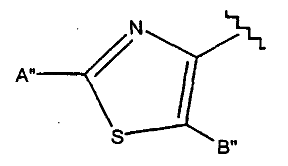

X is selected from the group consisting of methylenoxycarbonyl, and furan-2,5-diyl; at least one Y group is -O-; and pharmaceutically acceptable salts and prodrugs thereof. More preferred are such compounds wherein when Y is -O-, then R¹ attached to - O- is independently selected from the group consisting of -H, optionally substituted phenyl,
-CH₂OC(O)-tBu, -CH₂OC(O)Et and -CH₂OC(O)-iPr;
when Y is -NR⁶-, then R¹ is attached to -NR⁶- independently selected from the group consisting of -C(R²)₂COOR³, -C(R⁴)₂COOR³, or
when Y is -O- or -NR⁶-, and at least one Y is -O-, then together R¹ and R¹ are 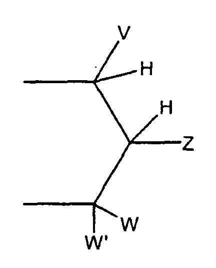
wherein
V is selected from the group consisting of optionally substituted aryl, and optionally substituted heteroaryl; and Z, W', and W are H; and
R⁶ is selected from the group consisting of -H, and lower alkyl.

The following such compounds and their salts are most preferred:
1) A" is -NH₂, X is furan-2,5-diyl, and B" is -CH₂-CH(CH₃)₂;
2) A" is -NH₂, X is furan-2,5-diyl, and B" is -COOEt;
3) A" is -NH₂, X is furan-2,5-diyl, and B" is -SCH₃;
4) A" is -NH₂, X is furan-2,5-diyl, and B" is -SCH₂CH₂SCH₃;
5) A" is -NH₂, X is methyleneoxycarbonyl, and B" is -CH(CH₃)₂.

In another particularly preferred aspect of formula I where M is R⁵-X-, R⁵ is

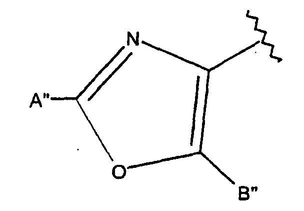

X is furan-2,5-diyl, and methyleneoxycarbonyl, and A" is -NH₂; at least one Y group is -O-; and pharmaceutically acceptable salts and prodrugs thereof Especially preferred are such compounds wherein
when Y is -O-, then each R¹ is independently selected from the group consisting of -H, optionally substituted phenyl, -CH₂OC(O)-tBu, -CH₂OC(O)Et, and -CH₂OC(O)-iPr;
or when Y is -NR⁶-, then each R¹ is independently selected from the group consisting of -C(R²)₂C(O)OR³, and -C(R⁴)₂COOR³;
or when Y is independently selected from -O- and -NR⁶-, then together R¹ and R¹ are 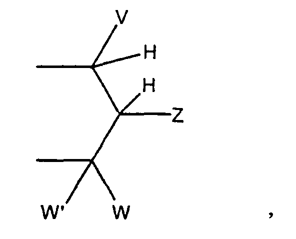
wherein
V selected from the group consisting of optionally substituted aryl and optionally substituted heteroaryl; and Z, W', and W are H. Also especially preferred are such compounds wherein B" is -SCH₂CH₂CH₃.

In another particularly preferred aspect of formula I where M is R⁵-X- and R⁵ is

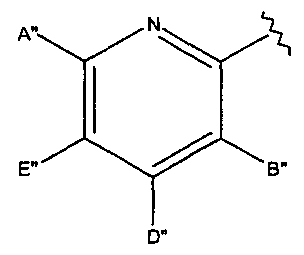

A" is -NH₂, E" and D" are -H, B" is n-propyl and cyclopropyl, X is furan-2,5-diyl and methyleneoxycarbonyl; at least one Y group is -0-; and pharmaceutically acceptable salts and prodrugs thereof. Especially preferred are such compounds wherein R¹ is selected from the group consisting of -H, optionally substituted phenyl -CH₂OC(O)-tBu, - CH₂OC(O)Et, and
-CH₂OC(O)-iPr,
or when Y is -NR⁶-, then each R¹ is independently selected from the group consisting of -C(R²)₂C(O)OR³, and -C(R⁴)₂COOR³;
or when either Y is independently selected from -O- and -NR⁶-, and at least one Y is -O-, then together R¹ and R¹ are 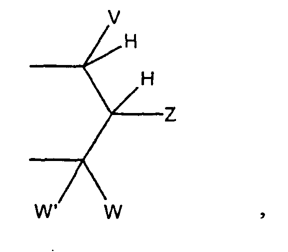
wherein
V is selected from the group consisting of optionally substituted aryl and optionally substituted heteroaryl; and Z, W', and W are H.

In another particularly preferred aspect of formula I where M is R⁵-X- and R⁵ is

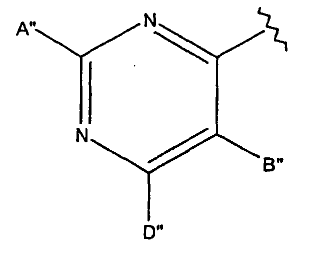

A" is -NH₂, D" is -H, B" is n-propyl and cyclopropyl, X is furan-2,5-diyl and methyleneoxycarbonyl; at least one Y group is -O-; and pharmaceutically acceptable salts and prodrugs thereof. Especially preferred are such compounds wherein when Y is -O-then R¹ is selected from the group consisting of -H, optionally substituted phenyl, - CH₂OC(O)-tBu,
-CH₂OC(O)Et, and -CH₂OC(O)-iPr;
or when one Y is -O- and its corresponding R¹ is -phenyl while the other Y is -NH- and its corresponding R¹ is -CH(Me)C(O)OEt, or
when at least one Y group is -O-, then together R¹ and R¹ are 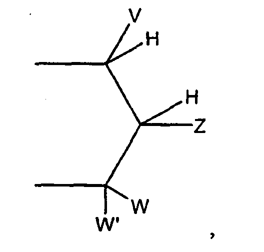
wherein
V is selected from the group consisting of optionally substituted aryl and optionally substituted heteroaryl; and Z, W', and W are H.

Preferred are compounds of formula (X): 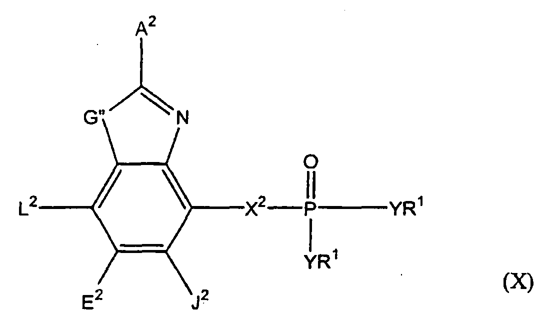 wherein:
G" is selected from the group consisting of -O- and -S-;
A², L², E², and J² are selected from the group consisting of -NR⁴₂, -NO₂, -H, -OR², -SR², -C(O)NR⁴₂, halo, -COR¹¹, -SO₂R³, guanidinyl, amidinyl, aryl, aralkyl, alkyoxyalkyl, -SCN, -NHSO₂R⁹, -SO₂NR⁴₂, -CN, -S(O)R³, perhaloacyl, perhaloalkyl, perhaloalkoxy, C1-C5 alkyl, C2-C5 alkenyl, C2-C5 alkynyl, and lower alicyclic, or together L² and E² or E² and J² form an annulated cyclic group;
X² is selected from the group consisting of -CR²₂-, -CF₂-, - OCR²₂-, -SCR²₂-,
-O-C(O)-, -S-C(O)-, -O-C(S)-, and -NR¹⁹CR²₂-, and wherein in the atom attached to the phosphorus is a carbon atom; with the proviso that X² is not substituted with -COOR², - SO₃H, or -PO₃R²₂;
R¹⁹ is selected from the group consisting of lower alkyl, -H, and -COR²; and
Y is independently selected from the group consisting of -O-, and -NR⁶-;
when Y is -O-, then R¹ attached to -O- is independently selected from the group consisting of -H, alkyl, optionally substituted aryl, optionally substituted alicyclic where the cyclic moiety contains a carbonate or thiocarbonate, optionally substituted -alkylaryl, -C(R²)₂OC(O)NR²₂, -NR²-C(O)-R³, -C(R²)₂- OC(O)R³, -C(R²)₂-O-C(O)OR³, -C(R²)₂OC(O)SR³, -alkyl-S-C(O)R³, -alkyl-S-S-alkylhydroxy, and -alkyl-S-S-S-alkylhydroxy,
when Y is -NR⁶-, then R¹ attached to -NR⁶- is independently selected from the group consisting of -H, -[C(R²)₂]_{q}-COOR³, -C(R⁴)₂COOR³, -[C(R²)₂]_{q}-C(O)SR, and -cycloalkylene-COOR³;
or when either Y is independently selected from -O- and -NR⁶-, then together R¹ and R¹ are -alkyl-S-S-alkyl- to form a cyclic group, or together R¹ and R¹ are 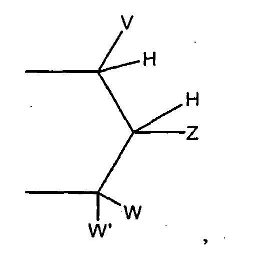
wherein
V, W, and W' are independently selected from the group consisting of -H, alkyl, aralkyl, alicyclic, aryl, substituted aryl, heteroaryl, substituted heteroaryl, 1-alkenyl, and 1-alkynyl; or
together V and Z are connected via an additional 3-5 atoms to form a cyclic group containing 5-7 atoms, optionally 1 heteroatom, substituted with hydroxy, acyloxy, alkoxycarbonyloxy, or aryloxycarbonyloxy attached to a carbon atom that is three atoms from both Y groups attached to the phosphorus; or
together V and Z are connected via an additional 3-5 atoms to form a cyclic group, optionally containing 1 heteroatom, that is fused to an aryl group at the beta and gamma position to the Y attached to the phosphorus;
together V and W are connected via an additional 3 carbon atoms to form an optionally substituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said carbon atoms that is three atoms from a Y attached to the phosphorus;
together Z and W are connected via an additional 3-5 atoms to form a cyclic group, optionally containing one heteroatom, and V must be aryl, substituted aryl, heteroaryl, or substituted heteroaryl;
together W and W' are connected via an additional 2-5 atoms to form a cyclic group, optionally containing 0-2 heteroatoms, and V must be aryl, substituted aryl, heteroaryl, or substituted heteroaryl;
Z is selected from the group consisting of -CHR²OH, -CHR²OC(O)R³, -CHR²OC(S)R³, -CHR²OC(S)OR³, -CHR²OC(O)SR³, -CHR²OCO₂R³, -OR², -SR², -CHR²N₃, -CH₂aryl, -CH(aryl)OH, -CH(CH=CR²₂)OH, -CH(C≡CR²)OH, -R² , -NR²₂, -OCOR³, -OCO₂R³, -SCOR³, -SCO₂R³, -NHCOR², -NHCO₂R³, -CH₂NHaryl, -(CH₂)ₚ₋ OR², and -(CH₂)ₚ-SR²;
p is an integer 2 or 3;
q is an integer 1 or 2;
with the provisos that:
   a) V, Z, W, W' are not all -H; and
   b) when Z is -R², then at least one of V, W, and W' is not -H, alkyl, aralkyl, or alicyclic;
R² is selected from the group consisting of R³ and -H;
R³ is selected from the group consisting of alkyl, aryl, alicyclic, and aralkyl;
each R⁴ is independently selected from the group consisting of -H, alkyl, or together R⁴ and R⁴ form a cyclic alkyl;
R⁶ is selected from the group consisting of -H, lower alkyl, acyloxyalkyl, alkoxycarbonyloxyalkyl, and lower acyl;
each R⁹ is independently selected from the group consisting of -H, alkyl, aralkyl, and alicyclic, or together R⁹ and R⁹ form a cyclic alkyl group;
R¹¹ is selected from the group consisting of alkyl, aryl, -NR²₂, and -OR²; and pharmaceutically acceptable prodrugs and salts thereof

More preferred are compounds wherein G" is -S-. Most preferred are compounds wherein A², L², E², and J² are independently selected from the group consisting of -H, -NR⁴_{2'} -S-C≡N, halogen, -OR³, hydroxy, -alkyl(OH), aryl, alkyloxycarbonyl, -SR³, lower perhaloalkyl, and C1-C5 alkyl, or together L² and E² form an annulated cyclic group. More preferably A², L², E² and J² are independently selected from the group consisting of -H, -NR⁴₂, -S-C≡N, halogen, lower alkoxy, hydroxy, lower alkyl(hydroxy), lower aryl, and C1-C5 alkyl, or together L² and E² form an annulated cyclic group.

Most preferred A² groups include -NH₂, -H, halo, and C1-C5 alkyl.

Most preferred L² and E² groups are those independently selected from the group consisting of -H, -S-C≡N, lower alkoxy, C1-C5 alkyl, lower alkyl(hydroxy), lower aryl, and halogen or together L² and E² form an annulated cyclic group containing an additional 4 carbon atoms.

Most preferred J² groups include -H, and C1-C5 alkyl.

Preferred X² groups include -CF₂-, -CH₂-, -OC(O)- -OCH₂-, -SCH₂-, -NHCH₂-, and -N(C(O)CH₃)-CH₂-. More preferred are -OCH₂-, -SCH₂-, and -N(C(O)CH₃)-CH₂-, Most preferred is -OCH₂-.
One preferred aspect include compound wherein A² is selected from the group consisting of -H, -NH₂, -CH₃, -Cl, and -Br;
L² is -H, lower alkyl, halogen, lower alkyloxy, hydroxy, -alkenylene-OH, or together with E² forms a cyclic group including aryl, cyclic alkyl, heteroaryls, heterocyclic alkyl;
E² is selected from the groups consisting of H, lower alkyl, halogen, SCN, lower alkyloxycarbonyl, lower alkyloxy, or together with L² forms a cyclic group including aryl, cyclic alkyl, heteroaryl, or heterocyclic alkyl;
J² is selected from the groups consisting of H, halogen, and lower alkyl;
G" is -S-;
X² is -OCH₂-;
and pharmaceutically acceptable salts and prodrugs thereof. More preferred are such compounds wherein
R¹⁸ is selected from the group consisting of -H, methyl, and ethyl;
R¹² and R¹³ are independently selected from the group consisting of -H, methyl, i-propyl, i-butyl, and benzyl, or together are connected via 2-5 carbon atoms to form a cycloalkyl group;
R¹⁴ is -OR¹⁷;
R¹⁷ is selected from the group consisting of methyl, ethyl, propyl, t-butyl, and benzyl; and
R¹⁵ and R¹⁶ are independently selected from the group consisting of lower alkyl, and lower aralkyl, or together R¹⁵ and R¹⁶ are connected via 2-6 atoms, optionally including 1 heteroatom selected from the group consisting of O, and N.

Also more preferred are such compounds where A² is NH₂, L² is selected from the group consisting of-Et and -Cl, E² is selected from the group consisting of-SCN, -Et, and -Br, and J² is -H. Particularly preferred are such compounds wherein 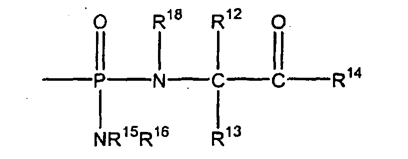 is selected from the group consisting of 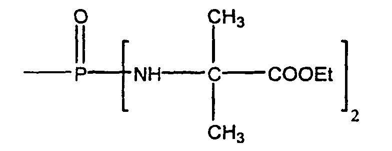 and 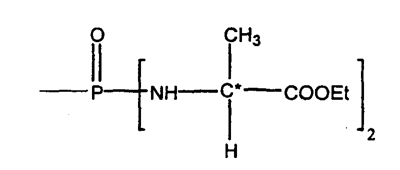 wherein C* has S stereochemistry.

Preferred R¹⁸ groups include -H, methyl, and ethyl. More preferred is -H and methyl. Especially preferred is -H.

Preferred compounds include those wherein each R¹² and R¹³ is independently selected from the group consisting of -H, methyl, ethyl, n-propyl, i-propyl, n-butyl, i-butyl, -CH₂CH₂-SCH₃, phenyl, and benzyl, or together R¹² and R¹³ are connected via 2-5 carbon atoms to form a cycloalkyl group. More preferred is each R¹² and R¹³ is independently selected from the group consisting of -H, methyl, i-propyl, i-butyl, and benzyl, or together R^{I2} and R¹³ are connected via 2-5 carbon atoms to form a cycloalkyl group. Also more preferred are such compounds wherein each R¹² and R¹³ is independently selected from the group consisting of -H, methyl, i-propyl, and benzyl, or together R¹² and R¹³ are connected via 4 carbon atoms to form a cyclopentyl group. Especially preferred are those compounds wherein R¹² and R¹³ are both -H, both methyl, or R¹² is H and R¹³ is selected from the group consisting of methyl, i-propyl, and benzyl. Most preferred are such compounds wherein n is 1, and R¹² is -H, then the carbon attached to R¹² and R¹³ has S stereochemistry.

Preferably, n is an integer of from 1-2. More preferred is when n is 1.

Preferred compounds include those wherein each R¹⁴ is independently selected from the group consisting of -OR¹⁷, and -SR¹⁷; and R¹⁷ is selected from the group consisting of optionally substituted methyl, ethyl, propyl, t-butyl, and benzyl. More preferred are such compounds wherein each R¹⁴ is independently selected from the group consisting of -OR¹⁷; and R¹⁷ is selected from the group consisting of methyl, ethyl, propyl, and benzyl. Most preferred are such compounds wherein R¹⁷ is selected from the group consisting of ethyl, and benzyl.

Preferred are compounds wherein R¹⁵ is not H. More preferred are compounds wherein R¹⁵ and R¹⁶ are independently selected from the group consisting of lower alkyl, and lower aralkyl, or together R¹⁵ and R¹⁶ are connected via 2-6 atoms, optionally including 1 heteroatom selected from the group consisting of O, N, and S. Also more preferred are compounds wherein R¹⁵ and R¹⁶ are independently selected from the group consisting of C1-C6 alkyl, or together R¹⁵ and R¹⁶ are connected via 2-6 atoms, optionally including 1 heteroatom selected from the group consisting of O, N, and S. In one aspect, particularly preferred are compounds wherein -NR¹⁵R¹⁶ is a cyclic amine. Especially preferred are such compounds wherein -NR¹⁵R¹⁶ is selected from the group consisting of morpholinyl and pyrrolidinyl.

Preferred are compounds R¹⁶ is -(CR¹²R¹³)ₙ-C(O)-R¹⁴.

More preferred are compounds where n is 1, and wherein
R¹⁸ is selected from the group consisting of -H, methyl, and ethyl;
R¹² and R¹³ are independently selected from the group consisting of -H, methyl, i-propyl, i-butyl, and benzyl, or together are connected via 2-5 carbon atoms to form a cycloalkyl group;
R¹⁴ is -OR¹⁷;
R¹⁷ is selected from the group consisting of methyl, ethyl, propyl, t-butyl, and benzyl; and
R¹⁵ and R¹⁶ are independently selected from the group consisting of lower alkyl, and lower aralkyl, or together R¹⁵ and R¹⁶ are connected via 2-6 atoms, optionally including 1 heteroatom selected from the group consisting of O, and N. Particularly preferred are such compounds that are of the formula:

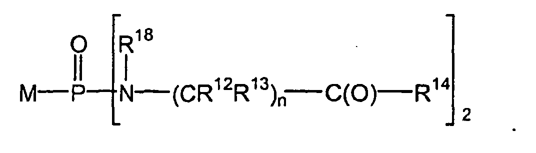

More preferred are such compounds wherein n is 1. Especially preferred are such compounds wherein when R¹² and R¹³ are not the same, then H₂N-CR¹²R¹³-C(O)-R¹⁴ is an ester, or thioester of a naturally occurring amino acid; and R¹⁴ is selected from the group consisting of -OR¹⁷ and -SR¹⁷.

In one aspect, preferred are compounds of formula IA or formula I wherein M is 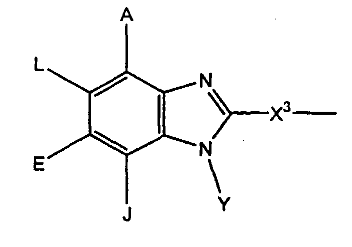 wherein:
A, E, and L are selected from the group consisting of -NR⁸₂, -NO₂, -H, -OR⁷, -SR⁷, -C(O)NR⁴₂, halo, -COR¹¹, -SO₂R³, guanidine, amidine, -NHSO₂R⁵, -SO₂NR⁴₂, -CN, sulfoxide, perhaloacyl, perhaloalkyl, perhaloalkoxy, C1-C5 alkyl, C2-C5 alkenyl, C2-C5 alkynyl, and lower alicyclic, or together A and L form a cyclic group, or together L and E form a cyclic group, or together E and J form a cyclic group including aryl, cyclic alkyl, and heterocyclic;
J is selected from the group consisting of -NR⁸₂, -NO₂, -H, -OR⁷, -SR⁷, -C(O)NR⁴₂, halo, -C(O)R¹¹, -CN, sulfonyl, sulfoxide, perhaloalkyl, hydroxyalkyl, perhaloalkoxy, alkyl, haloalkyl, aminoalkyl, alkenyl, alkynyl, alicyclic, aryl, and aralkyl, or together with Y forms a cyclic group including aryl, cyclic alkyl and heterocyclic alkyl;
X³ is selected from the group consisting of -alkyl(hydroxy)-, -alkyl-, -alkynyl-, -aryl-, -carbonylalkyl-, -1,1-dihaloalkyl-, -alkoxyalkyl-, -alkyloxy-, -alkylthioalkyl-, -alkylthio-, -alkylaminocarbonyl-, -alkylcarbonylamino-, -alicyclic-, -aralkyl-, -alkylaryl-, -alkoxycarbonyl-, -carbonyloxyalkyl-, -alkoxycarbonylamino-, and -alkylaminocarbonylamino-, all optionally substituted; with the proviso that X³ is not substituted with -COOR², -SO₃H, or -PO₃R²₂;
Y³ is selected from the group consisting of -H, alkyl, alkenyl, alkynyl, aryl, alicyclic, aralkyl, aryloxyalkyl, alkoxyalkyl, -C(O)R³, -S(O)₂R³, -C(O)-R¹¹, -CONHR³, -NR²₂, and -OR³, all except H are optionally substituted;
R² is selected from the group consisting of R³ and -H;
R³ is selected from the group consisting of alkyl, aryl, alicyclic, and aralkyl;
each R⁴ is independently selected from the group consisting of -H, and alkyl, or together R⁴ and R⁴ form a cyclic alkyl group;
R⁵ is selected from the group consisting of lower alkyl, lower aryl, lower aralkyl, and lower alicyclic;
R⁷ is independently selected from the group consisting of -H, lower alkyl, lower alicyclic, lower aralkyl, lower aryl, and -C(O)R¹⁰;
R⁸ is independently selected from the group consisting of -H, lower alkyl, lower aralkyl, lower aryl, lower alicyclic, -C(O)R¹⁰, or together they form a bidendate alkyl;
each R⁹ is independently selected from the group consisting of -H, alkyl, aralkyl, and alicyclic, or together R⁹ and R⁹ form a cyclic alkyl group;
R¹⁰ is selected from the group consisting of -H, lower alkyl, -NH₂, lower aryl, and lower perhaloalkyl;
R¹¹ is selected from the group consisting of alkyl, aryl, -NR²₂, and -OR²; and pharmaceutically acceptable prodrugs and salts thereof; with the provisos that:
   a) when X³ is alkyl or alkene, then A is -N(R⁸₂);
   b) X³ is not alkylamine and alkylaminoalkyl substituted with phosphonic esters and acids; and
   c) A, L, E, J, and Y³ together may only form 0-2 cyclic groups.

More preferred are such compounds wherein X³ is not -alkoxyalkyl-, -alkyloxy-, -alkythioalkyl-, and -alkylthio-. Particularly preferred are such compounds with the additional proviso that when X³ is aryl or alkylaryl, said aryl or alkylaryl group is not linked 1,4 through a six-membered aromatic ring.

Especially preferred benzimidazole compounds include those wherein A, L, and E are independently selected from the group consisting of -H, -NR⁸₂, -NO₂, hydroxy, halogen, -OR⁷, alkylaminocarbonyl, -SR⁷, lower perhaloalkyl, and C1-C5 alkyl, or together E and J together form a cyclic group; and wherein J is selected from the group consisting of -H, halogen, lower alkyl, lower hydroxyalkyl, -NR⁸₂, lower R⁸₂N-alkyl, lower haloalkyl, lower perhaloalkyl, lower alkenyl, lower alkynyl, lower aryl, heterocyclic, and alicyclic; and wherein Y is selected from the group consisting of alicyclic and lower alkyl; wherein X³ is selected from the group consisting of-heteroaryl-, -alkylcarbonylamino-, -alkylaminocarbonyl-, and -alkoxycarbonyl-. More preferred are such compounds wherein
R¹⁸ is selected from the group consisting of -H, methyl, and ethyl;
R¹² and R¹³ are independently selected from the group consisting of -H, methyl, i-propyl, i-butyl, and benzyl, or together are connected via 2-5 carbon atoms to form a cycloalkyl group;
R¹⁴ is -OR¹⁷;
R¹⁷ is selected from the group consisting of methyl, ethyl, propyl, t-butyl, and benzyl; and
R¹⁵ and R¹⁶ are independently selected from the group consisting of lower alkyl, and lower aralkyl, or together R¹⁵ and R¹⁶ are connected via 2-6 atoms, optionally including 1 heteroatom selected from the group consisting of O, and N. Most preferred are such compounds wherein A is selected from the group consisting of -H, -NH₂, -F, and -CH₃;
L is selected from the group consisting of -H, -F, -OCH₃, Cl and -CH₃;
E is selected from the group consisting of -H, and -Cl;
J is selected from the group consisting of -H, halo, C1-C5 hydroxyalkyl, C1-C5 haloalkyl, C1-C5 R⁸₂ N-alkyl, C1-C5 alicyclic, and C1-C5 alkyl;
X³ is selected from the group consisting of -CH₂OCH₂-, -methyleneoxycarbonyl-, and -furan-2,5-diyl-; and
Y is lower alkyl.

Also more preferred are such benzimidazoles where A is -NH₂, L is -F, E is -H, J is ethyl, Y is isobutyl, and X³ is -furan-2,5-diyl-; or
where A is -NH₂, L is -F, E is -H, J is N,N-dimethylaminopropyl, Y is isobutyl, and X³ is -furan-2,5-diyl-.

Particularly preferred are those compounds wherein 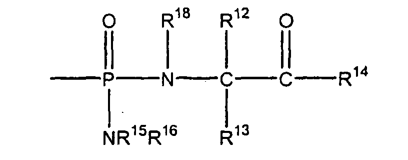 is selected from the group consisting of 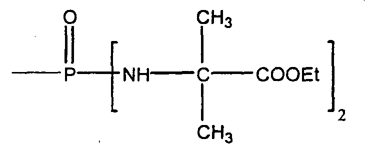 and 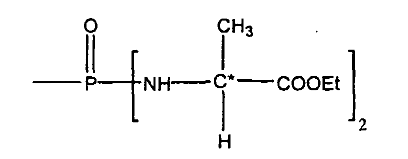 wherein C* has S stereochemistry.
Preferably, oral bioavailability is at least 5%. More preferably, oral bioavailability is at least 10%.

The prodrugs of formula IA may have two isomeric forms around the phosphorus. Preferred is when the phosphorus is not chiral. Also preferred is when there is no chiral center in the amino groups attached to the phosphorus. Also preferred is when n is 1 and R¹² is -H, then the carbon attached to R¹² and R¹³ has S stereochemistry.

In one aspect, preferred are compounds of formula X wherein A² is selected from the group consisting of -H, -NH₂, -CH₃, -Cl, and -Br;

L² is -H, lower alkyl, halogen, lower alkyloxy, hydroxy, -alkenylene-OH, or together with E² forms a cyclic group including aryl, cyclic alkyl, heteroaryls, heterocyclic alkyl;
E² is selected from the groups consisting of H, lower alkyl, halogen, SCN, lower alkyloxycarbonyl; lower alkyloxy, or together with L² forms a cyclic group including aryl, cyclic alkyl, heteroaryl, or heterocyclic alkyl;
J² is selected from the groups consisting of H, halogen, and lower alkyl;
G" is -S-;
X² is -OCH₂-; and
at least one Y group is -O-; and pharmaceutically acceptable salts and prodrugs thereof. Also particularly preferred are such compounds where A² is NH₂, G" is -S-, L² is Et, E² is SCN, and J² is H. More preferred are such compounds wherein one Y is -O- and its corresponding R¹ is optionally substituted phenyl, while the other Y is -NH-, and its corresponding R¹ is -C(R²)₂-COOR³. When R¹ is -CHR³COOR³, then the corresponding -NR⁶-*CHR³COOR³, preferably has L stereochemistry.

Also more preferred are such compounds wherein one Y is -O-, and its corresponding R¹ is -phenyl, while the other Y is -NH- and its corresponding R¹ is -CH(Me)CO₂Et.

In compounds of formula I, II, III, IV, V-1, V-2, VI, or X, preferably both Y groups are -O-; or one Y is -O- and one Y is -NR⁶-. When only one Y is -NR⁶-, preferably the Y closest to W and W' is -O-. Most preferred are prodrugs where both Y groups are - O-;

In another particularly preferred aspect, both Y groups are -O-, and R¹ and R¹ together are 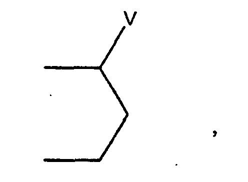 and V is phenyl substituted with 1-3 halogens. Especially preferred are such 3-bromo-4-fluorophenyl, 3-chlorophenyl, 3-bromophenyl, and 3,5-dichlorophenyl.

In another particularly preferred aspect, one Y is -O- and its corresponding R¹ is phenyl, or phenyl substituted with 1-2 substituents selected from -NHC(O)CH₃, -F, -Cl, - Br, -C(O)OCH₂CH₃, and -CH₃; while the other Y is -NR⁶- and its corresponding R¹ is -C(R²)COOR³; each R² is independently selected from -H, -CH₃, and -CH₂CH₃. More preferred R⁶ is -H, and R¹ attached to -NH- is -CH(Me)CO₂Et.

In general, preferred substituents, V, Z, W, and W' of formulae I, II, III, IV, V-1, V-2, VI or X are chosen such that they exhibit one or more of the following properties:
(1) enhance the oxidation reaction since this reaction is likely to be the rate determining step and therefore must compete with drug elimination processes.
(2) enhance stability in aqueous solution and in the presence of other non-p450 enzymes;
(3) enhance cell penetration, *e.g.* substituents are not charged or of high molecular weight since both properties can limit oral bioavailability as well as cell penetration;
(4) promote the β-elimination reaction following the initial oxidation by producing ring-opened products that have one or more of the following properties:
   a) fail to recyclize;
   b) undergo limited covalent hydration;
   c) promote β-elimination by assisting in the proton abstraction;
   d) impede addition reactions that form stable adducts, e.g. thiols to the initial hydroxylated product or nucleophilic addition to the carbonyl generated after ring opening; and
   e) limit metabolism of reaction intermediates (e.g. ring-opened ketone);
(5) lead to a non-toxic and non-mutagenic by-product with one or more of the following characteristics. Both properties can be minimized by using substituents that limit Michael additions, reactions, *e.g.*
   a) electron donating Z groups that decrease double bond polarization;
   b) W groups that sterically block nucleophilic addition to β-carbon;
   c) Z groups that eliminate the double bond after the elimination reaction either through retautomerization (enol->keto) or hydrolysis (e.g. enamine);
   d) V groups that contain groups that add to the α,β-unsaturated ketone to form a ring;
   e) Z groups that form a stable ring via Michael addition to double bond; and
   f) groups that enhance detoxification of the by-product by one or more of the following characteristics:
      (i) confine to liver; and
      (ii) make susceptible to detoxification reactions (e.g. ketone reduction); and
(6) capable of generating a pharmacologically active product.

In another aspect, it is preferred when Y is -O-, then R¹ attached to -O- is independently selected from the group consisting of -H, optionally substituted aryl, optionally substituted alicyclic where the cyclic moiety contains a carbonate or thiocarbonate, optionally substituted -alkylaryl, -C(R²)₂OC(O)R³, -C(R²)₂-O-C(O)OR³, - C(R²)₂OC(O)SR³, -alkyl-S-C(O)R³, and -alkyl-S-S-alkylhydroxy;
when Y is -NR⁶-, then R¹ attached to -NR⁶- is independently selected from the group consisting of -H, -[C(R²)₂]_{q}-COOR³, -[C(R²)₂]_{q} -C (O)SR³, -C(R⁴)₂COOR³, and -cycloalkylene-COOR³;
or when either Y is independently selected from -O- and -NR⁶-, then together R¹ and R¹ are 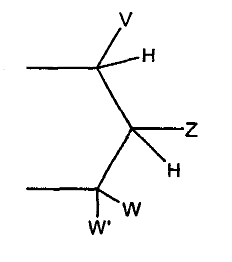
wherein
V, W, and W' are independently selected from the group consisting of -H, alkyl, aralkyl, alicyclic, aryl, substituted aryl, heteroaryl, substituted heteroaryl, 1-alkenyl, and 1-alkynyl, or
together V and W are connected via an additional 3 carbon atoms to form an optionally substituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said carbon atoms that is three atoms from a Y attached to the phosphorus;
together Z and W are connected via an additional 3-5 atoms to form a cyclic group, optionally containing one heteroatom, and V must be aryl, substituted aryl, heteroaryl, or substituted heteroaryl;
together W and W' are connected via an additional 2-5 atoms to form a cyclic group, optionally containing 0-2 heteroatoms, and V must be aryl, substituted aryl, heteroaryl, or substituted heteroaryl;
Z is selected from the group consisting of -CHR²OH , -CHR²OC(O)R³, -CHR²OC(S)R³, -CHR²OC(S)OR³, -CHR²OC(O)SR³, -CHR²OCO₂R³, -OR², -SR², -R², -NHCOR², -NHCO₂R³, -(CH₂)ₚ-OR², and -(CH₂)ₚ-SR²;
p is an integer 2 or 3;
q is an integer 1 or 2;
with the provisos that:
   a) V, Z, W, W' are not all -H;
   b) when Z is -R², then at least one of V, W, and W' is not -H, alkyl, aralkyl, or alicyclic; and
   c) both Y groups are not -NR⁶-;
R² is selected from the group consisting of R³ and -H;
R³ is selected from the group consisting of alkyl, aryl, alicyclic, and aralkyl;
R⁶ is selected from the group consisting of -H, and lower alkyl.

More preferred are such compounds wherein when both Y groups are -O-, then R¹ is independently selected from the group consisting of optionally substituted aryl, optionally substituted benzyl, -C(R²)₂OC(O)R³, -C(R²)₂OC(O)OR³, and -H; and
when Y is -NR⁶-, then the R¹ attached to said -NR⁶- group is selected from the group consisting of -C(R⁴)₂-COOR³, and -C(R²)₂COOR³; and the other Y group is -O- and then R¹ attached to said -O- is selected from the group consisting of optionally substituted aryl,
-C(R²)₂OC(O)R³, and -C(R²)₂OC(O)OR³.

In another aspect, when one Y is -O-, then its corresponding R¹ is phenyl, and the other Y is -NH-, and its corresponding R¹ is -CH₂CO₂Et.

In another preferred aspect, when one Y is -O-, its corresponding R¹ is phenyl, and the other Y is -NH- and its corresponding R¹ is -C(Me)₂CO₂Et.

In another preferred aspect, when one Y is -O-, its corresponding R¹ is 4-NHC(O)CH₃-phenyl, and the other Y is -NH-, and its corresponding R¹ is -CH₂COOEt.

In another preferred aspect, when one Y is -O-, its corresponding R¹ is 2-CO₂Et-phenyl, and the other Y is -NH- and its corresponding R¹ is -CH₂CO₂Et.

In another preferred aspect, when one Y is -O-, then its corresponding R¹ is 2-CH₃-phenyl, and the other Y is -NH, and its corresponding, R¹ is -CH₂CO₂Et.

In another aspect, preferred are compounds wherein both Y groups are -O-, and R¹ is aryl, or -C(R²)₂-aryl.

Also preferred are compounds wherein both Y groups are O-, and at least one R¹ is selected from the group consisting of -C(R²)₂-OC(O)R³, and -C(R²)₂-OC(O)OR³.

In another aspect, preferred are compounds wherein both Y groups are -O- and at least one R¹ is -alkyl-S-S-alkylhydroxyl, -alkyl-S-C(O)R³, and -alkyl-S-S-S-alkylhydroxy, or together R¹ and R¹ are -alkyl-S-S-alkyl- to form a cyclic group.

In one aspect, particularly preferred are compounds wherein both Y groups are -O-, and R¹ is H.

In another aspect, particularly preferred are compounds where both Y groups are -O-, and R¹ is -CH₂OC(O)OEt.

More preferred are compounds wherein at least one Y is -O-, and together R¹ and R¹ are 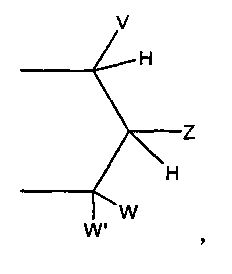 wherein
V, W, and W' are independently selected from the group consisting of -H, alkyl, aralkyl, alicyclic, aryl, substituted aryl, heteroaryl, substituted heteroaryl, 1-alkenyl, and 1-alkynyl, or
together V and W are connected via an additional 3 carbon atoms to form an optionally substituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said carbon atoms that is three atoms from a Y attached to the phosphorus;
together Z and W are connected via an additional 3-5 atoms to form a cyclic group, optionally containing one heteroatom, and V must be aryl, substituted aryl, heteroaryl, or substituted heteroaryl;
together W and W' are connected via an additional 2-5 atoms to form a cyclic group, optionally containing 0-2 heteroatoms, and V must be aryl, substituted aryl, heteroaryl, or substituted heteroaryl;
Z is selected from the group consisting of -CHR²OH , -CHR²OC(O)R³, -CHR²OC(S)R³, -CHR²OC(S)OR³, -CHR²OC(O)SR³, -CHR²OCO₂R³, -OR², -SR², -R², -NHCOR², -NHCO₂R³, -(CH₂)ₚ-OR², and -(CH₂)ₚ-SR²;
p is an integer 2 or 3;
with the provisos that:
   a) V, Z, W, W' are not all -H;
   b) when Z is -R², then at least one of V, W, and W' is not -H, alkyl, aralkyl, or alicyclic; and
   c) both Y groups are not -NR⁶-;
R² is selected from the group consisting of R³ and -H;
R³ is selected from the group consisting of alkyl, aryl, alicyclic, and aralkyl;
R⁶ is selected from the group consisting of -H, and lower alkyl.

In an other aspect, more preferred are compounds wherein one Y is -O-, and R¹ is optionally substituted aryl; and the other Y is -NR⁶-, where R¹ on said -NR⁶- is selected from the group consisting of -C(R⁴)₂COOR³, and -C(R²)₂C(O)OR³. Particularly preferred are such compounds where R¹ attached to -O- is -phenyl, and R1 to -NH- is - CH(Me)CO₂Et, and
-NH*CH(Me)CO₂Et is in the L configuration.

Especially preferred are such compounds where R¹ attached to -O- is selected from the group consisting of phenyl and phenyl substituted with 1-2 substituents selected from the group consisting of -NHAc, -F, -Cl, -Br, -COOEt, and -CH₃; and R¹ attached to -NR⁶, is -C(R²)₂COOR³ where R² and R³ independently is -H, -CH₃, and -Et. Of such compounds, when R¹ attached to -O- is phenyl substituted with -NHAc or -COOEt, then preferably any -NHAc is at the 4-position, and any -COOEt is at the 2-position. More preferred are such compounds where the substituents on the substituted phenyl is 4-NHC(O)CH₃, -Cl, -Br, 2-C(O)OCH₃CH₃, or -CH₃.

More preferred V groups of formula 6-i are aryl, substituted aryl, heteroaryl, and substituted heteoaryl. Preferably Y is -O-. Particularly preferred aryl and substituted aryl groups include phenyl, and phenyl substituted with 1-3 halogens. Especially preferred are 3,5-dichlorophenyl, 3-bromo-4-fluorophenyl, 3-chlorophenyl, and 3-bromophenyl.

It is also especially preferred when V is selected from the group consisting of monocyclic heteroaryl and monocyclic substituted heteroaryl containing at least one nitrogen atom. Most preferred is when such heteroaryl and substituted heteroaryl is 4-pyridyl, and 3-bromopyridyl, respectively.

It is also preferred when together V and Z are connected via an additional 3-5 atoms to form a cyclic group, optionally containing 1 heteroatom, that is fused to an aryl group at the beta and gamma positions to the Y attached to phosphorus. In such compounds preferably said aryl group is an optionally substituted monocyclic aryl group and the connection between Z and the gamma position of the aryl group is selected from the group consisting of O, CH₂, CH₂CH₂, OCH₂ or CH₂O.

In another aspect, it is preferred when together V and W are connected via an additional 3 carbon atoms to form an optionally substituted cyclic group containing 6 carbon atoms and monosubstituted with one substituent selected from the group consisting ofhydroxy, acyloxy, alkosycarbonyloxy, alkylthiocarbonyloxy, and aryloxycarbonyloxy attached to one of said additional carbon atoms that is three atoms from a Y attached to the phosphorus. In such compounds, it is more preferred when together V and W form a cyclic group selected from the group consisting of -CH₂-CH(OH)-CH₂-, CH₂CH(OCOR³)-CH₂-, and -CH₂CH(OCO₂)R³)-CH₂-.

Another preferred V group is 1-alkene. Oxidation by p450 enzymes is known to occur at benzylic and allylic carbons.

In one aspect, a preferred V group is -H, when Z is selected from the group consisting of -CHR²OH, -CHR²OCOR³, and -CHR²OCO₂R³.

In another aspect, when V is aryl, substituted aryl, heteroaryl, or substituted heteroaryl, preferred Z groups include-OR², -SR², -CHR²N₃, -R², -NR²₂, -OCOR², -OCO₂R³, -SCOR³, -SCO₂R³, -NHCOR², -NHCO₂R³, -CH₂NHaryl, -(CH₂)ₚOR², and -(CH₂)ₚ-SR². More preferred Z groups include-OR², -R², -OCOR², -OCO₂R³, -CH₃, -NHCOR², -NHCO₂R³, -(CH₂)ₚ-OR², and, -(CH₂)ₚ-SR². Most preferred Z groups include -OR², -H , -OCOR², -OCO₂R³, and -NHCOR².

Preferred W and W' groups include H, R³, aryl, substituted aryl, heteroaryl, and substituted aryl. Preferably, W and W' are the same group. More preferred is when W and W' are H.

In one aspect, prodrugs of formula 6-i are preferred: 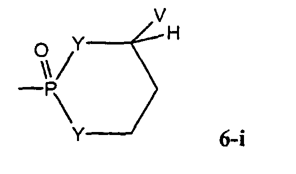 wherein
V is selected from the group consisting of aryl, substituted aryl, heteroaryl, and substituted heteroaryl, 1-alkenyl, and 1-alkynyl. More preferred V groups of formula VI are aryl, substituted, heteroaryl, and substituted heteoaryl. Preferably Y is -O-. Particularly preferred aryl and substituted aryl groups include phenyl and substituted phenyl. Particularly preferred heteroaryl groups include monocyclic substiutted and unsubstituted heteroaryl groups. Especially preferred are 4-pyridyl and 3-bromopyridyl.

In one aspect, the compounds of formula VI preferably have a group Z which is H, alkyl, alicyclic, hydroxy, alkoxy, 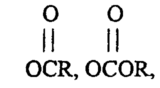 or NHCOR. Preferred are such groups in which Z decreases the propensity of the byproduct, vinyl aryl ketone to undergo Michael additions. Preferred Z groups are groups that donate electrons to the vinyl group which is a known strategy for decreasing the propensity of α,β-unsaturated carbonyl compounds to undergo a Michael addition. For example, a methyl group in a similar position on acrylamide results in no mutagenic activity whereas the unsubstituted vinyl analog is highly mutagenic. Other groups could serve a similar functiori, e.g. Z=OR, NHAc, etc. Other groups may also prevent the Michael addition especially groups that result in removal of the double bond altogether such as Z = OH, -OC(O)R, -OCO₂R, and NH₂, which will rapidly undergo retautomerization after the elimination reaction. Certain W and W' groups are also advantageous in this role since the group(s) impede the addition reaction to the β-carbon or destabilize the product. Another preferred Z group is one that contains a nucleophilic group capable of adding to the α,β-unsaturated double bond after the elimination reaction *i.e.* (CH₂)ₚSH or (CH₂)ₙOH where p is 2 or 3. Yet another preferred group is a group attached to V which is capable of adding to the α,β-unsaturated double bond after the elimination reaction:

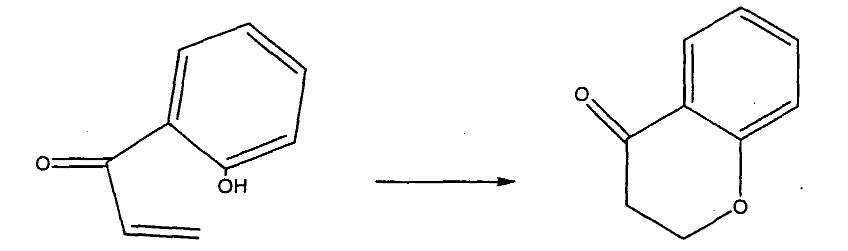

In another aspect, prodrugs of formula 7-i are preferred: 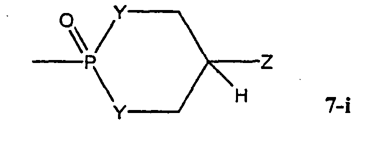 wherein
Z is selected from the group consisting of: -CHR²OH, -CHR²OCOR³, -CHR²OC(S)R³, -CHR²OCO₂R³, -CHR²OC(O)SR³, and -CHR²OC(S)OR³. Preferably Y is -O-. More preferred groups include -CHR²OH, -CHR²OC(O)R³, and -CHR²OCO₂R³.

In another aspect, prodrugs of formula 8-i are preferred: 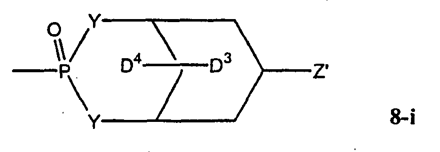 wherein
Z' is selected from the group consisting of -OH, -OC(O)R³, -OCO₂ R³, and -OC(O)S R³;
D⁴ and D³ are independently selected from the group consisting of -H, alkyl, OR², -OH, and -OC(O)R³; with the proviso that at least one of D⁴ and D³ are -H. Preferably Y is -O-.

In one preferred embodiment, W' and Z are -H, W and V are both the same aryl, substituted aryl, heteroaryl, or substituted heteroaryl such that the phosphonate prodrug moiety: 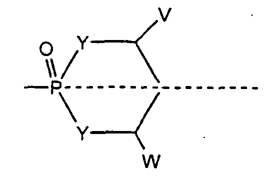 has a plane of symmetry. Preferably Y is -O-.

In another preferred embodiment, W and W' are H, V is selected from the group consisting of aryl, substituted aryl, heteroaryl, substituted heteroaryl, and Z is selected from the group consisting of-H, OR², and -NHCOR². More preferred are such compounds where Z is -H.

p450 oxidation can be sensitive to stereochemistry which might either be at phosphorus or at the carbon bearing the aromatic group. The prodrugs of the present invention have two isomeric forms around the phosphorus. Preferred is the stereochemistry that enables both oxidation and the elimination reaction. Preferred is the cis-stereochemistry.

The preferred compounds of formula 8-i utilize a Z' group that is capable of undergoing an oxidative reaction that yields an unstable intermediate which via elimination reactions breaks down to the corresponding R⁵-X-PO₃²⁻, R⁵-X-P(O)(NHR⁶)₂, or R⁵-X-P(O)(O⁻)(NHR⁶). Especially preferred Z' groups is OH. Groups D⁴ and D³ are preferably hydrogen, alkyl, and -OR², -OC(O)R³, but at least one of D⁴ or D³ must be H.

The following prodrugs of formulae I, II, III, IV, V-1, V-2, VI, and X are preferred:
Acyloxyalkyl esters;
Alkoxycarbonyloxyalkyl esters;
Aryl esters;
Benzyl and substituted benzyl esters;
Disulfide containing esters;
Substituted (1,3-dioxolen-2-one)methyl esters;
Substituted 3-phthalidyl esters;
Cyclic-[5-hydroxycyclohexan-1,3-diyl) diesters and hydroxy protected forms;
Cyclic-[2-hydroxymethylpropan-1,3-diyl] diesters and hydroxy protected forms;
Cyclic-(1-arylpropan-1,3-diyl);
Bis Omega substituted lactone esters; and all mixed esters resulted from possible combinations of above esters;

More preferred are the following:
Bis-pivaloyloxymethyl esters;
Bis-isobutyryloxymethyl esters;
Cyclic-[2-hydroxymethylpropan-1,3-diyl] diester;
Cyclic-[2-acetoxymethylpropan-1,3-diyl] diester;
Cyclic-[2-methyloxycarbonyloxymethylpropan-1,3-diyl] diester;
Cyclic-[1-phenylpropan-1,3-diyl] diesters;
Cyclic-[1-(2-pyridyl)propan-1,3-diyl)] diesters;
Cyclic-[1-(3-pyridyl)propan-1,3-diyl] diesters;
Cyclic-[1-(4-pyridyl)propan-1,3-diyl] diesters;
Cyclic-[5-hydroxycyclohexan-1,3-diyl] diesters and hydroxy protected forms;
Bis-benzoylthiomethyl esters;
Bis-benzoylthioethyl esters;
Bis-benzoyloxymethyl esters;
Bis-*p*-fluorobenzoyloxymethyl esters;
Bis-6-chloronicotinoyloxymethyl esters;
Bis-5-bromonicotinoyloxymethyl esters;
Bis-thiophenecarbonyloxymethyl esters;
Bis-2-furoyloxymethyl esters;
Bis-3-furoyloxymethyl esters;
Diphenyl esters;
Bis-(4-methoxyphenyl) esters;
Bis-(2-methoxyphenyl) esters;
Bis-(2-ethoxyphenyl) esters;
Mono-(2-ethoxyphenyl) esters;
Bis-(4-acetamidophenyl) esters;
Bis-(4-acetoxyphenyl) esters;
Bis-(4-hydroxyphenyl) esters;
Bis-(2-acetoxyphenyl) esters;
Bis-(3-acetoxyphenyl) esters;
Bis-(4-morpholinophenyl) esters;
Bis-[4-(1-triazolophenyl) esters;
Bis-(3-*N*,*N*-dimethylaminophenyl) esters;
Bis-(1,2,3,4-tetrahydronapthalen-2-yl) esters;
Bis-(3-chloro-4-methoxy)benzyl esters;
Bis-(3-bromo-4-methoxy)benzyl esters;
Bis-(3-cyano-4-methoxy)benzyl esters;
Bis-(3-chloro-4-acetoxy)benzyl esters;
Bis-(3-bromo-4-acetoxy)benzyl esters;
Bis-(3-cyano-4-acetoxy)benzyl esters;
Bis-(4-chloro)benzyl esters;
Bis-(4-acetoxy)benzyl esters;
Bis-(3,5-dimethoxy-4-acetoxy)benzyl esters;
Bis-(3-methyl-4-acetoxy)benzyl esters;
Bis-(benzyl)esters;
Bis-(3-methoxy-4-acetoxy)benzyl esters;
Bis-(6'-hydroxy-3',4'-dithia)hexyl esters;
Bis-(6'-acetoxy-3',4'-dithia)hexyl esters;
(3,4-dithiahexan-1,6-diyl) esters;
Bis-(5-methyl-1,3-dioxolen-2-one-4-yl)methyl esters;
Bis-(5-ethyl-1,3-dioxolen-2-one-4-yl)methyl esters;
Bis-(5-tert-butyl-1,3-dioxolen-2-one-4-yl)methyl esters;
Bis-3-(5,6,7-trimethoxy)phthalidyl esters;
Bis-(cyclohexyloxycarbonyloxymethyl) esters;
Bis-(isopropyloxycarbonyloxymethyl) esters;
Bis-(ethyloxycarbonyloxymethyl) esters;
Bis-(methyloxycarbonyloxymethyl) esters;
Bis-(isopropylthiocarbonyloxymethyl) esters;
Bis-(phenyloxycarbonyloxymethyl) esters;
Bis-(benzyloxycarbonyloxymethyl) esters;
Bis-(phenylthiocarbonyloxymethyl) esters;
Bis-(*p*-methoxyphenoxycarbonyloxymethyl) esters;
Bis-(*m*-methoxyphenoxycarbonyloxymethyl) esters;
Bis-(*o*-methoxyphenoxycarbonyloxymethyl) esters;
Bis-(*o*-methylphenoxycarbonyloxymethyl) esters;
Bis-(*p*-chlorophenoxycarbonyloxymethyl) esters;
Bis-(1,4-biphenoxycarbonyloxymethyl) esters;
Bis-[(2-phthalimidoethyl)oxycarbonyloxymethyl]esters;
Bis-(*N*-phenyl-*N*-methylcarbamoyloxymethyl) esters;
Bis-(2,2,2-trichloroethyl) esters;
Bis-(2-bromoethyl) esters;
Bis-(2-iodoethyl) esters;
Bis-(2-azidoethyl) esters;
Bis-(2-acetoxyethyl) esters;
Bis-(2-aminoethyl) esters;
Bis-(2-*N*,*N*-dimethylaminoethyl) esters;
Bis-(2-aminoethyl) esters;
Bis-(methoxycarbonylmethyl) esters;
Bis-(2-aminoethyl) esters;
Bis-[*N*,*N*-di(2-hydroxyethyl)]carbamoylmethylesters;
Bis-(2-aminoethyl) esters;
Bis-(2-methyl-5-thiazolomethyl) esters;
Bis-(bis-2-hydroxyethylcarbamoylmethyl) esters.
O-phenyl-[N-(1-ethoxycarbonyl)ethyl]phosphoramidates (-P(O)(OPh)(N(H)-CH(Me)CO₂Et)
O-phenyl-[N-(1-methoxycarbonyl)ethyl]phosphoramidates (-P(O)(OPh)(N(H)-CH(Me)CO₂Me)
O-(3-chlorophenyl)-[N-(1-ethoxycarbonyl)ethyl]phosphoramidates (-P(O)(OPh-3-Cl)(NH-CH(Me)CO₂Et)
O-(2-chlorophenyl)-[N-(1-ethoxycarbonyl)ethyl]phosphoramidates (-P(O)(OPh-2-Cl)(NH-CH(Me)CO₂Et)
O-(4-chlorophenyl)-[N-(1-ethoxycarbonyl)ethyl]phosphoramidates (-P(O)(OPh-4-Cl)(NH-CH(Me)CO₂Et)
O-(4-acetamidophenyl)-[N-(1-ethoxycarbonyl)ethyl]phosphoramidates (-P(O)(OPh-4-NHAc)(NH-CH(Me)CO₂Et)
O-(2-ethoxycarbonylphenyl)-[N-(1-ethoxycarbonyl)ethyl]phosphoramidates (-P(O)(OPh-2-CO₂Et)(NH-CH(Me)CO₂Et)
O-phenyl-[N-(1-ethoxycarbonyl-1-methyl)ethyl]phosphoramidates (-P(O)(OPh)(NH-C(Me)₂CO₂Et)
O-phenyl-[N-(1-methoxycarbonyl-1-methyl)ethyl]phosphoramidates (-P(O)(OPh)(NH-C(Me)₂CO₂Me)
O-(3-chlorophenyl)-[N-(1-ethoxycarbonyl-1-methyl)ethyl]phosphoramidates (-P(O)(OPh-3-Cl)(NH-C(Me)₂CO₂Et)
O-(2-chlorophenyl)-[N-(1-ethoxycarbonyl-1-methyl)ethyl]phosphoramidates (-P(O)(OPh-2-Cl)(NH-C(Me)₂CO₂Et)
O-(4-chlorophenyl)-[N-(1-ethoxycarbonyl-1-methyl)ethyl]phosphoramidates (-P(O)(OPh-4-Cl)(NH-C(Me)₂CO₂Et)
O-(4-acetamidophenyl)-[N-(1-ethoxycarbonyl-1-methyl)ethyl]phosphoramidates (-P(O)(OPh-4-NHAc)(NH-C(Me)₂CO₂Et)
O-(2-ethoxycarbonylphenyl)-[N-(1-ethoxycarbonyl-1-methyl)ethyl]phosphoramidates (-P(O)(OPh-2-CO₂Et)(NH-C(Me)₂CO₂Et)
O-phenyl-[N-(ethoxycarbonyl)methyl]phosphoramidates (-P(O)(OPh)(NH-CH₂CO₂Et)
O-phenyl-[N-(methoxycarbonyl)methyl]phosphoramidates (-P(O)(OPh)(NH-CH₂CO₂Me)
O-(3-chlorophenyl)-[N-(ethoxycarbonyl)methyl]phosphoramidates (-P(O)(OPh-3-Cl)(NH-CH₂CO₂Et)
O-(2-chlorophenyl)-[N-(ethoxycarbonyl)methyl]phosphoramidates (-P(O)(OPh-2-Cl)(NH-CH₂CO₂Et)
O-(4-chlorophenyl)-[N-(ethoxycarbonyl)methyl]phosphoramidates (-P(O)(OPh-4-Cl)(NH-CH₂CO₂Et)
O-(4-acetamidophenyl)-[N-(ethoxycarbonyl)methyl]phosphoramidates (-P(O)(OPh-4-NHAc)(NH-CH₂CO₂Et)
O-(2-ethoxycarbonylphenyl)-[N-(ethoxycarbonyl)methyl]phosphoramidates (-P(O)(OPh-2-CO₂Et)(NH-CH₂CO₂Et)

Most preferred are the following:
Bis-pivaloyloxymethyl esters;
Bis-isobutyryloxymethyl esters;
Cyclic-(2-hydroxymethylpropan-1,3-diyl) ester;
Cyclic-(2-acetoxymethylpropan-1,3-diyl) ester;
Cyclic-(2-methyloxycarbonyloxymethylpropan-1,3-diyl) ester;
Cyclic-(2-cyclohexylcarbonyloxymethylpropan-1,3-diyl) ester;
Cyclic-[phenylpropan-1,3-diyl] diesters;
Cyclic-[1-(2-pyridyl)propan-1,3-diyl)] diesters;
Cyclic-[1-(3-pyridyl)propan-1,3-diyl] diesters;
Cyclic-[1-(4-pyridyl)propan-1,3-diyl] diesters;
Cyclic-[5-hydroxycyclohexan-1,3-diyl] diesters and hydroxy protected forms;
Bis-benzoylthiomethyl esters;
Bis-benzoylthioethylesters;
Bis-benzoyloxymethyl esters;
Bis-*p*-fluorobenzoyloxymethyl esters;
Bis-6-chloronicotinoyloxymethyl esters;
Bis-5-bromonicotinoyloxymethyl esters;
Bis-thiophenecarbonyloxymethyl esters;
Bis-2-furoyloxymethyl esters;
Bis-3-furoyloxymethyl esters;
Diphenyl esters;
Bis-(2-methylphenyl) esters;
Bis-(2-methoxyphenyl) esters;
Bis-(2-ethoxyphenyl) esters;
Bis-(4-methoxyphenyl) esters;
Bis-(3-bromo-4-methoxybenzyl) esters;
Bis-(4-acetoxybenzyl) esters;
Bis-(3,5-dimethoxy-4-acetoxybenzyl) esters;
Bis-(3-methyl-4-acetoxybenzyl) esters;
Bis-(3-methoxy-4-acetoxybenzyl) esters;
Bis-(3-chloro-4-acetoxybenzyl) esters;
Bis-(cyclohexyloxycarbonyloxymethyl) esters;
Bis-(isopropyloxycarbonyloxymethyl) esters;
Bis-(ethyloxycarbonyloxymethyl) esters;
Bis-(methyloxycarbonyloxymethyl) esters;
Bis-(isopropylthiocarbonyloxymethyl) esters;
Bis-(phenyloxycarbonyloxymethyl) esters;
Bis-(benzyloxycarbonyloxymethyl) esters;
Bis-(phenylthiocarbonyloxymethyl) esters;
Bis-(*p*-methoxyphenoxycarbonyloxymethyl) esters;
Bis-(*m*-methoxyphenoxycarbonyloxymethyl) esters;
Bis-(*o*-methoxyphenoxycarbonyloxymethyl) esters;
Bis-(*o*-methylphenoxycarbonyloxymethyl) esters;
Bis-(*p*-chlorophenoxycarbonyloxymethyl) esters;
Bis-(1,4-biphenoxycarbonyloxymethyl) esters;
Bis-[(2-phthalimidoethyl)oxycarbonyloxymethyl]esters;
Bis-(6-hydroxy-3,4-dithia)hexyl esters;
Cyclic-(3,4-dithiahexan-1,6-diyl) esters;
Bis-(2-bromoethyl) esters;
Bis-(2-aminoethyl) esters;
Bis-(2-*N*,*N*-diaminoethyl) esters;
O-phenyl-[N-(1-ethoxycarbonyl)ethyl]phosphoramidates (-P(O)(OPh)(NH-*CH(Me)CO₂Et)
O-phenyl-[N-(1-methoxycarbonyl)ethyl]phosphoramidates (-P(O)(OPh)(NH-*CH(Me)CO₂Me)
O-(3-chlorophenyl)-[N-(1-ethoxycarbonyl)ethyl]phosphoramidates (-P(O)(OPh-3-Cl)(NH-*CH(Me)CO₂Et)
O-(2-chlorophenyl)-[N-(1-ethoxycarbonyl)ethyl]phosphoramidates (-P(O)(OPh-2-Cl)(NH-^{*}CH(Me)C0₂Et)
O-(4-chlorophenyl)-[N-(1-ethoxycarbonyl)ethyl]phosphoramidates (-P(O)(OPh-4-Cl)(NH-*CH(Me)CO₂Et)
O-(4-acetamidophenyl)-[N-(1-ethoxycarbonyl)ethyl]phosphoramidates (-P(O)(OPh-4-NHAc)(NH-*CH(Me)CO₂Et)
O-(2-ethoxycarbonylphenyl)-[N-(1-ethoxycarbonyl)ethyl]phosphoramidates (-P(O)(OPh-2-CO₂Et)(NH-*CH(Me)CO₂Et)
O-phenyl-[N-(1-ethoxycarbonyl-1-methyl)ethyl]phosphoramidates (-P(O)(OPh)(NH-C(Me)₂CO₂Et)
O-phenyl-[N-(1-methoxycarbonyl-1-methyl)ethyl]phosphoramidates (-P(O)(OPh)(NH-C(Me)₂CO₂Me)
O-(3-chlorophenyl)-[N-(1-ethoxycarbonyl-1-methyl)ethyl]phosphoramidates (-P(O)(OPh-3-Cl)(NH-C(Me)₂CO₂Et)
O-(2-chlorophenyl)-[N-(1-ethoxycarbonyl-1-methyl)ethyl]phosphoramidates (-P(O)(OPh-2-Cl)(NH-C(Me)₂CO₂Et)
O-(4-chlorophenyl)-[N-(1-ethoxycarbonyl-1-methyl)ethyl]phosphoramidates (-P(O)(OPh-4-Cl)(NH-C(Me)₂CO₂Et)
O-(4-acetamidophenyl)-[N-(1-ethoxycarbonyl-1-methyl)ethyl]phosphoramidates (-P(O)(OPh-4-NHAc)(NH-C(Me)₂CO₂Et)
O-(2-ethoxycarbonylphenyl)-[N-(1-ethoxycarbonyl-1-methyl)ethyl]phosphoramidates (-P(O)(OPh-2-CO₂Et)(NH-C(Me)₂CO₂Et)

In the above prodrugs an asterisk (*) on a carbon refers to the L-configuration.
O-phenyl-[N-(ethoxycarbonyl)methyl]phosphoramidates (-P(O)(OPh)(NH-CH₂CO₂Et)
O-phenyl-[N-(methoxycarbonyl)methyl]phosphoramidates (-P(O)(OPh)(NH-CH₂CO₂Me)
O-(3-chlorophenyl)-[N-(ethoxycarbonyl)methyl]phosphoramidates (-P(O)(OPh-3-Cl)(NH-CH₂CO₂Et)
O-(2-chlorophenyl)-[N-(ethoxycarbonyl)methyl]phosphoramidates (-P(O)(OPh-2-Cl)(NH-CH₂CO₂Et)
O-(4-chlorophenyl)-[N-(ethoxycarbonyl)methyl]phosphoramidates (-P(O)(OPh-4-Cl)(NH-CH₂CO₂Et)
O-(4-acetamidophenyl)-[N-(ethoxycarbonyl)methyl]phosphoramidates (-P(O)(OPh-4-NHAc)(NH-CH₂CO₂Et)
O-(2-ethoxycarbonylphenyl)-[N-(ethoxycarbonyl)methyl]phosphoramidates (-P(O)(OPh-2-CO₂Et)(NH-CH₂CO₂Et)

The compounds designated in Table 1 refer to preferred compounds of formula I-A where M is R⁵-X- as defined in the following formulae: formula i, formula ii, and formula iii.

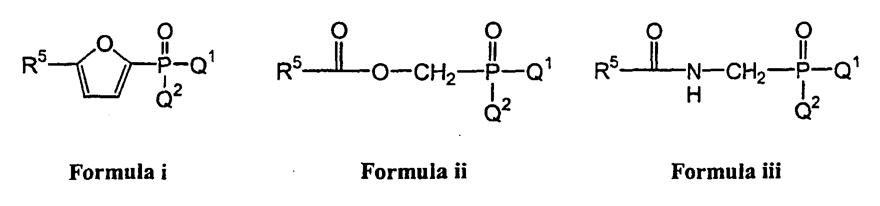

In the above formulae i, ii, and iii, R⁵ may be substituted by A and B. The preferred compounds of formulae i, ii, and iii are listed in Table 1 by designated numbers assigned to R⁵, A, B, Q¹, and Q² in the above formulae i, ii, and iii according to the following convention: Q¹.Q². R⁵.B.A. For each moiety, structures assigned to a number shown in the following tables for R5, A, B, Q¹ and Q².

Variable R⁵ is divided into two groups, each listing four different structures.

Compounds named in Table 1 of formulae i, ii, and iii wherein the R⁵ moieties are assigned the following numbers:

### Group 1:

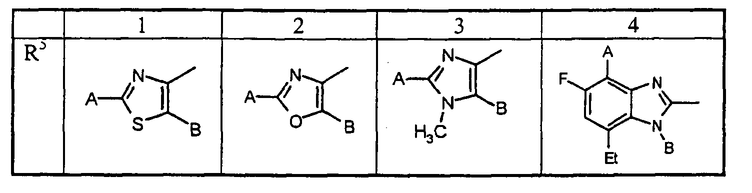

### Group 2:

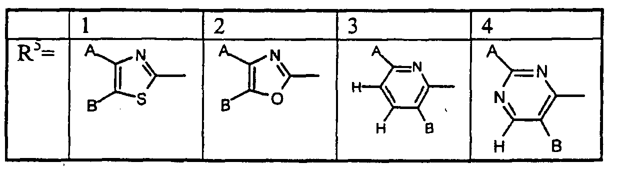

Variable A moieties are assigned the following numbers:

| | 1 | 2 | 3 | 4 |
|---|---|---|---|---|
| A= | NH2 | H | Me | Cl |

Variable B moieties are assigned the following numbers:

| | 1 | 2 | 3 | 4 | 5 | 6 | 7 | 8 |
|---|---|---|---|---|---|---|---|---|
| B= | -SCH3 | -*i*Bu | -*c*Pr | -S-*n*Pr | -SEt | -iPr | -nPr | -CH2cPr |

Variables Q¹ and Q² are divided into three groups, each listing eight different substituents.

Q¹ and Q² moieties are assigned the following numbers:

### Group 1:

### Q¹ and Q²

1. -NH-CH2-C(O)R14
2. -NH-CH(CH3)-C(O)R14
3. -NH-C(CH3)2-C(O)R14
4. -NH-C(CH3)2CH2-C(O)R14
5. -NH-CH(CH(CH3)2))-C(O)R14
6. -NH-CH(CH2(CH(CH3)2)))-C(O)R14
7. -NH-CH(CH2CH2SCH3)-C(O)R14
8. -NH-CH(CH2SCH2Ph)-C(O)R14

### Group 2:

### Q¹ and Q²

1. -NH-CH2CH2-C(O)R14
2. -NH-CH(CH2CH2COR14)-C(O)R14
3. -NH-CH(CH2COR14)-C(O)R14
4. -NH-CH(CH2CONH2)-C(O)R14
5. -NH-CH(COR14)CH2-C(O)R14
6. -NH-CH(CH2OR17)-C(O)R14
7. -NH-CH(CH2CH2COR14)-C(O)R14
8. -NH-CH(CH2OH)-C(O)R14

### Group 3:

### Q¹ and Q²

1. -NH-CH(CH2-C6H5OH)-C(O)R14
2. -NH-C(c-propyl)-C(O)R14
3. -NH-C(c-pentyl)-C(O)R14
4. -NH-C(c-hexyl)-C(O)R14
5. -NH-CH(CH2Ph)-C(O)R14
6. -N(CH3)-CH2-C(O)R14
7. 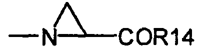
8. -NR18R19
   where R⁴ is selected from the groups consisting of OMe, OEt, OBn, O-*t*Bu, O-*n*Pr, OPh, - N(Me)₂, morpholine, SMe, SEt; R¹⁷ is methyl, ethyl, benzyl, and propyl; R¹⁸ is H, Me, Et, Bn, Pr and Ph and R19 is Me, Et, Bn, Pr and Ph; R18 and R19 is morpholinyl and pyrrolidinyl.

Thus, the compound 3.3.1.2.1 in Group 1 corresponds to the structure below for formula i: 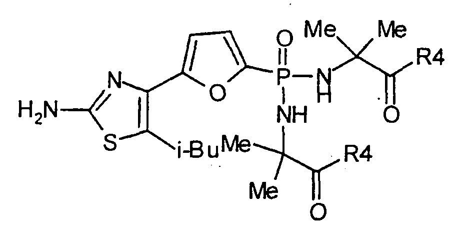 and when R4 is ethoxy the structure would be

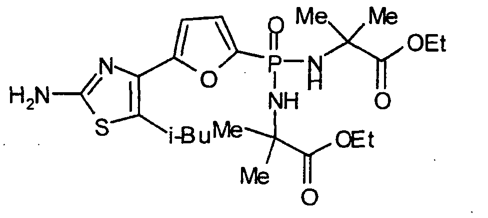

The numbers designated in Table 1 also refer to preferred benzothiazole and benzoxazole compounds of formula X. These preferred compounds are shown in formulae iv and v.

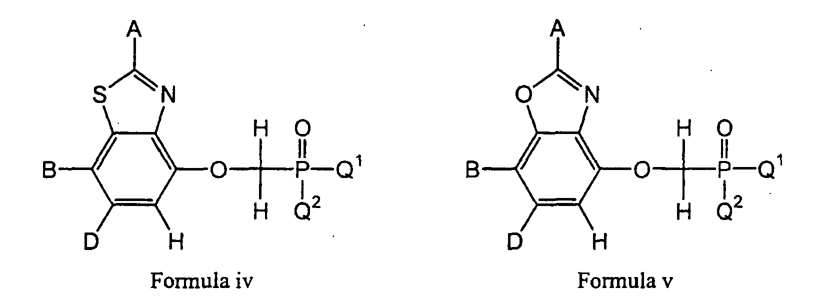

The preferred compounds of formulae iv and formula v are listed in Table 1 by designated numbers assigned to A, B, D, Q¹, and Q² in the above formulae iv and v according to the following convention: Q¹.Q².A.B.D. For each moiety, structures assigned to a number shown in the following tables for A, B, D, Q¹ and Q².

Variables Q¹ and Q² are divided into three groups, each listing eight different substituents.

### Group 1:

Q¹ and Q² moieties are assigned the following numbers:

### Q¹ and Q²

1. -NH-CH2-C(O)R14
2. -NH-CH(CH3)-C(O)R14
3. -NH-C(CH3)2-C(O)R14
4. -NH-C(CH3)2CH2-C(O)R14
5. -NH-CH(CH(CH3)2))-C(O)R14
6. -NH-CH(CH2(CH(CH3)2)))-C(O)R14
7. -NH-CH(CH2CH2SCH3)-C(O)R14
8. -NH-CH(CH2SCH2Ph)-C(O)R14

### Group 2:

### Q¹ and Q²

1. -NH-CH2CH2-C(O)R14
2. -NH-CH(CH2CH2COR14)-C(O)R14
3. -NH-CH(CH2COR14)-C(O)R14
4. -NH-CH(CH2CONH2)-C(O)R14
5. -NH-CH(COR14)CH2-C(O)R14
6. -NH-CH(CH2OR17)-C(O)R14
7. -NH-CH(CH2CH2COR14)-C(O)R14
8. -NH-CH(CH2OH)-C(O)R14

### Group 3:

### Q¹ and Q²

1. -NH-CH(CH2-C6H5OH)-C(O)R14
2. -NH-C(c-propyl)-C(O)R14
3. -NH-C(c-pentyl)-C(O)R14
4. -NH-C(c-hexyl)-C(O)R14
5. -NH-CH(CH2Ph)-C(O)R14
6. -N(CH3)-CH2-C(O)R14
7. 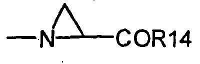
8. -NR18R19

Variable B is divided into three groups, each listing eight different substituents.

### Group 1:

B moieties are assigned the following numbers:

| | 1 | 2 | 3 | 4 | 5 | 6 | 7 | 8 |
|---|---|---|---|---|---|---|---|---|
| B= | H | Me | Et | *n*Pr | Br | iPr | Cl | *c*Pr |

### Group 2:

| | 1 | 2 | 3 | 4 | 5 | 6 | 7 | 8 |
|---|---|---|---|---|---|---|---|---|
| B= | CN | F | OMe | OEt | SMe | SEt | 2-furanyl | C(O)OEt |

### Group 3:

| | 1 | 2 | 3 | 4 | 5 | 6 | 7 | 8 |
|---|---|---|---|---|---|---|---|---|
| B= | B&D are connected to form cyclohexyl ring | B&D are connected to form phenyl ring | B&D are connected to form furanyl ring (O attached at B) | B&D are connected to form furanyl ring (O attached at D) | B&D are connected to form cyclohexyl ring | B&D are connected to form phenyl ring | B&D are connected to form furanyl ring (O attached at B) | B&D are connected to form furanyl ring (O attached at D) |

Group 3 for Variable B can only be combined with Group 3 variable for D.

Variable D is divided into three groups, each listing four different substituents.

### Group 1:

| | 1 | 2 | 3 | 4 |
|---|---|---|---|---|
| D= | H | Me | Et | SCN |

### Group 2:

Variable D is replaced with the moieties assigned in the following numbers:

| | 1 | 2 | 3 | 4 |
|---|---|---|---|---|
| D= | SMe | SEt | CH2OMe | OMe |

### Group 3:

| | 1 | 2 | 3 | 4 |
|---|---|---|---|---|
| D= | null | null | null | null |

Compounds named in Table 1 of formulae iv and v wherein the A moieties are assigned the following numbers:

| | 1 | 2 | 3 | 4 |
|---|---|---|---|---|
| A= | NH2 | H | Me | Cl |

where R4 is selected from the groups consisting of OMe, OEt, OBn, O-*t*Bu, O-*n*Pr, OPh, - N(Me)2, morpholine, SMe, SEt; R17 is methyl, ethyl, benzyl, and propyl; R18 is H, Me, Et, Bn, Pr and Ph and R19 is Me, Et, Bn, Pr and Ph; R18 and R19 is morpholinyl and pyrrolidinyl

Thus, the compound 2.2.1.7.4 from Group 1 for B, D, Q¹ and Q² corresponds to the structure below for formula iv 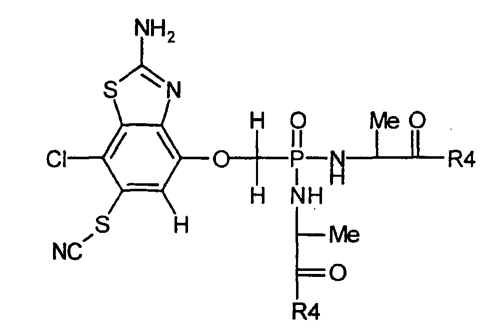 and when R4 is ethoxy the structure would be

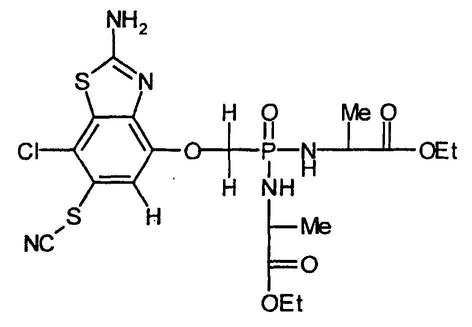

Similarly, in group 3 for variable B, the compound 2.2.1.7.4 corresponds to the structure below for formula iv 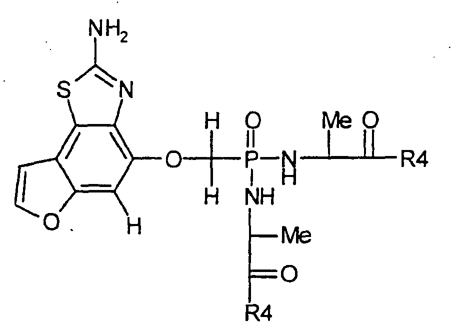 and when R4 is ethoxy the structure would be

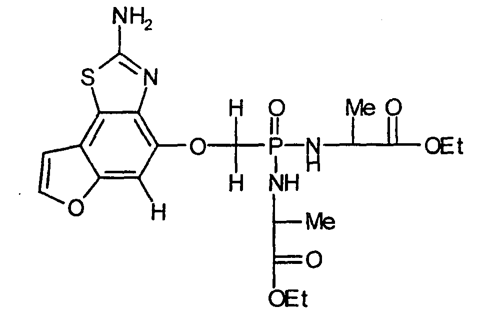

**Table 1**

| | | | | | | | |
|---|---|---|---|---|---|---|---|
| 1.1.1.1.1 | 1.1.1.1.2 | 1.1.1.1.3 | 1.1.1.1.4 | 1.1.1.2.1 | 1.1.1.2.2 | 1.1.1.2.3 | 1.1.1.2.4 |
| 1.1.1.3.1 | 1.1.1.3.2 | 1.1.1.3.3 | 1.1.1.3.4 | 1.1.1.4.1 | 1.1.1.4.2 | 1.1.1.4.3 | 1.1.1.4.4 |
| 1.1.1.5.1 | 1.1.1.5.2 | 1.1.1.5.3 | 1.1.1.5.4 | 1.1.1.6.1 | 1.1.1.6.2 | 1.1.1.6.3 | 1.1.1.6.4 |
| 1.1.1.7.1 | 1.1.1.7.2 | 1.1.1.7.3 | 1.1.1.7.4 | 1.1.1.8.1 | 1.1.1.8.2 | 1.1.1.8.3 | 1.1.1.8.4 |
| 1.1.2.1.1 | 1.1.2.1.2 | 1.1.2.1.3 | 1.1.2.1.4 | 1.1.2.2.1 | 1.1.2.2.2 | 1.1.2.2.3 | 1.1.2.2.4 |
| 1.1.2.3.1 | 1.1.2.3.2 | 1.1.2.3.3 | 1.1.2.3.4 | 1.1.2.4.1 | 1.1.2.4.2 | 1.1.2.4.3 | 1.1.2.4.4 |
| 1.1.2.5.1 | 1.1.2.5.2 | 1.1.2.5.3 | 1.1.2.5.4 | 1.1.2.6.1 | 1.1.2.6.2 | 1.1.2.6.3 | 1.1.2.6.4 |
| 1.1.2.7.1 | 1.1.2.7.2 | 1.1.2.7.3 | 1.1.2.7.4 | 1.1.2.8.1 | 1.1.2.8.2 | 1.1.2.8.3 | 1.1.2.8.4 |
| 1.1.3.1.1 | 1.1.3.1.2 | 1.1.3.1.3 | 1.1.3.1.4 | 1.1.3.2.1 | 1.1.3.2.2 | 1.1.3.2.3 | 1.1.3.2.4 |
| 1.1.3.3.1 | 1.1.3.3.2 | 1.1.3.3.3 | 1.1.3.3.4 | 1.1.3.4.1 | 1.1.3.4.2 | 1.1.3.4.3 | 1.1.3.4.4 |
| 1.1.3.5.1 | 1.1.3.5.2 | 1.1.3.5.3 | 1.1.3.5.4 | 1.1.3.6.1 | 1.1.3.6.2 | 1.1.3.6.3 | 1.1.3.6.4 |
| 1.1.3.7.1 | 1.1.3.7.2 | 1.1.3.7.3 | 1.1.3.7.4 | 1.1.3.8.1 | 1.1.3.8.2 | 1.1.3.8.3 | 1.1.3.8.4 |
| 1.1.4.1.1 | 1.1.4.1.2 | 1.1.4.1.3 | 1.1.4.1.4 | 1.1.4.2.1 | 1.1.4.2.2 | 1.1.4.2.3 | 1.1.4.2.4 |
| 1.1.4.3.1 | 1.1.4.3.2 | 1.1.4.3.3 | 1.1.4.3.4 | 1.1.4.4.1 | 1.1.4.4.2 | 1.1.4.4.3 | 1.1.4.4.4 |
| 1.1.4.5.1 | 1.1.4.5.2 | 1.1.4.5.3 | 1.1.4.5.4 | 1.1.4.6.1 | 1.1.4.6.2 | 1.1.4.6.3 | 1.1.4.6.4 |
| 1.1.4.7.1 | 1.1.4.7.2 | 1.1.4.7.3 | 1.1.4.7.4 | 1.1.4.8.1 | 1.1.4.8.2 | 1.1.4.8.3 | 1.1.4.8.4 |
| 1.2.1.1.1 | 1.2.1.1.2 | 1.2.1.1.3 | 1.2.1.1.4 | 1.2.1.2.1 | 1.2.1.2.2 | 1.2.1.2.3 | 1.2.1.2.4 |
| 1.2.1.3.1 | 1.2.1.3.2 | 1.2.1.3.3 | 1.2.1.3.4 | 1.2.1.4.1 | 1.2.1.4.2 | 1.2.1.4.3 | 1.2.1.4.4 |
| 1.2.1.5.1 | 1.2.1.5.2 | 1.2.1.5.3 | 1.2.1.5.4 | 1.2.1.6.1 | 1.2.1.6.2 | 1.2.1.6.3 | 1.2.1.6.4 |
| 1.2.1.7.1 | 1.2.1.7.2 | 1.2.1.7.3 | 1.2.1.7.4 | 1.2.1.8.1 | 1.2.1.8.2 | 1.2.1.8.3 | 1.2.1.8.4 |
| 1.2.2.1.1 | 1.2.2.1.2 | 1.2.2.1.3 | 1.2.2.1.4 | 1.2.2.2.1 | 1.2.2.2.2 | 1.2.2.2.3 | 1.2.2.2.4 |
| 1.2.2.3.1 | 1.2.2.3.2 | 1.2.2.3.3 | 1.2.2.3.4 | 1.2.2.4.1 | 1.2.2.4.2 | 1.2.2.4.3 | 1.2.2.4.4 |
| 1.2.2.5.1 | 1.2.2.5.2 | 1.2.2.5.3 | 1.2.2.5.4 | 1.2.2.6.1 | 1.2.2.6.2 | 1.2.2.6.3 | 1.2.2.6.4 |
| 1.2.2.7.1 | 1.2.2.7.2 | 1.2.2.7.3 | 1.2.2.7.4 | 1.2.2.8.1 | 1.2.2.8.2 | 1.2.2.8.3 | 1.2.2.8.4 |
| 1.2.3.1.1 | 1.2.3.1.2 | 1.2.3.1.3 | 1.2.3.1.4 | 1.2.3.2.1 | 1.2.3.2.2 | 1.2.3.2.3 | 1.2.3.2.4 |
| 1.2.3.3.1 | 1.2.3.3.2 | 1.2.3.3.3 | 1.2.3.3.4 | 1.2.3.4.1 | 1.2.3.4.2 | 1.2.3.4.3 | 1.2.3.4.4 |
| 1.2.3.5.1 | 1.2.3.5.2 | 1.2.3.5.3 | 1.2.3.5.4 | 1.2.3.6.1 | 1.2.3.6.2 | 1.2.3.6.3 | 1.2.3.6.4 |
| 1.2.3.7.1 | 1.2.3.7.2 | 1.2.3.7.3 | 1.2.3.7.4 | 1.2.3.8.1 | 1.2.3.8.2 | 1.2.3.8.3 | 1.2.3.8.4 |
| 1.2.4.1.1 | 1.2.4.1.2 | 1.2.4.1.3 | 1.2.4.1.4 | 1.2.4.2.1 | 1.2.4.2.2 | 1.2.4.2.3 | 1.2.4.2.4 |
| 1.2.4.3.1 | 1.2.4.3.2 | 1.2.4.3.3 | 1.2.4.3.4 | 1.2.4.4.1 | 1.2.4.4.2 | 1.2.4.4.3 | 1.2.4.4.4 |
| 1.2.4.5.1 | 1.2.4.5.2 | 1.2.4.5.3 | 1.2.4.5.4 | 1.2.4.6.1 | 1.2.4.6.2 | 1.2.4.6.3 | 1.2.4.6.4 |
| 1.2.4.7.1 | 1.2.4.7.2 | 1.2.4.7.3 | 1.2.4.7.4 | 1.2.4.8.1 | 1.2.4.8.2 | 1.2.4.8.3 | 1.2.4.8.4 |
| 1.3.1.1.1 | 1.3.1.1.2 | 1.3.1.1.3 | 1.3.1.1.4 | 1.3.1.2.1 | 1.3.1.2.2 | 1.3.1.2.3 | 1.3.1.2.4 |
| 1.3.1.3.1 | 1.3.1.3.2 | 1.3.1.3.3 | 1.3.1.3.4 | 1.3.1.4.1 | 1.3.1.4.2 | 1.3.1.4.3 | 1.3.1.4.4 |
| 1.3.1.5.1 | 1.3.1.5.2 | 1.3.1.5.3 | 1.3.1.5.4 | 1.3.1.6.1 | 1.3.1.6.2 | 1.3.1.6.3 | 1.3.1.6.4 |
| 1.3.1.7.1 | 1.3.1.7.2 | 1.3.1.7.3 | 1.3.1.7.4 | 1.3.1.8.1 | 1.3.1.8.2 | 1.3.1.8.3 | 1.3.1.8.4 |
| 1.3.2.1.1 | 1.3.2.1.2 | 1.3.2.1.3 | 1.3.2.1.4 | 1.3.2.2.1 | 1.3.2.2.2 | 1.3.2.2.3 | 1.3.2.2.4 |
| 1.3.2.3.1 | 1.3.2.3.2 | 1.3.2.3.3 | 1.3.2.3.4 | 1.3.2.4.1 | 1.3.2.4.2 | 1.3.2.4.3 | 1.3.2.4.4 |
| 1.3.2.5.1 | 1.3.2.5.2 | 1.3.2.5.3 | 1.3.2.5.4 | 1.3.2.6.1 | 1.3.2.6.2 | 1.3.2.6.3 | 1.3.2.6.4 |
| 1.3.2.7.1 | 1.3.2.7.2 | 1.3.2.7.3 | 1.3.2.7.4 | 1.3.2.8.1 | 1.3.2.8.2 | 1.3.2.8.3 | 1.3.2.8.4 |
| 1.3.3.1.1 | 1.3.3.1.2 | 1.3.3.1.3 | 1.3.3.1.4 | 1.3.3.2.1 | 1.3.3.2.2 | 1.3.3.2.3 | 1.3.3.2.4 |
| 1.3.3.3.1 | 1.3.3.3.2 | 1.3.3.3.3 | 1.3.3.3.4 | 1.3.3.4.1 | 1.3.3.4.2 | 1.3.3.4.3 | 1.3.3.4.4 |
| 1.3.3.5.1 | 1.3.3.5.2 | 1.3.3.5.3 | 1.3.3.5.4 | 1.3.3.6.1 | 1.3.3.6.2 | 1.3.3.6.3 | 1.3.3.6.4 |
| 1.3.3.7.1 | 1.3.3.7.2 | 1.3.3.7.3 | 1.3.3.7.4 | 1.3.3.8.1 | 1.3.3.8.2 | 1.3.3.8.3 | 1.3.3.8.4 |
| 1.3.4.1.1 | 1.3.4.1.2 | 1.3.4.1.3 | 1.3.4.1.4 | 1.3.4.2.1 | 1.3.4.2.2 | 1.3.4.2.3 | 1.3.4.2.4 |
| 1.3.4.3.1 | 1.3.4.3.2 | 1.3.4.3.3 | 1.3.4.3.4 | 1.3.4.4.1 | 1.3.4.4.2 | 1.3.4.4.3 | 1.3.4.4.4 |
| 1.3.4.5.1 | 1.3.4.5.2 | 1.3.4.5.3 | 1.3.4.5.4 | 1.3.4.6.1 | 1.3.4.6.2 | 1.3.4.6.3 | 1.3.4.6.4 |
| 1.3.4.7.1 | 1.3.4.7.2 | 1.3.4.7.3 | 1.3.4.7.4 | 1.3.4.8.1 | 1.3.4.8.2 | 1.3.4.8.3 | 1.3.4.8.4 |
| 1.4.1.1.1 | 1.4.1.1.2 | 1.4.1.1.3 | 1.4.1.1.4 | 1.4.1.2.1 | 1.4.1.2.2 | 1.4.1.2.3 | 1.4.1.2.4 |
| 1.4.1.3.1 | 1.4.1.3.2 | 1.4.1.3.3 | 1.4.1.3.4 | 1.4.1.4.1 | 1.4.1.4.2 | 1.4.1.4.3 | 1.4.1.4.4 |
| 1.4.1.5.1 | 1.4.1.5.2 | 1.4.1.5.3 | 1.4.1.5.4 | 1.4.1.6.1 | 1.4.1.6.2 | 1.4.1.6.3 | 1.4.1.6.4 |
| 1.4.1.7.1 | 1.4.1.7.2 | 1.4.1.7.3 | 1.4.1.7.4 | 1.4.1.8.1 | 1.4.1.8.2 | .1.4.1.8.3 | 1.4.1.8.4 |
| 1.4.2.1.1 | 1.4.2.1.2 | 1.4.2.1.3 | 1.4.2.1.4 | 1.4.2.2.1 | 1.4.2.2.2 | 1.4.2.2.3 | 1.4.2.2.4 |
| 1.4.2.3.1 | 1.4.2.3.2 | 1.4.2.3.3 | 1.4.2.3.4 | 1.4.2.4.1 | 1.4.2.4.2 | 1.4.2.4.3 | 1.4.2.4.4 |
| 1.4.2.5.1 | 1.4.2.5.2 | 1.4.2.5.3 | 1.4.2.5.4 | 1.4.2.6.1 | 1.4.2.6.2 | 1.4.2.6.3 | 1.4.2.6.4 |
| 1.4.2.7.1 | 1.4.2.7.2 | 1.4.2.7.3 | 1.4.2.7.4 | 1.4.2.8.1 | 1.4.2.8.2 | 1.4.2.8.3 | 1.4.2.8.4 |
| 1.4.3.1.1 | 1.4.3.1.2 | 1.4.3.1.3 | 1.4.3.1.4 | 1.4.3.2.1 | 1.4.3.2.2 | 1.4.3.2.3 | 1.4.3.2.4 |
| 1.4.3.3.1 | 1.4.3.3.2 | 1.4.3.3.3 | 1.4.3.3.4 | 1.4.3.4.1 | 1.4.3.4.2 | 1.4.3.4.3 | 1.4.3.4.4 |
| 1.4.3.5.1 | 1.4.3.5.2 | 1.4.3.5.3 | 1.4.3.5.4 | 1.4.3.6.1 | 1.4.3.6.2 | 1.4.3.6.3 | 1.4.3.6.4 |
| 1.4.3.7.1 | 1.4.3.7.2 | 1.4.3.7.3 | 1.4.3.7.4 | 1.4.3.8.1 | 1.4.3.8.2 | 1.4.3.8.3 | 1.4.3.8.4 |
| 1.4.4.1.1 | 1.4.4.1.2 | 1.4.4.1.3 | 1.4.4.1.4 | 1.4.4.2.1 | 1.4.4.2.2 | 1.4.4.2.3 | 1.4.4.2.4 |
| 1.4.4.3.1 | 1.4.4.3.2 | 1.4.4.3.3 | 1.4.4.3.4 | 1.4.4.4.1 | 1.4.4.4.2 | 1.4.4.4.3 | 1.4.4.4.4 |
| 1.4.4.5.1 | 1.4.4.5.2 | 1.4.4.5.3 | 1.4.4.5.4 | 1.4.4.6.1 | 1.4.4.6.2 | 1.4.4.6.3 | 1.4.4.6.4 |
| 1.4.4.7.1 | 1.4.4.7.2 | 1.4.4.7.3 | 1.4.4.7.4 | 1.4.4.8.1 | 1.4.4.8.2 | 1.4.4.8.3 | 1.4.4.8.4 |
| 1.5.1.1.1 | 1.5.1.1.2 | 1.5.1.1.3 | 1.5.1.1.4 | 1.5.1.2.1 | 1.5.1.2.2 | 1.5.1.2.3 | 1.5.1.2.4 |
| 1.5.1.3.1 | 1.5.1.3.2 | 1.5.1.3.3 | 1.5.1.3.4 | 1.5.1.4.1 | 1.5.1.4.2 | 1.5.1.4.3 | 1.5.1.4.4 |
| 1.5.1.5.1 | 1.5.1.5.2 | 1.5.1.5.3 | 1.5.1.5.4 | 1.5.1.6.1 | 1.5.1.6.2 | 1.5.1.6.3 | 1.5.1.6.4 |
| 1.5.1.7.1 | 1.5.1.7.2 | .1.5.1.7.3 | 1.5.1.7.4 | 1.5.1.8.1 | 1.5.1.8.2 | 1.5.1.8.3 | 1.5.1.8.4 |
| 1.5.2.1.1 | 1.5.2.1.2 | 1.5.2.1.3 | 1.5.2.1.4 | 1.5.2.2.1 | 1.5.2.2.2 | 1.5.2.2.3 | 1.5.22.4 |
| 1.5.2.3.1 | 1.5.2.3.2 | 1.5.2.3.3 | 1.5.2.3.4 | 1.5.2.4.1 | 1.5.2.4.2 | 1.5.2.4.3 | 1.5.2.4.4 |
| 1.5.2.5.1 | 1.5.2.5.2 | 1.5.2.5.3 | 1.5.2.5.4 | 1.5.2.6.1 | 1.5.2.6.2 | 1.5.2.6.3 | 1.5.2.6.4 |
| 1.5.2.7.1 | 1.5.2.7.2 | 1.5.2.7.3 | 1.5.2.7.4 | 1.5.2.8.1 | 1.5.2.8.2 | 1.5.2.8.3 | 1.5.2.8.4 |
| 1.5.3.1.1 | 1.5.3.1.2 | 1.5.3.1.3 | 1.5.3.1.4 | 1.5.3.2.1 | 1.5.3.2.2 | 1.5.3.2.3 | 1.5.3.2.4 |
| 1.5.3.3.1 | 1.5.3.3.2 | 1.5.3.3.3 | 1.5.3.3.4 | 1.5.3.4.1 | 1.5.3.4.2 | 1.5.3.4.3 | 1.5.3.4.4 |
| 1.5.3.5.1 | 1.5.3.5.2 | 1.5.3.5.3 | 1.5.3.5.4 | 1.5.3.6.1 | 1.5.3.6.2 | 1.5.3.6.3 | 1.5.3.6.4 |
| 1.5.3.7.1 | 1.5.3.7.2 | 1.5.3.7.3 | 1.5.3.7.4 | 1.5.3.8.1 | 1.5.3.8.2 | 1.5.3.8.3 | 1.5.3.8.4 |
| 1.5.4.1.1 | 1.5.4.1.2 | 1.5.4.1.3 | 1.5.4.1.4 | 1.5.4.2.1 | 1.5.4.2.2 | 1.5.4.2.3 | 1.5.4.2.4 |
| 1.5.4.3.1 | 1.5.4.3.2 | 1.5.4.3.3 | 1.5.4.3.4 | 1.5.4.4.1 | 1.5.4.4.2 | 1.5.4.4.3 | 1.5.4.4.4 |
| 1.5.4.5.1 | 1.5.4.5.2 | 1.5.4.5.3 | 1.5.4.5.4 | 1.5.4.6.1 | 1.5.4.6.2 | 1.5.4.6.3 | 1.5.4.6.4 |
| 1.5.4.7.1 | 1.5.4.7.2 | 1.5.4.7.3 | 1.5.4.7.4 | 1.5.4.8.1 | 1.5.4.8.2 | 1.5.4.8.3 | 1.5.4.8.4 |
| 1.6.1.1.1 | 1.6.1.1.2 | 1.6.1.1.3 | 1.6.1.1.4 | 1.6.1.2.1 | 1.6.1.2.2 | 1.6.1.2.3 | 1.6.1.2.4 |
| 1.6.1.3.1 | 1.6.1.3.2 | 1.6.1.3.3 | 1.6.1.3.4 | 1.6.1.4.1 | 1.6.1.4.2 | 1.6.1.4.3 | 1.6.1.4.4 |
| 1.6.1.5.1 | 1.6.1.5.2 | 1.6.1.5.3 | 1.6.1.5.4 | 1.6.1.6.1 | 1.6.1.6.2 | 1.6.1.6.3 | 1.6.1.6.4 |
| 1.6.1.7.1 | 1.6.1.7.2 | 1.6.1.7.3 | 1.6.1.7.4 | 1.6.1.8.1 | 1.6.1.8.2 | 1.6.1.8.3 | 1.6.1.8.4 |
| 1.6.2.1.1 | 1.6.2.1.2 | 1.6.2.1.3 | 1.6.2.1.4 | 1.6.2.2.1 | 1.6.2.2.2 | 1.6.2.2.3 | 1.6.2.2.4 |
| 1.6.2.3.1 | 1.6.2.3.2 | 1.6.2.3.3 | 1.6.2.3.4 | 1.6.2.4.1 | 1.6.2.4.2 | 1.6.2.4.3 | 1.6.2.4.4 |
| 1.6.2.5.1 | 1.6.2.5.2 | 1.6.2.5.3 | 1.6.2.5.4 | 1.6.2.6.1 | 1.6.2.6.2 | 1.6.2.6.3 | 1.6.2.6.4 |
| 1.6.2.7.1 | 1.6.2.7.2 | 1.6.2.7.3 | 1.6.2.7.4 | 1.6.2.8.1 | 1.6.2.8.2 | 1.6.2.8.3 | 1.6.2.8.4 |
| 1.6.3.1.1 | 1.6.3.1.2 | 1.6.3.1.3 | 1.6.3.1.4 | 1.6.3.2.1 | 1.6.3.2.2 | 1.6.3.2.3 | 1.6.3.2.4 |
| 1.6.3.3.1 | 1.6.3.3.2 | 1.6.3.3.3 | 1.6.3.3.4 | 1.6.3.4.1 | 1.6.3.4.2 | 1.6.3.4.3 | 1.6.3.4.4 |
| 1.6.3.5.1 | 1.6.3.5.2 | 1.6.3.5.3 | 1.6.3.5.4 | 1.6.3.6.1 | 1.6.3.6.2 | 1.6.3.6.3 | 1.6.3.6.4 |
| 1.6.3.7.1 | 1.6.3.7.2 | 1.6.3.7.3 | 1.6.3.7.4 | 1.6.3.8.1 | 1.6.3.8.2 | 1.6.3.8.3 | 1.6.3.8.4 |
| 1.6.4.1.1 | 1.6.4.1.2 | 1.6.4.1.3 | 1.6.4.1.4 | 1.6.4.2.1 | 1.6.4.2.2 | 1.6.4.2.3 | 1.6.4.2.4 |
| 1.6.4.3.1 | 1.6.4.3.2 | 1.6.4.3.3 | 1.6.4.3.4 | 1.6.4.4.1 | 1.6.4.4.2 | 1.6.4.4.3 | 1.6.4.4.4 |
| 1.6.4.5.1 | 1.6.4.5.2 | 1.6.4.5.3 | 1.6.4.5.4 | 1.6.4.6.1 | 1.6.4.6.2 | 1.6.4.6.3 | 1.6.4.6.4 |
| 1.6.4.7.1 | 1.6.4.7.2 | 1.6.4.7.3 | 1.6.4.7.4 | 1.6.4.8.1 | 1.6.4.8.2 | 1.6.4.8.3 | 1.6.4.8.4 |
| 1.7.1.1.1 | 1.7.1.1.2 | 1.7.1.1.3 | 1.7.1.1.4 | 1.7.1.2.1 | 1.7.1.2.2 | 1.7.1.2.3 | 1.7.1.2.4 |
| 1.7.1.3.1 | 1.7.1.3.2 | 1.7.1.3.3 | 1.7.1.3.4 | 1.7.1.4.1 | 1.7.1.4.2 | 1.7.1.4.3 | 1.7.1.4.4 |
| 1.7.1.5.1 | 1.7.1.5.2 | 1.7.1.5.3 | 1.7.1.5.4 | 1.7.1.6.1 | 1.7.1.6.2 | 1.7.1.6.3 | 1.7.1.6.4 |
| 1.7.1.7.1 | 1.7.1.7.2 | 1.7.1.7.3 | 1.7.1.7.4 | 1.7.1.8.1 | 1.7.1.8.2 | 1.7.1.8.3 | 1.7.1.8.4 |
| I.7.2.1.1 | 1.7.2.1.2 | 1.7.2.1.3 | 1.7.2.1.4 | 1.7.2.2.1 | 1.7.2.2.2 | 1.7.2.2.3 | 1.7.2.2.4 |
| 1.7.2.3.1 | 1.7.2.3.2 | 1.7.2.3.3 | 1.7.2.3.4 | 1.7.2.4.1 | 1.7.2.4.2 | 1.7.2.4.3 | 1.7.2.4.4 |
| 1.7.2.5.1 | 1.7.2.5.2 | 1.7.2.5.3 | 1.7.2.5.4 | 1.7.2.6.1 | 1.7.2.6.2 | 1.7.2.6.3 | 1.7.2.6.4 |
| 1.7.2.7.1 | 1.7.2.7.2 | 1.7.2.7.3 | 1.7.2,7.4 | 1.7.2.8.1 | 1.7.2.8.2 | 1.7.2.8.3 | 1.7.2.8.4 |
| 1.7.3.1.1 | 1.7.3.1.2 | 1.7.3.1.3 | 1.7.3.1.4 | 1.7.3.2.1 | 1.7.3.2.2 | 1.7.3.2.3 | 1.7.3.2.4 |
| 1.7.3.3.1 | 1.7.3.3.2 | 1.7.3.3.3 | 1.7.3.3.4 | 1.7.3.4.1 | 1.7.3.4.2 | 1.7.3.4.3 | 1.7.3.4.4 |
| 1.7.3.5.1 | 1.7.3.5.2 | 1.7.3.5.3 | 1.7.3.5.4 | 1.7.3.6.1 | 1.7.3.6.2 | 1.7.3.6.3 | 1.7.3.6.4 |
| 1.7.3.7.1 | 1.7.3.7.2 | 1.7.3.7.3 | 1.7.3.7.4 | 1.7.3.8.1 | 1.7.3.8.2 | 1.7.3.8.3 | 1.7.3.8.4 |
| 1.7.4.1.1 | 1.7.4.1.2 | 1.7.4.1.3 | 1.7.4.1.4 | 1.7.4.2.1 | 1.7.4.2.2 | 1.7.4.2.3 | 1.7.4.2.4 |
| 1.7.4.3.1 | 1.7.4.3.2 | 1.7.4.3.3 | I.7.4.3.4 | 1.7.4.4.1 | 1.7.4.4.2 | 1.7.4.4.3 | 1.7.4.4.4 |
| 1.7.4.5.1 | 1.7.4.5.2 | 1.7.4.5.3 | 1.7.4.5.4 | 1.7.4.6.1 | 1.7.4.6.2 | 1.7.4.6.3 | 1.7.4.6.4 |
| 1.7.4.7.1 | 1.7.4.7.2 | 1.7.4.7.3 | 1.7.4.7.4 | 1.7.4.8.1 | 1.7.4.8.2 | 1.7.4.8.3 | .7.4.8.4 |
| 1.8.1.1.1 | 1.8.1.1.2 | 1.8.1.1.3 | 1.8.1.1.4 | 1.8.1.2.1 | 1.8.1.2.2 | 1.8.1.2.3 | 1.8.1.2.4 |
| 1.8.1.3.1 | 1.8.1.3.2 | 1.8.1.3.3 | 1.8.1.3.4 | 1.8.1.4.1 | 1.8.1.4.2 | 1.8.1.4.3 | 1.8.1.4.4 |
| 1.8.1.5.1 | 1.8.1.5.2 | 1.8.1.5.3 | 1.8.1.5.4 | 1.8.1.6.1 | 1.8.1.6.2 | 1.8.1.6.3 | 1.8.1.6.4 |
| 1.8.1.7.1 | 1.8.1.7.2 | 1.8.1.7.3 | 1.8.1.7.4 | 1.8.1.8.1 | 1.8.1.8.2 | 1.8.1.8.3 | 1.8.1.8.4 |
| 1.8.2.1.1 | 1.8.2.1.2 | 1.8.2.1.3 | 1.8.2.1.4 | 1.8.2.2.1 | 1.8.2.2.2 | 1.8.2.2.3 | 1.8.2.2.4 |
| 1.8.2.3.1 | 1.8.2.3.2 | 1.8.2.3.3 | 1.8.2.3.4 | 1.8.2.4.1 | 1.8.2.4.2 | 1.8.2.4.3 | 1.8.2.4.4 |
| 1.8.2.5.1 | 1.8.2.5.2 | 1.8.2.5.3 | 1.8.2.5.4 | 1.8.2.6.1 | 1.8.2.6.2 | 1.8.2.6.3 | 1.8.2.6.4 |
| 1.8.2.7.1 | 1.8.2.7.2 | 1.8.2.7.3 | 1.8.2.7.4 | 1.8.2.8.1 | 1.8.2.8.2 | 1.8.2.8.3 | 1.8.2.8.4 |
| 1.8.3.1.1 | 1.8.3.1.2 | 1.8.3.1.3 | 1.8.3.1.4 | 1.8.3.2.1 | 1.8.3.2.2 | 1.8.3.2.3 | 1.8.3.2.4 |
| 1.8.3.3.1 | 1.8.3.3.2 | 1.8.3.3.3 | 1.8.3.3.4 | 1.8.3.4.1 | 1.8.3.4.2 | 1.8.3.4.3 | 1.8.3.4.4 |
| 1.8.3.5.1 | 1.8.3.5.2 | 1.8.3.5.3 | 1.8.3.5.4 | 1.8.3.6.1 | 1.8.3.6.2 | 1.8.3.6.3 | 1.8.3.6.4 |
| 1.8.3.7.1 | 1.8.3.7.2 | 1.8.3.7.3 | 1.8.3.7.4 | 1.8.3.8.1 | 1.8.3.8.2 | 1.8.3.8.3 | 1.8.3.8.4 |
| 1.8.4.1.1 | 1.8.4.1.2 | 1.8.4.1.3 | 1.8.4.1.4 | 1.8.4.2.1 | 1.8.4.2.2 | 1.8.4.2.3 | 1.8.4.2.4 |
| 1.8.4.3.1 | 1.8.4.3.2 | 1.8.4.3.3 | 1.8.4.3.4 | 1.8.4.4.1 | 1.8.4.4.2 | 1.8.4.4.3 | 1.8.4.4.4 |
| 1.8.4.5.1 | 1.8.4.5.2 | 1.8.4.5.3 | 1.8.4.5.4 | 1.8.4.6.1 | 1.8.4.6.2 | 1.8.4.6.3 | 1.8.4.6.4 |
| 1.8.4.7.1 | 1.8.4.7.2 | 1.8.4.7.3 | 1.8.4.7.4 | 1.8.4.8.1 | 1.8.4.8.2 | 1.8.4.8.3 | 1.8.4.8.4 |
| 2.1.1.1.1 | 2.1.1.1.2 | 2.1.1.1.3 | 2.1.1.1.4 | 2.1.1.2.1 | 2.1.1.2.2 | 2.1.1.2.3 | 2.1.1.2.4 |
| 2.1.1.3.1 | 2.1.1.3.2 | 2.1.1.3.3 | 2.1.1.3.4 | 2.1.1.4.1 | 2.1.1.4.2 | 2.1.1.4.3 | 2.1.1.4.4 |
| 2.1.1.5.1 | 2.1.1.5.2 | 2.1.1.5.3 | 2.1.1.5.4 | 2.1.1.6.1 | 2.1.1.6.2 | 2.1.1.6.3 | 2.1.1.6.4 |
| 2.1.1.7.1 | 2.1.1.7.2 | 2.1.1.7.3 | 2.1.1.7.4 | 2.1.1.8.1 | 2.1.1.8.2 | 2.1.1.8.3 | 2.1.1.8.4 |
| 2.1.2.1.1 | 2.1.2.1.2 | 2.1.2.1.3 | 2.1.2.1.4 | 2.1.2.2.1 | 2.1.2.2.2 | 2.1.2.2.3 | 2.1.2.2.4 |
| 2.1.2.3.1 | 2.1.2.3.2 | 2.1.2.3.3 | 2.1.2.3.4 | 2.1.2.4.1 | 2.1.2.4.2 | 2.1.2.4.3 | 2.1.2.4.4 |
| 2.1.2.5.1 | 2.1.2.5.2 | 2.1.2.5.3 | 2.1.2.5.4 | 2.1.2.6.1 | 2.1.2.6.2 | 2.1.2.6.3 | 2.1.2.6.4 |
| 2.1.2.7.1 | 2.1.2.7.2 | 2.1.2.7.3 | 2.1.2.7.4 | 2.1.2.8.1 | 2.1.2.8.2 | 2.1.2.8.3 | 2.1.2.8.4 |
| 2.1.3.1.1 | 2.1.3.1.2 | 2.1.3.1.3 | 2.1.3.1.4 | 2.1.3.2.1 | 2.1.3.2.2 | 2.1.3.2.3 | 2.1.3.2.4 |
| 2.1.3.3.1 | 2.1.3.3.2 | 2.1.3.3.3 | 2.1.3.3.4 | 2.1.3.4.1 | 2.1.3.4.2 | 2.1.3.4.3 | 2.1.3.4.4 |
| 2.1.3.5.1 | 2.1.3.5.2 | 2.1.3.5.3 | 2.1.3.5.4 | 2.1.3.6.1 | 2.1.3.6.2 | 2.1.3.6.3 | 2.1.3.6.4 |
| 2.1.3.7.1 | 2.1.3.7.2 | 2.1.3.7.3 | 2.1.3.7.4 | 2.1.3.8.1 | 2.1.3.8.2 | 2.1.3.8.3 | 2.1.3.8.4 |
| 2.1.4.1.1 | 2.1.4.1.2 | 2.1.4.1.3 | 2.1.4.1.4 | 2.1.4.2.1 | 2.1.4.2.2 | 2.1.4.2.3 | 2.1.4.2.4 |
| 2.1.4.3.1 | 2.1.4.3.2 | 2.1.4.3.3 | 2.1.4.3.4 | 2.1.4.4.1 | 2.1.4.4.2 | 2.1.4.4.3 | 2.1.4.4.4 |
| 2.1.4.5.1 | 2.1.4.5.2 | 2.1.4.5.3 | 2.1.4.5.4 | 2.1.4.6.1 | 2.1.4.6.2 | 2.1.4.6.3 | 2.1.4.6.4 |
| 2.1.4.7.1 | 2.1.4.7.2 | 2.1.4.7.3 | 2.1.4.7.4 | 2.1.4.8.1 | 2.1.4.8.2 | 2.1.4.8.3 | 2.1.4.8.4 |
| 2.2.1.1.1 | 2.2.1.1.2 | 2.2.1.1.3 | 2.2.1.1.4 | 2.2.1.2.1 | 2.2.1.2.2 | 2.2.1.2.3 | 2.2.1.2.4 |
| 2.2.1.3.1 | 2.2.1.3.2 | 2.2.1.3.3 | 2.2.1.3.4 | 2.2.1.4.1 | 2.2.1.4.2 | 2.2.1.4.3 | 2.2.1.4.4 |
| 2.2.1.5.1 | 2.2.1.5.2 | 2.2.1.5.3 | 2.2.1.5.4 | 2.2.1.6.1 | 2.2.1.6.2 | 2.2.1.6.3 | 2.2.1.6.4 |
| 2.2.1.7.1 | 2.2.1.7.2 | 2.2.1.7.3 | 2.2.1.7.4 | 2.2.1.8.1 | 2.2.1.8.2 | 2.2.1.8.3 | 2.2.1.8.4 |
| 2.2.2.1.1 | 2.2.2.1.2 | 2.2.2.1.3 | 2.2.2.1.4 | 2.2.2.2.1 | 2.2.2.2.2 | 2.2.2.2.3 | 2.2.2.2.4 |
| 2.2.2.3.1 | 2.2.2.3.2 | 2.2.2.3.3 | 2.2.2.3.4 | 2.2.2.4.1 | 2.2.2.4.2 | 2.2.2.4.3 | 2.2.2.4.4 |
| 2.2.2.5.1 | 2.2.2.5.2 | 2.2.2.5.3 | 2.2.2.5.4 | 2.2.2.6.1 | 2.2.2.6.2 | 2.2.2.6.3 | 2.2.2.6.4 |
| 2.2.2.7.1 | 2.2.2.7.2 | 2.2.2.7.3 | 2.2.2.7.4 | 2.2.2.8.1 | 2.2.2.8.2 | 2.2.2.8.3 | 2.2.2.8.4 |
| 2.2.3.1.1 | 2.2.3.1.2 | 2.2.3.1.3 | 2.2.3.1.4 | 2.2.3.2.1 | 2.2.3.2.2 | 2.2.3.2.3 | 2.2.3.2.4 |
| 2.2.3.3.1 | 2.2.3.3.2 | 2.2.3.3.3 | 2.2.3.3.4 | 2.2.3.4.1 | 2.2.3.4.2 | 2.2.3.4.3 | 2.2.3.4.4 |
| 2.2.3.5.1 | 2.2.3.5.2 | 2.2.3.5.3 | 2.2.3.5.4 | 2.2.3.6.1 | 2.2.3.6.2 | 2.2.3.6.3 | 2.2.3.6.4 |
| 2.2.3.7.1 | 2.2.3.7.2 | 2.2.3.7.3 | 2.2.3.7.4 | 2.2.3.8.1 | 2.2.3.8.2 | 2.2.3.8.3 | 2.2.3.8.4 |
| 2.2.4.1.1 | 2.2.4.1.2 | 2.2.4.1.3 | 2.2.4.1.4 | 2.2.4.2.1 | 2.2.4.2.2 | 2.2.4.2.3 | 2.2.4.2.4 |
| 2.2.4.3.1 | 2.2.4.3.2 | 2.2.4.3.3 | 2.2.4.3.4 | 2.2.4.4.1 | 2.2.4.4.2 | 2.2.4.4.3 | 2.2.4.4.4 |
| 2.2.4.5.1 | 2.2.4.5.2 | 2.2.4.5.3 | 2.2.4.5.4 | 2.2.4.6.1 | 2.2.4.6.2 | 2.2.4.6.3 | 2.2.4.6.4 |
| 2.2.4.7.1 | 2.2.4.7.2 | 2.2.4.7.3 | 2.2.4.7.4 | 2.2.4.8.1 | 2.2.4.8.2 | 2.2.4.8.3 | 2.2.4.8.4 |
| 2.3.1.1.1 | 2.3.1.1.2 | 2.3.1.1.3 | 2.3.1.1.4 | 2.3.1.2.1 | 2.3.1.2.2 | 2.3.1.2.3 | 2.3.1.2.4 |
| 2.3.1.3.1 | 2.3.1.3.2 | 2.3.1.3.3 | 2.3.1.3.4 | 2.3.1.4.1 | 2.3.1.4.2 | 2.3.1.4.3 | 2.3.1.4.4 |
| 2.3.1.5.1 | 2.3.1.5.2 | 2.3.1.5.3 | 2.3.1.5.4 | 2.3.1.6.1 | 2.3.1.6.2 | 2.3.1.6.3 | 2.3.1.6.4 |
| 2.3.1.7.1 | 2.3.1.7.2 | 2.3.1.7.3 | 2.3.1.7.4 | 2.3.1.8.1 | 2.3.1.8.2 | 2.3.1.8.3 | 2.3.1.8.4 |
| 2.3.2.1.1 | 2.3.2.1.2 | 2.3.2.1.3 | 2.3.2.1.4 | 2.3.2.2.1 | 2.3.2.2.2 | 2.3.2.2.3 | 2.3.2.2.4 |
| 2.3.2.3.1 | 2.3.2.3.2 | 2.3.2.3.3 | 2.3.2.3.4 | 2.3.2.4.1 | 2.3.2.4.2 | 2.3.2.4.3 | 2.3.2.4.4 |
| 2.3.2.5.1 | 2.3.2.5.2 | 2.3.2.5.3 | 2.3.2.5.4 | 2.3.2.6.1 | 2.3.2.6.2 | 2.3.2.6.3 | 2.3.2.6.4 |
| 2.3.2.7.1 | 2.3.2.7.2 | 2.3.2.7.3 | 2.3.2.7.4 | 2.3.2.8.1 | 2.3.2.8.2 | 2.3.2.8.3 | 2.3.2.8.4 |
| 2.3.3.1.1 | 2.3.3.1.2 | 2.3.3.1.3 | 2.3.3.1.4 | 2.3.3.2.1 | 2.3.3.2.2 | 2.3.3.2.3 | 2.3.3.2.4 |
| 2.3.3.3.1 | 2.3.3.3.2 | 2.3.3.3.3 | 2.3.3.3.4 | 2.3.3.4.1 | 2.3.3.4.2 | 2.3.3.4.3 | 2.3.3.4.4 |
| 2.3.3.5.1 | 2.3.3.5.2 | 2.3.3.5.3 | 2.3.3.5.4 | 2.3.3.6.1 | 2.3.3.6.2 | 2.3.3.6.3 | 2.3.3.6.4 |
| 2.3.3.7.1 | 2.3.3.7.2 | 2.3.3.7.3 | 2.3.3.7.4 | 2.3.3.8.1 | 2.3.3.8.2 | 2.3.3.8.3 | 2.3.3.8.4 |
| 2.3.4.1.1 | 2.3.4.1.2 | 2.3.4.1.3 | 2.3.4.1.4 | 2.3.4.2.1 | 2.3.4.2.2 | 2.3.4.2.3 | 2.3.4.2.4 |
| 2.3.4.3.1 | 2.3.4.3.2 | 2.3.4.3.3 | 2.3.4.3.4 | 2.3.4.4.1 | 2.3.4.4.2 | 2.3.4.4.3 | 2.3.4.4.4 |
| 2.3.4.5.1 | 2.3.4.5.2 | 2.3.4.5.3 | 2.3.4.5.4 | 2.3.4.6.1 | 2.3.4.6.2 | 2.3.4.6.3 | 2.3.4.6.4 |
| 2.3.4.7.1 | 2.3.4.7.2 | 2.3.4.7.3 | 2.3.4.7.4 | 2.3.4.8.1 | 2.3.4.8.2 | 2.3.4.8.3 | 2.3.4.8.4 |
| 2.4.1.1.1 | 2.4.1.1.2 | 2.4.1.1.3 | 2.4.1.1.4 | 2.4.1.2.1 | 2.4.1.2.2 | 2.4.1.2.3 | 2.4.1.2.4 |
| 2.4.1.3.1 | 2.4.1.3.2 | 2.4.1.3.3 | 2.4.1.3.4 | 2.4.1.4.1 | 2.4.1.4.2 | 2.4.1.4.3 | 2.4.1.4.4 |
| 2.4.1.5.1 | 2.4.1.5.2 | 2.4.1.5.3 | 2.4.1.5.4 | 2.4.1.6.1 | 2.4.1.6.2 | 2.4.1.6.3 | 2.4.1.6.4 |
| 2.4.1.7.1 | 2.4.1.7.2 | 2.4.1.7.3 | 2.4.1.7.4 | 2.4.1.8.1 | 2.4.1.8.2 | 2.4.1.8.3 | 2.4.1.8.4 |
| 2.4.2.1.1 | 2.4.2.1.2 | 2.4.2.1.3 | 2.4.2.1.4 | 2.4.2.2.1 | 2.4.2.2.2 | 2.4.2.2.3 | 2.4.2.2.4 |
| 2.4.2.3.1 | 2.4.2.3.2 | 2.4.2.3.3 | 2.4.2.3.4 | 2.4.2.4.1 | 2.4.2.4.2 | 2.4.2.4.3 | 2.4.2.4.4 |
| 2.4.2.5.1 | 2.4.2.5.2 | 2.4.2.5.3 | 2.4.2.5.4 | 2.4.2.6.1 | 2.4.2.6.2 | 2.4.2.6.3 | 2.4.2.6.4 |
| 2.4.2.7.1 | 2.4.2.7.2 | 2.4.2.7.3 | 2.4.2.7.4 | 2.4.2.8.1 | 2.4.2.8.2 | 2.4.2.8.3 | 2.4.2.8.4 |
| 2.4.3.1.1 | 2.4.3.1.2 | 2.4.3.1.3 | 2.4.3.1.4 | 2.4.3.2.1 | 2.4.3.2.2 | 2.4.3.2.3 | 2.4.3.2.4 |
| 2.4.3.3.1 | 2.4.3.3.2 | 2.4.3.3.3 | 2.4.3.3.4 | 2.4.3.4.1 | 2.4.3.4.2 | 2.4.3.4.3 | 2.4.3.4.4 |
| 2.4.3.5.1 | 2.4.3.5.2 | 2.4.3.5.3 | 2.4.3.5.4 | 2.4.3.6.1 | 2.4.3.6.2 | 2.4.3.6.3 | 2.4.3.6.4 |
| 2.4.3.7.1 | 2.4.3.7.2 | 2.4.3.7.3 | 2.4.3.7.4 | 2.4.3.8.1 | 2.4.3.8.2 | 2.4.3.8.3 | 2.4.3.8.4 |
| 2.4.4.1.1 | 2.4.4.1.2 | 2.4.4.1.3 | 2.4.4.1.4 | 2.4.4.2.1 | 2.4.4.2.2 | 2.4.4.2.3 | 2.4.4.2.4 |
| 2.4.4.3.1 | 2.4.4.3.2 | 2.4.4.3.3 | 2.4.4.3.4 | 2.4.4.4.1 | 2.4.4.4.2 | 2.4.4.4.3 | 2.4.4.4.4 |
| 2.4.4.5.1 | 2.4.4.5.2 | 2.4.4.5.3 | 2.4.4.5.4 | 2.4.4.6.1 | 2.4.4.6.2 | 2.4.4.6.3 | 2.4.4.6.4 |
| 2.4.4.7.1 | 2.4.4.7.2 | 2.4.4.7.3 | 2.4.4.7.4 | 2.4.4.8.1 | 2.4.4.8.2 | 2.4.4.8.3 | 2.4.4.8.4 |
| 2.5.1.1.1 | 2.5.1.1.2 | 2.5.1.1.3 | 2.5.1.1.4 | 2.5.1.2.1 | 2.5.1.2.2 | 2.5.1.2.3 | 2.5.1.2.4 |
| 2.5.1.3.1 | 2.5.1.3.2 | 2.5.1.3.3 | 2.5.1.3.4 | 2.5.1.4.1 | 2.5.1.4.2 | 2.5.1.4.3 | 2.5.1.4.4 |
| 2.5.1.5.1 | 2.5.1.5.2 | 2.5.1.5.3 | 2.5.1.5.4 | 2.5.1.6.1 | 2.5.1.6.2 | 2.5.1.6.3 | 2.5.1.6.4 |
| 2.5.1.7.1 | 2.5.1.7.2 | 2.5.1.7.3 | 2.5.1.7.4 | 2.5.1.8.1 | 2.5.1.8.2 | 2.5.1.8.3 | 2.5.1.8.4 |
| 2.5.2.1.1 | 2.5.2.1.2 | 2.5.2.1.3 | 2.5.2.1.4 | 2.5.2.2.1 | 2.5.2.2.2 | 2.5.2.2.3 | 2.5.2.2.4 |
| 2.5.2.3.1 | 2.5.2.3.2 | 2.5.2.3.3 | 2.5.2.3.4 | 2.5.2.4.1 | 2.5.2.4.2 | 2.5.2.4.3 | 2.5.2.4.4 |
| 2.5.2.5.1 | 2.5.2.5.2 | 2.5.2.5.3 | 2.5.2.5.4 | 2.5.2.6.1 | 2.5.2.6.2 | 2.5.2.6.3 | 2.5.2.6.4 |
| 2.5.2.7.1 | 2.5.2.7.2 | 2.5.2.7.3 | 2.5.2.7.4 | 2.5.2.8.1 | 2.5.2.8.2 | 2.5.2.8.3 | 2.5.2.8.4 |
| 2.5.3.1.1 | 2.5.3.1.2 | 2.5.3.1.3 | 2.5.3.1.4 | 2.5.3.2.1 | 2.5.3.2.2 | 2.5.3.2.3 | 2.5.3.2.4 |
| 2.5.3.3.1 | 2.5.3.3.2 | 2.5.3.3.3 | 2.5.3.3.4 | 2.5.3.4.1 | 2.5.3.4.2 | 2.5.3.4.3 | 2.5.3.4.4 |
| 2.5.3.5.1 | 2.5.3.5.2 | 2.5.3.5.3 | 2.5.3.5.4 | 2.5.3.6.1 | 2.5.3.6.2 | 2.5.3.6.3 | 2.5.3.6.4 |
| 2.5.3.7.1 | 2.5.3.7.2 | 2.5.3.7.3 | 2.5.3.7.4 | 2.5.3.8.1 | 2.5.3.8.2 | 2.5.3.8.3 | 2.5.3.8.4 |
| 2.5.4.1.1 | 2.5.4.1.2 | 2.5.4.1.3 | 2.5.4.1.4 | 2.5.4.2.1 | 2.5.4.2.2 | 2.5.4.2.3 | 2.5.4.2.4 |
| 2.5.4.3.1 | 2.5.4.3.2 | 2.5.4.3.3 | 2.5.4.3.4 | 2.5.4.4.1 | 2.5.4.4.2 | 2.5.4.4.3 | 2.5.4.4.4 |
| 2.5.4.5.1 | 2.5.4.5.2 | 2.5.4.5.3 | 2.5.4.5.4 | 2.5.4.6.1 | 2.5.4.6.2 | 2.5.4.6.3 | 2.5.4.6.4 |
| 2.5.4.7.1 | 2.5.4.7.2 | 2.5.4.7.3 | 2.5.4.7.4 | 2.5.4.8.1 | 2.5.4.8.2 | 2.5.4.8.3 | 2.5.4.8.4 |
| 2.6.1.1.1 | 2.6.1.1.2 | 2.6.1.1.3 | 2.6.1.1.4 | 2.6.1.2.1 | 2.6.1.2.2 | 2.6.1.2.3 | 2.6.1.2.4 |
| 2.6.1.3.1 | 2.6.1.3.2 | 2.6.1.3.3 | 2.6.1.3.4 | 2.6.1.4.1 | 2.6.1.4.2 | 2.6.1.4.3 | 2.6.1.4.4 |
| 2.6.1.5.1 | 2.6.1.5.2 | 2.6.1.5.3 | 2.6.1.5.4 | 2.6.1.6.1 | 2.6.1.6.2 | 2.6.1.6.3 | 2.6.1.6.4 |
| 2.6.1.7.1 | 2.6.1.7.2 | 2.6.1.7.3 | 2.6.1.7.4 | 2.6.1.8.1 | 2.6.1.8.2 | 2.6.1.8.3 | 2.6.1.8.4 |
| 2.6.2.1.1 | 2.6.2.1.2 | 2.6.2.1.3 | 2.6.2.1.4 | 2.6.2.2.1 | 2.6.2.2.2 | 2.6.2.2.3 | 2.6.2.2.4 |
| 2.6.2.3.1 | 2.6.2.3.2 | 2.6.2.3.3 | 2.6.2.3.4 | 2.6.2.4.1 | 2.6.2.4.2 | 2.6.2.4.3 | 2.6.2.4.4 |
| 2.6.2.5.1 | 2.6.2.5.2 | 2.6.2.5.3 | 2.6.2.5.4 | 2.6.2.6.1 | 2.6.2.6.2 | 2.6.2.6.3 | 2.6.2.6.4 |
| 2.6.2.7.1 | 2.6.2.7.2 | 2.6.2.7.3 | 2.6.2.7.4 | 2.6.2.8.1 | 2.6.2.8.2 | 2.6.2.8.3 | 2.6.2.8.4 |
| 2.6.3.1.1 | 2.6.3.1.2 | 2.6.3.1.3 | 2.6.3.1.4 | 2.6.3.2.1 | 2.6.3.2.2 | 2.6.3.2.3 | 2.6.3.2.4 |
| 2.6.3.3.1 | 2.6.3.3.2 | 2.6.3.3.3 | 2.6.3.3.4 | 2.6.3.4.1 | 2.6.3.4.2 | 2.6.3.4.3 | 2.6.3.4.4 |
| 2.6.3.5.1 | 2.6.3.5.2 | 2.6.3.5.3 | 2.6.3.5.4 | 2.6.3.6.1 | 2.6.3.6.2 | 2.6.3.6.3 | 2.6.3.6.4 |
| 2.6.3.7.1 | 2.6.3.7.2 | 2.6.3.7.3 | 2.6.3.7.4 | 2.6.3.8.1 | 2.6.3.8.2 | 2.6.3.8.3 | 2.6.3.8.4 |
| 2.6.4.1.1 | 2.6.4.1.2 | 2.6.4.1.3 | 2.6.4.1.4 | 2.6.4.2.1 | 2.6.4.2.2 | 2.6.4.2.3 | 2.6.4.2.4 |
| 2.6.4.3.1 | 2.6.4.3.2 | 2.6.4.3.3 | 2.6.4.3.4 | 2.6.4.4.1 | 2.6.4.4.2 | 2.6.4.4.3 | 2.6.4.4.4 |
| 2.6.4.5.1 | 2.6.4.5.2 | 2.6.4.5.3 | 2.6.4.5.4 | 2.6.4.6.1 | 2.6.4.6.2 | 2.6.4.6.3 | 2.6.4.6.4 |
| 2.6.4.7.1 | 2.6.4.7.2 | 2.6.4.7.3 | 2.6.4.7.4 | 2.6.4.8.1 | 2.6.4.8.2 | 2.6.4.8.3 | 2.6.4.8.4 |
| 2.7.1.1.1 | 2.7.1.1.2 | 2.7.1.1.3 | 2.7.1.1.4 | 2.7.1.2.1 | 2.7.1.2.2 | 2.7.1.2.3 | 2.7.1.2.4 |
| 2.7.1.3.1 | 2.7.1.3.2 | 2.7.1.3.3 | 2.7.1.3.4 | 2.7.1.4.1 | 2.7.1.4.2 | 2.7.1.4.3 | 2.7.1.4.4 |
| 2.7.1.5.1 | 2.7.1.5.2 | 2.7.1.5.3 | 2.7.1.5.4 | 2.7.1.6.1 | 2.7.1.6.2 | 2.7.1.6.3 | 2.7.1.6.4 |
| 2.7.1.7.1 | 2.7.1.7.2 | 2.7.1.7.3 | 2.7.1.7.4 | 2.7.1.8.1 | 2.7.1.8.2 | 2.7.1.8.3 | 2.7.1.8.4 |
| 2.7.2.1.1 | 2.7.2.1.2 | 2.7.2.1.3 | 2.7.2.1.4 | 2.7.2.2.1 | 2.7.2.2.2 | 2.7.2.2.3 | 2.7.2.2.4 |
| 2.7.2.3.1 | 2.7.2.3.2 | 2.7.2.3.3 | 2.7.2.3.4 | 2.7.2.4.1 | 2.7.2.4.2 | 2.7.2.4.3 | 2.7.2.4.4 |
| 2.7.2.5.1 | 2.7.2.5.2 | 2.7.2.5.3 | 2.7.2.5.4 | 2.7.2.6.1 | 2.7.2.6.2 | 2.7.2.6.3 | 2.7.2.6.4 |
| 2.7.2.7.1 | 2.7.2.7.2 | 2.7.2.7.3 | 2.7.2.7.4 | 2.7.2.8.1 | 2.7.2.8.2 | 2.7.2.8.3 | 2.7.2.8.4 |
| 2.7.3.1.1 | 2.7.3.1.2 | 2.7.3.1.3 | 2.7.3.1.4 | 2.7.3.2.1 | 2.7.3.2.2 | 2.7.3.2.3 | 2.7.3.2.4 |
| 2.7.3.3.1 | 2.7.3.3.2 | 2.7.3.3.3 | 2.7.3.3.4 | 2.7.3.4.1 | 2.7.3.4.2 | 2.7.3.4.3 | 2.7.3.4.4 |
| 2.7.3.5.1 | 2.7.3.5.2 | 2.7.3.5.3 | 2.7.3.5.4 | 2.7.3.6.1 | 2.7.3.6.2 | 2.7.3.6.3 | 2.7.3.6.4 |
| 2.7.3.7.1 | 2.7.3.7.2 | 2.7.3.7.3 | 2.7.3.7.4 | 2.7.3.8.1 | 2.7.3.8.2 | 2.7.3.8.3 | 2.7.3.8.4 |
| 2.7.4.1.1 | 2.7.4.1.2 | 2.7.4.1.3 | 2.7.4.1.4 | 2.7.4.2.1 | 2.7.4.2.2 | 2.7.4.2.3 | 2.7.4.2.4 |
| 2.7.4.3.1 | 2.7.4.3.2 | 2.7.4.3.3 | 2.7.4.3.4 | 2.7.4.4.1 | 2.7.4.4.2 | 2.7.4.4.3 | 2.7.4.4.4 |
| 2.7.4.5.1 | 2.7.4.5.2 | 2.7.4.5.3 | 2.7.4.5.4 | 2.7.4.6.1 | 2.7.4.6.2 | 2.7.4.6.3 | 2.7.4.6.4 |
| 2.7.4.7.1 | 2.7.4.7.2 | 2.7.4.7.3 | 2.7.4.7.4 | 2.7.4.8.1 | 2.7.4.8.2 | 2.7.4.8.3 | 2.7.4.8.4 |
| 2.8.1.1.1 | 2.8.1.1.2 | 2.8.1.1.3 | 2.8.1.1.4 | 2.8.1.2.1 | 2.8.1.2.2 | 2.8.1.2.3 | 2.8.1.2.4 |
| 2.8.1.3.1 | 2.8.1.3.2 | 2.8.1.3.3 | 2.8.1.3.4 | 2.8.1.4.1 | 2.8.1.4.2 | 2.8.1.4.3 | 2.8.1.4.4 |
| 2.8.1.5.1 | 2.8.1.5.2 | 2.8.1.5.3 | 2.8.1.5.4 | 2.8.1.6.1 | 2.8.1.6.2 | 2.8.1.6.3 | 2.8.1.6.4 |
| 2.8.1.7.1 | 2.8.1.7.2 | 2.8.1.7.3 | 2.8.1.7.4 | 2.8.1.8.1 | 2.8.1.8.2 | 2.8.1.8.3 | 2.8.1.8.4 |
| 2.8.2.1.1 | 2.8.2.1.2 | 2.8.2.1.3 | 2.8.2.1.4 | 2.8.2.2.1 | 2.8.2.2.2 | 2.8.2.2.3 | 2.8.2.2.4 |
| 2.8.2.3.1 | 2.8.2.3.2 | 2.8.2.3.3 | 2.8.2.3.4 | 2.8.2.4.1 | 2.8.2.4.2 | 2.8.2.4.3 | 2.8.2.4.4 |
| 2.8.2.5.1 | 2.8.2.5.2 | 2.8.2.5.3 | 2.8.2.5.4 | 2.8.2.6.1 | 2.8.2.6.2 | 2.8.2.6.3 | 2.8.2.6.4 |
| 2.8.2.7.1 | 2.8.2.7.2 | 2.8.2.7.3 | 2.8.2.7.4 | 2.8.2.8.1 | 2.8.2.8.2 | 2.8.2.8.3 | 2.8.2.8.4 |
| 2.8.3.1.1 | 2.8.3.1.2 | 2.8.3.1.3 | 2.8.3.1.4 | 2.8.3.2.1 | 2.8.3.2.2 | 2.8.3.2.3 | 2.8.3.2.4 |
| 2.8.3.3.1 | 2.8.3.3.2 | 2.8.3.3.3 | 2.8.3.3.4 | 2.8.3.4.1 | 2.8.3.4.2 | 2.8.3.4.3 | 2.8.3.4.4 |
| 2.8.3.5.1 | 2.8.3.5.2 | 2.8.3.5.3 | 2.8.3.5.4 | 2.8.3.6.1 | 2.8.3.6.2 | 2.8.3.6.3 | 2.8.3.6.4 |
| 2.8.3.7.1 | 2.8.3.7.2 | 2.8.3.7.3 | 2.8.3.7.4 | 2.8.3.8.1 | 2.8.3.8.2 | 2.8.3.8.3 | 2.8.3.8.4 |
| 2.8.4.1.1 | 2.8.4.1.2 | 2.8.4.1.3 | 2.8.4.1.4 | 2.8.4.2.1 | 2.8.4.2.2 | 2.8.4.2.3 | 2.8.4.2.4 |
| 2.8.4.3.1 | 2.8.4.3.2 | 2.8.4.3.3 | 2.8.4.3.4 | 2.8.4.4.1 | 2.8.4.4.2 | 2.8.4.4.3 | 2.8.4.4.4 |
| 2.8.4.5.1 | 2.8.4.5.2 | 2.8.4.5.3 | 2.8.4.5.4 | 2.8.4.6.1 | 2.8.4.6.2 | 2.8.4.6.3 | 2.8.4.6.4 |
| 2.8.4.7.1 | 2.8.4.7.2 | 2.8.4.7.3 | 2.8.4.7.4 | 2.8.4.8.1 | 2.8.4.8.2 | 2.8.4.8.3 | 2.8.4.8.4 |
| 3.1.1.1.1 | 3.1.1.1.2 | 3.1.1.1.3 | 3.1.1.1.4 | 3.1.1.2.1 | 3.1.1.2.2 | 3.1.1.2.3 | 3.1.1.2.4 |
| 3.1.1.3.1 | 3.1.1.3.2 | 3.1.1.3.3 | 3.1.1.3.4 | 3.1.1.4.1 | 3.1.1.4.2 | 3.1.1.4.3 | 3.1.1.4.4 |
| 3.1.1.5.1 | 3.1.1.5.2 | 3.1.1.5.3 | 3.1.1.5.4 | 3.1.1.6.1 | 3.1.1.6.2 | 3.1.1.6.3 | 3.1.1.6.4 |
| 3.1.1.7.1 | 3.1.1.7.2 | 3.1.1.7.3 | 3.1.1.7.4 | 3.1.1.8.1 | 3.1.1.8.2 | 3.1.1.8.3 | 3.1.1.8.4 |
| 3.1.2.1.1 | 3.1.2.1.2 | 3.1.2.1.3 | 3.1.2.1.4 | 3.1.2.2.1 | 3.1.2.2.2 | 3.1.2.2.3 | 3.1.2.2.4 |
| 3.1.2.3.1 | 3.1.2.3.2 | 3.1.2.3.3 | 3.1.2.3.4 | 3.1.2.4.1 | 3.1.2.4.2 | 3.1.2.4.3 | 3.1.2.4.4 |
| 3.1.2.5.1 | 3.1.2.5.2 | 3.1.2.5.3 | 3.1.2.5.4 | 3.1.2.6.1 | 3.1.2.6.2 | 3.1.2.6.3 | 3.1.2.6.4 |
| 3.1.2.7.1 | 3.1.2.7.2 | 3.1.2.7.3 | 3.1.2.7.4 | 3.1.2.8.1 | 3.1.2.8.2 | 3.1.2.8.3 | 3.1.2.8.4 |
| 3.1.3.1.1 | 3.1.3.1.2 | 3.1.3.1.3 | 3.1.3.1.4 | 3.1.3.2.1 | 3.1.3.2.2 | 3.1.3.2.3 | 3.1.3.2.4 |
| 3.1.3.3.1 | 3.1.3.3.2 | 3.1.3.3.3 | 3.1.3.3.4 | 3.1.3.4.1 | 3.1.3.4.2 | 3.1.3.4.3 | 3.1.3.4.4 |
| 3.1.3.5.1 | 3.1.3.5.2 | 3.1.3.5.3 | 3.1.3.5.4 | 3.1.3.6.1 | 3.1.3.6.2 | 3.1.3.6.3 | 3.1.3.6.4 |
| 3.1.3.7.1 | 3.1.3.7.2 | 3.1.3.7.3 | 3.1.3.7.4 | 3.1.3.8.1 | 3.1.3.8.2 | 3.1.3.8.3 | 3.1.3.8.4 |
| 3.1.4.1.1 | 3.1.4.1.2 | 3.1.4.1.3 | 3.1.4.1.4 | 3.1.4.2.1 | 3.1.4.2.2 | 3.1.4.2.3 | 3.1.4.2.4 |
| 3.1.4.3.1 | 3.1.4.3.2 | 3.1.4.3.3 | 3.1.4.3.4 | 3.1.4.4.1 | 3.1.4.4.2 | 3.1.4.4.3 | 3.1.4.4.4 |
| 3.1.4.5.1 | 3.1.4.5.2 | 3.1.4.5.3 | 3.1.4.5.4 | 3.1.4.6.1 | 3.1.4.6.2 | 3.1.4.6.3 | 3.1.4.6.4 |
| 3.1.4.7.1 | 3.1.4.7.2 | 3.1.4.7.3 | 3.1.4.7.4 | 3.1.4.8.1 | 3.1.4.8.2 | 3.1.4.8.3 | 3.1.4.8.4 |
| 3.2.1.1.1 | 3.2.1.1.2 | 3.2.1.1.3 | 3.2.1.1.4 | 3.2.1.2.1 | 3.2.1.2.2 | 3.2.1.2.3 | 3.2.1.2.4 |
| 3.2.1.3.1 | 3.2.1.3.2 | 3.2.1.3.3 | 3.2.1.3.4 | 3.2.1.4.1 | 3.2.1.4.2 | 3.2.1.4.3 | 3.2.1.4.4 |
| 3.2.1.5.1 | 3.2.1.5.2 | 3.2.1.5.3 | 3.2.1.5.4 | 3.2.1.6.1 | 3.2.1.6.2 | 3.2.1.6.3 | 3.2.1.6.4 |
| 3.2.1.7.1 | 3.2.1.7.2 | 3.2.1.7.3 | 3.2.1.7.4 | 3.2.1.8.1 | 3.2.1.8.2 | 3.2.1.8.3 | 3.2.1.8.4 |
| 3.2.2.1.1 | 3.2.2.1.2 | 3.2.2.1.3 | 3.2.2.1.4 | 3.2.2.2.1 | 3.2.2.2.2. | 3.2.2.2.3 | 3.2.2.2.4 |
| 3.2.2.3.1 | 3.2.2.3.2 | 3.2.2.3.3 | 3.2.2.3.4 | 3.2.2.4.1 | 3.2.2.4.2 | 3.2.2.4.3 | 3.2.2.4.4 |
| 3.2.2.5.1 | 3.2.2.5.2 | 3.2.2.5.3 | 3.2.2.5.4 | 3.2.2.6.1 | 3.2.2.6.2 | 3.2.2.6.3 | 3.2.2.6.4 |
| 3.2.2.7.1 | 3.2.2.7.2 | 3.2.2.7.3 | 3.2.2.7.4 | 3.2.2.8.1 | 3.2.2.8.2 | 3.2.2.8.3 | 3.2.2.8.4 |
| 3.2.3.1.1 | 3.2.3.1.2 | 3.2.3.1.3 | 3.2.3.1.4 | 3.2.3.2.1 | 3.2.3.2.2 | 3.2.3.2.3 | 3.2.3.2.4 |
| 3.2.3.3.1 | 3.2.3.3.2 | 3.2.3.3.3 | 3.2.3.3.4 | 3.2.3.4.1 | 3.2.3.4.2 | 3.2.3.4.3 | 3.2.3.4.4 |
| 3.2.3.5.1 | 3.2.3.5.2 | 3.2.3.5.3 | 3.2.3.5.4 | 3.2.3.6.1 | 3.2.3.6.2 | 3.2.3.6.3 | 3.2.3.6.4 |
| 3.2.3.7.1 | 3.2.3.7.2 | 3.2.3.7.3 | 3.2.3.7.4 | 3.2.3.8.1 | 3.2.3.8.2 | 3.2.3.8.3 | 3.2.3.8.4 |
| 3.2.4.1.1 | 3.2.4.1.2 | 3.2.4.1.3 | 3.2.4.1.4 | 3.2.4.2.1 | 3.2.4.2.2 | 3.2.4.2.3 | 3.2.4.2.4 |
| 3.2.4.3.1 | 3.2.4.3.2 | 3.2.4.3.3 | 3.2.4.3.4 | 3.2.4.4.1 | 3.2.4.4.2 | 3.2.4.4.3 | 3.2.4.4.4 |
| 3.2.4.5.1 | 3.2.4.5.2 | 3.2.4.5.3 | 3.2.4.5.4 | 3.2.4.6.1 | 3.2.4.6.2 | 3.2.4.6.3 | 3.2.4.6.4 |
| 3.2.4.7.1 | 3.2.4.7.2 | 3.2.4.7.3 | 3.2.4.7.4 | 3.2.4.8.1 | 3.2.4.8.2 | 3.2.4.8.3 | 3.2.4.8.4 |
| 3.3.1.1.1 | 3.3.1.1.2 | 3.3.1.1.3 | 3.3.1.1.4 | 3.3.1.2.1 | 3.3.1.2.2 | 3.3.1.2.3 | 3.3.1.2.4 |
| 3.3.1.3.1 | 3.3.1.3.2 | 3.3.1.3.3 | 3.3.1.3.4 | 3.3.1.4.1 | 3.3.1.4.2 | 3.3.1.4.3 | 3.3.1.4.4 |
| 3.3.1.5.1 | 3.3.1.5.2 | 3.3.1.5.3 | 3.3.1.5.4 | 3.3.1.6.1 | 3.3.1.6.2 | 3.3.1.6.3 | 3.3.1.6.4 |
| 3.3.1.7.1 | 3.3.1.7.2 | 3.3.1.7.3 | 3.3.1.7.4 | 3.3.1.8.1 | 3.3.1.8.2 | 3.3.1.8.3 | 3.3.1.8.4 |
| 3.3.2.1.1 | 3.3.2.1.2 | 3.3.2.1.3 | 3.3.2.1.4 | 3.3.2.2.1 | 3.3.2.2.2 | 3.3.2.2.3 | 3.3.2.2.4 |
| 3.3.2.3.1 | 3.3.2.3.2 | 3.3.2.3.3 | 3.3.2.3.4 | 3.3.2.4.1 | 3.3.2.4.2 | 3.3.2.4.3 | 3.3.2.4.4 |
| 3.3.2.5.1 | 3.3.2.5.2 | 3.3.2.5.3 | 3.3.2.5.4 | 3.3.2.6.1 | 3.3.2.6.2 | 3.3.2.6.3 | 3.3.2.6.4 |
| 3.3.2.7.1 | 3.3.2.7.2 | 3.3.2.7.3 | 3.3.2.7.4 | 3.3.2.8.1 | 3.3.2.8.2 | 3.3.2.8.3 | 3.3.2.8.4 |
| 3.3.3.1.1 | 3.3.3.1.2 | 3.3.3.1.3 | 3.3.3.1.4 | 3.3.3.2.1 | 3.3.3.2.2 | 3.3.3.2.3 | 3.3.3.2.4 |
| 3.3.3.3.1 | 3.3.3.3.2 | 3.3.3.3.3 | 3.3.3.3.4 | 3.3.3.4.1 | 3.3.3.4.2 | 3.3.3.4.3 | 3.3.3.4.4 |
| 3.3.3.5.1 | 3.3.3.5.2 | 3.3.3.5.3 | 3.3.3.5.4 | 3.3.3.6.1 | 3.3.3.6.2 | 3.3.3.6.3 | 3.3.3.6.4 |
| 3.3.3.7.1 | 3.3.3.7.2 | 3.3.3.7.3 | 3.3.3.7.4 | 3.3.3.8.1 | 3.3.3.8.2 | 3.3.3.8.3 | 3.3.3.8.4 |
| 3.3.4.1.1 | 3.3.4.1.2 | 3.3.4.1.3 | 3.3.4.1.4 | 3.3.4.2.1 | 3.3.4.2.2 | 3.3.4.2.3 | 3.3.4.2.4 |
| 3.3.4.3.1 | 3.3.4.3.2 | 3.3.4.3.3 | 3.3.4.3.4 | 3.3.4.4.1 | 3.3.4.4.2 | 3.3.4.4.3 | 3.3.4.4.4 |
| 3.3.4.5.1 | 3.3.4.5.2 | 3.3.4.5.3 | 3.3.4.5.4 | 3.3.4.6.1 | 3.3.4.6.2 | 3.3.4.6.3 | 3.3.4.6.4 |
| 3.3.4.7.1 | 3.3.4.7.2 | 3.3.4.7.3 | 3.3.4.7.4 | 3.3.4.8.1 | 3.3.4.8.2 | 3.3.4.8.3 | 3.3.4.8.4 |
| 3.4.1.1.1 | 3.4.1.1.2 | 3.4.1.1.3 | 3.4.1.1.4 | 3.4.1.2.1 | 3.4.1.2.2 | 3.4.1.2.3 | 3.4.1.2.4 |
| 3.4.1.3.1 | 3.4.1.3.2 | 3.4.1.3.3 | 3.4.1.3.4 | 3.4.1.4.1 | 3.4.1.4.2 | 3.4.1.4.3 | 3.4.1.4.4 |
| 3.4.1.5.1 | 3.4.1.5.2 | 3.4.1.5.3 | 3.4.1.5.4 | 3.4.1.6.1 | 3.4.1.6.2 | 3.4.1.6.3 | 3.4.1.6.4 |
| 3.4.1.7.1 | 3.4.1.7.2 | 3.4.1.7.3 | 3.4.1.7.4 | 3.4.1.8.1 | 3.4.1.8.2 | 3.4.1.8.3 | 3.4.1.8.4 |
| 3.4.2.1.1 | 3.4.2.1.2 | 3.4.2.1.3 | 3.4.2.1.4 | 3.4.2.2.1 | 3.4.2.2.2 | 3.4.2.2.3 | 3.4.2.2.4 |
| 3.4.2.3.1 | 3.4.2.3.2 | 3.4.2.3.3 | 3.4.2.3.4 | 3.4.2.4.1 | 3.4.2.4.2 | 3.4.2.4.3 | 3.4.2.4.4 |
| 3.4.2.5.1 | 3.4.2.5.2 | 3.4.2.5.3 | 3.4.2.5.4 | 3.4.2.6.1 | 3.4.2.6.2 | 3.4.2.6.3 | 3.4.2.6.4 |
| 3.4.2.7.1 | 3.4.2.7.2 | 3.4.2.7.3 | 3.4.2.7.4 | 3.4.2.8.1 | 3.4.2.8.2 | 3.4.2.8.3 | 3.4.2.8.4 |
| 3.4.3.1.1 | 3.4.3.1.2 | 3.4.3.1.3 | 3.4.3.1.4 | 3.4.3.2.1 | 3.4.3.2.2 | 3.4.3.2.3 | 3.4.3.2.4 |
| 3.4.3.3.1 | 3.4.3.3.2 | 3.4.3.3.3 | 3.4.3.3.4 | 3.4.3.4.1 | 3.4.3.4.2 | 3.4.3.4.3 | 3.4.3.4.4 |
| 3.4.3.5.1 | 3.4.3.5.2 | 3.4.3.5.3 | 3.4.3.5.4 | 3.4.3.6.1 | 3.4.3.6.2 | 3.4.3.6.3 | 3.4.3.6.4 |
| 3.4.3.7.1 | 3.4.3.7.2 | 3.4.3.7.3 | 3.4.3.7.4 | 3.4.3.8.1 | 3.4.3.8.2 | 3.4.3.8.3 | 3.4.3.8.4 |
| 3.4.4.1.1 | 3.4.4.1.2 | 3.4.4.1.3 | 3.4.4.1.4 | 3.4.4.2.1 | 3.4.4.2.2 | 3.4.4.2.3 | 3.4.4.2.4 |
| 3.4.4.3.1 | 3.4.4.3.2 | 3.4.4.3.3 | 3.4.4.3.4 | 3.4.4.4.1 | 3.4.4.4.2 | 3.4.4.4.3 | 3.4.4.4.4 |
| 3.4.4.5.1 | 3.4.4.5.2 | 3.4.4.5.3 | 3.4.4.5.4 | 3.4.4.6.1 | 3.4.4.6.2 | 3.4.4.6.3 | 3.4.4.6.4 |
| 3.4.4.7.1 | 3.4.4.7.2 | 3.4.4.7.3 | 3.4.4.7.4 | 3.4.4.8.1 | 3.4.4.8.2 | 3.4.4.8.3 | 3.4.4.8.4 |
| 3.5.1.1.1 | 3.5.1.1.2 | 3.5.1.1.3 | 3.5.1.1.4 | 3.5.1.2.1 | 3.5.1.2.2 | 3.5.1.2.3 | 3.5.1.2.4 |
| 3.5.1.3.1 | 3.5.1.3.2 | 3.5.1.3.3 | 3.5.1.3.4 | 3.5.1.4.1 | 3.5.1.4.2 | 3.5.1.4.3 | 3.5.1.4.4 |
| 3.5.1.5.1 | 3.5.1.5.2 | 3.5.1.5.3 | 3.5.1.5.4 | 3.5.1.6.1 | 3.5.1.6.2 | 3.5.1.6.3 | 3.5.1.6.4 |
| 3.5.1.7.1 | 3.5.1.7.2 | 3.5.1.7.3 | 3.5.1.7.4 | 3.5.1.8.1 | 3.5.1.8.2 | 3.5.1.8.3 | 3.5.1.8.4 |
| 3.5.2.1.1 | 3.5.2.1.2 | 3.5.2.1.3 | 3.5.2.1.4 | 3.5.2.2.1 | 3.5.2.2.2 | 3.5.2.2.3 | 3.5.2.2.4 |
| 3.5.2.3.1 | 3.5.2.3.2 | 3.5.2.3.3 | 3.5.2.3.4 | 3.5.2.4.1 | 3.5.2.4.2 | 3.5.2.4.3 | 3.5.2.4.4 |
| 3.5.2.5.1 | 3.5.2.5.2 | 3.5.2.5.3 | 3.5.2.5.4 | 3.5.2.6.1 | 3.5.2.6.2 | 3.5.2.6.3 | 3.5.2.6.4 |
| 3.5.2.7.1 | 3.5.2.7.2 | 3.5.2.7.3 | 3.5.2.7.4 | 3.5.2.8.1 | 3.5.2.8.2 | 3.5.2.8.3 | 3.5.2.8.4 |
| 3.5.3.1.1 | 3.5.3.1.2 | 3.5.3.1.3 | 3.5.3.1.4 | 3.5.3.2.1 | 3.5.3.2.2 | 3.5.3.2.3 | 3.5.3.2.4 |
| 3.5.3.3.1 | 3.5.3.3.2 | 3.5.3.3.3 | 3.5.3.3.4 | 3.5.3.4.1 | 3.5.3.4.2 | 3.5.3.4.3 | 3.5.3,4.4 |
| 3.5.3.5.1 | 3.5.3.5.2 | 3.5.3.5.3 | 3.5.3.5.4 | 3.5.3.6.1 | 3.5.3.6.2 | 3.5.3.6.3 | 3.5.3.6.4 |
| 3.5.3.7.1 | 3.5.3.7.2 | 3.5.3.7.3 | 3.5.3.7.4 | 3.5.3.8.1 | 3.5.3.8.2 | 3.5.3.8.3 | 3.5.3.8.4 |
| 3.5.4.1.1 | 3.5.4.1.2 | 3.5.4.1.3 | 3.5.4.1.4 | 3.5.4.2.1 | 3.5.4.2.2 | 3.5.4.2.3 | 3.5.4.2.4 |
| 3.5.4.3.1 | 3.5.4.3.2 | 3.5.4.3.3 | 3.5.4.3.4 | 3.5.4.4.1 | 3.5.4.4.2 | 3.5.4.4.3 | 3.5.4.4.4 |
| 3.5.4.5.1 | 3.5.4.5.2 | 3.5.4.5.3 | 3.5.4.5.4 | 3.5.4.6.1 | 3.5.4.6.2 | 3.5.4.6.3 | 3.5.4.6.4 |
| 3.5.4.7.1 | 3.5.4.7.2 | 3.5.4.7.3 | 3.5.4.7.4 | 3.5.4.8.1 | 3.5.4.8.2 | 3.5.4.8.3 | 3.5.4.8.4 |
| 3.6.1.1.1 | 3.6.1.1.2 | 3.6.1.1.3 | 3.6.1.1.4 | 3.6.1.2.1 | 3.6.1.2.2 | 3.6.1.2.3 | 3.6.1.2.4 |
| 3.6.1.3.1 | 3.6.1.3.2 | 3.6.1.3.3 | 3.6.1.3.4 | 3.6.1.4.1 | 3.6.1.4.2 | 3.6.1.4.3 | 3.6.1.4.4 |
| 3.6.1.5.1 | 3.6.1.5.2 | 3.6.1.5.3 | 3.6.1.5.4 | 3.6.1.6.1 | 3.6.1.6.2 | 3.6.1.6.3 | 3.6.1.6.4 |
| 3.6.1.7.1 | 3.6.1.7.2 | 3.6.1.7.3 | 3.6.1.7.4 | 3.6.1.8.1 | 3.6.1.8.2 | 3.6.1.8.3 | 3.6.1.8.4 |
| 3.6.2.1.1 | 3.6.2.1.2 | 3.6.2.1.3 | 3.6.2.1.4 | 3.6.2.2.1 | 3.6.2.2.2 | 3.6.2.2.3 | 3.6.2.2.4 |
| 3.6.2.3.1 | 3.6.2.3.2 | 3.6.2.3.3 | 3.6.2.3.4 | 3.6.2.4.1 | 3.6.2.4.2 | 3.6.2.4.3 | 3.6.2.4.4 |
| 3.6.2.5.1 | 3.6.2.5.2 | 3.6.2.5.3 | 3.6.2.5.4 | 3.6.2.6.1 | 3.6.2.6.2 | 3.6.2.6.3 | 3.6.2.6.4 |
| 3.6.2.7.1 | 3.6.2.7.2 | 3.6.2.7.3 | 3.6.2.7.4 | 3.6.2.8.1 | 3.6.2.8.2 | 3.6.2.8.3 | 3.6.2.8.4 |
| 3.6.3.1.1 | 3.6.3.1.2 | 3.6.3.1.3 | 3.6.3.1.4 | 3.6.3.2.1 | 3.6.3.2.2 | 3.6.3.2.3 | 3.6.3.2.4 |
| 3.6.3.3.1 | 3.6.3.3.2 | 3.6.3.3.3 | 3.6.3.3.4 | 3.6.3.4.1 | 3.6.3.4.2 | 3.6.3.4.3 | 3.6.3.4.4 |
| 3.6.3.5.1 | 3.6.3.5.2 | 3.6.3.5.3 | 3.6.3.5.4 | 3.6.3.6.1 | 3.6.3.6.2 | 3.6.3.6.3 | 3.6.3.6.4 |
| 3.6.3.7.1 | 3.6.3.7.2 | 3.6.3.7.3 | 3.6.3.7.4 | 3.6.3.8.1 | 3.6.3.8.2 | 3.6.3.8.3 | 3.6.3.8.4 |
| 3.6.4.1.1 | 3.6.4.1.2 | 3.6.4.1.3 | 3.6.4.1.4 | 3.6.4.2.1 | 3.6.4.2.2 | 3.6.4.2.3 | 3.6.4.2.4 |
| 3.6.4.3.1 | 3.6.4.3.2 | 3.6.4.3.3 | 3.6.4.3.4 | 3.6.4.4.1 | 3.6.4.4.2 | 3.6.4.4.3 | 3.6.4.4.4 |
| 3.6.4.5.1 | 3.6.4.5.2 | 3.6.4.5.3 | 3.6.4.5.4 | 3.6.4.6.1 | 3.6.4.6.2 | 3.6.4.6.3 | 3.6.4.6.4 |
| 3.6.4.7.1 | 3.6.4.7.2 | 3.6.4.7.3 | 3.6.4.7.4 | 3.6.4.8.1 | 3.6.4.8.2 | 3.6.4.8.3 | 3.6.4.8.4 |
| 3.7.1.1.1 | 3.7.1.1.2 | 3.7.1.1.3 | 3.7.1.1.4 | 3.7.1.2.1 | 3.7.1.2.2 | 3.7.1.2.3 | 3.7.1.2.4 |
| 3.7.1.3.1 | 3.7.1.3.2 | 3.7.1.3.3 | 3.7.1.3.4 | 3.7.1.4.1 | 3.7.1.4.2 | 3.7.1.4.3 | 3.7.1.4.4 |
| 3.7.1.5.1 | 3.7.1.5.2 | 3.7.1.5.3 | 3.7.1.5.4 | 3.7.1.6.1 | 3.7.1.6.2 | 3.7.1.6.3 | 3.7.1.6.4 |
| 3.7.1.7.1 | 3.7.1.7.2 | 3.7.1.7.3 | 3.7.1.7.4 | 3.7.1.8.1 | 3.7.1.8.2 | 3.7.1.8.3 | 3.7.1.8.4 |
| 3.7.2.1.1 | 3.7.2.1.2 | 3.7.2.1.3 | 3.7.2.1.4 | 3.7.2.2.1 | 3.7.2.2.2 | 3.7.2.2.3 | 3.7.2.2.4 |
| 3.7.2.3.1 | 3.7.2.3.2 | 3.7.2.3.3 | 3.7.2.3.4 | 3.7.2.4.1 | 3.7.2.4.2 | 3.7.2.4.3 | 3.7.2.4.4 |
| 3.7.2.5.1 | 3.7.2.5.2 | 3.7.2.5.3 | 3.7.2.5.4 | 3.7.2.6.1 | 3.7.2.6.2 | 3.7.2.6.3 | 3.7.2.6.4 |
| 3.7.2.7.1 | 3.7.2.7.2 | 3.7.2.7.3 | 3.7.2.7.4 | 3.7.2.8.1 | 3.7.2.8.2 | 3.7.2.8.3 | 3.7.2.8.4 |
| 3.7.3.1.1 | 3.7.3.1.2 | 3.7.3.1.3 | 3.7.3.1.4 | 3.7.3.2.1 | 3.7.3.2.2 | 3.7.3.2.3 | 3.7.3.2.4 |
| 3.7.3.3.1 | 3.7.3.3.2 | 3.7.3.3.3 | 3.7.3.3.4 | 3.7.3.4.1 | 3.7.3.4.2 | 3.7.3.4.3 | 3.7.3.4.4 |
| 3.7.3.5.1 | 3.7.3.5.2 | 3.7.3.5.3 | 3.7.3.5.4 | 3.7.3.6.1 | 3.7.3.6.2 | 3.7.3.6.3 | 3.7.3.6.4 |
| 3.7.3.7.1 | 3.7.3.7.2 | 3.7.3.7.3 | 3.7.3.7.4 | 3.7.3.8.1 | 3.7.3.8.2 | 3.7.3.8.3 | 3.7.3.8.4 |
| 3.7.4.1.1 | 3.7.4.1.2 | 3.7.4.1.3 | 3.7.4.1.4 | 3.7.4.2.1 | 3.7.4.2.2 | 3.7.4.2.3 | 3.7.4.2.4 |
| 3.7.4.3.1 | 3.7.4.3.2 | 3.7.4.3.3 | 3.7.4.3.4 | 3.7.4.4.1 | 3.7.4.4.2 | 3.7.4.4.3 | 3.7.4.4.4 |
| 3.7.4.5.1 | 3.7.4.5.2 | 3.7.4.5.3 | 3.7.4.5.4 | 3.7.4.6.1 | 3.7.4.6.2 | 3.7.4.6.3 | 3.7.4.6.4 |
| 3.7.4.7.1 | 3.7.4.7.2 | 3.7.4.7.3 | 3.7.4.7.4 | 3.7.4.8.1 | 3.7.4.8.2 | 3.7.4.8.3 | 3.7.4.8.4 |
| 3.8.1.1.1 | 3.8.1.1.2 | 3.8.1.1.3 | 3.8.1.1.4 | 3.8.1.2.1 | 3.8.1.2.2 | 3.8.1.2.3 | 3.8.1.2.4 |
| 3.8.1.3.1 | 3.8.1.3.2 | 3.8.1.3.3 | 3.8.1.3.4 | 3.8.1.4.1 | 3.8.1.4.2 | 3.8.1.4.3 | 3.8.1.4.4 |
| 3.8.1.5.1 | 3.8.1.5.2 | 3.8.1.5.3 | 3.8.1.5.4 | 3.8.1.6.1 | 3.8.1.6.2 | 3.8.1.6.3 | 3.8.1.6.4 |
| 3.8.1.7.1 | 3.8.1.7.2 | 3.8.1.7.3 | 3.8.1.7.4 | 3.8.1.8.1 | 3.8.1.8.2 | 3.8.1.8.3 | 3.8.1.8.4 |
| 3.8.2.1.1 | 3.8.2.1.2 | 3.8.2.1.3 | 3.8.2.1.4 | 3.8.2.2.1 | 3.8.2.2.2 | 3.8.2.2.3 | 3.8.2.2.4 |
| 3.8.2.3.1 | 3.8.2.3.2 | 3.8.2.3.3 | 3.8.2.3.4 | 3.8.2.4.1 | 3.8.2.4.2 | 3.8.2.4.3 | 3.8.2.4.4 |
| 3.8.2.5.1 | 3.8.2.5.2 | 3.8.2.5.3 | 3.8.2.5.4 | 3.8.2.6.1 | 3.8.2.6.2 | 3.8.2.6.3 | 3.8.2.6.4 |
| 3.8.2.7.1 | 3.8.2.7.2 | 3.8.2.7.3 | 3.8.2.7.4 | 3.8.2.8.1 | 3.8.2.8.2 | 3.8.2.8.3 | 3.8.2.8.4 |
| 3.8.3.1.1 | 3.8.3.1.2 | 3.8.3.1.3 | 3.8.3.1.4 | 3.8.3.2.1 | 3.8.3.2.2 | 3.8.3.2.3 | 3.8.3.2.4 |
| 3.8.3.3.1 | 3.8.3.3.2 | 3.8.3.3.3 | 3.8.3.3.4 | 3.8.3.4.1 | 3.8.3.4.2 | 3.8.3.4.3 | 3.8.3.4.4 |
| 3.8.3.5.1 | 3.8.3.5.2 | 3.8.3.5.3 | 3.8.3.5.4 | 3.8.3.6.1 | 3.8.3.6.2 | 3.8.3.6.3 | 3.8.3.6.4 |
| 3.8.3.7.1 | 3.8.3.7.2 | 3.8.3.7.3 | 3.8.3.7.4 | 3.8.3.8.1 | 3.8.3.8.2 | 3.8.3.8.3 | 3.8.3.8.4 |
| 3.8.4.1.1 | 3.8.4.1.2 | 3.8.4.1.3 | 3.8.4.1.4 | 3.8.4.2.1 | 3.8.4.2.2 | 3.8.4.2.3 | 3.8.4.2.4 |
| 3.8.4.3.1 | 3.8.4.3.2 | 3.8.4.3.3 | 3.8.4.3.4 | 3.8.4.4.1 | 3.8.4.4.2 | 3.8.4.4.3 | 3.8.4.4.4 |
| 3.8.4.5.1 | 3.8.4.5.2 | 3.8.4.5.3 | 3.8.4.5.4 | 3.8.4.6.1 | 3.8.4.6.2 | 3.8.4.6.3 | 3.8.4.6.4 |
| 3.8.4.7.1 | 3.8.4.7.2 | 3.8.4.7.3 | 3.8.4.7.4 | 3.8.4.8.1 | 3.8.4.8.2 | 3.8.4.8.3 | 3.8.4.8.4 |
| 4.1.1.1.1 | 4.1.1.1.2 | 4.1.1.1.3 | 4.1.1.1.4 | 4.1.1.2.1 | 4.1.1.2.2 | 4.1.1.2.3 | 4.1.1.2.4 |
| 4.1.1.3.1 | 4.1.1.3.2 | 4.1.1.3.3 | 4.1.1.3.4 | 4.1.1.4.1 | 4.1.1.4.2 | 4.1.1.4.3 | 4.1.1.4.4 |
| 4.1.1.5.1 | 4.1.1.5.2 | 4.1.1.5.3 | 4.1.1.5.4 | 4.1.1.6.1 | 4.1.1.6.2 | 4.1.1.6.3 | 4.1.1.6.4 |
| 4.1.1.7.1 | 4.1.1.7.2 | 4.1.1.7.3 | 4.1.1.7.4 | 4.1.1.8.1 | 4.1.1.8.2 | 4.1.1.8.3 | 4.1.1.8.4 |
| 4.1.2.1.1 | 4.1.2.1.2 | 4.1.2.1.3 | 4.1.2.1.4 | 4.1.2.2.1 | 4.1.2.2.2 | 4.1.2.2.3 | 4.1.2.2.4 |
| 4.1.2.3.1 | 4.1.2.3.2 | 4.1.2.3.3 | 4.1.2.3.4 | 4.1.2.4.1 | 4.1.2.4.2 | 4.1.2.4.3 | 4.1.2.4.4 |
| 4.1.2.5.1 | 4.1.2.5.2 | 4.1.2.5.3 | 4.1.2.5.4 | 4.1.2.6.1 | 4.1.2.6.2 | 4.1.2.6.3 | 4.1.2.6.4 |
| 4.1.2.7.1 | 4.1.2.7.2 | 4.1.2.7.3 | 4.1.2.7.4 | 4.1.2.8.1 | 4.1.2.8.2 | 4.1.2.8.3 | 4.1.2.8.4 |
| 4.1.3.1.1 | 4.1.3.1.2 | 4.1.3.1.3 | 4.1.3.1.4 | 4.1.3.2.1 | 4.1.3.2.2 | 4.1.3.2.3 | 4.1.3.2.4 |
| 4.1.3.3.1 | 4.1.3.3.2 | 4.1.3.3.3 | 4.1.3.3.4 | 4.1.3.4.1 | 4.1.3.4.2 | 4.1.3.4.3 | 4.1.3.4.4 |
| 4.1.3.5.1 | 4.1.3.5.2 | 4.1.3.5.3 | 4.1.3.5.4 | 4.1.3.6.1 | 4.1.3.6.2 | 4.1.3.6.3 | 4.1.3.6.4 |
| 4.1.3.7.1 | 4.1.3.7.2 | 4.1.3.7.3 | 4.1.3.7.4 | 4.1.3.8.1 | 4.1.3.8.2 | 4.1.3.8.3 | 4.1.3.8.4 |
| 4.1.4.1.1 | 4.1.4.1.2 | 4.1.4.1.3 | 4.1.4.1.4 | 4.1.4.2.1 | 4.1.4.2.2 | 4.1.4.2.3 | 4.1.4.2.4 |
| 4.1.4.3.1 | 4.1.4.3.2 | 4.1.4.3.3 | 4.1.4.3.4 | 4.1.4.4.1 | 4.1.4.4.2 | 4.1.4.4.3 | 4.1.4.4.4 |
| 4.1.4.5.1 | 4.1.4.5.2 | 4.1.4.5.3 | 4.1.4.5.4 | 4.1.4.6.1 | 4.1.4.6.2 | 4.1.4.6.3 | 4.1.4.6.4 |
| 4.1.4.7.1 | 4.1.4.7.2 | 4.1.4.7.3 | 4.1.4.7.4 | 4.1.4.8.1 | 4.1.4.8.2 | 4.1.4.8.3 | 4.1.4.8.4 |
| 4.2.1.1.1 | 4.2.1.1.2 | 4.2.1.1.3 | 4.2.1.1.4 | 4.2.1.2.1 | 4.2.1.2.2 | 4.2.1.2.3 | 4.2.1.2.4 |
| 4.2.1.3.1 | 4.2.1.3.2 | 4.2.1.3.3 | 4.2.1.3.4 | 4.2.1.4.1 | 4.2.1.4.2 | 4.2.1.4.3 | 4.2.1.4.4 |
| 4.2.1.5.1 | 4.2.1.5.2 | 4.2.1.5.3 | 4.2.1.5.4 | 4.2.1.6.1 | 4.2.1.6.2 | 4.2.1.6.3 | 4.2.1.6.4 |
| 4.2.1.7.1 | 4.2.1.7.2 | 4.2.1.7.3 | 4.2.1.7.4 | 4.2.1.8.1 | 4.2.1.8.2 | 4.2.1.8.3 | 4.2.1.8.4 |
| 4.2.2.1.1 | 4.2.2.1.2 | 4.2.2.1.3 | 4.2.2.1.4 | 4.2.2.2.1 | 4.2.2.2.2 | 4.2.2.2.3 | 4.2.2.2.4 |
| 4.2.2.3.1 | 4.2.2.3.2 | 4.2.2.3.3 | 4.2.2.3.4 | 4.2.2.4.1 | 4.2.2.4.2 | 4.2.2.4.3 | 4.2.2.4.4 |
| 4.2.2.5.1 | 4.2.2.5.2 | 4.2.2.5.3 | 4.2.2.5.4 | 4.2.2.6.1 | 4.2.2.6.2 | 4.2.2.6.3 | 4.2.2.6.4 |
| 4.2.2.7.1 | 4.2.2.7.2 | 4.2.2.7.3 | 4.2.2.7.4 | 4.2.2.8.1 | 4.2.2.8.2 | 4.2.2.8.3 | 4.2.2.8.4 |
| 4.2.3.1.1 | 4.2.3.1.2 | 4.2.3.1.3 | 4.2.3.1.4 | 4.2.3.2.1 | 4.2.3.2.2 | 4.2.3.2.3 | 4.2.3.2.4 |
| 4.2.3.3.1 | 4.2.3.3.2 | 4.2.3.3.3 | 4.2.3.3.4 | 4.2.3.4.1 | 4.2.3.4.2 | 4.2.3.4.3 | 4.2.3.4.4 |
| 4.2.3.5.1 | 4.2.3.5.2 | 4.2.3.5.3 | 4.2.3.5.4 | 4.2.3.6.1 | 4.2.3.6.2 | 4.2.3.6.3 | 4.2.3.6.4 |
| 4.2.3.7.1 | 4.2.3.7.2 | 4.2.3.7.3 | 4.2.3.7.4 | 4.2.3.8.1 | 4.2.3.8.2 | 4.2.3.8.3 | 4.2.3.8.4 |
| 4.2.4.1.1 | 4.2.4.1.2 | 4.2.4.1.3 | 4.2.4.1.4 | 4.2.4.2.1 | 4.2.4.2.2 | 4.2.4.2.3 | 4.2.4.2.4 |
| 4.2.4.3.1 | 4.2.4.3.2 | 4.2.4.3.3 | 4.2.4.3.4 | 4.2.4.4.1 | 4.2.4.4.2 | 4.2.4.4.3 | 4.2.4.4.4 |
| 4.2.4.5.1 | 4.2.4.5.2 | 4.2.4.5.3 | 4.2.4.5.4 | 4.2.4.6.1 | 4.2.4.6.2 | 4.2.4.6.3 | 4.2.4.6.4 |
| 4.2.4.7.1 | 4.2.4.7.2 | 4.2.4.7.3 | 4.2.4.7.4 | 4.2.4.8.1 | 4.2.4.8.2 | 4.2.4.8.3 | 4.2.4.8.4 |
| 4.3.1.1.1 | 4.3.1.1.2 | 4.3.1.1.3 | 4.3.1.1.4 | 4.3.1.2.1 | 4.3.1.2.2 | 4.3.1.2.3 | 4.3.1.2.4 |
| 4.3.1.3.1 | 4.3.1.3.2 | 4.3.1.3.3 | 4.3.1.3.4 | 4.3.1.4.1 | 4.3.1.4.2 | 4.3.1.4.3 | 4.3.1.4.4 |
| 4.3.1.5.1 | 4.3.1.5.2 | 4.3.1.5.3 | 4.3.1.5.4 | 4.3.1.6.1 | 4.3.1.6.2 | 4.3.1.6.3 | 4.3.1.6.4 |
| 4.3.1.7.1 | 4.3.1.7.2 | 4.3.1.7.3 | 4.3.1.7.4 | 4.3.1.8.1 | 4.3.1.8.2 | 4.3.1.8.3 | 4.3.1.8.4 |
| 4.3.2.1.1 | 4.3.2.1.2 | 4.3.2.1.3 | 4.3.2.1.4 | 4.3.2.2.1 | 4.3.2.2.2 | 4.3.2.2.3 | 4.3.2.2.4 |
| 4.3.2.3.1 | 4.3.2.3.2 | 4.3.2.3.3 | 4.3.2.3.4 | 4.3.2.4.1 | 4.3.2.4.2 | 4.3.2.4.3 | 4.3.2.4.4 |
| 4.3.2.5.1 | 4.3.2.5.2 | 4.3.2.5.3 | 4.3.2.5.4 | 4.3.2.6.1 | 4.3.2.6.2 | 4.3.2.6.3 | 4.3.2.6.4 |
| 4.3.2.7.1 | 4.3.2.7.2 | 4.3.2.7.3 | 4.3.2.7.4 | 4.3.2.8.1 | .4.3 2.8.2 | 4.3.2.8.3 | 4.3.2.8.4 |
| 4.3.3.1.1 | 4.3.3.1.2 | 4.3.3.1.3 | 4.3.3.1.4 | 4.3.3.2.1 | 4.3.3.2.2 | 4.3.3.2.3 | 4.3.3.2.4 |
| 4.3.3.3.1 | 4.3.3.3.2 | 4.3.3.3.3 | 4.3.3.3.4 | 4.3.3.4.1 | 4.3.3.4.2 | 4.3.3.4.3 | 4.3.3.4.4 |
| 4.3.3.5.1 | 4.3.3.5.2 | 4.3.3.5.3 | 4.3.3.5.4 | 4.3.3.6.1 | 4.3.3.6.2 | 4.3.3.6.3 | 4.3.3.6.4 |
| 4.3.3.7.1 | 4.3.3.7.2 | 4.3.3.7.3 | 4.3.3.7.4 | 4.3.3.8.1 | 4.3.3.8.2 | 4.3.3.8.3 | 4.3.3.8.4 |
| 4.3.4.1.1 | 4.3.4.1.2 | 4.3.4.1.3 | 4.3.4.1.4 | 4.3.4.2.1 | 4.3.4.2.2 | 4.3.4.2.3 | 4.3.4.2.4 |
| 4.3.4.3.1 | 4.3.4.3.2 | 4.3.4.3.3 | 4.3.4.3.4 | 4,3.4.4.1 | 4.3.4.4.2 | 4.3.4.4.3 | 4.3.4.4.4 |
| 4.3.4.5.1 | 4.3.4.5.2 | 4.3.4.5.3 | 4.3.4.5.4 | 4.3.4.6.1 | 4.3.4.6.2 | 4.3.4.6.3 | 4.3.4.6.4 |
| 4.3.4.7.1 | 4.3.4.7.2 | 4.3.4.7.3 | 4.3.4.7.4 | 4.3.4.8.1 | 4.3.4.8.2 | 4.3.4.8.3 | 4.3.4.8.4 |
| 4.4.1.1.1 | 4.4.1.1.2 | 4.4.1.1.3 | 4.4.1.1.4 | 4.4.1.2.1 | 4.4.1.2.2 | 4.4.1.2.3 | 4.4.1.2.4 |
| 4.4.1.3.1 | 4.4.1.3.2 | 4.4.1.3.3 | 4.4.1.3.4 | 4.4.1.4.1 | 4.4.1.4.2 | 4.4.1.4.3 | 4.4.1.4.4 |
| 4.4.1.5.1 | 4.4.1.5.2 | 4.4.1.5.3 | 4.4.1.5.4 | 4.4.1.6.1 | 4.4.1.6.2 | 4.4.1.6.3 | 4.4.1.6.4 |
| 4.4.1.7.1 | 4.4.1.7.2 | 4.4.1.7.3 | 4.4.1.7.4 | 4.4.1.8.1 | 4.4.1.8.2 | 4.4.1.8.3 | 4.4.1.8.4 |
| 4.4.2.1.1 | 4.4.2.1.2 | 4.4.2.1.3 | 4.4.2.1.4 | 4.4.2.2.1 | 4.4.2.2.2 | 4.4.2.2.3 | 4.4.2.2.4 |
| 4.4.2.3.1 | 4.4.2.3.2 | 4.4.2.3.3 | 4.4.2.3.4 | 4.4.2.4.1 | 4.4.2.4.2 | 4.4.2.4.3 | 4.4.2.4.4 |
| 4.4.2.5.1 | 4.4.2.5.2 | 4.4.2.5.3 | 4.4.2.5.4 | 4.4.2.6.1 | 4.4.2.6.2 | 4.4.2.6.3 | 4.4.2.6.4 |
| 4.4.2.7.1 | 4.4.2.7.2 | 4.4.2.7.3 | 4.4.2.7.4 | 4.4.2.8.1 | 4.4.2.8.2 | 4.4.2.8.3 | 4.4.2.8.4 |
| 4.4.3.1.1 | 4.4.3.1.2 | 4.4.3.1.3 | 4.4.3.1.4 | 4.4.3.2.1 | 4.4.3.2.2 | 4.4.3.2.3 | 4.4.3.2.4 |
| 4.4.3.3.1 | 4.4.3.3.2 | 4.4.3.3.3 | 4.4.3.3.4 | 4.4.3.4.1 | 4.4.3.4.2 | 4.4.3.4.3 | 4.4.3.4.4 |
| 4.4.3.5.1 | 4.4.3.5.2 | 4.4.3.5.3 | 4.4.3.5.4 | 4.4.3.6.1 | 4.4.3.6.2 | 4.4.3.6.3 | 4.4.3.6.4 |
| 4.4.3.7.1 | 4.4.3.7.2 | 4.4.3.7.3 | 4.4.3.7.4 | 4.4.3.8.1 | 4.4.3.8.2 | 4.4.3.8.3 | 4.4.3.8.4 |
| 4.4.4.1.1 | 4.4.4.1.2 | 4.4.4.1.3 | 4.4.4.1.4 | 4.4.4.2.1 | 4.4.4.2.2 | 4.4.4.2.3 | 4.4.4.2.4 |
| 4.4.4.3.1 | 4.4.4.3.2 | 4.4.4.3.3 | 4.4.4.3.4 | 4.4.4.4.1 | 4.4.4.4.2 | 4.4.4.4.3 | 4.4.4.4.4 |
| 4.4.4.5.1 | 4.4.4.5.2 | 4.4.4.5.3 | 4.4.4.5.4 | 4.4.4.6.1 | 4.4.4.6.2 | 4.4.4.6.3 | 4.4.4.6.4 |
| 4.4.4.7.1 | 4.4.4.7.2 | 4.4.4.7.3 | 4.4.4.7.4 | 4.4.4.8.1 | 4.4.4.8.2 | 4.4.4.8.3 | 4.4.4.8.4 |
| 4.5.1.1.1 | 4.5.1.1.2 | 4.5.1.1.3 | 4.5.1.1.4 | 4.5.1.2.1 | 4.5.1.2.2 | 4.5.1.2.3 | 4.5.1.2.4 |
| 4.5.1.3.1 | 4.5.1.3.2 | 4.5.1.3.3 | 4.5.1.3.4 | 4.5.1.4.1 | 4.5.1.4.2 | 4.5.1.4.3 | 4.5.1.4.4 |
| 4.5.1.5.1 | 4.5.1.5.2 | 4.5.1.5.3 | 4.5.1.5.4 | 4.5.1.6.1 | 4.5.1.6.2 | 4.5.1.6.3 | 4.5.1.6.4 |
| 4.5.1.7.1 | 4.5.1.7.2 | 4.5.1.7.3 | 4.5.1.7.4 | 4.5.1.8.1 | 4.5.1.8.2 | 4.5.1.8.3 | 4.5.1.8.4 |
| 4.5.2.1.1 | 4.5.2.1.2 | 4.5.2.1.3 | 4.5.2.1.4 | 4.5.2.2.1 | 4.5.2.2.2 | 4.5.2.2.3 | 4.5.2.2.4 |
| 4.5.2.3.1 | 4.5.2.3.2 | 4.5.2.3.3 | 4.5.2.3.4 | 4.5.2.4.1 | 4.5.2.4.2 | 4.5.2.4.3 | 4.5.2.4.4 |
| 4.5.2.5.1 | 4.5.2.5.2 | 4.5.2.5.3 | 4.5.2.5.4 | 4.5.2.6.1 | 4.5.2.6.2 | 4.5.2.6.3 | 4.5.2.6.4 |
| 4.5.2.7.1 | 4.5.2.7.2 | 4.5.2.7.3 | 4.5.2.7.4 | 4.5.2.8.1 | 4.5.2.8.2 | 4.5.2.8.3 | 4.5.2.8.4 |
| 4.5.3.1.1 | 4.5.3.1.2 | 4.5.3.1.3 | 4.5.3.1.4 | 4.5.3.2.1 | 4.5.3.2.2 | 4.5.3.2.3 | 4.5.3.2.4 |
| 4.5.3.3.1 | 4.5.3.3.2 | 4.5.3.3.3 | 4.5.3.3.4 | 4.5.3.4.1 | 4.5.3.4.2 | 4.5.3.4.3 | 4.5.3.4.4 |
| 4.5.3.5.1 | 4.5.3.5.2 | 4.5.3.5.3 | 4.5.3.5.4 | 4.5.3.6.1 | 4.5.3.6.2 | 4.5.3.6.3 | 4.5.3.6.4 |
| 4.5.3.7.1 | 4.5.3.7.2 | 4.5.3.7.3 | 4.5.3.7.4 | 4.5.3.8.1 | 4.5.3.8.2 | 4.5.3.8.3 | 4.5.3.8.4 |
| 4.5.4.1.1 | 4.5.4.1.2 | 4.5.4.1.3 | 4.5.4.1.4 | 4.5.4.2.1 | 4.5.4.2.2 | 4.5.4.2.3 | 4.5.4.2.4 |
| 4.5.4.3.1 | 4.5.4.3.2 | 4.5.4.3.3 | 4.5.4.3.4 | 4.5.4.4.1 | 4.5.4.4.2 | 4.5.4.4.3 | 4.5.4.4.4 |
| 4.5.4.5.1 | 4.5.4.5.2 | 4.5.4.5.3 | 4.5.4.5.4 | 4.5.4.6.1 | 4.5.4.6.2 | 4.5.4.6.3 | 4.5.4.6.4 |
| 4.5.4.7.1 | 4.5.4.7.2 | 4.5.4.7.3 | 4.5.4.7.4 | 4.5.4.8.1 | 4.5.4.8.2 | 4.5.4.8.3 | 4.5.4.8.4 |
| 4.6.1.1.1 | 4.6.1.1.2 | 4.6.1.1.3 | 4.6.1.1.4 | 4.6.1.2.1 | 4.6.1.2.2 | 4.6.1.2.3 | 4.6.1.2.4 |
| 4.6.1.3.1 | 4.6.1.3.2 | 4.6.1.3.3 | 4.6.1.3.4 | 4.6.1.4.1 | 4.6.1.4.2 | 4.6.1.4.3 | 4.6.1.4.4 |
| 4.6.1.5.1 | 4.6.1.5.2 | 4.6.1.5.3 | 4.6.1.5.4 | 4.6.1.6.1 | 4.6.1.6.2 | 4.6.1.6.3 | 4.6.1.6.4 |
| 4.6.1.7.1 | 4.6.1.7.2 | 4.6.1.7.3 | 4.6.1.7.4 | 4.6.1.8.1 | 4.6.1.8.2 | 4.6.1.8.3 | 4.6.1.8.4 |
| 4.6.2.1.1 | 4.6.2.1.2 | 4.6.2.1.3 | 4.6.2.1.4 | 4.6.2.2.1 | 4.6.2.2.2 | 4.6.2.2.3 | 4.6.2.2.4 |
| 4.6.2.3.1 | 4.6.2.3.2 | 4.6.2.3.3 | 4.6.2.3.4 | 4.6.2.4.1 | 4.6.2.4.2 | 4.6.2.4.3 | 4.6.2.4.4 |
| 4.6.2.5.1 | 4.6.2.5.2 | 4.6.2.5.3 | 4.6.2.5.4 | 4.6.2.6.1 | 4.6.2.6.2 | 4.6.2.6.3 | 4.6.2.6.4 |
| 4.6.2.7.1 | 4.6.2.7.2 | 4.6.2.7.3 | 4.6.2.7.4 | 4.6.2.8.1 | 4.6.2.8.2 | 4.6.2.8.3 | 4.6.2.8.4 |
| 4.6.3.1.1 | 4.6.3.1.2 | 4.6.3.1.3 | 4.6.3.1.4 | 4.6.3.2.1 | 4.6.3.2.2 | 4.6.3.2.3 | 4.6.3.2.4 |
| 4.6.3.3.1 | 4.6.3.3.2 | 4.6.3.3.3 | 4.6.3.3.4 | 4.6.3.4.1 | 4.6.3.4.2 | 4.6.3.4.3 | 4.6.3.4.4 |
| 4.6.3.5.1 | 4.6.3.5.2 | 4.6.3.5.3 | 4.6.3.5.4 | 4.6.3.6.1 | 4.6.3.6.2 | 4.6.3.6.3 | 4.6.3.6.4 |
| 4.6.3.7.1 | 4.6.3.7.2 | 4.6.3.7.3 | 4.6.3.7.4 | 4.6.3.8.1 | 4.6.3.8.2 | 4.6.3.8.3 | 4.6.3.8.4 |
| 4.6.4.1.1 | 4.6.4.1.2 | 4.6.4.1.3 | 4.6.4.1.4 | 4.6.4.2.1 | 4.6.4.2.2 | 4.6.4.2.3 | 4.6.4.2.4 |
| 4.6.4.3.1 | 4.6.4.3.2 | 4.6.4.3.3 | 4.6.4.3.4 | 4.6.4.4.1 | 4.6.4.4.2 | 4.6.4.4.3 | 4.6.4.4.4 |
| 4.6.4.5.1 | 4.6.4.5.2 | 4.6.4.5.3 | 4.6.4.5.4 | 4.6.4.6.1 | 4.6.4.6.2 | 4.6.4.6.3 | 4.6.4.6.4 |
| 4.6.4.7.1 | 4.6.4.7.2 | 4.6.4.7.3 | 4.6.4.7.4 | 4.6.4.8.1 | 4.6.4.8.2 | 4.6.4.8.3 | 4.6.4.8.4 |
| 4.7.1.1.1 | 4.7.1.1.2 | 4.7.1.1.3 | 4.7.1.1.4 | 4.7.1.2.1 | 4.7.1.2.2 | 4.7.1.2.3 | 4.7.1.2.4 |
| 4.7.1.3.1 | 4.7.1.3.2 | 4.7.1.3.3 | 4.7.1.3.4 | 4.7.1.4.1 | 4.7.1.4.2 | 4.7.1.4.3 | 4.7.1.4.4 |
| 4.7.1.5.1 | 4.7.1.5.2 | 4.7.1.5.3 | 4.7.1.5.4 | 4.7.1.6.1 | 4.7.1.6.2 | 4.7.1.6.3 | 4.7.1.6.4 |
| 4.7.1.7.1 | 4.7.1.7.2 | 4.7.1.7.3 | 4.7.1.7.4 | 4.7.1.8.1 | 4.7.1.8.2 | 4.7.1.8.3 | 4.7.1.8.4 |
| 4.7.2.1.1 | 4.7.2.1.2 | 4.7.2.1.3 | 4.7.2.1.4 | 4.7.2.2.1 | 4.7.2.2.2 | 4.7.2.2.3 | 4.7.2.2.4 |
| 4.7.2.3.1 | 4.7.2.3.2 | 4.7.2.3.3 | 4.7.2.3.4 | 4.7.2.4.1 | 4.7.2.4.2 | 4.7.2.4.3 | 4.7.2.4.4 |
| 4.7.2.5.1 | 4.7.2.5.2 | 4.7.2.5.3 | 4.7.2.5.4 | 4.7.2.6.1 | 4.7.2.6.2 | 4.7.2.6.3 | 4.7.2.6.4 |
| 4.7.2.7.1 | 4.7.2.7.2 | 4.7.2.7.3 | 4.7.2.7.4 | 4.7.2.8.1 | 4.7.2.8.2 | 4.7.2.8.3 | 4.7.2.8.4 |
| 4.7.3.1.1 | 4.7.3.1.2 | 4.7.3.1.3 | 4.7.3.1.4 | 4.7.3.2.1 | 4.7.3.2.2 | 4.7.3.2.3 | 4.7.3.2.4 |
| 4.7.3.3.1 | 4.7.3.3.2 | 4.7.3.3.3 | 4.7.3.3.4 | 4.7.3.4.1 | 4.7.3.4.2 | 4.7.3.4.3 | 4.7.3.4.4 |
| 4.7.3.5.1 | 4.7.3.5.2 | 4.7.3.5.3 | 4.7.3.5.4 | 4.7.3.6.1 | 4.7.3.6.2 | 4.7.3.6.3 | 4.7.3.6.4 |
| 4.7.3.7.1 | 4.7.3.7.2 | 4.7.3.7.3 | 4.7.3.7.4 | 4.7.3.8.1 | 4.7.3.8.2 | 4.7.3.8.3 | 4.7.3.8.4 |
| 4.7.4.1.1 | 4.7.4.1.2 | 4.7.4.1.3 | 4.7.4.1.4 | 4.7.4.2.1 | 4.7.4.2.2 | 4.7.4.2.3 | 4.7.4.2.4 |
| 4.7.4.3.1 | 4.7.4.3.2 | 4.7.4.3.3 | 4.7.4.3.4 | 4.7.4.4.1 | 4.7.4.4.2 | 4.7.4.4.3 | 4.7.4.4.4 |
| 4.7.4.5.1 | 4.7.4.5.2 | 4.7.4.5.3 | 4.7.4.5.4 | 4.7.4.6.1 | 4.7.4.6.2 | 4.7.4.6.3 | 4.7.4.6.4 |
| 4.7.4.7.1 | 4.7.4.7.2 | 4.7.4.7.3 | 4.7.4.7.4 | 4.7.4.8.1 | 4.7.4.8.2 | 4.7.4.8.3 | 4.7.4.8.4 |
| 4.8.1.1.1 | 4.8.1.1.2 | 4.8.1.1.3 | 4.8.1.1.4 | 4.8.1.2.1 | 4.8.1.2.2 | 4.8.1.2.3 | 4.8.1.2.4 |
| 4.8.1.3.1 | 4.8.1.3.2 | 4.8.1.3.3 | 4.8.1.3.4 | 4.8.1.4.1 | 4.8.1.4.2 | 4.8.1.4.3 | 4.8.1.4.4 |
| 4.8.1.5.1 | 4.8.1.5.2 | 4.8.1.5.3 | 4.8.1.5.4 | 4.8.1.6.1 | 4.8.1.6.2 | 4.8.1.6.3 | 4.8.1.6.4 |
| 4.8.1.7.1 | 4.8.1.7.2 | 4.8.1.7.3 | 4.8.1.7.4 | 4.8.1.8.1 | 4.8.1.8.2 | 4.8.1.8.3 | 4.8.1.8.4 |
| 4.8.2.1.1 | 4.8.2.1.2 | 4.8.2.1.3 | 4.8.2.1.4 | 4.8.2.2.1 | 4.8.2.2.2 | 4.8.2.2.3 | 4.8.22.4 |
| 4.8.2.3.1 | 4.8.2.3.2 | 4.8.2.3.3 | 4.8.2.3.4 | 4.8.2.4.1 | 4.8.2.4.2 | 4.8.2.4.3 | 4.8.2.4.4 |
| 4.8.2.5.1 | 4.8.2.5.2 | 4.8.2.5.3 | 4.8.2.5.4 | 4.8.2.6.1 | 4.8.2.6.2 | 4.8.2.6.3 | 4.8.2.6.4 |
| 4.8.2.7.1 | 4.8.2.7.2 | 4.8.2.7.3 | 4.8.2.7.4 | 4.8.2.8.1 | 4.8.2.8.2 | 4.8.2.8.3 | 4.8.2.8.4 |
| 4.8.3.1.1 | 4.8.3.1.2 | 4.8.3.1.3 | 4.8.3.1.4 | 4.8.3.2.1 | 4.8.3.2.2 | 4.8.3.2.3 | 4.8.3.2.4 |
| 4.8.3.3.1 | 4.8.3.3.2 | 4.8.3.3.3 | 4.8.3.3.4 | 4.8.3.4.1 | 4.8.3.4.2 | 4.8.3.4.3 | 4.8.3.4.4 |
| 4.8.3.5.1 | 4.8.3.5.2 | 4.8.3.5.3 | 4.8.3.5.4 | 4.8.3.6.1 | 4.8.3.6.2 | 4.8.3.6.3 | 4.8.3.6.4 |
| 4.8.3.7.1 | 4.8.3.7.2 | 4.8.3.7.3 | 4.8.3.7.4 | 4.8.3.8.1 | 4.8.3.8.2 | 4.8.3.8.3 | 4.8.3.8.4 |
| 4.8.4.1.1 | 4.8.4.1.2 | 4.8.4.1.3 | 4.8.4.1.4 | 4.8.4.2.1 | 4.8.4.2.2 | 4.8.4.2.3 | 4.8.4.2.4 |
| 4.8.4.3.1 | 4.8.4.3.2. | 4.8.4.3.3 | 4.8.4.3.4 | 4.8.4.4.1 | 4.8.4.4.2 | 4.8.4.4.3 | 4.8.4.4.4 |
| 4.8.4.5.1 | 4.8.4.5.2 | 4.8.4.5.3 | 4.8.4.5.4 | 4.8.4.6.1 | 4.8.4.6.2 | 4.8.4.6.3 | 4.8.4.6.4 |
| 4.8.4.7.1 | 4.8.4.7.2 | 4.8.4.7.3 | 4.8.4.7.4 | 4.8.4.8.1 | 4.8.4.8.2 | 4.8.4.8.3 | 4.8.4.8.4 |
| 5.1.1.1.1 | 5.1.1.1.2 | 5.1.1.1.3 | 5.1.1.1.4 | 5.1.1.2.1 | 5.1.1.2.2 | 5.1.1.2.3 | 5.1.1.2.4 |
| 5.1.1.3.1 | 5.1.1.3.2 | 5.1.1.3.3 | 5.1.1.3.4 | 5.1.1.4.1 | 5.1.1.4.2 | 5.1.1.4.3 | 5.1.1.4.4 |
| 5.1.1.5.1 | 5.1.1.5.2 | 5.1.1.5.3 | 5.1.1.5.4 | 5.1.1.6.1 | 5.1.1.6.2 | 5.1.1.6.3 | 5.1.1.6.4 |
| 5.1.1.7.1 | 5.1.1.7.2 | 5.1.1.7.3 | 5.1.1.7.4 | 5.1.1.8.1 | 5.1.1.8.2 | 5.1.1.8.3 | 5.1.1.8.4 |
| 5.1.2.1.1 | 5.1.2.1.2 | 5.1.2.1.3 | 5.1.2.1.4 | 5.1.2.2.1 | 5.1.2.2.2 | 5.1.2.2.3 | 5.1.2.2.4 |
| 5.1.2.3.1 | 5.1.2.3.2 | 5.1.2.3.3 | 5.1.2.3.4 | 5.1.2.4.1 | 5.1.2.4.2 | 5.1.2.4.3 | 5.1.2.4.4 |
| 5.1.2.5.1 | 5.1.2.5.2 | 5.1.2.5.3 | 5.1.2.5.4 | 5.1.2.6.1 | 5.1.2.6.2 | 5.1.2.6.3 | 5.1.2.6.4 |
| 5.1.2.7.1 | 5.1.2.7.2 | 5.1.2.7.3 | 5.1.2.7.4 | 5.1.2.8.1 | 5.1.2.8.2 | 5.1.2.8.3 | 5.1.2.8.4 |
| 5.1.3.1.1 | 5.1.3.1.2 | 5.1.3.1.3 | 5.1.3.1.4 | 5.1.3.2.1 | 5.1.3.2.2 | 5.1.3.2.3 | 5.1.3.2.4 |
| 5.1.3.3.1 | 5.1.3.3.2 | 5.1.3.3.3 | 5.1.3.3.4 | 5.1.3.4.1 | 5.1.3.4.2 | 5.1.3.4.3 | 5.1.3.4.4 |
| 5.1.3.5.1 | 5.1.3.5.2 | 5.1.3.5.3 | 5.1.3.5.4 | 5.1.3.6.1 | 5.1.3.6.2 | 5.1.3.6.3 | 5.1.3.6.4 |
| 5.1.3.7.1 | 5.1.3.7.2 | 5.1.3.7.3 | 5.1.3.7.4 | 5.1.3.8.1 | 5.1.3.8.2 | 5.1.3.8.3 | 5.1.3.8.4 |
| 5.1.4.1.1 | 5.1.4.1.2 | 5.1.4.1.3 | 5.1.4.1.4 | 5.1.4.2.1 | 5.1.4.2.2 | 5.1.4.2.3 | 5.1.4.2.4 |
| 5.1.4.3.1 | 5.1.4.3.2 | 5.1.4.3.3 | 5.1.4.3.4 | 5.1.4.4.1 | 5.1.4.4.2 | 5.1.4.4.3 | 5.1.4.4.4 |
| 5.1.4.5.1 | 5.1.4.5.2 | 5.1.4.5.3 | 5.1.4.5.4 | 5.1.4.6.1 | 5.1.4.6.2 | 5.1.4.6.3 | 5.1.4.6.4 |
| 5.1.4.7.1 | 5.1.4.7.2 | 5.1.4.7.3 | 5.1.4.7.4 | 5.1.4.8.1 | 5.1.4.8.2 | 5.1.4.8.3 | 5.1.4.8.4 |
| 5.2.1.1.1 | 5.2.1.1.2 | 5.2.1.1.3 | 5.2.1.1.4 | 5.2.1.2.1 | 5.2.1.2.2 | 5.2.1.2.3 | 5.2.1.2.4 |
| 5.2.1.3.1 | 5.2.1.3.2 | 5.2.1.3.3 | 5.2.1.3.4 | 5.2.1.4.1 | 5.2.1.4.2 | 5.2.1.4.3 | 5.2.1.4.4 |
| 5.2.1.5.1 | 5.2.1.5.2 | 5.2.1.5.3 | 5.2.1.5.4 | 5.2.1.6.1 | 5.2.1.6.2 | 5.2.1.6.3 | 5.2.1.6.4 |
| 5.2.1.7.1 | 5.2.1.7.2 | 5.2.1.7.3 | 5.2.1.7.4 | 5.2.1.8.1 | 5.2.1.8.2 | 5.2.1.8.3 | 5.2.1.8.4 |
| 5.2.2.1.1 | 5.2.2.1.2 | 5.2.2.1.3 | 5.2.2.1.4 | 5.2.2.2.1 | 5.2.2.2.2 | 5.2.2.2.3 | 5.2.2.2.4 |
| 5.2.2.3.1 | 5.2.2.3.2 | 5.2.2.3.3 | 5.2.2.3.4 | 5.2:2.4.1 | 5.2.2.4.2 | 5.2.2.4.3 | 5.2.2.4.4 |
| 5.2.2.5.1 | 5.2.2.5.2 | 5.2.2.5.3 | 5.2.2.5.4 | 5.2.2.6.1 | 5.2.2.6.2 | 5.2.2.6.3 | 5.2.2.6.4 |
| 5.2.2.7.1 | 5.2.2.7.2 | 5.2.2.7.3 | 5.2.2.7.4 | 5.2.2.8.1 | 5.2.2.8.2 | 5.2.2.8.3 | 5.2.2.8.4 |
| 5.2.3.1.1 | 5.2.3.1.2 | 5.2.3.1.3 | 5.2.3.1.4 | 5.2.3.2.1 | 5.2.3.2.2 | 5.2.3.2.3 | 5.2.3.2.4 |
| 5.2.3.3.1 | 5.2.3.3.2 | 5.2.3.3.3 | 5.2.3.3.4 | 5.2.3.4.1 | 5.2.3.4.2 | 5.2.3.4.3 | 5.2.3.4.4 |
| 5.2.3.5.1 | 5.2.3.5.2 | 5.2.3.5.3 | 5.2.3.5.4 | 5.2.3.6.1 | 5.2.3.6.2 | 5.2.3.6.3 | 5.2.3.6.4 |
| 5.2.3.7.1 | 5.2.3.7.2 | 5.2.3.7.3 | 5.2.3.7.4 | 5.2.3.8.1 | 5.2.3.8.2 | 5.2.3.8.3 | 5.2.3.8.4 |
| 5.2.4.1.1 | 5.2.4.1.2 | 5.2.4.1.3 | 5.2.4.1.4 | 5.2.4.2.1 | 5.2.4.2.2 | 5.2.4.2.3 | 5.2.4.2.4 |
| 5.2.4.3.1 | 5.2.4.3.2 | 5.2.4.3.3 | 5.2.4.3.4 | 5.2.4.4.1 | 5.2.4.4.2 | 5.2.4.4.3 | 5.2.4.4.4 |
| 5.2.4.5.1 | 5.2.4.5.2 | 5.2.4.5.3 | 5.2.4.5.4 | 5.2.4.6.1 | 5.2.4.6.2 | 5.2.4.6.3 | 5.2.4.6.4 |
| 5.2.4.7.1 | 5.2.4.7.2 | 5.2.4.7.3 | 5.2.4.7.4 | 5.2.4.8.1 | 5.2.4.8.2 | 5.2.4.8.3 | 5.2.4.8.4 |
| 5.3.1.1.1 | 5.3.1.1.2 | 5.3.1.1.3 | 5.3.1.1.4 | 5.3.1.2.1 | 5.3.1.2.2 | 5.3.1.2.3 | 5.3.1.2.4 |
| 5.3.1.3.1 | 5.3.1.3.2 | 5.3.1.3.3 | 5.3.1.3.4 | 5.3.1.4.1 | 5.3.1.4.2 | 5.3.1.4.3 | 5.3.1.4.4 |
| 5.3.1.5.1 | 5.3.1.5.2 | 5.3.1.5.3 | 5.3.1.5.4 | 5.3.1.6.1 | 5.3.1.6.2 | 5.3.1.6.3 | 5.3.1.6.4 |
| 5.3.1.7.1 | 5.3.1.7.2 | 5.3.1.7.3 | 5.3.1.7.4 | 5.3.1.8.1 | 5.3.1.8.2 | 5.3.1.8.3 | 5.3.1.8.4 |
| 5.3.2.1.1 | 5.3.2.1.2 | 5.3.2.1.3 | 5.3.2.1.4 | 5.3.2.2.1 | 5.3.2.2.2 | 5.3.2.2.3 | 5.3.2.2.4 |
| 5.3.2.3.1 | 5.3.2.3.2 | 5.3.2.3.3 | 5.3.2.3.4 | 5.3.2.4.1 | 5.3.2.4.2 | 5.3.2.4.3 | 5.3.2.4.4 |
| 5.3.2.5.1 | 5.3.2.5 2 | 5.3.2.5.3 | 5.3.2.5.4 | 5.3.2.6.1 | 5.3.2.6.2 | 5.3.2.6.3 | 5.3.2.6.4 |
| 5.3.2.7.1 | 5.3.2.7.2 | 5.3.2.7.3 | 5.3.2.7.4 | 5.3.2.8.1 | 5.3.2.8.2 | 5.3.2.8.3 | 5.3.2.8.4 |
| 5.3.3.1.1 | 5.3.3.1.2 | 5.3.3.1.3 | 5.3.3.1.4. | 5.3.3.2.1 | 5.3.3.2.2 | 5.3.3.2.3 | 5.3.3.2.4 |
| 5.3.3.3.1 | 5.3.3.3:2 | 5.3.3.3.3 | 5.3.3.3.4 | 5.3.3.4.1 | 5.3.3.4.2 | 5.3.3.4.3 | 5.3.3.4.4 |
| 5.3.3.5.1 | 5.3.3.5.2 | 5.3.3.5.3 | 5.3.3.5.4 | 5.3.3.6.1 | 5.3.3.6.2 | 5.3.3.6.3 | 5.3.3.6.4 |
| 5.3.3.7.1 | 5.3.3.7.2 | 5.3.3.7.3 | 5.3.3.7.4 | 5.3.3.8.1 | 5.3.3.8.2 | 5.3.3.8.3 | 5.3.3.8.4 |
| 5.3.4.1.1 | 5.3.4.1.2 | 5.3.4.1.3 | 5.3.4.1.4 | 5.3.4.2.1 | 5.3.4.2.2 | 5.3.4.2.3 | 5.3.4.2.4 |
| 5.3.4.3.1 | 5.3.4.3.2 | 5.3.4.3.3 | 5.3.4.3.4 | 5.3.4.4.1 | 5.3.4.4.2 | 5.3.4.4.3 | 5.3.4.4.4 |
| 5.3.4.5.1 | 5.3.4.5.2 | 5.3.4.5.3 | 5.3.4.5.4 | 5.3.4.6.1 | 5.3.4.6.2 | 5.3.4.6.3 | 5.3.4.6.4 |
| 5.3.4.7.1 | 5.3.4.7.2 | 5.3.4.7.3 | 5.3.4.7.4 | 5.3.4.8.1 | 5.3.4.8.2 | 5.3.4.8.3 | 5.3.4.8.4 |
| 5.4.1.1.1 | 5.4.1.1.2 | 5.4.1.1.3 | 5.4.1.1.4 | 5.4.1.2.1 | 5.4.1.2.2 | 5.4.1.2.3 | 5.4.1.2.4 |
| 5.4.1.3.1 | 5.4.1.3.2 | 5.4.1.3.3 | 5.4.1.3.4 | 5.4.1.4.1 | 5.4.1.4.2 | 5.4.1.4.3 | 5.4.1.4.4 |
| 5.4.1.5.1 | 5.4.1.5.2 | 5.4.1.5.3 | 5.4.1.5.4 | 5.4.1.6.1 | 5.4.1.6.2 | 5.4.1.6.3 | 5.4.1.6.4 |
| 5.4.1.7.1 | 5.4.1.7.2 | 5.4.1.7.3 | 5.4.1.7.4 | 5.4.1.8.1 | 5.4.1.8.2 | 5.4.1.8.3 | 5.4.1.8.4 |
| 5.4.2.1.1 | 5.4.2.1.2 | 5.4.2.1.3 | 5.4.2.1.4 | 5.4.2.2.1 | 5.4.2.2.2 | 5.4.2.2.3 | 5.4.2.2.4 |
| 5.4.2.3.1 | 5.4.2.3.2 | 5.4.2.3.3 | 5.4.2.3.4 | 5.4.2.4.1 | 5.4.2.4.2 | 5.4.2.4.3 | 5.4.2.4.4 |
| 5.4.2.5.1 | 5.4.2.5.2 | 5.4.2.5.3 | 5.4.2.5.4 | 5.4.2.6.1 | 5.4.2.6.2 | 5.4.2.6.3 | 5.4.2.6.4 |
| 5.4.2.7.1 | 5.4.2.7.2 | 5.4.2.7.3 | 5.4.2.7.4 | 5.4.2.8.1 | 5.4.2.8.2 | 5.4.2.8.3 | 5.4.2.8.4 |
| 5.4.3.1.1 | 5.4.3.1.2 | 5.4.3.1.3 | 5.4.3.1.4 | 5.4.3.2.1 | 5.4.3.2.2 | 5.4.3.2.3 | 5.4.3.2.4 |
| 5.4.3.3.1 | 5.4.3.3.2 | 5.4.3.3.3 | 5.4.3.3.4 | 5.4.3.4.1 | 5.4.3.4.2 | 5.4.3.4.3 | 5.4.3.4.4 |
| 5.4.3.5.1 | 5.4.3.5.2 | 5.4.3.5.3 | 5.4.3.5.4 | 5.4.3.6.1 | 5.4.3.6.2 | 5.4.3.6.3 | 5.4.3.6.4 |
| 5.4.3.7.1 | 5.4.3.7.2 | 5.4.3.7.3 | 5.4.3.7.4 | 5.4.3.3.1 | 5.4.3.8.2 | 5.4.3.8.3 | 5.4.3.8.4 |
| 5.4.4.1.1 | 5.4.4.1.2 | 5.4.4.1.3 | 5.4.4.1.4 | 5.4.4.2.1 | 5.4.4.2.2 | 5.4.4.2.3 | 5.4.4.2.4 |
| 5.4.4.3.1 | 5.4.4.3.2 | 5.4.4.3.3 | 5.4.4.3.4 | 5.4.4.4.1 | 5.4.4.4.2 | 5.4.4.4.3 | 5.4.4.4.4 |
| 5.4.4.5.1 | 5.4.4.5.2 | 5.4.4.5.3 | 5.4.4.5.4 | 5.4.4.6.1 | 5.4.4.6.2 | 5.4.4.6.3 | 5.4.4.6.4 |
| 5.4.4.7.1 | 5.4.4.7.2 | 5.4.4.7.3 | 5.4.4.7.4 | 5.4.4.8.1 | 5.4.4.8.2 | 5.4.4.8.3 | 5.4.4.8.4 |
| 5.5.1.1.1 | 5.5.1.1.2 | 5.5.1.1.3 | 5.5.1.1.4 | 5.5.1.2.1 | 5.5.1.2.2 | 5.5.1.2.3 | 5.5.1.2.4 |
| 5.5.1.3.1 | 5.5.1.3.2 | 5.5.1.3.3 | 5.5.1.3.4 | 5.5.1.4.1 | 5.5.1.4.2 | 5.5.1.4.3 | 5.5.1.4.4 |
| 5.5.1.5.1 | 5.5.1.5.2 | 5.5.1.5.3 | 5.5.1.5.4 | 5.5.1.6.1 | 5.5.1.6.2 | 5.5.1.6.3 | 5.5.1.6.4 |
| 5.5.1.7.1 | 5.5.1.7.2 | 5.5.1.7.3 | 5.5.1.7.4 | 5.5.1.8.1 | 5.5.1.8.2 | 5.5.1.8.3 | 5.5.1.8.4 |
| 5.5.2.1.1. | 5.5.2.1.2 | 5.5.2.1.3 | 5.5.2.1.4 | 5.5.2.2.1 | 5.5.2.2.2 | 5.5.2.2.3 | 5.5.2.2.4 |
| 5.5.2.3.1 | 5.5.2.3.2 | 5.5.2.3.3 | 5.5.2.3.4 | 5.5.2.4.1 | 5.5.2.4.2 | 5.5.2.4.3 | 5.5.2.4.4 |
| 5.5.2.5.1. | 5.5.2.5.2 | 5.5.2.5.3 | 5.5.2.5.4 | 5.5.2.6.1 | 5.5.2.6.2 | 5.5.2.6.3 | 5.5.2.6.4 |
| 5.5.2.7.1 | 5.5.2.7.2 | 5.5.2.7.3 | 5.5.2.7.4 | 5.5.2.8.1 | 5.5.2.8.2 | 5.5.2.8.3 | 5.5.2.8.4 |
| 5.5.3.1.1 | 5.5.3.1.2 | 5.5.3.1.3 | 5.5.3.1.4 | 5.5.3.2.1 | 5.5.3.2.2 | 5.5.3.2.3 | 5.5.3.2.4 |
| 5.5.3.3.1 | 5.5.3.3.2 | 5.5.3.3.3 | 5.5.3.3.4 | 5.5.3.4.1 | 5.5.3.4.2 | 5.5.3.4.3 | 5.5.3.4.4 |
| 5.5.3.5.1 | 5.5.3.5.2 | 5.5.3.5.3 | 5.5.3.5.4 | 5.5.3.6.1 | 5.5.3.6.2 | 5.5.3.6.3 | 5.5.3.6.4 |
| 5.5.3.7.1 | 5.5.3.7.2 | 5.5.3.7.3 | 5.5.3.7.4 | 5.5.3.8.1 | 5.5.3.8.2 | 5.5.3.8.3 | 5.5.3.8.4 |
| 5.5.4.1.1 | 5.5.4.1.2 | 5.5.4.1.3 | 5.5.4.1.4 | 5.5.4.2.1 | 5.5.4.2.2 | 5.5.4.2.3 | 5.5.4.2.4 |
| 5.5.4.3.1 | 5.5.4.3.2 | 5.5.4.3.3 | 5.5.4.3.4 | 5.5.4.4.1 | 5.5.4.4.2 | 5.5.4.4.3 | 5.5.4.4.4 |
| 5.5.4.5.1 | 5.5.4.5.2 | 5.5.4.5.3 | 5.5.4.5.4 | 5.5.4.6.1 | 5.5.4.6.2 | 5.5.4.6.3 | 5.5.4.6.4 |
| 5.5.4.7.1 | 5.5.4.7.2 | 5.5.4.7.3 | 5.5.4.7.4 | 5.5.4.8.1 | 5.5.4.8.2 | 5.5.4.8.3 | 5.5.4.8.4 |
| 5.6.1.1.1 | 5.6.1.1.2 | 5.6.1.1.3 | 5.6.1.1.4 | 5.6.1.2.1 | 5.6.1.2.2 | 5.6.1.2.3 | 5.6.1.2.4 |
| 5.6.1.3.1 | 5.6.1.3.2 | 5.6.1.3.3 | 5.6.1.3.4 | 5.6.1.4.1 | 5.6.1.4.2 | 5.6.1.4.3 | 5.6.1.4.4 |
| 5.6.1.5.1 | 5.6.1.5.2 | 5.6.1.5.3 | 5.6.1.5.4 | 5.6.1.6.1 | 5.6.1.6.2 | 5.6.1.6.3 | 5.6.1.6.4 |
| 5.6.1.7.1 | 5.6.1.7.2 | 5.6.1.7.3 | 5.6.1.7.4 | 5.6.1.8.1 | 5.6.1.8.2 | 5.6.1.8.3 | 5.6.1.8.4 |
| 5.6.2.1.1 | 5.6.2.1.2 | 5.6.2.1.3 | 5.6.2.1.4 | 5.6.2.2.1 | 5.6.2.2.2 | 5.6.2.2.3 | 5.6.2.2.4 |
| 5.6.2.3.1 | 5.6.2.3.2 | 5.6.2.3.3 | 5.6.2.3.4 | 5.6.2.4.1 | 5.6.2.4.2 | 5.6.2.4.3 | 5.6.2.4.4 |
| 5.6.2.5.1 | 5.6.2.5.2 | 5.6.2.5.3 | 5.6.2.5.4 | 5.6.2.6.1 | 5.6.2.6.2 | 5.6.2.6.3 | 5.6.2.6.4 |
| 5.6.2.7.1 | 5.6.2.7.2 | 5.6.2.7.3 | 5.6.2.7.4 | 5.6.2.8.1 | 5.6.2.8.2 | 5.6.2.8.3 | 5.6.2.8.4 |
| 5.6.3.1.1 | 5.6.3.1.2 | 5.6.3.1.3 | 5.6.3.1.4 | 5.6.3.2.1 | 5.6.3.2.2 | 5.6.3.2.3 | 5.6.3.2.4 |
| 5.6.3.3.1 | 5.6.3.3.2 | 5.6.3.3.3 | 5.6.3.3.4 | 5.6.3.4.1 | 5.6.3.4.2 | 5.6.3.4.3 | 5.6.3.4.4 |
| 5.6.3.5.1 | 5.6.3.5.2 | 5.6.3.5.3 | 5.6.3.5.4 | 5.6.3.6.1 | 5.6.3.6.2 | 5.6.3.6.3 | 5.6.3.6.4 |
| 5.6.3.7.1 | 5.6.3.7.2 | 5.6.3.7.3 | 5.6.3.7.4 | 5.6.3.8.1 | 5.6.3.8.2 | 5.6.3.8.3 | 5.6.3.8.4 |
| 5.6.4.1.1 | 5.6.4.1.2 | 5.6.4.1.3 | 5.6.4.1.4 | 5.6.4.2.1 | 5.6.4.2.2 | 5.6.4.2.3 | 5.6.4.2.4 |
| 5.6.4.3.1 | 5.6.4.3.2 | 5.6.4.3.3 | 5.6.4.3.4 | 5.6.4.4.1 | 5.6.4.4.2 | 5.6.4.4.3 | 5.6.4.4.4 |
| 5.6.4.5.1 | 5.6.4.5.2 | 5.6.4.5.3 | 5.6.4.5.4 | 5.6.4.6.1 | 5.6.4.6.2 | 5.6.4.6.3 | 5.6.4.6.4 |
| 5.6.4.7.1 | 5.6.4.7.2 | 5.6.4.7.3 | 5.6.4.7.4 | 5.6.4.8.1 | 5.6.4.8.2 | 5.6.4.8.3 | 5.6.4.8.4 |
| 5.7.1.1.1 | 5.7.1.1.2 | 5.7.1.1.3 | 5.7.1.1.4 | 5.7.1.2.1 | 5.7.1.2.2 | 5.7.1.2.3 | 5.7.1.2.4 |
| 5.7.1.3.1 | 5.7.1.3.2 | 5.7.1.3.3 | 5.7.1.3.4 | 5.7.1.4.1 | 5.7.1.4.2 | 5.7.1.4.3 | 5.7.1.4.4 |
| 5.7.1.5.1 | 5.7.1.5.2 | 5.7.1.5.3 | 5.7.1.5.4 | 5.7.1.6.1 | 5.7.1.6.2 | 5.7.1.6.3 | 5.7.1.6.4 |
| 5.7.1.7.1 | 5.7.1.7.2 | 5.7.1.7.3 | 5.7.1.7.4 | 5.7.1.8.1 | 5.7.1.8.2 | 5.7.1.8.3 | 5.7.1.8.4 |
| 5.7.2.1.1 | 5.7.2.1.2 | 5.7.2.1.3 | 5.7.2.1.4 | 5.7.2.2.1 | 5.7.2.2.2 | 5.7.2.2.3 | 5.7.2.2.4 |
| 5.7.2.3.1 | 5.7.2.3.2 | 5.7.2.3.3 | 5.7.2.3.4 | 5.7.2.4.1 | 5.7.2.4.2 | 5.7.2.4.3 | 5.7.2.4.4 |
| 5.7.2.5.1 | 5.7.2.5.2 | 5.7.2.5.3 | 5.7.2.5.4 | 5.7.2.6.1 | 5.7.2.6.2 | 5.7.2.6.3 | 5.7.2.6.4 |
| 5.7.2.7.1 | 5.7.2.7.2 | 5.7.2.7.3 | 5.7.2.7.4 | 5.7.2.8.1 | 5.7.2.8.2 | 5.7.2.8.3 | 5.7.2.8.4 |
| 5.7.3.1.1 | 5.7.3.1.2 | 5.7.3.1.3 | 5.7.3.1.4 | 5.7.3.2.1 | 5.7.3.2.2 | 5.7.3.2.3 | 5.7.3.2.4 |
| 5.7.3.3.1 | 5.7.3.3.2 | 5.7.3.3.3 | 5.7.3.3.4 | 5.7.3.4.1 | 5.7.3.4.2 | 5.7.3.4.3 | 5.7.3.4.4 |
| 5.7.3.5.1 | 5.7.3.5.2 | 5.7.3.5.3 | 5.7.3.5.4 | 5.7.3.6.1 | 5.7.3.6.2 | 5.7.3.6.3 | 5.7.3.6.4 |
| 5.7.3.7.1 | 5.7.3.7.2 | 5.7.3.7.3 | 5.7.3.7.4 | 5.7.3.8.1 | 5.7.3.8.2 | 5.7.3.8.3 | 5.7.3.8.4 |
| 5.7.4.1.1 | 5.7.4.1.2 | 5.7.4.1.3 | 5.7.4.1.4 | 5.7.4.2.1 | 5.7.4.2.2 | 5.7.4.2.3 | 5.7.4.2.4 |
| 5.7.4.3.1 | 5.7.4.3.2 | 5.7.4.3.3 | 5.7.4.3.4 | 5.7.4.4.1 | 5.7.4.4.2 | 5.7.4.4.3 | 5.7.4.4.4 |
| 5.7.4.5.1 | 5.7.4.5.2 | 5.7.4.5.3 | 5.7.4.5.4 | 5.7.4.6.1 | 5.7.4.6.2 | 5.7.4.6.3 | 5.7.4.6.4 |
| 5.7.4.7.1 | 5.7.4.7.2 | 5.7.4.7.3 | 5.7.4.7.4 | 5.7.4.8.1 | 5.7.4.8.2 | 5.7.4.8.3 | 5.7.4.8.4 |
| 5.8.1.1.1 | 5.8.1.1.2 | 5.8.1.1.3 | 5.8.1.1.4 | 5.8.1.2.1 | 5.8.1.2.2 | 5.8.1.2.3 | 5.8.1.2.4 |
| 5.8.1.3.1 | 5.8.1.3.2 | 5.8.1.3.3 | 5.8.1.3.4 | 5.8.1.4.1 | 5.8.1.4.2 | 5.8.1.4.3 | 5.8.1.4.4 |
| 5.8.1.5.1 | 5.8.1.5.2 | 5.8.1.5.3 | 5.8.1.5.4 | 5.8.1.6.1 | 5.8.1.6.2 | 5.8.1.6.3 | 5.8.1.6.4 |
| 5.8.1.7.1 | 5.8.1.7.2 | 5.8.1.7.3 | 5.8.1.7.4 | 5.8.1.8.1 | 5.8.1.8.2 | 5.8.1.8.3 | 5.8.1.8.4 |
| 5.8.2.1.1 | 5.8.2.1.2 | 5.8.2.1.3 | 5.8.2.1.4 | 5.8.2.2.1 | 5.8.2.2.2 | 5.8.2.2.3 | 5.8.2.2.4 |
| 5.8.2.3.1 | 5.8.2.3.2 | 5.8.2.3.3 | 5.8.2.3.4 | 5.8.2.4.1 | 5.8.2.4.2 | 5.8.2.4.3 | 5.8.2.4.4 |
| 5.8.2.5.1 | 5.8.2.5.2 | 5.8.2.5.3 | 5.8.2.5.4 | 5.8.2.6.1 | 5.8.2.6.2 | 5.8.2.6.3 | 5.8.2.6.4 |
| 5.8.2.7.1 | 5.8.2.7.2 | 5.8.2.7.3 | 5.8.2.7.4 | 5.8.2.8.1 | 5.8.2.8.2 | 5.8.2.8.3 | 5.8.2.8.4 |
| 5.8.3.1.1 | 5.8.3.1.2 | 5.8.3.1.3 | 5.8.3.1.4 | 5.8.3.2.1 | 5.8.3.2.2 | 5.8.3.2.3 | 5.8.3.2.4 |
| 5.8.3.3.1 | 5.8.3.3.2 | 5.8.3.3.3 | 5.8.3.3.4 | 5.8.3.4.1 | 5.8.3.4.2 | 5.8.3.4.3 | 5.8.3.4.4 |
| 5.8.3.5.1 | 5.8.3.5.2 | 5.8.3.5.3 | 5.8.3.5.4 | 5.8.3.6.1 | 5.8.3.6.2 | 5.8.3.6.3 | 5.8.3.6.4 |
| 5.8.3.7.1 | 5.8.3.7.2 | 5.8.3.7.3 | 5.8.3.7.4 | 5.8.3.8.1 | 5.8.3.8.2 | 5.8.3.8.3 | 5.8.3.8.4 |
| 5.8.4.1.1 | 5.8.4.1.2 | 5.8.4.1.3 | 5.8.4.1.4 | 5.8.4.2.1 | 5.8.4.2.2 | 5.8.4.2.3 | 5.8.4.2.4 |
| 5.8.4.3.1 | 5.8.4.3.2 | 5.8.4.3.3 | 5.8.4.3.4 | 5.8.4.4.1 | 5.8.4.4.2 | 5.8.4.4.3 | 5.8.4.4.4 |
| 5.8.4.5.1 | 5.8.4.5.2 | 5.8.4.5.3 | 5.8.4.5.4 | 5.8.4.6.1 | 5.8.4.6.2 | 5.8.4.6.3 | 5.8.4.6.4 |
| 5.8.4.7.1 | 5.8.4.7.2 | 5.8.4.7.3 | 5.8.4.7.4 | 5.8.4.8.1 | 5.8.4.8.2 | 5.8.4.8.3 | 5.8.4.8.4 |
| 6.1.1.1.1 | 6.1.1.1.2 | 6.1.1.1.3 | 6.1.1.1.4 | 6.1.1.2.1 | 6.1.1.2.2 | 6.1.1.2.3 | 6.1.1.2.4 |
| 6.1.1.3.1 | 6.1.1.3.2 | 6.1.1.3.3 | 6.1.1.3.4 | 6.1.1.4.1 | 6.1.1.4.2 | 6.1.1.4.3 | 6.1.1.4.4 |
| 6.1.1.5.1 | 6.1.1.5.2 | 6.1.1.5.3 | 6.1.1.5.4 | 6.1.1.6.1 | 6.1.1.6.2 | 6.1.1.6.3 | 6.1.1.6.4 |
| 6.1.1.7.1 | 6.1.1.7.2 | 6.1.1.7.3 | 6.1.1.7.4 | 6.1.1.8.1 | 6.1.1.8.2 | 6.1.1.8.3 | 6.1.1.8.4 |
| 6.1.2.1.1 | 6.1.2.1.2 | 6.1.2.1.3 | 6.1.2.1.4 | 6.1.2.2.1 | 6.1.2.2.2 | 6.1.2.2.3 | 6.1.2.2.4 |
| 6.1.2.3.1 | 6.1.2.3.2 | 6.1.2.3.3 | 6.1.2.3.4 | 6.1.2.4.1 | 6.1.2.4.2 | 6.1.2.4.3 | 6.1.2.4.4 |
| 6.1.2.5.1 | 6.1.2.5.2 | 6.1.2.5.3 | 6.1.2.5.4 | 6.1.2.6.1 | 6.1.2.6.2 | 6.1.2.6.3 | 6.1.2.6.4 |
| 6.1.2.7.1 | 6.1.2.7.2 | 6.1.2.7.3 | 6.1.2.7.4 | 6.1.2.8.1 | 6.1.2.8.2 | 6.1.2.8.3 | 6.1.2.8.4 |
| 6.1.3.1.1 | 6.1.3.1.2 | 6.1.3.1.3 | 6.1.3.1.4 | 6.1.3.2.1 | 6.1.3.2.2 | 6.1.3.2.3 | 6.1.3.2.4 |
| 6.1.3.3.1 | 6.1.3.3.2 | 6.1.3.3.3 | 6.1.3.3.4 | 6.1.3.4.1 | 6.1.3.4.2 | 6.1.3.4.3 | 6.1.3.4.4 |
| 6.1.3.5.1 | 6.1.3.5.2 | 6.1.3.5.3 | 6.1.3.5.4 | 6.1.3.6.1 | 6.1.3.6.2 | 6.1.3.6.3 | 6.1.3.6.4 |
| 6.1.3.7.1 | 6.1.3.7.2 | 6.1.3.7.3 | 6.1.3.7.4 | 6.1.3.8.1 | 6.1.3.8.2 | 6.1.3.8.3 | 6.1.3.8.4 |
| 6.1.4.1.1 | 6.1.4.1.2 | 6.1.4.1.3 | 6.1.4.1.4 | 6.1.4.2.1 | 6.1.4.2.2 | 6.1.4.2.3 | 6.1.4.2.4 |
| 6.1.4.3.1 | 6.1.4.3.2 | 6.1.4.3.3 | 6.1.4.3.4 | 6.1.4.4.1 | 6.1.4.4.2 | 6.1.4.4.3 | 6.1.4.4.4 |
| 6.1.4.5.1 | 6.1.4.5.2 | 6.1.4.5.3 | 6.1.4.5.4 | 6.1.4.6.1 | 6.1.4.6.2 | 6.1.4.6.3 | 6.1.4.6.4 |
| 6.1.4.7.1 | 6.1.4.7.2 | 6.1.4.7.3 | 6.1.4.7.4 | 6.1.4.8.1 | 6.1.4.8.2 | 6.1.4.8.3 | 6.1.4.8.4 |
| 6.2.1.1.1 | 6.2.1.1.2 | 6.2.1.1.3 | 6.2.1.1.4 | 6.2.1.2.1 | 6.2.1.2.2 | 6.2.1.2.3 | 6.2.1.2.4 |
| 6.2.1.3.1 | 6.2.1.3.2 | 6.2.1.3.3 | 6.2.1.3.4 | 6.2.1.4.1 | 6.2.1.4.2 | 6.2.1.4.3 | 6.2.1.4.4 |
| 6.2.1.5.1 | 6.2.1.5.2 | 6.2.1.5.3 | 6.2.1.5.4 | 6.2.1.6.1 | 6.2.1.6.2 | 6.2.1.6.3 | 6.2.1.6.4 |
| 6.2.1.7.1 | 6.2.1.7.2 | 6.2.1.7.3 | 6.2.1.7.4 | 6.2.1.8.1 | 6.2.1.8.2 | 6.2.1.8.3 | 6.2.1.8.4 |
| 6.2.2.1.1 | 6.2.2.1.2 | 6.2.2.1.3 | 6.2.2.1.4 | 6.2.2.2.1 | 6.2.2.2.2 | 6.2.2.2.3 | 6.2.2.2.4 |
| 6.2.2.3.1 | 6.2.2.3.2 | 6.2.2.3.3 | 6.2.2.3.4 | 6.2.2.4.1 | 6.2.2.4.2 | 6.2.2.4.3 | 6.2.2.4.4 |
| 6.2.2.5.1 | 6.2.2.5.2 | 6.2.2.5.3 | 6.2.2.5.4 | 6.2.2.6.1 | 6.2.2.6.2 | 6.2.2.6.3 | 6.2.2.6.4 |
| 6.2.2.7.1 | 6.2.2.7.2 | 6.2.2.7.3 | 6.2.2.7.4 | 6.2.2.8.1 | 6.2.2.8.2 | 6.2.2.8.3 | 6.2.2.8.4 |
| 6.2.3.1.1 | 6.2.3.1.2 | 6.2.3.1.3 | 6.2.3.1.4 | 6.2.3.2.1 | 6.2.3.2.2 | 6.2.3.2.3 | 6.2.3.2.4 |
| 6.2.3.3.1 | 6.2.3.3.2 | 6.2.3.3.3 | 6.2.3.3.4 | 6.2.3.4.1 | 6.2.3.4.2 | 6.2.3.4.3 | 6.2.3.4.4 |
| 6.2.3.5.1 | 6.2.3.5.2 | 6.2.3.5.3 | 6.2.3.5.4 | 6.2.3.6.1 | 6.2.3.6.2 | 6.2.3.6.3 | 6.2.3.6.4 |
| 6.2.3.7.1 | 6.2.3.7.2 | 6.2.3.7.3 | 6.2.3.7.4 | 6.2.3.8.1 | 6.2.3.8.2 | 6.2.3.8.3 | 6.2.3.8.4 |
| 6.2.4.1.1 | 6.2.4.1.2 | 6.2.4.1.3 | 6.2.4.1.4 | 6.2.4.2.1 | 6.2.4.2.2 | 6.2.4.2.3 | 6.2.4.2.4 |
| 6.2.4.3.1 | 6.2.4.3.2 | 6.2.4.3.3 | 6.2.4.3.4 | 6.2.4.4.1 | 6.2.4.4.2 | 6.2.4.4.3 | 6.2.4.4.4 |
| 6.2.4.5.1 | 6.2.4.5.2 | 6.2.4.5.3 | 6.2.4.5.4 | 6.2.4.6.1 | 6.2.4.6.2 | 6.2.4.6.3 | 6.2.4.6.4 |
| 6.2.4.7.1 | 6.2.4.7.2 | 6.2.4.7.3 | 6.2.4.7.4 | 6.2.4.8.1 | 6.2.4.8.2 | 6.2.4.8.3 | .6.2.4.8.4 |
| 6.3.1.1.1 | 6.3.1.1.2 | 6.3.1.1.3 | 6.3.1.1.4 | 6.3.1.2.1 | 6.3.1.2.2 | 6.3.1.2.3 | 6.3.1.2.4 |
| 6.3.1.3.1 | 6.3.1.3.2 | 6.3.1.3.3 | 6.3.1.3.4 | 6.3.1.4.1 | 6.3.1.4.2 | 6.3.1.4.3 | 6.3.1.4.4 |
| 6.3.1.5.1 | 6.3.1.5.2 | 6.3.1.5.3 | 6.3.1.5.4 | 6.3.1.6.1 | 6.3.1.6.2 | 6.3.1.6.3 | 6.3.1.6.4 |
| 6.3.1.7.1 | 6.3.1.7.2 | 6.3.1.7.3 | 6.3.1.7.4 | 6.3.1.8.1 | 6.3.1.8.2 | 6.3.1.8.3 | 6.3.1.8.4 |
| 6.3.2.1.1 | 6.3.2.1.2 | 6.3.2.1.3 | 6.3.2.1.4 | 6.3.2.2.1 | 6.3.2.2.2 | 6.3.2.2.3 | 6.3.2.2.4 |
| 6.3.2.3.1 | 6.3.2.3.2 | 6.3.2.3.3 | 6.3.2.3.4 | 6.3.2.4.1 | 6.3.2.4.2 | 6.3.2.4.3 | 6.3.2.4.4 |
| 6.3.2.5.1 | 6.3.2.5.2 | 6.3.2.5.3 | 6.3.2.5.4 | 6.3.2.6.1 | 6.3.2.6.2 | 6.3.2.6.3 | 6.3.2.6.4 |
| 6.3.2.7.1 | 6.3.2.7.2 | 6.3.2.7.3 | 6.3.2.7.4 | 6.3.2.8.1 | 6.3.2.8.2 | 6.3.2.8.3 | 6.3.2.8.4 |
| 6.3.3.1.1 | 6.3.3.1.2 | 6.3.3.1.3 | 6.3.3.1.4 | 6.3.3.2.1 | 6.3.3.2.2 | 6.3.3.2.3 | 6.3.3.2.4 |
| 6.3.3.3.1 | 6.3.3.3.2 | 6.3.3.3.3 | 6.3.3.3.4 | 6.3.3.4.1 | 6.3.3.4.2 | 6.3.3.4.3 | 6.3.3.4.4 |
| 6.3.3.5.1 | 6.3.3.5.2 | 6.3.3.5.3 | 6.3.3.5.4 | 6.3.3.6.1 | 6.3.3.6.2 | 6.3.3.6.3 | 6.3.3.6.4 |
| 6.3.3.7.1 | 6.3.3.7.2 | 6.3.3.7.3 | 6.3.3.7.4 | 6.3.3.8.1 | 6.3.3.8.2 | 6.3.3.8.3 | 6.3.3.8.4 |
| 6.3.4.1.1 | 6.3.4.1.2 | 6.3.4.1.3 | 6.3.4.1.4 | 6.3.4.2.1 | 6.3.4.2.2 | 6.3.4.2.3 | 6.3.4.2.4 |
| 6.3.4.3.1 | 6.3.4.3.2 | 6.3.4.3.3 | 6.3.4.3.4 | 6.3.4.4.1 | 6.3.4.4.2 | 6.3.4.4.3 | 6.3.4.4.4 |
| 6.3.4.5.1 | 6.3.4.5.2 | 6.3.4.5.3 | 6.3.4.5.4 | 6.3.4.6.1 | 6.3.4.6.2 | 6.3.4.6.3 | 6.3.4.6.4 |
| 6.3.4.7.1 | 6.3.4.7.2 | 6.3.4.7.3 | 6.3.4.7.4 | 6.3.4.8.1 | 6.3.4.8.2 | 6.3.4.8.3 | 6.3.4.8.4 |
| 6.4.1.1.1 | 6.4.1.1.2 | 6.4.1.1.3 | 6.4.1.1.4 | 6.4.1.2.1 | 6.4.1.2.2 | 6.4.1.2.3 | 6.4.1.2.4 |
| 6.4.1.3.1 | 6.4.1.3.2 | 6.4.1.3.3 | 6.4.1.3.4 | 6.4.1.4.1 | 6.4.1.4.2 | 6.4.1.4.3 | 6.4.1.4.4 |
| 6.4.1.5.1 | 6.4.1.5.2 | 6.4.1.5.3 | 6.4.1.5.4 | 6.4.1.6.1 | 6.4.1.6.2 | 6.4.1.6.3 | 6.4.1.6.4 |
| 6.4.1.7.1 | 6.4.1.7.2 | 6.4.1.7.3 | 6.4.1.7.4 | 6.4.1.8.1 | 6.4.1.8.2 | 6.4.1.8.3 | 6.4.1.8.4 |
| 6.4.2.1.1 | 6.4.2.1.2 | 6.4.2.1.3 | 6.4.2.1.4 | 6.4.2.2.1 | 6.4.2.2.2 | 6.4.2.2.3 | 6.4.2.2.4 |
| 6.4.2.3.1 | 6.4.2.3.2 | 6.4.2.3.3 | 6.4.2.3.4 | 6.4.2.4.1 | 6.4.2.4.2 | 6.4.2.4.3 | 6.4.2.4.4 |
| 6.4.2.5.1 | 6.4.2.5.2 | 6.4.2.5.3 | 6.4.2.5.4 | 6.4.2.6.1 | 6.4.2.6.2 | 6.4.2.6.3 | 6.4.2.6.4 |
| 6.4.2.7.1 | 6.4.2.7.2 | 6.4.2.7.3 | 6.4.2.7.4 | 6.4.2.8.1 | 6.4.2.8.2 | 6.4.2.8.3 | 6.4.2.8.4 |
| 6.4.3.1.1 | 6.4.3.1.2 | 6.4.3.1.3 | 6.4.3.1.4 | 6.4.3.2.1 | 6.4.3.2.2 | 6.4.3.2.3 | 6.4.3.2.4 |
| 6.4.3.3.1 | 6.4.3.3.2 | 6.4.3.3.3 | 6.4.3.3.4 | 6.4.3.4.1 | 6.4.3.4.2 | 6.4.3.4.3 | 6.4.3.4.4 |
| 6.4.3.5.1 | 6.4.3.5.2 | 6.4.3.5.3 | 6.4.3.5.4 | 6.4.3.6.1 | 6.4.3.6.2 | 6.4.3.6.3 | 6.4.3.6.4 |
| 6.4.3.7.1 | 6.4.3.7.2 | 6.4.3.7.3 | 6.4.3.7.4 | 6.4.3.8.1 | 6.4.3.8.2 | 6.4.3.8.3 | 6.4.3.8.4 |
| 6.4.4.1.1 | 6.4.4.1.2 | 6.4.4.1.3 | 6.4.4.1.4 | 6.4.4.2.1 | 6.4.4.2.2 | 6.4.4.2.3 | 6.4.4.2.4 |
| 6.4.4.3.1 | 6.4.4.3.2 | 6.4.4.3.3 | 6.4.4.3.4 | 6.4.4.4.1 | 6.4.4.4.2 | 6.4.4.4.3 | 6.4.4.4.4 |
| 6.4.4.5.1 | 6.4.4.5.2 | 6.4.4.5.3 | 6.4.4.5.4 | 6.4.4.6.1 | 6.4.4.6.2 | 6.4.4.6.3 | 6.4.4.6.4 |
| 6.4.4.7.1 | 6.4.4.7.2 | 6.4.4.7.3 | 6.4.4.7.4 | 6.4.4.8.1 | 6.4.4.8.2 | 6.4.4.8.3 | 6.4.4.8.4 |
| 6.5.1.1.1 | 6.5.1.1.2 | 6.5.1.1.3 | 6.5.1.1.4 | 6.5.1.2.1 | 6.5.1.2.2 | 6.5.1.2.3 | 6.5.1.2.4 |
| 6.5.1.3.1 | 6.5.1.3.2 | 6.5.1.3.3 | 6.5.1.3.4 | 6.5.1.4.1 | 6.5.1.4.2 | 6.5.1.4.3 | 6.5.1.4.4 |
| 6.5.1.5.1 | 6.5.1.5.2 | 6.5.1.5.3 | 6.5.1.5.4 | 6.5.1.6.1 | 6.5.1.6.2 | 6.5.1.6.3 | 6.5.1.6.4 |
| 6.5.1.7.1 | 6.5.1.7.2 | 6.5.1.7.3 | 6.5.1.7.4 | 6.5.1.8.1 | 6.5.1.8.2 | 6.5.1.8.3 | 6.5.1.8.4 |
| 6.5.2.1.1 | 6.5.2.1.2 | 6.5.2.1.3 | 6.5.2.1.4 | 6.5.2.2.1 | 6.5.2.2.2 | 6.5.2.2.3 | 6.5.2.2.4 |
| 6.5.2.3.1 | 6.5.2.3.2 | 6.5.2.3.3 | 6.5.2.3.4 | 6.5.2.4.1 | 6.5.2.4.2 | 6.5.2.4.3 | 6.5.2.4.4 |
| 6.5.2.5.1 | 6.5.2.5.2 | 6.5.2.5.3 | 6.5.2.5.4 | 6.5.2.6.1 | 6.5.2.6.2 | 6.5.2.6.3 | 6.5.2.6.4 |
| 6.5.2.7.1 | 6.5.2.7.2 | 6.5.2.7.3 | 6.5.2.7.4 | 6.5.2.8.1 | 6.5.2.8.2 | 6.5.2.8.3 | 6.5.2.8.4 |
| 6.5.3.1.1 | 6.5.3.1.2 | 6.5.3.1.3 | 6.5.3.1.4 | 6.5.3.2.1 | 6.5.3.2.2 | 6.5.3.2.3 | 6.5.3.2.4 |
| 6.5.3.3.1 | 6.5.3.3.2 | 6.5.3.3.3 | 6.5.3.3.4 | 6.5.3.4.1 | 6.5.3.4.2 | 6.5.3.4.3 | 6.5.3.4.4 |
| 6.5.3.5.1 | 6.5.3.5.2 | 6.5.3.5.3 | 6.5.3.5.4 | 6.5.3.6.1 | 6.5.3.6.2 | 6.5.3.6.3 | 6.5.3.6.4 |
| 6.5.3.7.1 | 6.5.3.7.2 | 6.5.3.7.3 | 6.5.3.7.4 | 6.5.3.8.1 | 6.5.3.8.2 | 6.5.3.8.3 | 6.5.3.8.4 |
| 6.5.4.1.1 | 6.5.4.1.2 | 6.5.4.1.3 | 6.5.4.1.4 | 6.5.4.2.1 | 6.5.4.2.2 | 6.5.4.2.3 | 6.5.4.2.4 |
| 6.5.4.3.1 | 6.5.4.3.2 | 6.5.4.3.3 | 6.5.4.3.4 | 6.5.4.4.1 | 6.5.4.4.2 | 6.5.4.4.3 | 6.5.4.4.4 |
| 6.5.4.5.1 | 6.5.4.5.2 | 6.5.4.5.3 | 6.5.4.5.4 | 6.5.4.6.1 | 6.5.4.6.2 | 6.5.4.6.3 | 6.5.4.6.4 |
| 6.5.4.7.1 | 6.5.4.7.2 | 6.5.4.7.3 | 6.5.4.7.4 | 6.5.4.8.1 | 6.5.4.8.2 | 6.5.4.8.3 | 6.5.4.8.4 |
| 6.6.1.1.1 | 6.6.1.1.2 | 6.6.1.1.3 | 6.6.1.1.4 | 6.6.1.2.1 | 6.6.1.2.2 | 6.6.1.2.3 | 6.6.1.2.4 |
| 6.6.1.3.1 | 6.6.1.3.2 | 6.6.1.3.3 | 6.6.1.3.4 | 6.6.1.4.1 | 6.6.1.4.2 | 6.6.1.4.3 | 6.6.1.4.4 |
| 6.6.1.5.1 | 6.6.1.5.2 | 6.6.1.5.3 | 6.6.1.5.4 | 6.6.1.6.1 | 6.6.1.6.2 | 6.6.1.6.3 | 6.6.1.6.4 |
| 6.6.1.7.1 | 6.6.1.7.2 | 6.6.1.7.3 | 6.6.1.7.4 | 6.6.1.8.1 | 6.6.1.8.2 | 6.6.1.8.3 | 6.6.1.8.4 |
| 6.6.2.1.1 | 6.6.2.1.2 | 6.6.2.1.3 | 6.6.2.1.4 | 6.6.2.2.1 | 6.6.2.2.2 | 6.6.2.2.3 | 6.6.2.2.4 |
| 6.6.2.3.1 | 6.6.2.3.2 | 6.6.2.3.3 | 6.6.2.3.4 | 6.6.2.4.1 | 6.6.2.4.2 | 6.6.2.4.3 | 6.6.2.4.4 |
| 6.6.2.5.1 | 6.6.2.5.2 | 6.6.2.5.3 | 6.6.2.5.4 | 6.6.2.6.1 | 6.6.2.6.2 | 6.6.2.6.3 | 6.6.2.6.4 |
| 6.6.2.7.1 | 6.6.2.7.2 | 6.6.2.7.3 | 6.6.2.7.4 | 6.6.2.8.1 | 6.6.2.8.2 | 6.6.2.8.3 | 6.6.2.8.4 |
| 6.6.3.1.1 | 6.6.3.1.2 | 6.6.3.1.3 | 6.6.3.1.4 | 6.6.3.2.1 | 6.6.3.2.2 | 6.6.3.2.3 | 6.6.3.2.4 |
| 6.6.3.3.1 | 6.6.3.3.2 | 6.6.3.3.3 | 6.6.3.3.4 | 6.6.3.4.1 | 6.6.3.4.2 | 6.6.3.4.3 | 6.6.3.4.4 |
| 6.6.3.5.1 | 6.6.3.5.2 | 6.6.3.5.3 | 6.6.3.5.4 | 6.6.3.6.1 | 6.6.3.6.2 | 6.6.3.6.3 | 6.6.3.6.4 |
| 6.6.3.7.1 | 6.6.3.7.2 | 6.6.3.7.3 | 6.6.3.7.4 | 6.6.3.8.1 | 6.6.3.8.2 | 6.6.3.8.3 | 6.6.3.8.4 |
| 6.6.4.1.1 | 6.6.4.1.2 | 6.6.4.1.3 | 6.6.4.1.4 | 6.6.4.2.1 | 6.6.4.2.2 | 6.6.4.2.3 | 6.6.4.2.4 |
| 6.6.4.3.1 | 6.6.4.3.2 | 6.6.4.3.3 | 6.6.4.3.4 | 6.6.4.4.1 | 6.6.4.4.2 | 6.6.4.4.3 | 6.6.4.4.4 |
| 6.6.4.5.1 | 6.6.4.5.2 | 6.6.4.5.3 | 6.6.4.5.4 | 6.6.4.6.1 | 6.6.4.6.2 | 6.6.4.6.3 | 6.6.4.6.4 |
| 6.6.4.7.1 | 6.6.4.7.2 | 6.6.4.7.3 | 6.6.4.7.4 | 6.6.4.8.1 | 6.6.4.8.2 | 6.6.4.8.3 | 6.6.4.8.4 |
| 6.7.1.1.1 | 6.7.1.1.2 | 6.7.1.1.3 | 6.7.1.1.4 | 6.7.1.2.1 | 6.7.1.2.2 | 6.7.1.2.3 | 6.7.1.2.4 |
| 6.7.1.3.1 | 6.7.1.3.2 | 6.7.1.3.3 | 6.7.1.3.4 | 6.7.1.4.1 | 6.7.1.4.2 | 6.7.1.4.3 | 6.7.1.4.4 |
| 6.7.1.5.1 | 6.7.1.5.2 | 6.7.1.5.3 | 6.7.1.5.4 | 6.7.1.6.1 | 6.7.1.6.2 | 6.7.1.6.3 | 6.7.1.6.4 |
| 6.7.1.7.1 | 6.7.1.7.2 | 6.7.1.7.3 | 6.7.1.7.4 | 6.7.1.8.1 | 6.7.1.8.2 | 6.7.1.8.3 | 6.7.1.8.4 |
| 6.7.2.1.1 | 6.7.2.1.2 | 6.7.2.1.3 | 6.7.2.1.4 | 6.7.2.2.1 | 6.7.2.2.2 | 6.7.2.2.3 | 6.7.2.2.4 |
| 6.7.2.3.1 | 6.7.2.3.2 | 6.7.2.3.3 | 6.7.2.3.4 | 6.7.2.4.1 | 6.7.2.4.2 | 6.7.2.4.3 | 6.7.2.4.4 |
| 6.7.2.5.1 | 6.7.2.5.2 | 6.7.2.5.3 | 6.7.2.5.4 | 6.7.2.6.1 | 6.7.2.6.2 | 6.7.2.6.3 | 6.7.2.6.4 |
| 6.7.2.7.1 | 6.7.2.7.2 | 6.7.2.7.3 | 6.7.2.7.4 | 6.7.2.8.1 | 6.7.2.8.2 | 6.7.2.8.3 | 6.7.2.8.4 |
| 6.7.3.1.1 | 6.7.3.1.2 | 6.7.3.1.3 | 6.7.3.1.4 | 6.7.3.2.1 | 6.7.3.2.2 | 6.7.3.2.3 | 6.7.3.2.4 |
| 6.7.3.3.1 | 6.7.3.3.2 | 6.7.3.3.3 | 6.7.3.3.4 | 6.7.3.4.1 | 6.7.3.4.2 | 6.7.3.4.3 | 6.7.3.4.4 |
| 6.7.3.5.1 | 6.7.3.5.2 | 6.7.3.5.3 | 6.7.3.5.4 | 6.7.3.6.1 | 6.7.3.6.2 | 6.7.3.6.3 | 6.7.3.6.4 |
| 6.7.3.7.1 | 6.7.3.7.2 | 6.7.3.7.3 | 6.7.3.7.4 | 6.7.3.8.1 | 6.7.3.8.2 | 6.7.3.8.3 | 6.7.3.8.4 |
| 6.7.4.1.1 | 6.7.4.1.2 | 6.7.4.1.3 | 6.7.4.1.4 | 6.7.4.2.1 | 6.7.4.2.2 | 6.7.4.2.3 | 6.7.4.2.4 |
| 6.7.4.3.1 | 6.7.4.3.2 | 6.7.4.3.3 | 6.7.4.3.4 | 6.7.4.4.1 | 6.7.4.4.2 | 6.7.4.4.3 | 6.7.4.4.4 |
| 6.7.4.5.1 | 6.7.4.5.2 | 6.7.4.5.3 | 6.7.4.5.4 | 6.7.4.6.1 | 6.7.4.6.2 | 6.7.4.6.3 | 6.7.4.6.4 |
| 6.7.4.7.1 | 6.7.4.7.2 | 6.7.4.7.3 | 6.7.4.7.4 | 6.7.4.8.1 | 6.7.4.8.2 | 6.7.4.8.3 | 6.7.4.8.4 |
| 6.8.1.1.1 | 6.8.1.1.2 | 6.8.1.1.3 | 6.8.1.1.4 | 6.8.1.2.1 | 6.8.1.2.2 | 6.8.1.2.3 | 6.8.1.2.4 |
| 6.8.1.3.1 | 6.8.1.3.2 | 6.8.1.3.3 | 6.8.1.3.4 | 6.8.1.4.1 | 6.8.1.4.2 | 6.8.1.4.3 | 6.8.1.4.4 |
| 6.8.1.5.1 | 6.8.1.5.2 | 6.8.1.5.3 | 6.8.1.5.4 | 6.8.1.6.1 | 6.8.1.6.2 | 6.8.1.6.3 | 6.8.1.6.4 |
| 6.8.1.7.1 | 6.8.1.7.2 | 6.8.1.7.3 | 6.8.1.7.4 | 6.8.1.8.1 | 6.8.1.8.2 | 6.8.1.8.3 | 6.8.1.8.4 |
| 6.8.2.1.1 | 6.8.2.1.2 | 6.8.2.1.3 | 6.8.2.1.4 | 6.8.2.2.1 | 6.8.2.2.2 | 6.8.2.2.3 | 6.8.2.2.4 |
| 6.8.2.3.1 | 6.8.2.3.2 | 6.8.2.3.3 | 6.8.2.3.4 | 6.8.2.4.1 | 6.8.2.4.2 | 6.8.2.4.3 | 6.8.2.4.4 |
| 6.8.2.5.1 | 6.8.2.5.2 | 6.8.2.5.3 | 6.8.2.5.4 | 6.8.2.6.1 | 6.8.2.6.2 | 6.8.2.6.3 | 6.8.2.6.4 |
| 6.8.2.7.1 | 6.8.2.7.2 | 6.8.2.7.3 | 6.8.2.7.4 | 6.8.2.8.1 | 6.8.2.8.2 | 6.8.2.8.3 | 6.8.2.8.4 |
| 6.8.3.1.1 | 6.8.3.1.2 | 6.8.3.1.3 | 6.8.3.1.4 | 6.8.3.2.1 | 6.8.3.2.2 | 6.8.3.2.3 | 6.8.3.2.4 |
| 6.8.3.3.1 | 6.8.3.3.2 | 6.8.3.3.3 | 6.8.3.3.4 | 6.8.3.4.1 | 6.8.3.4.2 | 6.8.3.4.3 | 6.8.3.4.4 |
| 6.8.3.5.1 | 6.8.3.5.2 | 6.8.3.5.3 | 6.8.3.5.4 | 6.8.3.6.1 | 6.8.3.6.2 | 6.8.3.6.3 | 6.8.3.6.4 |
| 6.8.3.7.1 | 6.8.3.7.2 | 6.8.3.7.3 | 6.8.3.7.4 | 6.8.3.8.1 | 6.8.3.8.2 | 6.8.3.8.3 | 6.8.3.8.4 |
| 6.8.4.1.1 | 6.8.4.1.2 | 6.8.4.1.3 | 6.8.4.1.4 | 6.8.4.2.1 | 6.8.4.2.2 | 6.8.4.2.3 | 6.8.4.2.4 |
| 6.8.4.3.1 | 6.8.4.3.2 | 6.8.4.3.3 | 6.8.4.3.4 | 6.8.4.4.1 | 6.8.4.4.2 | 6.8.4.4.3 | 6.8.4.4.4 |
| 6.8.4.5.1 | 6.8.4.5.2 | 6.8.4.5.3 | 6.8.4.5.4 | 6.8.4.6.1 | 6.8.4.6.2 | 6.8.4.6.3 | 6.8.4.6.4 |
| 6.8.4.7.1 | 6.8.4.7.2 | 6.8.4.7.3 | 6.8.4.7.4 | 6.8.4.8.1 | 6.8.4.8.2 | 6.8.4.8.3 | 6.8.4.8.4 |
| 7.1.1.1.1 | 7.1.1.1.2 | 7.1.1.1.3 | 7.1.1.1.4 | 7.1.1.2.1 | 7.1.1.2.2 | 7.1.1.2.3 | 7.1.1.2.4 |
| 7.1.1.3.1 | 7.1.1.3.2 | 7.1.1.3.3 | 7.1.1.3.4 | 7.1.1.4.1 | 7.1.1.4.2 | 7.1.1.4.3 | 7.1.1.4.4 |
| 7.1.1.5.1 | 7.1.1.5.2 | 7.1.1.5.3 | 7.1.1.5.4 | 7.1.1.6.1 | 7.1.1.6.2 | 7.1.1.6.3 | 7.1.1.6.4 |
| 7.1.1.7.1 | 7.1.1.7.2 | 7.1.1.7.3 | 7.1.1.7.4 | 7.1.1.8.1 | 7.1.1.8.2 | 7.1.1.8.3 | 7.1.1.8.4 |
| 7.1.2.1.1 | 7.1.2.1.2 | 7.1.2.1.3 | 7.1.2.1.4 | 7.1.2.2.1 | 7.1.2.2.2 | 7.1.2.2.3 | 7.1.2.2.4 |
| 7.1.2.3.1 | 7.1.2.3.2 | 7.1.2.3.3 | 7.1.2.3.4 | 7.1.2.4.1 | 7.1.2.4.2 | 7.1.2.4.3 | 7.1.2.4.4 |
| 7.1.2.5.1 | 7.1.2.5.2 | 7.1.2.5.3 | 7.1.2.5.4 | 7.1.2.6.1 | 7.1.2.6.2 | 7.1.2.6.3 | 7.1.2.6.4 |
| 7.1.2.7.1 | 7.1.2.7.2 | 7.1.2.7.3 | 7.1.2.7.4 | 7.1.2.8.1 | 7.1.2.8.2 | 7.1.2.8.3 | 7.1.2.8.4 |
| 7.1.3.1.1 | 7.1.3.1.2 | 7.1.3.1.3 | 7.1.3.1.4 | 7.1.3.2.1 | 7.1.3.2.2 | 7.1.3.2.3 | 7.1.3.2.4 |
| 7.1.3.3.1 | 7.1.3.3.2 | 7.1.3.3.3 | 7.1.3.3.4 | 7.1.3.4.1 | 7.1.3.4.2 | 7.1.3.4.3 | 7.1.3.4.4 |
| 7.1.3.5.1 | 7.1.3.5.2 | 7.1.3.5.3 | 7.1.3.5.4 | 7.1.3.6.1 | 7.1.3.6.2 | 7.1.3.6.3 | 7.1.3.6.4 |
| 7.1.3.7.1 | 7.1.3.7.2 | 7.1.3.7.3 | 7.1.3.7.4 | 7.1.3.8.1 | 7.1.3.8.2 | 7.1.3.8.3 | 7.1.3.8.4 |
| 7.1.4.1.1 | 7.1.4.1.2 | 7.1.4.1.3 | 7.1.4.1.4 | 7.1.4.2.1 | 7.1.4.2.2 | 7.1.4.2.3 | 7.1.4.2.4 |
| 7.1.4.3.1 | 7.1.4.3.2 | 7.1.4.3.3 | 7.1.4.3.4 | 7.1.4.4.1 | 7.1.4.4.2 | 7.1.4.4.3 | 7.1.4.4.4 |
| 7.1.4.5.1 | 7.1.4.5.2 | 7.1.4.5.3 | 7.1.4.5.4 | 7.1.4.6.1 | 7.1.4.6.2 | 7.1.4.6.3 | 7.1.4.6.4 |
| 7.1.4.7.1 | 7.1.4.7.2 | 7.1.4.7.3 | 7.1.4.7.4 | 7.1.4.8.1 | 7.1.4.8.2 | 7.1.4.8.3 | 7.1.4.8.4 |
| 7.2.1.1.1 | 7.2.1.1.2 | 7.2.1.1.3 | 7.2.1.1.4 | 7.2.1.2.1 | 7.2.1.2.2 | 7.2.1.2.3 | 7.2.1.2.4 |
| 7.2.1.3.1 | 7.2.1.3.2 | 7.2.1.3.3 | 7.2.1.3.4 | 7.2.1.4.1 | 7.2.1.4.2 | 7.2.1.4.3 | 7.2.1.4.4 |
| 7.2.1.5.1 | 7.2.1.5.2 | 7.2.1.5.3 | 7.2.1.5.4 | 7.2.1.6.1 | 7.2.1.6.2 | 7.2.1.6.3 | 7.2.1.6.4 |
| 7.2.1.7.1 | 7.2.1.7.2 | 7.2.1.7.3 | 7.2.1.7.4 | 7.2.1.8.1 | 7.2.1.8.2 | 7.2.1.8.3 | 7.2.1.8.4 |
| 7.2.2.1.1 | 7.2.2.1.2 | 7.2.2.1.3 | 7.2.2.1.4 | 7.2.2.2.1 | 7.2.2.2.2 | 7.2.2.2.3 | 7.2.2.2.4 |
| 7.2.2.3.1 | 7.2.2.3.2 | 7.2.2.3.3 | 7.2.2.3.4 | 7.2.2.4.1 | 7.2.2.4.2 | 7.2.2.4.3 | 7.2.2.4.4 |
| 7.2.2.5.1 | 7.2.2.5.2 | 7.2.2.5.3 | 7.2.2.5.4 | 7.2.2.6.1 | 7.2.2.6.2 | 7.2.2.6.3 | 7.2.2.6.4 |
| 7.2.2.7.1 | 7.2.2.7.2 | 7.2.2.7.3 | 7.2.2.7.4 | 7.2.2.8.1 | 7.2.2.8.2 | 7.2.2.8.3 | 7.2.2.8.4 |
| 7.2.3.1.1 | 7.2.3.1.2 | 7.2.3.1.3 | 7.2.3.1.4 | 7.2.3.2.1 | 7.2.3.2.2 | 7.2.3.2.3 | 7.2.3.2.4 |
| 7.2.3.3.1 | 7.2.3.3.2 | 7.2.3.3.3 | 7.2.3.3.4 | 7.2.3.4.1 | 7.2.3.4.2 | 7.2.3.4.3 | 7.2.3.4.4 |
| 7.2.3.5.1 | 7.2.3.5.2 | 7.2.3.5.3 | 7:2.3.5.4 | 7.2.3.6.1 | 7.2.3.6.2 | 7.2.3.6.3 | 7.2.3.6.4 |
| 7.2.3.7.1 | 7.2.3.7.2 | 7.2.3.7.3 | 7.2.3.7.4 | 7.2.3.8.1 | 7.2.3.8.2 | 7.2.3.8.3 | 7.2.3.8.4 |
| 7.2.4.1.1 | 7.2.4.1.2 | 7.2.4.1.3 | 7.2.4.1.4 | 7.2.4.2.1 | 7.2.4.2.2 | 7.2.4.2.3 | 7.2.4.2.4 |
| 7.2.4.3.1 | 7.2.4.3.2 | 7.2.4.3.3 | 7.2.4.3.4 | 7.2.4.4.1 | 7.2.4.4.2 | 7.2.4.4.3 | 7.2.4.4.4 |
| 7.2.4.5.1 | 7.2.4.5.2 | 7.2.4.5.3 | 7.2.4.5.4 | 7.2.4.6.1 | 7.2.4.6.2 | 7.2.4.6.3 | 7.2.4.6.4 |
| 7.2.4.7.1 | 7.2.4.7.2 | 7.2.4.7.3 | 7.2.4.7.4 | 7.2.4.8.1 | 7.2.4.8.2 | 7.2.4.8.3 | 7.2.4.8.4 |
| 7.3.1.1.1 | 7.3.1.1.2 | 7.3.1.1.3 | 7.3.1.1.4 | 7.3.1.2.1 | 7.3.1.2.2 | 7.3.1.2.3 | 7.3.1.2.4 |
| 7.3.1.3.1 | 7.3.1.3.2 | 7.3.1.3.3 | 7.3.1.3.4 | 7.3.1.4.1 | 7.3.1.4.2 | 7.3.1.4.3 | 7.3.1.4.4 |
| 7.3.1.5.1 | 7.3.1.5.2 | 7.3.1.5.3 | 7.3.1.5.4 | 7.3..1.6.1 | 7.3.1.6.2 | 7.3.1.6.3 | 7.3.1.6.4 |
| 7.3.1.7.1 | 7.3.1.7.2 | 7.3.1.7.3 | 7.3.1.7.4 | 7.3.1.8.1 | 7.3.1.8.2 | 7.3.1.8.3 | 7.3.1.8.4 |
| 7.3.2.1.1 | 7.3.2.1.2 | 7.3.2.1.3 | 7.3.2.1.4 | 7.3.2.2.1 | 7.3.2.2.2 | 7.3.2.2.3 | 7.3.2.2.4 |
| 7.3.2.3.1 | 7.3.2.3.2 | 7.3.2.3.3 | 7.3.2.3.4 | 7.3.2.4.1 | 7.3.2.4.2 | 7.3.2.4.3 | 7.3.2.4.4 |
| 7.3.2.5.1 | 7.3.2.5.2 | 7.3.2.5.3 | 7.3.2.5.4 | 7.3.2.6.1 | 7.3.2.6.2 | 7.3.2.6.3 | 7.3.2.6.4 |
| 7.3.2.7.1 | 7.3.2.7.2 | 7.3.2.7.3 | 7.3.2.7.4 | 7.3.2.8.1 | 7.3.2.8.2 | 7.3.2.8.3 | 7.3.2.8.4 |
| 7.3.3.1.1 | 7.3.3.1.2 | 7.3.3.1.3 | 7.3.3.1.4 | 7.3.3.2.1 | 7.3.3.2.2 | 7.3.3.2.3 | 7.3.3.2.4 |
| 7.3.3.3.1 | 7.3.3.3.2 | 7.3.3.3.3 | 7.3.3.3.4 | 7.3.3.4.1 | 7.3.3.4.2 | 7.3.3.4.3 | 7.3.3.4.4 |
| 7.3.3.5.1 | 7.3.3.5.2 | 7.3.3.5.3 | 7.3.3.5.4 | 7.3.3.6.1 | 7.3.3.6.2 | 7.3.3.6.3 | 7.3.3.6.4 |
| 7.3.3.7.1 | 7.3.3.7.2 | 7.3.3.7.3 | 7.3.3.7.4 | 7.3.3.8.1 | 7.3.3.8.2 | 7.3.3.8.3 | 7.3.3.8.4 |
| 7.3.4.1.1 | 7.3.4.1.2 | 7.3.4.1.3 | 7.3.4.1.4 | 7.3.4.2.1 | 7.3.4.2.2 | 7.3.4.2.3 | 7.3.4.2.4 |
| 7.3.4.3.1 | 7.3.4.3.2 | 7.3.4.3.3 | 7.3.4.3.4 | 7.3.4.4.1 | 7.3.4.4.2 | 7.3.4.4.3 | 7.3.4.4.4 |
| 7.3.4.5.1 | 7.3.4.5.2 | 7.3.4.5.3 | 7.3.4.5.4 | 7.3.4.6.1 | 7.3.4.6.2 | 7.3.4.6.3 | 7.3.4.6.4 |
| 7.3.4.7.1 | 7.3.4.7.2 | 7.3.4.7.3 | 7.3.4.7.4 | 7.3.4.8.1 | 7.3.4.8.2 | 7.3.4.8.3 | 7.3.4.8.4 |
| 7.4.1.1.1 | 7.4.1.1.2 | 7.4.1.1.3 | 7.4.1.1.4 | 7.4.1.2.1 | 7.4.1.2.2 | 7.4.1.2.3 | 7.4.1.2.4 |
| 7.4.1.3.1 | 7.4.1.3.2 | 7.4.1.3.3 | 7.4.1.3.4 | 7.4.1.4.1 | 7.4.1.4.2 | 7.4.1.4.3 | 7.4.1.4.4 |
| 7.4.1.5.1 | 7.4.1.5.2 | 7.4.1.5.3 | 7.4.1.5.4 | 7.4.1.6.1 | 7.4.1.6.2 | 7.4.1.6.3 | 7.4.1.6.4 |
| 7.4.1.7.1 | 7.4.1.7.2 | 7.4.1.7.3 | 7.4.1.7.4 | 7.4.1.8.1 | 7.4.1.8.2 | 7.4.1.8.3 | 7.4.1.8.4 |
| 7.4.2.1.1 | 7.4.2.1.2 | 7.4.2.1.3 | 7.4.2.1.4 | 7.4.2.2.1 | 7.4.2.2.2 | 7.4.2.2.3 | 7.4.2.2.4 |
| 7.4.2.3.1 | 7.4.2.3.2 | 7.4.2.3.3 | 7.4.2.3.4 | 7.4.2.4.1 | 7.4.2.4.2 | 7.4.2.4.3 | 7.4.2.4.4 |
| 7.4.2.5.1 | 7.4.2.5.2 | 7.4.2.5.3 | 7.4.2.5.4 | 7.4.2.6.1 | 7.4.2.6.2 | 7.4.2.6.3 | 7.4.2.6.4 |
| 7.4.2.7.1 | 7.4.2.7.2 | 7.4.2.7.3 | 7.4.2.7.4 | 7.4.2.8.1 | 7.4.2.8.2 | 7.4.2.8.3 | 7.4.2.8.4 |
| 7.4.3.1.1 | 7.4.3.1.2 | 7.4.3.1.3 | 7.4.3.1.4 | 7.4.3.2.1 | 7.4.3.2.2 | 7.4.3.2.3 | 7.4.3.2.4 |
| 7.4.3.3.1 | 7.4.3.3.2 | 7.4.3.3.3 | 7.4.3.3.4 | 7.4.3.4.1 | 7.4.3.4.2 | 7.4.3.4.3 | 7.4.3.4.4 |
| 7.4.3.5.1 | 7.4.3.5.2 | 7.4.3.5.3 | 7.4.3.5.4 | 7.4.3.6.1 | 7.4.3.6.2 | 7.4.3.6.3 | 7.4.3.6.4 |
| 7.4.3.7.1 | 7.4.3.7.2 | 7.4.3.7.3 | 7.4.3.7.4 | 7.4.3.8.1 | 7.4.3.8.2 | 7.4.3.8.3 | 7.4.3.8.4 |
| 7.4.4.1.1 | 7.4.4.1.2 | 7.4.4.1.3 | 7.4.4.1.4 | 7.4.4.2.1 | 7.4.4.2.2 | 7.4.4.2.3 | 7.4.4.2.4 |
| 7.4.4.3.1 | 7.4.4.3.2 | 7.4.4.3.3 | 7.4.4.3.4 | 7.4.4.4.1 | 7.4.4.4.2 | 7.4.4.4.3 | 7.4.4.4.4 |
| 7.4.4.5.1 | 7.4.4.5.2 | 7.4.4.5.3 | 7.4.4.5.4 | 7.4.4.6.1 | 7.4.4.6.2 | 7.4.4.6.3 | 7.4.4.6.4 |
| 7.4.4.7.1 | 7.4.4.7.2 | 7.4.4.7.3 | 7.4.4.7.4 | 7.4.4.8.1 | 7.4.4.8.2 | 7.4.4.8.3 | 7.4.4.8.4 |
| 7.5.1.1.1 | 7.5.1.1.2 | 7.5.1.1.3 | 7.5.1.1.4 | 7.5.1.2.1 | 7.5.1.2.2 | 7.5.1.2.3 | 7.5.1.2.4 |
| 7.5.1.3.1 | 7.5.1.3.2 | 7.5.1.3.3 | 7.5.1.3.4 | 7.5.1.4.1 | 7.5.1.4.2 | 7.5.1.4.3 | 7.5.1.4.4 |
| 7.5.1.5.1 | 7.5.1.5.2 | 7.5.1.5.3 | 7.5.1.5.4 | 7.5.1.6.1 | 7.5.1.6.2 | 7.5.1.6.3 | 7.5.1.6.4 |
| 7.5.1.7.1 | 7.5.1.7.2 | 7.5.1.7.3 | 7.5.1.7.4 | 7.5.1.8.1 | 7.5.1.8.2 | 7.5.1.8.3 | 7.5.1.8.4 |
| 7.5.2.1.1 | 7.5.2.1.2 | 7.5.2.1.3 | 7.5.2.1.4 | 7.5.2.2.1 | 7.5.2.2.2 | 7.5.2.2.3 | 7.5.2.2.4 |
| 7.5.2.3.1 | 7.5.2.3.2 | 7.5.2.3.3 | 7.5.2.3.4 | 7.5.2.4.1 | 7.5.2.4.2 | 7.5.2.4.3 | 7.5.2.4.4 |
| 7.5.2.5.1 | 7.5.2.5.2 | 7.5.2.5.3 | 7.5.2.5.4 | 7.5.2.6.1 | 7.5.2.6.2 | 7.5.2.6.3 | 7.5.2.6.4 |
| 7.5.2.7.1 | 7.5.2.7.2 | 7.5.2.7.3 | 7.5.2.7.4 | 7.5.2.8.1 | 7.5.2.8.2 | 7.5.2.8.3 | 7.5.2.8.4 |
| 7.5.3.1.1 | 7.5.3.1.2 | 7.5.3.1.3 | 7.5.3.1.4 | 7.5.3.2.1 | 7.5.3.2.2 | 7.5.3.2.3 | 7.5.3.2.4 |
| 7.5.3.3.1 | 7.5.3.3.2 | 7.5.3.3.3 | 7.5.3.3.4 | 7.5.3.4.1 | 7.5.3.4.2 | 7.5.3.4.3 | 7.5.3.4.4 |
| 7.5.3.5.1 | 7.5.3.5.2 | 7.5.3.5.3 | 7.5.3.5.4 | 7.5.3.6.1 | 7.5.3.6.2 | 7.5.3.6.3 | 7.5.3.6.4 |
| 7.5.3.7.1 | 7.5.3.7.2 | 7.5.3.7.3 | 7.5.3.7.4 | 7.5.3.8.1 | 7.5.3.8.2 | 7.5.3.8.3 | 7.5.3.8.4 |
| 7.5.4.1.1 | 7.5.4.1.2 | 7.5.4.1.3 | 7.5.4.1.4 | 7.5.4.2.1 | 7.5.4.2.2 | 7.5.4.2.3 | 7.5.4.2.4 |
| 7.5.4.3.1 | 7.5.4.3.2 | 7.5.4.3.3 | 7.5.4.3.4 | 7.5.4.4.1 | 7.5.4.4.2 | 7.5.4.4.3 | 7.5.4.4.4 |
| 7.5.4.5.1 | 7.5.4.5.2 | 7.5.4.5.3 | 7.5.4.5.4 | 7.5.4.6.1 | 7.5.4.6.2 | 7.5.4.6.3 | 7.5.4.6.4 |
| 7.5.4.7.1 | 7.5.4.7.2 | 7.5.4.7.3 | 7.5.4.7.4 | 7.5.4.8.1 | 7.5.4.8.2 | 7.5.4.8.3 | 7.5.4.8.4 |
| 7.6.1.1.1 | 7.6.1.1.2 | 7.6.1.1.3 | 7.6.1.1.4 | 7.6.1.2.1 | 7.6.1.2.2 | 7.6.1.2.3 | 7.6.1.2.4 |
| 7.6.1.3.1 | 7.6.1.3.2 | 7.6.1.3.3 | 7.6.1.3.4 | 7.6.1.4.1 | 7.6.1.4.2 | 7.6.1.4.3 | 7.6.1.4.4 |
| 7.6.1.5.1 | 7.6.1.5.2 | 7.6.1.5.3 | 7.6.1.5.4 | 7.6.1.6.1 | 7.6.1.6.2 | 7.6.1.6.3 | 7.6.1.6.4 |
| 7.6.1.7.1 | 7.6.1.7.2 | 7.6.1.7.3 | 7.6.1.7.4 | 7.6.1.8.1 | 7.6.1.8.2 | 7.6.1.8.3 | 7.6.1.8.4 |
| 7.6.2.1.1 | 7.6.2.1.2 | 7.6.2.1.3 | 7.6.2.1.4 | 7.6.2.2.1 | 7.6.2.2.2 | 7.6.2.2.3 | 7.6.2.2.4 |
| 7.6.2.3.1 | 7.6.2.3.2 | 7.6.2.3.3 | 7.6.2.3.4 | 7.6.2.4.1 | 7.6.2.4.2 | 7.6.2.4.3 | 7.6.2.4.4 |
| 7.6.2.5.1 | 7.6.2.5.2 | 7.6.2.5.3 | 7.6.2.5.4 | 7.6.2.6.1 | 7.6.2.6.2 | 7.6.2.6.3 | 7.6.2.6.4 |
| 7.6.2.7.1 | 7.6.2.7.2 | 7.6.2.7.3 | 7.6.2.7.4 | 7.6.2.8.1 | 7.6.2.8.2 | 7.6.2.8.3 | 7.6.2.8.4 |
| 7.6.3.1.1 | 7.6.3.1.2 | 7.6.3.1.3 | 7.6.3.1.4 | 7.6.3.2.1 | 7.6.3.2.2 | 7.6.3.2.3 | 7.6.3.2.4 |
| 7.6.3.3.1 | 7.6.3.3.2 | 7.6.3.3.3 | 7.6.3.3.4 | 7.6.3.4.1 | 7.6.3.4.2 | 7.6.3.4.3 | 7.6.3.4.4 |
| 7.6.3.5.1 | 7.6.3.5.2 | 7.6.3.5.3 | 7.6.3.5.4 | 7.6.3.6.1 | 7.6.3.6.2 | 7.6.3.6.3 | 7.6.3.6.4 |
| 7.6.3.7.1 | 7.6.3.7.2 | 7.6.3.7.3 | 7.6.3.7.4 | 7.6.3.8.1 | 7.6.3.8.2 | 7.6.3.8.3 | 7.6.3.8.4 |
| 7.6.4.1.1 | 7.6.4.1.2 | 7.6.4.1.3 | 7.6.4.1.4 | 7.6.4.2.1 | 7.6.4.2.2 | 7.6.4.2.3 | 7.6.4.2.4 |
| 7.6.4.3.1 | 7.6.4.3.2 | 7.6.4.3.3 | 7.6.4.3.4 | 7.6.4.4.1 | 7.6.4.4.2 | 7.6.4.4.3 | 7.6.4.4.4 |
| 7.6.4.5.1 | 7.6.4.5.2 | 7.6.4.5.3 | 7.6.4.5.4 | 7.6.4.6.1 | 7.6.4.6.2 | 7.6.4.6.3 | 7.6.4.6.4 |
| 7.6.4.7.1 | 7.6.4.7.2 | 7.6.4.7.3 | 7.6.4.7.4 | 7.6.4.8.1 | 7.6.4.8.2 | 7.6.4.8.3 | 7.6.4.8.4 |
| 7.7.1.1.1 | 7.7.1.1.2 | 7.7.1.1.3 | 7.7.1.1.4 | 7.7.1.2.1 | 7.7.1.2.2 | 7.7.1.2.3 | 7.7.1.2.4 |
| 7.7.1.3.1 | 7.7.1.3.2 | 7.7.1.3.3 | 7.7.1.3.4 | 7.7.1.4.1 | 7.7.1.4.2 | 7.7.1.4.3 | 7.7.1.4.4 |
| 7.7.1.5.1 | 7.7.1.5.2 | 7.7.1.5.3 | 7.7.1.5.4 | 7.7.1.6.1 | 7.7.1.6.2 | 7.7.1.6.3 | 7.7.1.6.4 |
| 7.7.1.7.1 | 7.7.1.7.2 | 7.7.1.7.3 | 7.7.1.7.4 | 7.7.1.8.1 | 7.7.1.8.2 | 7.7.1.8.3 | 7.7.1.8.4 |
| 7.7.2.1.1 | 7.7.2.1.2 | 7.7.2.1.3 | 7.7.2.1.4 | 7.7.2.2.1 | 7.7.2.2.2 | 7.7.2.2.3 | 7.7.2.2.4 |
| 7.7.2.3.1 | 7.7.2.3.2 | 7.7.2.3.3 | 7.7.2.3.4 | 7.7.2.4.1 | 7.7.2.4.2 | 7.7.2.4.3 | 7.7.2.4.4 |
| 7.7.2.5.1 | 7.7.2.5.2 | 7.7.2.5.3 | 7.7.2.5.4 | 7.7.2.6.1 | 7.7.2.6.2 | 7.7.2.6.3 | 7.7.2.6.4 |
| 7.7.2.7.1 | 7.7.2.7.2 | 7.7.2.7.3 | 7.7.2.7.4 | 7.7.2.8.1 | 7.7.2.8.2 | 7.7.2.8.3 | 7.7.2.8.4 |
| 7.7.3.1.1 | 7.7.3.1.2 | 7.7.3.1.3 | 7.7.3.1.4 | 7.7.3.2.1 | 7.7.3.2.2 | 7.7.3.2.3 | 7.7.3.2.4 |
| 7.7.3.3.1 | 7.7.3.3.2 | 7.7.3.3.3 | 7.7.3.3.4 | 7.7.3.4.1 | 7.7.3.4.2 | 7.7.3.4.3 | 7.7.3.4.4 |
| 7.7.3.5.1 | 7.7.3.5.2 | 7.7.3.5.3 | 7.7.3.5.4 | 7.7.3.6.1 | 7.7.3.6.2 | 7.7.3.6.3 | 7.7.3.6.4 |
| 7.7.3.7.1 | 7.7.3.7.2 | 7.7.3.7.3 | 7.7.3.7.4 | 7.7.3.8.1 | 7.7.3.8.2 | 7.7.3.8.3 | 7.7.3.8.4 |
| 7.7.4.1.1 | 7.7.4.1.2 | 7.7.4.1.3 | 7.7.4.1.4 | 7.7.4.2.1 | 7.7.4.2.2 | 7.7.4.2.3 | 7.7.4.2.4 |
| 7.7.4.3.1 | 7.7.4.3.2 | 7.7.4.3.3 | 7.7.4.3.4 | 7.7.4.4.1 | 7.7.4.4.2 | 7.7.4.4.3 | 7.7.4.4.4 |
| 7.7.4.5.1 | 7.7.4.5.2 | 7.7.4.5.3 | 7.7.4.5.4 | 7.7.4.6.1 | 7.7.4.6.2 | 7.7.4.6.3 | 7.7.4.6.4 |
| 7.7.4.7.1 | 7.7.4.7.2 | 7.7.4.7.3 | 7.7.4.7.4 | 7.7.4.8.1 | 7.7.4.8.2 | 7.7.4.8.3 | 7.7.4.8.4 |
| 7.8.1.1.1 | 7.8.1.1.2 | 7.8.1.1.3 | 7.8.1.1.4 | 7.8.1.2.1 | 7.8.1.2.2 | 7.8.1.2.3 | 7.8.1.2.4 |
| 7.8.1.3.1 | 7.8.1.3.2 | 7.8.1.3.3 | 7.8.1.3.4 | 7.8.1.4.1 | 7.8.1.4.2 | 7.8.1.4.3 | 7.8.1.4.4 |
| 7.8.1.5.1 | 7.8.1.5.2 | 7.8.1.5.3 | 7.8.1.5.4 | 7.8.1.6.1 | 7.8.1.6.2 | 7.8.1.6.3 | 7.8.1.6.4 |
| 7.8.1.7.1 | 7.8.1.7.2 | 7.8.1.7.3 | 7.8.1.7.4 | 7.8.1.8.1 | 7.8.1.8.2 | 7.8.1.8.3 | 7.8.1.8.4 |
| 7.8.2.1.1 | 7.8.2.1.2 | 7.8.2.1.3 | 7.8.2.1.4 | 7.8.2.2.1 | 7.8.2.2.2 | 7.8.2.2.3 | 7.8.2.2.4 |
| 7.8.2.3.1 | 7.8.2.3.2 | 7.8.2.3.3 | 7.8.2.3.4 | 7.8.2.4.1 | 7.8.2.4.2 | 7.8.2.4.3 | 7.8.2.4.4 |
| 7.8.2.5.1 | 7.8.2.5.2 | 7.8.2.5.3 | 7.8.2.5.4 | 7.8.2.6.1 | 7.8.2.6.2 | 7.8.2.6.3 | 7.8.2.6.4 |
| 7.8.2.7.1 | 7.8.2.7.2 | 7.8.2.7.3 | 7.8.2.7.4 | 7.8.2.8.1 | 7.8.2.8.2 | 7.8.2.8.3 | 7.8.2.8.4 |
| 7.8.3.1.1 | 7.8.3.1.2 | 7.8.3.1.3 | 7.8.3.1.4 | 7.8.3.2.1 | 7.8.3.2.2 | 7.8.3.2.3 | 7.8.3.2.4 |
| 7.8.3.3.1 | 7.8.3.3.2 | 7.8.3.3.3 | 7.8.3.3.4 | 7.8.3.4.1 | 7.8.3.4.2 | 7.8.3.4.3 | 7.8.3.4.4 |
| 7.8.3.5.1 | 7.8.3.5.2 | 7.8.3.5.3 | 7.8.3.5.4 | 7.8.3.6.1 | 7.8.3.6.2 | 7.8.3.6.3 | 7.8.3.6.4 |
| 7.8.3.7.1 | 7.8.3.7.2 | 7.8.3.7.3 | 7.8.3.7.4 | 7.8.3.8.1 | 7.8.3.8.2 | 7.8.3.8.3 | 7.8.3.8.4 |
| 7.8.4.1.1 | 7.8.4.1.2 | 7.8.4.1.3 | 7.8.4.1.4 | 7.8.4.2.1 | 7.8.4.2.2 | 7.8.4.2.3 | 7.8.4.2.4 |
| 7.8.4.3.1 | 7.8.4.3.2 | 7.8.4.3.3 | 7.8.4.3.4 | 7.8.4.4.1 | 7.8.4.4.2 | 7.8.4.4.3 | 7.8.4.4.4 |
| 7.8.4.5.1 | 7.8.4.5.2 | 7.8.4.5.3 | 7.8.4.5.4 | 7.8.4.6.1 | 7.8.4.6.2 | 7.8.4.6.3 | 7.8.4.6.4 |
| 7.8.4.7.1 | 7.8.4.7.2 | 7.8.4.7.3 | 7.8.4.7.4 | 7.8.4.8.1 | 7.8.4.8.2 | 7.8.4.8.3 | 7.8.4.8.4 |
| 8.1.1.1.1 | 8.1.1.1.2 | 8.1.1.1.3 | 8.1.1.1.4 | 8.1.1.2.1 | 8.1.1.2.2 | 8.1.1.2.3 | 8.1.1.2.4 |
| 8.1.1.3.1 | 8.1.1.3.2 | 8.1.1.3.3 | 8.1.1.3.4 | 8.1.1.4.1 | 8.1.1.4.2 | 8.1.1.4.3 | 8.1.1.4.4 |
| 8.1.1.5.1 | 8.1.1.5.2 | 8.1.1.5.3 | 8.1.1.5.4 | 8.1.1.6.1 | 8.1.1.6.2 | 8.1.1.6.3 | 8.1.1.6.4 |
| 8.1.1.7.1 | 8.1.1.7.2 | 8.1.1.7.3 | 8.1.1.7.4 | 8.1.1.8.1 | 8.1.1.8.2 | 8.1.1.8.3 | 8.1.1.8.4 |
| 8.1.2.1.1 | 8.1.2.1.2 | 8.1.2.1.3 | 8.1.2.1.4 | 8.1.2.2.1 | 8.1.2.2.2 | 8.1.2.2.3 | 8.1.2.2.4 |
| 8.1.2.3.1 | 8.1.2.3.2 | 8.1.2.3.3 | 8.1.2.3.4 | 8.1.2.4.1 | 8.1.2.4.2 | 8.1.2.4.3 | 8.1.2.4.4 |
| 8.1.2.5.1 | 8.1.2.5.2 | 8.1.2.5.3 | 8.1.2.5.4 | 8.1.2.6.1 | 8.1.2.6.2 | 8.1.2.6.3 | 8.1.2.6.4 |
| 8.1.2.7.1 | 8.1.2.7.2 | 8.1.2.7.3 | 8.1.2.7.4 | 8.1.2.8.1 | 8.1.2.8.2 | 8.1.2.8.3 | 8.1.2.8.4 |
| 8.1.3.1.1 | 8.1.3.1.2 | 8.1.3.1.3 | 8.1.3.1.4 | 8.1.3.2.1 | 8.1.3.2.2 | 8.1.3.2.3 | 8.1.3.2.4 |
| 8.1.3.3.1 | 8.1.3.3.2 | 8.1.3.3.3 | 8.1.3.3.4 | 8.1.3.4.1 | 8.1.3.4.2 | 8.1.3.4.3 | 8.1.3.4.4 |
| 8.1.3.5.1 | 8.1.3.5.2 | 8.1.3.5.3 | 8.1.3.5.4 | 8.1.3.6.1 | 8.1.3.6.2 | 8.1.3.6.3 | 8.1.3.6.4 |
| 8.1.3.7.1 | 8.1.3.7.2 | 8.1.3.7.3 | 8.1.3.7.4 | 8.1.3.8.1 | 8.1.3.8.2 | 8.1.3.8.3 | 8.1.3.8.4 |
| 8.1.4.1.1 | 8.1.4.1.2 | 8.1.4.1.3 | 8.1.4.1.4 | 8.1.4.2.1 | 8.1.4.2.2 | 8.1.4.2.3 | 8.1.4.2.4 |
| 8.1.4.3.1 | 8.1.4.3.2 | 8.1.4.3.3 | 8.1.4.3.4 | 8.1.4.4.1 | 8.1.4.4.2 | 8.1.4.4.3 | 8.1.4.4.4 |
| 8.1.4.5.1 | 8.1.4.5.2 | 8.1.4.5.3 | 8.1.4.5.4 | 8.1.4.6.1 | 8.1.4.6.2 | 8.1.4.6.3 | 8.1.4.6.4 |
| 8.1.4.7.1 | 8.1.4.7.2 | 8.1.4.7.3 | 8.1.4.7.4 | 8.1.4.8.1 | 8.1.4.8.2 | 8.1.4.8.3 | 8.1.4.8.4 |
| 8.2.1.1.1 | 8.2.1.1.2 | 8.2.1.1.3 | 8.2.1.1.4 | 8.2.1.2.1 | 8.2.1.2.2 | 8.2.1.2.3 | 8.2.1.2.4 |
| 8.2.1.3.1 | 8.2.1.3.2 | 8.2.1.3.3 | 8.2.1.3.4 | 8.2.1.4.1 | 8.2.1.4.2 | 8.2.1.4.3 | 8.2.1.4.4 |
| 8.2.1.5.1 | 8.2.1.5.2 | 8.2.1.5.3 | 8.2.1.5.4 | 8.2.1.6.1 | 8.2.1.6.2 | 8.2.1.6.3 | 8.2.1.6.4 |
| 8.2.1.7.1 | 8.2.1.7.2 | 8.2.1.7.3 | 8.2.1.7.4 | 8.2.1.8.1 | 8.2.1.8.2 | 8.2.1.8.3 | 8.2.1.8.4 |
| 8.2.2.1.1 | 8.2.2.1.2 | 8.2.2.1.3 | 8.2.2.1.4 | 8.2.2.2.1 | 8.2.2.2.2 | 8.2.2.2.3 | 8.2.2.2.4 |
| 8.2.2.3.1 | 8.2.2.3.2 | 8.2.2.3.3 | 8.2.2.3.4 | 8.2.2.4.1 | 8.2.2.4.2 | 8.2.2.4.3 | 8.2.2.4.4 |
| 8.2.2.5.1 | 8.2.2.5.2 | 8.2.2.5.3 | 8.2.2.5.4 | 8.2.2.6.1 | 8.2.2.6.2 | 8.2.2.6.3 | 8.2.2.6.4 |
| 8.2.2.7.1 | 8.2.2.7.2 | 8.2.2.7.3 | 8.2.2.7.4 | 8.2.2.8.1 | 8.2.2.8.2 | 8.2.2.8.3 | 8.2.2.8.4 |
| 8.2.3.1.1 | 8.2.3.1.2 | 8.2.3.1.3 | 8.2.3.1.4 | 8.2.3.2.1 | 8.2.3.2.2 | 8.2.3.2.3 | 8.2.3.2.4 |
| 8.2.3.3.1 | 8.2.3.3.2 | 8.2.3.3.3 | 8.2.3.3.4 | 8.2.3.4.1 | 8.2.3.4.2 | 8.2.3.4.3 | 8.2.3.4.4 |
| 8.2.3.5.1 | 8.2.3.5.2 | 8.2.3.5.3 | 8.2.3.5.4 | 8.2.3.6.1 | 8.2.3.6.2 | 8.2.3.6.3 | 8.2.3.6.4 |
| 8.2.3.7.1 | 8.2.3.7.2 | 8.2.3.7.3 | 8.2.3.7.4 | 8.2.3.8.1 | 8.2.3.8.2 | 8.2.3.8.3 | 8.2.3.8.4 |
| 8.2.4.1.1 | 8.2.4.1.2 | 8.2.4.1.3 | 8.2.4.1.4 | 8.2.4.2.1 | 8.2.4.2.2 | 8.2.4.2.3 | 8.2.4.2.4 |
| 8.2.4.3.1 | 8.2.4.3.2 | 8.2.4.3.3 | 8.2.4.3.4 | 8.2.4.4.1 | 8.2.4.4.2 | 8.2.4.4.3 | 8.2.4.4.4 |
| 8.2.4.5.1 | 8.2.4.5.2 | 8.2.4.5.3 | 8.2.4.5.4 | 8.2.4.6.1 | 8.2.4.6.2 | 8.2.4.6.3 | 8.2.4.6.4 |
| 8.2.4.7.1 | 8.2.4.7.2 | 8.2.4.7.3 | 8.2.4.7.4 | 8.2.4.8.1 | 8.2.4.8.2 | 8.2.4.8.3 | 8.2.4.8.4 |
| 8.3.1.1.1 | 8.3.1.1.2 | 8.3.1.1.3 | 8.3.1.1.4 | 8.3.1.2.1 | 8.3.1.2.2 | 8.3.1.2.3 | 8.3.1.2.4 |
| 8.3.1.3.1 | 8.3.1.3.2 | 8.3.1.3.3 | 8.3.1.3.4 | 8.3.1.4.1 | 8.3.1.4.2 | 8.3.1.4.3 | 8.3.1.4.4 |
| 8.3.1.5.1 | 8.3.1.5.2 | 8.3.1.5.3 | 8.3.1.5.4 | 8.3.1.6.1 | 8.3.1.6.2 | 8.3.1.6.3 | 8.3.1.6.4 |
| 8.3.1.7.1 | 8.3.1.7.2 | 8.3.1.7.3 | 8.3.1.7.4 | 8.3.1.8.1 | 8.3.1.8.2 | 8.3.1.8.3 | 8.3.1.8.4 |
| 8.3.2.1.1 | 8.3.2.1.2 | 8.3.2.1.3 | 8.3.2.1.4 | 8.3.2.2.1 | 8.3.2.2.2 | 8.3.2.2.3 | 8.3.2.2.4 |
| 8.3.2.3.1 | 8.3.2.3.2 | 8.3.2.3.3 | 8.3.2.3.4 | 8.3.2.4.1 | 8.3.2.4.2 | 8.3.2.4.3 | 8.3.2.4.4 |
| 8.3.2.5.1 | 8.3.2.5.2 | 8.3.2.5.3 | 8.3.2.5.4 | 8.3.2.6.1 | 8.3.2.6.2 | 8.3.2.6.3 | 8.3.2.6.4 |
| 8.3.2.7.1 | 8.3.2.7.2 | 8.3.2.7.3 | 8.3.2.7.4 | 8.3.2.8.1 | 8.3.2.8.2 | 8.3.2.8.3 | 8.3.2.8.4 |
| 8.3.3.1.1 | 8.3.3.1.2 | 8.3.3.1.3 | 8.3.3.1.4 | 8.3.3.2.1 | 8.3.3.2.2 | 8.3.3.2.3 | 8.3.3.2.4 |
| 8.3.3.3.1 | 8.3.3.3.2 | 8.3.3.3.3 | 8.3.3.3.4 | 8.3.3.4.1 | 8.3.3.4.2 | 8.3.3.4.3 | 8.3.3.4.4 |
| 8.3.3.5.1 | 8.3.3.5.2 | 8.3.3.5.3 | 8.3.3.5.4 | 8.3.3.6.1 | 8.3.3.6.2 | 8.3.3.6.3 | 8.3.3.6.4 |
| 8.3.3.7.1 | 8.3.3.7.2 | 8.3.3.7.3 | 8.3.3.7.4 | 8.3.3.8.1 | 8.3.3.8.2 | 8.3.3.8.3 | 8.3.3.8.4 |
| 8.3.4.1.1 | 8.3.4.1.2 | 8.3.4.1.3 | 8.3.4.1.4 | 8.3.4.2.1 | 8.3.4.2.2 | 8.3.4.2.3 | 8.3.4.2.4 |
| 8.3.4.3.1 | 8.3.4.3.2 | 8.3.4.3.3 | 8.3.4.3.4 | 8.3.4.4.1 | 8.3.4.4.2 | 8.3.4.4.3 | 8.3.4.4.4 |
| 8.3.4.5.1 | 8.3.4.5.2 | 8.3.4.5.3 | 8.3.4.5.4 | 8.3.4.6.1 | 8.3.4.6.2 | 8.3.4.6.3 | 8.3.4.6.4 |
| 8.3.4.7.1 | 8.3.4.7.2 | 8.3.4.7.3 | 8.3.4.7.4 | 8.3.4.8.1 | 8.3.4.8.2 | 8.3.4.8.3 | 8.3.4.8.4 |
| 8.4.1.1.1 | 8.4.1.1.2 | 8.4.1.1.3 | 8.4.1.1.4 | 8.4.1.2.1 | 8.4.1.2.2 | 8.4.1.2.3 | 8.4.1.2.4 |
| 8.4.1.3.1 | 8.4.1.3.2 | 8.4.1.3.3 | 8.4.1.3.4 | 8.4.1.4.1 | 8.4.1.4.2 | 8.4.1.4.3 | 8.4.1.4.4 |
| 8.4.1.5.1 | 8.4.1.5.2 | 8.4.1.5.3 | 8.4.1.5.4 | 8.4.1.6.1 | 8.4.1.6.2 | 8.4.1.6.3 | 8.4.1.6.4 |
| 8.4.1.7.1 | 8.4.1.7.2 | 8.4.1.7.3 | 8.4.1.7.4 | 8.4.1.8.1 | 8.4.1.8.2 | 8.4.1.8.3 | 8.4.1.8.4 |
| 8.4.2.1.1 | 8.4.2.1.2 | 8.4.2.1.3 | 8.4.2.1.4 | 8.4.2.2.1 | 8.4.2.2.2 | 8.4.2.2.3 | 8.4.2.2.4 |
| 8.4.2.3.1 | 8.4.2.3.2 | 8.4.2.3.3 | 8.4.2.3.4 | 8.4.2.4.1 | 8.4.2.4.2 | 8.4.2.4.3 | 8.4.2.4.4 |
| 8.4.2.5.1 | 8.4.2.5.2 | 8.4.2.5.3 | 8.4.2.5.4 | 8.4.2.6.1 | 8.4.2.6.2 | 8.4.2.6.3 | 8.4.2.6.4 |
| 8.4.2.7.1 | 8.4.2.7.2 | 8.4.2.7.3 | 8.4.2.7.4 | 8.4.2.8.1 | 8.4.2.8.2 | 8.4.2.8.3 | 8.4.2.8.4 |
| 8.4.3.1.1 | 8.4.3.1.2 | 8.4.3.1.3 | 8.4.3.1.4 | 8.4.3.2.1 | 8.4.3.2.2 | 8.4.3.2.3 | 8.4.3.2.4 |
| 8.4.3.3.1 | 8.4.3.3.2 | 8.4.3.3.3 | 8.4.3.3.4 | 8.4.3.4.1 | 8.4.3.4.2 | 8.4.3.4.3 | 8.4.3.4.4 |
| 8.4.3.5.1 | 8.4.3.5.2 | 8.4.3.5.3 | 8.4.3.5.4 | 8.4.3.6.1 | 8.4.3.6.2 | 8.4.3.6.3 | 8.4.3.6.4 |
| 8.4.3.7.1 | 8.4.3.7.2 | 8.4.3.7.3 | 8.4.3.7.4 | 8.4.3.8.1 | 8.4.3.8.2 | 8.4.3.8.3 | 8.4.3.8.4 |
| 8.4.4.1.1 | 8.4.4.1.2 | 8.4.4.1.3 | 8.4.4.1.4 | 8.4.4.2.1 | 8.4.4.2.2 | 8.4.4.2.3 | 8.4.4.2.4 |
| 8.4.4.3.1 | 8.4.4.3.2 | 8.4.4.3.3 | 8.4.4.3.4 | 8.4.4.4.1 | 8.4.4.4.2 | 8.4.4.4.3 | 8.4.4.4.4 |
| 8.4.4.5.1 | 8.4.4.5.2 | 8.4.4.5.3 | 8.4.4.5.4 | 8.4.4.6.1 | 8.4.4.6.2 | 8.4.4.6.3 | 8.4.4.6.4 |
| 8.4.4.7.1 | 8.4.4.7.2 | 8.4.4.7.3 | 8.4.4.7.4 | 8.4.4.8.1 | 8.4.4.8.2 | 8.4.4.8.3 | 8.4.4.8.4 |
| 8.5.1.1.1 | 8.5.1.1.2 | 8.5.1.1.3 | 8.5.1.1.4 | 8.5.1.2.1 | 8.5.1.2.2 | 8.5.1.2.3 | 8.5.1.2.4 |
| 8.5.1.3.1 | 8.5.1.3.2 | 8.5.1.3.3 | 8.5.1.3.4 | 8.5.1.4.1 | 8.5.1.4.2 | 8.5.1.4.3 | 8.5.1.4.4 |
| 8.5.1.5.1 | 8.5.1.5.2 | 8.5.1.5.3 | 8.5.1.5.4 | 8.5.1.6.1 | 8.5.1.6.2 | 8.5.1.6.3 | 8.5.1.6.4 |
| 8.5.1.7.1 | 8.5.1.7.2 | 8.5.1.7.3 | 8.5.1.7.4 | 8.5.1.8.1 | 8.5.1.8.2 | 8.5.1.8.3 | 8.5.1.8.4 |
| 8.5.2.1.1 | 8.5.2.1.2 | 8.5.2.1.3 | 8.5.2.1.4 | 8.5.2.2.1 | 8.5.2.2.2 | 8.5.2.2.3 | 8.5.2.2.4 |
| 8.5.2.3.1 | 8.5.2.3.2 | 8.5.2.3.3 | 8.5.2.3.4 | 8.5.2.4.1 | 8.5.2.4.2 | 8.5.2.4.3 | 8.5.2.4.4 |
| 8.5.2.5.1 | 8.5.2.5.2 | 8.5.2.5.3 | 8.5.2.5.4 | 8.5.2.6.1 | 8.5.2.6.2 | 8.5.2.6.3 | 8.5.2.6.4 |
| 8.5.2.7.1 | 8.5.2.7.2 | 8.5.2.7.3 | 8.5.2.7.4 | 8.5.2.8.1 | 8.5.2.8.2 | 8.5.2.8.3 | 8.5.2.8.4 |
| 8.5.3.1.1 | 8.5.3.1.2 | 8.5.3.1.3 | 8.5.3.1.4 | 8.5.3.2.1 | 8.5.3.2.2 | 8.5.3.2.3 | 8.5.3.2.4 |
| 8.5.3.3.1 | 8.5.3.3.2 | 8.5.3.3.3 | 8.5.3.3.4 | 8.5.3.4.1 | 8.5.3.4.2 | 8.5.3.4.3 | 8.5.3.4.4 |
| 8.5.3.5.1 | 8.5.3.5.2 | 8.5.3.5.3 | 8.5.3.5.4 | 8.5.3.6.1 | 8.5.3.6.2 | 8.5.3.6.3 | 8.5.3.6.4 |
| 8.5.3.7.1 | 8.5.3.7.2 | 8.5.3.7.3 | 8.5.3.7.4 | 8.5.3.8.1 | 8.5.3.8.2 | 8.5.3.8.3 | 8.5.3.8.4 |
| 8.5.4.1.1 | 8.5.4.1.2 | 8.5.4.1.3 | 8.5.4.1.4 | 8.5.4.2.1 | 8.5.4.2.2 | 8.5.4.2.3 | 8.5.4.2.4 |
| 8.5.4.3.1 | 8.5.4.3.2 | 8.5.4.3.3 | 8.5.4.3.4 | 8.5.4.4.1 | 8.5.4.4.2 | 8.5.4.4.3 | 8.5.4.4.4 |
| 8.5.4.5.1 | 8.5.4.5.2 | 8.5.4.5.3 | 8.5.4.5.4 | 8.5.4.6.1 | 8.5.4.6.2 | 8.5.4.6.3 | 8.5.4.6.4 |
| 8.5.4.7.1 | 8.5.4.7.2 | 8.5.4.7.3 | 8.5.4.7.4 | 8.5.4.8.1 | 8.5.4.8.2 | 8.5.4.8.3 | 8.5.4.8.4 |
| 8.6.1.1.1 | 8.6.1.1.2 | 8.6.1.1.3 | 8.6.1.1.4 | 8.6.1.2.1 | 8.6.1.2.2 | 8.6.1.2.3 | 8.6.1.2.4 |
| 8.6.1.3.1 | 8.6.1.3.2 | 8.6.1.3.3 | 8.6.1.3.4 | 8.6.1.4.1 | 8.6.1.4.2 | 8.6.1.4.3 | 8.6.1.4.4 |
| 8.6.1.5.1 | 8.6.1.5.2 | 8.6.1.5.3 | 8.6.1.5.4 | 8.6.1.6.1 | 8.6.1.6.2 | 8.6.1.6.3 | 8.6.1.6.4 |
| 8.6.1.7.1 | 8.6.1.7.2 | 8.6.1.7.3 | 8.6.1.7.4 | 8.6.1.8.1 | 8.6.1.8.2 | 8.6.1.8.3 | 8.6.1.8.4 |
| 8.6.2.1.1 | 8.6.2.1.2 | 8.6.2.1.3 | 8.6.2.1.4 | 8.6.2.2.1 | 8.6.2.2.2 | 8.6.2.2.3 | 8.6.2.2.4 |
| 8.6.2.3.1 | 8.6.2.3.2 | 8.6.2.3.3 | 8.6.2.3.4 | 8.6.2.4.1 | 8.6.2.4.2 | 8.6.2.4.3 | 8.6.2.4.4 |
| 8.6.2.5.1 | 8.6.2.5.2 | 8.6.2.5.3 | 8.6.2.5.4 | 8.6.2.6.1 | 8.6.2.6.2 | 8.6.2.6.3 | 8.6.2.6.4 |
| 8.6.2.7.1 | 8.6.2.7.2 | 8.6.2.7.3 | 8.6.2.7.4 | 8.6.2.8.1 | 8.6.2.8.2 | 8.6.2.8.3 | 8.6.2.8.4 |
| 8.6.3.1.1 | 8.6.3.1.2 | 8.6.3.1.3 | 8.6.3.1.4 | 8.6.3.2.1 | 8.6.3.2.2 | 8.6.3.2.3 | 8.6.3.2.4 |
| 8.6.3.3.1 | 8.6.3.3.2 | 8.6.3.3.3 | 8.6.3.3.4 | 8.6.3.4.1 | 8.6.3.4.2 | 8.6.3.4.3 | 8.6.3.4.4 |
| 8.6.3.5.1 | 8.6.3.5.2 | 8.6.3.5.3 | 8.6.3.5.4 | 8.6.3.6.1 | 8.6.3.6.2 | 8:6.3.6.3 | 8.6.3.6.4 |
| 8.6.3.7.1 | 8.6.3.7.2 | 8.6.3.7.3 | 8.6.3.7.4 | 8.6.3.8.1 | 8.6.3.8.2 | 8.6.3.8.3 | 8.6.3.8.4 |
| 8.6.4.1.1 | 8.6.4.1.2 | 8.6.4.1.3 | 8.6.4.1.4 | 8.6.4.2.1 | 8.6.4.2.2 | 8.6.4.2.3 | 8.6.4.2.4 |
| 8.6.4.3.1 | 8.6.4.3.2 | 8.6.4.3.3 | 8.6.4.3.4 | 8.6.4.4.1 | 8.6.4.4.2 | 8.6.4.4.3 | 8.6.4.4.4 |
| 8.6.4.5.1 | 8.6.4.5.2 | 8.6.4.5.3 | 8.6.4.5.4 | 8.6.4.6.1 | 8.6.4.6.2 | 8.6.4.6.3 | 8.6.4.6.4 |
| 8.6.4.7.1 | 8.6.4.7.2 | 8.6.4.7.3 | 8.6.4.7.4 | 8.6.4.8.1 | 8.6.4.8.2 | 8.6.4.8.3 | 8.6.4.8.4 |
| 8.7.1.1.1 | 8.7.1.1.2 | 8.7.1.1.3 | 8.7.1.1.4 | 8.7.1.2.1 | 8.7.1.2.2 | 8.7.1.2.3 | 8.7.1.2.4 |
| 8.7.1.3.1 | 8.7.1.3.2 | 8.7.1.3.3 | 8.7.1.3.4 | 8.7.1.4.1 | 8.7.1.4.2 | 8.7.1.4.3 | 8.7.1.4.4 |
| 8.7.1.5.1 | 8.7.1.5.2 | 8.7.1.5.3 | 8.7.1.5.4 | 8.7.1.6.1 | 8.7.1.6.2 | 8.7.1.6.3 | 8.7.1.6.4 |
| 8.7.1.7.1 | 8.7.1.7.2 | 8.7.1.7.3 | 8.7.1.7.4 | 8.7.1.8.1 | 8.7.1.8.2 | 8.7.1.8.3 | 8.7.1.8.4 |
| 8.7.2.1.1 | 8.7.2.1.2 | 8.7.2.1.3 | 8.7.2.1.4 | 8.7.2.2.1 | 8.7.2.2.2 | 8.7.2.2.3 | 8.7.2.2.4 |
| 8.7.2.3.1 | 8.7.2.3.2 | 8.7.2.3.3 | 8.7.2.3.4 | 8.7.2.4.1 | 8.7.2.4.2 | 8.7.2.4.3 | 8.7.2.4.4 |
| 8.7.2.5.1 | 8.7.2.5.2 | 8.7.2.5.3 | 8.7.2.5.4 | 8.7.2.6.1 | 8.7.2.6.2 | 8.7.2.6.3 | 8.7.2.6.4 |
| 8.7.2.7.1 | 8.7.2.7.2 | 8.7.2.7.3 | 8.7.2.7.4 | 8.7.2.8.1 | 8.7.2.8.2 | 8.7.2.8.3 | 8.7.2.8.4 |
| 8.7.3.1.1 | 8.7.3.1.2 | 8.7.3.1.3 | 8.7.3.1.4 | 8.7.3.2.1 | 8.7.3.2.2 | 8.7.3.2.3 | 8.7.3.2.4 |
| 8.7.3.3.1 | 8.7.3.3.2 | 8.7.3.3.3 | 8.7.3.3.4 | 8.7.3.4.1 | 8.7.3.4.2 | 8.7.3.4.3 | 8.7.3.4.4 |
| 8.7.3.5.1 | 8.7.3.5.2 | 8.7.3.5.3 | 8.7.3.5.4 | 8.7.3.6.1 | 8.7.3.6.2 | 8.7.3.6.3 | 8.7.3.6.4 |
| 8.7.3.7.1 | 8.7.3.7.2 | 8.7.3.7.3 | 8.7.3.7.4 | 8.7.3.8.1 | 8.7.3.8.2 | 8.7.3.8.3 | 8.7.3.8.4 |
| 8.7.4.1.1 | 8.7.4.1.2 | 8.7.4.1.3 | 8.7.4.1.4 | 8.7.4.2.1 | 8.7.4.2.2 | 8.7.4.2.3 | 8.7.4.2.4 |
| 8.7.4.3.1 | 8.7.4.3.2 | 8.7.4.3.3 | 8.7.4.3.4 | 8.7.4.4.1 | 8.7.4.4.2 | 8.7.4.4.3 | 8.7.4.4.4 |
| 8.7.4.5.1 | 8.7.4.5.2 | 8.7.4.5.3 | 8.7.4.5.4 | 8.7.4.6.1 | 8.7.4.6.2 | 8.7.4.6.3 | 8.7.4.6.4 |
| 8.7.4.7.1 | 8.7.4.7.2 | 8.7.4.7.3 | 8.7.4.7.4 | 8.7.4.8.1 | 8.7.4.8.2 | 8.7.4.8.3 | 8.7.4.8.4 |
| 8.8.1.1.1 | 8.8.1.1.2 | 8.8.1.1.3 | 8.8.1.1.4 | 8.8.1.2.1 | 8.8.1.2.2 | 8.8.1.2.3 | 8.8.1.2.4 |
| 8.9.1.3.1 | 8.8.1.3.2 | 8.8.1.3.3 | 8.8.1.3.4 | 8.8.1.4.1 | 8.8.1.4.2 | 8.8.1.4.3 | 8.8.1.4.4 |
| 8.8.1.5.1 | 8.8.1.5.2 | 8.8.1.5.3 | 8.8.1.5.4 | 8.8.1.6.1 | 8.8.1.6.2 | 8.8.1.6.3 | 8.8.1.6.4 |
| 8.8.1.7.1 | 8.8.1.7.2 | 8.8.1.7.3 | 8.8.1.7.4 | 8.8.1.8.1 | 8.8.1.8.2 | 8.8.1.8.3 | 8.8.1.8.4 |
| 8.8.2.1.1 | 8.8.2.1.2 | 8.8.2.1.3 | 8.8.2.1.4 | 8.8.2.2.1 | 8.8.2.2.2 | 8.8.2.2.3 | 8.8.2.2.4 |
| 8.8.2.3.1 | 8.8.2.3.2 | 8.8.2.3.3 | 8.8.2.3.4 | 8.8.2.4.1 | 8.8.2.4.2 | 8.8.2.4.3 | 8.8.2.4.4 |
| 8.8.2.5.1 | 8.8.2.5.2 | 8.8.2.5.3 | 8.8.2.5.4 | 8.8.2.6.1 | 8.8.2.6.2 | 8.8.2.6.3 | 8.8.2.6.4 |
| 8.8.2.7.1 | 8.8.2.7.2 | 8.8.2.7.3 | 8.8.2.7.4 | 8.8.2.8.1 | 8.8.2.8.2 | 8.8.2.8.3 | 8.8.2.8.4 |
| 8.8.3.1.1 | 8.8.3.1.2 | 8.8.3.1.3 | 8.8.3.1.4 | 8.8.3.2.1 | 8.8.3.2.2 | 8.8.3.2.3 | 8.8.3.2.4 |
| 8.8.3.3.1 | 8.8.3.3.2 | 8.8.3.3.3 | 8.8.3.3.4 | 8.8.3.4.1 | 8.8.3.4.2 | 8.8.3.4.3 | 8.8.3.4.4 |
| 8.8.3.5.1 | 8.8.3.5.2 | 8.8.3.5.3 | 8.8.3.5.4 | 8.8.3.6.1 | 8.8.3.6.2 | 8.8.3.6.3 | 8.8.3.6.4 |
| 8.8.3.7.1 | 8.8.3.7.2 | 8.8.3.7.3 | 8.8.3.7.4 | 8.8.3.8.1 | 8.8.3.8.2 | 8.8.3.8.3 | 8.8.3.8.4 |
| 8.8.4.1.1 | 8.8.4.1.2 | 8.8.4.1.3 | 8.8.4.1.4 | 8.8.4.2.1 | 8.8.4.2.2 | 8.8.4.2.3 | 8.8.4.2.4 |
| 8.8.4.3.1 | 8.8.4.3.2 | 8.8.4.3.3 | 8.8.4.3.4 | 8.8.4.4.1 | 8.8.4.4.2 | 8.8.4.4.3 | 8.8.4.4.4 |
| 8.8.4.5.1 | 8.8.4.5.2 | 8.8.4.5.3 | 8.8.4.5.4 | 8.8.4.6.1 | 8.8.4.6.2 | 8.8.4.6.3 | 8.8.4.6.4 |
| 8.8.4.7.1 | 8.8.4.7.2 | 8.8.4.7.3 | 8.8.4.7.4 | 8.8.4.8.1 | 8.8.4.8.2 | 8.8.4.8.3 | 8.8.4.8.4 |

The best mode of practicing the claimed invention is with compounds of Example numbers **48.6, 48.9, 48.15,** and **48.20.**

Preferred insulin sensitizers are compounds disclosed in the following publications and patents:
(1) Tamura et al. W09737688
(2) Nagao et al., Eur. Pat. Appl. EP-787727
(3) Kallam et al. Can. Pat. Appl. CA2173660 AA
(4) Inman et al. W09639401 A1
(5) Yanagisawa et al. W09638427
(6) Fujita et al., EP-745600 A1
(7) Ohara et al., W09626207 A1
(8) Ohara et al. W09611196 A1
(9) Malamas et al., US 5532256 A
(10) Yanagisawa et al., EP-708098 A1
(11) Regnier et al., US5478853 A
(12) US5468762 A
(13) Ohara et al., W0952637 A1
(14) Antonucci et al. US5457109 A
(15) Yoshioka et al. JP07002852 A2
(16) Shibata et al., W09401433 A1
(17) Fujita et al., EP-543662 A2
(18) De Nanteuil et al., EP-559571 A1
(19) Zask et al., US5236941 A
(20) Ohnota et al., W09214719
(21) Miyaoka et al., EP-489663 A1
(22) Arita et a]., EP-506273 A2
(23) Hulin et al., J. Med. Chem. 35, 1853 (1992)
(24) Zask et al., J. Med. Chem. 33: 1418-1423 (1990)
(25) Clark US4791125A
(26) Iijima et al., EP-283035 A1
(27) Kees et al., US4728739 A
(28) Meguro et al., EP-177353 A2
(29) Hasler et al., EP-129747 A2
(30) Kawamatsu et al., EP-91761 A2
(31) Tontonez et al. Gene & Develop 8: 1224-1234 (1994)
(32) Tontonez et al. Cell 79: 1147-1156
(33) Lehmann et al., J. Biol. Chem. 270, 1-4 (1994)
(34) Amri et al. J. Lipid Res. 32: 1449-1456 (1991)
(35) Grimaldi et al. Proc. Natl. Acad. Sci. USA 89: 10930 (1992)
(36) EP0745600

All references are incorporated by reference. While such disclosures constitute a large number of the insulin sensitizers, the instant invention is not so limited and can utilize any insulin sensitizer compound. The insulin sensitizers encompassed by the invention are compounds that improve insulin sensitivity as measured, for instance, by conducting standard assays such as those described in Examples H through M.

More preferred are the following insulin sensitizers:

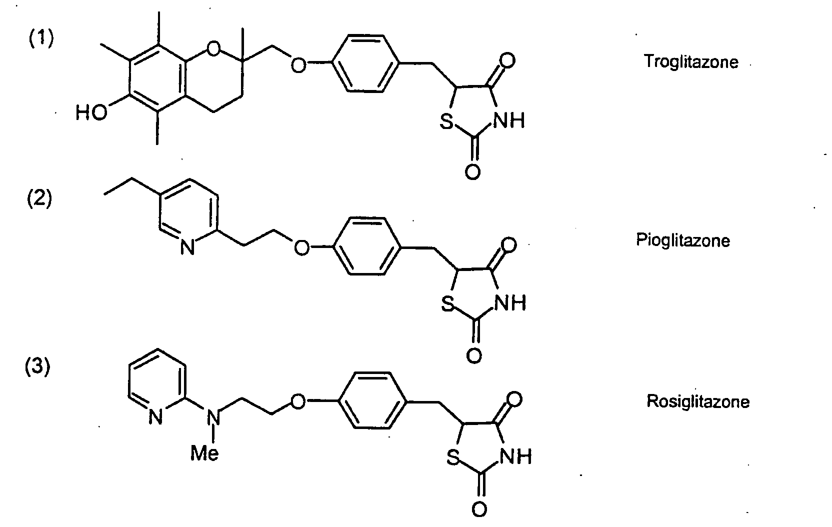

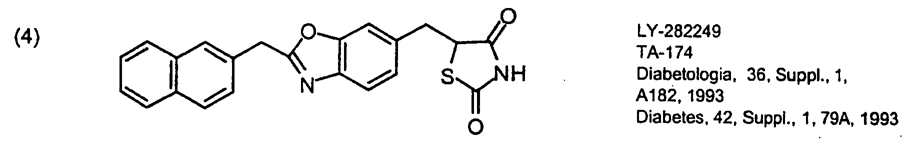

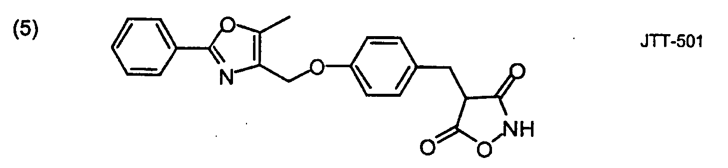

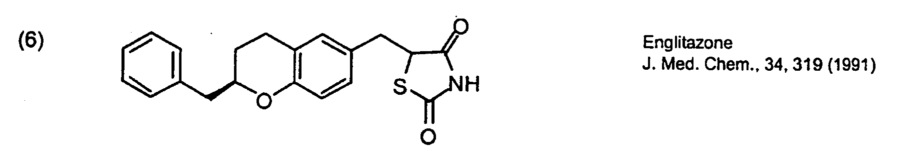

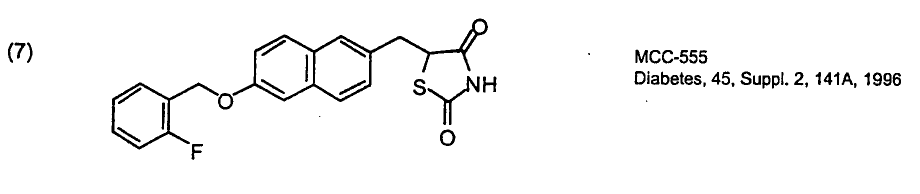

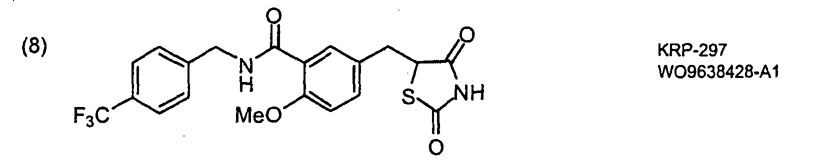

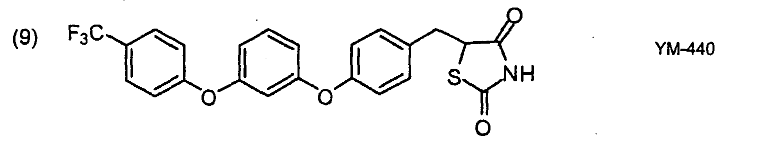

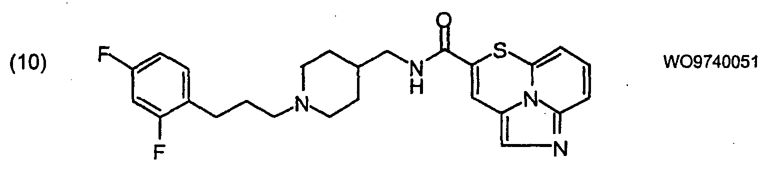

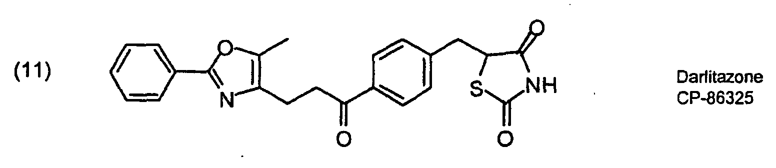

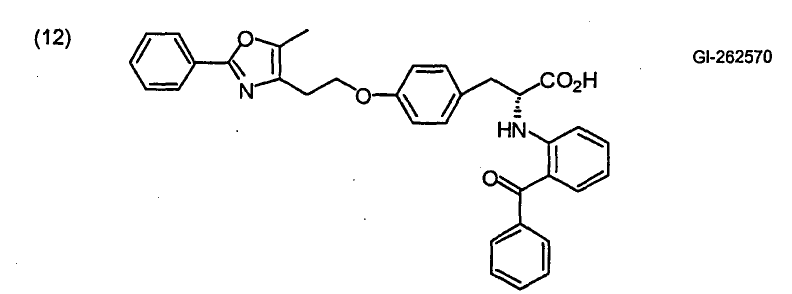

Especially preferred PPAR γ agonists are Troglitazone, Pioglitazone, ciglitazone, WAY-120,744, englitazone, AD 5075, SB219994, SB219993, BRL49653, G1-262570, darglitazone and analogs thereof.

Preferred RXR ligands are described in e.g. Heyman et al., WO9710819 A1; Especially preferred RXR ligands are LG100268, LGD 1069, ALRT 1057 and analogs thereof.

Other classes of insulin sensitizers are within the scope of the invention and include non-thiazolidinediones) such as SB 236636 and SB 219994, which are 3-aryl-2-alkoxy propanoic acids, PKC inhibitors, angiotensin II antagonists, and angiotensin converting enzyme inhibitors.

As expected from their mechanism of action, insulin-sensitizers are primarily effective in the hyperinsulinemic, early stages of type 2 diabetes. Efficacy is considerably reduced in advanced diabetes which is associated with severely disturbed beta-cell function and hence diminished insulin levels. This drug profile has been observed both in animal models of the disease as well as in the clinic. Hyperglycemia in young, hyperinsulinemic ZDF rats, for instance, is completely reversed by treatment with Troglitazone. Sreenan et al. Am. J. Physiol. 271: E742-747. ZDF rats in a more advanced, hypoinsulinemic phase of the disease, however, respond poorly to insulin sensitizer treatment. Brown et al. Diabetes 48: 1415-1424 (1999). In addition, hypoinsulinemic, streptozotocin induced diabetic mice do not respond to Troglitazone treatment. Fujiwara et al. Diabetes 37: 1549-1558 (1988). Clinical trials with Troglitazone have brought to light similarly variable responses in type 2 diabetics, with the non-response rate ranging from 35-62%. Valiquett T. et al. Diabetes 44 (Suppl.1): 406A (1995). In these trials it was found that the best predictor of efficacy was fasting insulin C peptide levels; the higher the C-peptide level, the greater the glucose-lowering effect in patients. Maggs DG et al. Ann. Intern. Med 128:176-185 (1998). Patients with sufficient pancreatic insulin secretory function thus respond well to therapy, whereas patients with decreased pancreatic function, characteristic of more advanced diabetes, respond poorly or are non-responders to therapy.

Insulin sensitizer treatment in general falls short of restoring euglycemia or normalizing HbAlc levels in,atients. In clinical trials with Pioglitazone, for example, average blood glucose lowering and HbAlc reductions were ~50 mg/dl and 0.6%, respectively. Mathisen et al. Diabetes 48 (Suppl.l): 441A (1998). In the patient populations treated, average reductions of >140 mg/dl and >3% would have been necessary to restore these parameters to normal values. A high rate of non-response and overall modest reductions in blood glucose levels have also been observed in clinical trials with Rosiglitazone. Patel et al. Diabetes, Obesity, and Metabolism 1: 165-172 (1999). There thus appears ample opportunity for agents such as the FBPase inhibitors to provide a benefit in combination with insulin sensitizers in the clinic.

FBPase inhibitors are likely to be efficacious both in early and advanced stages of type 2 diabetes. In animal studies they significantly lowered blood glucose in the hyperinsulinemic db/db mouse (a model of early type 2 diabetes, Examples S and T) as well as in a model of advanced type 2 diabetes: the insulinopenic streptozotocin-induced diabetic rat. In the ZDF rat, FBPase inhibitors were effective both in early diabetes (8-9 weeks of age, Examples N-R) as well as in advanced diabetes (16 weeks of age). Based on the pharmacological profile described above, the combination of FBPase inhibitors and insulin sensitizers will be effective across a broad patient population. In early stage diabetics, the insulin sensitizer and FBPase inhibitor are both likely to be fully effective, whereas in advanced diabetics, the response to insulin sensitizers may be partial whereas the FBPase inhibitor will maintain robust efficacy. The benefit of the combination in advanced diabetes will be a significant reduction in the number of non-responders to therapy (Example R). While the initial response of the combination may in large part be due to treatment with the FBPase inhibitor, blood glucose lowering may improve pancreatic function (Example P) and allow the insulin sensitizer to become more fully effective over time and in the long term provide enhanced glycemic control. In some cases, insulin sensitizers are best used in combination with agents that improve the actions of the insulin sensitizer, such as insulin, insulin analogs, RXR ligands, or insulin secretagogues (eg. the sulfonylureas). With the actions of the insulin sensitizer thus enhanced, combination treatment with an FBPase inhibitor will result in more effective glycemic control. Moreover, long term treatment will diminish the need for agents that enhance insulin levels.

FBPase inhibitors lower blood glucose both in the fasted (example T) and the fed state (examples N-S). This provides a broad opportunity for therapy in combination with insulin sensitizers. The combination.could, for instance, be administered at mealtime and provide enhanced glycemic control over either agent alone by simultaneously enhancing glucose disposal and reducing the contribution of gluconeogenesis to hyperglycemia during the postprandial period. Meal time administration has the additional benefit of reducing the potential risk of hypoglycemia that could ensue from treatment with an FBPase inhibitor. Another possible dosing regimen may be the administation of the insulin sensitizer during the daytime, and administration of the FBPase inhibitor separately at bedtime. The insulin sensitizer will thus provide glycemic control by enhancing glucose disposal following daytime meals, whereas the FBPase inhibitor will control excessive glucose production by the liver known to occur to a greater extent during the overnight fast. There is precedent for the use of a hepatic glucose production suppressor during the overnight fast; insulin has been widely used in this application. Riddle, The Lancet 192-195 (1985).

An additional benefit of combination treatment is that it will allow a reduction in dose of both agents thereby reducing potential side effects. The most common side-effect of Troglitazone, for example, is hepatotoxicity which manifests itself as the elevation of liver enzymes (1% of patients). This side-effect has resulted in a recommendation for liver function monitoring every month for the first six months of treatment. In addition, an association between Troglitazone and increased heart weight in animals has led to recommendations that this drug be used cautiously in patients with congestive heart failure. Rosiglitazone treatment, although not reported to cause liver enzyme elevations, is known to significantly decrease haematocrit. All insulin sensitizers cause weight gain. As discussed above, in some cases efficacy with an insulin sensitizer can only be achieved with supplemental insulin or sulfonylurea administrations. Insulin has the undesirable side-effects of promoting weight gain, of exacerbating insulin resistance, and predisposing to hypoglycemia. Sulfonylureas also promote weight gain, increase the risk of hypoglycemia, and by overstimulating the pancreas can promote beta-cell degeneration. In certain animal models, FBPase inhibitors are known to elevate blood lactate and triglycerides and could therefore predispose to systemic acidosis and the vascular complication associated with hypertriglyceridemia. Combination treatment with an insulin sensitizer may suppress the potential lactate and triglyceride elevations associated with FBPase inhibitor treatment (Examples O and Q). Other side effects of FBPase inhibitors may manifest themselves in man. By treating patients with the combination of an insulin sensitizer and an FBPase inhibitor, the reduced dosages feasible are likely to significantly decrease the risks of the (potential) side-effects associated with the individual therapies.

While an insulin sensitizer-FBPase inhibitor combination is primarily envisaged for the treatment of type 2 diabetes and the associated renal, neuronal, retinal, micro- and macro-vascular and metabolic complications, treatment of other diseases that respond to improved glycemic control and improved insulin sensitivity is also possible. Patients with impaired glucose tolerance (IGT) are minimally hyperglycemic under ordinary circumstances but can become hyperglycemic following the ingestion of large glucose loads. IGT is a predictor of future diabetes and patients with this condition have become the target of diabetes prevention trials in recent years. Combination treatment of these patients, particularly at mealtime, restores a normal glucose response and reduces the risk of the development of diabetes. Another distinct group of subjects at high risk for the development of type 2 diabetes are women who suffer from polycystic ovary syndrome (POCS). Combination treatment is of benefit in these patients as well since they are typically, hyperinsulinemic, insulin resistant, and can suffer from IGT. Combination treatment is also useful for treating renal dysfunction and hypertension particularly in obese, insulin resistant, hyperinsulinemic patients with IGT. Other applications of combination treatment include gestational diabetes, poorly controlled type 1 diabetes, obesity and dyslipidemia.

### Formulations

For the purposes of this invention, the compounds may be administered by a variety of means including orally, parenterally, by inhalation spray, topically, or rectally in formulations containing pharmaceutically acceptable carriers, adjuvants and vehicles. The term parenteral as used here includes subcutaneous, intravenous, intramuscular, and intraarterial injections with a variety of infusion techniques.

Intraarterial and intravenous injection as used herein includes administration through catheters. Oral administration is generally preferred.

Pharmaceutical compositions containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example calcium phosphate or kaolin, or as soft gelatin capsules wherein the active ingredient is mixed with water or an oil medium, such as peanut oil, liquid paraffin or olive oil.

Aqueous suspensions of the invention contain the active materials in admixture with excipients suitable for the manufacture of aqueous suspensions. Such excipients include a suspending agent, such as sodium carboxymethyl cellulose, methylcellulose, hydroxypropyl methylcelluose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

Oil suspensions may be formulated by suspending the active ingredient in a vegetable oil, such as arachis oil, olive oil, sesame oil or coconut oil, or in a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an antioxidaht such as ascorbic acid.

Dispersible powders and granules of the invention suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

The pharmaceutical compositions of the invention may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents.

Syrups and elixirs may be formulated with sweetening agents, such as glycerol, sorbitol or sucrose. Such formulations may also contain a demulcent, a preservative, a flavoring or a coloring agent.

The pharmaceutical compositions of the invention may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,3-butane-diol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.

Compounds of the invention may be administered as a daily dose or an appropriate fraction of the daily dose (*e.g*. bid). Administration of the FBPase inhibitor may occur at or near the time in which the insulin sensitizer active ingredient is administered or at a different time. Simultaneous administration of the active ingredients is achieved either by administration of the active ingredients in the same or different formulations. Formulations include time-release formulations intended to release either both of the active ingredients simultaneously or to stage the release of the active ingredients such that release, absorption and systemic exposure occurs with one of the ingredients before the other.

The amount of active ingredient that may be combined with the carrier material to produce a single dosage form will vary depending upon the host treated and the particular mode of administration. For example, a formulation intended for oral administration to humans may contain 20 to 2000 µmol (approximately 10 to 1000 mg) of active material compounded with an appropriate and convenient amount of carrier material which may vary from about 5 to about 95% of the total compositions.

As noted above, formulations of the present invention suitable for oral administration may be presented as discrete units such as capsules, cachets or tablets each containing a predetermined amount of the active ingredient; as a powder or granules; as a solution or a suspension in an aqueous or nonaqueous liquid; or as an oil-in-water liquid emulsion or a water in-oil liquid emulsion. The active ingredient may also be administered as a bolus, electuary or paste.

A tablet may be made by compression or molding, optionally with one or more accessory ingredients. Compressed tablets may be prepared by compressing in a suitable machine the active ingredient in a free flowing form such as a powder or granules, optionally mixed with a binder (e.g., povidone, gelatin, hydroxypropylmethyl cellulose), lubricant, inert diluent, preservative, disintegrant (e.g., sodium starch glycolate, cross-linked povidone, cross-linked sodium carboxymethyl cellulose) surface active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein using, for example, hydroxypropyl methylcellulose in varying proportions to provide the desired release profile. Tablets may optionally be provided with an enteric coating, to provide release in parts of the gut other than the stomach.

Formulations suitable for topical administration in the mouth include lozenges comprising the active ingredient in a flavored base, usually sucrose and acacia or tragacanth; pastilles comprising the active ingredient in an inert base such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, or an appropriate fraction thereof, a fructose-1,6-bisphosphatase inhibitor compound and an insulin sensitizer.

It will be understood, however, that the specific dose level for any particular patient will depend on a variety of factors including the activity of the specific compound employed; the age, body weight, general health, sex and diet of the individual being treated; the time and route of administration; the rate of excretion; other drugs which have previously been administered; and the severity of the particular disease undergoing therapy, as is well understood by those skilled in the art.

Capsules comprising FBPase inhibitors suitable for oral administration according to the methods of the present invention may be prepared as follows: (1) for a 10,000 capsule preparation: up to 5000 g of FBPase inhibitor is blended with other ingredients (as described above) and filled into capsules which are suitable for administration depending on dose, from about 1 capsules per day to about 8 capsules per day (2 capsules per 6 hours), to an adult human.

Capsules comprising insulin sensitizers suitable for oral administration according to the methods of the present invention may be prepared as follows: (1) for a 10,000 capsule preparation: up to 5000 g of insulin sensitizer is blended with other ingredients (as described above) and filled into capsules which are suitable for administration depending on dose, from about 1 capsules per day to about 8 capsules per day (2 capsules per 6 hours), to an adult human.

Capsules comprising FBPase inhibitors and insulin sensitizers suitable for oral administration according to the methods of the present invention may be prepared as follows: (1) for a 10,000 capsule preparation: up to 2500 g of FBPase inhibitor and up to 2500 g of insulin sensitizer are blended with other ingredients (as described above) and filled into capsules which are suitable for administration depending on dose, from about 1 capsules per day to about 8 capsules per day (2 capsules per 6 hours), to an adult human.

### EXAMPLES

Compounds of formula VI are prepared according to the literature procedures with modifications and additions well understood by those skilled in the art. In general, these compounds are synthesized by the method of Srivastava, J.Med. Chem. (1976). Other methodology is described by Wood et al. J. Med. Chem. 28: 1198-1203 (1985); Sagi et al., J. Med. Chem.35: 4549-4556 (1992); Paul, Jr. J. Med. Chem. 28: 1704-1716 (1985); Cohen et al., J. Am. Chem. Soc. 95: 4619-4624 (1973).

Compounds of formulae II-IV are prepared according to the procedures described in PCT publication numbers WO 98/39344, WO 98/39343, and WO 98/39342.

### Section 1.

### Synthesis of Compounds of Formula I

Synthesis of compounds encompassed by the present invention typically includes some or all of the following general steps: (1) preparation of a phosphonate prodrug; (2) deprotection of a phosphonate ester; (3) modification of a heterocycle; (4) coupling of a heterocycle with a phosphonate component; (5) construction of a heterocycle; (6) ring closure to construct a heterocycle with a phosphonate moiety present and (7) preparation of useful intermediates. These steps are illustrated in the following scheme for compounds of formula 2 wherein R⁵ is a 5-membered heteroaromatic ring. Compounds of formula 2 wherein R⁵ is a 6-member heteroaromatic ring or other heteroaromatic rings are prepared in an analogous manner.

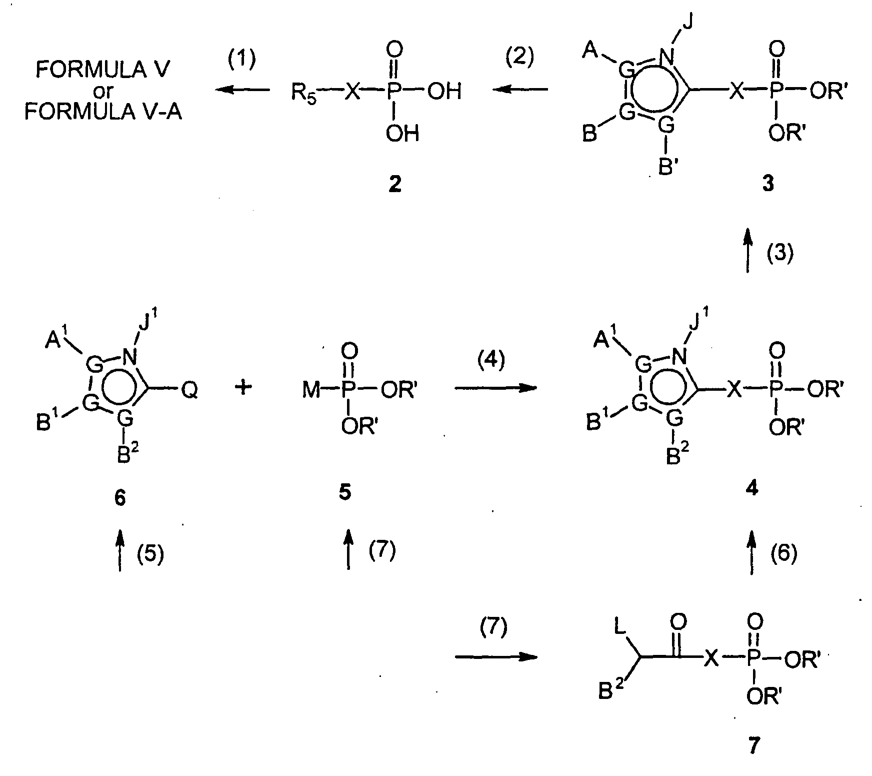

### (1a) Preparation of a phosphonate prodrug

Prodrugs can be introduced at different stages of the synthesis. Most often these prodrugs are made from the phosphonic acids of formula 2, because of their lability. Advantageously, these prodrugs can be introduced at an earlier stage, provided that it can withstand the reaction conditions of the subsequent steps.

Compounds of formula 2, can be alkylated with electrophiles (such as alkyl halides, alkyl sulfonates, etc) under nucleophilic substitution reaction conditions to give phosphonate esters. For example, compounds of formula I, wherein R¹ is an acyloxyalkyl group can be synthesized through direct alkylation of compounds of formula 2 with an appropriate acyloxyalkyl halide (e.g. Cl, Br, I; Elhaddadi, et al *Phosphorus Sulfur*, **1990**, *54(1-4)*: 143; Hoffmann, *Synthesis*, **1988**, 62) in the presence of a base (e.g. *N, N'*-dicyclohexyl-4-morpholinecarboxamidine, Hunigs base, etc.) in suitable solvents such as 1,1-dimethyl formamide ("DMF") (Starrett, et al, *J. Med. Chem*., **1994**, 1857). The carboxylate component of these acyloxyalkyl halides includes but is not limited to acetate, propionate, isobutyrate, pivalate, benzoate, and other carboxylates. When appropriate, further modification are envisioned after the formation of these acyloxyalkyl phosphonate esters such as reduction of a nitro group. For example, compounds of formula 3 wherein A is a NO₂ group can be converted to compounds of formula 3 wherein A is an H₂N- group under suitable reduction conditions (Dickson, et al, *J. Med. Chem*., **1996**, *39:* 661; Iyer, et al, *Tetrahedron Lett.,* **1989**, *30*:7141; Srivastva, et al, *Bioorg. Chem*., **1984**, *12:* 118). These methods can be extended to the synthesis of other types of prodrugs, such as compounds of formula I where R¹ is a 3-phthalidyl, a 2-oxo-4,5-didehydro-1,3-dioxolanemethyl, or a 2-oxotetrahydrofuran-5-yl group (Biller et al., US S,157,027; Serafinowska et al., *J. Med. Chem.* **1995,***38:* 1372; Starrett et al., *J. Med Chem.* **1994,** *37:* 1857; Martin et al., *J. Pharm. Sci.* **1987,** *76:* 180; Alexander et al., *Collect. Czech. Chem. Commun,* **1994,** *59:* 1853; EPO 0632048A1). NN-Dimethylformamide dialkyl acetals can also be used to alkylate phosphonic acids (Alexander, P., et al *Collect. Czech. Chem. Commun*., **1994,** *59,* 1853).

Alternatively, these phosphonate prodrugs can also be synthesized by reactions of the corresponding dichlorophosphonates with an alcohol (Alexander et al, *Collect. Czech. Chem. Commun*., **1994,** *59:* 1853). For example, reactions of a dichlorophosphonate with substituted phenols and aralkyl alcohols in the presence of base (e.g. pyridine, triethylamine, etc) yield compounds of formula V where R¹ is an aryl group (Khamnei et al., *J. Med Chem*., **1996,** *39:* 4109; Serafinowska et al., *J. Med Chem*., **1995**, *38*: 1372; De Lombaert et al., *J. Med. Chem.,* **1994,** *37:* 498) or an arylalkyl group (Mitchell et al., *J. Chem. Soc. Perkin Trans. 1, 1992, 38:* 2345). The disulfide-containing prodrugs (Puech et al., *Antiviral Res.,* **1993,** 22: 155) can also be prepared from a dichlorophosphonate and 2-hydroxyethyl disulfide under standard conditions.

Such reactive dichlorophosphonates can be generated from the corresponding phosphonic acids with a chlorinating agent (e.g. thionyl chloride: Starrett et al., *J. Med Chem*., **1994**, 1857, oxalyl chloride: Stowell et al., *Tetrahedron Lett*., **1990**, *31*: 3261, and phosphorus pentachloride: Quast et al., *Synthesis*, **1974**, 490). Alternatively, a dichlorophosphonate can also be generated from its corresponding disilyl phosphonate esters (Bhongle et al., *Synth. Commun*., **1987**, *17*: 1071) or dialkyl phosphonate esters (Still et al., *Tetrahedron Lett*., **1983**, *24*: 4405; Patois et al., *Bull. Soc. Chim. Fr*., **1993**, *130*: 485).

Furthermore, these prodrugs can be prepared using Mitsunobu reactions (Mitsunobu, *Synthesis,* **1981,** 1; Campbell, *J. Org.Chem.,* **1992,** *52:* 6331), and other coupling reactions (e.g. using carbodiimides: Alexander et al., *Collect. Czech. Chem. Commun.,* **1994,** *59:* 1853; Casara et al., *Bioorg. Med. Chem. Lett*., **1992***, 2*: 145; Ohashi et al., *Tetrahedron Lett*., **1988,** *29***:** 1189, and benzotriazolyloxytris-(dimethylamino)phosphonium salts: Campagne et al., *Tetrahedron Lett*., **1993**, *34*: 6743). Compounds of formula I wherein R¹ is a cyclic carbonate, a lactone or a phthalidyl group can also be synthesized via direct alkylation of the free phosphonic acid with appropriate halides in the presence of a suitable base (e.g. NaH or diisopropylethylamine, Biller et al., US 5,157,027; Serafinowska et al., *J. Med. Chem*. **1995,** *38:* 1372; Starrett et al., *J. Med. Chem.* **1994,** *37*: 1857; Martin et al., *J. Pharm. Sci.* **1987,** *76:* 180; Alexander et al., *Collect. Czech. Chem. Commun*, **1994,** *59:* 1853;EPO 0632048A1).

R¹ can also be introduced at an early stage of the synthesis provided that it is compatible with the subsequent reaction steps. For example, compounds of formula I where R¹ is an aryl group can be prepared by metalation of a 2-furanyl heterocycle (e.g. using LDA) followed by trapping the anion with a diaryl chlorophosphate.

It is envisioned that compounds of formula V can be mixed phosphonate esters (e.g. phenyl and benzyl esters, or phenyl and acyloxyalkyl esters) including the chemically combined mixed esters such as the phenyl and benzyl combined prodrugs reported by Meier, et al. *Bioorg. Med Chem. Lett.,* **1997**, *7*: 99.

### (lb) Preparation of a bisamidate phosphonate

### General synthesis of bis-phosphoroamidate prodrugs:

In general, the bis-phosphoroamidates of formula I, where both -NR¹⁵R¹⁶ and -N(R¹⁸)-(CR¹²R¹³)ₙ-C(O)-R¹⁴ are from the same amino acid residues can be prepared from the activated phosphonates for example, dichlorophosphonate, by coupling with an aminoacid ester for example, glycine ethylester with or without base for example, N-methylimidazole. The reactive dichloridates, can be prepared as described above in the general prodrug section

Alternatively, these bis-phosphoroamidates can be prepared by reacting the corresponding phosphonic acid with an aminoacid ester for example, glycine ethylester in presence of PPh₃ and 2,2'-dipyridyl disulfide in pyridine as described in WO 95/07920 or Mukaiyama, T. et al, *J Am. Chem. Soc*., **1972**, *94*, 8528.

Synthesis of mixed bis-phosphoroamidates of formula IA, where -NR¹⁵R¹⁶ and -N(R¹⁸)-(CR¹²R¹³)ₙC(O)-R¹⁴ are different aminoacid esters or a combination of an aminoacid ester and a substituted amine can be prepared by direct conversion via dichloridate as described above (sequential addition) followed by separation of the required product by column chromatography or HPLC. Alternatively, these mixed bis-phosphoroamidates can be prepared starting with an appropriate phosphonate monoester such as phenyl ester or benzyl ester to give the mixed phosphonoesteramide via the chloridate, followed by ester hydrolysis under conditions where the amide bond is stable. The resultant mono-amide can be converted to a mixed bis-amide by condensation with a second amino ester or a substituted amine via the chloridate, as described above. Synthesis of such monoesters can be prepared using the reported procedure (EP 481 214).

The substituted cyclic propyl phosphonate esters can be synthesized by reactions of the corresponding dichlorophosphonate with a substituted 1,3-propanediol. Some of the methods useful for the preparation of a substituted 1,3-propanediol are discussed below.

### Synthesis of a 1,3-propanediol

Various synthetic methods can be used to prepare numerous types of 1,3-propanediols: (i) 1-substituted, (ii) 2-substituted, (iii) 1,2- or 1,3-annulated 1,3-propanediols. Substituents on the prodrug moiety of compounds of formula I (i.e. substituents on the 1,3-propanediol moiety) can be introduced or modified either during the synthesis of these diols or after the coupling of these diols to compounds of formula 2.

### (i) 1-Substituted 1,3-propanediols

1,3-Propanediols useful in the synthesis of compounds in the present invention can be prepared using various synthetic methods. Additions of a aryl Grignard to a 1-hydroxy-propan-3-al give 1-aryl-substituted 1,3-propanediols (path a). This method is suitable for the conversion of various aryl halides to 1-arylsubstituted-1,3-propanediols (Coppi et. al., *J. Org. Chem*., **1988**, *53*, 911). Conversions of aryl halides to 1-substituted 1,3-propanediols can also be achieved using Heck reactions (e.g. couplings with a 1,3-diox-4-ene) followed by reductions and subsequent hydrolysis reactions (Sakamoto et. al., *Tetrahedron Lett*., **1992**, *33*, 6845). Various aromatic aldehydes can also be converted to 1-substituted-1,3-propanediols using alkenyl Grignard addition reactions followed by hydroboration reactions (path b). Additions of a metallated t-butyl acetate to aromatic aldehydes followed by reduction of the ester (path e) are also useful for the synthesis of 1,3-propanediols (Turner., *J. Org. Chem*., **1990**, *55* 4744). In another method, epoxidations of cinnamyl alcohols using known methods (e.g. Sharpless epoxidations and other asymmetric epoxidation reactions) followed by a reduction reaction (e.g. using Red-Al) give various 1,3-propanediols (path c). Alternatively, enantiomerically pure 1,3-propanediols can be obtained using chiral borane reduction reactions of hydroxyethyl aryl ketone derivatives (Ramachandran et. al., *Tetrahedron Lett.,* **1997**, *38* 761). Propan-3-ols with a 1-heteroaryl substituent (e.g. a pyridyl, a quinolinyl or an isoquinolinyl) can be oxygenated to give 1-substituted 1,3-propanediols using N-oxide formation reactions followed by a rearrangement reaction in acetic anhydride conditions (path d) (Yamamoto et. al., *Tetrahedron ,* **1981,** *37*, 1871).

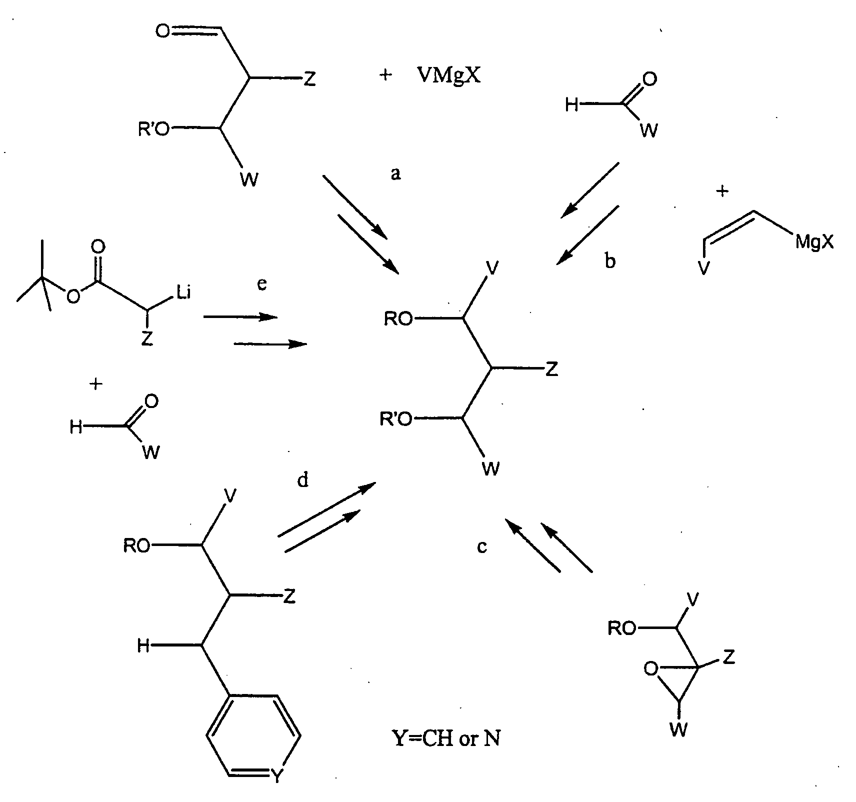

### (ii) 2-Substituted 1,3-propanediols

A variety of 2-substituted 1,3-propanediols useful for the synthesis of compounds of formula I can be prepared from 2-(hydroxymethyl)-1,3-propanediols using known chemistry (Larock, *Comprehensive Organic Transformations,* VCH, New York, **1989**). For example, reductions of a trialkoxycarbonylmethane under known conditions give a triol via complete reduction (path a) or a bis(hydroxymethyl)acetic acid via selective hydrolysis of one of the ester groups followed by reduction of the remaining two other ester groups. Nitrotriols are also known to give triols via reductive elimination (path b) (Latour et. al., *Synthesis,* **1987,** *8,* 742). Furthermore, a 2-(hydroxymethyl)-1,3-propanediol can be converted to a mono acylated derivative (e.g. acetyl, methoxycarbonyl) using an acyl chloride or an alkyl chloroformate (e.g. acetyl chloride or methyl chloroformate) (path d) using known chemistry (Greene et al., *Protective Groups In Organic Synthesis* ; Wiley, New York, **1990).** Other functional group manipulations can also be used to prepare 1,3-propanediols such as oxidation of one the hydroxylmethyl groups in a 2-(hydroxymethyl)-1,3-propanediol to an aldehyde followed by addition reactions with an aryl Grignard (path c). Aldehydes can also be converted to alkyl amines via reductive amination reactions (path e).

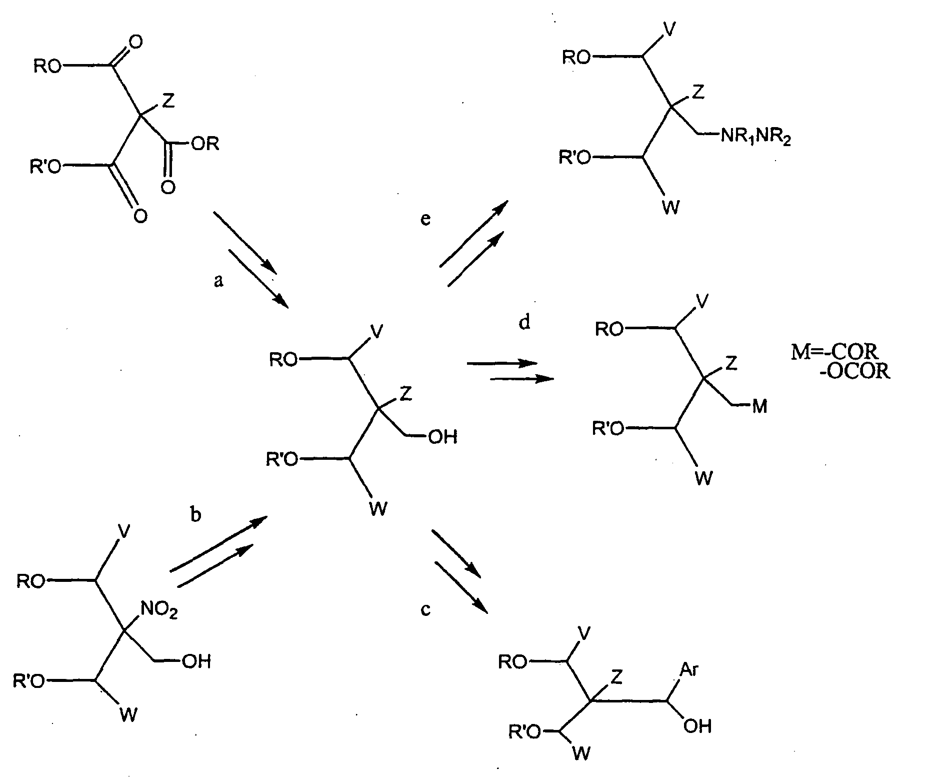

### (iii) Annulated 1,3-pronane diols

Compounds of formula I wherein V and Z or V and W are connected by four carbons to form a ring can be prepared from a 1,3-cyclohexanediol. For example, *cis, cis*-1,3,5-cyclohexanetriol can be modified as described for 2-substituted 1,3-propanediols. It is envisioned that these modifications can be performed either before or after formation of a cyclic phosphonate 1,3-propanediol ester. Various 1,3-cyclohexanediols can also be prepared using Diels-Alder reactions (e.g. using a pyrone as the diene: Posner et. al., *Tetrahedron Lett*., **1991**, *32*, 5295). 1,3-Cyclohexanediol derivatives are also prepared via other cycloaddition reaction methodologies. For example, cycloadditon of a nitrile oxide to an olefin followed by conversion of the resulting cycloadduct to a 2-ketoethanol derivative can be converted to a 1,3-cylohexanediol using known chemistry (Curran, et. al., *J. Am. Chem. Soc.,* **1985,** *107,* 6023). Alternatively, precursors to 1,3-cyclohexanediol can be made from quinic acid (Rao, et. al., *Tetrahedron Lett.,* **1991**, *32*, 547.)

### 2) Deprotection of a phosphonate ester

Compounds of formula I wherein R¹ is H may be prepared from phosphonate esters using known phosphate and phosphonate ester cleavage conditions. Silyl halides are generally used to cleave various phosphonate esters, and subsequent mild hydrolysis of the resulting silyl phosphonate esters give the desired phosphonic acids. When required, acid scavengers (e.g. 1,1,1,3,3,3-hexamethyldisilazane, 2,6-lutidine, etc.) can be used for the synthesis of acid labile compounds. Such silyl halides include chlorotrimethylsilane (Rabinowitz, *J. Org. Chem*., **1963,** *28:* 2975), and bromotrimethylsilane (McKenna, et al, *Tetrahedron Lett.,* **1977**, 155), and iodotrimethylsilane (Blackburn, et al, *J. Chem. Soc., Chem. Commun.,1978,* 870). Alternately, phosphonate esters can be cleaved under strong acidic conditions (e.g. HBr or HCI : Moffatt, et al, *U.S. Patent 3,524,846,* **1970)**. These esters can also be cleaved via dichlorophosphonates, prepared by treating the esters with halogenating agents (e.g. phosphorus pentachloride, thionyl chloride, BBr₃ : Pelchowicz et al, *J. Chem. Soc*., **1961**, 238) followed by aqueous hydrolysis to give phosphonic acids. Aryl and benzyl phosphonate esters can be cleaved under hydrogenolysis conditions (Lejczak, et al, *Synthesis,* **1982,** 412; Elliott, et al, *J. Med. Chem.,* **1985,** *28:* 1208; Baddiley, et al, *Nature,* **1953**, *171:* 76) or metal reduction conditions (Shafer, et al, *J. Am. Chem. Soc*., **1977,** *99:* 5118). Electrochemical (Shono, et al, *J. Org. Chem.,* **1979,** *44:* 4508) and pyrolysis (Gupta, et al, *Synth. Commun.,* **1980,** *10*: 299) conditions have also been used to cleave various phosphonate esters.

### (3) Modification of an existing heterocycle

Syntheses of the heterocycles encompassed in the disclosed compounds have been well studied and described in numerous reviews (see section 4). Although it is advantageous to have the desired substituents present in these heterocycles before synthesis of compounds of formula 4, in some cases, the desired substituents are not compatible with subsequent reactions, and therefore modifications of an existing heterocycle are required late in the synthetic scheme using conventional chemistry (Larock, *Comprehensive organic transformations,* VCH, New York, **1989;** Trost, *Comprehensive organic synthesis;* Pergamon press, New York, **1991).** For example, compounds of formula I wherein A, A", or B is a halo or a cyano group can be prepared from the corresponding amine group by conversion to the diazonium group and reaction with various copper (I) salts (e.g. CuI, CuBr, CuCl, CuCN). Halogens can also be introduced by direct halogenations of various heterocycles. For example, 5-unsubstituted-2-aminothiazoles can be converted to 2-amino-5-halothiazoles using various reagents (e.g. NIS, NBS, NCS). Heteroaryl halides are also useful intermediates and are often readily converted to other substituents (such as A, A", B, B", C", D, D", E and E") via transition metal assisted coupling reactions such as Suzuki, Heck or Stille reactions (Farina et al, *Organic Reactions, Vol. 50;* Wiley, New York, **1997;** Mitchell, *Synthesis*, **1992**, 808; Suzuki, *Pure App. Chem.,* **1991**, *63*, 419; Heck *Palladium Reagents in Organic Synthesis;* Academic Press: San Diego, **1985).** Compounds of formula I wherein A is a carbamoyl group can be made from their corresponding alkyl carboxylate esters via aminolysis with various amines, and conventional functional group modifications of the alkyl carboxylate esters are useful for syntheses of compounds of formula I wherein A is a -CH₂OH group or a -CH₂-halo group. Substitution reactions of haloheterocycles (e.g. 2-bromothiazole, 5-bromothiazole) with various nucleophiles (e.g. HSMe, HOMe, etc.) represents still another method for introducing substituents such as A, A", B and B". For example, substitution of a 2-chlorothiazole with methanethiol gives the corresponding 2-methylthiothiazole.

It is envisioned that when necessary alkylation of nitrogen atoms in the heterocycles (e.g. imidazoles, 1,2,4-triazoles and 1,2,3,4-tetrazoles) can be readily performed using for example standard alkylation reactions (with an alkyl halide, an-aralkyl halide, an alkyl sulfonate or an aralkyl sulfonate), or Mitsunobu reactions (with an alcohol).

### (4) Coupling of a heterocycle with a phosphonate component

When feasible compounds disclosed in the present invention are advantageously prepared via a convergent synthetic route entailing the coupling of a heterocycle with a phosphonate diester component.

Transition metal catalyzed coupling reactions such as Stille or Suzuki reactions are particularly suited for the synthesis of compounds of formula I. Coupling reactions between a heteroaryl halide or triflate (e.g. 2-bromopyridine) and a M-PO₃R' wherein M is a 2-(5-tributylstannyl)furanyl or a 2-(5-boronyl)furanyl group under palladium catalyzed reaction conditions (Farina et al, *Organic Reactions, Vol. 50;* Wiley, New York,**1997**; Mitchell, *Synthesis,* **1992**, 808; Suzuki, *Pure App. Chem.,* **1991,** *63,* 419) yield compounds of formula I wherein X is a furan-2,5-diyl group. It is envisioned that the nature of the coupling partners for these reactions can also be reversed (e.g. coupling of trialkylstannyl or boronyl heterocycles with a halo-X-P(O)(O-alkyl)₂). Other coupling reactions between organostannes and an alkenyl halide or an alkenyl triflate are also reported which may be used to prepared compounds of formula I wherein X is an alkenyl group. The Heck reaction may be used to prepare compounds of formula V wherein X is an alkynyl group (Heck *Palladium Reagents in Organic Synthesis;* Academic Press: San Diego, **1985).** These reactions are particularly suited for syntheses of various heteroaromatics as R⁵ for compounds of formula I given the availability of numerous halogenated heterocycles, and these reactions are particularly suitable for parallel synthesis (e.g. combinatorial synthesis on solid phase(Bunin, B. A., *The Combinatorial Index,;* Academic press: San Diego, 1998) or in solution phase (Flynn, D. L. et al., *Curr. Op. Drug. Disc. Dev.,* **1998**, *1,* 1367)) to generate large combinatorial libraries. For example, ethyl 5-iodo-2-furanylphosphonate can be coupled to Wang's resin under suitable coupling reaction conditions. The resin-coupled 5-iodo-2-[5-(O-ethyl-O-Wang's resin)phosphono]furan can then be subjected to transition metal catalyzed Suzuki and Stille reactions (as described above) with organoboranes and organotins in a parallel manner to give libraries of compounds of formula 3 wherein X is furan-2,5-diyl.

Substitution reactions are useful for the coupling of a heterocycle with a phosphonate diester component. For example, cyanuric chloride can be substituted with dialkyl mercaptoalkylphosphonates or dialkyl aminoalkylphosphonates to give compounds of formula 2 wherein R⁵ is a 1,3,5-triazine, X is an alkylthio or an alkylamino group. Alkylation reactions are also used for the coupling of a heterocycle with a phosphonate diester component. For example, a heteroaromatic thiol (e.g. a 1,3,4-thiadiazole-2-thiol) can be alkylated with a dialkyl methylphosphonate derivative (e.g. ICH₂P(O)(OEt)₂, TsOCH₂P(O)(OEt)₂, TfOCH₂P(O)(OEt)₂) to lead to compounds of formula I wherein X is an alkylthio group. In another aspect, alkylation reactions of a heteroaromatic carboxylic acid (e.g. a thiazole-4-carboxylic acid) with a dialkyl methylphosphonate derivative (e.g. ICH₂P(O)(OEt)₂, TsOCH₂P(O)(OEt)₂, TfOCH₂P(O)(OEt)₂) lead to compounds of formula I wherein X is an alkoxycarbonyl group, while alkylation reactions of a heteroaromatic thiocarboxylic acid (e.g. a thiazole-4-thiocarboxylic acid) with a dialkyl methylphosphonate derivative (e.g. ICH₂P(O)(OEt)₂, TsOCH₂P(O)(OEt)₂, TfOCH₂P(O)(OEt)₂) lead to compounds of formula I wherein X is an alkylthiocarbonyl group. Substitutions of haloalkyl heterocycles (e.g. 4-haloalkylthiazole) with nucleophiles containing the phosphonate group (diethyl hydroxymethylphosphonate) are useful for the preparation of compounds of formula I wherein X is an alkoxyalkyl or an alkylthioalkyl group. For example, compounds of formula I where X is a -CH₂OCH₂- group can be prepared from 2-chloromethylpyridine or 4-chloromethylthiazole using dialkyl hydroxymethylphosphonates and a suitable base (e.g. sodium hydride). It is possible to reverse the nature of the nucleophiles and electrophiles for the substitution reactions, i.e. haloalkyl- and/or sulfonylalkylphosphonate esters can be substituted with heterocycles containing a nucleophile (e.g. a 2-hydroxyalkylpyridine, a 2-mercaptoalkylpyridine, or a 4-hydroxyalkyloxazole).

Known amide bond formation reactions (e.g. the acyl halide method, the mixed anhydride method, the carbodiimide method) can also be used to couple a heteroaromatic carboxylic acid with a phosphonate diester component leading to compounds of formula 4 wherein X is an alkylaminocarbonyl or an alkoxycarbonyl group. For example, couplings of a thiazole-4-carboxylic acid with a dialkyl aminoalkylphosphonate or a dialkyl hydroxyalkylphosphonate give compounds of formula 4 wherein R⁵ is a thiazole, and X is an alkylaminocarbonyl or an alkoxycarbonyl group. Alternatively, the nature of the coupling partners can be reversed to give compounds of formula 4 wherein X is an alkylcarbonylamino group. For example, 2-aminothiazoles can be coupled with (RO)₂P(O)-alkyl-CO₂H (e.g. diethylphosphonoacetic acid) under these reaction conditions to give compounds of formula 4 wherein R⁵ is a thiazole and X is an alkylcarbonylamino group. These reactions are also useful for parallel synthesis of compound libraries through combinatorial chemistry on solid phase or in solution phase. For example, HOCH₂P(O)(OEt)(O-resin), H₂NCH₂P(O)(OEt)(O-resin) and HOOCCH₂P(O)(OEt)(O-resin) (prepared using known methods) can be coupled to various heterocycles using the above described reactions to give libraries of compounds of formula 3 wherein X is a - C(O)OCH₂-, or a -C(O)NHCH₂-, or a -NHC(O)CH₂-.

Rearrangement reactions can also be used to prepare compounds covered in the present invention. For example, the Curtius's rearrangement of a thiazole-4-carboxylic acid in the presence of a dialkyl hydroxyalkylphosphonate or a dialkyl aminoalkylphosphonate lead to compounds of formula 4 wherein X is an alkylaminocarbonylamino or an alkoxycarbonylamino group. These reactions can also be adopted for combinatorial synthesis of various libraries of compounds of formula 3. For example, Curtius's rearrangement reactions between a heterocyclic carboxylic acid and HOCH₂P(O)(OEt)(O-resin), or H₂NCH₂P(O)(OEt)(O-resin) can lead to libraries of compounds of formula I wherein X is a -NHC(O)OCH₂-, or a -NHC(O)NHCH₂-.

For compounds of formula V wherein X is an alkyl group, the phosphonate group can be introduced using other common phosphonate formation methods such as Michaelis-Arbuzov reaction (Bhattacharya et al., *Chem. Rev.,* **1981,** *81*: 415), Michaelis-Becker reaction (Blackburn et al., *J. Organomet. Chem*., **1988**, *348*: 55), and addition reactions of phosphorus to electrophiles (such as aldehydes, ketones, acyl halides, imines and other carbonyl derivatives).

Phosphonate component can also be introduced via lithiation reactions. For example, lithiation of an 2-ethynylpyridine using a suitable base followed by trapping the thus generated anion with a dialkyl chlorophosphonate lead to compounds of formula 3 wherein R5 is a pyridyl, X is a 1-(2-phosphono)ethynyl group.

### (5) Construction of a heterocycle

Although existing heterocycles are useful for the synthesis of compounds of formula V, when required, heterocycles can also be constructed leading to compounds in the current invention, and in some cases may be preferred for the preparations of certain compounds. The construction of heterocycles have been well described in the literature using a variety of reaction conditions (Joule et al., *Heterocyclic Chemistry*; Chapman hall, London, **1995**; Boger, Weinreb, *Hetero Diels-Alder Methodology In Organic Synthesis*; Academic press, San Diego, **1987**; Padwa, *1,3-Dipolar Cycloaddition Chemistry*; Wiley, New York, **1984**; Katritzsky et al., *Comprehensive Heterocyclic Chemistry*; Pergamon press, Oxford; Newkome et al., *Contemporary Heterocyclic Chemistry: Syntheses, Reaction and Applications*; Wiley, New York, **1982**; *Syntheses of Heterocyclic Compounds*; Consultants Bureau, New York). Some of the methods which are useful to prepare compounds in the present invention are given as examples in the following discussion.

### (i) Construction of a thiazole ring system

Thiazoles useful for the present invention can be readily prepared using a variety of well described ring-forming reactions (Metzger, *Thiazole and its derivatives, part 1 and part 2*; Wiley & Sons, New York, **1979**). Cyclization reactions of thioamides (e.g. thioacetamide, thiourea) and alpha-halocarbonyl compounds (such as alpha-haloketones, alpha-haloaldehydes) are particularly useful for the construction of a thiazole ring system. For example, cyclization reactions between thiourea and 5-diethylphosphono-2-[(-2-bromo-l-oxo)alkyl]furans are useful for the synthesis of compounds of formula 2 wherein R⁵ is a thiazole, A is an amino group and X is a furan-2,5-diyl group; cyclization reaction between thiourea and a bromopyruvate alkyl ester give a 2-amino-4-alkoxycarbonylthiazole which is useful for the preparations of compounds of formula 2 wherein R5 is a thiazole and X is an alkylaminocarbonyl, an alkoxycarbonyl, an alkylaminocarbonylamino, or an alkoxyacarbonylamino group. Thioamides can be prepared using reactions reported in the literature (Trost, *Comprehensive organic synthesis, Vol. 6*, ; Pergamon press, New York, **1991**, pages 419 - 434) and alpha-halocarbonyl compounds are readily accessible via conventional reactions (Larock, *Comprehensive organic transformations*, VCH, New York, **1989**). For example, amides can be converted to thioamides using Lawesson's reagent or P₂S₅, and ketones can be halogenated using various halogenating reagents (e.g. NBS, CuBr₂).

### (ii) Construction of an oxazole ring system

Oxazoles useful for the present invention can be prepared using various methods in the literature (Turchi, *Oxazoles;* Wiley & Sons, New York, **1986).** Reactions between isocyanides (e.g. tosylmethylisocyanide) and carbonyl compounds (e.g. aldehydes and acyl chlorides) can be used to construct oxazole ring systems (van Leusen et al, *Tetrahedron Lett.,* **1972**, 2369). Alternatively, cyclization reactions of amides (e.g. urea, carboxamides) and alpha-halocarbonyl compounds are commonly used for the construction of an oxazole ring system. For example, the reactions of urea and 5-diethylphosphono-2-[(-2-bromo-1-oxo)alkyl]furans are useful for the synthesis of compounds of formula 2 wherein R⁵ is an oxazole, A is an amino group and X is a furan-2,5-diyl group. Reactions between amines and imidates are also used to construct the oxazole ring system (Meyers et al, *J. Org. Chem.,* **1986,** *51 (26),* 5111).

### (iii) Construction of a pyridine ring system

Pyridines useful for the synthesis of compounds of formula I can be prepared using various known synthetic methods (Klingsberg, Pyridine and Its Derivatives; Interscience Publishers, New York, **1960 - 1984).** 1,5-Dicarbonyl compounds or their equivalents can be reacted with ammonia or compounds which can generate ammonia to produce 1,4-dihydropyridines which are easily dehydrogenated to pyridines. When unsaturated 1,5-dicarbonyl compounds, or their equivalents (e.g. pyrylium ions) are used to react with ammonia, pyridines can be generated directly. 1,5-Dicarbonyl compounds or their equivalents can be prepared using conventional chemistry. For example, 1,5-diketones are accessible via a number of routes, such as Michael addition of an enolate to an enone (or precursor Mannich base (Gill et al, *J. Am. Chem. Soc*., **1952**, *74*, 4923)), ozonolysis of a cyclopentene precursor, or reaction of silyl enol ethers with 3-methoxyallylic alcohols (Duhamel et al, *Tetrahedron*, **1986**, *42*, 4777): When one of the carbonyl carbons is at the acid oxidation state, then this type of reaction produces 2-pyridones which can be readily converted to 2-halopyridines (Isler et al, *Helv. Chim. Acta*, **1955**, *38*, 1033) or 2-aminopyridines (Vorbruggen et al, *Chem. Ber*., **1984**, *117*, 1523). Alternatively, a pyridine can be prepared from an aldehyde, a 1,3-dicarbonyl compound and ammonia via the classical Hantzsch synthesis (Bossart et al, *Angew. Chem. Int. Ed. Engl*., **1981**, *20*, 762). Reactions of 1,3-dicarbonyl compounds (or their equivalents) with 3-amino-enones or 3-amino-nitriles have also been used to produce pyridines (such as the Guareschi synthesis, Mariella, *Org. Synth*., *Coll. Vol. IV*, **1963**, 210). 1,3-Dicarbonyl compounds can be made via oxidation reactions on corresponding 1,3-diols or aldol reaction products (Mukaiyama, *Org, Reactions,* **1982,** 28, 203). Cycloaddition reactions have also been used for the synthesis of pyridines, for example cycloaddition reactions between oxazoles and alkenes (Naito et al., *Chem. Pharm. Bull.,* **1965,** *13*, 869), and Diels-Alder reactions between 1,2,4-triazines and enamines (Boger et al., I *Org. Chem.,* **1981,** *46*, 2179).

### (iv) Construction of a pyrimidine ring system

Pyrimidine ring systems useful for the synthesis of compounds of formula V-2 are readily available (Brown, The pyrimidines; Wiley, New York, **1994**). One method for pyrimidine synthesis involves the coupling of a 1,3-dicarbonyl component (or its equivalent) with an N-C-N fragment. The selection of the N-C-N component - urea (Sherman et al., *Org. Synth., Coll. Vol. IV*, **1963,** 247), amidine (Kenner et al., *J. Chem. Soc*., **1943,** 125) or guanidine (Burgess, *J. Org. Chem*., **1956,** *21*, 97; VanAllan, *Org. Synth., Coll. Vol. IV,* **1963**, 245) - governs the substitution at C-2 in the pyrimidine products. This method is particular useful for the synthesis of compounds of formula V-2 with various A groups. In another method, pyrimidines can be prepared via cycloaddition reactions such as aza-Diels-Alder reactions between a 1,3,5-triazine and an enamine or an ynamine (Boger et al., *J. Org. Chem*., **1992**, *57*, 4331 and references cited therein).

### (v) Construction of an imidazole ring system

Imidazoles useful for the synthesis of compounds of formula V-1 are readily prepared using a variety of different synthetic methodologies. Various cyclization reactions are generally used to synthesize imidazoles such as reactions between amidines and alpha-haloketones (Mallick et al, *J. Am. Chem. Soc*., **1984**, *106(23),* 7252) or alpha-hydroxyketones (Shi et al, *Synthetic Comm*., **1993**, *23(18),* 2623), reactions between urea and alpha-haloketones, and reactions between aldehydes and 1,2-dicarbonyl compounds in the presence of amines.

### (vi) Construction of an isoxazole ring system

Isoxazoles useful for the synthesis of compounds of formula V-1 are readily synthesized using various methodologies (such as cycloaddition reactions between nitrile oxides and alkynes or active methylene compounds, oximation of 1,3-dicarbonyl compounds or alpha, beta-acetylenic carbonyl compounds or alpha,beta-dihalocarbonyl compounds, etc.) can be used to synthesize an isoxazole ring system (Grunanger et al., *Isoxazoles*; Wiley & Sons, New York, **1991).** For example, reactions between alkynes and 5-diethylphosphono-2-chlorooximidofuran in the presence of base (e.g. triethylamine, Hunig's base, pyridine) are useful for the synthesis of compounds of formula 2 wherein R⁵ is an isoxazole and X is a furan-2,5-diyl group.

### (vii) Construction of a pyrazole ring system

Pyrazoles useful for the synthesis of compounds of formula V-1 are readily prepared using a variety of methods (Wiley, *Pyrazoles, Pyrazolines, Pyrazolidines, Indazoles, and Condensed Rings;* Interscience Publishers, New York, **1967)** such as reactions between hydrazines and 1,3-dicarbonyl compounds or 1,3-dicarbonyl equivalents (e.g. one of the carbonyl group is masked as an enamine or ketal or acetal), and additions of hydrazines to acrylonitriles followed by cyclization reactions (Dom et al, *Org. Synth.,* **1973,** *Coll. Vol. V,* 39). Reaction of 2-(2-alkyl-3-N,N-dimethylamino)acryloyl-5-diethylphosphonofurans with hydrazines are useful for the synthesis of compounds of formula I wherein R⁵ is a pyrazole, X is a furan-2,5-diyl group and B" is an alkyl group.

### (viii) Construction of a 1,2,4-triazole ring system

1,2,4-Triazoles useful for the synthesis of compounds of formula V-1 are readily available via various methodologies (Montgomery, 1,2,4-Triazoles; Wiley, New York, **1981).** For example, reactions between hydrazides and imidates or thioimidates (Sui et al, *Bioorg. Med. Chem. Lett.,* **1998**, *8*, 1929; Catarzi et al, *J. Med Chem.,* **1995,** *38(2),* 2196), reactions between 1,3,5-triazine and hydrazines (Grundmann et al, *J. Org. Chem*., **1956,** *21*, 1037), and reactions between aminoguanidine and carboxylic esters (Ried et al, *Chem. Ber.,* **1968,** *101,* 2117) are used to synthesize 1,2,4-triazoles.

### (6) Ring closure to construct a heterocycle with a phosphonate

Compounds of formula 4 can also be prepared using a ring closure reaction to construct the heterocycle from precursors that contain the phosphonate component. For example, cyclization reactions between thiourea and 5-diethylphosphono-2-[(-2-bromo-1-oxo)alkyl]furans are useful for the synthesis of compounds of formula 2 wherein R⁵ is a thiazole, A is an amino group and X is a furan-2,5-diyl group. Oxazoles of the present invention can also be prepared using a ring closure reaction. In this case, reactions of urea and 5-diethylphosphono-2-[(-2-bromo-1-oxo)alkyl]furans are useful for the synthesis of compounds of formula I wherein R⁵ is an oxazole, A is an amino group and X is a furan-2,5-diyl group. Reactions between 5-diethylphosphono-2-furaldehyde, an alkyl amine, a 1,2-diketone and ammonium acetate are useful to synthesize compounds of formula 2 wherein R⁵ is an imidazole and X is a furan-2,5-diyl group. These types of ring closure reactions can also be used for the synthesis of pyridines or pyrimidines useful in the present invention. For example, reaction of 5-diethylphosphono-2-[3-dimethylamino-2-alkyl)acryloyl]furans and cyanoacetamide in the presence of base gives 5-alkyl-3-cyano-6-[2-(5-diethylphosphono)furanyl]-2-pyridones (Jain et al., *Tetrahedron Lett.,* **1995,** *36*, 3307). Subsequent conversion of these 2-pyridones to the corresponding 2-halopyridines (see references cited in section 3 for the modifications of heterocycles) will lead to compounds of formula I wherein R⁵ is a pyridine, A is a halo group, X is a furan-2,5-diyl group, and B is an alkyl group. Reactions of 5-diethylphosphono-2-[3-dimethylamino-2-alkyl)acryloyl]furans and amidines in the presence of base give 5-alkyl-6-[2-(5-diethylphosphono)-furanyl]pyrimidines which will lead to compounds of formula 2 wherein R⁵ is a pyrimidine, X is a furan-2,5-diyl group and B is an alkyl group.

### (7) Preparation of various precursors useful for cyclization reactions

Intermediates required for the synthesis of compounds in the present invention are generally prepared using either an existing method in the literature or a modification of an existing method. Syntheses of some of the intermediates useful for the synthesis of compounds in the present invention are described herein.

Various aryl phosphonate dialkyl esters are particularly useful for the synthesis of compounds of formula I. For example, compounds of formula 3 wherein X is a furan-2,5-diyl group can be prepared from a variety of furanyl precursors. It is envisioned that synthesis of other precursors may follow some or all of these reaction steps, and some modifications of these reactions may be required for different precursors. 5-Dialkylphosphono-2-furancarbonyl compounds (e.g. 5-diethylphosphono-2-furaldehyde, 5-diethylphosphono-2-acetylfuran) are well suited for the synthesis of compounds of formula I wherein X is a furan-2,5-diyl group. These intermediates are prepared from furan or furan derivatives using conventional chemistry such as lithiation reactions, protection of carbonyl groups and deprotection of carbonyl groups. For example, lithiation of furan using known methods (Gschwend *Org. React.* **1979**, *26*: 1) followed by addition of phosphorylating agents (e.g. ClPO₃R₂) gives 2-dialkylphosphono-furans (e.g. 2-diethylphosphonofuran). This method can also be applied to a 2-substituted furan (e.g. 2-furoic acid) to give a 5-dialkylphosphono-2-substituted furan (e.g. 5-diethylphosphono-2-furoic acid). It is envisioned that other aryl phosphonate esters can also be prepared using this approach or a modification of this approach. Alternatively, other methods such as transition metal catalyzed reactions of aryl halides or triflates (Balthazar et al. *J. Org. Chem*., **1980,** *45:* 5425; Petrakis et al. *J. Am. Chem. Soc*., **1987**, *109*: 2831; Lu et al. *Synthesis,* **1987,** 726) are used to prepare aryl phosphonates. Aryl phosphonate esters can also be prepared from aryl phosphates under anionic rearrangement conditions (Melvin, *Tetrahedron Lett*., **1981**, *22:* 3375; Casteel et al. *Synthesis,* **1991,** 691). N-Alkoxy aryl salts with alkali metal derivatives of dialkyl phosphonate provide another general synthesis for heteroaryl-2-phosphonate esters (Redmore *J. Org. Chem*., **1970**, *35:* 4114).

A second lithiation step can be used to incorporate a second group on the aryl phosphonate dialkyl ester such as an aldehyde group, a trialkylstannyl or a halo group, although other methods known to generate these functionalities (e.g. aldehydes) can be envisioned as well (e.g. Vilsmeier-Hack reaction or Reimar-Teimann reaction for aldehyde synthesis). In the second lithiation step, the lithiated aromatic ring is treated with reagents that either directly generate the desired functional group (e.g. for an aldehyde using DMF, HCO₂R, etc.) or with reagents that lead to a group that is subsequently transformed into the desired functional group using known chemistry (e.g. alcohols, esters, nitriles, alkenes can be transformed into aldehydes). For example, lithiation of a 2-dialkylphosphonofuran (e.g. 2-diethylphosphonofuran) under normal conditions (e.g. LDA in THF) followed by trapping of the thus generated anion with an electrophile (e.g. tributyltin chloride or iodine) produces a 5-functionalized-2-dialkylphosphonofuran (e.g. 5-tributylstannyl-2-diethylphosphonofuran or 5-iodo-2-diethylphosphonofuran). It is also envisioned that the sequence of these reactions can be reversed, i.e. the aldehyde moiety can be incorporated first followed by the phosphorylation reaction. The order of the reaction will be dependent on reaction conditions and protecting groups. Prior to the phosphorylation, it is also envisioned that it may be advantageous to protect some of these functional groups using a number of well-known methods (e.g. protection of aldehydes as acetals, aminals; protection of ketones as ketals). The protected functional group is then unmasked after phosphorylation. (*Protective groups in Organic Synthesis,* Greene, T. W., **1991,** Wiley, New York). For example, protection of 2-furaldehyde as 1,3-propanediol acetal followed by a lithiation step (using for example LDA) and trapping the anion with a dialkyl chlorophosphate (e.g. diethyl chlorophosphate), and subsequent deprotection of the acetal functionality under normal deprotection conditions produces the 5-dialkylphosphono-2-furaldehyde (e.g. 5-diethylphosphono-2-furaldehyde). Another example is the preparation of 5-keto-2-dialkylphosphonofurans which encompass the following steps: acylations of furan under Friedel-Crafts reaction conditions give 2-ketofuran, subsequent protection of the ketone as ketals (e.g. 1,3-propanediol cyclic ketal) followed by a lithiation step as described above gives the 5-dialkylphosphono-2-furanketone with the ketone being protected as a 1,3-propanediol cyclic ketal, and final deprotection of the ketal under, for example, acidic conditions gives 2-keto-5-dialkylphosphonofurans (e.g. 2-acetyl-5-diethylphosphonofuran). Alternatively, 2-ketofurans can be synthesized via a palladium catalyzed reaction between 2-trialkylstannylfurans (e.g. 2-tributylstannylfuran) and an acyl chloride (e.g. acetyl chloride, isobutyryl chloride). It is advantageous to have the phosphonate moiety present in the 2-trialkylstannylfurans (e.g. 2-tributylstannyl-S-diethylphosphonofuran). 2-Keto-5-dialkylphosphonofurans can also be prepared from a 5-dialkylphosphono-2-furoic acid (e.g. 5-diethylphosphono-2-furoic acid) by conversion of the acid to the corresponding acyl chloride and followed by additions of a Grignard reagent.

Some of the above described intermediates can also be used for the synthesis of other useful intermediates. For example, a 2-keto-5-dialkylphosphonofuran can be further converted to a 1,3-dicarbonyl derivative which is useful for the preparation of pyrazoles, pyridines or pyrimidines. Reaction of a 2-keto-5-dialkylphosphonofuran (e.g. 2-acetyl-5-diethylphosphonofuran) with a dialkylformamide dialkyl acetal (e.g. dimethylformamide dimethyl acetal) gives a 1,3-dicarbonyl equivalent as a 2-(3-dialkylamino-2-alkyl-acryloyl)-5-dialkylphosphonofuran (e.g. 2-(3-dimethylaminoacryloyl)-5-diethylphosphonofuran).

It is envisioned that the above described methods for the synthesis of furan derivatives can be either directly or with some modifications applied to syntheses of various other useful intermediates such as aryl phosphonate esters (e.g. thienyl phosphonate esters, phenyl phosphonate esters or pyridyl phosphonate esters).

It is conceivable that when applicable the above described synthetic methods can be adopted for parallel synthesis either on solid phase or in solution to provide rapid SAR (structure activity relationship) exploration of FBPase inhibitors encompassed in the current invention, provided method development for these reactions are successful.

### Section 2.

### Synthesis of Compounds of Formula X

Synthesis of the compounds encompassed by the present invention typically includes some or all of the following general steps: (1) preparation of a phosphonate prodrug ; (2) deprotection of a phosphonate ester; (3) construction of a heterocycle; (4) introduction of a phosphonate component; (5) synthesis of an aniline derivative. Step (1) and step (2) were discussed in section 1, and discussions of step (3), step (4) and step (5) are given below. These methods are also generally applicable to compounds of Formula X.

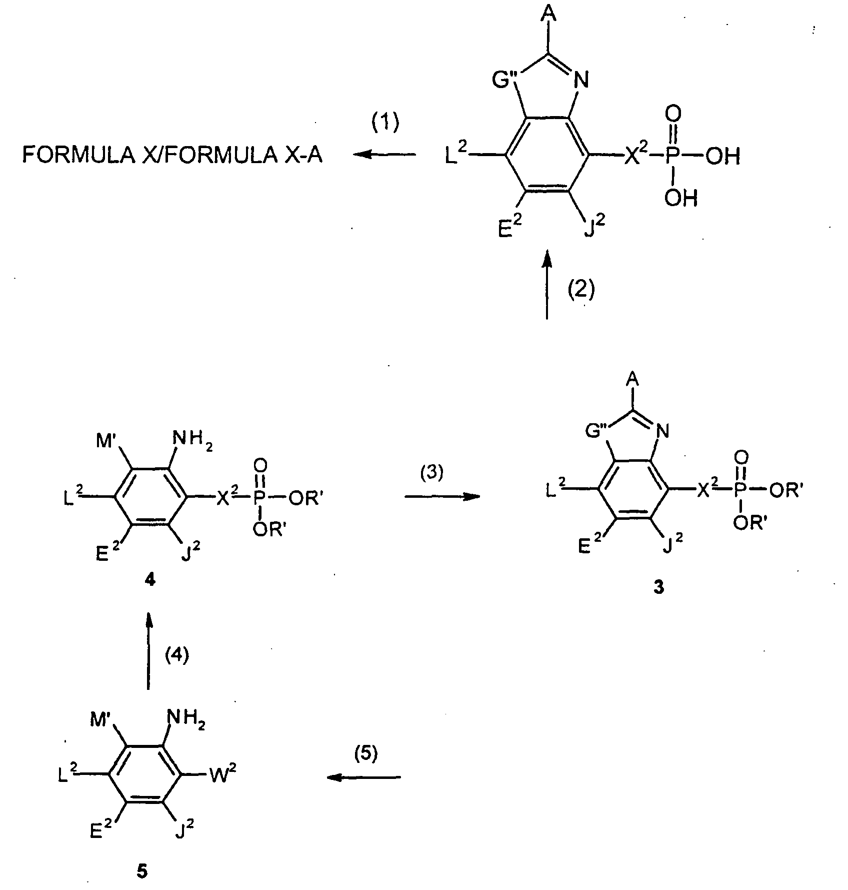

### (3) Construction of a heterocycle

### (i) Benzothiazole ring system

Compounds of formula 3 wherein G"=S, i.e. benzothiazoles, can be prepared using various synthetic methods reported in the literature. Two of these methods are given as examples as discussed below. One method is the modification of commercially available benzothiazole derivatives to give the appropriate functionality on the benzothiazole ring. Another method is the annulation of various anilines (e.g. compounds of formula 4) to construct the thiazole portion of the benzothiazole ring. For example, compounds of formula 3 wherein G"=S, A=NH₂, L²,E²,J²=H, X²=CH₂O, and R'=Et can be prepared from the commercially available 4-methoxy-2-amino thiazole via a two-step sequence: conversion 4-methoxy-2-aminobenzothiazole to 4-hydroxy-2-aminobenzothiazole with reagents such as BBr₃ (Node, M.; et al *J. Org. Chem. 45*, 2243-2246, **1980**) or AlCl₃ in presence of a thiol (e.g. EtSH) (McOmie, J. F. W.; et al. *Org. Synth*., *Collect. Vol*. V, 412, **1973**) followed alkylation of the phenol group with diethylphosphonomethyl trifluoromethylsulfonate (Phillion, D. P.; et al. *Tetrahedron Lett.* 27, 1477-1484, **1986**) in presence of a suitable base (e.g. NaH) in polar aprotic solvents (e.g. DMF) provide the required compound.

Several methods can be used to convert various anilines to benzothiazoles (Sprague, J. M.; Land, A. H. *Heterocycle. Compd*. *5*, 506-13, **1957**). For example, 2-aminobezothiazoles (formula 3 wherein A=NH₂) can be prepared by annulation of compounds of formula 4 wherein W² =H, using various common methods. One method involves the treatment of a suitably substituted aniline with a mixture of KSCN and CuS0₄ in methanol to give a substituted 2-aminobezothiazole (Ismail, I. A.; Sharp, D. E; Chedekel, M. R. *J. Org. Chem. 45*, 2243-2246, **1980).** Alternatively, a 2-aminobenzothiazole can also be prepared by the treatment of Br₂ in presence of KSCN in acetic acid (Patil, D. G.; Chedekel, M. R. *J. Org. Chem. 49,* 997-1000, **1984).** This reaction can also be done in two step sequence. For example treatment of substituted phenylthioureas with Br₂ in CHCl₃ gives substituted 2-aminobenzothiazoles (Patil, D. G.; Chedekel, M. R. *J. Org. Chem. 49*, 997-1000, 1984). 2-Aminobenzothiazoles can also be made by condensation of *ortho* iodo anilines with thiourea in presence ofNi catalyst (NiCl₂ (PPh₃)₂) (Takagi, K.*Chem*. *Lett*. 265-266, **1986**).

Benzothiazoles can undergo electrophilic aromatic substitution to give 6-substituted benzothiazoles (Sprague, J. M.; Land, A. H. *Heterocycle. Compd. 5*, 606-13, **1957).** For example bromination of formula 3 wherein G"=S, A=NH₂, L²,E²,J²=H, X²=CH₂O and R'=Et with bromine in polar solvents such as AcOH gave compound of formula 3 wherein E²=Br.

Furthermore, compounds of formula 3 wherein A is a halo, H, alkoxy, alkylthio or an alkyl can be prepared from the corresponding amino compound (Larock, *Comprehensive organic transformations*, VCH, New York, **1989**; Trost, *Comprehensive organic synthesis*; Pergamon press, New York, **1991**).

### (ii) Benzoxazoles

Compounds of formula 3 wherein G"=O, i.e. benzoxazoles, can be prepared by the annulation of *ortho* aminophenols with suitable reagent (e.g. cyanogen halide (A=NH₂; Alt, K. O.; et al *J. Heterocyclic Chem. 12,* 775, **1975)** or acetic acid (A=CH₃; Saa, J. M.; *J. Org. Chem. 57,* 589-594, **1992**) or trialkyl orthoformate (A=H; *Org. Prep. Proced. Int., 22,* 613,**1990**)).

### (4) Introduction of a Phosphonate Component

Compounds of formula 4 (wherein X²=CH₂O and R'=alkyl) can made in different ways (e.g. using alkylation and nucleophilic substitution reactions). Typically, compounds of formula 5 wherein M'=OH is treated with a suitable base (e.g. NaH) in polar aprotic solvent (e.g. DMF, DMSO) and the resulting phenoxide anion can be alkylated with a suitable electrophile preferably with a phosphonate component present (e.g. diethyl iodomethylphosphonate, diethyl trifluoromethylsulphonomethyl phosphonate, diethyl p-methyltoluenesulphonomethylphosphonate). The alkylation method can also be applied to the precursor compounds to compounds of formula 5 wherein a phenol moiety is present and it can be alkylated with a phosphonate containing component. Alternately, compounds of formula 4 can also be made from the nucleophilic substitution of the precursor compounds to compounds of formula 5 (wherein a halo group, preferably a fluoro or a chloro, is present ortho to a nitro group). For example, a compound of formula 4 (wherein X²=CH₂O and R'=Et) can be prepared from a 2-chloro-1-nitrobenzene derivative by treatment with NaOCH₂P(O)(OEt)₂ in DMF. Similarly, compounds of formula 4 where X²= -alkyl-S- or -alkyl-N- can also be made.

### (5) Synthesis of an aniline derivative

Numerous synthetic methods have been reported for the synthesis of aniline derivatives, these methods can be applied to the synthesis of useful intermediates which can lead to compounds of formula X. For example, various alkenyl or aryl groups can be introduced on to a benzene ring via transition metal catalyzed reactions (Kasibhatla, S. R., et al. WO 98/39343 and the references cited in); anilines can be prepared from their corresponding nitro derivatives via reduction reactions (e.g. hydrogenation reactions in presence of 10 % Pd/C, or reduction reactions using SnCl₂ in HCl (Patil, D. G.; Chedekel, M. R. *J. Org. Chem. 49,* 997-1000, **1984**)).

### EXAMPLES

### Example 1

### Preparation of 5-diethylphosphono-2-furaldehyde (1).

**Step A.** A solution of 2-furaldehyde diethyl acetal (1 mmole) in THF (tetrahydrofuran) was treated with nBuLi (1 mmole) at -78 °C. After 1 h, diethyl chlorophosphate (1.2 mmole) was added and the reaction was stirred for 40 min. Extraction and evaporation gave a brown oil.
**Step B.** The resulting brown oil was treated with 80 % acetic acid at 90 °C for 4 h. Extraction and chromatography gave compound 1 as a clear yellow oil. Alternatively this aldehyde can be prepared from furan as described below.
**Step C.** A solution of furan (1 mmole) in diethyl ether was treated with TMEDA (N,N,NN'-tetramethylethylenediamine) (1 mmole) and nBuLi (2 mmole) at -78 °C for 0.5 h. Diethyl chlorophosphate (1.2 mmole) was added to the reaction mixture and stirred for another hour. Extraction and distillation gave diethyl 2-furanphosphonate as a clear oil.
**Step D.** A solution of diethyl 2-furanphosphonate (1 mmole) in THF was treated with LDA (1.12 mmole, lithium N,N-diisopropylamide) at -78 °C for 20 min. Methyl formate (1.5 mmole) was added and the reaction was stirred for 1 h. Extraction and chromatography gave compound 1 as a clear yellow oil. Preferably this aldehyde can be prepared from 2-furaldehyde as described below.
**Step E.** A solution of 2-furaldehyde (1 mmole) and N,N'-dimethylethylene diamine (1 mmole) in toluene was refluxed while the resulting water being collected through a Dean-Stark trap. After 2 h the solvent was removed in vacuo and the residue was distilled to give furan-2-(N,N'-dimethylimidazolidine) as a clear colorless oil. bp 59 - 61 °C (3 mm Hg).
**Step F.** A solution of furan-2-(N,N'-dimethylimidazolidine) (1 mmole) and TMEDA (1 mmole) in THF was treated with nBuLi (1.3 mmole) at -40 to -48 °C. The reaction was stirred at 0 °C for 1.5 h and then cooled to -55 °C and treated with a solution ofdiethylchlorophosphate (1.1 mmole) in THF. After stirring at 25 °C for 12 h the reaction mixture was evaporated and subjected to extraction to give 5-diethylphosphonofuran-2-(N,N'-dimethylimidazolidine) as a brown oil.
**Step G.** A solution of 5-diethylphosphonofuran-2-(N,N'-dimethyl- imidazolidine) (1 mmole) in water was treated with concentrated sulfuric acid until pH = 1. Extraction and chromatography gave compound 1 as a clear yellow oil.

### Example 2

### Preparation of 5-diethylphosphono-2-[(1-oxo)alkyl]furans and 6-diethylphosphono-2-[(1-oxo)alkyl]pyridines.

**Step A.** A solution of furan (1.3 mmole) in toluene was treated with 4-methyl pentanoic acid (1 mmole), trifluoroacetic anhydride (1.2 mmole) and boron trifluoride etherate (0.1 mmole) at 56°C for 3.5 h. The cooled reaction mixture was quenched with aqueous sodium bicarbonate (1.9 mmole), filtered through a celite pad. Extraction, evaporation and distillation gave 2-[(4-methyl-1-oxo)pentyl]furan as a brown oil (bp 65 - 77°C, 0.1 mmHg).
**Step B.** A solution of 2-[(4-methyl-1-oxo)pentyl]furan (1 mmole) in benzene was treated with ethylene glycol (2.1 mmole) and p-toluenesulfonic acid (0.05 mmole) at reflux for 60 h while removing water via a Dean-Stark trap. Triethyl orthoformate (0.6 mmole) was added and resulting mixture was heated at reflux for an additional hour. Extraction and evaporation gave 2-(2-furanyl)-2-[(3-methyl)butyl]-1,3-dioxolane as an orange liquid.
**Step C.** A solution of 2-(2-furanyl)-2-[(3-methyl)butyl]-1,3-dioxolane (1 mmole) in THF was treated with TMEDA (1 mmole) and nBuLi (1.1 mmole) at -45° C, and the resulting reaction mixture was stirred at -5 to 0° C for 1 h. The resulting reaction mixture was cooled to -45° C, and cannulated into a solution of diethyl chlorophosphate in THF at -45° C. The reaction mixture was gradually warmed to ambient temperature over 1.25 h. Extraction and evaporation gave 2-[2-(5-diethylphosphono)furanyl]-2-[(3-methyl)butyl]-1,3-dioxolane as a dark oil.
**Step D.** A solution of 2-[2-(5-diethylphosphono)furanyl]-2-[(3-methyl)butyl]-1,3-dioxolane (1 mmole) in methanol was treated with 1 N hydrochloric acid (0.2 mmole) at 60 °C for 18 h. Extraction and distillation gave 5-diethylphosphono-2-[(4-methyl-1-oxo)pentyl]furan (2.1) as a light orange oil (bp 152 - 156 °C , 0.1 mmHg).
   The following compounds were prepared according to this procedure:
   (**2.2**) 5-diethylphosphono-2-acetylfuran: bp 125 - 136 °C, 0.1 mmHg.
   (**2.3**) 5-diethylphosphono-2-[(1-oxo)butyl]furan: bp 130 - 145 °C, 0.08 mmHg.
   Alternatively these compounds can be prepared using the following procedures:

**Step E.** A solution of 2-[(4-methyl-1-oxo)pentyl]furan (1 mmole, prepared as in Step A) in benzene was treated with N,N-dimethyl hydrazine (2.1 mmole) and trifluoroacetic acid (0.05 mmole) at reflux for 6 h. Extraction and evaporation gave 2-[(4-methyl-1-oxo)pentyl]furan N,N-dimethyl hydrazone as a brown liquid.
**Step F.** 2-[(4-Methyl-1-oxo)pentyl]furan N,N-dimethyl hydrazone was subjected to the procedures of Step C to give 2-[(4-methyl-1-oxo)pentyl]-5-diethylphosphonofuran N,N-dimethyl hydrazone as a brown liquid which was treated with copper (II) chloride (1.1 equivalent) in ethanol-water at 25 °C for 6 h. Extraction and distillation gave compound **2.1** as a light orange oil.
   Some of 5-diethylphosphono-2-[(1-oxo)alkyl]furans are prepared using the following procedures:

**Step G.** A solution of compound 1 (1 mmole) and 1,3-propanedithiol (1.1 mmole) in chloroform was treated with borontrifluoride etherate (0.1 mmole) at 25 °C for 24 h. Evaporation and chromatography gave 2-(2-(5-diethylphosphono)furanyl)-1,3-dithiane as a light yellow oil.

A solution of 2-(2-(5-diethylphosphono)furanyl)-1,3-dithiane (1 mmole) in THF was cooled to -78°C and treated with nBuLi (1.2 mmole). After 1 h. at -78 °C the reaction mixture was treated with cyclopropanemethyl bromide and reaction was stirred at -78 °C for another hour. Extraction and chromatography gave 2-(2-(5-diethylphosphono)furanyl)-2-cyclopropanemethyl-1,3-dithiane as an oil.

A solution of 2-(2-(5-diethylphosphono)furanyl)-2-cyclopropanemethyl-1,3-dithiane (1 mmole) in acetonitrile - water was treated with [bis(trifluoroacetoxy)iodo]benzene (2 mmole) at 25 °C for 24 h. Extraction and chromatography gave 5-diethylphosphono-2-(2-cyclopropylacetyl)furan as a light orange oil.

The following compounds were prepared according to this procedure:
**(2.4)** 5-Diethylphosphono-2-(2-ethoxycarbonylacetyl)furan
**(2.5)** 5-Diethylphosphono-2-(2-methylthioacetyl)furan
**(2.6)** 6-Diethylphosphono-2-acetylpyridine

### Example 3.

### Preparation of 4-[2-(5-phosphono)furanl]thiazoles. 4-[2-(6-phosphono)pyridyl]thiazoles and 4-[2-(5-phosphono)furanyl]selenazoles.

**Step A.** A solution of compound **2.1** (1 mmole) in ethanol was treated with copper (II) bromide (2.2 mmole) at reflux for 3 h. The cooled reaction mixture was filtered and the filtrate was evaporated to dryness. The resulting dark oil was purified by chromatography to give 5-diethylphosphono-2-[(2-bromo-4-methyl-1-oxo)pentyl]furan as an orange oil.
**Step B.** A solution of 5-diethylphosphono-2-[(2-bromo-4-methyl-1-oxo)pentyl]furan (I mmole) and thiourea (2 mmole) in ethanol was heated at reflux for 2 h. The cooled reaction mixture was evaporated to dryness and the resulting yellow foam was suspended in saturated sodium bicarbonate and water (pH = 8). The resulting yellow solid was collected through filtration to give 2-amino-5-isobutyl-4-[2-(5-diethylphosphono)furanyl]thiazole.
**Step C.** A solution of 2-amino-5-isobutyl-4-[2-(5-diethylphosphono)-furanyl]thiazole (1 mmole) in methylene chloride was treated with bromotrimethylsilane (10 mmole) at 25 °C for 8 h. The reaction mixture was evaporated to dryness and the residue was suspended in water. The resulting solid was collected through filtration to give 2-amino-5-isobutyl-4-[2-(5-phosphono)furanyl]thiazole (**3.1**) as an off-white solid. mp > 250 °C. Anal. calcd. for C₁₁H₁₅N₂O₄PS + 1.25HBr: C: 32.75; H: 4.06; N: 6.94. Found: C: 32.39; H: 4.33; N: 7.18.
   According to the above procedures or in some cases with minor modifications of these procedures using conventional chemistry the following compounds were prepared: **(3.2)** 2-Methyl-5-isobutyl-4-[2-(5-phosphono)furanyl]thiazole. Anal. calcd. for C₁₂H₁₆NO₄PS + HBr + 0.1CH₂Cl₂: C: 37.20; H: 4.44; N: 3.58. Found: C: 37.24; H: 4.56; N: 3.30.
   **(3.3)** 4-[2-(5-Phosphono)furanyl]thiazole. Anal. calcd. for C₇H₆NO₄PS + 0.65 HBr: C: 29.63; H: 2.36; N: 4.94. Found: C: 29.92; H: 2.66; N: 4.57.
   **(3.4)** 2-Methyl-4-[2-(5-phosphono)furanyl]thiazole. mp 235 - 236 °C. Anal. calcd. for C₈H₈NO₄PS + 0.25H₂O: C: 38..48; H: 3.43; N: 5.61. Found: C: 38.68; H: 3.33; N: 5.36.
   **(3.5)** 2-Phenyl-5-isobutyl-4-[2-(5-phosphono)furanyl]thiazole. Anal. calcd. for C₁₇H₁₈NO₄PS + HBr: C: 45.96; H: 4.31; N: 3.15. Found: C: 45.56; H: 4.26; N: 2.76.
   **(3.6)** 2-Isopropyl-4-[2-(5-phosphono)furanyl]thiazole. mp 194 - 197 °C. Anal. calcd. for C₁₀H₁₂N0₄PS: C: 43.96; H: 4.43; N: 5.13. Found: C: 43.70; H: 4.35; N: 4.75.
   **(3.7)** 5-Isobutyl-4-[2-(5-phosphono)furanyl]thiazole. mp 164 - 166 °C. Anal. calcd. for C₁₁H₁₄NO₄PS: C: 45.99; H: 4.91; N: 4.88. Found: C: 45.63; H: 5.01; N: 4.73.
   (**3.8**) 2-Aminothiocarbonyl-4-[2-(5-phosphono)furanyl]thiazole. mp 189 - 191°C. Anal. calcd. for C₈H₇N₂O₄PS₂: C: 33.10; H: 2.43; N: 9.65. Found: C: 33.14; H: 2.50; N: 9.32.
   **(3.9)** 2-(I-Piperidyl)-5-isobutyl-4-[2-(5-phosphono)furanyl]thiazole. Anal. calcd. for C₁₆H₂₃N₂O₄PS + 1.3HBr: C: 40.41; H: 5.15; N: 5.89. Found: C: 40.46; H: 5.36; N: 5.53.
   **(3.10)** 2-(2-Thienyl)-5-isobutyl-4-[2-(5-phosphono)furanyl]thiazole. Anal. calcd. for C₁₅H₁₆NO₄PS₂ + 0.75H₂O: C: 47.05; H: 4.61; N: 3.66. Found: C: 47.39; H: 4.36; N: 3.28.
   **(3.11)** 2-(3-Pyridyl)-5-isobutyl-4-[2-(5-phosphono)furanyl]thiazole. Anal. calcd. for C₁₆H₁₇N₂O₄PS + 3.75HBr: C: 28.78; H: 3.13; N: 4.20. Found: C: 28.73; H: 2.73; N: 4.53.
   **(3.12)** 2-Acetamido-5-isobutyl-4-[2-(5-phosphono)furanyl]thiazole. mp 179 -181 °C. Anal. calcd. for C₁₃H₁₇N₂O₅PS + 0.25H₂O: C: 44.76; H: 5.06; N: 8.03. Found: C: 44.73; H: 5.07; N: 7.89.
   **(3.13)** 2-Amino-4-[2-(5-phosphono)furanyl]thiazole. Anal. calcd. for C₇H₇N₂O₄PS: C: 34.15; H: 2.87; N: 11.38. Found: C: 33.88; H: 2.83; N: 11.17.
   (**3.14**) 2-Methylamino-5-isobutyl-4-[2-(5-phosphono)furanyl]thiazole. mp 202 - 205 °C. Anal. calcd. for C₁₂H₁₇N₂O₄PS + 0.5H₂O: C: 44.30; H: 5.58; N: 8.60. Found: C: 44.67; H: 5.27; N: 8.43.
   (**3.15**) 2-(N-amino-N-methyl)amino-5-isobutyl-4-[2-(5-phosphono)furanyl]thiazole. mp 179 -181 °C. Anal. calcd. for C₁₂H₁₈N₃O₄PS + 1.25HBr: C: 33.33; H: 4.49; N: 9.72. Found: C: 33.46; H: 4.81; N: 9.72.
   **(3.16)** 2-Amino-5-methyl-4-[2-(5-phosphono)furanyl]thiazole. mp 200-220 °C. Anal. calcd. for C₈H₉N₂O₄PS + 0.65HBr: C: 30.72; H: 3.11; N: 8.96. Found: C: 30.86; H: 3.33; N: 8.85.
   **(3.17)** 2,5-Dimethyl-4-[2-(5-phosphono)furanyl]thiazole. mp 195 °C (decomp). Anal. calcd. for C₉H₁₀NO₄PS + 0.7HBr: C: 34.22; H: 3.41; N: 4.43. Found: C: 34.06; H: 3.54; N: 4.12.
   **(3.18)** 2-Aminothiocarbonyl-5-isobutyl-4-[2-(5-phosphono)furanyl]thiazole. Anal. calcd. for C₁₂H₁₅N₂O₄PS₂ + 0.1HBr+ 0.3EtOAc: C: 41.62; H: 4.63; N: 7.35. Found: C: 41.72; H: 4.30; N: 7.17.
   **(3.19)** 2-Ethoxycarbonyl-4-[2-(5-phosphono)furanyl]thiazole. mp 163 - 165 °C. Anal. calcd. for C₁₀H₁₀NO₆PS + 0.5H₂O: C: 38.47; H: 3.55; N: 4.49. Found: C: 38.35; H: 3.30; N: 4.42.
   **(3.20)** 2-Amino-5-isopropyl-4-[2-(5-phosphono)furanyl]thiazole. Anal. calcd. for C₁₀H₁₃N₂O₄PS + IHBr: C: 32.53; H: 3.82; N: 7.59. Found: C: 32.90; H: 3.78; N: 7.65.
   **(3.21)** 2-Amino-5-ethyl-4-[2-(5-phosphono)furanyl]thiazole mp > 250 °C. Anal. calcd. for C₉H₁₁N₂O₄PS: C: 39.42; H: 4.04; N: 10.22. Found: C: 39.02; H: 4.15; N: 9.92.
   **(3.22)** 2-Cyanomethyl-4-[2-(5-phosphono)furanyl]thiazole. mp 204 - 206 °C. Anal. calcd. for C₉H₇N₂O₄PS: C: 40.01; H: 2.61; N: 10.37. Found: C: 39.69; H: 2.64; N: 10.03.
   **(3.23)** 2-Aminothiocarbonylamino-5-isobutyl-4-[2-(5-phosphono)furanyl]thiazole. mp 177 - 182°C. Anal. calcd. for C₁₂H₁₆N₃O₄PS₂ + 0.2hexane + 0.3HBr: C: 39.35; H: 4.78; N: 10.43. Found: C: 39.61; H: 4.48; N: 10.24.
   **(3.24)** 2-Amino-5-propyl-4-[2-(5-phosphono)furanyl]thiazole. mp 235-237 °C. Anal. calcd. for C₁₀H₁₃N₂O₄PS + 0.3H₂O: C: 40.90; H: 4.67; N: 9.54. Found: C: 40.91; H: 4.44; N: 9.37.
   **(3.25)** 2-Amino-5-ethoxycarbonyl-4-[2-(5-phosphono)furanyl]thiazole. mp 248-250 °C. Anal. calcd. for C₁₀H₁₁N₂O₆PS + 0.1HBr: C: 36.81; H: 3.43; N: 8.58. Found: C: 36.99; H: 3.35; N: 8.84.
   **(3.26)** 2-Amino-5-methylthio-4-[2-(5-phosphono)furanyl]thiazole. mp 181-184 °C. Anal. calcd. for C₈H₉N₂O₄PS₂ + 0.4H₂O: C: 32.08; H: 3.30; N: 9.35. Found: C:32.09; H: 3.31; N: 9.15.
   **(3.27)** 2-Amino-S-cyclopropyl-4-[2-(5-phosphono)furanyl]thiazole. Anal. calcd. for C₁₀H₁₁N₂O₄PS + 1H₂O + 0.75HBr: C: 32.91; H: 3.80; N: 7.68. Found: C: 33.10; H: 3.80; N: 7.34.
   **(3.28)** 2-Amino-5-methanesulfinyl-4-[2-(S-phosphono)furanyl]thiazole. mp > 250 °C. Anal. calcd. for C₈H₉N₂O₅PS₂ + 0.35NaCl: C: 29.23; H: 2.76; N: 8.52. Found: C: 29.37; H: 2.52; N: 8.44.
   **(3.29)** 2-Amino-S-benzyloxycarbonyl-4-[2-(5-phosphono)furanyl]thiazole. Anal. calcd for C₁₅H₁₃N₂O₆PS+ 0.2H₂O: C: 46.93; H: 3.52; N: 7.30. Found: C: 46.64; H: 3.18; N: 7.20.
   (**3.30**) 2-Amino-5-cyclobutyl-4-[2-(5-phosphono)furanyl]thiazole. Anal. calcd for C₁₁H₁₃N₂O₄PS+0.15 HBr+0.15H₂O:C:41.93;H:4.30;N:8.89. Found: C: 42.18; H: 4.49; N: 8.53.
   **(3.31)** 2-Amino-5-cyclopropyl-4-[2-(5-phosphono)furanyl]thiazole hydrobromide. Anal. calcd for C₁₀H₁₁N₂O₄PSBr+ 0.73HBr + 0.15MeOH + 0.5H₂O: C: 33.95; H: 3.74; N: 7.80; S: 8.93; Br: 16.24. Found: C: 33.72; H: 3.79; N: 7.65; S: 9.26; Br: 16.03.
   (**3.32**) 2-Amino-5-[(N,N-dimethyl)aminomethyl]-4-[2-(5-phosphono)furanyl]thiazole dihydrobromide. Anal. calcd for C₁₀H₁₆N₃O₄Br₂PS+ 0.8CH₂Cl₂: C: 24.34; H: 3.33; N: 7.88. Found: C: 24.23; H: 3.35; N: 7.64.
   **(3.33)** 2-Amino-S-methoxycarbonyl-4-[2-(5-phosphono)furanyl]thiazole. Mp 227 °C (decomp). Anal. calcd for C₉H₉N₂O₆PS + 0.1H₂O + 0.2HBr: C: 33.55; H: 2.94; N: 8.69. Found: C: 33.46; H: 3.02; N: 8.49.
   **(3.34)** 2-Amino-5-ethylthiocarbonyl-4-[2-(5-phosphono)furanyl]thiazole . Mp 245 °C (decomp). Anal. calcd for C₁₀H₁₁N₂O₅PS₂: C: 35.93; H: 3.32; N: 8.38. Found: C: 35.98; H: 3.13; N: 8.17.
   **(3.35)** 2-Amino-5-propyloxycarbonyl-4-[2-(5-phosphono)furanyl]thiazole . Mp 245 °C (decomp). Anal. calcd for C₁₁H₁₃N₂O₆PS : C: 39.76; H: 3.94; N: 8.43. Found: C: 39.77; H: 3.72; N: 8.19.
   **(3.36)** 2-Amino-5-benzyl-4-[2-(5-phosphono)furanyl]thiazole. Anal. calcd for C₁₄H₁₃N₂O₄PS +H₂O: C: 47.46; H: 4.27; N: 7.91. Found: C: 47.24; H: 4.08; N: 7.85.
   **(3.37)** 2-Amino-5-[(N,N-diethyl)aminomethyl]-4-[2-(5-phosphono)furanyl]thiazole dihydrobromide. Anal. calcd for C₁₂H₂₀N₃O₄Br₂PS + 0.1HBr + 1.4 MeOH : C: 29.47; H: 4.74; N: 7.69. Found: C: 29.41; H: 4.60; N: 7.32.
   **(3.38)** 2-Amino-5-[(N,N-dimethyl)carbamoyl]-4-[2-(5-phosphono)furanyl]thiazole. Anal. calcd for C₁₀H₁₂N₃O₅PS + 1.3HBr + 1.0H₂O + 0.3 Acetone: C: 28.59; H: 3.76; N: 9.18. Found: C: 28.40; H: 3.88; N: 9.01.
   **(3.39)** 2-Amino-5-carboxyl-4-[2-(5-phosphono)furanyl]thiazole. Anal. calcd for C₈H₇N₂O₆PS + 0.2HBr + 0.1 H₂O: C: 31.18; H: 2.42; N: 9.09. Found: C: 31.11; H: 2.42; N: 8.83.
   **(3.40)** 2-Amino-5-isopropyloxycarbonyl-4-[2-(5-phosphono)furanyl]thiazole . Mp 240 °C (decomp). Anal. calcd for C₁₁H₁₃N₂O₆PS : C: 39.76; H: 3.94; N: 8.43. Found: C: 39.42; H: 3.67; N: 8.09.
   **(3.41)** 2-Methyl-5-ethyl-4-[2-(5-phosphono)furanyl]thiazole. Anal. calcd for C₁₀H₁₂O₄PNS + 0.75HBr + 0.35H₂O: C: 36.02; H: 4.13; N: 4.06. Found: C: 36.34; H: 3.86; N: 3.69.
   **(3.42)** 2-Methyl-5-cyclopropyl-4-[2-(5-phosphono)furanyl]thiazole. Anal. calcd for C₁₁H₁₂NO₄PS + 0.3HBr + 0.5CHCl₃: C: 37.41; H: 3.49; N: 3.79. Found: C: 37.61; H: 3.29; N: 3.41.
   **(3.43)** 2-Methyl-5-ethoxycarbonyl-4-[2-(5-phosphono)furanyl]thiazole. Anal. calcd for C₁₁H₁₂NO₆PS : C: 41.64; H: 3.81; N: 4.40. Found: C: 41.61; H: 3.78; N: 4.39.
   (**3.44**) 2-[(N-acetyl)amino]-5-methoxymethyl-4-[2-(5-phosphono)furanyl]thiazole. Anal. calcd for C₁₁H₁₃N₂O₆PS + 0.15HBr: C: 38.36; H: 3.85; N: 8.13. Found: C: 38.74; H: 3.44; N: 8.13.
   **(3.45)** 2-Amino-5-(4-morpholinyl)methyl-4-[2-(5-phosphono)furanyl]thiazole dihydrobromide. Anal. calcd for C₁₂H₁₈Br₂N₃O₅PS + 0.25HBr: C: 27.33; H: 3.49; N: 7.97. Found: C: 27.55; H: 3.75; N: 7.62.
   **(3.46)** 2-Amino-5-cyclopropylmethoxycarbonyl-4-[2-(5-phosphono)furanyl]thiazole. Mp 238 °C (decomp). Anal. calcd for C₁₂H₁₃N₂O₆PS : C: 41.86; H: 3.81; N: 8.14. Found: C: 41.69; H: 3.70; N: 8.01.
   **(3.47)** 2-Amino-5-methylthio-4-[2-(5-phosphono)furanyl]thiazole N,N-dicyclohexylammonium salt. Mp >250 °C. Anal. calcd for C₈H₉N₂O₄PS₂ + 1.15 C₁₂H₂₃N: C: 52.28; H: 7.13; N: 8.81. Found: C: 52.12; H: 7.17; N: 8.81.
   **(3.48)** 2-[(N-Dansyl)amino]-5-isobutyl-4-[2-(5-phosphono)furanyl]thiazole. Anal. calcd for C₂₃H₂₆N₃O₆PS₂ + 0.5HBr: C: 47.96; H: 4.64; N: 7.29. Found: C: 48.23; H: 4.67; N: 7.22.
   **(3.49)** 2-Amino-5-(2,2,2-trifluoroethyl)-4-[2-(5-phosphono)furanyl]thiazole. Anal. calcd for C₉H₈N₂F₃O₄PS : C:32.94, H:2.46, N:8.54. Found: C:32.57, H:2.64, N:8.14.
   **(3.50)** 2-Methyl-5-methylthio-4-[2-(5-phosphono)furanyl]thiazole. Anal. calcd for C₉H₁₀NO₄PS₂: C: 37.11; H: 3.46; N: 4.81. Found: C: 36.72; H: 3.23; N: 4.60.
   **(3.51)** 2-Amino-5-methylthio-4-[2-(5-phosphono)furanyl]thiazole ammonium salt. Anal. calcd for C₈H₁₂N₃O₄PS₂ : C: 31.07; H: 3.91; N: 13.59. Found: C: 31.28; H: 3.75; N: 13.60.
   **(3.52)** 2-Cyano-5-ethyl-4-[2-(5-phosphono)furanyl]thiazole. Anal. calcd for C₁₀H₉N₂O₄PS: C: 42.26; H: 3.19; N: 9.86. Found: C: 41.96; H: 2.95; N: 9.76.
   **(3.53)** 2-Amino-S-hydroxymethyl-4-[2-(5-phosphono)furanyl]thiazole. Anal. calcd for C₈H₉N₂O₅PS: C: 34.79; H: 3.28; N: 10.14. Found: C: 34.57; H: 3.00; N: 10.04.
   **(3.54)** 2-Cyano-5-isobutyl-4-[2-(5-phosphono)furanyl]thiazole. Anal. calcd for C₁₂H₁₃N₂O₄SP+ 0.09HBr: C: 46.15; H: 4.20; N: 8.97. Found: C: 44.81; H: 3.91; N: 8.51.
   **(3.55)** 2-Amino-5-isopropylthio-4-[2-(5-phosphono)furanyl]thiazole hydrobromide. Anal. calcd for C₁₀H₁₄BrN₂O₄PS₂: C: 29.94; H: 3.52; N: 6.98. Found: C: 30.10; H: 3.20; N: 6.70.
   **(3.56)** 2-Amino-5-phenylthio-4-[2-(5-phosphono)furanyl]thiazole. Anal. calcd for C₁₃H₁₁N₂O₄PS₂: C: 44.07; H: 3.13; N: .91. Found: C: 43.83; H: 3.07; N: 7.74.
   **(3.57)** 2-Amino-5-tert-butylthio-4-[2-(5-phosphono)furanyl]thiazole. Anal. calcd for C₁₁H₁₅N₂O₄PS₂ + 0.6CH₂Cl₂: C: 36.16; H: 4.24; N: 7.27. Found: C: 36.39; H: 3.86; N: 7.21.
   **(3.58)** 2-Amino-5-propylthio-4-[2-(5-phosphono)furanyl]thiazole hydrobromide. Anal. calcd for C₁₀H₁₄BrN₂O₄PS₂: C: 29.94; H: 3.52; N: 6.98. Found: C: 29.58; H: 3.50; N: 6.84.
   **(3.59)** 2-Amino-5-ethylthio-4-[2-(5-phosphono)furanyl]thiazole. Anal. calcd for C₉H₁₁N₂O₄PS₂+ 0.25HBr: C: 33.11; H: 3.47; N: 8.58. Found: C: 33.30; H: 3.42; N: 8.60.
   **(3.60)** 2-[(N-tert-butyloxycarbonyl)amino]-5-methoxymethyl-4-[2-(5-phosphono)furanyl]thiazole. Anal. calcd for C₁₄H₁₉N₂O₇PS: C: 43.08; H: 4.91; N: 7.18. Found: C: 42.69; H: 4.58; N: 7.39.
   (**3.61**) 2-Hydroxyl-4-[2-(5-phosphono)furanyl]thiazole. Anal. calcd for C₇H₆NO₅PS: C: 34.02; H: 2.45; N: 5.67. Found: C: 33.69; H: 2.42; N: 5.39.
   **(3.62)** 2-Hydroxyl-5-ethyl-4-[2-(5-phosphono)furanyl]thiazole. Anal. calcd for C₉H₁₀NO₅PS: C: 39.28; H: 3.66; N: 5.09. Found: C: 39.04; H: 3.44; N: 4.93.
   **(3.63)** 2-Hydroxyl-5-isopropyl-4-[2-(5-phosphono)furanyl]thiazole. Anal. calcd for C₁₀H₁₂NO₅PS + 0.1HBr: C: 40.39; H: 4.10; N: 4.71. Found: C: 40.44; H: 4.11; N: 4.68. **(3.64)** 2-Hydroxyl-5-isobutyl-4-[2-(5-phosphono)furanyl]thiazole. Anal. calcd for C₁₁H₁₄NO₅PS: C: 43.57; H: 4.65; N: 4.62. Found: C: 43.45; H: 4.66; N: 4.46.
   **(3.65)** 5-Ethoxycarbonyl-4-[2-(5-phosphono)furanyl]thiazole. Anal. calcd for C₁₀H₁₀NO₆PS: C: 39.61; H: 3.32; N: 4.62. Found: C: 39.60; H: 3.24; N: 4.47.
   **(3.66)** 2-Amino-5-vinyl-4-[2-(5-phosphono)furanyl]thiazole. Anal. calcd for C₉H₉N₂O₄PS + 0.28HCl: C: 37.66; H: 3.26; N: 9.46. Found: C: 37.96; H: 3.37; N: 9.10.
   **(3.67)** 2-Amino-4-[2-(6-phosphono)pyridyl]thiazole hydrobromide.
   **(3.68)** 2-Methylthio-5-isobutyl-4-[2-(5-phosphono)furanyl]thiazole. Anal. calcd for C₁₂H₁₆NO₄PS₂: C: 43.24; H: 4.84; N: 4.20. Found: C: 43.55; H: 4.63; N: 4.46.
   **(3.69)** 2-Amino-5-isobutyl-4-[2-(3-phosphono)furanyl]thiazole. Anal. calcd for C₁₁H₁₅N₂O₄PS + 0.1 H₂O: C: 43.45; H: 5.04; N: 9.21. Found: C: 43.68; H: 5.38; N: 8.98.
   **(3.70)** 2-Amino-5-isobutyl-4-[2-(5-phosphono)furanyl]selenazole. Anal. calcd for C₁₁H₁₅N₂O₄PSe + 0.14 HBr + 0.6 EtOAc : C: 38.93; H: 4.86; N: 6.78. Found: C: 39.18; H: 4.53; N: 6.61.
   **(3.71)** 2-Amino-5-methylthio-4-[2-(5-phosphono)furanyl]selenazole. Anal. calcd for C₈H₉N₂O₄PSSe + 0.7 HBr + 0.2 EtOAc : C: 25.57; H: 2.75; N: 6.78. Found: C: 25.46; H: 2.49; N: 6.74.
   **(3.72)** 2-Amino-5-ethyl-4-[2-(5-phosphono)furanyl]selenazole. Anal. calcd for C₉H₁₁N₂O₄PSe + HBr: C: 26.89; H: 3.01; N: 6.97. Found: C: 26.60; H: 3.16; N: 6.81.

### Example 4.

### Preparation of 5-halo-4-[2-(5-phosphono)furanyl]thiazoles.

**Step A.** A solution of 2-amino-4-[2-(5-diethylphosphono)furanyl]thiazole (prepared as in Step B of Example 3) (1 mmole) in chloroform was treated with N-bromo succinimide (NBS) (1.5 mmole) at 25 °C for 1 h. Extraction and chromatography gave 2-amino-5-bromo-4-[2-(5-diethylphosphono)furanyl]-thiazole as a brown solid.
**Step B.** 2-Amino-5-bromo-4-[2-(5-diethylphosphono)furanyl]thiazole was subjected to Step C of Example 3 to give 2-amino-5-bromo-4-[2-(5-phosphono)-furanyl]thiazole (4.1) as a yellow solid. mp > 230 °C. Anal. calcd. for C₇H₆N₂O₄PSBr: C: 25.86; H: 1.86; N: 8.62. Found: C: 25.93; H: 1.64; N: 8.53.

The following compounds were prepared according to this procedure:
**(4.2)** 2-Amino-5-chloro-4-[2-(5-phosphono)furanyl]thiazole. Anal. calcd. for C₇H₆N₂O₄PSCl: C: 29.96; H: 2.16; N: 9.98. Found: C: 29.99; H: 1.97; N: 9.75.
**(4.3)** 2-Amino-5-iodo-4-[2-(5-phosphono)furanyl]thiazole. Anal. calcd. for C₇H₆N₂O₄PSI: C: 22.42; H: 2.28; N: 6.70. Found: C: 22.32; H: 2.10; N: 6.31.
**(4.4)** 2,5-Dibromo-4-[2-(5-phosphono)furanyl]thiazole. Anal. calcd. for C₇H₄NO₄PSBr₂: C: 21.62; H: 1.04; N: 3.60. Found: C: 21.88; H: 0.83; N: 3.66.

### Examples 5.

### Preparation of 2-halo-4-f2-(5-uhosphoao)furanvllthiazoles.

**Step A.** A solution of 2-amino-5-isobutyl-4-[2-(5-diethylphosphono)-furanyl]thiazole (prepared as in Step B of Example 3) (1 mmole) in acetonitrile was treated with copper (II) bromide (1.2 mmole) and isoamyl nitrite (1.2 mmole) at 0 °C for 1 h. Extraction and chromatography gave 2-bromo-5-isobutyl-4-[2-(5-diethylphosphono)furanyl]thiazole as a brown solid.
**Step B.** 2-Bromo-5-isobutyl-4-[2-(5-diethylphosphono)furanyl]thiazole was subjected to Step C of Example 3 to give 2-bromo-5-isobutyl-4-[2-(5-phosphono)furanyl]thiazole (5.1) as a yellow hygroscopic solid. Anal. calcd. for C₁₁H₁₃NO₄PSBr: C: 36.08; H: 3.58; N: 3.83. Found: C: 36.47; H: 3.66; N: 3.69.

The following compound was prepared according to this procedure:
**(5.2)** 2-Chloro-5-isobutyl-4-[2-(5-phosphono)furanyl]thiazole: Anal. calcd. for C₁₁H₁₃NO₄PSCl: C: 41.07; H: 4.07; N: 4.35. Found: C: 40.77; H: 4.31; N: 4.05.
(**5.3**) 2-Bromo-5-methylthio-4-[2-(5-phosphono)furanyl]thiazole: Anal. calcd. for C₈H₇NO₄PS₂Br: C: 26.98; H: 1.98; N: 3.93. Found: C: 27.21; H: 1.82; N: 3.84.

### Example 6.

### Preparation of Various 2- and 5-substituted 4-[2-(5-phosphono)furanyl]thiazoles.

**Step A.** A solution of 2-bromo-5-isobutyl-4-[2-(5-diethylphosphono)-furanyl]thiazole (1 mmole, prepared as in the Step A of Example 5) in DMF was treated with tributyl(vinyl)tin (5 mmole) and palladium bis(triphenylphosphine) dichloride (0.05 mmole) at 100 °C under nitrogen. After 5 h the cooled reaction mixture was evaporated and the residue was subjected to chromatography to give 2-vinyl-5-isobutyl-4-[2-(5-diethylphosphono)furanyl]thiazole as a yellow solid.
**Step B.** 2-Vinyl-5-isobutyl-4-[2-(5-diethylphosphono)furanyl]thiazole was subjected to Step C of Example 3 to give 2-vinyl-5-isobutyl-4-[2-(5-phosphono)-furanyl]thiazole **(6.1)** as a yellow solid. Anal. calcd. for C₁₃H₁₆NO₄PS + IHBr + 0.1H₂O: C: 39.43; H: 4.38; N: 3.54. Found: C: 39.18; H: 4.38; N: 3.56.
   This method can also be used to prepare various 5-substituted 4-[2-(5-phosphono)furanyl]thiazoles from their corresponding halides.
**Step C.** 2-Amino-5-bromo-4-[2-(5-diethylphosphono)furanyl]thiazole was subjected to Step A using 2-tributylstannylfuran as the coupling partner to give 2-amino-5-(2-furanyl)-4-[2-(5-diethylphosphono)furanyl]thiazole.
**Step D.** 2-Amino-5-(2-furanyl)-4-[2-(5-diethylphosphono)furanyl]thiazole was subjected to Step C of Example 3 to give 2-amino-5-(2-furanyl)-4-[2-(5-phosphono)furanyl]thiazole **(6.2).** mp 190-210 °C. Anal. calcd. for C₁₁H₉N₂O₅PS + 0.25HBr: C: 39.74; H: 2.80; N: 8.43. Found: C: 39.83; H: 2.92; N: 8.46.

The following compound was prepared according to this procedure:
(**6.3**) 2-Amino-5-(2-thienyl)-4-[2-(5-diethylphosphono)furanyl]thiazole. Anal. calcd. for C₁₁H₉N₂O₄PS₂ + 0.3EtOAc + 0.11HBr: C: 40.77; H: 3.40; N: 7.79. Found: C: 40.87; H: 3.04; N: 7.45.

### Example 7.

### Preparation of 2-ethyl-4-[2-(5-phosphono)furanyl]thiazoles.

**Step A.** A solution of 2-vinyl-5-isobutyl-4-[2-(5-diethylphosphono)furanyl]-thiazole (1 mmole, prepared as in the Step A of Example 6) in ethanol was treated with palladium on carbon (0.05 mmole) under 1 atmosphere of hydrogen for 12 h. The reaction mixture was filtered, the filtrate was evaporated and the residue was purified by chromatography to give 2-ethyl-5-isobutyl-4-[2-(5-diethylphosphono)furanyl]thiazole as a yellow foam.
**Step B.** 2-Ethyl-5-isobutyl-4-[2-(5-diethylphosphono)furanyl]thiazole was subjected to Step C of Example 3 to give 2-ethyl-5-isobutyl-4-[2-(5-phosphono)-furanyl]thiazole (7.1) as a yellow solid. Anal. calcd. for C₁₃H₁₈NO₄PS + 1HBr: C: 39.41; H: 4.83; N: 3.53. Found: C: 39.65; H: 4.79; N: 3.61.

### Example 8.

### Preparation of 4-phosphonomethoxymethylthiazoles.

**Step A.** A solution of diethyl hydroxymethylphosphonate (1 mmole) in DMF was treated with sodium hydride (1.2 mmole) followed by 2-methyl-4-chloromethylthiazole (1 mmole) at 0 °C and stirred at 25 °C for 12 h. Extraction and chromatography gave 2-methyl-4-(diethylphosphonomethoxymethyl)thiazole.
**Step B.** 2-Methyl-4-diethylphosphonomethoxymethylthiazole was subjected to Step C of Example 3 to give 2-methyl-4-phosphonomethoxymethylthiazole (**8.1**). Anal. calcd. for C₆H₁₀NO₄PS + 0.5HBr + 0.5H₂O: C: 26.43; H: 4.25; N: 5.14. Found: C: 26.52; H: 4.22; N: 4.84.
**Step C.** 2-Methyl-4-diethylphosphonomethoxymethylthiazole was subjected to Step A of Example 4 and followed by Step C of Example 3 to give 5-bromo-2-methyl-4-phosphonomethoxymethylthiazole (**8.2**). Anal. calcd. for C₆H₉NO₄PSBr + 0.5HBr: C: 21.04; H: 2.80; N: 4.09. Found: C: 21.13; H: 2.69; N: 4.01.
**Step D.** A solution of ethyl 2-[(N-Boc)amino]-4-thiazolecarboxylate (1 mmole) in CH₂Cl₂ (10 mL) was cooled to -78°C, and treated with DIBAL-H (1M, 5 mL). The reaction was stirred at -60°C for 3 h, and quenched with a suspension of NaF/H₂O (1 g/l mL). The resulting mixture was filtered and the filtrate was concentrated to give 2-[(N-Boc)amino]-4-hydroxymethylthiazole as a solid.
**Step E.** A solution of 2-[(N-Boc)amino]-4-hydroxymethylthiazole (1 mmole) in DMF (10 mL) was cooled to 0°C, and treated with NaH (1.1 mmole). The mixture was stirred at room temperature for 30 min, then phosphonomethyl trifluoromethanesulfonate (1.1 mmole) was added. After stirring at room temperature for 4 h, the reaction was evaporated to dryness. Chromatography of the residue gave 2-[(N-Boc)amino]-4-diethylphosphonomethoxylmethylthiazole as a solid.
**Step F.** 2-[(N-Boc)amino]-4-diethylphosphonomethoxylmethylthiazole was subjected to Step C of Example 3 to give 2-amino-4-phosphonomethoxymethylthiazole **(8.3)** as a solid. Anal. calcd. for C₅H₉N₂O₄PS + 0.16 HBr + 0.1 MeOH: C: 25.49; H: 4.01; N: 11.66. Found: C: 25.68; H: 3.84; N: 11.33.

### Example 9.

### Preparation of 2-carbamoyl-4-[2-(5-phosphono)furanyl]thiazoles.

**Step A.** A solution of 2-ethoxycarbonyl-5-isobutyl-4-[2-(5-diethylphosphono)furanyl]thiazole (1 mmole) in saturated methanolic ammonia solution at 25 °C for 12 h. Evaporation and chromatography gave 2-carbamoyl-5-isobutyl-4-[2-(5-diethylphosphono)furanyl]thiazole as a white solid.
**Step B.** 2-Carbamoyl-5-isobutyl-4-[2-(5-diethylphosphono)furanyl]thiazole was subjected to Step C of Example 3 to give 2-carbamoyl-5-isobutyl-4-[2-(5-phosphono)furanyl]thiazole (**9.1**) as a solid. mp 185 - 186 °C. Anal. calcd. for C₁₂H₁₅N₂O₅PS: C: 43.64; H: 4.58; N: 8.48. Found: C: 43.88; H: 4.70; N: 8.17.
   The following compound was prepared according to this procedure:
   (**9.2**) 2-Carbamoyl-4-[2-(5-phosphono)furanyl]thiazole. mp 195 - 200 °C. Anal. calcd. for C₈H₇N₂O₅PS + 0.25H₂O: C: 34.48; H: 2.71; N: 10.05. Found: C: 34.67; H: 2.44; N: 9.84.
   2-Ethoxycarbonyl-4-[2-(5-diethylphosphono)furanyl]thiazoles can also be converted to other 2-substituted 4-[2-(5-phosphono)furanyl]thiazoles.
**Step C.** A solution of 2-ethoxycarbonyl-4-[2-(5-diethylphosphono)furanyl]thiazole (1 mmole) in methanol was treated with sodium borohydride (1.2 mmole) at 25 °C for 12 h. Extraction and chromatography gave 2-hydroxymethyl -4-[2-(5-diethylphosphono)furanyl]thiazole.
**Step D.** 2-Hydroxymethyl-4-[2-(5-diethylphosphono)furanyl]-thiazole was subjected to Step C of Example 3 to give 2-hydroxymethyl-4-[2-(5-phosphono)furanyl]thiazole **(9.3).** mp 205-207 °C. Anal. calcd. for C₈H₈NO₅PS+ 0.25H₂O: C: 36.16; H: 3.22; N: 5.27. Found: C: 35.98; H: 2.84; N: 5.15.
   The following compound was prepared according to this procedure:
   **(9.4)** 2-Hydroxymethyl-5-isobutyl-4-[2-(5-phosphono)furanyl]thiazole. mp 160-170 °C. Anal. calcd. for C₁₂H₁₆NO₅PS + 0.75HBr: C: 38.13; H: 4.47; N: 3.71. Found: C: 37.90; H: 4.08; N: 3.60.
**Step E** A solution of 2-hydroxymethyl-5-isobutyl-4-[2-(5-diethylphosphono)furanyl]thiazole (1 mmole) in methylene chloride was treated with phosphorus tribromide (1.2 mmole) at 25 °C for 2 h. Extraction and chromatography gave 2-bromomethyl-5-isobutyl-4-[2-(5-diethylphosphono)furanyl]thiazole.
**Step F.** 2-Bromomethyl-5-isobutyl-4-[2-(5-diethylphosphono)furanyl]-thiazole was subjected to Step C of Example 3 to give 2-bromomethyl-5-isobutyl-4-[2-(5-phosphono)furanyl]thiazole (**9.5**). mp 161-163 °C. Anal. calcd. for C₁₂H₁₅BrNO₄PS + 0.25HBr: C: 35.99; H: 3.84; N: 3.50. Found: C: 36.01; H: 3.52; N: 3.37.
   The following compound was prepared according to this procedure:
   (**9.6**) 2-Bromomethyl-4-[2-(5-phosphono)furanyl]thiazole. mp > 250 °C. Anal. calcd. for C₈H₇BrNO₄PS: C: 29.65; H: 2.18; N: 4.32. Found: C: 29.47; H: 1.99; N: 4.16.
**Step G.** A solution of 2-hydroxymethyl-5-isobutyl-4-[2-(5-diethylphosphono)furanyl]thiazole (1 mmole) in methylene chloride was treated with thionyl chloride (1.2 mmole) at 25 °C for 2 h. Extraction and chromatography gave 2-chloromethyl-5-isobutyl-4-[2-(5-diethylphosphono)furanyl]thiazole.
**Step H.** 2-Chloromethyl-5-isobutyl-4-[2-(5-diethylphosphono)furanyl]-thiazole was subjected to Step C of Example 3 to give 2-chloromethyl-5-isobutyl -4-[2-(5-phosphono)furanyl]thiazole (**9.7**). mp 160-162 °C. Anal. calcd. for C₁₂H₁₅ClNO₄PS + 0.45HBr: C: 38.73; H: 4.18; N: 3.76. Found: C: 38.78; H: 4.14; N: 3.73.
**Step I.** A solution of 2-bromomethyl-5-isobutyl-4-[2-(5-diethylphosphono)furanyl]thiazole (1 mmole) in DMF was treated with potassium phthalimide (1.2 mmole) at 25 °C for 12 h. Extraction and chromatography gave 2-phthalimidomethyl-5-isobutyl-4-[2-(5-diethylphosphono)furanyl]thiazole.
**Step J.** 2-Phthalimidomethyl-5-isobutyl-4-[2-(5-diethylphosphono)furanyl]-thiazole (1 mmole) in ethanol was treated with hydrazine (1.5 mmole) at 25 °C for 12 h. Filtration, evaporation and chromatography gave 2-aminomethyl-5-isobutyl-4-[2-(5-diethylphosphono)furanyl]thiazole.
**Step K.** 2-Aminomethyl-5-isobutyl-4-[2-(5-diethylphosphono)furanyl]-thiazole was subjected to Step C of Example 3 to give 2-aminomethyl-5-isobutyl-4-[2-(5-phosphono)furanyl]thiazole (**9.8**). mp 235-237 °C. Anal. calcd. for C₁₂H₁₇N₂O₄PS + 0.205HBr: C: 43.30; H: 5.21; N: 8.41. Found: C: 43.66; H: 4.83; N: 8.02.

According to the above procedures or in some cases with some minor modifications of the above procedures, the following compounds were prepared:
**(9.9)** 2-Carbamoyl-5-cyclopropyl-4-[2-(5-phosphono)furanyl]thiazole. Anal. calcd for C₁₁H₁₁N₂O₅PS+ 0.15HBr: C: 40.48; H: 3.44; N: 8.58. Found: C: 40.28; H: 3.83; N: 8.34.
**(9.10)** 2-Carbamoyl-5-ethyl-4-[2-(5-phosphono)furanyl]thiazole. Anal. calcd for C₁₀H₁₁N₂O₅PS + 0.75H₂O: C: 38.04; H: 3.99; N: 8.87. Found: C: 37.65; H: 3.93; N: 8.76.

### Example 10.

### Preparation of 4-[2-(5-phosphono)furanyl]oxazoles and 4-[2-(5-phosphono)furanyl]imidazoles

**Step A.** A solution of 5-diethylphosphono-2-[(2-bromo-4-methyl-1-oxo)pentyl]furan (1 mmole) in t-BuOH was treated with urea (10 mmole) at reflux for 72 h. Filtration, evaporation and chromatography gave 2-amino-5-isobutyl-4-[2-(5-diethylphosphono)furanyl]oxazole, and 2-hydroxy-5-isobutyl-4-[2-(5-diethylphosphono)furanyl]imidazole.
**Step B.** 2-Amino-5-isobutyl-4-[2-(5-diethylphosphono)furanyl]oxazole was subjected to Step C of Example 3 to give 2-amino-5-isobutyl-4-[2-(5-phosphono)furanyl]oxazole (10.1). mp 250 °C (decomp.). Anal. Calcd. for C₁₁H₁₅N₂O₅P: C: 46.16; H: 5.28 ; N: 9.79. Found: C: 45.80; H: 5.15; N: 9.55.
**Step C.** 2-Hydroxy-5-isobutyl-4-[2-(5-diethylphosphono)furanyl]imidazole was subjected to Step C of Example 3 to give 2-hydroxy-5-isobutyl-4-[2-(5-phosphono)furanyl]imidazole **(10.14).** mp 205 °C (decomp). Anal. Calcd. for C₁₁H₁₅N₂O₅P: C: 46.16; H: 5.28 ; N: 9.79. Found: C: 45.80; H: 4.90 ; N: 9.73.
   Alternatively 4-[2-(5-phosphono)furanyl]oxazoles and 4-[2-(5-phosphono)furanyl]imidazoles can be prepared as following:
**Step D.** A solution of 5-diethylphosphono-2-[(2-bromo-4-methyl-1-oxo)pentyl]furan (1 mmole) in acetic acid was treated with sodium acetate (2 mmole) and ammonium acetate (2 mmole) at 100 °C for 4 h. Evaporation and chromatography gave 2-methyl-5-isobutyl-4-[2-(5-diethylphosphono)furanyl]-oxazole, 2-methyl-4-isobutyl-5-[2-(5-diethylphosphono)furanyl]oxazole and 2-methyl-5-isobutyl-4-[2-(5-diethylphosphono)furanyl]imidazole.
**Step E.** 2-Methyl-5-isobutyl-4-[2-(5-diethylphosphono)furanyl]oxazole, -2-methyl-4-isobutyl-5-[2-(5-diethylphosphono)furanyl]oxazole and 2-methyl-5-isobutyl-4-[2-(5-diethylphosphono)furanyl]imidazole were subjected to Step C of Example 3 to give the following compounds:
   **(10.18)** 2-Methyl-4-isobutyl-5-[2-(5-phosphono)furanyl]oxazole hydrogen bromide. mp > 230 °C ; Anal. Calcd. for C₁₂H₁₇BrNO₅P + 0.4H₂O: C: 38.60; H: 4.81; N: 3.75. Found: C: 38.29; H: 4.61 ; N: 3.67.
   **(10.19)** 2-Methyl-5-isobutyl-4-[2-(5-phosphono)furanyl]oxazole hydrogen bromide. Anal. Calcd. for C₁₂H₁₇BrNO₅P: C: 39.36; H: 4.68 ; N: 3.83. Found: C: 39.33; H: 4.56; N: 3.85. **(10.21)** 2-Methyl-S-isobutyl-4-[2-(5-phosphono)furanyl]imidazole hydrogen bromide. Anal. Calcd. for C₁₂H₁₈BrN₂O₄P + 0.2NH₄Br: C: 37.46 ; H: 4.93; N: 8.01. Found: C: 37.12; H: 5.11; N: 8.28.
   Alternatively 4-[2-(5-phosphono)furanyl]imidazoles can be prepared as following:
**Step F.** A solution of 5-diethylphosphono-2-(bromoacetyl)furan (1 mmole) in ethanol was treated with trifluoroacetamidine (2 mmole) at 80 °C for 4 h. Evaporation and chromatography gave 2-trifluoromethyl-4-[2-(5-diethylphosphono)furanyl]imidazole as an oil.
**Step G.** 2-Trifluoromethyl-4-[2-(5-diethylphosphono)furanyl]imidazole was subjected to Step C of Example 3 to give 2-trifluoromethyl-4-[2-(5-phosphono)-furanyl]imidazole (**10.22**). mp 188 °C (dec.); Anal. Calcd. for C₈H₆F₃N₂O₄P + 0.5HBr: C: 29.79 ; H: 2.03; N: 8.68. Found: C: 29.93; H: 2.27; N: 8.30.
   Alternatively 4,5-dimethyl-1-isobutyl-2-[2-(5-phosphono)furanyl]-imidazole can be prepared as following:
**Step H.** A solution of 5-diethylphosphono-2-furaldehyde (1 mmole), ammonium acetate (1.4 mmole), 3,4-butanedione (3 mmole) and isobutylamine (3 mmole) in glacial acetic acid was heated at 100 °C for 24 h. Evaporation and chromatography gave 4,5-dimethyl-1-isobutyl-2-[2-(5-diethylphosphono)furanyl]imidazole as an yellow solid.
**Step I.** 4,5-Dimethyl-1-isobutyl-2-[2-(5-diethylphosphono)furanyl]-imidazole was subjected to Step C of Example 3 to give 4,5-dimethyl-1-isobutyl-2-[2-(5-phosphono)furanyl]imidazole (10.23); Anal. Calcd. for C₁₃H₁₉N₂O₄P + 1.35HBr: C: 38.32; H: 5.03; N: 6.87. Found: C: 38.09; H: 5.04; N: 7.20.

According to the above procedures or in some cases with some minor modifications of the above procedures, the following compounds were prepared:
(**10.2**) 2-Amino-5-propyl-4-[2-(5-phosphono)furanyl]oxazole. mp 250 °C (decomp.); Anal. Calcd. for C₁₀H₁₃N₂O₅P: C: 44.13; H: 4.81 ; N: 10.29. Found: C: 43.74; H: 4.69; N: 9.92.
(**10.3**) 2-Amino-5-ethyl-4-[2-(5-phosphono)furanyl]oxazole. Anal. Calcd. for C₉H₁₁N₂O₅P + 0.4H₂O: C: 40.73; H: 4.48 ; N: 10.56. Found: C: 40.85; H: 4.10 ; N: 10.21.
**(10.4)** 2-Amino-5-methyl-4-[2-(5-phosphono)furanyl]oxazoIe. Anal. Calcd. for C₈H₉N₂O₅P + 0.1H₂O: C: 39.07 ; H: 3.77 ; N: 11.39. Found: C: 38.96; H: 3.59; N: 11.18.
**(10.5)** 2-Amino-4-[2-(5-phosphono)furanyl]oxazole. Anal. Calcd. for C₇H₇N₂O₅P + 0.6H₂O: C: 34.90; H: 3.43 ; N: 11.63. Found: C: 34.72; H: 3.08 ; N: 11.35.
**(10.6)** 2-Amino-5-isobutyl-4-[2-(5-phosphono)furanyl]oxazole hydrogen bromide. Anal. Calcd. for C₁₁H₁₆N₂O₅BrP + 0.4H₂O: C: 35.29; H: 4.52 ; N: 7.48. Found: C: 35.09; H: 4.21 ; N: 7.34.
(**10.7**) 2-Amino-5-phenyl-4-[2-(5-phosphono)furanyl]oxazole. Anal. Calcd. for C₁₃H₁₁N₂O₅P: C: 50.99; H: 3.62 ; N: 9.15. Found: C: 50.70; H: 3.43 ; N: 8.96.
**(10.8)** 2-Amino-5-benzyl-4-[2-(5-phosphono)furanyl]oxazole. Anal. Calcd. for C₁₄H₁₃N₂O₅P + 1.1H₂O: C: 49.45; H: 4.51 ; N: 8.24. Found: C: 49.35; H: 4.32 ; N: 8.04.
(**10.9**) 2-Amino-5-cyclohexylmethyl-4-[2-(5-phosphono)furanyl]oxazole. Anal. Calcd. for C₁₄H₁₉N₂O₅P + 0.3H₂O: C: 50.70; H: 5.96 ; N: 8.45. Found: C: 50.60; H: 5.93 ; N: 8.38.
(**10.10**) 2-Amino-5-allyl-4-[2-(5-phosphono)furanyl]oxazole. Anal. Calcd. for C₁₀H₁₁N₂O₅P + 0.4HBr + 0.3H₂O: C: 39.00; H: 3.93 ; N: 9.10. Found: C: 39.31; H: 3.83; N: 8.76.
(**10.11**) 5-Isobutyl-4-[2-(5-phosphono)furanyl]oxazole. Anal. Calcd. for C₁₁H₁₄NO₅P: C: 48.72; H: 5.20 ; N: 5.16. Found: C: 48.67; H: 5.02 ; N: 5.10.
**(10.12)** 2-Amino-5-butyl-4-[2-(5-phosphono)furanyl]oxazole. Anal. Calcd. for C₁₁H₁₅N₂O₅P + 0.2H₂O: C: 45.59; H: 5.36 ; N: 9.67. Found: C: 45.32; H: 5.29; N: 9.50.
**(10.13)** 5-Isobutyl-4-[2-(5-phosphono)furanyl]oxazole-2-one. Anal. Calcd. for C₁₁H₁₄NO₆P + 0.39HBr: C: 41.45; H: 4.55 ; N: 4.39. Found: C: 41.79; H: 4.22 ; N: 4.04.
**(10.15)** 5-Cyclohexylmethyl-2-hydroxy-4-[2-(5-phosphono)furanyl]imidazole. Anal. Calcd. for C₁₄H₁₉N₂O₅P + 0.05HBr: C: 50.90; H:5.81; N:8.48. Found: C: 51.06; H: 5.83; N: 8.25.
**(10.16)** 5-Butyl-2-hydroxy-4-[2-(5-phosphono)furanyl]. Anal. Calcd. for C₁₁H₁₅N₂O₅P + 0.2H₂O: C: 45.59; H: 5.36; N: 9.67. Found: C: 45.77; H: 5.34; N: 9.39.
**(10.17)** 5-Benzyl-2-hydroxy-4-[2-(5-phosphono)furanyl]imidazole. Anal. Calcd. for C₁₄H₁₃N₂O₅P: C: 52.51; H: 4.09; N: 8.75. Found: C: 52.29; H: 4.15; N: 8.36.
**(10.20)** 2-Methyl-5-propyl-4-[2-(5-phosphono)furanyl]imidazole hydrogen bromide. Anal. Calcd. for C₁₁H₁₆BrN₂O₄P + 0.5H₂O: C: 36.69; H: 4.76; N: 7.78. Found: C: 36.81; H: 4.99 ; N: 7.42.
(**10.24**) 2-Amino-5-(2-thienylmethyl)-4-[2-(5-phosphono)furanyl]oxazole. Anal.
calcd for C₁₂H₁₁N₂O₅PS + 0.9HBr: C: 36.12; H: 3.01; N: 7.02. Found: C: 36.37; H: 2.72; N: 7.01.
**(10.25)** 2-Dimethylamino-5-isobutyl-4-[2-(5-phosphono)furanyl]oxazole hydrogen bromide. Anal. Calcd for C₁₃H₂₀BrN₂O₅P + 0.05HBr: C: 39.11; H: 5.06; N: 7.02. Found: C: 39.17; H: 4.83; N: 6.66
**(10.26)** 2-Isopropyl-5-isobutyl-4-[2-(5-phosphono)furanyl]oxazole. Anal. Calcd for C₁₄H₂₀NO₅P + 0.8HBr: C: 44.48; H: 5.55; N: 3.71. Found: C: 44.45; H: 5.57; N: 3.73.
**(10.27)** 2-Amino-5-ethoxycarbonyl-4-[2-(5-phosphono)furanyl]oxazole. mp 245 °C (decomp.). Anal. Calcd for C₁₀H₁₁N₂O₇P: C: 39.75; H: 3.67; N: 9.27. Found: C: 39.45; H: 3.71; N: 8.87
**(10.28)** 2-Methylamino-5-isobutyl-4-[2-(5-phosphono)furanyl]oxazole hydrogen bromide. Anal. Calcd for C₁₂H₁₈BrN₂O₅P + 0.7H₂O: C: 36.60; H: 4.97; N: 7.11. Found: C: 36.50; H: 5.09; N: 7.04.
**(10.29)** 2-Ethyl-5-isobutyl-4-[2-(5-phosphono)furanyl]oxazole hydrogen bromide. Anal. Calcd for C₁₃H₁₉BrNO₅P: C: 41.07; H: 5.04; N: 3.68. Found: C: 41.12; H: 4.84; N: 3.62.
**(10.30)** 2-Ethylamino-S-isobutyl-4-[2-(5-phosphono)furanyl]oxazole hydrogen bromide. Anal. Calcd for C₁₃H₂₀BrN₂O₅P: C: 39.51; H: 5.10; N: 7.09. Found: C: 39.03; H: 5.48; N: 8.90.
**(10.31)** 2-Vinyl-5-isobutyl-4-[2-(5-phosphono)furanyl]oxazole. Anal. Calcd for C₁₃H₁₆NO₅P + 0.25HBr: C: 49.18; H: 5.16; N: 4.41. Found: C: 48.94; H: 5.15; N: 4.40.
**(10.32)** 2-Amino-5-pentyl-4-[2-(5-phosphono)furanyl]oxazole. Anal. Calcd for C₁₂H₁₇N₂O₅P + 0.5H₂O: C: 46.61; H: 5.87; N: 9.06. Found: C: 46.38; H: 5.79; N: 9.07.
**(10.33)** 5-Pentyl-2-hydroxy-4-[2-(5-phosphono)furanyl]imidazole. Anal. Calcd. for C₁₂H₁₇N₂O₅P: C: 48.00; H: 5.71; N: 9.33. Found: C: 48.04; H: 5.58; N: 9.26.
**(10.45)** 2-Amino-5-methylthio-4-[2-(5-phosphono)furanyl]oxazole. mp 196 °C (decomp). Anal. calcd. for C₈H₉N₂O₅PS: C:34.79; H:3.28; N: 10.14. Found: C: 34.60; H: 2.97; N: 10.00.
**(10.35)** 2-Amino-5-benzyloxycarbonyl-4-[2-(5-phosphono)furanyl]oxazole. mp 230 °C (decomp). Anal. calcd for C₁₅H₁₃N₂O₇P+ 0.7H₂O: C: 47.81; H: 3.85; N: 7.43. Found: C: **47.85;** H: 3.88; N: 7.21.
**(10.36)** 2-Amino-5-isopropyloxycarbonyl-4-[2-(5-phosphono)furanyl]oxazole. mp 221 °C (decomp). Anal. calcd for C₁₁H₁₃N₂O₇P + 0.9H₂O: C: 39.75; H: 4.49; N: 8.43. Found: C: 39.72; H: 4.25; N: 8.20.
**(10.37)** 2-Amino-5-methoxycarbonyl-4-[2-(5-phosphono)furanyl]oxazole. mp 240 °C (decomp). Anal. calcd for C₉H₉N₂O₇P + 0.3H₂O + 0.1Acetone: C: 37.31; H: 3.43; N: 9.36. Found: C: 37.37; H: 3.19; N: 9.01.
**(10.38)** 2-Amino-5-[(N-methyl)carbamoyl]-4-[2-(5-phosphono)furanyl]oxazole. mp 235 °C (decomp). Anal. calcd for C₉H₁₀N₃O₆P: C: 37.64; H: 3.51; N: 14.63. Found: C: 37.37; H: 3.22; N: 14.44.
**(10.39)** 2-Amino-5-ethylthiocarbonyl-4-[2-(5-phosphono)furanyl]oxazole. mp 225 °C (decomp). Anal. calcd for C₁₀H₁₁N₂O₆PS: C: 37.74; H: 3.48; N: 8.80. Found: C: 37.67; H: 3.27; N: 8.46.
**(10.40)** 2-Amino-5-isopropylthio-4-[2-(5-phosphono)furanyl]oxazole. Anal. calcd for C₁₀H₁₃N₂O₅PS + 0.2HBr: C: 37.48; H: 4.15; N: 8.74. Found: C: 37.39; H: 4.11; N: 8.56. **(10.41)** 2-Amino-5-phenylthio-4-[2-(5-phosphono)furanyl]oxazole. Anal. calcd for C₁₃H₁₁N₂O₅PS + 0.25 HBr: C: 43.55; H: 3.16; N: 7.81. Found: C: 43.82 H: 3.28; N: 7.59. **(10.42)** 2-Amino-5-ethylthio-4-[2-(5-phosphono)furanyl]oxazole. Anal. calcd for C₉H₁₁N₂O₅PS + 0.85HBr: C: 30.11; H: 3.33; N: 7.80. Found: C: 30.18; H: 3.44; N: 7.60.
**(10.43)** 2-Amino-5-propylthio-4-[2-(5-phosphono)furanyl]oxazole. Anal. calcd for C₁₀H₁₃N₂O₅ + H₂O: C: 37.27; H: 4.69; N: 8.69; H₂O: 5.59. Found: C: 37.27; H: 4.67; N: 8.60; H₂O: 5.66.
**(10.44)** 2-Amino-5-tert-butylthio-4-[2-(5-phosphono)furanyl]oxazole. Anal. calcd for C₁₁H₁₅N₂O₅PS + 0.25HBr: C: 39.03; H: 4.54; N: 8.28. Found: C: 39.04; H: 4.62; N: 8.06. **(10.34)** 4,5-Dimethyl-2-[2-(5-phosphono)furanyl]imidazole. Anal. Calcd. for C₉H₁₁N₂O₄P + 1.25 H₂O: C: 40.84; H:5.14; N: 10.58. Found: C: 41.02; H: 5.09; N: 10.27.

### Example 11.

### Preparation of N-alkylated 4-[2-(5-phosphono)furanyl]imidazoles and 4-[2-(5-phosphono)furanyl]oxazoles.

**Step A.** A suspension of cesium carbonate (1.5 mmole) and 2-methyl-5-isobutyl-4-[2-(5-diethylphosphono)furanyl]imidazole (1 mmole) in DMF was treated with iodomethane (1.5 mmole) at 25 °C for 16 h. Extraction and chromatography gave 1,2-dimethyl-4-isobutyl-5-[2-(5-diethylphosphono)-furanyl]imidazole and 1,2-dimethyl-5-isobutyl-4-[2-(5-diethylphosphono)-furanyl]imidazole.
**Step B.** 1,2-Dimethyl-4-isobutyl-5-[2-(5-diethylphosphono)furanyl]-imidazole and 1,2-dimethyl-5-isobutyl-4-[2-(5-diethylphosphono)furanyl]-imidazole were subjected to Step C of Example 3 to give the following compounds:
   **(11.1)** 1, 2-Dimethyl-5-isobutyl-4-[2-(5-phosphono)furanyl]imidazole hydrogen bromide. Anal. Calcd. for C₁₃H₂₀N₂O₄PBr + 0.8H₂O: C: 39.67; H: 5.53; N: 7.12. Found: C: 39.63; H: 5.48 ; N: 7.16.

### Example 12.

### Preparation of 2-[2-(6-phosphono)pyridyl]pyridine.

**Step A.** A solution of 2,2'-bipyridyl (1 mmole) in dichloromethane was treated with m-chloroperoxybenzoic acid (2 mmole) at 0 °C, and the reaction mixture was stirred at 25 °C for 2 h. Extraction and chromatography gave 2,2'-bipyridyl-N-oxide.
**Step B.** (Redmore, D., *J*. *Org. Chem*., **1970**, *35*, 4114) A solution of 2,2'-bipyridyl-N-oxide methyl ether (1 mmole, prepared from dimethyl sulfate and 2,2'-bipyridyl-N-oxide in diethyl phosphite) was added slowly at -30 °C to a solution of n-butyl lithium (1mmole) in diethyl phosphite at -30 °C. The resulting reaction mixture was stirred at 25 °C for 12 h. Extraction and chromatography gave 2-[2-(6-diethylphosphono)pyridyl]pyridine.
**Step C.** 2-[2-(6-Diethylphosphono)pyridyl]pyridine was subjected to Step C of Example 3 to give 2-[2-(6-phosphono)pyridyl]pyridine (**12.1**). mp 158 - 162 °C. Anal. Calcd. for C₁₀H₉N₂O₃P + 0.5H₂O + 0.1HBr: C: 47.42; H: 4.02; N: 11.06. Found: C: 47.03; H: 3.67; N: 10.95.

### Example 13.

### Preparation of 4,6-dimethyl-2-(phosphonomethoxymethyl)pyridine.

**Step A.** A solution of 2,4,6-collidine (1 mmole) in carbon tetrachloride was treated with NBS (5 mmole) and dibenzoyl peroxide (0.25 mmole) at 80 °C for 12 h. The reaction mixture was cooled to 0 °C and the precipitate was filtered. The filtrate was concentrated under vacuum. Chromatography gave 2-bromomethyl-4,6-dimethylpyridine.
**Step B.** A solution of diethyl hydroxymethylphosphonate (1 mmole) in toluene was treated with sodium hydride (1.1 mmole) at 0 °C, and after 15 min 2-bromomethyl-4,6-dimethylpyridine (1 mmole) was added. After 3 h the reaction mixture was subjected to extraction and chromatography to give 2-diethylphosphonomethyl-4,6-dimethylpyridine.
**Step C.** 2-Diethylphosphonomethyl-4,6-dimethylpyridine was subjected to Step C of Example 3 to give 4,6-dimethyl-2-(phosphonomethoxymethyl)pyridine **(13.1).** mp 109 - 112 °C. Anal. Calcd. for C₉H₁₄NO₄P + 1.0H₂O + 0.5HBr: C: 37.32; H: 5.74; N: 4.84. Found: C: 37.18; H: 5.38; N: 4.67.

The following compound was prepared similarly:
**(13.2)** 2-Amino-4-methyl-5-propyl-6-phosphonomethoxymethylpyrimidine. mp 153 - 156 °C. Anal. Calcd. for C₁₀H₁₈N₃O₄P + 1.25H₂O + 1.6HBr: C: 28.11; H: 5.21; N: 9.84. Found: C: 28.25; H: 4.75; N: 9.74.

### Example 14.

### Preparation of diethyl 5-tributylstannyl-2-furanphosnhonate (14).

A solution of diethyl 2-furanphosphonate (1 mmole, prepared as in Step C of Example 1) in THF was cooled at -78 °C and cannulated to a solution of lithium N-isopropyl-N-cyclohexylamide in THF at -78 °C over 15 min. The resulting mixture was stirred at -78 °C for 2 h and cannulated into a solution of tributyltin chloride (1 mmole) in THF at -78 °C over 20 min. The mixture was then stirred at -78 °C for 1 h, and at 25 °C for 12 h. Extraction and chromatography gave compound (14) as a light yellow oil.

### Example 15.

### Preparation of 6-[2-(5-phosphono)furanyl]pyridines.

**Step A.** A solution of 2,6-dichloropyridine (120mmol) in ethanol.was treated with aqueous ammonia solution (28 %, excess) at 160 - 165 °C for 60 h in a sealed tube. Extraction and chromatography gave 2-amino-6-chloropyridine as a white solid.
**Step B.** A solution of 2-amino-6-chloropyridine (1 mmole) and compound 14 (1 mmole) in p-xylene was treated with tetrakis(triphenylohosphine) palladium (0.05 mmole) at reflux for 12 h. Extraction and chromatography gave 2-amino-6-[2-(5-diethylphosphono)furanyl]pyridine as a light yellow solid.
**Step C.** 2-Amino-6-[2-(5-diethylphosphono)furanyl]pyridine was subjected to Step C of Example 3 to give 2-amino-6-[2-(5-phosphono)furanyl]pyridine **(15.1).** mp 186 - 187 °C. Anal. Calcd. for C₉H₉N₂O₄P + 0.4HBr: C: 39.67; H: 3.48; N: 10.28. Found: C: 39.95; H: 3.36; N: 10.04.
**Step D.** A solution of 2-amino-6-[2-(5-diethylphosphono)furanyl]pyridine (1 mmole) in acetic acid was treated with a solution of bromine in acetic acid (1N, 1 mmole) at 25 °C for 0.5 h. Evaporation and chromatography gave 2-amino-5-bromo-6-[2-(5-diethylphosphono)furanyl]pyridine and 2-amino-3,5-dibromo-6-[2-(5-diethylphosphono)furanyl]pyridine.
**Step E.** 2-Amino-5-bromo-6-[2-(5-diethylphosphono)furanyl]pyridine and 2-amino-3,5-dibromo-6-[2-(5-diethylphosphono)furanyl]pyridine were subjected to Step C of Example 3 to give the following compounds:
   **(15.2)** 6-Amino-3-bromo-2-[2-(5-phosphono)furanyl]pyridine. Anal. Calcd. for C₉H₈BrN₂O₄P + 0.7H₂O + 0.9HBr + 0.12PhCH₃: C: 28.44; H: 2.73; N: 6.74. Found: C: 28.64; H: 2.79; N: 6.31.
   **(15.3)** 6-Amino-3,5-dibromo-2-[2-(5-phosphono)furanyl]pyridine. mp 233 - 235 °C. Anal. Calcd. for C₉H₇Br₂N₂O₄P + 1.2HBr: C: 21.84; H: 1.67; N: 5.66. Found: C: 21.90; H: 1.52; N: 5.30.
**Step F.** A solution of 2-amino-3,5-dibromo-6-[2-(5-diethylphosphono)-furanyl]pyridine (1 mmole) in DMF was treated with tributyl(vinyl)tin (1.2 mmole) and tetrakis(triphenylphosphine) palladium (0.2 mmole) at 85 °C for 4 h. Evaporation and chromatography gave 2-amino-3,5-bis(vinyl)-6-[2-(5-diethylphosphono)furanyl]pyridine.
**Step G.** A solution of 2-amino-3,5-bis(vinyl)-6-[2-(5-diethylphosphono)-furanyl]pyridine (1 mmole) in ethyl acetate was treated with palladium on carbon (10 %) at 25 °C under 1 atmosphere of hydrogen for 12 h. Filtration, evaporation and chromatography gave 2-amino-3,5-diethyl-6-[2-(5-diethylphosphono)furanyl]pyridine.
**Step H.** 2-Amino-3,5-diethyl-6-[2-(5-diethylphosphono)furanyl]pyridine was subjected to Step C of Example 3 to give 2-amino-3,5-diethyl-6-[2-(5-phosphono)furanyl]pyridine **(15.4).** mp 217 - 218 °C. Anal. Calcd. for C₁₃H₁₇N₂O₄P + 0.7H₂O + 1.0HBr: C: 40.06; H: 5.02; N: 7.19. Found: C: 40.14; H: 4.70; N: 6.87.
**Step I.** A solution of 2-amino-6-picoline (1 mmole) in 48 % hydrobromic acid (4.4 mmole) was treated with bromine (3 mmole) at 0 °C for 1 h. An aqueous solution of sodium nitrite (2.5 mmole) was then added and the reaction mixture was stirred at 0 °C for 0.5 h. An aqueous solution of sodium hydroxide (9.4 mmole) was then added and the reaction mixture was stirred at 25 °C for 1 h. Extraction and chromatography gave 2,3-dibromo-6-picoline and 2,3,5-tribromo-6-picoline.
**Step J.** 2,3-Dibromo-6-picoline was subjected to Step B of Example 15 and followed by Step C of Example 3 to give 5-bromo-2-methyl-6-[2-(5-phosphono)furanyl]pyridine (**15.5**). mp 207 - 208 °C. Anal. Calcd. for C₁₀H₉BrNO₄P + 0.6HBr: C: 32.76; H: 2.64; N: 3.88. Found: C: 32.62; H: 2.95; N: 3.55.

Following compounds were prepared according to the above described procedures or with some minor modifications of these procedures using conventional chemistry.
(**15.6**) 2-[2-(5-Phosphono)furanyl]pyridine. mp 220 - 221 °C. Anal. Calcd. for C₉H₈NO₄P + 0.1H₂O + 0.45HBr: C: 41.05; H: 3.31; N: 5.32. Found: C: 41.06; H: 3.10; N: 5.10.
(**15.7**) 2-Amino-3-nitro-6-[2-(5-phosphono)furanyl]pyridine. mp 221 - 222 °C. Anal. Calcd. for C₉H₈N₃O₆P + 0.55HBr + 0.02PhCH₃: C: 33.12; H: 2.65; N: 12.68. Found: C: 33.22; H: 2.43; N: 12.26.
**(15.8)** 2,3-Diamino-6-[2-(5-phosphono)furanyl]pyridine. mp 150 - 153 °C. Anal. Calcd. for C₉H₁₀N₃O₄P+ 1.5HBr + 0.05PhCH₃: C: 29.46; H: 3.15; N: 11.02. Found: C: 29.50; H: 3.29; N: 10.60.
**(15.9)** 2-Chloro-6-[2-(5-phosphono)furanyl]pyridine. mp 94 - 96 °C. Anal. Calcd. for C₉H₇ClNO₄P + 0.25HBr: C: 38.63; H: 2.61; N: 5.01. Found: C: 38.91; H: 3.00; N: 5.07.
**(15.10)** 3,5-Dichloro-2-[2-(5-phosphono)furanyl]pyridine. mp 180 - 181 °C. Anal. Calcd. for C₉H₆Cl₂NO₄P + 0.7HBr: C: 31.61; H: 2.01; N: 3.94. Found: C: 31.69; H: 2.09; N: 3.89.
**(15.11)** 3-Chloro-5-trifluoromethyl-2-[2-(5-phosphono)furanyl]pyridine. mp 253 - 254 °C. Anal. Calcd. for C₁₀H₆ClF₃NO₄P: C: 36.67; H: 1.85; N: 4.28. Found: C: 36.69; H: 1.89; N: 4.30.
**(15.12)** 2-Amino-3-ethyl-6-[2-(5-phosphono)furanyl] pyridine. mp 220 - 221 °C. Anal. Calcd. for C₁₁H₁₃N₂O₄P + 0.6HBr + 0.2H₂O: C: 41.24; H: 4.40; N: 8.74. Found: C: 41.02; H: 4.57; N: 8.68.
**(15.13)** 6-Amino-3-ethyl-2-[2-(5-phosphono)furanyl] pyridine. Anal. Calcd. for C₁₁H₁₃N₂O₄P + 1.0HBr + 0.3H₂O: C: 37.27; H: 4.15; N: 7.90. Found: C: 37.27; H: 4.19; N: 7.51.
**(15.14)** 6-Amino-3-propyl-2-[2-(5-phosphono)furanyl] pyridine. mp 252 - 253 °C. Anal. Calcd. for C₁₂H₁₅N₂O₄P + 1.0HBr + 1.0H₂O + 0.32PhCH₃: C: 41.65; H: 5.05; N: 6.82. Found: C: 41.97; H: 5.19; N: 6.83.
**(15.15)** 2,4-Dimethyl-3-bromo-6-[2-(5-phosphono)furanyl]pyridine. mp 232 - 233 °C. Anal. Calcd. for C₁₁H₁₁BrNO₄P + 0.45HBr: C: 35.85; H: 3.13; N: 3.80. Found: C: 35.98; H: 3.10; N: 3.71.
**(15.16)** 2-Chloro-4-amino-6-[2-(5-phosphono)furanyl]pyridine. Anal. Calcd. for C₉H₈N₂O₄PCl + HBr + 0.5 H₂O + MeOH: C: 30.99; H: 3.38; N: 7.23. Found: C: 31.09; H: 3.21; N: 6.96.
**(15.17)** 3-Hydroxyl-2-[2-(5-phosphono)furanyl]pyridine. Anal. Calcd. for C₉H₈NO₅P + 1.1HBr + 0.3 CH₃Ph: C: 37.26; H: 3.24; N: 3.91. Found: C: 37.66; H: 3.55; N: 3.84.
**(15.19)** 2-Amino-3-cyclopropyl-6-[2-(5-phosphono)furanyl]pyridine. Anal. Calcd. for C₁₂H₁₃N₂O₄PCl + HBr + 0.4 H₂O: C: 39.13; H: 4.05; N: 7.61. Found: C: 39.06; H: 3.85; N: 7.37.
**(15.20)** 2-Amino-5-cyclopropyl-6-[2-(5-phosphono)furanyl]pyridine. Anal. Calcd. for C₁₂H₁₃N₂O₄P + HBr + 0.7 CH₃Ph: C: 47.69; H: 4.64; N: 6.58. Found: C: 47.99; H: 4.62; N: 6.91.
**(15.21)** 5-Amino-2-methoxy-6-[2-(5-phosphono)furanyl]pyridine. Anal. Calcd. for C₁₀H₁₁N₂O₅P + 0.2 H₂O: C: 43.87; H: 4.20; N: 10.23. Found: C: 43.71; H: 3.77; N: 9.77.
**(15.22)** 2-Methyl-5-cyano-6-[2-(5-phosphono)furanyl]pyridine. Anal. Calcd. for C₁₁H₉N₂O₄P + 0.75 HBr + 0.5 H₂O + 0.5 MePh: C: 45.84; H: 3.91; N: 7.37. Found: C: 45.93; H: 3.56; N: 7.36.
**(15.23)** 2-Amino-3,5-bis(cyano)-4-methyl-6-[2-(5-phosphono)furanyl]pyridine. Anal. Calcd. for C₁₂H₉N₄O₄P + 0.7 H₂O: C: 45.49; H: 3.31; N: 17.68. Found: C: 45.48; H: 3.06; N: 17.51.
**(15.24)** 2-Chloro-4-cyano-6-[2-(5-phosphono)furanyl]pyridine. Anal. Calcd. for C₁₀H₆N₂O₄PCl: C: 42.20; H: 2.13; N: 9.84. Found: C: 41.95; H: 2.10; N: 9.47.

### Example 16.

### Preparation of 2-[2-(5-phosphono)furanyl]pyrimidines and 4-[2-(5-phosphono)furanyl]pyrimidines

**Step A.** A solution of 5-diethylphosphono-2-[(1-oxo)pentyl]furan in N, N-dimethylformamide dimethyl acetal was heated at reflux for 12 h. Evaporation and chromatography gave diethyl 5-(2-propyl-3-N,N-dimethylamino)acryloyl-2-furanphosphonate.
**Step B**. A solution of diethyl 5-(2-propyl-3-N,N-dimethylamino)acryloyl-2-furanphosphonate (1 mmole) in ethanol was treated with guanidine hydrogen chloride (1.2 mmole) and sodium ethoxide (1 mmole) at 80 °C for 12 h. The reaction mixture was evaporated, and residue was dissolved in water. The aqueous solution was neutralized with HCl (2 N), and concentrated under reduced pressure. The residue was co-evaporated with toluene to give 2-amino-5-propyl-4-[2-(5-ethylphosphono)-furanyl]pyrimidine as a yellow solid. **Step C.** 2-Amino-5-propyl-4-[2-(5-ethylphosphono)furanyl]pyrimidine (1 mmole) and thionyl chloride was heated at reflux for 2 h. The reaction mixture was evaporated to dryness and the residue was dissolved in methylene chloride, and treated with excess pyridine and ethanol at 25 °C for 12 h. Evaporation and chromatography gave 2-amino-5-propyl-4-[2-(5-diethylphosphono)furanyl]pyrimidine.
**Step D.** 2-Amino-5-propyl-4-[2-(5-diethylphosphono)furanyl]pyrimidine was subjected to Step C of Example 3 to give 2-amino-5-propyl-4-[2-(5-phosphono)furanyl]pyrimidine (**16.1**). mp 258 - 259 °C. Anal. Calcd. for C₁₁H₁₄N₃O₄P + 1.33H₂O: C: 43.01; H: 5.47; N: 13.68. Found: C: 43.18; H: 5.31; N: 13.30.
   The following compound was prepared according to this procedure:
   (**16.2**) 2-Amino-5-isobutyl-4-[2-(5-phosphono)furanyl]pyrimidine. mp 218 - 220 °C. Anal. Calcd. for C₁₂H₁₆N₃O₄P + 0.75HBr + 0.3PhCH₃: C: 43.92; H: 5.01; N: 10.90. Found: C: 44.02; H: 4.62 ; N: 10.69.
   Alternatively other 4-[2-(5-phosphono)furanyl]pyrimidines can be prepared according to the following procedures:
**Step E.** Compound 2.2 was subjected to Step A of Example 16 to give diethyl 5-(3-N,N-dimethylamino)acryloyl-2-furanphosphonate as an orange solid.
**Step F.** A solution of diethyl 5-(3-N,N-dimethylamino)acryloyl-2-furanphosphonate (1 mmole), sodium ethoxide ethanol solution (2 mmole) and guanidine hydrochloride (1.1 mmole) was heated at 55 °C for 2 h. The reaction mixture was cooled in an ice bath and was neutralized with 1N HCl. Evaporation and chromatography gave 2-amino-4-[2-(5-diethylphosphono)-furanyl]pyrimidine as a yellow solid.
**Step G.** 2-Amino-4-[2-(5-diethylphosphono)furanyl]pyrimidine was subjected to Step C of Example 3 to give 2-amino-4-[2-(5-phosphono)furanyl]-pyrimidine (**16.3**). mp > 230 °C. Anal. Calcd. for C₈H₈N₃O₄P + 0.75H₂O + 0.2HBr: C: 35.48; H: 3.61; N: 15.51. Found: C: 35.42; H: 3.80; N: 15.30.
**Step H.** A solution of 2-amino-4-[2-(5-diethylphosphono)furanyl]pyrimidine (1 mmole) in methanol and chloroform was treated with NBS (1.5 mmole) at 25 °C for 1 h. Extraction and chromatography gave 2-amino-5-bromo-4-[2-(5-diethylphosphono)furanyl]pyrimidine as a yellow solid.
**Step I.** 2-Amino-5-bromo-4-[2-(5-diethylphosphono)furanyl]pyrimidine was subjected to Steps F and G of Example 15 followed by Step C of Example 3 to give 2-amino-5-ethyl-4-[2-(5-phosphono)furanyl]pyrimidine (**16.4**). mp > 225 °C. Anal. Calcd. for C₁₀H₁₂N₃O₄P + 1.4H₂O + 0.2HBr + 0.25PhCH₃: C: 42.30; H: 5.14; N: 12.59. Found: C: 42.74; H: 4.94; N: 12.13.

The following compounds were prepared according to the above described procedures or with some minor modifications using conventional chemistry:
**(16.5)** 2-[2-(5-Phosphono)furanyl]pyrimidine. mp 194 - 196 °C. Anal. Calcd. for C₈H₇N₂O₄P + 0.1H₂O + 0.55HBr: C: 35.27; H: 2.87; N: 10.28. Found: C: 35.26; H: 2.83; N: 9.89.
**(16.6)** 2-Amino-6-methyl-4-[2-(5-phosphono)furanyl]pyrimidine. mp 238 - 239 °C. Anal. Calcd. for C₉H₁₀N₃O₄P + 0.9HBr: C: 32.96; H: 3.35; N: 12.81. Found: C: 33.25; H: 3.34; N: 12.46.
**(16.7)** 2-Methylthio-4-[2-(5-phosphono)furanyl]pyrimidine. mp 228 - 229 °C. Anal. Calcd. for C₉H₉N₂O₄PS + 0.5H₂O: C: 38.44; H: 3.58; N: 9.96. Found: C: 38.19; H: 3.25; N: 9.66.
**(16.8)** 2-Methyl-4-[2-(5-phosphono)furanyl]pyrimidine. mp 206 - 212 °C. Anal. Calcd. for C₉H₉N₂O₄P + 0.9H₂O + 0.25HBr: C: 34.05; H: 3.30; N: 8.82. Found: C: 34.02; H: 3.06; N: 8.75.
**(16.9)** 4,6-Dimethyl-5-bromo-2-[2-(5-phosphono)furanyl]pyrimidine. mp 251 - 252 °C. Anal. Calcd. for C₁₀H₁₀BrN₂O₄P: C: 36.06; H: 3.03; N: 8.41. Found: C: 35.89; H: 2.82; N: 8.11.
**(16.10)** 2-Amino-5-chloro-4-[2-(5-phosphono)furanyi]pyrimidine. Anal. Calcd.
for C₈H₇ClN₃O₄P + 0.5H₂O: C: 33.76; H: 2.83; N: 14.76. Found: C: 33.91; H: 2.86; N: 14.20.
**(16.11)** 2-Amino-6-methylthio-4-[2-(5-phosphono)furanyl]pyrimidine. Anal. Calcd. for C₉H₁₀N₃O₄PS + HBr: C: 29.36; H: 3.01; N: 11.41. Found: C: 29.63; H: 3.02; N: 11.27. **(16.12)** 2-Amino-5-bromo-6-methylthio-4-[2-(5-phosphono)furanyl]pyrimidine. Anal. Calcd. for C₉H₉N₃O₄PSBr + 0.8 HBr + 0.2 MePh: C: 27.80; H: 2.56; N: 9.35. Found: C: 27.74; H: 2.40; N: 8.94.
**(16.13)** 2-Amino-(4-morpholino)-4-[2-(5-phosphono)furanyl]pyrimidine. Mp > 230 °C. Anal. Calcd. for C₁₂H₁₅N₄O₅P + HBr + 0.05 MePh: C: 36.02; H: 4.01; N: 13.61. Found: C: 35.98; H: 4.04; N: 13.33.
**(16.14)** 6-Amino-4-chloro-2-[2-(5-phosphono)furanyl]pyrimidine. Mp > 230 °C. Anal. Calcd. for C₈H₇N₃O₄PCl + 0.5 H₂O: C: 33.76; H: 2.83; N: 14.76. Found: C: 33.83; H: 2.54; N: 14.48.

### Example 17.

### Preparation of 2-[2-(5-phosphono)furanyl]pyrazines and 2-[2-(5-phosphono)furanyl]triazines

**Step A.** The procedures described in Example 16 can also be applied to the synthesis of 2-[2-(5-phosphono)furanyl]pyrazine and 2-[2-(5-phosphono)furanyl]triazine analogs and in some cases with minor modifications of these procedures using conventional chemistry methods. The following compounds were prepared accordingly:
   **(17.1)** 2,5-Dimethyl-3-[2-(5-phosphono)furanyl]pyrazine. mp 212 - 213 °C. Anal. Calcd. for C₁₀H₁₁N₂O₄P + 0.75HBr: C: 38.15; H: 3.76; N: 8.90. Found: C: 38.41; H: 3.93; N: 8.76.
   **(17.2)** 2-Chloro-6-[2-(5-phosphono)furanyl]pyrazine. mp 204 - 205 °C. Anal. Calcd. for C₈H₆ClN₂O₄P + 0.3HBr + 0.02PhCH₃: C: 34.10; H: 2.27; N: 9.77. Found: C: 34.36; H: 2.07; N: 9.39.
   **(17.3)** 2-Amino-3-propyl-6-[2-(5-phosphono)furanyl]pyrazine. mp 227 - 228 °C. Anal. Calcd. for C₁₁H₁₄N₃O₄P + 0.7HBr: C: 38.87; H: 4.36; N: 12.36. Found: C: 39.19; H: 4.36; N: 11.92.
   **(17.4)** 2-Amino-6-[2-(5-phosphono)furanyl]pyrazine. mp 235 - 236°C. Anal. calcd. for C₈H₈N₃O₄P + 1.15H₂O + 0.03PhCH₃; C: 37.26; H: 4.01; N: 15.88. Found: C: 37.09; H: 3.67; N: 15.51.
   (**17.5**) 2-Amino-3-bromo-6-[2-(5-phosphono)furanyl]pyrazine. Anal. calcd. for C₈H₇N₃O₄PBr + 1HBr: C: 23.97; H: 2.01; N: 10.48. Found: C: 24.00; H: 2.00; N: 10.13.
   (**17.6**) 3-Methylthio-2-[2-(5-phosphono)furanyl]pyrazine. Anal. calcd. for C₉H₉N₂O₄PS + 0.3 H₂O: C: 38.94; H: 3.49; N: 10.09. Found: C: 38.99; H: 3.11; N: 9.67.
   (**17.7**) 6-Amino-3-methylthio-2-[2-(5-phosphono)furanyl]pyrazine. Anal. calcd. for C₉H₁₀N₃O₄PS + 1.5 H₂O + 1.7 HBr + 0.25 MePh: C: 27.19; H: 3.54; N: 8.85. Found: C: 27.10; H: 3.85; N: 8.49. (**17.8**) 6-Amino-5-methylthio-2-[2-(5-phosphono)furanyl]pyrazine. Anal. calcd. for C₉H₁₀N₃O₄PS + 1.1 HBr + 0.05 MePh: C: 29.49; H: 3.04; N: 11.03. Found: C: 29.23; H: 2.79; N: 10.87.
   **(17.9)** 6-Amino-5-methoxycarbonyl-3-chloro-2-[2-(5-phosphono)furanyl]pyrazine. Anal. calcd. for C₁₀H₉N₃O₆PCl + 0.3 HBr + 0.04 MePh: C: 34.15; H: 2.68; N: 11.62. Found: C: 34.20; H: 2.90; N: 11.21.
   **(17.10)** 6-Amino-3-methyIthio-2-[2-(5-phosphono)furanyI]pyrazine ammonium salt. Anal. calcd. for C₉H₁₃N₄O₄PS + 0.8 HBr: C: 29.30; H: 3.77; N: 15.18. Found: C: 29.03; H: 3.88; N: 15.08.
   **(17.11)** 2-Amino-4-phenyl-6-[2-(5-phosphono)furanyl]triazine. Anal. calcd. for C₁₃H₁₁N₄O₄P + HBr + 0.1 EtOAc: C: 39.45; H: 3.16; N: 13.73. Found: C: 39.77; H: 3.26; N: 13.48.

### Example 18.

### Preparation of analogs with X being methoxycarbonyl, methylthiocarbonyl, methylaminocarbonyl and methylcarbonylamino.

### Preparations of 4-phosphonomethoxycarbonylthiazoles and 4-phosphonomethoxycarbonyloxazoles

**Step A.** A solution of 2-amino-4-ethoxycarbonylthiazole (1 mmole) in 1,4-dioxane (5 mL) was treated with di-tert-butyl dicarbonate (1.2 mmole), TMEDA (0.1 mmole) and DMAP (0.1 mmole) at room temperature. After the reaction was stirred for 20 h, it was evaporated to dryness. The residue was subjected to extraction to give 2-[N-Boc(amino)]-4-ethoxycarbonyl thiazole as a yellow solid.
**Step B.** A solution of 2-[N-Boc(amino)]-4-ethoxycarbonylthiazole (1 mmole) in a 2:1 mixture of EtOH:H₂O (10 mL) was treated with NaOH (3N, 3 mmole) and the reaction was stirred at 60 °C for 4 h. The reaction was cooled to 0 °C and neutralized to pH 5 with 3 N HCl, and the resulting solid was collected via filtration to give 2-[N-Boc(amino)]-4-carboxylthiazole as a white solid.
**Step C.** A suspension of 2-[N-Boc(amino)]-4-carboxylthiazole (1 mmole) in CH₂Cl₂ (5 mL) was treated with thionyl chloride (4 mmole) at room temperature. After stirring for 4 h the reaction was evaporated to dryness. The residue was dissolved in CH₂Cl₂ (5 mL) and added to a solution of diethyl (hydroxymethyl)phosphonate (1.5 mmole) and pyridine (2 mmole) in CH₂Cl₂ (5 mL) at 0 °C. The reaction was warmed to room temperature and stirred for 4 h. The reaction was quenched with water and the mixture was subjected to extraction to give 2-[N-Boc(amino)]-4-diethylphosphonomethoxycarbonylthiazole as a thick yellow oil.
   Alternatively the ester linkage can be formed using a mixed anhydride method as exemplified in the following procedures:
   A solution of 2-[N-Boc(amino)]-4-carboxylthiazole (1 mmole) in pyridine (5 mL) was treated with para-toluenesulfonyl chloride (2 mmole) followed by diethyl (hydroxymethyl)phosphonate (2 mmole) at room temperature for 4 h. Evaporation, extraction and chromatography gave 2-[N-Boc(amino)]-4-diethylphosphonomethoxycarbonylthiazole as a thick yellow oil.
**Step D.** A solution of 2-[N-Boc(amino)]-4-diethylphosphonomethoxycarbonylthiazole (1 mmole) and anisole (0.1 mmole) in methylene chloride (5 mL) and trifluoroacetic acid (5 mL) was stirred at 0 °C for 1 h, and at room temperature for 1 h. Evaporation, extraction and chromatography gave 2-amino-4-diethyllphosphonomethoxycarbonylthiazole as a solid.
**Step E.** 2-Amino-4-diethyllphosphonomethoxycarbonylthiazole was subjected to Step C of Example 3 to give 2-amino-4-phosphonomethoxycarbonylthiazole **(18.1)** as a solid. Mp > 240 °C (decomp). Anal. Calcd. for C₅H₇N₂O₅PS: C: 25.22; H: 2.96; N: 11.76. Found: C: 25.30; H: 2.86; N: 11.77.
**Step F.** A solution of 2-[N-Boc(amino)]-4-diethylphosphonomethoxycarbonylthiazole (1 mmole) in CH₂Cl₂ (5 mL) was treated with bromine (2 mmole) at room temperature for 4 h. Evaporation and extraction gave 2-[N-Boc(amino)]-5-bromo-4-diethylphosphonomethoxycarbonylthiazole as an orange oil which was subjected to Step D of Example 18 followed by Step C of Example 3 to give 2-amino-5-bromo-4-phosphonomethoxycarbonylthiazole **(18.2)** as a solid. Mp > 230 °C (decomp). Anal. Calcd. for C₅H₆N₂O₅PSBr: C: 18.94; H: 1.91; N: 8.84. Found: C: 19.08; H: 1.76; N: 8.67.
**Step G.** A solution of 2-[N-Boc(amino)]-5-bromo-4-diethylphosphonomethoxycarbonylthiazole (1 mmole) and dichlorobis(triphenylphosphine)palladium(II) (0.1 mmole) in DMF (5 mL) was treated with tributyl(vinyl)tin (2.5 mmole) and the reaction was stirred at 60 °C for 2 h. The solvent was removed and the residue taken up in EtOAc and stirred with 2 mmol NaF in 5 ml water for 1 h. Extraction and chromatography gave 2-[N-Boc(amino)]-5-vinyl-4-diethylphosphonomethoxycarbonylthiazole as a yellow solid.
**Step H.** A suspension of 2-[N-Boc(amino)]-5-vinyl-4-diethylphosphonomethoxycarbonyl thiazole (1 mmole) and 10 % Pd/C (0.5 mmole) in MeOH (5 mL) was stirred under an atmosphere of H₂ (balloon) at room temperature for 15 h. Filtration and evaporation gave 2-[N-Boc(amino)]-5-ethyl-4-diethylphosphonomethoxycarbonylthiazole as a yellow solid, which was subjected to Step D of Example 18 followed by Step C of Example 3 to give 2-amino-5-ethyl-4-phosphonomethoxycarbonylthiazole (18.3) as a solid. Mp > 230 °C (decomp). Anal. Calcd. for C₇H₁₁N₂O₅PS: 31.58; H: 4.16; N: 10.52. Found: C: 31.80; H: 4.04; N: 10.18.
**Step I.** A solution of N-[Bis(methylthio)methylene]glycine methyl ester (1 mmole) in anhydrous THF (2 mL) was added to a solution of t-BuOK (1.4 mmole) in anhydrous THF (10 mL) at -78 °C and the mixture was stirred for 30 min. Then a solution of ethyl isothiocyanate (1 mmole) in anhydrous THF (2 mL) was added and the reaction was stirred at -78 °C for 30min and at room temperature for 2 h. The reaction was quenched with water. Extraction and chromatography gave 2-methylthio-5-(N-ethylamino)-4-methoxycarbonylthiazole as a yellow solid, which was subjected to Step B and C of Example 18 followed by Step C of Example 3 to give 2-methylthio-5-(N-ethylamino)-4-phosphonomethoxycarbonylthiazole (**18.4**) as a solid. Mp > 200 °C (decomp). Anal. Calcd. for C₈H₁₃N₂O₅PS₂ + 0.1 HBr: C: 29.99; H: 4.12; N: 8.74. Found: C: 29.71; H: 4.10; N: 8.60.

### I. Preparation of 4-phosphonomethylthiocarbonylthiazole

**Step J.** A solution of 1mmol of 2-[N-Boc(amino)]-4-thiazolecarboxylate acid chloride (1 mmole) and pyridine (2 mmole) in CH₂Cl₂ (5 mL) was cooled to -78 °C and H₂S(g) was bubbled through the solution for 10 min. The reaction was stirred at -78 °C for 30 min and then warmed to room temperature. The mixture was washed with 3 N HCl. The organic phase was separated, dried and concentrated to give 2-[N-Boc(amino)]-4-thiazolethiocarboxylic acid as a yellow solid.
**Step K.** A solution of give 2-[N-Boc(amino)]-4-thiazolethiocarboxylic acid (1 mmole) in THF (5 mL) was cooled to -78 °C and treated with NaH (2 mmole) in small portions. After 10 min the reaction was treated with a solution of diethylphosphonomethyl triflate in THF (5 mL). The reaction was stirred at -78 °C for 1h, and then quenched with H₂O. Extraction and chromatography gave 2-[N-Boc(amino)]-4-diethylphosphonomethylthiocarbonylthiazole as a thick oil, which was subjected to Step D of Example 18 followed by Step C of Example 3 to give 2-amino-4-phosphonomethylthiocarbonylthiazole **(18.5)** as a solid. Mp > 230 °C (decomp). Anal. Calcd. for C₅H₇N₂O₄PS₂: C: 23.62; H: 2.78; N: 11.02. Found: C: 23.77; H: 2.61; N: 10.73. Preparation of 4-[(N-phosphonomethyl)carbamoyl]thiazole, 3-[N-phosphonomethyl)-carbamoyl]isothiazole and 2-[N-phosphonomethyl)carbamoyl]pyridine
**Step L.** A solution of 2-[N-Boc(amino)]-4-thiazolecarboxylic acid (1 mmole) in DMF (5 mL) was treated with 1-(3-dimethylaminopropyl)-3-ethylcarbodiimide hydrochloride (EDCI, 1.5 mmole) and 1-hydroxylbenzotriazole hydrate (HOBt, 1.5 mmole) followed by addition of diethyl aminomethylphosphonate (1.5 mmole) at room temperature for 24 h. The reaction was subjected to evaporation, extraction and chromatography to give 2-[N-Boc(amino)]-4-[(N-diethylphosphonomethyl)carbamoyl]thiazole as a white solid, which was subjected to Step D of Example 18 followed by Step C of Example 3 to give 2-amino-4-[(N-phosphonomethyl)carbamoyl]thiazole **(18.6)** as a light brown solid. Mp > 245 °C (decomp). Anal. Calcd. for C₅H₈N₃O₄PS + 1.05 HBr: C: 18.64; H: 2.83; N: 13.04. Found: C: 18.78; H: 2.43; N: 12.97.

### Preparation of 2-[(N-phosphonoacetyl)amino]thiazole and 2-[(N-phosphonoacetyl)amino]pyridine

**Step M.** A solution of 2-amino-4,5-dimethylthiazole hydrochloride (2 mmole) and diethyl phosphonoacetica acid (1 mmole) in DMF (5 mL) was treated with EDCI (1.5 mmole), HOBt (1.5 mmole) and triethylamine (2 mmole) at room temperature for 24 h. The reaction was subjected to evaporation, extraction and chromatography to give 2-[(N-diethylphosphonoacetyl)amino]-4,5-dimethylthiazole as a yellow solid, which was subjected to Step D of Example 18 followed by Step C of Example 3 to give 4,5-dimethyl-2-[(N-phosphonoacetyl)amino]thiazole **(18.7)** as a light brown solid. Mp > 250 °C. Anal. Calcd. for C₇H₁₁N₂O₄PS: C: 33.60; H: 4.43; N: 11.20. Found: C: 33.62; H: 4.29; N: 10.99.

The following compounds were prepared using some of the above described procedures or some of the above procedures with some minor modifications using conventional chemistry:
(**18.8**) 2-[(N-phosphonomethyl)carbamoyl]pyridine . Anal. Calcd. for C₇H₉N₂O₄P + HBr + 0.67 H₂O: C: 27.20; H: 3.70; N: 9.06. Found: C: 27.02; H: 3.71; N: 8.92.
(**18.9**) 2-[(N-phosphonoacetyl)amino]pyridine. Anal. Calcd. for C₇H₉N₂O₄P + HBr + 0.67 H₂O: C: 27.20; H: 3.70; N: 9.06. Found: C: 27.05; H: 3.59; N: 8.86.
(**18.10**) 4-Ethoxycarbonyl-2-[(N-phosphonoacetyl)amino]thiazole. Anal. Calcd. for C₈H₁₁N₂O₆PS: C: 32.66; H: 3.77; N: 9.52. Found: C: 32.83; H: 3.58; N: 9.20.
(**18.11**) 2-Amino-5-bromo-4-[(N-phosphonomethyl)carbamoyl]thiazole. Mp 232 °C (decomp). Anal. Calcd. for C₅H₇N₃O₄PSBr + 0.15HBr + 0.1 hexane: C: 19.97; H: 2.56; N: 12.48. Found: C: 19.90; H: 2.29; N: 12.33.
(**18.12**) 2-Amino-5-(2-thienyl)-4-[(N-phosphonomethyl)carbamoyl]thiazole. Mp 245 °C (decomp). Anal. Calcd. for C₉H₁₀N₃O₄PS₂ + HBr + 0.1 EtOAc: C: 27.60; H: 2.91; N: 10.27. Found: C: 27.20; H: 2.67; N: 9.98.
(**18.13**) 4,5-Dichloro-3-[(N-phosphonomethyl)carbamoyl]isothiazole. Mp 189 - 191 °C. Anal. Calcd. for C₅H₅N₂O₄PSCl₂: C: 20.63; H: 1.73; N: 9.62. Found: C: 20.43; H: 1.54; N: 9.51.
(**18.14**) 2-Amino-5-bromo-4-{[N-(1-phosphono-1-phenyl)methyl]carbamoyl}thiazole. Mp > 250 °C. Anal. Calcd. for C₁₁H₁₁N₃O₄PSBr: C: 33.69; H: 2.83; N: 10.71. Found: C: 33.85; H: 2.63; N: 10.85.
**(18.15)** 2-Amino-5-(2-thienyl)-4-phosphonomethoxycarbonylthiazole. Mp > 230 °C (decomp). Anal. Calcd. for C₉H₉N₂O₅PS₂: C: 33.75; H: 2.83; N: 8.75. Found: C: 33.40; H: 2.74; N: 8.51.
**(18.16)** 2-Amino-5-benzyl-4-phosphonomethoxycarbonylthiazole. Mp > 230 °C (decomp). Anal. Calcd. for C₁₂H₁₃N₂O₅PS: C: 43.91; H: 3.99; N: 8.53. Found: C: 43.77; H: 4.03; N: 8.25.
(**18.17**) 2-Methylthio-5-methylamino-4-phosphonomethoxycarbonylthiazole. Anal. Calcd. for C₇H₁₁N₂O₅PS₂+ 0.2 HBr: C: 26.74; H: 3.59; N: 8.91. Found: C: 26.79; H: 3.89; N: 8.89.
(**18.18**) 2-Amino-5-ethyl-4-[(N-phosphonomethyl)carbamoyl]thiazole. Mp 180 °C (decomp). Anal. Calcd. for C₇H₁₂N₃O₄PS + HBr + 0.4 CH₂Cl₂: C: 23.49; H: 3.67; N: 11.18. Found: C: 23.73; H: 3.29; N: 11.42.
**(18.19)** 2-Amino-5-isopropyl-4-[(N-phosphonomethyl)carbamoyl]thiazole. Mp 247 - 250 °C. Anal. Calcd. for C₈H₁₄N₃O₄PS: C: 34.41; H: 5.05; N: 15.05. Found: C: 34.46; H:4.80; N: 14.68.
**(18.20)** 2-Amino-5-isopropyl-4-phosphonomethoxycarbonylthiazole . Mp > 230 °C. Anal. Calcd. for C₈H₁₃N₂O₅PS: C: 34.29; H: 4.68; N: 10.00. Found: C: 33.97; H: 4.49; N: 9.70.
**(18.21)** 2-Amino-5-phenyl-4-phosphonomethoxycarbonylthiazole. Mp > 230 °C. Anal. Calcd. for C₁₁H₁₁N₂O₅PS: C: 42.04; H: 3.53; N: 8.91. Found: C: 42.04; H: 3.40; N: 8.72.
**(18.22)** 2-Amino-4-phosphonomethoxycarbonyloxazole. Anal. Calcd. for C₅H₇N₂O₆P + 0.09 HBr: C: 26.18; H: 3.12; N: 12.21. Found: C: 26.29; H: 3.04; N: 11.90.
**(18.23)** 2-Amino-6-[(N-phosphonoacetyl)amino]pyridine. Anal. Calcd. for C₇H₁₀N₃O₄P + 1.1 HBr + 0.25 MeOH: C: 26.54; H: 3.72; N: 12.80. Found: C: 26.79; H: 3.63; N: 12.44.
**(18.24)** 2-Amino-5-methyl-4-[(N-phosphonomethyl)carbainoyl]thiazole. Mp > 250 °C. Anal. Calcd. for C₆H₁₀N₃O₄PS + 0.06 EtOAc: C: 29.22; H: 4.12; N: 16.38. Found: C: 29.03; H: 3.84; N: 16.01.
**(18.25)** 2-Amino-3-bromo-6-[(N-phosphonoacetyl)amino]pyridine. Anal. Calcd. for C₇H₉N₃O₄PBr + 1.25 HBr + 0.8 EtOAc: C: 25.43; H: 3.48; N: 8.72. Found: C: 25.58; H: 3.71; N: 8.56.
**(18.26)** 2-Amino-3,5-dibromo-6-[(N-phosphonoacetyl)amino]pyridine. Anal. Calcd. for C₇H₈N₃O₄PBr₂ + HBr + 0.5 EtOAc: C: 21.03; H: 2.55; N: 8.18. Found: C: 21.28; H: 2.55; N: 7.91.
**(18.27)** 2-Amino-5-methyl-4-phosphonomethoxycarbonylthiazole. Mp 230 °C (decomp). Anal. Calcd. for C₆H₉N₂O₅PS: C: 28.58; H: 3.60; N: 11.11. Found: C: 28.38; H: 3.49; N: 11.10.
(**18.28**) 2-Amino-3,5-diethyl-6-[(N-phosphonoacetyl)amino]pyridine. MS calcd. for C₁₁H₁₈N₃O₄P + H: 288, found 288.
(**18.29**) 2-Amino-3,5-dibromo-6-{[N-(2,2-dibromo-2-phosphono)acetyl]amino}pyridine. Anal. Calcd. for C₇H₆N₃O₄PBr₄ + 0.5 HBr + EtOAc: C: 19.56; H: 2.16; N: 6.22. Found: C: 19.26; H: 2.29; N: 5.91.
**(18.30)** 2-Amino-5-isopropyl-4-phosphonomethoxycarbonyloxazole. Anal. Calcd. for C₈H₁₃N₂O₆P + 0.2 HBr: C: 34.27; H: 4.75; N: 9.99. Found: C: 34.47; H: 4.84; N: 9.83.
**(18.31)** 2-Amino-5-[1-(2-cyclohexylmethyl)ethynyl]-4-phosphonomethoxycarbonylthiazole. Mp 230 °C (decomp). Anal. Calcd. for C₁₄H₁₉N₂O₅PS + 0.1 HBr: C: 45.89; H: 5.25; N: 7.64. Found: C: 45.85; H: 4.96; N: 7.44.
**(18.32)** 2-Amino-5-[1-(4-cyano)butynyl]-4-phosphonomethoxycarbonylthiazole. Mp 230 °C (decomp). Anal. Calcd. for C₁₀H₁₀N₃O₅PS + 0.25 HBr: C: 35.80; H: 3.08; N: 12.53. Found: C: 35.92; H: 2.99; N: 12.20.
(**18.33**) 2-Amino-5-methyl-4-phosphonomethoxycarbonyloxazole. Anal. Calcd. for C₆H₉N₂O₆P + 0.15 HBr: C: 29.03; H: 3.71; N: 11.28. Found: C: 28.98; H: 3.66; N: 11.21.
(**18.34**) 2-Amino-5-[1-(4-cyano)butyl]-4-phosphonomethoxycarbonylthiazole. Mp 230 °C (decomp). Anal. Calcd. for C₁₀H₁₄N₃O₅PS: C: 37.62; H: 4.42; N: 13.16. Found: C: 37.23; H: 4.18; N: 12.79.
**(18.35)** 2-Amino-5-pentyl-4-phosphonomethoxycarbonyloxazole. Anal. Calcd. for C₁₀H₁₇N₂O₆P: C: 41.10; H: 5.86; N: 9.59. Found: C: 41.16; H: 5.75; N: 9.50.
**(18.36)** 2-[N-Boc(amino)]-4-[(2-phosphono)ethoxycarbonyl]thiazole. Anal. Calcd. for C₁₁H₁₇N₂O₇PS: C: 37.50; H: 4.86; N: 7.95. Found: C: 37.10; H: 4.59; N: 7.84.
**(18.37)** 2-Amino-4-[(2-phosphono)ethoxycarbonyl]thiazole hydrobromide. Anal. Calcd. for C₆H₉N₂O₅PS + HBr: C: 21.63; H: 3.03; N: 8.41. Found: C: 22.01; H: 2.99; N: 8.15.
**(18.38)** 2-Amino-5-butyl-4-phosphonomethoxycarbonyloxazole. Anal. Calcd. for C₉H₁₅N₂O₆P: C: 38.86; H: 5.43; N: 10.07. Found: C: 38.59; H: 5.43; N: 9.96.
**(18.39)** 2-Amino-5-[1-oxo-2,2-dimethyl)propyl]-4-phosphonomethoxycarbonylthiazole. Anal. Calcd. for C₁₀H₁₅N₂O₆PS: C: 37.27; H: 4.69; N: 8.69. Found: C: 37.03; H: 4.69; N: 8.39.
**(18.40)** 2-Amino-5-propyl-4-phosphonomethoxycarbonyloxazole. Anal. Calcd. for C₈H₁₃N₂O₆P + 0.35 EtOAc + 0.05 HBr: C: 37.75; H: 5.34; N: 9.37. Found: C: 37.69; H: 5.21; N: 9.03.
**(18.41)** 2-Amino-5-propyl-4-phosphonomethoxycarbonylthiazole. Mp 134 °C (decomp). Anal. Calcd. for C₈H₁₃N₂O₅PS: C: 34.29; H: 4.68; N: 10.00. Found: C: 33.90; H: 4.30; N: 9.61.
**(18.42)** 2-Amino-5-pentyl-4-phosphonomethoxycarbonylthiazole. Mp 130 °C (decomp). Anal. Calcd. for C₁₀H₁₇N₂O₅PS: C: 38.96; H: 5.56; N: 9.09. Found: C: 38.69; H: 5.25; N: 8.85.
**(18.43)** 2-Amino-5-bromo-4-phosphonomethylthiocarbonylthiazole. Mp 230 °C (decomp). Anal. Calcd. for C₅H₆N₂O₅PS₂Br: C: 18.03; H: 1.82; N: 8.41. Found: C: 18.40; H: 1.93; N: 8.18.
**(18.44)** 2-Amino-5-(2-furanyl)-4-phosphonomethoxycarbonylthiazole. Mp 230 °C (decomp). Anal. Calcd. for C₉H₉N₂O₆PS: C: 35.53; H: 2.98; N: 9.21. Found: C: 35.78; H: 3.05; N: 8.11.
**(18.45)** 2-Amino-5-ethyl-4-phosphonomethoxycarbonyloxazole. Mp 141 °C (decomp). Anal. Calcd. for C₇H₁₁N₂O₆P: C: 33.61; H: 4.43; N: 11.20. Found: C: 33.79; H: 4.47; N: 11.09.
**(18.46)** 5-Methyl-4-[(N-phosphonomethyl)carbamoyl]imidazole. Anal. calcd. for C₆H₁₀N₃O₄P: C: 32.89; H: 4.60; N: 19.18. Found; C: 33.04; H: 4.65; N: 18.84.

### Example 19.

### Preparation of various phosphonate diesters as prodrugs.

A suspension of 2-methyl-5-isobutyl-4-[2-(5-phosphono)furanyl]thiazole (1 mmole) in thionyl chloride (5 mL) was warmed at reflux for 4 h. The cooled reaction mixture was evaporated to dryness and the resulting yellow residue was dissolved in methylene chloride and treated with a solution of the corresponding benzyl alcohol (4 mmole) and pyridine (2.5 mmole) in methylene chloride. After stirring at 25 °C for 24 h the reaction mixture was subjected to extraction and chromatography to give the titled compounds. The following compounds were prepared according to this procedure:
(**19.1**) 2-Methyl-5-isobutyl-4-{2-[5-bis(4-pivaloyloxybenzyl)phosphono]furanyl}thiazole. Anal. Calcd. for C₃₆H₄₄NO₈PS + 0.4H₂O: C: 62.76; H: 6.55; N: 2.03. Found: C: 62.45; H: 6.44; N: 2.04.
(**19.2**) 2-Methyl-5-isobutyl-4-{2-[5-bis(3,4-diacetoxybenzyl)phosphono]furanyl}thiazole. Anal. Calcd. for C₃₄H₃₆NO₁₂PS + 0.8H₂O: C: 56.09; H: 5.21; N: 1.92. Found: C: 55.90; H: 4.98; N: 1.94.
(**19.3**) 2-Methyl-5-isobutyl-4-{2-[5-bis(4-acetoxy-3-methoxybenzyl)phosphono]furanyl}thiazole. Anal. Calcd. for C₃₂H₃₆NO₁₀PS: C: 58.44; H: 5.52; N: 2.13. Found: C: 58.16; H: 5.34; N: 2.13.
**(19.4)** 2-Methyl-5-isobutyl-4-{2-[5-bis(4-acetoxy-3-methylbenzyl)phosphono]furanyl} thiazole. Anal. Calcd. for C₃₂H₃₆NO₈PS: C: 61.43; H: 5.80; N: 2.24. Found: C: 61.34; H: 5.89; N: 2.25.
(**19.5**)2-Amino-5-isobutyl-4-{2-[5-bis(3,4-diacetoxybenzyl)phosphono]furanyl}thiazole. Anal. Calcd. for C₃₃H₃₅N₂O₁₂PS: C: 55.46; H: 4.94; N: 3.92. Found: C: 55.06; H: 4.96; N: 3.79.
**(19.6)** 2-Amino-5-isobutyl-4-{2-[5-bis(4-acetoxybenzyl)phosphono]furanyl}thiazo!e. Anal. Calcd. for C₂₉H₃₁N₂O₈PS: C: 58.19; H: 5.22; N: 4.68. Found: C: 57.82; H: 4.83; N: **4.50.**

This method is also useful for the preparation of phenyl phosphonate esters as prodrugs, and the following compound was prepared:
**(19.7)** 2-Methyl-5-isobutyl-4-[2-(5-diphenylphosphono)furanyl]thiazole. Anal. Calcd. for C₂₄H₂₄NO₄PS + 0.1H₂O: C: 63.31; H: 5.36; N: 3.08. Found: C: 63.22; H: 5.34; N: 3.14. **(19.63)** 2-Amino-5-isobutyl-4-[2-(5-diphenylphosphono)furanyl]thiazole. Mp 128 -129 0 °C. Anal. Calcd. for C₂₃H₂₃N₂O₄PS: C: 60.78; H: 5.10; N: 6.16. Found: C: 60.68; H: 4.83; N: 6.17.
(**19.64**) 2-Amino-5-isobutyl-4-[2-(5-phenylphosphono)furanyl]thiazole. Mp >250 0 °C. Anal. Calcd. for C₁₇H₁₉N₂O₄PS: C: 53.96; H: 5.06; N: 7.40. Found: C: 53.81; H: 4.87; N:7.41.
**(19.65)** 2-Amino-5-isobutyl-4-[2-(5-bis(3-chlorophenyl)phosphono)furanyl]thiazole. Anal. Calcd. for C₂₃H₂₁N₂O₄PSCl₂ + 0.5 H₂O: C: 51.89; H: 4.17; N: 5.26. Found C: 51.55; H: 3.99; N: 5.22.
**(19.67)** 2-Amino-5-isobutyl-4-[2-(5-bis(4-methoxyphenyl)phosphono)furanyl]thiazole. Anal. Calc. for C₂₅H₂₇N₂O₆PS + 0.5 H₂O: C: 57.35; H: 5.39; N: 5.35. Found C: 57.11; H: 5.36; N: 5.75.

This method is also useful for the preparation of some thio-containing phosphonate esters as prodrugs, and the following compounds were prepared:
**(19.8)** 2-Methyl-5-isobutyl-4-{2-[5-bis(2-methylcarbonylthioethyl)phosphono]furanyl}thiazole. Anal. Calcd. for C₂₀H₂₈NO₆PS₃: C: 47.51; H: 5.58; N: 2.77. Found: C: 47.32; H: 5.56; N: 2.77.
**(19.9)** 2-Methyl-5-isobutyl-4-{2-[5-bis(thiobenzoylmethyl)phosphono]furanyl}thiazole. Anal. Calcd. for C₂₈H₂₈NO₆PS₃: C: 55.89; H: 4.69; N: 2.33. Found: C: 55.73; H: 4.72; N: 2.28.

This method is also useful for the preparation of cyclic phosphonate esters (e.g. cyclic 1,3-propanediol phosphonate esters) as prodrugs by coupling of phosphonic acids with various diols (e.g. 1,3-propanediols see Example 21 for the synthesis of some 1,3-propanediols), and the following compounds were made:
**(19.10)** 5-Isobutyl-2-methyl-4-{2-[5-(1-hydroxy-3,5-cyclohexyl)phosphono]furanyl}thiazole (minor isomer). Anal. Calcd. for C₁₈H₂₄NO₅PS + 0.33H₂O: C: 53.60; H: 6.16; N: 3.47. Found: C: 53.75; H: 6.53; N: 3.45.
**(19.11)** 5-Isobutyl-2-methyl-4-{2-[5-(1-hydroxy-3,5-cyclohexyl)phosphono]furanyl}thiazole (major isomer). Anal. Calcd. for C₁₈H₂₄NO₅PS:
C: 54.40; H: 6.09; N: 3.52. Found: C: 54.44; H: 6.11; N: 3.63.
   **(19.12)** 5-Isobutyl-2-methyl-4-{2-[5-(2-hydroxymethyl-1,3-propyI)phosphono]furanyl}thiazole. Anal. Calcd. for C₁₆H₂₂NO₅PS + 0.3CH₂Cl₂ + 0.5H₂O: C: 48.24; H: 5.86; N: 3.45. Found: C: 47.94; H: 5.59; N: 3.57.
   **(19.13)** 5-Isobutyl-2-methyl-4-{2-[5-(1-phenyl-1,3 -propyl)phosphono]furanyl}thiazole, (minor isomer). Anal. Calcd. for C₂₁H₂₄NO₄PS + 0.25H₂O: C: 59.77; H: 5.85; N: 3.32. Found: C: 59.76; H: 5.69; N: 3.38.
   **(19.14)** 5-Isobutyl-2-methyl-4-{2-[5-(1-phenyl-1,3-propyl)phosphono]furanyl}thiazole, (major isomer). Anal. Calcd. for C₂₁H₂₄NO₄PS + 0.5 H₂O: C: 59.14; H: 5.91; N: 3.28. Found: C: 59.27; H: 5.85; N: 3.38.
   **(19.15)** 2-Amino-5-isobutyl-4-[2-(5-[2-(methoxycarbonyloxymethyl)-propan-1,3-yl]phosphono)furanyl]thiazole (minor isomer). mp 170 - 173°C. Anal. Calcd. for C₁₇H₂₃N₂O₇PS: C: 47.44; H: 5.39; N: 6.51. Found: C: 47.28; H: 5.27; N: 6.47.
   (**19.16**) 2-Amino-5-isobutyl-4-[2-(5-[2-(methoxycarbonyloxymethyl)-propan-1,3-yl]phosphono)furanyl]thiazole (major isomer). Anal. Calcd. for C₁₇H₂₃N₂O₇PS + 0.5 H₂O: C: 46.47; H: 5.51; N: 6.38. Found: C: 46.38; H: 5.29; N: 6.20.
   (**19.17**) 5-Isobutyl-2-methyl-4-{2-[5-(1-(4-pyridyl)-1,3-propyl)phosphono]furanyl}-thiazole. Anal. Calcd. for C₂₀H₂₃N₂O₄PS + 2H₂O + 0.4CH₂Cl₂: C: 50.16; H: 5.74; N: 5.74. Found: C: 50.36; H: 5.36; N: 5.80.
   (**19.18**) 2-Amino-5-isobutyl-4-(2-{5-[1-(4-pyridyl)-propan-1,3-yl]phosphono}furanyl)-thiazole. mp 101 - 106 °C. Anal. Calcd. for C₁₉H₂₂N₃O₄PS + 0.75H₂O: C: 52.71; H: 5.47; N: 9.71. Found: C: 52.59; H: 5.49; N: 9.65.
   (**19.20**) 2-Amino-5-isobutyl-4-{2-[5-(1-phenyl-1,3-propyl)phosphono]furanyl}thiazole (minor isomer). Anal. Calcd. for C₂₀H₂₃N₂O₄PS + 0.33HCl: C: 55.80; H: 5.46; N: 6.51. Found: C: 55.95; H: 5.36; N: 6.46.
   (**19.21**) 2-Amino-5-isobutyl-4-{2-[5-(1-phenyl-1,3-propyl)phosphono]furanyl}thiazole (major isomer). Anal. Calcd. for C₂₀H₂₃N₂O₄PS + 0.33HCl: C:55.80; H: 5.46; N: 6.51. Found: C: 55.77; H: 5.19; N: 6.44.
   **(19.22)** 2-Amino-5-ethyl-4-{2-[5-(1-phenyl-1,3-propyl)phosphono]furanyl}thiazole (less polar isomer). Anal. Calcd. for C₁₈H₁₉N₂O₄PS + 0.2HCl + 0.25 H₂O: C: 53.75; H: 4.94; N: 6.97. Found: C: 53.86; H: 4.70; N: 6.87.
   **(19.23)** 2-Amino-5-ethyl-4-{2-[5-(1-phenyl-1,3-propyl)phosphono]furanyl}-thiazole (more polar isomer). Anal. Calcd. for C₁₈H₁₉N₂O₄PS + 0.2HCl + 0.25 H₂O: C: 53.75; H: 4.94; N: 6.97. Found: C: 53.92; H: 4.82; N: 6.92.
   **(19.24)** 2-Amino-5-ethyl-4-{2-[5-(1-{4-pyridyl}-1,3-propyl)phosphono]furanyl}thiazole. Anal. Calcd. for C₁₇H₁₈N₃O₄PS + 0.1HCl + 0.5 H₂O: C: 50.54; H: 4.76; N: 10.40.
   Found: C: 50.38; H: 4.53; N: 10.25.
   **(19.25)** 2-Methyl-4-{2-[5-(2-acetoxymethylpropan-1,3-diyl)phosphono]furanyl}thiazole. Anal. calcd. for C₁₄H₁₆NO₆PS + 0.5H₂O: C: 45.90; H: 4.68; N: 3.82. Found C: 45.50; H: 4.55; N: 3.45.
   **(19.26)** 2-Methyl-4-(2-{5-[1-(4-pyridyl)propan-1,3-diyl]phosphono}furanyl)thiazole. Anal. calcd. for C₁₆H₁₅N₂O₄PS + 0.75H₂O: C: 51.13; H: 4.42; N: 7.45. Found: C: 50.86; H: 4.72; N: 7.11.
   **(19.27)** 2-Amino-5-methylthio-4-(2-{5-[1-(4-pyridyl)propan-1,3-diyl]phosphono}furanyl)thiazole. Anal. calcd. for C₁₆H₁₆N₃O₄PS₂ + 0.4 HC1: C: 45.32; H: 3.90; N: 9.91. Found: C: 45.29; H: 3.80; N: 9.83.
   **(19.28)** 2-Amino-5-isobutyl-4-{2-[5-(1-(3-bromophenyl)propan-1,3-diyl)phosphono]furanyl}thiazole, major isomer. Anal. calcd. for C₂₀H₂₂N₂O₄PBrS: C: 48.30; H: 4.46; N: 5.63. Found: C: 48.51; H: 4.21; N: 5.33.
   **(19.29)** 2-Amino-5-methylthio-4-{2-[5-(1-(R)-phenyl-1,3-propyl)phosphono]furanyl}thiazole. Anal. Calcd. for C₁₇H₁₇N₂O₄PS +HCI: C: 49.46; H: 4.39; N: 6.79. Found: C: 49.77; H: 4.13; N: 6.54.
   (**19.30**) 2-Amino-5-isobutyl-4-{2-[5-(1-(3-bromophenyl)-1,3-propyl)phosphono]furanyl}thiazole, minor isomer. Anal. Calcd. for C₂₀H₂₂N₂O₄PSBr + 0.25HCI: C: 47.43; H: 4.43; N: 5.53. Found: C: 47.58; H: 4.16; N: 5.31.
   **(19.31)** 2-Amino-5-isobutyl-4-{2-[5-(2-benzyl-1,3-propyl)phosphono]furanyl}thiazole. Anal. Calcd. for C₂₁H₂₅N₂O₄PS: C: 58.32; H: 5.83; N: 6.48. Found: C: 57.98; H: 5.65; N: 6.47.
   (**19.32**) 2-Amino-5-cycloproyl-4-{2-[5-(1-(4-pyridyl)-1,3-propyl)phosphono]furanyl}thiazole. Anal. Calcd. for C₁₈H₁₈N₃O₄PS + 0.5H₂O: C: 52.42; H: 4.64; N: 10.19. Found: C: 52.62; H: 4.51; N: 9.89.
   **(19.33)** 2-Methyl-5-isobutyl-4-{2-[5-(1-(S)-phenyl-1,3-propyl)phosphono]furanyl}thiazole, minor isomer. Anal. Calcd. for C₂₁H₂₄NO₄PS: C: **60.42;** H: 5.79; N: 3.36. Found: C: 60.10; H: 5.58; N: 3.32.
   (**19.34**) 2-Methyl-5-isobutyl-4-{2-[5-(1-(S)-phenyl-1,3-propyl)phosphono]furanyl}thiazole, major isomer. Anal. Calcd. for C₂₁H₂₄NO₄PS + 0.33 H₂O: C: 59.57; H: 5.87; N: 3.31. Found: C: 59.45; H: 5.83; N: 3.30.
   (**19.35**) 2-Azido-5-ethyl-4-{2-[5-(1-phenyl-1,3-propyl)phosphono]furanyl}thiazole, major isomer. Anal. Calcd. for C₁₈H₁₇N₄O₄PS + 0.25H₂O + 0.1 isoamyl alcohol (C₅H₁₂O): C: 51.71; H: 4.39; N: 13.04. Found: C: 51.80; H: 4.20; N: 12.78.
   (**19.36**) 2-Azido-5-ethyl-4-{2-[5-(1-phenyl-1,3-propyl)phosphono]furanyl}thiazole, minor isomer. Anal. Calcd. for C₁₈H₁₇N₄O₄PS + 0.15 isoamyl alcohol (C₅H₁₂O): C: 52.42; H: 4.41; N: 13.04. Found: C: 52.27; H: 4.47; N: 12.76.
   (**19.37**) 2-Amino-5-isobutyl-4-{2-[5-(1-(1-naphthyl)-1,3-propyl)phosphono]furanyl}thiazole. Anal. Calcd. for C₂₄H₂₅N₂O₄PS: C: 61.53; H: 5.38; N: 5.98. Found: C: 61.40; H: 5.12; N: 6.11.
   (**19.38**) 2-Amino-5-isobutyl-4-{2-[5-(1-(2-bromophenyl)-1,3-propyl)phosphono]furanyl}thiazole. Anal. Calcd. for C₂₀H₂₂N₂O₄PSBr + 0.1 C₅H₅N: C: 48.73; H: 4.49; N: 5.82. Found: C: 48.63; H: 4.26; N: 5.70.
   (**19.39**) 2-Amino-5-isobutyl-4-{2-[5-(1-(4-bromophenyl)-1,3-propyl)phosphono]furanyl}thiazole, minor isomer. Anal. Calcd. for C₂₀H₂₂N₂O₄PSBr: C: 48.30; H: 4.46; N: 5.63. Found: C: 48.23; H: 4.30; N: 5.77.
   (**19.40**) 2-Amino-5-isobutyl-4-{2-[5-(1-(4-bromophenyl)-1,3-propyl)phosphono]furanyl}thiazole, major isomer. Anal. Calcd. for C₂₀H₂₂N₂O₄PSBr: C: 48.30; H: 4.46; N: 5.63. Found: C: 48.20; H: 4.63; N: 5.41.
   (**19.41**) 2-Amino-5-isobutyl-4-{2-[5-(1-(4-fluoro-3-bromophenyl)-1,3-propyl)phosphono]furanyl}thiazole, minor isomer. Anal. Calcd. for C₂₀H₂₁N₂O₄PSBrF + 0.1 C₅H₅N: C: 47.06; H: 4.14; N: 5.62. Found: C: 47.00; H: 3.84; N: 5.48.
   (**19.42**) 2-Amino-5-isobutyl-4-{2-[5-(1-(4-fluoro-3-bromophenyl)-1,3-propyl)phosphono]furanyl}thiazole, major isomer. Anal. Calcd. for C₂₀H₂₁N₂O₄PSBrF: C: 46.61; H: 4.11; N: 5.44; P: 6.01. Found: C: 46.81; H: 4.23; N: 5.65; P: 5.65.
   (**19.43**) 2-Amino-5-isobutyl-4-{2-[5-(1-(4-trifluoromethylphenyl)-1,3-propyl)phosphono]furanyl}thiazole, minor isomer. Anal. Calcd. for C₂₁H₂₂N₂O₄PSF₃ + 0.1 H₂O: C: 51.66; H: 4.58; N: 5.74. Found: C: 51.54; H: 4.28; N: 5.46.
   (**19.44**) 2-Amino-5-isobutyl-4-{2-[5-(1-(4-trifluoromethylphenyl)-1,3-propyl)phosphono]furanyl}thiazole, major isomer. Anal. Calcd. for C₂₁H₂₂N₂O₄PSF₃ + 0.1 H₂O: C: 51.66; H: 4.58; N: 5.74. Found: C: 51.48; H: 4.62; N: 5.81.
   (**19.45**) 2-Amino-5-isobutyl-4-{2-[5-(1-(3-chlorophenyl)-1,3-propyl)phosphono]furanyl}thiazole, minor isomer. Anal. Calcd. for C₂₀H₂₂N₂O₄PSCl + 0.5 H₂O: C: 52.01; H: 5.02; N: 6.06. Found: C: 52.10; H: 4.92; N: 5.82.
   (**19.46**) 2-Amino-5-isobutyl-4-{2-[5-(1-(3-chlorophenyl)-1,3-propyl)phosphono]furanyl}thiazole, major isomer. Anal. Calcd. for C₂₀H₂₂N₂O₄PSCl + 0.25 H₂O: C: 52.52; H: 4.96; N: 6.12. Found: C: 52.70; H: 4.79; N: 5.91.
   (**19.47**) 2-Amino-5-isobutyl-4-{2-[5-(1-(3,5-dichlorophenyl)-1,3-propyl)phosphono]furanyl}thiazole, minor isomer. Anal. Calcd. for C₂₀H₂₁N₂O₄PSCl₂: C: 49.29; H: 4.34; N: 5.75. Found: C: 49.47; H: 4.60; N: 5.89.
   **(19.48)** 2-Amino-5-isobutyl-4-{2-[5-(1-(3,5-dichlorophenyl)-1,3-propyl)phosphono]furanyl}thiazole, major isomer. Anal. Calcd. for C₂₀H₂₁N₂O₄PSCl₂: C: 49.29; H: 4.34; N: 5.75; Cl: 14.55. Found: C: 49.26; H: 4.36; N: 5.71; Cl: 14.66.
   **(19.49)** 2-Amino-5-isobutyl-4-{2-[5-(2-(4-methoxybenzyl)-1,3-propyl)phosphono]furanyl}thiazole. Mp 185 - 188 °C. Anal. Calcd. for C₂₂H₂₇N₂O₅PS: C: 57.13; H: 5.88; N: 6.06. Found: C: 56.86; H: 5.71; N: 5.73.
   **(19.50)** 2-Amino-5-isobutyl-4-{2-[5-(2-methanesulfonyloxymethyl-1,3-propyl)phosphono]furanyl}thiazole. Anal. Calcd. for C₁₆H₂₃N₂O₇PS₂ + 0.2 H₂O: C: 42.32; H: 5.19; N: 6.17. Found: C: 42.15; H: 4.94; N: 5.95.
   **(19.51)** 2-Amino-5-isobutyl-4-{2-[5-(2-azidomethyl-1,3-propyl)phosphono]furanyl}thiazole. Mp 187 - 189 °C. Anal. Calcd. for C₁₅H₂₀N₅O₄PS: C: **45.34;** H: 5.07; N: 17.62. Found: C: 45.09; H: 4.82; N: 17.72.
   **(19.52)** 2-Amino-5-isobutyl-4-{2-[5-(2-aminomethyl-1,3-propyl)phosphono]furanyl}thiazole. Anal. Calcd. for C₁₅H₂₂N₃O₄PS + 0.3 H₂O + 0.1 HCl: C: 47.36; H: 6.01; N: 11.04. Found: C: 47.55; H: 5.62; N: 10.64.
   **(19.53)** 2-Amino-5-isobutyl-4-{2-[5-(1-(4-tert-butylphenyl)-1,3-propyl)phosphono]furanyl}thiazole, minor isomer. Mp 141 - 143 °C. Anal. Calcd. for C₂₄H₃₁N₂O₄PS + 1.5 HCI: C: 54.47; H: 6.19; N: 5.29. Found: C: 54.44; H: 5.85; N: 4.92.
   **(19.54)** 2-Amino-5-isobutyl-4-{2-[5-(1-(4-tert-butylphenyl)-1,3-propyI)phosphono]furanyl}thiazole, major isomer. Mp 178 °C (decomp). Anal. Calcd. for C₂₄H₃₁N₂O₄PS + H₂O: C: 58.52; H: 6.75; N: 5.69. Found: C: 58.20; H: 6.31; N: 5.29.
   **(19.55)** 2-Amino-5-isobutyl-4-{2-[5-(1-(4-chlorophenyl)-1,3-propyl)phosphono]furanyl}thiazole, major isomer. Mp 102 - 104 °C. Anal. Calcd. for C₂₀H₂₂N₂O₄PSCl + H₂O + 0.2EtOAc: C: 51.14; H: 5.28; N: 5.73. Found: C: 50.86; H: 5.09; N: 5.34.
   **(19.56)** 2-Amino-5-isobutyl-4-{2-[5-(1-(2,4-dichlorophenyl)-1,3-propyl)phosphono]furanyl}thiazole, major isomer. Mp 173-174 °C. Anal. Calcd. for C₂₀H₂₁N₂O₄PSCl₂: C: 49.29; H: 4.34; N: 5.75. Found: C: 49.55; H: 4.32; N: 5.46.
   **(19.57)** 2-Amino-5-isobutyl-4-{2-[5-(1,3-(S,S)-diphenyl)-1,3-propyl)phosphono]furanyl}thiazole. Mp 105 - 107 °C. Anal. Calcd. for C₂₆H₂₇N₂O₄PS + 0.5H₂O + 0.5HCl: C: 59.85; H: 5.51; N: 5.37. Found: C: 59.83; H: 5.18; N: 5.27.
   **(19.58)** 2-Amino-5-isobutyl-4-{2-[5-(1-(4-chlorophenyl)-1,3-propyl)phosphono]furanyl}thiazole, minor isomer. Mp 102 - 104 °C. Anal. Calcd. for C₂₀H₂₂N₂O₄PSCl: C: 53.04; H: 4.90; N: 6.19. Found: C: 52.80; H: 4.70; N: 6.07.
   **(19.59)** 2-Amino-5-isobutyl-4-{2-[5-(1-(3,5-difluorophenyl)-1,3-propyl)phosphono]furanyl}thiazole, minor isomer. Mp 152 - 154 °C. Anal. Calcd. for C₂₀H₂₁N₂O₄PSF₂ + 0.5 H₂O + 0.3 EtOAc: C: 51.98; H: 5.02; N: 5.72. Found: C: 51.67; H: 4.77; N: 5.42.
   **(19.60)** 2-Amino-5-isobutyl-4-{2-[5-(1-(3,5-difluorophenyl)-1,3-propyl)phosphono]furanyl}thiazole, major isomer. Mp 94 - 95 °C. Anal. Calcd. for C₂₀H₂₁N₂O₄PSF₂: C: 52.86; H: 4.66; N: 6.16. Found: C: 52.68; H: 4.73; N: 5.90.
   **(19.61)** 2-Amino-5-isobutyl-4-{2-[5-(1-(3,5-dibromophenyl)-1,3-propyl)phosphono]furanyl}thiazole, major isomer. Mp 113 - 115 °C. Anal. Calcd. for C₂₀H₂₁N₂O₄PSBr₂ + 0.3 EtOAc: C: 42.25; H: 3.91; N: 4.65. Found: C: 42.52; H: 3.91; N: 4.96.
   **(19.62)** 2-Amino-5-isobutyl-4-{2-[5-(1-(3,5-dibromophenyl)-1,3-propyl)phosphono]furanyl}thiazole, minor isomer. Mp 209 - 210 °C. Anal. Calcd. for C₂₀H₂₁N₂O₄PSBr₂: C: 41.69; H: 3.67; N: 4.86. Found: C: 41.93; H: 3.71; N: 4.74.
   **(19.66)** 2-Amino-5-isobutyl-4-{2-[5-(1-(3-pyridyl)-1,3-propyl)phosphono]furanyl}thiazole dihydrochloride. Anal. Calcd. for C₁₉H₂₂N₃O₄PS + 2HCl + 2H₂O: C: 43.19; H: 5.34; N: 7.95. Found: C: 43.10; H: 5.25; N: 7.85.
   **(19.68)** 2-Amino-5-isobutyl-4-{2-[5-(1-oxo-1-phospha-2,5,8-trioxa-3,4-benzo)cyclooctan-1-yl]furanyl}thiazole. Anal. Calcd. for C₁₉H₂₁N₂O₅PS + 0.75 H₂O: C: 52.59; H: 5.23; N: 6.46. Found: C: 52.38; H: 4.85; N: 6.08.

Preferably the cyclic 1,3-propanediol phosphonate esters were prepared using 1,3-dicyclohexylcarbodiimide (DCC) coupling reaction conditions as following.

A suspension of 2-amino-5-isobutyl-4-[2-(5-phosphono)furanyl]thiazole (1 mmole) in DMF:pyridine (5:1, 10 mL) was treated with DCC (2 mmole) followed by 3-(3,5-dichloro)phenyl-1,3-propanediol (1.1 mmole). The resulting mixture was heated at 80°C for 8 h. Evaporation followed by column chromatography gave 2-amino-5-isobutyl-4-{2-[5-(1-(3,5-dichlorophenyl)-1,3-propyl)phosphono]furanyl}thiazole, major isomer. **(19.48)** as a solid.

This method is also useful for the preparation of (5-substituted 2-oxo-1,3-dioxolen-4-yl)methyl and (5-substituted 2-thiocarbonyl-1,3-dioxolen-4-yl)methyl phosphonate prodrugs by coupling of phosphonic acids with 5-methyl-4-hydroxymethyl-2-oxo-1,3-dioxolene and 5-methyl-4-hydroxymethyl-2-thiocarbonyl-1,3-dioxolene (prepared from 4,5-dimethyl-2-oxo-1,3-dioxolene as described in Example 23). The following compound was made using this method.
**(19.19)** 2-Methyl-5-isobutyl-4-{2-[5-(bis(5-methyl-2-thioxo-1,3-dioxolen-4-yl)methyl)-phosphono]furanyl}thiazole. Anal. Calcd. for C₂₂H₂₄NO₈PS₃: C: 47.39; H: 4.34; N: 2.51. Found: C: 47.42; H: 4.30; N: 2.52.

Alternatively, these compounds can be prepared according to reported procedures (*Chem. Pharm. Bull.* **1984**, *32(6)*, 2241) by reaction of phosphonic acids with 5-methyl-4-bromomethyl-2-oxo-1,3-dioxolene in DMF in the presence of sodium hydride at 25 °C.

2-Amino-5-isobutyl-4- {2-[5-bis(3-phthalidyl-2-ethyl)phosphono]furanyl}-thiazole is also prepared following the above described procedures using 2-(3-phthalidyl)ethanol which was prepared from phthalide-3-acetic acid in Example 22.

### Example 20.

### Preparation of acyloxyalkyl and alkyloxycarbonyloxyalkyl phosphonate diesters as prodrugs.

A solution of 2-methyl-4-[2-(5-phosphono)furanyl]thiazole (1 mmole) in acetonitrile and N,N,N-diisopropylethylamine (5 mmole) was treated with pivaloyloxymethyl iodide (4 mmole) at 0 °C for 24 h. Extraction and chromatography gave 2-methyl-4-[2-(5-dipivaloyloxymethylphosphono)furanyl]-thiazole **(20.1).** Anal. Calcd. for C₂₀H₂₈NO₈PS: C: 50.59; H: 6.03; N: 2.65. Found: C: 50.73; H: 5.96; N: 2.96.

The following compounds were prepared according to this procedure:
**(20.2)** 2-Methyl-5-isobutyl-4-{2-[5-(O-isobutyryloxymethyl-O-pivaloyloxymethyl)-phosphono]furanyl}thiazole. Anal. Calcd. for C₂₃H₃₄NO₈PS: C: 53.58; H: 6.65; N: 2.72. Found: C: 53.81; H: 6.83; N: 2.60.
**(20.3)** 2-Methyl-5-isobutyl-4-{2-[5-(dipivaloyloxymethyl)phosphono]furanyl}thiazole. Anal. Calcd. for C₂₄H₃₆NO₈PS: C: 54.43; H: 6.85; N: 2.64. Found: C: 54.46; H: 7.04; N: 2.55.
**(20.4)** 2-Amino-5-isobutyl-4-{2-[5-(dipivaloyloxymethyl)phosphono]furanyl}thiazole. Anal. Calcd. for C₂₃H₃₅N₂O₈PS: C: 52.07; H: 6.65; N: 5.28. Found: C: 52.45; H: 6.78; N: 5.01.
(**20.5**) 2-Bromo-5-isobutyl-4-{2-[5-(dipivaloyloxymethyl)phosphono]furanyl}thiazole. Anal. Calcd. for C₂₃H₃₃NO₈PSBr: C: 47.00; H: 5.75; N: 2.32. Found: C: 47.18; H: 5.46; N:2.30.

The cyclic acyloxyalkyl phosphonate esters can also be prepared in a similar manner according to Farquhar's procedure (Farquhar, D. et al, *Tetrahedron Lett.* **1995**, 36, 655).
**(20.13)** 2-Amino-5-isobutyl-4-{2-[5-(1-benzoyloxypropane-1,3-diyl)phosphono]furanyl} thiazole, more polar isomer. MS calcd for C₂₁H₂₃N₂O₆PS + H:463, found 463.
**(20.14)** 2-Amino-5-isobutyl-4-{2-[5-(1-benzoyloxypropane-1,3-diyl)phosphono]furanyl}thiazole, less polar isomer. MS calcd for C₂₁H₂₃N₂O₆PS + H: 463, found 463.

Alkyloxycarbonyloxyalkyl phosphonate esters were also prepared according to the above procedures with slight modifications described below:
A solution of 2-methyl-5-isobutyl-4-[2-(5-phosphono)furanyl]thiazole (1 mmole) in DMF was treated with N,N'-dicyclohexyl-4-morpholinecarboxamidine (5 mmole) and ethylpropyloxycarbonyloxymethyl iodide (5 mmole) which was prepared from chloromethyl chloroformate according to the reported procedure (Nishimura et al. *J*. *Antibiotics,* **1987**, *40(1)*, 81 - 90). The reaction mixture was stirred at 25 °C for 24 h, and evaporation followed by chromatography gave 2-methyl-5-isobutyl-4-{-2-[5-bis(ethoxycarbonyloxymethyl)phosphono]furanyl}thiazole (**20.6**). Anal. Calcd. for C₂₀H₂₈NO₁₀PS: C: 47.52; H: 5.58; N: 2.77. Found: C: 47.52; H: 5.67; N: 2.80.

The following compounds were prepared according to this procedure:
**(20.7)** 2-Methyl-5-isobutyl-4-{-2-[5-bis(isopropyloxycarbonyloxymethyl)phosphono]-furanyl}thiazole. Anal. Calcd. for C₂₂H₃₂NO₁₀PS: C: 49.53; H: 6.05; N: 2.63. Found: C: 49.58; H: 6.14; N: 2.75.
**(20.8)** 2-Amino-5-isobutyl-4-{-2-[5-bis(phenoxycarbonyloxymethyl)phosphono]-furanyl}thiazole. Anal. Calcd. for C₂₇H₂₇N₂O₁₀PS: C: 53.82; H: 4.52; N: 4.65. Found: C: 54.03; H: 4.16; N: 4.30.
**(20.9)** 2-Amino-5-isobutyl-4-{-2-[5-bis(ethoxycarbonyloxymethyl)phosphono]-furanyl}thiazole. Anal. Calcd. for C₁₉H₂₇N₂O₁₀PS: C: 45.06; H: 5.37; N: 5.53. Found: C: 45.11; H: 5.30; N: 5.43.
**(20.10)** 2-Methyl-5-isobutyl-4-{-2-[S-bis(isopropylthiocarbonyloxymethyl)-phosphono]furanyl}thiazole. Anal. Calcd. for C₂₂H₃₂NO₈PS₃ + 0.2 EtOAc: C: 46.95; H: 5.81; N: 2.40. Found: C: 47.06; H: 5.86; N: 2.73.
(**20.11**) 2-Amino-5-isobutyl-4-{2-[5-bis(isopropyloxycarbonyloxymethyl)phosphono]furanyl}thiazole. Anal. calcd. for C₂₁H₃₁N₂O₁₀PS: C: 47.19; H: 5.85; N: 5.24. Found: C: 47.33; H: 5.66; N:5.57.
(**20.12**) 2-Methyl-5-isobutyl-4-{2-[5-bis(benzoyloxymethyl)phosphono]furanyl}thiazole. Anal. calcd. for C₂₈H₂₈NO₈PS + 0.2CH₂Cl₂: C: 59.31; H: 5.40; N: 2.64. Found: C: 59.25; H: 5.27; N: 2.44.
(**20.15**) 2-Amino-5-isobutyl-4-{2-[5-bis(1-(1-ethoxycarbonyloxy)ethyl)phosphono]-furanyl}thiazole. Mp 76 - 78 °C. Anal. calcd. for C₂₁H₃₁N₂O₁₀PS: C: 47.19; H: 5.85; N: 5.42. Found C: 48.06; H: 5.80; N: 5.16.
2-Amino-5-isobutyl-4-{2-[5-bis(3-(5,6,7-trimethoxy)phthalidyl)-phosphono]furanyl}thiazole is also synthesized following this procedure using 3-bromo-5,6,7-trimethoxyphthalide as the alkylating reagent.

### Example 21.

### Preparation of 3-(2-pyridyl)propan-1,3-diol:

**Step A.** (*J. Org. Chem*., **1957**, *22*, 589) A solution of 3-(2-pyridyl)propanol in acetic acid was treated with 30 % hydrogen peroxide at 80 °C for 16 h. The reaction was concentrated under vacuum and the residue was dissolved in acetic anhydride and heated at 110 °C for 12 h. Evaporation and chromatography gave 3-(2-pyridyl)-1,3-propanediol diacetate.
**Step B.** A solution of 3-(2-pyridyl)-1,3-propanediol diacetate (1 mmole) in methanol-water (3:1) was treated with potassium carbonate (5 mmole) at 25 °C for 3 h. Evaporation and chromatography gave 3-(2-pyridyl)-1,3-propanediol as a solid.

### Example 22.

### Preparation of 3-(2-hydroxyethyl)phthalide.

A solution of phthalide-3-acetic acid (1 mmole) in THF was treated with borane dimethylsulfide (1.5 mmole) at 0 °C for 1 h, and at 25 °C for 24 h. Extraction and chromatography gave 2-(3-phthalidyl)ethanol as a light yellow oil: Rf = 0.25, 50 % EtOAc - hexane.

### Example 23.

### Preparation of 5-methyl-4-hydroxymethyl-2-oxo-1,3-dioxolene.

A solution of 4,5-dimethyl-2-oxo-1,3-dioxolene (1 mmole) and selenium dioxide (2.5 mmole) in dioxane was heated at reflux for 1 h. Evaporation, extraction and chromatography gave 5-methyl-4-hydroxymethyl-2-oxo-1,3-dioxolene as a yellow oil. TLC: Rf = 0.5, 5 % MeOH-dichloromethane.

A solution of 5-methyl-4-hydroxymethyl-2-oxo-1,3-dioxolene (1 mmole) in DMF was treated with tert-butyldimethylsilane (1.2 mmole) and imidazole (2.2 mmole) at 25 °C for 24 h. Extraction and chromatography gave 5-methyl-4-tert-butyldimethylsilyloxymethyl-2-oxo-1,3-dioxolene.

A solution of 5-methyl-4-tert-butyldimethylsilyloxymethyl-2-oxo-1,3-dioxolene (1 mmole) and Lawesson's reagent (1.2 mmole) in toluene was heated to 120 °C for 12 h. Extraction and chromatography gave 5-methyl-4-tert-butyldimethylsilyloxymethyl-2-thio-1,3-dioxolene.

A solution of 5-methyl-4-tert-butyldimethylsilyloxymethyl-2-thio-1,3-dioxolene in methanolic hydrogen chloride was stirred at 0 °C for 1 h and 25 °C for 12 h. Extraction and chromatography gave 5-methyl-4-hydroxymethyl-2-thio-1,3-dioxolene.

### Example 24.

### Preparation of hydroxyethyldisulfidylethylphosphonate diester.

A suspension of 2-methyl-5-isobutyl-4-[2-(5-phosphono)furanyl]thiazole (1 mmole) in thionyl chloride (5 mL) is warmed at reflux for 4 h. The cooled reaction mixture is evaporated to dryness and the resulting yellow residue is treated with a solution of 2-hydroxyethyl disulfide (4 mmole), pyridine (2.5 mmole) in methylene chloride. After stirring at 25 °C for 4 h. the reaction is subjected to extraction and chromatography to give two compounds: 2-methyl-5-isobutyl-4-{2-[5-bis(6'-hydroxy-3',4'-disulfide)hexylphosphono]furanyl}thiazole and 2-methyl-5-isobutyl-4-{2-[5-(3',4'-disulfide)nonacyclicphosphono]-furanyl } thiazole.

### Example 25.

### Preparation of 3-[2-(5-phosphono)furanyl]pyrazoles

**Step A.** A solution of diethyl 5-(2-isobutyl-3-N,N-dimethylamino)acryloyl-2-furanphosphonate (1 mmole, prepared according to Step A of Example 17) in ethanol was treated with hydrazine (1.2 mmole) 80 °C for 12 h. Evaporation and chromatography gave 4-isobutyl-3-[2-(5-diethylphosphono)furanyl]pyrazole.
**Step B.** 4-Isobutyl-3-[2-(5-diethylphosphono)furanyl]pyrazole was subjected to Step C of Example 3 to give 4-isobutyl-3-[2-(5-phosphono)furanyl]pyrazole **(25.1).** mp 210 - 215 °C. Anal. Calcd. for C₁₁H₁₅N₂O₄P: C: 48.89; H: 5.60; N: 10.37. Found: C: 48.67; H: 5.55; N: 10.20.
**Step C.** 4-Isobutyl-3-[2-(5-diethylphosphono)furanyl]pyrazole was subjected to Step A of Example 11 to give 1-methyl-4-isobutyl-3-[2-(5-diethylphosphono)furanyl]pyrazole.
**Step D.** 1-Methyl-4-isobutyl-3-[2-(5-diethylphosphono)furanyl]pyrazole was subjected to Step C of Example 3 to give 1-methyl-4-isobutyl-3-[2-(5-phosphono)furanyl]pyrazole **(25.2).** Anal. Calcd. for C₁₂H₁₇N₂O₄P + 0.85HBr + 0.75 H₂O: C: 39.32; H: 5.32; N: 7.64. Found: C: 39.59; H: 5.30; N: 7.47.

### Example 26.

### Preparation of 3-[2-(5-phosphono)furanyl]isoxazoles

**Step A.** A solution of 5-diethylphosphono-2-furaldehyde (1 mmole) in ethanol was treated with hydroxylamine (1.1 mmole) and sodium acetate (2.2 mmole) at 25 °C for 12 h. Extraction and chromatography gave 5-diethylphosphono-2-furaldehyde oxime.
**Step B.** A solution of 5-diethylphosphono-2-furaldehyde oxime (1 mmole) in DMF was treated with N-chlorosuccinimide (1.1 mmole) at 25 °C for 12 h. Extraction gave 5-diethylphosphono-2-chlorooximidofuran.
**Step C.** A solution of 5-diethylphosphono-2-chlorooximidofuran (1 mmole) and ethyl propiolate (5 mmole) in diethyl ether was treated with triethylamine (2 mmole) at 25 °C for 12 h. Extraction and chromatography gave 5-ethoxycarbonyl-3-{2-(5-diethylphosphono)furanyl]isoxazole.
**Step D.** 5-Ethoxycarbonyl-3-{2-(5-diethylphosphono)furanyl]isoxazole was subjected to Step A of Example 9 followed by Step C of Example 3 to give 5-carbamoyl-3-[2-(5-phosphono)furanyl]isoxazole (**26.1**). mp 221 - 225 °C. Anal. Calcd. for C₈H₇N₂O₆P + 0.25EtOH: C: 37.86; H: 3.18; N: 10.39. Found: C: 37.90; H: 3.02; N: 10.05.

The following compound was prepared according to this procedure:
**(26.2)** 5-Ethoxycarbonyl-4-methyl-3-[2-(5-phosphono)furanyl]isoxazole. mp 150 - 152 °C. Anal. Calcd. for C₁₁H₁₂NO₇P + 0.25H₂O + 0.15HBr: C: 41.57; H: 4.01; N: 4.41. Found: C: 41.57; H: 4.20; N: 4.54.
**(26.3)** 4,5-Bis(ethoxycarbonyl)-3-[2-(5-phosphono)furanyl]isoxazole. Anal. calcd for C₁₃H₁₄NO₉P: C: 43.47; H: 3.93; N: 3.90. Found: C: 43.26; H: 3.92; N: 3.97.
**(26.4)** 5-Amino-4-ethoxycarbonyl-3-[2-(5-phosphono)furanyl]isoxazole. mp 190 °C (decomp). Anal. calcd for C₁₀H₁₁N₂O₇P + 0.25HBr: C: 37.25; H: 3.52; N: 8.69. Found: C: 37.56; H: 3.50; N: 8.85.
**(26.5)** 4,5-bis(carbamoyl)-3-[2-(5-phosphono)furanyl]isoxazole. mp > 220 °C. Anal. calcd for C₉H₈N₃O₇P: C: 35.90; H: 2.68; N: 13.95. Found: C: 35.67; H: 2.55; N: 13.62.
**(26.6)** 4-Ethoxycarbonyl-5-trifluoromethyl-3-[2-(5-phosphono)furanyl]isoxazole. Anal. calcd for C₁₁H₉F₃NO₇P + 0.25HBr: C: 35.20; H: 2.48; N: 3.73. Found: C: 35.25; H: 2.34; N: 3.98.
**(26.7)** 5-Amino-4-(2-furyl)-3-[2-(5-phosphono)furanyl]isoxazole. mp > 220 °C. Anal. calcd for C₁₂H₉N₂O₇P + 0.1AcOEt: C: 44.73; H: 2.97; N: 8.41. Found: C: 45.10; H: 2.58; N: 8.73.
(**26.8**) 4-Amino-5-cyano-3-[2-(5-phosphono)furanyl]isoxazole. Anal. calcd for C₈H₆N₃O₅P + 0.1H₂O + 0.2HBr: C: 35.18; H: 2.36; N: 15.39. Found: C: 35.34; H: 2.50; N: 15.08.
(**26.9**) 4-Cyano-5-phenyl-3-[2-(5-phosphono)furanyl]isoxazole. Anal. calcd for C₁₄H₉N₂O₅P + 0.15HBr: C: 51.21; H: 2.81; N: 8.53. Found: C: 51.24; H: 3.09; N: 8.33.

### Example 27.

### Preparation of 2-[2-(5-phosphono)furanyl]thiazoles

**Step A.** Diethyl 5-tributylstannyl-2-furanphosphonate (**14**) and 2-bromo-4-ethoxycarbonylthiazole was subjected to Step A of Example 6 to give 4-ethoxycarbonyl-2-[2-(5-diethylphosphono)furanyl]thiazole.
**Step B.** 4-Ethoxycarbonyl-2-[2-(5-diethylphosphono)furanyl]thiazole was subjected to Step A of Example 9 followed by Step C of Example 3 to give 4-carbamoyl-2-[2-(5-phosphono)furanyl]thiazole (**27.1**). mp 239 - 240 °C. Anal. Calcd. for C₈H₇N₂O₅PS + 0.2H₂O: C: 34.59; H: 2.68; N: 10.08. Found: C: 34.65; H: 2.69; N: 9.84.

### Example 28.

### Preparation of 4-(3,3-difluoro-3-phosphono-1-propyl)thiazoles

**Step A.** A solution of 3-(tert-butyl-diphenylsilyloxy)-1-propanol (1 mmole) in methylene chloride (7 mL) was treated with powder molecular sieves (4 A, 0.5 equiv. wt/wt) and pyridinium chlorochromate (1.5 mmole) at 0 °C. The resulting mixture was stirred at room temperature for 2 h, and diluted with diethyl ether (7 mL) and stirred at room temperature for another 30 min. Filtration, evaporation and chromatography gave 3-(tert-butyldiphenylsilyloxy)-1-propanal as a clear oil.
**Step B.** A solution of LDA (1.06 mmole) in THF was treated with a solution of diethyl difluoromethylphosphonate (1 mmole) at -78 °C for 45 min. The reaction was then treated with a THF solution of 3-(tert-butyldiphenylsilyloxy)-1-propanal (1.07 mmole) and the resulting soultion was stirred at -78 °C for another 4 h. The reaction was quenched with phenyl chlorothioformate (2.14 mmole), and the reaction mixture was subjected to extraction and chromatography to give diethyl 4-(tert-butyldiphenylsilyloxy)-3-phenoxythiocarbonyloxy-2,2-difluorobutylphosphonate as a clear oil.
**Step C.** A solution of diethyl 4-(tert-butyldiphenylsilyloxy)-3-phenoxythiocarbonyloxy-2,2-difluorobutylphosphonate (1 mmole) in toluene (1 mL) was treated with tri-n-butyltin hydride (1.5 mmole) and AIBN (0.1 mmole), and the resulting reaction mixture was heated to reflux for 2 h. Evaporation and chromatography gave diethyl 4-(tert-butyldiphenylsilyloxy)-2,2-difluorobutylphosphonate as a clear oil.
**Step D.** A solution of diethyl 4-(tert-butyldiphenylsilyloxy)-2,2-difluorobutylphosphonate (1 mmole) in methanol (1 mL) was treated with hydrochloric acid (4 N, 4 mmole) at 0 °C, and the resulting reaction was stirred at room temperature for 2 h. Evaporation and chromatography gave diethyl 4-hydroxy-2,2-difluorobutylphosphonate as a clear oil.
**Step E.** A solution of gave diethyl 4-hydroxy-2,2-difluorobutylphosphonate (1 mmole) in acetone (10 mL) was treated with Jones's reagent (10 mmole) at 0 °C for 30 min. The reaction was quenched with 2-propanol (10 mL), and the resulting mixture was filtered through a Celite pad. Evaporation of the filtrate followed by extraction gave diethyl 3-carboxyl-2,3-difluoropropylphosphonate as an oil.
**Step F.** A solution of diethyl 3-carboxyl-2,3-difluoropropylphosphonate (1 mmole) in thionyl chloride (3 mL) was heated to reflux for 2 h. The reaction was evaporated to dryness, and the residue was dissolved in diethyl ether (1 mL) was treated with an etheral solution of diazomethane (10 mmole) at 0 °C for 30 min. A solution ofHBr in acetic acid (30 %, 1 mL) was added to the reaction, and the resulting solution was stirred at room temperature for 1 h. The reaction was evaporated to dryness and the residue was dissolved in THF-EtOH (1:1, 5 mL) and treated with thiourea (1 mmole). The resulting reaction mixture was heated to 75 °C for 1 h. Evaporation followed by extraction and chromatography gave 2-amino-4-[1-(3-diethylphosphono-3,3-difluoro)propyl]thiazole as a solid, which was subjected to Step C of Example 3 to give gave 2-amino-4-[1-(3-phosphono-3,3-difluoro)propyl]thiazole **(28.1)** as a solid. Anal. Calcd. for C₆H₉N₂O₃PSF₂ + HBr: C: 21.25; H: 2.97; N: 8.26. Found: C: 21.24; H: 3.25; N: 8.21.

The following compound was prepared in a similar manner: 2-Amino-5-methylthio-4-[1-(3-phosphono-3,3-difluoro)propyl]thiazole **(28.2).** MS *m*/*e* 305 (M+H).

### Example 29.

### Preparation of 2-methylthio-5-phosphonomethylthio-1,3,4-thiadiazole and 2-phosphonomethylthiopyridine

**Step A.** A solution of 2-methylthio-1,3,4-thiadiazole-5-thiol (1 mmole) in THF (5 mL) was treated with sodium hydride (60 %, 1.1 mmole) at 0 °C and the resulting mixture was stirred at room temperature for 30 min. The reaction was then cooled to 0 °C and treated with diethylphosphonomethyl trifluoromethanesulfonate (1.1 mmole). After stirring at room temperature for 12 h, the reaction was quenched with saturated ammonium chloride. Extraction and chromatography gave 2-methylthio-5-diethylphosphonomethylthio-1,3,4-thiadiazole as an oil.
**Step B.** 2-Methylthio-5-diethylphosphonomethylthio-1,3,4-thiadiazole was subjected to Step C of Example 3 to give 2-methylthio-5-phosphonomethylthio-1,3,4-thiadiazole **(29.1)** as a yellow solid. Anal. Calcd. for C₄H₇N₂O₃PS₃ + 0.2 HBr: C: 17.50; H: 2.64; N: 10.21. Found: C: 17.64; H: 2.56; N: 10.00.
   Alternatively, phosphonomethylthio substituted heteroaromatics are made using the following method as exemplified by the synthesis of 2-phosphonomethylthiopyridine:

**Step C.** A solution of 2,2'-dipyridyl disulfide (1 mmole) in THF was treated with tri-n-butylphosphine (1 mmole) and diethyl hydroxymethylphosphonate at 0 °C. The resulting reaction solution was stirred at room temperature for 18 h. Extraction and chromatography gave 2-diethylphosphonomethylthiopyridine as a yellow oil.
**Step D.** 2-Diethylphosphonomethylthiopyridine was subjected to Step C of Example 3 to give 2-phosphonomethylthiopyridine **(29.2)** as a yellow solid. Anal. Calcd. for C₆H₈NO₃PS + 0.62 HBr: C: 28.22; H: 3.40; N: 5.49. Found: C: 28.48; H: 3.75; N: 5.14.

### Example 30.

### Preparation of 2-[(2-phosphono)ethynyl]pyridine

**Step A.** A solution of 2-ethynylpyridine (1 mmole) in THF (5 mL) was treated with LDA (1.2 mmole) at 0 °C for 40 min. Diethyl chlorophosphate (1.2 mmole) was added to the reaction and the resulting reaction solution was stirred at room temperature for 16 h. The reaction was quenched with saturated ammonium chloride followed by extraction and chromatography to give 2-[(2-diethylphosphono)ethynyl]pyridine as a yellow oil.
**Step B.** 2-[(2-Diethylphosphono)ethynyl]pyridine was subjected to Step C of Example 3 to give 2-[1-(2-phosphono)ethynyl]pyridine (**30.1**) as a brown solid. Mp 160 °C (decomp). MS *m*/*e* 184 (M + H).

### Example 31.

### A. Preparation of various phosphoramides as prodrugs

**Step A.** A solution of 2-methyl-5-isopropyl-4-[2-(5-phosphono)furanyl]thiazole dichloridate (generated as in Example 19) (1 mmole) in dichloromethane (5 mL) was cooled to 0 °C and treated with a solution of benzyl alcohol (0.9 mmole) in dichloromethane (0.5 mL) and pyridine (0.3 mL). The resulting reaction solution was stirred at 0 °C for 1h, and then added a solution of ammonia (excess) in THF. After stirring at room temperature for 16 h, the reaction was evaporated to dryness and the residue was purified by chromatography to give 2-methyl-5-isopropyl-4-[2-(5-phosphonomonoamido)furanyl]thiazole (31.1) as a yellow hard gum and 2-methyl-5-isopropyl-4-[2-(5-phosphorodiainido)furanyl]thiazole (31.2) as a yellow hard gum.
   **(31.1)** 2-Methyl-5-isopropyl-4-[2-(5-phosphonomonoamido)furanyl]thiazole: MS *m*/*e* 299 (M-H).
   **(31.2)** 2-Methyl-5-isopropyl-4-[2-(5-phosphorodiamido)furanyl]thiazole: MS *m*/*e* 298 (M-H).
   Alternatively, a different method was used to prepare other phosphoramides as exemplified in the following procedure:

**Step B.** A suspension of 2-amino-5-methylthio-4-[2-(5-phosphono)furanyl]thiazole dichloridate (generated as in Example 19) (1 mmole) in dichloromethane (5 mL) was cooled to 0 °C and ammonia (excess) was bubbled through the reaction for 10 min. After stirring at room temperature for 16 h, the reaction was evaporated to dryness and the residue was purified by chromatography to give 2-amino-5-methylthio-4-[2-(5-phosphorodiamido)furanyl]thiazole **(31.3)** as a foam. Anal. Calcd for C₈H₁₁N₄O₂PS₂ + 1.5 HCl + 0.2 EtOH: C: 28.48; H: 3.90; N: 15.82. Found: C: 28.32; H: 3.76; N: 14.21.
   The following compounds were prepared according to the above described procedures or in some cases with minor modifications of these procedures:
   **(31.4)** 2-Amino-5-isobutyl-4-[2-(5-phosphonomonoamido)furanyl]thiazole. Mp 77 -81 °C. Anal. Calcd for C₁₁H₁₆N₃O₃PS + H₂O + 0.8 Et₃N: C: 47.41; H: 7.55; N: 13.30. Found: C: 47.04; H: 7.55; N: 13.67.
   **(31.5)** 2-Amino-5-isobutyl-4-[2-(5-phosphorodiamido)furanyl]thiazole. Anal. Calcd for C₁₁H₁₇N₄O₂PS + 0.5H₂O + 0.75 HCl: C: 39.24; H: 5.61; N: 16.64. Found: C: 39.05; H: 5.43; N: 15.82.
   **(31.28)** 2-Amino-5-isobutyl-4-{2-[5-(N,N'-diisobutyl)phosphoroadiamido]furanyl}-thiazole. Mp 182 - 183 °C. Anal. Calcd. for C₁₉H₃₃N₄O₂PS: C: 55.32; H: 8.06; N: 13.58. Found: C: 54.93; H: 7.75; N: 13.20.
   **(31.29)** 2-Amino-5-isobutyl-4-{2-[5-(N,N'-(1,3-bis(ethoxycarbonyl)-1-propyl)phosphoro)diamido]furanyl}thiazole. Anal. Calcd for C₂₉H₄₅N₄O₁₀PS: C: 51.78: H: 6.74; N: 8.33. Found: C: 51.70; H: 6.64; N: 8.15.
   **(31.30)** 2-Amino-5-isobutyl-4-{2-[5-(N,N'-(1-benzyloxycarbonyl)-1-ethyl)phosphorodiamido]furanyl}thiazole. Anal. Calcd for C₃₁H₃₇N₄O₆PS: C: 59.60; H: 5.97; N: 8.97. Found C: 59.27; H: 5.63; N: 8.74.
   **(31.31)** 2-Amino-5-isobutyl-4-{2-[5-bis(2-methoxycarbonyl-1-azirdinyl)phosphorodiamido]furanyl}thiazole. Anal. Calcd for C₁₉H₂₅N₄O₆PS + 0.3CH₂Cl₂: C: 46.93; H: 5.22; N: 11.34. Found: C: 58.20; H: 5.26; N: 9.25.
   **(31.39)** 2-Amino-5-isobutyl-4-{2-[5-(N,N'-2-(1-ethoxycarbonyl)propyl)phosphorodiamido]furanyl}thiazole. Anal. Calcd for C₂₃H₃₇N₄O₆PS + 0.6EtOAc + 0.1 CH₂Cl₂: C: 51.91; H: 7.18; N: 9.50. Found: C: 51.78; H: 7.17; N: 9.26.
   The monophenyl-monophosphonamide derivatives of compounds of formula I can also be prepared according to the above described procedures:

**Step C.** A solution of 2-amino-S-isobutyl-4-[2-(5-diphenylphosphono)furanyl]thiazole (prepared according to the procedures of Example 19) (1 mmole) in acetonitrile (9 mL) and water (4 mL) was treated with lithium hydroxide (1N, 1.5 mmole) at room temperature for 4 h. The reaction solution was evaporated to dryness, and the residue was dissolved in water (10 mL), cooled to 0 °C and the pH of the solution was adjusted to 4 by addition of 6 N HCl. The resulting white solid was collected through filtration to give 2-amino-5-isobutyl-4-[2-(5-phenylphosphono)furanyl]thiazole (**19.64**).
**Step D.** A suspension of 2-amino-5-isobutyl-4-[2-(5-phenylphosphono)furanyl]thiazole (1 mmole) in thionyl chloride (3 mL) was heated to reflux for 2 h. The reaction solution was evaporated to dryness, and the residue was dissolved in anhydrous dichloromethane (2 mL) and the resulting solution was added to a solution of L-alanine methyl ester hydrochloride (1.2 mmole) in pyridine (0.8 mL) and dichloromethane (3 mL) at 0 °C. The resulting reaction solution was stirred at room temperature for 14 h. Evaporation and chromatography gave 2-amino-5-isobutyl-4-{2-[5-(O-phenyl-N-(1-methoxycarbonyl)ethyl)phosphonamido]furanyl}thiazole (31.6) as an oil. Anal. calcd. for C₂₁H₂₆N₃O₅PS: C: 54.42; H: 5.65; N: 9.07. Found: C: 54.40; H: 6.02; N: 8.87.

The following compounds were prepared according to the above described procedures:
(**31.7**) 2-amino-5-isobutyl-4-{2-[5-(O-phenylphosphonamido)]furanyl}thiazole. mp 205 °C (decomp). Anal. calcd. for C₁₇H₂₀N₃O₃PS + 0.3 H₂O + 0.3 HCl: C: 51.86; H: 5.35; N: 10.67. Found: C: 51.58; H: 4.93; N: 11.08.
(**31.8**) 2-amino-5-isobutyl-4-{2-[5-(O-phenyl-N-ethoxycarbonylmethyl)phosphonamido]furanyl}thiazole. Anal. calcd. for C₂₁H₂₆N₃O₅PS: C: 54.42; H: 5.65; N: 9.07. Found: C: 54.78; H: 5.83; N: 8.67.
**(31.9)** 2-amino-5-isobutyl-4-{2-[5-(O-phenyl-N-isobutyl)phosphonamido]furanyl}thiazole. mp 151 - 152 °C. Anal. calcd. for C₂₁H₂₈N₃O₃PS: C: 58.18; H: 6.51; N: 9.69. Found: C: 58.12; H: 6.54; N: 9.59.
**(31.18)** 2-ammo-5-isobutyl-4-{2-[5-(O-phenyl-N-(1-(1-ethoxycarbonyl-2-phenyl)ethyl)phosphonamido)]furanyl}thiazole. Anal. calcd. for C₂₈H₃₂N₃O₅PS: C: 60.75; H: 5.83; N: 7.59. Found: C: 60.35; H: 5.77; N: 7.37.
**(31.19)** 2-amino-5-isobutyl-4-{2-[5-(O-phenyl-N-(1-(1-ethoxycarbonyl-2-methyl)propyl)phosphonamido)]furanyl}thiazole. Anal. calcd. for C₂₃H₃₀N₃O₅PS: C: 56.20; H: 6.15; N: 8.55. Found: C: 55.95; H: 5.80; N: 8.35.
**(31.20)** 2-amino-5-isobutyl-4-{2-[5-(O-phenyl-N-(1-(1,3-bis(ethoxycarbonyl) propyl)phosphonamido)]furanyl}thiazole. Anal. calcd. for C₂₆H₃₄N₃O₇PS + 0.2 CH₂Cl₂: C: 54.20; H: 5.97; N: 7.24. Found C: 54.06; H: 5.68; N: 7.05.
**(31.21)** 2-amino-5-isobutyl-4-(2-[5-(O-(3-chlorophenyl)-N-(1-(1-methoxycarbonyl)ethyl) propyl)phosphonamido)]furanyl}thiazole. Anal. calcd. for C₂₁H₂₅N₃O₅PSCl: C: 50.65; H: 5.06; N: 8.44. Found: C: 50.56; H: 4.78; N: 8.56.
**(31.22)** 2-amino-5-isobutyl-4-{2-[5-(O-(4-chlorophenyl) -N-(1-(1-methoxycarbonyl)ethyl)phosphonamido)]furanyl}thiazole. Anal. calcd. for C₂₁H₂₅N₃O₅PSCl + 1HCI + 0.2 H₂O: C: 46.88; H: 4.95; N: 7.81. Found: C: 47.33; H: 4.71; N: 7.36.
**(31.23)** 2-amino-5-isobutyl-4-{2-[5-(O-phenyl-N-(1-(1-bis(ethoxycarbonyl)methyl) phosphonamido)]furanyl}thiazole. Anal. calcd. for C₂₄H₃₀N₃O₇PS: C: 53.83; H: 5.65; N: 7.85. Found: C: 53.54 H: 5.63; N: 7.77
**(31.24)** 2-amino-5-isobutyl-4-{2-[5-(O-phenyl-N-(1-morpholinyl) phosphonamido)]furanyl}thiazole. Anal. calcd. for C₂₁H₂₆N₃O₄PS: C: 56.37; H: 5.86; N: 9.39. Found: C: 56.36; H: 5.80; N: 9.20.
(**31.25**) 2-amino-5-isobutyl-4-{2-[5-(O-phenyl-N-(1-(1-benzyloxycarbonyl)ethyl)phosphonamido)]furanyl}thiazole. Anal. calcd. for C₂₇H₃₀N₃O₅PS: C: 60.10; H: 5.60; N: 7.79. Found: C: 59.80; H: 5.23; N: 7.53.
(**31.32**) 2-amino-5-isobutyl-4-{2-[5-(O-phenyl-N-benzyloxycarbonylmethyl)phosphonamido)]furanyl}thiazole. Anal. calcd. for C₂₆H₂₈N₃O₅PS: C: 59.42; H: 5.37; N: 8.00. Found: C: 59.60; H: 5.05; N: 7.91.
(**31.36**) 2-amino-5-isobutyl-4-{2-[5-(O-(4-methyoxyphenyl)-N-(1-(1-methoxycarbonyl)ethyl) phosphonamido)]furanyl}thiazole. Anal. calcd. for C₂₂H₂₈N₃O₆PS + 0.1 CHCl₃ + 0.1 MeCN: C: 52.56; H: 5.62; N: 8.52. Found: C: 52.77; H: 5.23: N: 8.87.
(**31.37**) 2-amino-5-isobutyl-4-{2-[5-(O-phenyl-N-2-methoxycarbonyl) propyl)phosphonamido)]furanyl}thiazole. Anal. calcd. for C₂₂H₂₈N₃O₅PS + 0.6 H₂O: C: 54.11; H: 6.03; N: 8.60. Found: C: 53.86; H: 5.97; N: 8.61.
**(31.38)** 2-amino-5-isobutyl-4-{2-[5-(O-phenyl-N-(2-(1-ethoxycarbonyl)propyl)phosphonamido)]furanyl}thiazole. Anal. calcd. for C₂₃H₃₀N₃O₅PS: C: 56.20; H: 6.15; N: 8.55. Found: C: 55.90; H: 6.29; N: 8.46.

The reaction of a dichlorophosphonate with a 1-amino-3-propanol in the presence of a suitable base (e.g. pyridine, triethylamine) can also be used to prepare cyclic phosphoramidates as prodrugs of phosphonates. The following compounds were prepared in this manner:
(**31.10**) 2-Methyl-5-isobutyl-4-{2-[5-(1-phenyl-1,3-propyl)phosphonamido]furanyl}thiazole minor isomer. Anal. calcd. for C₂₁H₂₅N₂O₃PS + 0.25 H₂O + 0.1 HCl: C: 59.40; H: 6.08; N: 6.60. Found: C: 59.42; H: 5.72; N: 6.44.
(**31.11**) 2-Methyl-5-isobutyl-4-{2-[5-(1-phenyl-1,3-propyl)phosphonamido]furanyl}thiazole major isomer. Anal. calcd. for C₂₁H₂₅N₂O₃PS + 0.25 H₂O: C: 59.91; H: 6.11; N: 6.65. Found: C: 60.17; H: 5.81; N: 6.52.
(**31.12**) 2-Amino-5-isobutyl-4-{2-[5-(1-phenyl-1,3-propyl)phosphonamido]furanyl}thiazole major isomer. Anal. calcd. for C₂₀H₂₄N₃O₃PS + 0.25 H₂O + 0.1 CH₂Cl₂: C: 55.27; H: 5.72; N: 9.57. Found: C: 55.03; H: 5.42; N: 9.37.
(**31.13**) 2-Amino-5-isobutyl-4-{2-[5-(1-phenyl-1,3-propyl)phosphonamido]furanyl}thiazole minor isomer. Anal. calcd. for C₂₀H₂₄N₃O₃PS + 0.15 CH₂Cl₂: C: 56.26; H: 5.69; N: 9.77. Found: C: 56.36; H: 5.46; N: 9.59.
(**31.14**) 2-Amino-5-methylthio-4-{2-[5-(1-phenyl-1,3-propyl)phosphonamido]furanyl}thiazole less polar isomer. Anal. calcd. for C₁₇H₁₈N₃O₃PS₂ + 0.4 HCl: C: 48.38; H: 4.39; N: 9.96. Found: C: 48.47; H: 4.21; N: 9.96.
(**31.15**) 2-Amino-5-methylthio-4-{2-[5-(1-phenyl-1,3-propyl)phosphonamido]furanyl}thiazole more polar isomer. Anal. calcd. for C₁₇H₁₈N₃O₃PS₂: C: 50.11; H: 4.45; N: 10.31. Found: C: 49.84; H: 4.19; N: 10.13.
(**31.16**) 2-Amino-5-methylthio-4-{2-[5-(N-methyl-1-phenyl-1,3-propyl)phosphonamido]furanyl}thiazole. Anal. calcd. for C₁₈H₂₀N₃O₃PS₂ + 0.25 HCl: C: 50.21; H: 4.74; N: 9.76. Found: C: 50.31; H: 4.46; N: 9.79.
(**31.17**) 2-Amino-5-methylthio-4-{2-[5-(1-phenyl-1,3-propyl)-N-acetylphosphonamido]furanyl}thiazole. Anal. calcd. for C₂₂H₂₆N₃O₄PS + 1.25 H₂O: C: 54.82; H: 5.96; N: 8.72. Found: C: 55.09; H: 5.99; N: 8.39.
**(31.26)** 2-amino-5-isobutyl-4-{2-[5-(1-oxo-1-phospha-2-oxa-7-aza-3,4-benocycloheptan-1-yl)]furanyl}thiazole, major isomer. Mp 233 - 234 °C. Anal. calcd. for C₂₁H₂₄N_{3O}O₅PS + 0.2 CHCl₃: C: 52.46; H: 5.03; N: 8.66. Found C: 52.08; H: 4.65; N: 8.58.
**(31.27)** 2-amino-5-isobutyl-4-{2-[5-(1-oxo-1-phospha-2-oxa-7-aza-3,4-benocycloheptan-1-yl)]furanyl}thiazole, minor isomer. MS calcd. for C₂₁H₂₄N₃O₅PS + H: 462, found 462.
**(31.34)** 2-amino-5-isobutyl-4-{2-[5-(3-(3,5-dichlorophenyl)-1,3-propyl)phosphonamido]furanyl}thiazole. Anal. calcd. for C₂₀H₂₂N₃O₃PSCl₂: C: 49.39; H: 4.56; N: 8.64. Found: C: 49.04; H: 4.51; N: 8.37.
**(31.35)** 2-amino-5-isobutyl-4-{2-[5-(4,5-benzo-1-oxo-1-phospha-2-oxa-6-aza)cyclohexan-1-yl]furanyl}thiazole. Anal. calcd. for C₁₈H₂₀N₃O₃PS + 0.7 H₂O: C; 53.78; H: 5.37; N: 10.45. Found C: 53.63; H: 5.13; N: 10.36.

### Example 32.

### Preparation of 5-[2-(5-phosphono)furanyl]tetrazole.

**Step A.** To a mixture of tetrazole (1 mmole) and powdered K₂CO₃ (1.5 mmole) in 1 mL DMF cooled to 0 °C was added benzyl chloromethyl ether (1.2 mmole) and the resulting mixture stirred for 30 min at 0 °C and then for 16 h at rt. The mixture was diluted with water and ether. Extraction and chromatography provided 2-benzyloxymethyltetrazole as a colorless oil.
**Step B.** To a solution of 2-benzyloxymethyltetrazole (1 mmole) and TMEDA (2 mmole) in 3 mL diethyl ether at -78 °C was added n-BuLi in hexanes (1 mmole). This was let stir for 5 min at -78 °C and then it was added to a precooled (-78 °C) solution of (*n*-Bu)₃SnCl (1 mmole) in 2 mL of diethyl ether. After stirring at -78 °C for 30 min it was diluted with water and diethyl ether. Extraction and chromatography provided 2-benzyloxymethyl-5-(tributylstannyl)tetrazole as a colorless oil.
**Step C.** A mixture of 5-iodo-2-diethylphosphonofuran (1 mmole), 2-benzyloxymethyl-5-(tributylstannyl)tetrazole (1.05 mmole), tetrakis(triphenylphosphine) palladium(O) (0.03 mmole) and copper(I) iodide (0.07 mmole) in 3 mL of toluene was refluxed at 110 °C for 20 h. Evaporation and chromatography provided 2-benzyloxymethyl-5-[2-(5-diethylphosphono)furanyl]tetrazole as an oil.
**Step D.** A mixture of 2-benzyloxymethyl-5-[2-(5-diethylphosphono)furanyl]tetrazole (1 mmole) and 6 M HCl (1 mL) in 10 mL ethanol was heated at 70 °C for 20 h and then the solvent concentrated by evaporation, made basic with 1 N NaOH and extracted with EtOAc. The aqueous layer was made acidic and extracted with EtOAc. This EtOAc extract was evaporated to provide 5-[2-(5-diethylphosphono)furanyl]tetrazole as a solid, which was subjected to Step C of Example 3 to give 5-[2-(5-phosphono)furanyl]tetrazole (32.1) as a solid: mp 186-188 °C. Anal. calcd. for C₅H₅N₄O₄P + 1.5 H₂O: C, 24.70; H, 3.32; N, 23.05. Found: C, 24.57; H, 2.57; N: 23.05.
**Step E.**
   **Step 1.** A mixture of 5-[2-(5-diethylphosphono)furanyl]tetrazole (1 mmole), 1-iodo-2-methylpropane (2 mmole) and powdered K₂CO₃ (2 mmole) in 5 mL DMF was stirred at 80 °C for 48 h and then diluted with CH₂Cl₂ and water and the layers separated. The CH ₂Cl₂ layer was evaporated and combined with the product of the following reaction for chromatography.
   **Step 2.** The aqueous layer of Step 1 was made acidic and extracted with EtOAc. This extract was evaporated and the residue heated at 80 °C in 2 mL of SOCl₂ for 3 h and then the solvent evaporated. The residue was dissolved in 5 mL CH₂CI₂ and 0.3 mL NEt₃ and 0.5 mL of EtOH was added. After stirring for 1 h at rt the mixture was diluted with CH₂Cl₂ and water. This organic extract was combined with that kept from Step 1 and chromatography provided 1-isobutyl-5-[2-(5-diethylphosphono)furanyl]tetrazole and 2-isobutyl-5-[2-(5-diethylphosphono)furanyl]tetrazole each as an oil.
   **Step 3.** I-Isobutyl-5-[2-(5-dicthylphosphono)furanyl]tetrazole was subjected to Step C of Example 3 to give 1-isobutyl-5-[2-(5-phosphono)furanyl]tetrazole (32.2) as a solid: mp 200-202 °C. Anal. calcd. for C₉H₁₃N₄O₄P: C: 39.71; H: 4.81; N: 20.58. Found: C: 39.64; H: 4.63; N: 20.21.
**Step F.** A mixture of 2-isobutyl-5-[2-(5-diethylphosphono)furanyl]tetrazole (1 mmole) and TMSBr (10 mmole) in 10 mL of CH₂Cl₂ was stirred at room temperature for 16 h. The solvent was evaporated and the residue dissolved in 10:1 CH₃CN:water, the solvent evaporated and the residue precipitated from acetone by addition of dicyclohexylamine (2 mmole) to provide 2-isobutyl-5-[2-(5-phosphono)furanyl]tetrazole N,N-dicyclohexyl ammonium salt.
   (**32.3**) as a solid: mp 226-228 °C. Anal. calcd. for C₉H₁₃N₄O₄P + C₁₂H₂₃N: C: 55.62; H: 8.00; N: 15.44. Found: C: 55.55; H: 8.03; N: 15.07.

### Example 33.

### High throughput synthesis of various 2-(5-phosphono)furanyl substituted heteroaromatic compounds.

**Step A.** Various 2-(5-diethylphosphono)furanyl substituted heteroaromatic compounds were prepared in a similar manner as Step B of Example 15, and some of these compounds were used for the high throughput synthesis of compounds listed in Table 33.1 and Table 33.2.
**Step B.** A mixture of 2-chloro-6-[2-(5-diethylphosphono)furanyl]pyridine (0.01 mmole) and TMSBr (0.1 mL) in CH₂CI₂ (0.5 mL) was stirred at room temperature for 16 h and then evaporated and diluted with 0.5 mL of 9:1 CH₃CN:water. Evaporation provided 2-chloro-6-[2-(5-phosphono)furnayl]pyridine.
**Step C.** A mixture of 2-chloro-6-[2-(5-diethylphosphono)furanyl]pyridine (0.01 mmole) and a solution of freshly prepared sodium propoxide in propanol (0.25 M, 0.4 mL) was let sit at 85 °C for 14 h. The reaction mixture was evaporated and the residue was subjected to Step B of Example 33 to give 2-propyloxy-6-[2-(5-phosphono)furanyl]pyridine.
**Step D.** A mixture of 2-chloro-6-[2-(5-diethylphosphono)furanyl]pyridine (0.01 mmol) and 1-methylpiperazine (0.2 mL) in ethylene glycol (0.2 mL) was heated at 145 °C for 24 h. The mixture was further diluted with 0.5 mL of CH₃CN and 0.1 mL of water and then 150 mg of Dowex 1 2-100 formate resin was added. After stirring this mixture 30 min it was filtered and the resin washed with DMF (2 10 min), CH₃CN (2 10 min) and then 9:1 CH₃CN:water (1 10 min). Finally the resin was stirred with 9:1 TFA:water for 30 min, filtered and the filtrate evaporated. The residue obtained subjected to Step B of example to give 2-[1-(4-methyl)piperazinyl]-6-[2-(5-phosphono)furanyl]pyridine.
**Step E.** A mixture of 3-chloro-5-[2-(5-diethylphosphono)furanyl]pyrazine (0.01 mmole), 5-tributylstannylthiophene (0.04 mmole), Pd(PPh₃)₄ (0.001 mmole) and CuI (0.002 mmole) in dioxane (0.5 mL) was heated at 85 °C for 16 h then the solvent was evaporated. The resulting residue and TMSBr (0.1 mL) in 0.5 mL CH₂Cl₂ was stirred at rt for 16 h and then evaporated and diluted with 0.5 mL of 9:1 CH₃CN:water. To this solution 150 mg of Dowex 1 2-100 formate resin was added and after stirring 30 min it was filtered and the resin washed with DMF (2 10 min), CH₃CN (2 10 min) and then 9:1 CH₃CN:water (1 10 min). Finally the resin was stirred with 9:1 TFA:water for 30 min, filtered and the filtrate evaporated to give 3-(2-thienyl)-5-[2-(5-phosphono)furnayl]pyrazine.
**Step F.** A mixture of 3-chloro-5-[2-(5-diethylphosphono)furanyl]pyrazine (0.01 mmole), 1-hexyne (0.04 mmole), diisopropylethylamine (0.1 mmole), Pd(PPh₃)₄ (0.001 mmole) and CuI (0.002 mmole) in dioxane (0.5 mL) was heated at 85 °C for 16 h then the solvent was evaporated. The resulting residue was subjected to Step B to give 3-(1-hexyn-1-yl)-5-[2-(5-phosphono)furanyl]pyrazine.

### Preparation of the Carboxyrnethylphosphonate Resin

**Step G.** A solution of trimethylphosphonoacetate (30.9 mmol), 2-(trimethylsiyl) ethanol (10.4 mmol) and DMAP (3.1 mmol) in toluene (25 mL) was refluxed for 48 h under N₂. After cooling, the solution was diluted with EtOAc and washed with 1N HCl followed by water. The organic solution was dried over sodium sulfate and concentrated under vacuum to give an oil. The residue was treated with LiI (10.4 mmol) in 2-butanone (30 mL), and refluxed overnight under N₂. The solution was diluted with EtOAc, washed with 1N HCl, dried over Na₂SO₄ and concentrated under vacuum to afford the SEM protected carboxy monomethylphosphonate as a colorless oil.
**Step H****.** Hydroxymethylpolystyrene (2.35 mmol) was prepared for coupling by combining with anhyrous THF (40 mL), gently skaking for 20 min. and then removing the excess solvent by cannula. This procedure was repeated 3 times. The swollen resin was then suspended in THF (40 mL) and DIPEA (21.2 mmol). To this mixture was added, by cannula, a solution of the SEM protected carboxy monomethylphosphonate (prepared in Step G) (7.1 mmol), DIAD (7.1 mmol) and tris(4-chlorophenyl)phosphine (7.1 mmol) in THF (15 mL) which had been stirred for 15 min. prior to addition. After shaking the mixture overnight under a blanket of N₂, the resin was filtered, rinsed with THF ( 3x 40 mL), DMF (3 x 40 mL), and THF again (3 x 40 mL) before drying under vacuum to afford 3.8 g of the coupled phosphonate resin.
**Step I****.** To coupled phosphonate resin (2.41 mmol) in THF (100 mL) was added 1M TBAF in THF solution (12 mL). The mixture was shaken overnight before being filtered and the resin rinsed with THF (3 x 40 mL) to afford the desired carboxymethylphosphonate resin as the tetrabutylammonium salt.

### Coupling of the carboxymethylphosphonate resin to a heteroaromatic amine

**Step J****.** In a 2 mL well, a heteroaromatic amine (0.14 mmol), resin (0.014 mmol), PyBOP (0.14 mmol) and TEA (0.36 mmol) in DMF (1.45 mL) were combined and shaken for 48 h at room temperature. The treated resin was then filtered, washed with DMF (3x) and CH₂Cl₂ (3x). The isolated resin was resuspended in CH₂Cl₂ (900 L), combined with TMSBr (100 L) and mixed for 6 h. The mixture was filtered, the resin washed with anhydrous CH₂Cl₂ (500 L) and the filtrate concentrated under vacuum. To the isolated residue was added a solution of CH₃CN/H₂O (9:1, 300 L). After shaking for 30 min. the solvents were removed to provide the desired [{N-(phosphono)acetyl]amino} substituted heteroaromatic analogs. Compounds **33.97 - 33.119** and **33.146 - 33.164** were synthesized according to these procedures and they are listed in Table 33.1 and Table 33.2.

### Preparation of the Aminomethylphosphonate Resin

**Step K****.** To a solution of dimethyl phthalimidomethylphosphonate (37 mmole) in 2-butanone (150 mL) was added LiI (38.9 mmol). After refluxing overnight under N₂, the solution was diluted with EtOAc, washed with 1N HCI, dried over MgSO₄ and concentrated under vacuum to afford monomethyl phthalimidomethylphosphonate as a white solid.
**Step L****.** As described above in Step H, monomethyl phthalimidomethylphosphonate was coupled to hydroxymethylpolystyrene to give the resin-coupled phthalimidomethylphosphonate monomethyl ester.
**Step M.** To the resin-coupled phthalimidomethylphosphonate monomethyl ester (6.8 mmol) in DMF (7 mL) was added anhydrous hydrazine (3 mL). After shaking at room temperature for 24 h the resin was filtered, rinsed with DMF (3 x 10 mL), CH₂Cl₂ (3 x 10 mL) and then dried under vacuum to afford 832 mg the desired resin-coupled aminomethylphosphonate monomethyl ester.

### Coupling of various heteroaromatic carboxylic acids to the resin-coupled aminomethylphosphonate monomethyl ester.

**STEP N**. In a 2 mL well, a heteroaromatic carboxylic acid (0.2 mmol), resin (0.02 mmol), EDC (0.2 mmol) and HOBT (0.2 mmol) in DMF (0.5 mL) were combined and shaken for 24 h at room temperature, The treated resin was then filtered, washed with DMF (3x) and CH₂Cl₂ (3x). The isolated resin was resuspended in CH₂Cl₂ (500 L), combined with TMSBr (50 L) and mixed for 6 h. The mixture was filtered, the resin washed with anhydrous CH₂Cl₂ (500 L) and the filtrate concentrated under vacuum. To the isolated residue was added a solution of CH₃CN/H₂O (9:1, 300 L). After shaking for 30 min the solvents were evaporated to provide the desired (N-phosphonomethyl)carbamoyl substituted heteroaromatic analogs. Compounds **33.120** - **33.145** were synthesized according to these procedures and they are-listed in Table 33.2.

The following compounds were prepared according to some or all of the above described procedures. These compounds were characterized by HPLC (as described below) and mass spectroscopy (APCI negative ion), and these characterization data are listed in Table 33.1 and Table 33.2.
HPLC was performed using a YMC ODS-Aq, Aq-303-5, 250 4.6 mm ID, S-5 µm, 120 A column with the UV detector set at 280 nm.

| HPLC Elution Program: 1.5 mL/min flow rate | | |
|---|---|---|
| Time (min) | % Acetonitrile (A) | % Buffer^{a} (B) |
| 0 | 10 | 90 |
| 7.5 | 90 | 10 |
| 12.4 | 90 | 10 |
| 12.5 | 10 | 90 |
| 15 | 10 | 90 |

| | | |
|---|---|---|
| ^{a} Buffer = 95:5:0.1 water:methanol:acetic acid | | |

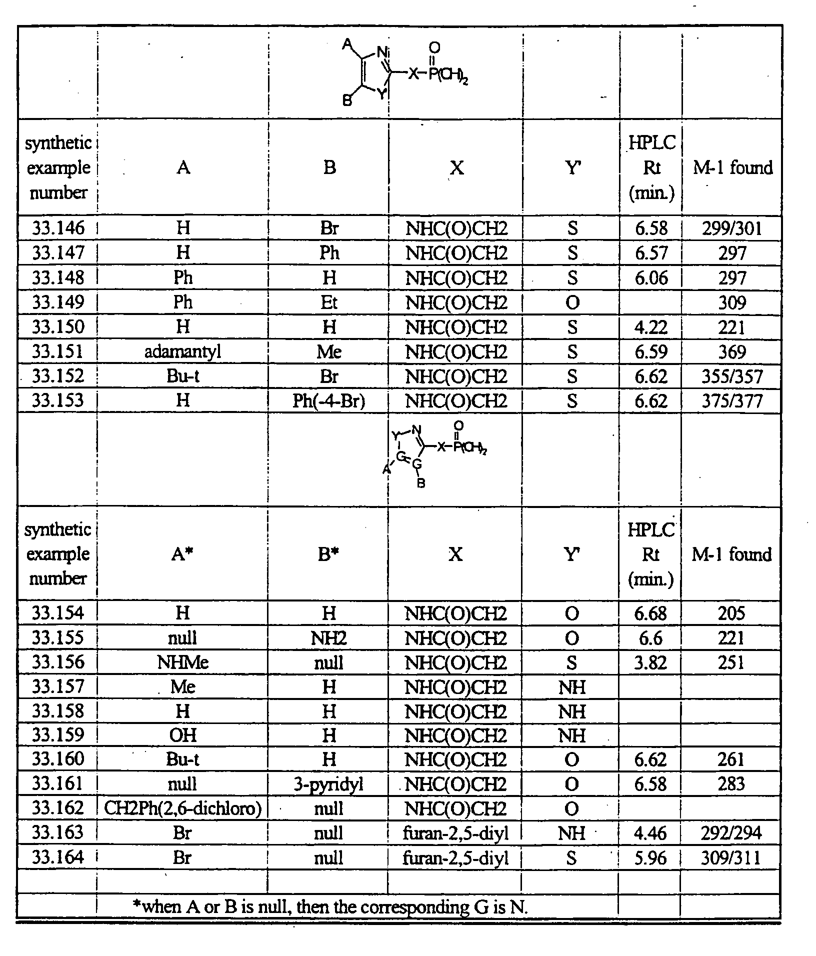

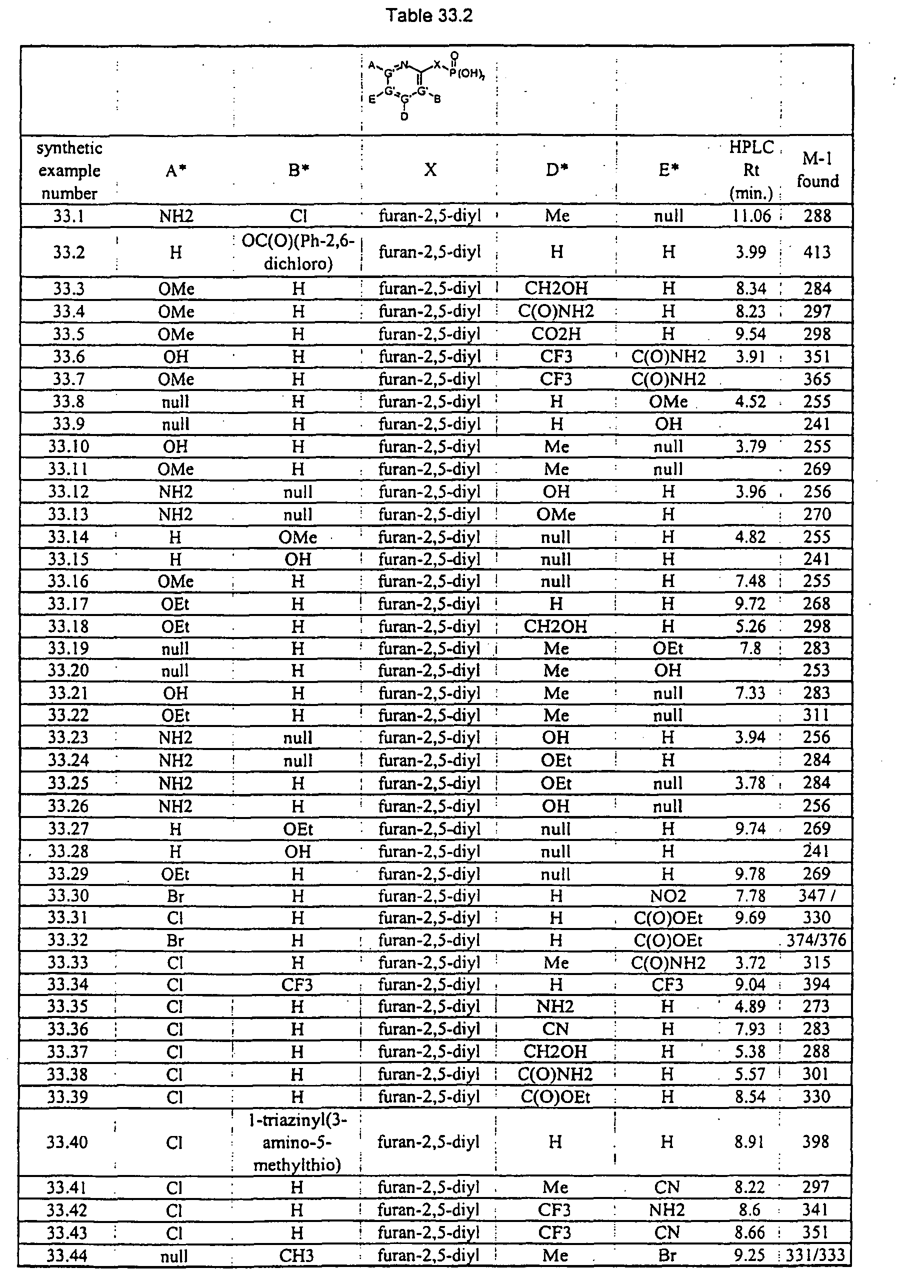

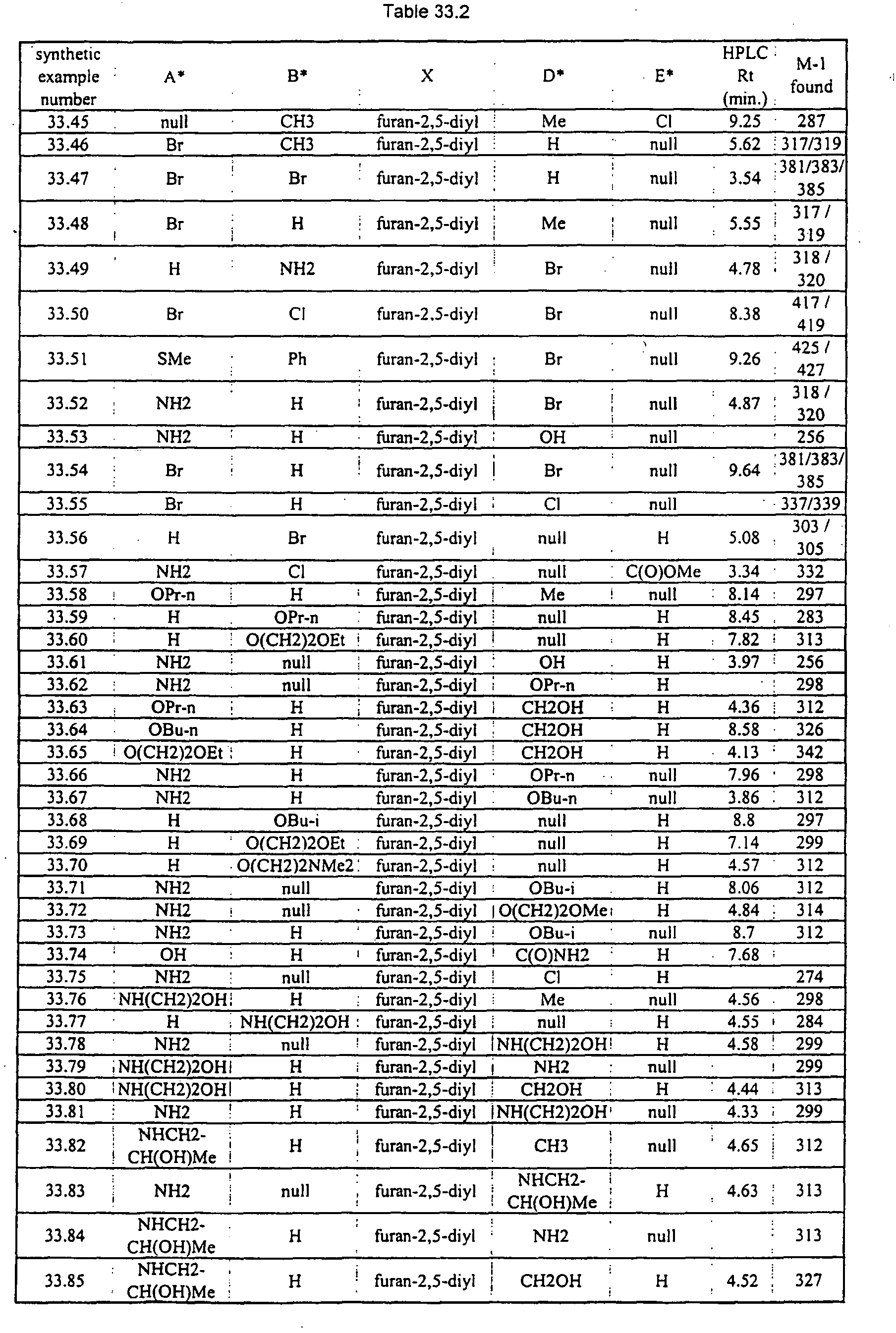

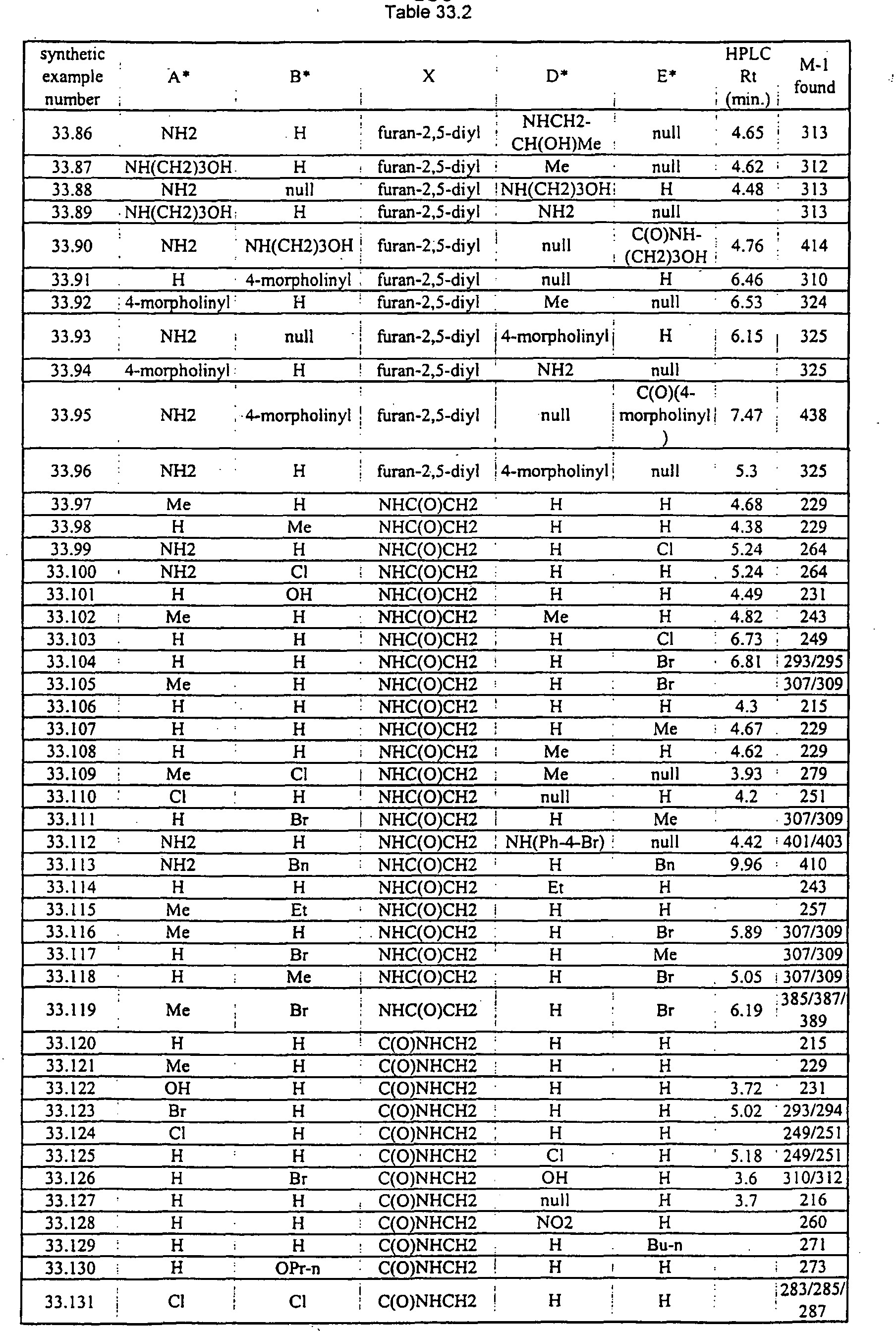

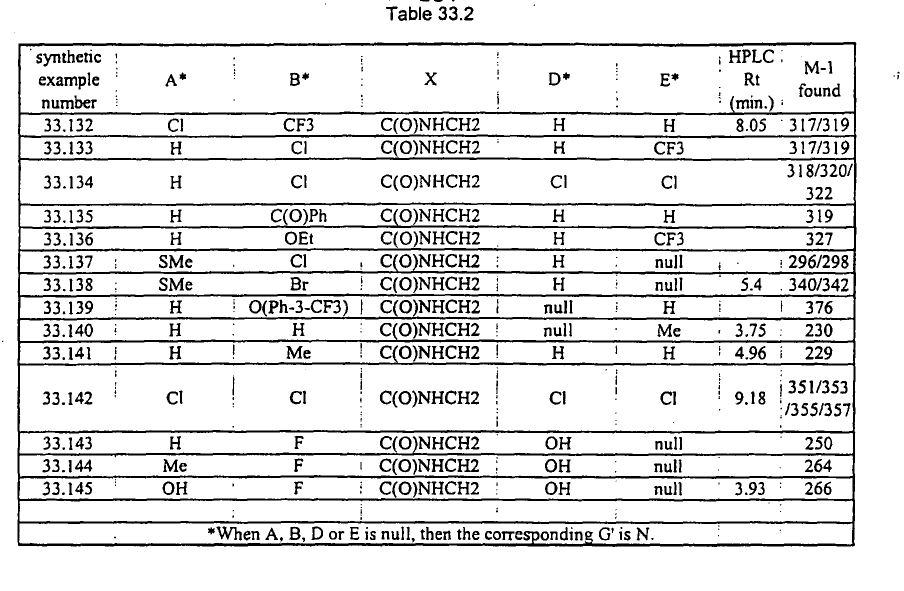

### Section 2.

### Synthesis of Compounds of Formula X

### Example 34.

### Preparation of 2-amino-4-phosphonomethyloxy-6-bromobenzothiazole.

**Step A.** A solution of AlCl₃ (5 mmole) in EtSH (10 mL) was cooled to 0 °C and treated with 2-amino-4-methoxybenzothiazole (1 mmole). The mixture was stirred at 0-5 °C for 2 h. Evaporation and extraction gave 2-amino-4-hydroxybenzothiazole as white solid.
**Step B.** A mixture of2-amino-4-hydroxybenzothiazole (1 mmole) and NaH (1.3 mmole) in DMF (5 mL) was stirred at 0 °C for 10 min, and then treated with diethylphosphonomethyl trifluoromethylsulfonate (1.2 mmole). After being stirred at room temperature for 8 h, the reaction was subjected to extraction and chromatography to give 2-amino-4-diethylphosphonomethyloxybenzothiazole as an oil.
**Step C.** A solution of 2-amino-4-(diethylphosphonomethyloxy)benzothiazole (1 mmole) in AcOH (6 mL) was cooled to 10 °C and treated with bromine (1.5 mmole) in AcOH (2 mL). After 5 min the mixture was stirred at room temperature for 2.5 h. The yellow precipitate was collected via filtration and washed with CH₂Cl₂ to give 2-amino-4-diethylphosphonomethyloxy-6-bromobenzothiazole.
**Step D.** A solution of 2-amino-4-diethylphosphonomethyloxy-6-bromobenzothiazole (1 mmole) in CH₂Cl₂ (4 mL) was treated with TMSBr (10 mmole) at 0 °C. After stirred for 8 h at room temperature the reaction was evaporated to dryness and the residue was taken into water (5 mL). The resulting precipitate was collected via filtration and washed with water to give 2-amino-4-phosphonomethyloxy-6-bromobenzothiazole (34.1) as white solid. mp >220 °C(dec.). Anal. Calcd. for C₈H₈N₂O₄PSBr: C:28.34; H:2.38; N:8.26. Found: C:28.32; H:2.24; N:8.06.

Similarly, the following compounds were prepared according to the above described procedures:
**(34.2)** 2-Amino-4-phosphonomethyloxybenzothiozole. mp >250 °C. Anal. Calcd. for C₈H₉N₂O₄PS + 0.4 H₂O: C:35.93; H:3.69; N:10.48. Found: C:35.90; H:3.37; N:10.37.

### Example 35.

### Preparation of 2-amino-4-phosphonomethyloxy-6-bromo-7-chlorobenzothiazole.

**Step A.** A solution of 1-(2-methoxy-5-chlorophenyl)-2-thiourea (1 mmole) in chloroform (10 mL) was cooled to 10 °C and treated with bromine (2.2 mmole) in chloroform (10 mL). The reaction was stirred at 10 °C for 20 min and at room temperature for 0.5 h. The resulting suspension was heated at reflux for 0.5 h. The precipitate was collected via filtration (washed with CH₂Cl₂) to give 2-amino-4-methoxy-7-chlorobenzothiazole which was subjected to Steps A, B, C and D of Example 34 to give 2-amino-4-phosphonomethoxy-6-bromo-7-chloro benzothiazole (**35.1**). mp >220 °C(dec.). Anal. Calcd. for C₈H₇N₂O₄PSClBr: C:25.72; H:1.89; N:7.50. Found: C:25.66; H:1.67; N:7.23.

Similarly, the following compounds were prepared according to the above described procedures:
**(35.2)** 2-Amino-4-phosphonomethoxy-6-bromo-7-methyl benzothiazole. mp >220 °C (dec.). Anal. Calcd. for C₉H₁₀N₂O₄PSBr: C:30.61; H:2.85; N:7.93 Found: C:30.25; H:2.50; N:7.77.
**(35.3)** 2-Amino-4-phosphonomethoxy-7-methylbenzothiazole. mp >220 °C (dec.). Anal. Calcd. for C₉H₁₁N₂O₄PS+1.0 H₂O: C:36.99; H:4.48; N:9.59. Found: C:36.73; H:4.23; N:9.38.
**(35.4)** 2-Amino-4-phosphonomethoxy-7-chlorobenzothiazole. mp >220 °C(dec.). Anal. Calcd. for C₈H₈N₂O₄PSCl + 0.1H₂O: C:32.41; H:2.79; N:9.45. Found: C:32.21; H:2.74; N:9.22.

### Example 36.

### Preparation of 2-Amino-4-phosphonomethoxy-5,6,7,8-tetrahydronaphtho[1,2-d]thiazole.

**Step A.** 3-Amino-2-hydroxy-5,6,7,8-tetrahydronaphthalene was subjected to Step B of Example 34 to give 3-amino-2-diethylphosphonomethyloxy-5,6,7,8-tetrahydronaphthlene.
**Step B.** A solution of KSCN (16 mmole) and CuSO₄ (7.7 mmole) in MeOH (10 mL) was treated with a solution of3-amino-2-diethylphosphonomethyloxy-5,6,7,8-tetrahydronaphthalene (1 mmole) in MeOH (5 mL) at room temperature. The mixture was heated at reflux for 2 h. Filtration, extraction and chromatography provided 2-amino-4-diethylphosphonomethyloxy-5,6,7,8-tetrahydronaphtho[1,2-d]thiazole as light brown solid.
**Step C.** 2-Amino-4-diethylphosphonomethyloxy-5,6,7,8-tetrahydronaphtho[1,2-d]thiazole was subjected to Step D of Example 34 to give 2-Amino-4-phosphonomethoxy-5,6,7,8-tetrahydronaphtho[1,2-d]thiazole (**36.1**). mp >220 °C (dec.). Anal. Calcd. for C₁₂H₁₅N₂O₄PS + 0.5 H₂O: C:45.86; H:4.81; N:8.91 Found: C:44.68; H:4.77; N:8.73.
   The following compounds were also prepared according to above procedures:
   **(36.2)** 2-Aniino-4-phosphonomethoxy-[1,2-d]naphthothiazole. mp >240 °C(dec.). Anal. Calcd. for C₁₂H₁₁N₂O₄PS + 0.2HBr: C:44.15; H:3.46; N:8.58. Found: C:44.13; H:3.46; N:8.59.
   (**36.3**) 2-Amino-5,7-dimethyl-6-thiocyanato-4-phosphonomethoxybenzothiazole. mp >240 °C(dec.). Anal. Calcd. for C₁₁H₁₂N₃O₄PS₂ + 0.2CH₂Cl₂: C:37.13; H:3.45; N:11.60. Found: C:37.03; H:3.25; N:11.65.

### Example 37.

### Preparation of 2-Amino-7-methoxy-6-thiocyanato-4-phosphonomethoxy-benzothiazole.

**Step A.** 2-Hydroxy-5-methoxynitrobenzene was subjected to Step B of Example 34 to give 2-diethylphosphonomethyloxy-5-methoxynitrobenzene.
**Step B.** A solution of SnCl₂ (4 mmole) in freshly prepared methonolic HCl (10 mL) was added to a cold (0 °C) solution of 2-diethylphosphonomethyloxy-5-methoxynitrobenzene (1 mmole) in MeOH (5 mL). The mixture was warmed to room temperature and stirred for 3 h. Evaporation, extraction and chromatography provided 2-diethylphosphonomethyloxy-5-methoxyaniline.
**Step C.** 2-Diethylphosphonomethyloxy-5-methoxyaniline was subjected to Step B of Example 36 to give 2-amino-4- diethylphosphonomethyloxy-6-thiocyano-7-methoxybenzothiazole, which was subjected to Step D of Example 34 to give 2-amino-7-methoxy-6-thiocyanato-4-phosphonomethoxybenzothiazole **(37.1).** mp >170 °C(dec.). Anal. Calcd. for C₁₀H₁₀N₃O₅PS₂: C:34.58; H:2.90; N:12.10. Found: C:34.23; H:2.68; N:11.77.

Similarly, the following compounds were prepared according to above procedures:
**(37.2)** 2-Amino-5,6-difluoro-4-phosphonomethoxybenzothiazole. mp >240 °C(dec.). Anal. Calcd. for C₈H₇N₂O₄PSF₂: C:32.44; H:2.38; N:9.46. Found: C:32.30; H:2.26; N:9.17.
**(37.3)** 2-Amino-5-fluoro-7-bromo-4-phosphonomethoxybenzothiazole. mp >190°C(dec.). Anal. Calcd. for C₈H₇N₂O₄PSBrF: C:26.91; H:1.98; N:7.84. Found: C:27.25; H:1.92; N:7.54.
**(37.4)** 2-Amino-7-ethoxycarbonyl-4-phosphonomethoxybenzothiazole. mp >240 °C(dec.). Anal. Calcd. for C₁₁H₁₃N₂O₆PS + 0.2HBr + 0.1DMF: C:38.15; H:3.94; N:8.27. Found: C:38.51; H:3.57; N:8.66.

### Example 38.

### Preparation of 2-Amino-7-bromo-6-thiocyanato-4-phosphonomethoxy benzothiazole.

**Step A.** A solution of 2-fluoro-5-bromonitrobenzene (1 mmole) in DMF (5 mL) was cooled to 0 °C, and treated with a solution of freshly prepared sodium salt of diethylhydroxymethylphosphonate (1.2 mmole) in DMF (5 mL). The mixture was stirred at room temperature for 16 h. Evaporation, extraction and chromatography provided 2-diethylphosphonomethyloxy-5-bromonitrobenzene.
**Step B.** 2-Diethylphosphonomethyloxy-5-bromonitrobenzene was subjected to Step B of Example 37, Step B of Example 36, and Step D of Example 34 to give 2-amino-7-bromo-6-thiocyanato-4-phosphonomethoxybenzothiazole **(38.1).** mp >250°C(dec.). Anal. Calcd. for C₉H₇N₃O₄PS₂ Br: C:27.29; H:1.78; N:10.61. Found: C:26.90; H:1.58; N:10.54.

Similarly, the following compound was prepared according to above procedures: (**38.2**) 2-Amino-7-fluoro-6-thiocyanato-4-phosphonomethoxybenzothiazole. mp >136°C(dec.). Anal. Calcd. for C₉H₇N₃O₄PFS₂ + 0.3HBr: C:30.07; H:2.05; N:11.69. Found: C:30.27; H:2.01; N:11.38.

### Example 39.

### Preparation of 2-Amino-7-hydroxymethyl-6-thiocyano-4-phosphonomethoxy benzothiazole.

**Step A.** 2-Chloro-5-formylnitrobenzene was subjected to Step A of Example 38 to give 2-diethylphosphonomethyloxy-5-formylnitrobenzene.
**Step B.** A solution of 2-diethylphosphonomethyloxy-5-formylnitrobenzene (1 mmole) in methanol (5 mL) was treated with 10 % palladium on carbon (0.05 mmole) under 1 atmosphere of hydrogen at room temperature for 12 h. Filtration followed by evaporation gave 2-diethylphosphonomethyloxy-5-hydroxymethylaniline which was subjected to Step B of Example 36 followed by Step D of Example 34 to give 2-amino-7-hydroxymethyl-6-thiocyanato-4-phosphonomethoxybenzothiazole (**39.1**). mp 181-184°C. Anal. Calcd. for C₁₀H₁₀N₃O₅PS₂+0.35H₂O: C:33.97; H:3.05; N:11.88. Found: C:33.76; H:2.66; N:11.61.

### Example 40.

### Preparation of 2-Amino-6-bromo-7-fluoro-4-phosphonomethoxybenzothiazole.

**Step A.** A solution of 2-diethylphosphonomethyloxy-4-bromo-5-fluoroaniline (1 mmole, prepared as in Example 4, Step B) and KSCN (2 mmole) in AcOH (8 mL) was cooled to 10 °C, and treated with a solution of bromine (2 mmole) in AcOH (5 mL). After being stirred at room temperature for 0.5 h, the reaction mixture was evaporated to dryness and the residue was purified by chromatography to provide 2-amino-7-fluorol-6-bromo-4-diethylphosphonomethyloxybenzothiazole which was subjected to Step D of Example 34 to give 2-amino-6-bromo-7-fluoro-4-phosphonomethoxybenzothiazole (**40.1**). Anal. Calcd. for C₈H₇N₂O₄PSBrF + 0.1HBr: C:26.31; H:1.96; N:7.67. Found: C:25.96; H:1.94; N:7.37.

### Example 41.

### Preparation of 2-Amino-7-ethyl-6-thiocyano-4-phosphonomethoxy benzothiazole:

**Step A.** A solution of 2-diethylphosphonomethyloxy-5-bromonitrobenzene (1 mmole, prepared as in Example 37, Step A) in DMF (5 mL) was treated with tributyl(vinyl)tin (1.2 mmole) and palladium bis(triphenylphosphine) dichloride (0.1 mmole), and the mixture was heated at 60 °C under nitrogen for 6 h. Evaporation and chromatography gave 2-diethylphosphonomethyloxy-5-vinylnitrobenzene as an oil which was subjected to Step B of Example 38, Step B of Example 36, and Step D of Example 34 to give 2-amino-7-ethyl-6-thiocyano-4-phosphonomethoxybenzothiazole (**41.1**). mp >167°C(dec.). Anal. Calcd. for C₁₁H₁₂N₃O₄PS₂: C:38.26; H:3.50; N:12.17. Found: C:37.87; H:3.47; N:11.93.

### Example 42.

### Preparation of 2-Amino-7-cyclopropyl-6-thiocyanato-4-phosphonomethoxy benzothiazole.

**Step A.** A suspension of 2-diethylphosphonomethyloxy-5-vinylnitrobenzene (1 mmole, prepared as in Step A of Example 40) and Pd(OAc)₂ (0.1 mmole) in ether (8 mL) was treated with a solution of diazomethane (generated from 3.0 g of 1-methyl-3-nitro-1-nitrosoguanidine) in ether at 0 °C. After being stirred at room temperature for 20 h the reaction was evaporated to dryness and the residue was chromatographed to give 2-diethylphosphonomethyloxy-5-cyclopropylnitrobenzene which was subjected to Step B of Example 37, Step B of Example 36, and Step D of Example 34 to give 2-amino-7-cyclopropyl-6-thiocyanato-4-phosphonomethoxybenzothiazole hydrogen bromide **(42.1).** Anal. Calcd. for C₁₂H₁₃N₃O₄PS₂Br + 0.1HBr: C:27.76; H:2.72; N:8.09. Found: C:27.54; H:3.05; N:7.83.

### Example 43.

### Preparation of 2-Amino-4-phosphonomethoxy-6-chloro-7-methyl benzothiazole:

**Step A.** 2-Methoxy-4-chloro-5-methylaniline was subjected to Steps A and B of Example 34, Step B of Example 36, and Step D of Example 34 to give 2-amino-4-phosphonomethoxy-6-chloro-7-methyl benzothiazole **(43.1)**. mp >250°C(dec.). Anal. Calcd. for C₉H₁₀N₂O₄PS₂Cl+0.3H₂O + 0.4 HBr: C:31.20; H:3.20; N:8.09. Found: C:31.37; H:2.87; N:7.89.

Similarly, the following compounds were prepared according to above procedures:
**(43.2)** 2-Amino-7-phenyl-6-thiocyanato-4-phosphonomethoxybenzothiazole. mp >250°C(dec.). Anal. Calcd. for C₁₅H₁₂N₃O₄PS₂ + 0.2H₂O: C:45.38; H:3.15; N:10.58. Found: C:45.25; H:3.21; N:10.53.

### Example 44.

### Preparation of 2-bromo-4-diethylphosphonomethoxy-5,6,7,8-tetrahydronaphtho[1,2-d]thiazole.

**Step A.** A solution of 2-amino-4-diethylphosphonomethoxy-5,6,7,8-tetrahydronaphtho[1,2-d]thiazole (1 mmole) in CH₃CN (4 mL) was cooled to 0°C, and treated with CuBr₂ (1.2 mmole) followed by isoamylnitrite (1.5 mmole) dropwisely. The resulting dark mixture was stirred for 3.5 h. Evaporation and chromatography gave 2-bromo-4-diethylphosphonomethoxy-5,6,7,8-tetrahydronaphtho[1,2-d]thiazole as an oil.
**Step B.** 2-Bromo-4-diethylphosphonomethoxy-5,6,7,8-tetrahydronaphtho[1,2-d]thiazole was subjected to Step D of Example 34 to give 2-bromo-4-phosphonomethoxy-5,6,7,8-tetrahydronaphtho[1,2-d]thiazole **(44.1)** as a solid. Mp 220-230 °C. Anal. Calcd. for C₁₂H₁₃NO₄PSBr: C:38.11; H:3.46; N:3.70. Found: C:37.75; H:3.26; N:3.69.

### Example 45.

### Preparation of 4-diethylphosphonomethoxy-5,6,7,8-tetrahydronaphtho[1,2-d]thiazole.

**Step A.** A solution of isoamylnitrite (1.5 mmole) in DMF (1 mL) at 65°C was treated with 2-amino-4-diethylphosphonomethoxy-5,6,7,8-tetrahydronaphtho[1,2-d]thiazole (1 mmole) in DMF (3 mL). After 30 min, the cooled reaction solution was subjected to evaporation and chromatography to provide 4-diethylphosphonomethoxy - 5,6,7,8-tetrahydronaphtho[1,2-d]thiazole as an oil, which was subjected to Step D of Example 34 to give 4-phosphonomethoxy-5,6,7,8-tetrahydronaphtho[1,2-d]thiazole **(45.1)** as a solid. Mp 215 - 220 °C. Anal. Calcd. for C₁₂H₁₄NO₄PS + 1.3HBr: C:35.63; H:3.81; N:3.46. Found: C:35.53; H:3.46; N:3.40.

### Example 46.

### Preparation of 2-Amino-4-phosphonomethythio benzothiazole.

**Step A.** 2-Diethylphosphonomethylthioaniline, prepared according to Step B of Example 34, was subjected to Step B of Example 36 to give 2-amino-4-diethylphosphonomethythiobenzothiazole.
**Step B.** 2-Amino-4-diethylphosphonomethythiobenzothiazole was subjected to Step D of Example 34 to give 2-amino-4-phosphonomethythiobenzothiazole **(46.1)** as a foam. Anal. Calcd. for C₈H₁₀N₂O₃PS₂ + 0.4H₂O: C:35.63; H:3.81; N:3.46. Found: C:35.53; H:3.46; N:3.40.

### Example 47.

### Preparation of various prodrugs of benzothiazoles.

**Step A.** A suspension of 2-amino-4-phosphonomethoxy-5,6,7,8-tetrahydronaphtho[1,2-d]thiazole (1 mmole) in DMF (10 mL) was treated with DCC (3 mmole) followed by 3-(3,5-dichloro)phenyl-1,3-propanediol (1.1 mmole). The resulting mixture was heated at 80°C for 8 h. Evaporation followed by column chromatography gave 2-amino-4-{[3-(3,5-dichlorophenyl)propane-1,3-diyl]phosphonomethoxy}-5,6,7,8-tetrahydronaphtho[1,2-d]thiazole **(47.1)** as solid. mp >230 °C. Anal. Calcd. for C₂₁H₂₁N₂O₄PSCl₂: C:50.51; H:4.24; N:5.61. Found: C:50.83; H:4.34; N:5.25.
**Step B.** A solution of 4-phosphonomethoxy-5,6,7,8-tetrahydronaphtho[1,2-d]thiazole dichloridate (generated as in Example 19) (1 mmole) in dichloromethane (5 mL) is cooled to 0 °C and treated with a solution of benzyl alcohol (0.9 mmole) in dichloromethane (0.5 mL) and pyridine (0.3 mL). The resulting reaction solution is stirred at 0 °C for 1h, and then added a solution of ammonia (excess) in THF. After stirring at room temperature for 16 h, the reaction is evaporated to dryness and the residue is purified by chromatography to give of 4-phosphonomonoamidomethoxy-5,6,7,8-tetrahydronaphtho[1,2-d]thiazole.
   Alternatively, a different method is used to prepare other phosphoramides as exemplified in the following procedure:
**Step C.** A suspension of 4-phosphonomethoxy-5,6,7,8-tetrahydronaphtho[1,2-d]thiazole dichloridate (generated as in Example 19) (1 mmole) in dichloromethane (5 mL) is cooled to 0 °C and ammonia (excess) is bubbled through the reaction for 10 min. After stirring at room temperature for 16 h, the reaction is evaporated to dryness and the residue is purified by chromatography to give 4-(phosphorodiamido)methoxy-5,6,7,8-tetrahydronaphtho[1,2-d]thiazole.
   The monophenyl-monophosphonamide derivatives of compounds of formula X can also be prepared according to the above described procedures:

**Step D.** A solution of 4-diphenylphosphonomethoxy-5,6,7,8-tetrahydronaphtho[1,2-d]thiazole (prepared according to the procedures of Example 19) (1 mmole) in acetonitrile (9 mL) and water (4 mL) is treated with lithium hydroxide (1N, 1.5 mmole) at room temperature for 24 h. The reaction solution is evaporated to dryness, and the residue is dissolved in water (10 mL), cooled to 0 °C and the pH of the solution is adjusted to 4 by addition of 6 N HCl. The resulting white solid is collected through filtration to give 4- phenylphosphonomethoxy-5,6,7,8-tetrahydronaphtho[1,2-d]thiazole.
**Step E.** A suspension of 4-phenylphosphonomethoxy-5,6,7,8-tetrahydronaphtho[1,2-d]thiazole (1 mmole) in thionyl chloride (3 mL) is heated to reflux for 2 h. The reaction solution is evaporated to dryness, and the residue is dissolved in anhydrous dichloromethane (2 mL) and the resulting solution is added to a solution of L-alanine ethyl ester hydrochloride (1.2 mmole) in pyridine (0.8 mL) and dichloromethane (3 mL) at 0 °C. The resulting reaction solution is stirred at room temperature for 14 h. Evaporation and chromatography give 4-[O-phenyl-N-(1-ethoxycarbonyl)ethylphosphonamido]methoxy-5,6,7,8-tetrahydronaphtho[l,2-d]thiazole.
**Step F.** A solution of 4-phosphonomethoxy-5,6,7,8-tetrahydronaphtho[1,2-d]thiazole (1 mmole) in DMF is treated with N,N'-dicyclohexyl-4-morpholinecarboxamidine (5 mmole) and ethylpropyloxycarbonyloxymethyl iodide (5 mmole) which was prepared from chloromethyl chloroformate according to the reported procedure (Nishimura et al. *J Antibiotics*, **1987**, *40*, 81). The reaction mixture is stirred at 25 oC for 24 h. Evaporation and chromatography give 4-bis(ethoxycarbonyloxymethyl)phosphonomethoxy-5,6,7,8-tetrahydronaphtho[1,2-d]thiazole.

4-(Dipivaloyloxymethyl)phosphonomethoxy-5,6,7,8-tetrahydronaphtho[1,2-d]thiazole and 4-bis(isobutyryloxymethyl)phosphonomethoxy-5,6,7,8-tetrahydronaphtho[1,2-d]thiazole are also prepared in a similar manner.

### Example 48.

### General procedure for bis-phosphoroamide prodrugs

### Dichloridate formation

To a suspension of 1 mmol of phosphonic acid in 5 mL of dichloroethane was added 0.1 mmol of pyridine (or 0.1 mmol of DMF) followed by 6 mmol of thionyl chloride and was heated to reflux for 2.5 h. Solvent and excess thionyl chloride were removed under reduced pressure and dried to give the dichloridate.

### Coupling reaction:

Method A: The crude dichloridate was taken into 5 mL of dry CH₂Cl₂, and was added 8 mmol of aminoacid ester at 0 °C. The resultant mixture was allowed to come to rt where it was stirred for 16h. The reaction mixture was subjected to aq. work up and chromatography.

Method B: The crude dichloridate was taken into 5 mL of dry CH₂Cl₂, and was added a mixture of 4 mmol of aminoacid ester and 4 mmol of N-methylimidazole at 0 °C. The resultant mixture was allowed to come to rt where it was stirred for 16 h. The reaction mixture was subjected to aq. work up and chromatography.
The following compounds were prepared in this manner.
(**48.1**) 2-Amino-5-isobutyl-4-[2-(5-N,N-bis(L-glutamic acid diethylester) phosphonoamido)furanyl]thiazole. Anal. cald. For C₂₉H₄₅N₄O₁₀PS: C: 51.78; H: 6.74; N: 8.33. Found: C: 51.70; H: 6.64; N: 8.15.
(**48.2**) 2-Amino-5-isobutyl-4-[2-(5-N,N-bis(L-alanine acid dibenzyl ester)phosphonoamido)furanyl]thiazole. Anal. cald. For C₃₁H₃₇N₄O₆PS: C: 59.60; H: 5.97; N: 8.97. Found: C: 59.27; H: 5.63; N: 8.74.
**(48.3)** 2-Amino-5-isobutyl-4-{2-[5-(N,Nbis(benzyloxycarbonylmethyl) phosphonodiamido]furanyl}thiazole. Anal. cald. for C₁₉H₂₅N₄O₆PS + 0.3 CH₂Cl₂: C: 46.93; H: 5.22; N: 11.34. Found: C: 46.92; H: 5.00; N: 11.22.
**(48.4)** 2-Amino-5-isobutyl-4-{2-[5-(N,N-bis(benzyloxycarbonylmethyl) phosphonodiamido]furanyl}thiazole. Anal. cald. For C₂₉ H₃₃ N₄ O₆ P S: C: 58.38; H: 5.57; N: 9.39. Found: C: 58.20; H: 5.26; N: 9.25.
**(48.5)** 2-Amino-5-isobutyl-4-{2-[5-(N,N'-bis((R)-1-methoxycarbonyl)ethyl) phosphonamido]furanyl}thiazole. Anal. cald. for C₁₉H₂₉N₄O₆PS + 0.6 CH2Cl2: C: 44.97; H: 5.82; N: 10.70. Found: C: 44.79; H: 5.46; N: 10.48.
**(48.6)** 2-Amino-5-isobutyl-4-{2-[5-(N,N'-bis((S)-1-ethoxycarbonyl)ethyl) phosphonamido]furanyl}thiazole. mp. 164-165 °C: Anal. cald. for C₂₁H₃₃N₄O₆PS + 0.61 CH₂CI₂: C: 46.99; H: 6.24; N: 10.14. Found: C: 47.35; H: 5.85; N: 9.85.
**(48.7)** 2-Amino-5-isobutyl-4-{2-[5-(N,N'-bis((t-butoxycarbonyl)methyl) phosphonamido]furanyl}thiazole. Anal. cald. for C₂₃H₃₇N₄O₆PS + 0.15 CH₂Cl₂: C: 51.36; H: 6.94; N: 10.35. Found: C: 51.34; H: 6.96; N: 10.06.
**(48.8)** 2-Amino-5-isobutyl-4-{2-[5-(N,N'-bis(ethoxycarbonyl) methyl)phosphonamido)]furanyl}thiazole. Anal. cald. for C₁₉H₂₉N₄O₆PS + 0.1 EtOAc + 0.47 CH₂Cl₂: C: 45.79; H: 5.94; N: 10.75. Found: C: 46.00; H: 5.96; N: 10.46.
**(48.9)** 2-Amino-5-isobutyl-4-{2-[5-(O-(2-bis(N-(1-methyl-1-ethoxycarbonyl)ethyl)phosphonamido]furanyl}thiazole. mp. 142-145 °C:; Anal. cald. for C₂₃H₃₇N₄O₆PS: C: 52.26; 7.06; 10.60. Found: C: 52.21; 6.93; 10.62.
**(48.10)** 2-Amino-5-isobutyl-4-{2-[5-(N,N'-bis(ethoxycarbonylmethyl) -N,N'-dimethylphosphonamido)]furanyl}thiazole. Anal. cald. for C₂₁H₃₃N₄O₆PS: C: 50.39; H: 6.65; N: 11.19. Found: C: 50.57; H: 6.56; N: 11.06.
**(48.11)** 2-Amino-5-isobutyl-4-{2-[5-(N,N'-bis((S)-1-benzyloxycarbonyl-2-methyl)propyl) phosphonamido]furanyl}thiazole. Anal. cald. for C₃₅ H₄₅ N₄ O₆ P S + 0.5 H₂O: C: 60.94; H: 6.72; N: 8.12. Found: C: 61.01: H: 6.48; N: 7.82.
**(48.12)** 2-Amino-5-isobutyl-4-{2-[5-(N,N'-bis((S)-1-methoxycarbonyl-3-methyl)butyl) phosphonamido]furanyllthiazole. Anal. cald. for C₂₅ H₄₁ N₄ O₆ P S: C: 53.94; H: 7.42; N: 10.06. Found: C: 54.12; H: 7.62; N: 9.82.
**(48.13)** 2-Amino-5-isobutyl-4-{2-[5-(N,N'-bis((R)-1-ethoxycarbonyl-2-(S-benzyl))ethyl) phosphonamido]furanyllthiazole. Anal. cald. for C₃₅ H₄₅ N₄ O₆ P S₃ + 0.4 toluene: C: 58.07; H: 6.21; N: 7.17. Found: C: 57.87; H: 6.14; N: 6.81.
(**48.14**) 2-Amino-5-isobutyl-4-{2-[5-(N,N'-bis((S)-1-ethoxycarbonyl-3-(S-methyl))butyl)phosphonamido]furanyl}thiazole. Anal. cald. for C₂₃ H₃₇ N₄ O₆ P S3: C: 46.61; H: 6.92; N: 9.45. Found: C: 46.26; H: 6.55; N: 9.06.
(**48.15**) 2-Amino-5-propylthio-4-{2-[5-(N,N'-(1-(S)-ethoxycarbonyl)ethyl)phosphonamido]furanyl}thiazole. Anal. cald. for C₂₀H₃₁N₄O₆PS₂: C: 46.32; H: 6.03; N: 10.80. Found: C: 46.52; H: 6.18; H: 10.44.
(**48.16**) 2-Amino-5-isobutyl-4-{2-[5-(N,N'-bis((S)-1-benzyloxycarbonyl-2-methyl)isobutyl) phosphonamido]furanyl}thiazole. Anal. cald. for C₃₇H₄₉N₄O₆PS: C: 62.69; H: 6.97; H: 7.90. Found: C: 62.85; h 7.06, 7.81.
(**48.17**) 2-Amino-5-isobutyl-4-{2-[5-(N,N'-bis((S)-1-ethoxycarbonyl-3-methyl)butyl) phosphonamido]furanyl}thiazole. Anal. cald. for C₂₇H₄₅N₄O₆PS: C: 55.46; H: 7.76; N: 9.58. Found: C: 55.35; H: 7.94; N: 9.41.
(**48.18**) 2-Amino-5-isobutyl-4-{2-[5-(N,N'-bis((S)-1-ethoxycarbonyl-2-methyl)propyl) phosphonamido]furanyl}thiazole. Anal. cald. for C₂₅H₄₁N₄O₆PS: C: 53.94; H: 7.42; N: 10.06. Found: C: 54.01; H: 7.58; N: 9.94.
**(48.19)** 2-Amino-5-isobutyl-4-{2-[5-(N,N'-bis((S)-1-ethoxycarbonyl-2-phenyl)ethyl) phosphonamido]furanyl}thiazole. Anal. cald. for C₃₃H₄₁N₄O₆PS + 0.15 CH₂Cl₂: C: 59.83; H: 6.26; H: 8.42. Found: C: 59.88; H: 6.28; H: 8.32.
**(48.20)** 2-Amino-5-propylthio-4-{2-[5-(N,N'-(1-methyl-lethoxycarbonyl)ethyl) phosphonamido]furanyl}thiazole. mp. 110-115 °C: Anal. cald. for C₂₂H₃₅N₄O₆PS₂ + 0.4HCI + 0.5Et₂O: C: 48.18; H: 6.81; N: 9.36. Found: C: 48.38; H: 6.60; H: 8.98.
**(48.21)** 2-Amino-5-methylthio-4-{2-[5-(N,N'-bis(1-methyl-1-ethoxycarbonyl)ethyl) phosphonamido]furanyl}thiazole. Anal. cald. for C₂₀H₃₁N₄O₆PS₂+0.5H₂O: C: 45.53; H: 6.11; N: 10.62. Found: C: 45.28; H: 5.85; N: 10.56.

### Example 49.

### General procedure for mixed bis-phosphoroamidate prodrugs

To a solution of crude dichloridate (I mmol, prepared as described in Example 40) in 5 mL of dry CH₂Cl₂ was added amine (1mmol) followed by 4-dimethylaminopyridine (3mmol) at 0 °C . The resulting mixture was allowed to warm to room temperature and stirred for 1h. The reaction was cooled back to 0 °C before adding aminoacid ester (2mmol) and left at room temperature for 16h. The reaction mixture was subjected to aq. work up and the mixed bis-phosphoroamidate prodrug was purified by column chromatography.
The following compounds were prepared in this manner.
(**49.1**) 2-Amino-5-isobutyl-4-{2-[5-(N-morpholino-N'-(1-methyl-1-ethoxycarbonyl)ethyl)phosphonamido]furanyl}thiazole. mp. 182-183 °C: Anal. cald. for C₂₁H₃₃N₄O₅PS: C: 52.05; H: 6.86; N: 11.56. Found: C: 51.66; H:6.68; N: 11.31.
(**49.2**) 2-Amino-5-isobutyl-4-{2-[5-(N-pyrrolidino-N'-(1-methyl-1-ethoxycarbonyl)ethyl)phosphonamido]furanyl}thiazole. mp. 189-190 °C: Anal. cald. for C₂₁H₃₃N₄O₄PS: C: 53.83; H: 7.10; N: 11.96. Found: C: 54.15; H: 7.48; N: 12.04.

### BIOLOGICAL EXAMPLES

The following examples may be useful for identifying compounds which 1) inhibit FBPase and gluconeogenesis in cellular and animal models of diabetes; or 2) enhance insulin sensitivity in cell culture or animal models of diabetes; or 3) exhibit superior pharmacological activity as combinations of FBPase inhibitors and insulin sensitizers relative to either agent alone.

The following compounds A-K are used in some of the Biological Examples which follow:

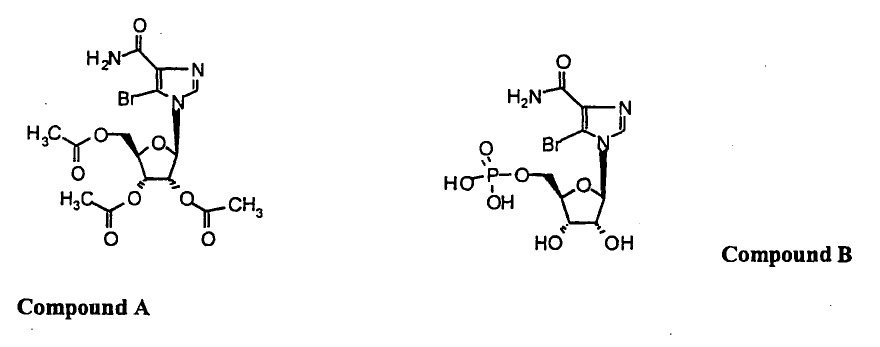

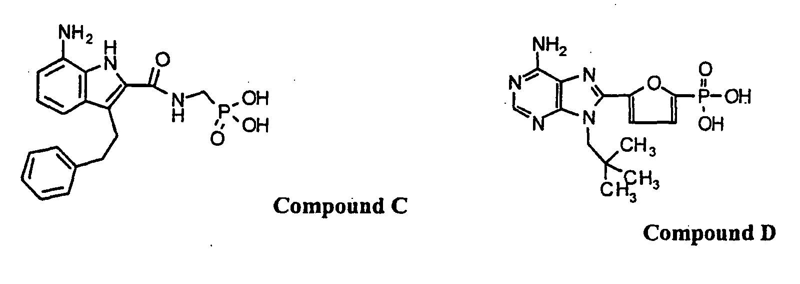

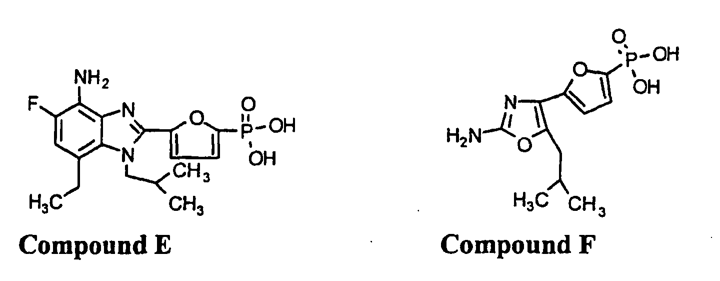

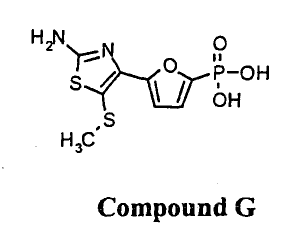

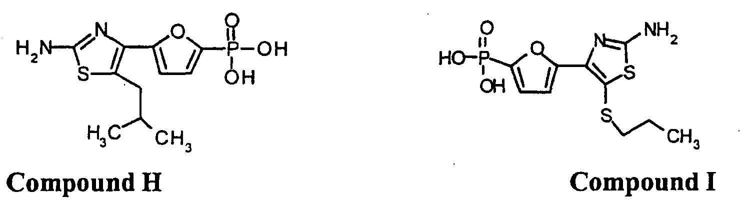

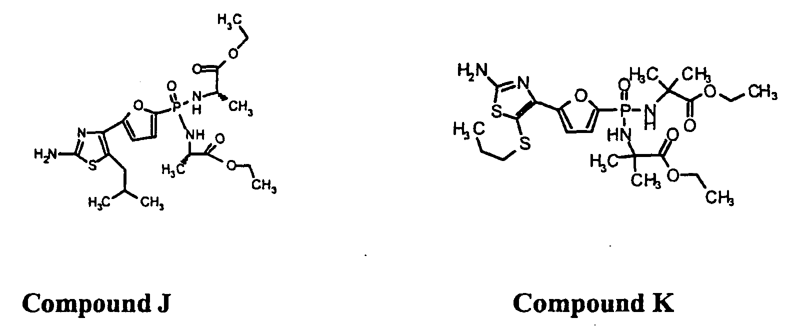

Compound F is prepared in Example 10.6, Compound G is prepared in example 3.26, compound H is prepared in Example 3.68, Compound I is prepared in Example 3.58, Compound J is prepared in Example 48.6, and Compound K is prepared in Example 48.2.

### Example A: Inhibition of Human Liver FBPase

*E. coli* strain BL21 transformed with a human liver FBPase-encoding plasmid was obtained from Dr. M. R. El-Maghrabi at the State University of New York at Stony Brook. hlFBPase was typically purified from 10 liters of *E. coli* culture as described by M. Gidh-Jain et al. J. Biol Chem. 269, 27732-27738 (1994). Enzymatic activity was measured spectrophotometrically in reactions that coupled the formation of product (fructose 6-phosphate) to the reduction of dimethylthiazoldiphenyltetrazolium bromide (MTT) via NADP and phenazine methosulfate (PMS), using phosphoglucose isomerase and glucose 6-phosphate dehydrogenase as the coupling enzymes. Reaction mixtures (200 µl) were made up in 96-well microtitre plates, and consisted of 50 mM Tris-HCl, pH 7.4, 100 mM KCl, 5 mM EGTA, 2 mM MgCl₂, 0.2 mM NADP, 1 mg/ml BSA, 1 mM MTT, 0.6 mM PMS, 1 unit/ml phosphoglucose isomerase, 2 units/ml glucose 6-phosphate dehydrogenase, and 0.150 mM substrate (fructose 1,6-bisphosphate). Inhibitor concentrations were varied from 0.01 µM to 10 µM. Reactions were started by the addition of 0.002 units of pure hlFBPase and were monitored for 7 minutes at 590 nm in a Molecular Devices Plate Reader (37°C).

The potencies of select compounds against human liver FBPase are shown in the table below:

**Table 1.**

| Compound | IC50, µM |
|---|---|
| AMP | 1.3 |
| E | 0.055 |
| D | 1.0 |
| B | 5.0 |
| C | 30 |
| F | 0.12 |
| G | 0.015 |
| H | 0.025 |
| I | 0.018 |
| Troglitazone | >100 |

### Example B: Inhibition of rat liver and mouse liver FBPase

*E*. *coli* strain BL21 transformed with a rat liver FBPase-encoding plasmid was obtained from Dr. M. R. El-Maghrabi at the State University of New York at Stony Brook, and purified as described (El-Maghrabi, M.R., and Pilkis, S.J. (1991) Biochem. Biophys. Res. Commun. 176: 137-144). Mouse liver FBPase was obtained by homogenizing freshly isolated mouse liver in 100 mM Tris-HCl buffer, pH 7.4, containing 1 mM EGTA, and 10% glycerol. The homogenate was clarified by centrifugation, and the 45-75% ammonium sulfate fraction prepared. This fraction was redissolved in the homogenization buffer and desalted on a PD-10 gel filtration column (Biorad) eluted with same. This partially purified fraction was used for enzyme assays. Both rat liver and mouse liver FBPase were assayed as described for human liver FBPase in Example A. Generally, as reflected by higher IC₅₀ values, the rat and mouse liver enzymes are less sensitive to inhibition by the compounds tested than the human liver enzyme.

The following Table depicts the IC₅₀ values for several compounds prepared in the Examples:

**Table 2.**

| Compound | IC₅₀ Rat Liver (µM) | IC₅₀ Mouse Liver (µM) |
|---|---|---|
| AMP | 25 | 15 |
| B | 140 | 33 |
| D | 1.25 | 55 |
| C | >100 | >100 |
| E | 0.4 | 1.1 |
| F | 2.0 | |
| G | 0.25 | |
| H | 0.175 | |
| I | 0.05 | |

### Example C: Inhibition of Gluconeogenesis in Rat Hepatocytes

Hepatocytes were prepared from fed Sprague-Dawley rats (250-300 g) according to the procedure of Berry and Friend (Berry, M.N., Friend, D.S., 1969, J. Cell. Biol. 43, 506-520) as modified by Groen (Groen, A.K., Sips, H.J., Vervoom, R.C., Tager, J.M. , 1982, Eur. J. Biochem. 122, 87-93). Hepatocytes (75 mg wet weight/ml) were incubated in 1 ml Krebs-bicarbonate buffer containing 10 mM Lactate, 1 mM pyruvate, 1 mg/ml BSA, and test compound concentrations from 0 to 500 µM. Incubations were carried out in a 95% oxygen, 5% carbon dioxide atmosphere in closed, 50-ml Falcon tubes submerged in a rapidly shaking water bath (37°C). After 1 hour, an aliquot (0.25 ml) was removed, transferred to an Eppendorf tube and centrifuged. 50 µl of supernatant was then assayed for glucose content using a Sigma Glucose Oxidase kit as per the manufacturer's instructions.

The following Table depicts the IC₅₀ values for several compounds prepared in the Examples:

**Table 3.**

| Compound | IC50 (µm) |
|---|---|
| Compound A | 50 |
| Compound D | 4.5 |
| Compound E | 2.5 |
| Compound C | > 100 |
| Compound F | 15 |
| Compound G | 10 |
| Compound H | 2.5 |
| Compound I | 2.0 |
| Compound J | 2.0 |
| Compound K | 2.1 |
| Troglitazone | >100 |

FBPase from rat liver is less sensitive to AMP than that from human liver. IC₅₀ values are consequently higher in rat hepatocytes than would be expected in human hepatocytes.

It is particularly advantageous to screen compounds of formula I on hepatocytes such as described in Examples C and D because these compounds are phosphorylated by the hepatocytes and thereby become FBPase inhibitors.

### Example D: Inhibition of Glucose Production and Elevation of Fructose-1,6-Bisphosphate Levels in Rat Hepatocytes Treated with FBPase Inhibitors.

Rat hepatocytes were isolated and incubated as described in Example C. Cell extracts, were analyzed for glucose content as described in Example C, and also for fructose 1,6-bisphosphate. Fructose 1,6-bisphosphate was assayed spectrophotometrically by coupling its enzymatic conversion to glycerol 3-phosphate to the oxidation of NADH, which was monitored at 340 nm. Reaction mixtures (1 ml) consisted of 200 mM Tris-HC1, pH 7.4, 0.3 mM NADH, 2 units/ml glycerol 3-phosphate dehydrogenase, 2 units/ml triosephosphate isomerase, and 50-100 µl cell extract. After a 30 minute preincubation at 37°C, 1 unit/mi of aldolase was added and the change in absorbance measured until a stable value was obtained. 2 moles of NADH are oxidized in this reaction per mole of fructose 1,6-bisphosphate present in the cell extract.

**Compound A** and **Compound E** inhibited glucose production in a dose-dependent manner with IC50's of 50 and 2.5 µM, respectively. Consistent with the inhibition of FBPase, dose-dependent elevation of intracellular fructose 1,6-bisphosphate was observed with both compounds.

### Example E: Analysis of Hepatic and Plasma Drug Metabolite Levels, Blood Glucose, and Hepatic Fructose 1,6-bisphosphate Levels After Administration of Compound A p.o. to Normal Fasted Rats.

**Compound A** was administered by oral gavage to freely-feeding SpragueDawley rats (250-300g). The compound was prepared as a suspension in carboxymethylcellulose, and administered at a dose of 250 mg/kg. For the determination of liver metabolites, rats were serially sacrificed over the course of 24 hours after drug administration. Livers were freeze-clamped, homogenized in perchloric acid, neutralized, and then analyzed for Compound B by anion exchange HPLC.

For the determination of plasma metabolites, rats were instrumented with carotid catheters prior to oral dosing. Blood samples were withdrawn via the catheters at appropriate time points over the course of 8 hours post drug administration. Plasma was prepared from the blood samples by centrifugation, and plasma protein precipitated by the addition of methanol to 60%. Compound A metabolites were quantitated by reverse phase HPLC in the deproteinated plasma samples. A C18 column (1.4 cm X 250 mm) was equilibrated with 10 mM sodium phosphate, pH 5.5 and eluted with a gradient from this buffer to acetonitrile. Detection was at 254 nm.

The effect of Compound A on blood glucose and hepatic fructose 1,6-bisphosphate levels was determined in 18-hour fasted Sprague-Dawley rats (250-300 g). Animals were dosed as described above. At appropriate time points post drug administration, rats were anesthetized with halothane and a liver biopsy (approx. 1 g) was taken, as well as a blood sample (2 ml) from the posterior vena cava. A heparin flushed syringe and needle was used for blood collection. The liver sample was immediately homogenized in ice-cold 10% perchloric acid (3 ml), centrifuged, and the supernatant neutralized with 1/3rd volume of 3 M KOH/3 M KH₂CO₃. Following centrifugation and filtration,the neutralized extract was analyzed for fructose 1,6-bisphosphate content as decribed for isolated hepatocytes in Example C. Blood glucose was measured by means of a Hemocue analyzer (Hemocue Inc, Mission Viejo, CA).

Analysis of liver metabolites revealed that Compound A was efficiently converted to Compound B, with intrahepatic levels of the latter reaching 3 µmoles/g tissue within 1 hour. Although levels declined slowly over time, Compound B was measurable out to the final, 24 hour time point. In plasma 5-bromo-1-βD-ribofuranosyl-imidazole-carboxamide but not Compound A was detectable, suggesting that Compound A was rapidly deacetylated at all three positions.

The single 250 mg/kg dose of Compound A markedly lowered blood glucose for approximately 8 hours, at which time levels in the treated animals rebounded slowly to those of the vehicle-treated controls. Drug treatment resulted in the elevation of hepatic fructose 1,6-bisphosphate levels. The time course of elevation of this gluconeogeneic intermediate correlated well with the time course of glucose lowering. Peak elevation was observed at near maximal glucose lowering, and as blood glucose levels rebounded, fructose 1,6-bisphosphate levels slowly returned to normal. The latter observations are consistent with the inhibition of gluconeogenesis by Compound A at the level of fructose 1,6-bisphosphatase.

### Example F: Analysis of Hepatic and Plasma Drug Levels After Administration Compounds D and E intraperitoneally to Normal Fasted Rats.

Sprague-Dawley rats (250-300 g) were fasted for 18 hours and then dosed intraperitoneally either with saline or FBPase inhibitor. The vehicle used for drug administration was 10 mM bicarbonate. One hour post injection, rats were anesthetized with halothane, and liver and blood samples were taken and processed as described in Example E. The neutralized liver extracts were analyzed for FBPase inhibitor content by HPLC. A reverse phase YMC ODS AQ column (250 X 4.6 cm) was used and eluted with a gradient from 10 mM sodium phosphate pH 5.5 to 75% acetonitrile. Absorbance was monitored at 310 nm. Glucose was measured in the blood sample as described in Example C. Plasma was prepared by centrifugation and extracted by addition of methanol to 60% (v/v). The methanolic extract was clarified by centrifugation and filtration and then analyzed by HPLC as described above.

Results for select compounds prepared in the examples are shown in the table below.

**Table 4.**

| Compound | Glucose Lowering, % | Plasma con. (µM) | Liver cone. |
|---|---|---|---|
| | | | (nmoles/g) |
| D | 31 | 8.8 | 27.2 |
| E | 44.4 | 79.2 | 38.4 |
| F | 51 | 18 | 35 |
| G | 73 | 56.1 | |

### Example G: Oral Bioavailability Determination

The oral bioavailability of prodrugs and parent compounds was determined by the urinary excretion method in the rat. Prodrugs were dissolved in 10% ethanol/90% polyethylene glycol (mw 400) and administered by oral gavage at doses of 10 to 40 mg/kg parent compound equivalents to 6-hour fasted, Sprague Dawley rats (220-240 g). Parent compounds were typically dissolved in deionized water, neutralized with sodium hydroxide, and then administered orally at 10-40 mg/kg or intravenously at ~10 mg/kg. The rats were subsequently placed in metabolic cages and urine was collected for 24 hours. The quantity of parent compound excreted into urine was determined by HPLC analysis as described in Example F. Analysis was performed as described in Example F. For prodrugs, the percentage oral bioavailability was estimated by comparison of the recovery in urine of the parent compound generated from the prodrug administered orally, to that recovered in urine following intravenous administration of the corresponding parent compound. For parent compounds, the percentage oral bioavailability was estimated by comparison of the recovery in urine of the parent compound when administered orally to that recovered when administered intravenously.

The estimated % oral bioavailability of select prodrugs and parent compounds is shown below.

| Compound | Oral bioavailability, % |
|---|---|
| G | 18 |
| H | 4 |
| I | 5 |
| J | 21 |

### Example H: PPAR γ binding

Human PPAR γ g extracts, obtained from Sf9 insect cells expressing the cloned human gene incorporated into a baculovirus expression vector using procedures described in Mangelsdorf et al. Cell 54: 199-207 (1991), are used for saturation binding analysis. Extracts are incubated at 4 C for 3 h in buffer containing 10 mM Tris (pH 8.0), 50 mM KCI, 10 mM dithiothreitol with [³H]-BRL-49653 in the presence or absence of the insulin sensitizer. Bound is separated from free radioactivity by elution through 1-mL Sephadex G-25 desalting columns. Bound radioactivity is eluted in the column void volume and is quantitated by liquid scintillation counting. Insulin sensitizer treatment displaces [3H]-BRL-49653 from the PPARγ receptor, and results in reduced recovery of radioactivity in the bound fraction. The tighter the affinity of the insulin sensitizer for the receptor, the more radioactivity is displaced.

### Example I: Adipocyte Binding

Adipocytes are prepared from adipose tissue obtained from mammary gland by collagenase digestion (2 mg/ml collagenase; 4 ml/g tissue) by the method of Rodbell. Krebs-Henseleit medium is used (NaCl; 123 mM, NaHCO₃; 26 mM, KCl; 5 mM, MgSO₄; 1.2 mM, KH₂PO₄; 1.25 mM, glucose 5.6 mM, pH 7.4) gassed with 95% O₂/5% CO₂. The medium includes 4% BSA and is supplemented with p-aminoclonidine (100 nM) and adenosine (200 nM) to inhibit lipolysis. Binding studies are performed by rinsing the adipocytes in Dulbecco's modified Eagles medium/Hams F-12 nutrient mix medium containing 15 mM HEPES, pH 7.4, supplemented with 200 nM adenosine. After three washes to remove collagenase and BSA, 0.5 ml of cells are added (in triplicate) tubes containing radiolabelled insulin sensitizer. Final concentration of insulin sensitizer in all incubations is 30 pM. Tubes are incubated at 37 C for 1 h in a shaking bath and cell-associated radioactivity is assessed by separation of cells from medium by centrifugation (200 µl of incubation medium, in duplicate) through silicone oil (Dow Corning 200/200 cs) for 20 s at 10000 g in a Beckman microfuge. Cell pellets are counted for ¹²⁵I content after cutting the microfuge tube. Non-specific binding is estimated in the presence of 3 µM RBL 49653. Affinity of the insulin sensitizer for adipocyte receptor is indicated by an increase in specific ¹²⁵I content in the cell pellets.

### Example J: Triglyceride Formation by Differentiated Adipocytes

The pre-adipocyte cell line, 3T3-L1 fibroblasts, are obtained from ATCC. The cells are grown to confluence and differentiated with insulin, dexamethasone and IBMX. Adipocyte differentiation is observed by oil red O staining which stains the lipid droplets within the cytoplasm red. The extent of adipocyte differentiation is then monitored by microscope observation. Mature, lipid-filled adipocytes are used 8-10 days post-differentiation. Treatment of cells with insulin, a thiazolidinedione, or RXR ligands induces triglyceride accumulation.

### Example K: Glucose Uptake

The pre-adipocyte cell line, 3T3-Ll fibroblasts are grown to confluence and differentiated with insulin, dexamethasone and IBMX. Mature, lipid-filled adipocytes are used 8-10 days post-differentiation. Glucose transport is assessed after treatment of differentiated cells with insulin sensitizers for 48 h. Compounds are added freshly each day. 2-deoxyglucose transport is determined by addition of 1 µC of 25 µM 2-deoxy-D-[2,6-³H] glucose. After 10 minutes, cells are freed of tracer by extensive washing with ice-cold PBS and cell-associated radioactivity determined by liquid scintillation counting of alkali-solubilized extracts. Glucose uptake rates are normalized for protein content. Insulin sensitizer treatment increases the rate of glucose transport into the cells as indicated by higher levels of cell-associated radioactivity.

### Example L: Activation of PPARγ/RXR Heterodimers

CV-1 cells are co-transfected with plasmids containing the genes encoding PPARγ, RXR, and a luciferase-based reporter plasmid containing one or more PPRE's. Cells are treated with a range of doses of ligand. A concentration-dependent increase in luciferase induction is observed with ligands of RXR or of PPARγ.

### Example M: Insulin Sensitivity Measured by Euglycemic-hyperinsulinemic Clamp Technique

ZDF Diabetic rats are treated with vehicle or an insulin sensitizer for 7 or more days, and then instrumented with jugular and carotid catheters. After a recovery period, animals are fasted overnight, and a constant infusion of insulin is initiated. Blood glucose levels are maintained at baseline with a variable rate glucose infusion. Samples for plasma glucose are drawn every 10 minutes for the duration of the 4-hour protocol. Steady state glucose infusion rates are higher in the insulin-sensitizer treated group, indicating improved glucose disposal/insulin sensitivity.

### Example N: Combination Treatment With Troglitazone and an FBPase Inhibitor (Compound G), and With Troglitazone and a Prodrug of an FBPase Inhibitor (Compound J) in the ZDF rat - Effects on Blood Glucose

The Zucker Diabetic Fatty (ZDF) rat is widely used as a model for human type 2 diabetes as the progression of the disease in these rodents is similar to that described for human patients (Clark and Palmer 1982, Terrettaz and Jeanrenaud 1983). The mature ZDF rat not only displays obesity, hyperglycemia, insulin resistance and accelerated hepatic glucose production, but also develops some of the common macro- and microvascular complications associated with type 2 diabetes.

Clark JB, Palmer CJ (1982) Diabetes 30: 126A Terrettaz J, Jeanrenaud B (1983) Endocrinology 112: 1346-1351.

The purpose of this study was to determine whether combination treatment with Troglitazone-Compound G or Troglitazone-Compound J results in improved glycemic control in the ZDF rat relative to either agent administered alone, and thus establish whether combination therapy will be of clinical benefit.
*(a) Troglitazone-Compound G Protocol:* Male ZDF rats were purchased at 8 weeks of age from Genetics Models Inc. (Indianapolis, Indiana). The rats were maintained under standard vivarium conditions (25°C, 12-hour light, 12-hour dark cycle) and received powdered Purina 5008 chow and water *ad libitum*. At 11 weeks of age, animals with blood glucose >500 mg/dl were selected and divided into 4 treatment groups (n=8/group). The treatments were control, Troglitazone, Compound G, and the combination of Troglitazone and Compound G. Drugs were administered as 0.2% food admixtures for 15 days. The dose of Troglitazone selected (0.2%) is a maximal dose, which in pilot studies was found to normalize blood glucose levels in 10-week old ZDF rats. It is higher than the dose reported to prevent the onset of hyperglycemia in prediabetic ZDF rats (Sreenan et al 1996). In animals with established diabetes such as those selected for this study, the effects of Troglitazone better approximate those in man, where modest glucose lowering effects are generally observed (Inzucchi et al 1998). The dose of Compound G selected (0.2%) is also a maximal dose; a pilot study in the ZDF rat revealed that higher doses were of no additional benefit (blood glucose lowering at 0.5% was equivalent to that at 0.2%). Blood glucose levels were measured in tail vein samples by means of a HemoCue glucose analyzer (HemoCue Inc., Mission Viejo, CA). Values are expressed as the mean plus or minus the standard error of the mean. Differences between groups were evaluated by analysis of variance with the Tukey-Kramer post hoc test. Results are considered significant with p<0.05.
*(b) Troglitazone-Compound J Protocol:* This study was carried out exactly as described in the Troglitazone-Compound G section above with two modifications: the treatment period was 21 days and the dose of Compound J used was 0.4%. In a pilot study, Compound J showed a trend (not significant) towards more pronounced blood glucose lowering at 0.4% than at 0.2%. The 0.4% dose was therefore chosen to ensure a maximal drug response.

Combination treatment with Troglitazone and Compound G resulted in significantly greater reductions in blood glucose levels than treatment with either agent alone (see table below). At the end of the treatment period, blood glucose levels in the combination group (-200 mg/dl) approximated those of normal fed rats (~150 mg/dl).

| Blood Glucose, mg/dl | | |
|---|---|---|
| Treatment | Day 0 | Day 14 |
| Control | 655±39 | 762±31* |
| Compound G | 653±55 | 530±48 |
| Troglitazone | 655±33 | 431±73 |
| Combination | 661±39 | 222±39** |

| | | |
|---|---|---|
| * p<0.05 versus all groups | | |
| ** p<0.05 versus all groups | | |

Similar results were observed in the Troglitazone-Compound J study:

| Blood Glucose, mg/dl | | |
|---|---|---|
| Treatment | Day 0 | Day 21 |
| Control | 678±19 | 815±34* |
| Compound J | 674±20 | 452±40 |
| Troglitazone | 669±23 | 514±135 |
| Combination | 675±31 | 232±39** |

| | | |
|---|---|---|
| * p<0.05 versus all groups | | |
| ** p<0.05 versus all groups | | |

The data suggest that the combination of an insulin sensitizer (Troglitazone) and an FBPase inhibitor (Compound G) or a prodrug of an FBPase inhibitor (Compound J) may provide better glycemic control than either agent alone in the treatment of type 2 diabetes.

### Example O: Combination Treatment With Troglitazone and an FBPase Inhibitor (Compound G), and With Troglitazone and a Prodrug of an FBPase Inhibitor (Compound J) in the ZDF rat - Effects on Plasma Triglycerides

Troglitazone treatment is known to reduce circulating triglycerides both in animal models of diabetes and in man. Triglyceride lowering is generally regarded as beneficial in Type 2 diabetics as these patients often suffer from hypertriglyceridemia and are at risk of the associated cardiovascular complications. The purpose of this study was to determine the effects of combination treatment on plasma triglycerides.

On the final day of treatment in the study described in Example N, blood samples were taken from the posterior vena cava of each rat while under halothane anesthesia. Plasma was prepared by centrifugation and triglyceride levels then measured by means of a standard assay kit according to the instructions of the manufacturer (Sigma Chemical Company). Differences between groups were evaluated by analysis of variance with the Tukey-Kramer post hoc test. Results are considered significant with p<0.05.

As illustrated in the tables below, troglitazone treatment had the expected effect of significantly reducing plasma triglyceride levels. In contrast, treatment with Compound G or Compound J resulted in an approximate 2-fold elevation of plasma triglycerides. Surprisingly, combination treatment resulted in the same degree of triglyceride lowering as treatment with Troglitazone alone.
(a) Troglitazone-Compound.G

| Treatment | Plasma Triglycerides, mg/dl (Mean ± SEM) |
|---|---|
| Control | 352±26 |
| Compound G | 795±88** |
| Troglitazone | 131±16* |
| Combination | 102±14* |

| | |
|---|---|
| *p < 0.05 vs Control | |
| **p<0.05 vs all groups | |

(b) Troglitazone-Compound J

| Treatment | Plasma Triglycerides, mg/dl (Mean ± SEM) |
|---|---|
| Control | 421±58 |
| Compound J | 759±76** |
| Troglitazone | 249±60* |
| Combination | 188±45* |

| | |
|---|---|
| *p < 0.05 vs Control | |
| **p<0.05 vs all groups | |

This study indicates that the beneficial triglycride lowering effects ofTroglitazone are maintained in combination with Compound G or Compound J. Remarkably, the increase in triglycerides elicited by FBPase inhibitor monotherapy was suppressed in combination with the insulin sensitizer.

### Example P: Combination Treatment With Troglitazone and an FBPase Inhibitor (Compound G), and With Troglitazone and a Prodrug of an FBPase Inhibitor (Compound J) in the ZDF rat - Effects on Plasma Insulin

Severe type 2 diabetes in man is often associated with the deterioration of pancreatic beta-cell function and eventually the inability of the pancreas to secrete the amount of insulin appropriate for the degree of hyperglycemia. This progression is also evident in the ZDF rat. Animals initially go through a hyperinsulinemic phase to compensate for elevated blood glucose levels but eventually overstimulation of the pancreas results in diminished insulin secretion and hypoinsulinemia can ensue (Pickavance L et al 1998). The purpose of this study was to determine whether the improved glycemic control afforded by combination treatment with an insulin sensitizer and an FBPase inhibitor can attenuate the deterioration of pancreatic beta-cell function.

On the final day of treatment in the study described in Example N, blood samples were taken from the posterior vena cava of each rat while under halothane anesthesia. Plasma was prepared by centrifugation and insulin levels then measured by means of an enzyme-linked immunoassay kit according to the instructions of the manufacturer (Amersham Life Sciences). Differences between groups were evaluated by analysis of variance with the Tukey-Kramer post hoc test. Results are considered significant with p<0.05.

There was no significant difference in plasma insulin levels between control rats and those on either Troglitazone or Compound G monotherapy (see tables below). Combination treatment, however, resulted in a significant increase in plasma insulin levels relative to all other groups, and in fact restored them to levels similar to those observed in non diabetes prone, lean ZDF rats (Pickavance et al 1998).

| Treatment | Plasma Insulin, ng/ml (Mean ± SEM) |
|---|---|
| Control | 1.88±0.3 |
| Compound G | 1.94±0.21 |
| Troglitazone | 3.61±1.03 |
| Combination | 8.23±2.67* |

| | |
|---|---|
| *p<0.05 vs all groups | |

The data show that combination treatment synergistically improves pancreatic endocrine function; insulin levels were increased significantly over those in controls only in the combination therapy group. This increase in plasma insulin could potentially be due to a reversal of lipotoxicity (Unger, 1997) and glucotoxicity (Leahy, 1990) in pancreatic islet cells. Attenuation of lipotoxicity may result from the triglyceride lowering effects of Troglitazone (Example O), while the attenuation of glucotoxicity may result from the combined glucose lowering effects of Trolitazone and Compound G (Example N).

### Example Q: Combination Treatment With Troglitazone and an FBPase Inhibitor (Compound G), and With Troglitazone and a Prodrug of an FBPase Inhibitor (Compound J) in the ZDF rat - Effects on Blood Lactate

The aim of this study was to determine the effects of combination treatment on blood lactate levels.

Blood samples were taken from the tail vein on days 0, 3, 7, 10 and 14 of the Troglitazone-Compound G combination study and on days 0,7,14, and 21 of the Troglitazone-Compound J combination study (study protocols are described in Example N). Following their acidification with perchloric acid and clarification by centrifugation, lactate was measured by means of a standard kit according to the instructions of the manufacturer (Sigma Chemical Company).

Baseline blood lactate levels for the Compound G and Compound J monotherapy groups were 1.98±0.17 mM and 2.24±0.08 mM, respectively. The maximal blood lactate elevations over baseline observed during the course of the studies were 2.5-fold for Compound G monotherapy and 3-fold for Compound J monotherapy. Blood lactate was typically elevated 2-fold over baseline in these groups. Blood lactate was not elevated above baseline (-2 mM) in the control or combination groups on any of the measurement days.

Combination treatment with Troglitazone suppressed the blood lactate elevations observed in the FBPase inhibitor or FBPase inhibitor prodrug monotherapy groups. Combination treatment may thus have the unexpected benefit of improving the safety profile of FBPase inhibitors and their prodrugs.

### Example R: Combination Treatment With Rosiglitazone and an FBPase Inhibitor (Compound G), in the ZDF rat - Effects on Blood Glucose

This study addressed whether combination treatment with Rosiglitazone and Compound G results in improved glycemic control in the Zucker Diabetic Fatty (ZDF) rat relative to either agent administered alone.

The protocol for this study was identical to the one described for the Troglitazone-Compound G combination in Example N with the following changes: (a) rats with blood glucose levels >600 mg/dl were included in the study, (b) the dose of Rosiglitazone used was 0.0045% based on a literature report that this dose prevented the onset of diabetes in the ZDF rat (Smith et al 1997), and (c) the length of the study was 25 days.

Blood glucose levels were significantly reduced in all treatment groups relative to the control group. Blood glucose lowering in the combination group tended to be more pronounced but was not significantly different than that in the Rosiglitazone or Compound G monotherapy groups. As illustrated in the table below, the response to Rosiglitazone was highly variable and was likely a factor in the less than expected efficacy of combination treatment. However, there was a clear benefit of adding Compound G treatment on top of Rosiglitazone treatment; whereas only 6 out of 10 rats responded to therapy in the Rosiglitazone monotherapy group, 10 out of 10 rats responded in the combination group.

| Treatment | Change in Blood glucose, mg/dl (day 25 vs day 0) | Responders^{a} |
|---|---|---|
| Control | +113±52 | 2/9 |
| Compound G | -120±34* | 8/9 |
| Rosiglitazone | -107±78* | 6/10 |
| Combination | -207±44* | 10/10 |

| | | |
|---|---|---|
| ^{a}Rats whose blood glucose levels were lower on day 25 than on day 0 | | |
| *p < 0.05 compared to control | | |

The variable response to Rosiglitazone observed in the ZDF rat in this study has also been encountered in the clinic where reductions in fasting plasma glucose observed have ranged from less than 45 mg/dl (60% of patients) to greater than 140 mg/dl (25% of patients) (Patel et al 1999). Based on the trend towards improved glycemic control as well as the higher response rate of rats in the combination versus other treatment groups, this study suggests that co-treatment with an FBPase inhibitor could benefit patients on Rosiglitazone monotherapy.

### Example S: Combination Treatment With Troglitazone and an FBPase Inhibitor (Compound G), in the db/db Mouse - Effects on Blood Glucose

The db/db mouse, like the ZDF rat, is a standard model of type 2 diabetes which displays many of the characteristics of human diabetes, including obesity, increased hepatic glucose output, insulin resistance, and hyperglycemia (Coleman and Hummel 1967). The purpose of this study was to determine whether combination treatment with an insulin sensitizer (Troglitazone) and an FBPase inhibitor (Compound G) could provide better antihyperglycemic activity than treatment with either agent alone.

Male db/db mice were purchased at 8 weeks of age from Jackson Labs (Bar Harbor, Maine). The mice were maintained under standard vivarium conditions (25°C, 12-hour light, 12-hour dark cycle) and received powdered Purina 5008 chow and water *ad libitum.* At 10 weeks of age, animals with blood glucose >400 mg/dl and <900 mg/dl were selected and divided into 4 treatment groups (n=5-6/group). The treatments were control, Troglitazone, Compound G, and the combination of Troglitazone and Compound G. Troglitazone was administered as an 0.1 % food admixture and Compound G as an 0.4% food admixture. Treatment was for 18 days. The dose of Troglitazone selected (0.1 %) is reported to exert the maximal glucose lowering possible in this model (Fujiwara et al., 1995). The dose of Compound G selected (0.4%) is also a maximal dose; a 6-day pilot study revealed that a higher dose (0.6%) was of no additional benefit. Blood glucose levels were measured in tail vein samples by means of a HemoCue glucose analyzer (HemoCue Inc., Mission Viejo, CA). Values are expressed as the mean plus or minus the standard error of the mean. Differences between groups were evaluated by analysis of variance with the Tukey-Kramer post hoc test. Results are considered significant with p<0.05.

As shown in the table below, on the last treatment day (day 18), blood glucose levels in the combination group were significantly lower than those in the control, as well as the Troglitazone and Compound G monotherapy groups.

| Blood Glucose, mg/dl | | |
|---|---|---|
| Treatment | Day 0 | Day 18 |
| Control | 707±65 | 870±32* |
| Compound G | 708±55 | 646±37 |
| Troglitazone | 710±44 | 509±70 |
| Combination | 709±42 | 263±49** |

| | | |
|---|---|---|
| * p<0.05 versus all groups | | |
| ** p<0.05 versus all groups | | |

Combination treatment with Troglitazone and Compound G profoundly reduced hyperglycemia in the db/db mouse and was significantly more efficacious than treatment with either agent alone. The data suggest that combination treatment with an insulin sensitizer and an FBPase inhibitor may be of clinical benefit in the treatment of type 2 diabetes.

### Example T: Combination Treatment With Troglitazone and an FBPase Inhibitor (Compound A), in the db/db Mouse - Effects on Blood Glucose

Treatment of mature db/db mice with maximal doses of Troglitazone results in a partial reduction of the hyperglycemia characteristic of this animal model of type 2 diabetes (Fujiwara et al, 1995). The aim of this study was to determine whether treatment with the FBPase inhibitor Compound A could further improve the glycemic control of db/db mice treated with a maximal dose of Troglitazone.

Male C57BL/KsJ-db/db mice were obtained from Jackson Laboratories (Bar Harbor, Maine) at 9 weeks of age. The mice were maintained under standard vivarium conditions (25°C, 12-hour light, 12-hour dark cycle) and received Harlan-Teklad mouse chow and water *ad libitum.* At approximately 11 weeks of age, mice with levels between 340 and 450 mg/dl were selected and divided into three statistically equivalent groups (n = 6/group). Group 1 (controls) received powdered Purina 5008 chow alone. Troglitazone was given as a powdered Purina 5008 admixture (0.1 %) for 7 days to Groups 2 and 3. This dose is reported to elicit the maximal hypoglycemic effect of Troglitazone in this model (Fujiwara et al 1995). Compound A was administered orally in polyethlene glycol (PEG 400) to Group 3 at a dose of 250 mg/kg on the 4th and 7th day of the study. Blood glucose levels were measured in tail vein samples by means of a HemoCue glucose analyzer (HemoCue Inc., Mission Viejo, CA). Values are expressed as the mean plus or minus the standard error of the mean. Differences between groups were evaluated by analysis of variance with the Tukey-Kramer post hoc test. Results are considered significant with p<0.05.

On the fourth day of the study, the Troglitazone-treated groups (2 and 3), as expected, showed significantly lower fed blood glucose values than the untreated controls (Group 1):

| Group | Treatment | Blood glucose, mg/dl (8 am day 4) |
|---|---|---|
| 1 | Control | 336.5 ± 13.4 |
| 2 | Troglitazone | 240 ± 21.3 * |
| 3 | Troglitazone | 237.5 ± 18.5* |

| | | |
|---|---|---|
| *p<0.05 vs Group 1 | | |

Following the above measurements, Group 3 was treated with a single oral dose of Compound A and Groups 1 and 2 with an equivalent volume of vehicle. Nine hours post administration, blood glucose levels were found to have increased in the control group (Group 1), to have stayed essentially the same in Group 2, but to have significantly decreased by 30% in the Compound A-treated group (Group 3):

| Group | Treatment | Blood glucose, mg/dl (5 pm day 4) |
|---|---|---|
| 1 | Control | 376.5 ± 8.4 |
| 2 | Troglitazone | 232.5 ± 17* |
| 3 | Troglitazone/Compound A | 167 ± 10.3** |

| | | |
|---|---|---|
| *p<0.05 vs Group 1 | | |
| *p<0.05 vs Groups 1 and 2 | | |

On the 7th day of the study, a second dose of Compound A (Group 3) or vehicle (Groups 1 and 2) was administered. Food was withheld for 6 hours post drug/vehicle administration. As shown in the table below, the mice in Group 3 (combination therapy) achieved blood glucose levels which were on average 44% lower than those of Group 2 (Troglitazone monotherapy) during the treatment period.

| Group | Treatment | Blood glucose, mg/dl 8 am, day 7 | 2 pm, day 7 |
|---|---|---|---|
| 1 | control) | 392 13.3 | 279.5±19. |
| 2 | Troglitazone | 237 25.5 | 195 ± 12.1* |
| 3 | Troglitazone/Compound A | 243 20.4 | 109.5 ± 10.7** |

| | | | |
|---|---|---|---|
| ^{*}p<0.05 vs Group 1 | | | |
| ^{**}p<0.05 vs Groups 1 and 2 | | | |

Combination treatment of Troglitazone and Compound A resulted in significantly lower blood glucose levels both in the fed and fasted state than when Troglitazone was administered alone. This study suggests that the addition of an FBPase inhibitor to the treatment regimen of patients on insulin sensitizer therapy could provide significantly improved glycemic control.

### References

Coleman DL, Hummel KP (1967) Diabetologia 3: 238-248

Fujiwara T et al (1995) Metabolism 44: 486-490

Inzucchi SE, Maggs DG, Spollett GR et al (1998) N. Engl. J. Med. 338: 867-872

Leahy JL (1990) Diabetes Care 13: 992-1010

Patel J, Anderson RJ, Rappaport EB (1999) Diabetes, Obesity & Metabolism 1: 165-172 Pickavance L, Widdowson PS, King P, Ishii S, Tanaka H, Williams G (1998) Br. J. Pharmacol. 125: 767-770

Smith S, Lister C, Hughes M, Buckingham R (1997) Diabetes 46, supplement 1, abstract 577

Sreenan S, Sturis J, Pugh W et al (1996) Am. J. Physiol. 271: E742-747

Unger RH (1997) Trends Endocrinol. Metab. 8: 276-281

## Claims

1. Use of an insulin sensitizer agent and an FBPase inhibitor or a prodrug or salt thereof in the manufacture of a medicament for preventing diabetes in an animal at risk of developing diabetes.

2. The use of claim 1 wherein said animal at risk of developing diabetes has a disease or condition which is impaired glucose tolerance, insulin resistance, hyperglycemia, obesity, accelerated gluconeogenesis, or increased hepatic glucose output.

3. Use of an insulin sensitizer agent and an FBPase inhibitor, or a prodrug or salt thereof in the manufacture of a medicament for treating impaired glucose tolerance.

4. Use of an insulin sensitizer agent and an FBPase inhibitor or a prodrug or salt thereof in the manufacture of a medicament for treating insulin resistance.

5. Use of an insulin sensitizer agent and an FBPase inhibitor or a prodrug or salt thereof in the manufacture of a medicament for treating or preventing a disease or condition which is hyperlipidemia, atherosclerosis, ischemic injury, hypertension or hypercholesterolemia.

6. Use of an insulin sensitizer and an FBPase inhibitor, or a prodrug or salt thereof in the manufacture of a medicament for the treatment of a cardiovascular complication associated with diabetes.

7. Use according to claim 6 in which the cardiovascular complication is hypertension.
